# EUROPEAN PATENT APPLICATION

(11) **EP 4 700 479 A1**
(43) Date of publication of application: **25.02.2026**
(21) Application number: 25196347.6
(22) Date of filing: 18.08.2025
(51) Int. Cl.: G03F 7/004, G03F 7/039, C07D 327/08, C07D 333/76, C07C 381/12

(54) **SULFONIUM SALT, CHEMICALLY AMPLIFIED POSITIVE RESIST COMPOSITION AND RESIST PATTERN FORMING PROCESS**

(30) Priority: 20.08.2024 JP 2024139117
(71) Applicant: Shin-Etsu Chemical Co., Ltd., Chiyoda-ku Tokyo 100-0005 (JP)
(72) Inventor: FUKUSHIMA, Masahiro, Joetsu-shi (JP)
(74) Representative: Ter Meer Steinmeister & Partner

(57) **Abstract**

A sulfonium salt monomer having the formula (A).

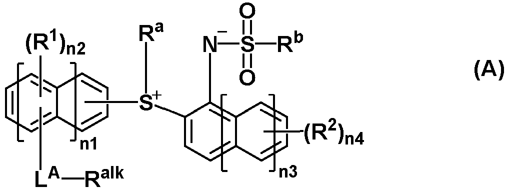

## Description

### TECHNICAL FIELD

This invention relates to a sulfonium salt, a chemically amplified positive resist composition and a resist pattern forming process.

### BACKGROUND ART

The currently increasing integration density of integrated circuits requires pattern formation to a smaller feature size. In the lithography process of forming patterns with a feature size of 0.2 µm or less, chemically amplified resist compositions utilizing acid as a catalyst are mostly used. Here, as the energy source for exposure, high-energy radiation such as UV, deep UV or EB is used. The EB lithography utilized as the ultrafine processing technology is indispensable for the processing of photomask blanks to produce photomasks for use in semiconductor device fabrication. The resist composition for use in the photolithography includes a positive resist composition for formation of a pattern in which an exposed region is dissolved and a negative resist composition for formation of a pattern in which an exposed region is retained. Of these, a resist composition is selected which is easier to use in light of a form of a required resist pattern.

In general, an image is written by EB without using a mask. In the case of positive resist, those regions of a resist film other than the regions to be retained are successively irradiated with EB having a minute area. In the case of negative resist, those regions of a resist film to be retained are successively irradiated. The operation of successively scanning all finely divided regions on the work surface takes a long time as compared with one-shot exposure through a photomask. To avoid any throughput decline, the resist film is required to have a high sensitivity. The long time of writing an image is likely to cause a difference between the region of an early written image and the region of a late written image. Temporal stability of the exposed region in vacuum is an important required performance item. One of the particularly important applications of chemically amplified resist material resides in processing of photomask blanks. Some photomask blanks have a surface material that can have an impact on the pattern profile of the overlying chemically amplified resist film, for example, a layer of a chromium compound, typically chromium oxide deposited on a photomask substrate. For high resolution and profile retention after etching, it is one important performance factor to maintain the profile of a resist film pattern rectangular independent of the type of substrate.

For the control of the sensitivity of the resist film and pattern profile, various improvements are made through a choice and combination of components in a resist composition and selection of processing conditions. One of such improvements addresses the problem of diffusion of acid that has a significant impact on the resolution of chemically amplified resist films. In the processing of photomasks, it is required that the profile of a resist pattern formed do not change with a lapse of time until post exposure baking (PEB). The major cause of such a change with time is diffusion of an acid generated upon exposure. The problem of acid diffusion has been widely studied not only in terms of photomask processing, but also in terms of general resist compositions because the acid diffusion has a significant impact on sensitivity and resolution.

Patent Documents 1 and 2 describe acid generators capable of generating bulky acids for controlling acid diffusion and reducing LER. Since these acid generators are still insufficient to control acid diffusion, it is desired to have an acid generator with shorter diffusion.

Patent Documents 3 to 6 disclose a resist composition comprising a resin to which a sulfonic acid generated upon exposure is bonded to control acid diffusion. This approach of controlling acid diffusion by introducing repeat units capable of generating acid upon exposure into a base polymer is effective in forming a pattern with small line edge roughness (LER). However, the base polymer having introduced therein repeat units capable of generating acid upon exposure sometimes encounters a problem with respect to its solubility in organic solvent, depending on the structure and proportion of the repeat units.

Polymers containing abundant aromatic skeletons with acidic side chains, for example, polyhydroxystyrene are used as the resist composition material for KrF lithography. These polymers are not used as the resist composition material for ArF lithography because of substantial absorption of light near to wavelength 200 nm. The above polymers are yet important, because of high etching resistance, as the resist compositions for the EB lithography and EUV lithography which are promising for forming patterns of smaller size than the processing limit of ArF lithography.

Often used as the base polymer in positive resist compositions for EB and EUV lithography is a material having an acidic functional group on phenol side chain masked with an acid labile group. Upon exposure to high-energy radiation, a photoacid generator generates an acid and the acid labile group (acid-decomposable protective group) is deprotected by the catalysis of the generated acid whereby the polymer turns soluble in alkaline developer. Tertiary alkyl, tert-butoxycarbonyl, and acetal groups are mainly used as the acid labile group. On use of acetal and similar protective groups requiring relatively small activation energy for deprotection, one advantage is that a resist film having a high sensitivity is obtained. Unless the diffusion of the generated acid is fully suppressed, however, deprotection reaction can take place even in the unexposed region of the resist film. There arise problems like degradation of LER and a loss of dimensional uniformity (CDU) of the pattern line width.

With the progress of miniaturization of resist patterns in recent years, it has been important to control diffusion of an acid generated from a photoacid generator, and various acid diffusion controlling agents (quenchers) have been proposed. Examples of the quencher include conventional amine compounds, and onium salt quenchers such as sulfonium salts and iodonium salts. Patent Document 7 discloses a carboxylic onium salt having a nitrogen-containing heterocyclic structure such as indole. Patent Documents 8 to 11 describe a quencher derived from a betaine onium salt in which a phenoxide anion, a carboxylate anion or a sulfonamidate anion is bonded to a sulfonium cation and an iodonium cation in one molecule. The use of such a quencher has been confirmed to improve performance to some extent, but not to a satisfactory extent. To meet the future demand for further miniaturization, development of a resist composition that ensures good lithographic performance and a good pattern profile is desired.

### [Citation List]

| | |
|---|---|
| Patent Document 1: | JP-A 2009-053518 |
| Patent Document 2: | JP-A 2010-100604 |
| Patent Document 3: | JP 4425776 |
| Patent Document 4: | JP 5201363 |
| Patent Document 5: | JP 5231357 |
| Patent Document 6: | WO 2008/081832 |
| Patent Document 7: | JP 6515831 |
| Patent Document 8: | JP 6848776 |
| Patent Document 9: | JP 6583136 |
| Patent Document 10: | JP 6246480 |
| Patent Document 11: | JP 7031537 |

### SUMMARY OF THE INVENTION

In recent years, there has been a demand for a resist composition that is excellent not only in line-and-space (LS), iso-line (IL), and iso-space (IS) but also hole pattern profile and profile after pattern formation.

The invention has been made in view of the above-described circumstances, and an object of the invention is to provide a chemically amplified positive resist composition which is processed by photolithography using, in particular, high-energy radiation such as KrF excimer laser, ArF excimer laser, EB or EUV, has good solvent solubility, and is excellent in lithographic performance such as resolution, LER and a pattern profile, and a pattern forming process using the chemically amplified resist composition.

The inventors have found that by using a betaine sulfonium salt having a C₆-C₁₈ chain alkyl group or a C₄-C₁₈ chain fluorinated alkyl group, and a sulfonium cation and a sulfoneamidate anion in one molecule, as a chemically amplified positive resist composition material, there is obtained a chemically amplified positive resist composition which is processed by, in particular, EB lithography and EUV lithography, has good solvent solubility, and is excellent in lithographic performance such as resolution, LER and a pattern profile.

In one aspect, the present invention provides a sulfonium salt having the formula (A).

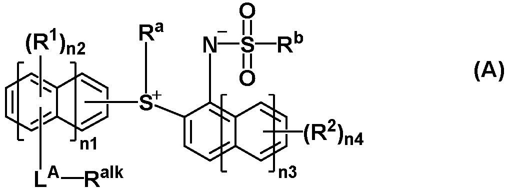

Herein n1 is 0 or 1, n2 is 0, 1, 2, 3, or 4, n3 is 0 or 1, n4 is 0, 1, 2, 3 or 4,
L^{A} is a single bond, ether bond, ester bond, sulfonic ester bond, carbonate bond, or carbamate bond,
R^{alk} is a C₆-C₁₈ alkyl group or a C₄-C₁₈ fluorinated alkyl group, wherein when R^{alk} is a C₆-C₁₈ alkyl group, the alkyl group has at least one straight chain structure having 6 or more carbon atoms, and when R^{alk} is a C₄-C₁₈ fluorinated alkyl group, the fluorinated alkyl group has at least two groups selected from -CF₂- and -CF₃, some constituent -CH₂- in the alkyl group and fluorinated alkyl group may be replaced by an ether bond or a carbonyl group, and the alkyl group and fluorinated alkyl group may contain a ring structure selected from a cyclopentane ring, a cyclohexane ring, an adamantane ring, a norbornene ring and a benzene ring at an end or in a carbon-carbon bond,
R^{a} is a C₁-C₃₀ hydrocarbyl group which may contain halogen atom or a heteroatom, two of R^{a} and two aromatic rings bonded to S⁺ may bond together to form a ring with a sulfur atom to which they are attached,
R^{b} is a C₁-C₁₈ hydrocarbyl group which may contain a heteroatom,
R¹ is halogen, nitro group, cyano group, hydroxy group, a C₁-C₂₀ hydrocarbyl group which may contain a heteroatom, C₁-C₂₀ hydrocarbyloxy group which may contain a heteroatom, a C₁-C₂₀ hydrocarbyloxy group, or a C₁-C₂₀ hydrocarbylthio group which may contain a heteroatom, a plurality of R¹ may be identical or different and a plurality of R¹ may bond together to form a ring with the carbon atoms to which they are attached, when n2 is 2, 3 or 4,
R² is halogen, nitro group, cyano group, hydroxy group, a C₁-C₂₀ hydrocarbyl group which may contain a heteroatom, a C₁-C₂₀ hydrocarbyloxy group which may contain a heteroatom, or a C₁-C₂₀ hydrocarbylthio group which may contain a heteroatom, and a plurality of R² may be identical or different and a plurality of R² may bond together to form a ring with the carbon atoms to which they are attached, when n4 is 2, 3 or 4.

In a preferred embodiment, the sulfonium salt has the formula (A1).

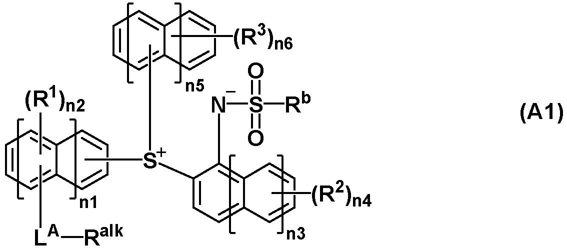

Herein n1 to n4, L^{A}, R^{alk}, R^{a}, R^{b}, R¹ and R² are as defined above,
n5 is 0 or 1, n6 is 0, 1, 2, 3, or 4,
R³ is halogen, nitro group, cyano group, hydroxy group, a C₁-C₂₀ hydrocarbyl group which may contain a heteroatom, a C₁-C₂₀ hydrocarbyloxy group which may contain a heteroatom, or a C₁-C₂₀ hydrocarbylthio group which may contain a heteroatom, and a plurality of R³ may be identical or different and a plurality of R³ may bond together to form a ring with the carbon atoms to which they are attached, when n6 is 2, 3 or 4.

In a preferred embodiment, the sulfonium salt has the formula (A2).

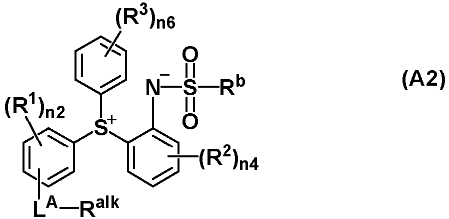

Herein n2, n4, n6, L^{A}, R^{alk}, R^{a}, R^{b}, R¹, R² and R³ are as defined above.

In another aspect, the invention provides a quencher in the form of the sulfonium salt defined herein.

In another aspect, the invention provides a chemically amplified positive resist composition comprising the quencher defined herein.

In a preferred embodiment, the chemically amplified positive resist composition comprises a base polymer. The base polymer comprises a polymer adapted to increase its solubility in an alkaline developer by degrading under the action of an acid.

In a preferred embodiment, the polymer further comprises repeat units having the formula (B1).

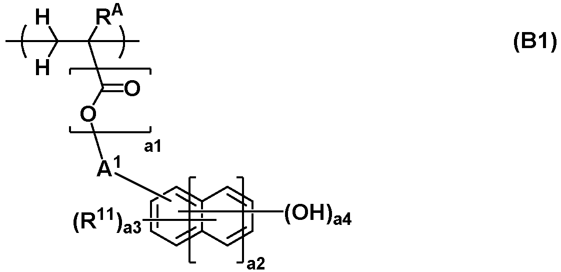

Herein a1 is 0 or 1, a2 is 0, 1 or 2, a3 is an integer which satisfies 0 ≤ a3 ≤ 5+2(a2)-a4, a4 is 1, 2 or 3,
R^{A} is hydrogen, fluorine, methyl group or trifluoromethyl group,
R¹¹ is a halogen atom, nitro group, carboxy group, a C₁-C₆ saturated hydrocarbyl group which may be substituted with a halogen atom, a C₁-C₆ saturated hydrocarbyloxy group which may be substituted with a halogen atom, or a C₂-C₈ saturated hydrocarbylcarbonyloxy group which may be substituted with a halogen atom, and
A¹ is a single bond or C₁-C₁₀ saturated hydrocarbylene group in which some constituent -CH₂- may be replaced by -O-.

In a preferred embodiment, the polymer further comprises repeat units having the formula (B2-1).

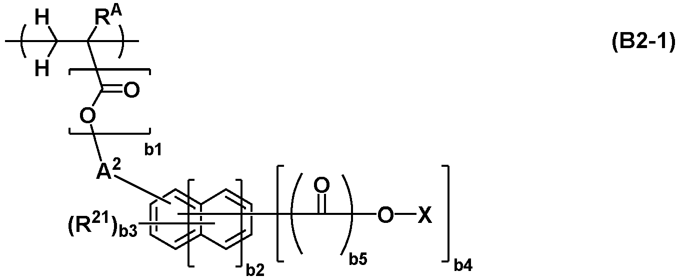

Herein R^{A} is hydrogen, fluorine, methyl, or trifluoromethyl,
b1 is 0 or 1, b2 is 0, 1 or 2, b3 is an integer which satisfies 0 ≤ b3 ≤ 5+2(b2)-b4, b4 is 1, 2 or 3, b5 is 0 or 1,
R²¹ is a halogen atom, C₁-C₆ saturated hydrocarbyl group which may be substituted with a halogen atom, C₁-C₆ saturated hydrocarbyloxy group which may be substituted with a halogen atom, or C₂-C₈ saturated hydrocarbylcarbonyloxy group which may be substituted with a halogen atom,
A² is a single bond or C₁-C₁₀ saturated hydrocarbylene group in which some constituent -CH₂- may be replaced by -O-, and
X is an acid labile group when b4 is 1, and X is hydrogen or an acid labile group, at least one being an acid labile group, when b4=2 or 3.

In a preferred embodiment, the polymer further comprises repeat units having the formula (B2-2).

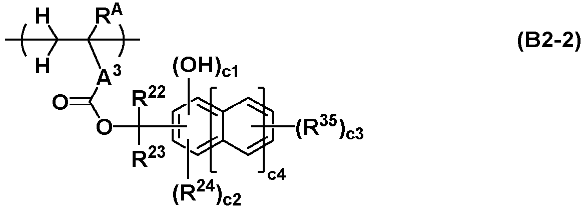

Herein c1 is 0, 1 or 2, c2 is 0, 1 or 2, c3 is 0, 1, 2, 3, 4 or 5, c4 is 0, 1 or 2,
R^{A} is hydrogen, fluorine, methyl group or trifluoromethyl group,
R²² and R²³ are each independently a C₁-C₁₀ hydrocarbyl group which may contain a heteroatom, and R²² and R²³ may bond together to form a ring with the carbon atom to which they are attached,
R²⁴ is each independently a fluorine atom, C₁-C₅ fluorinated alkyl group or C₁-C₅ fluorinated alkoxy group,
R²⁵ is each independently a C₁-C₁₀ hydrocarbyl group which may contain a heteroatom,
A³ is a single bond, phenylene group, naphthylene group or *-C(=O)-O-A³¹-, and A³¹ is a C₁-C₂₀ aliphatic hydrocarbylene group which may contain a hydroxy group, ether bond, ester bond or lactone ring, or a phenylene or naphthylene group, * designates a point of attachment to the carbon atom in the backbone.

In a preferred embodiment, the polymer further comprises repeat units of at least one type selected from repeat units having the formula (B3), repeat units having the formula (B4) and repeat units having the formula (B5).

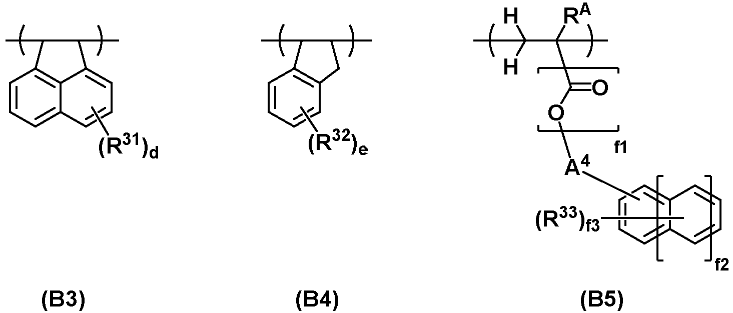

Herein d is 0, 1, 2, 3, 4, 5 or 6, e is 0, 1, 2, 3 or 4, f1 is 0 or 1, f2 is 0, 1 or 2, f3 is 0, 1, 2, 3, 4 or 5,
R^{A} is hydrogen, fluorine, methyl group or trifluoromethyl group,
R³¹ and R³² are each independently hydroxy group, halogen, a C₁-C₆ saturated hydrocarbyl group which may be substituted with a halogen atom, a C₁-C₆ saturated hydrocarbyloxy group which may be substituted with a halogen atom, or a C₂-C₈ saturated hydrocarbylcarbonyloxy group which may be substituted with halogen,
R³³ is a C₁-C₂₀ saturated hydrocarbyl group, a C₁-C₂₀ saturated hydrocarbyloxy group, a C₂-C₂₀ saturated hydrocarbylcarbonyloxy group, a C₂-C₂₀ saturated hydrocarbyloxyhydrocarbyl group, a C₂-C₂₀ saturated hydrocarbylthiohydrocarbyl group, halogen atom, nitro group, or cyano group, and may be a hydroxy group when f2 is 1 or 2, and
A⁴ is a single bond or C₁-C₁₀ saturated hydrocarbylene group in which some constituent -CH₂- may be replaced by -O-.

In a preferred embodiment, the polymer further comprises repeat units of at least one type selected from repeat units having the formula (B6), repeat units having the formula (B7), repeat units having the formula (B8), repeat units having the formula (B9), and repeat units having the formula (B10).

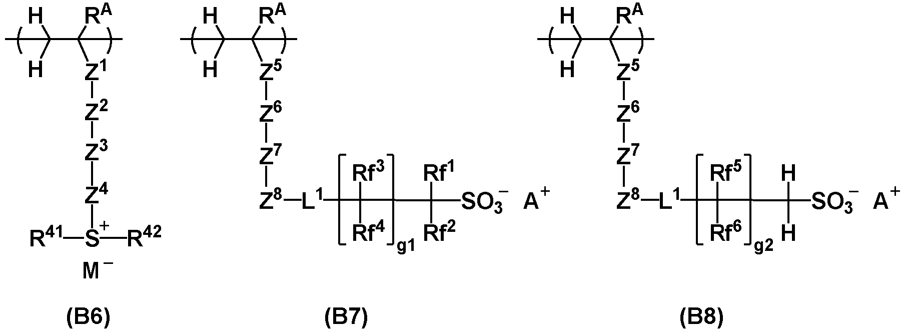

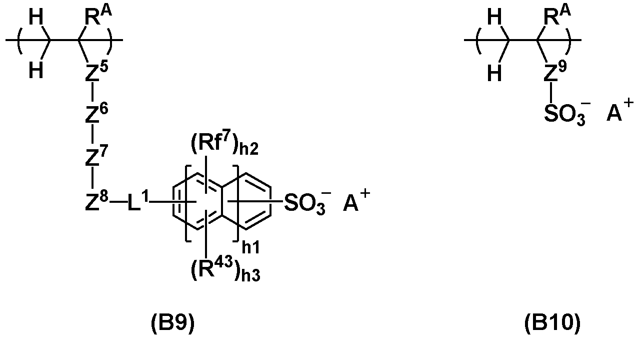

Herein g1 and g2 are each independently 0, 1, 2 or 3, h1 is 0 or 1, h2 is 0, 1, 2, 3 or 4, h3 is 0, 1, 2, 3 or 4, h2+h3 is from 0 to 4 when h1 is 0, and h2+h3 is from 0 to 6 when h1 is 1,
R^{A} is each independently hydrogen, fluorine, methyl group or trifluoromethyl group,
Z¹ is a single bond or optionally substituted phenylene group,
Z² is a single bond, *-C(=O)-O-Z²¹-, **-C(=O)-NH-Z²¹- or **-O-Z²¹-, Z²¹ is a C₁-C₆ aliphatic hydrocarbylene group, a phenylene group or a divalent group obtained by combining the foregoing, which may contain halogen, carbonyl moiety, ester bond, ether bond or hydroxy group,
Z³ is a single bond, ether bond, ester bond, amide bond, sulfonate ester bond, sulfonamide bond, carbonate bond or carbamate bond,
Z⁴ is a single bond, or a C₁-C₆ aliphatic hydrocarbylene group, phenylene group or a divalent group obtained by combining the foregoing, which may contain halogen, carbonyl group, ester bond, ether bond or hydroxy group,
Z⁵ is each independently a single bond, optionally substituted phenylene group, naphthylene group, or *-C(=O)-O-Z⁵¹-, Z⁵¹ is a C₁-C₁₀ aliphatic hydrocarbylene group which may contain halogen, hydroxy moiety, ether bond, ester bond or lactone ring, or phenylene or naphthylene group,
Z⁶ is a single bond, ether bond, ester bond, amide bond, sulfonate ester bond, sulfonamide bond, carbonate bond or carbamate bond,
Z⁷ is each independently a single bond, ***-Z⁷¹-C(=O)-O-, ***-C(=O)-NH-Z⁷¹-, or ***-O-Z⁷¹-, Z⁷¹ is a C₁-C₂₀ hydrocarbylene group which may contain a heteroatom,
Z⁸ is each independently a single bond, ****-Z⁸¹-C(=O)-O-, ****-C(=O)-NH-Z⁸¹-, or ****-O-Z⁸¹-, Z⁸¹ is a C₁-C₂₀ hydrocarbylene group which may contain a heteroatom,
Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, Z⁹¹ is a C₁-C₆ aliphatic hydrocarbylene group, phenylene group, fluorinated phenylene group or trifluoromethyl substituted phenylene group, which may contain a carbonyl moiety, ester bond, ether bond or hydroxy group,
* designates a point of attachment to the carbon atom in the backbone, ** designates a point of attachment to Z^{1,} *** designates a point of attachment to Z^{6,} **** designates a point of attachment to Z^{7,}
L¹ is a single bond, ether bond, ester bond, carbonyl group, sulfonate ester bond, sulfonamide bond, carbonate bond or carbamate bond,
Rf¹ and Rf² are each independently fluorine, or a C₁-C₆ fluorinated saturated hydrocarbyl group,
Rf³ and Rf⁴ are each independently hydrogen, fluorine, or a C₁-C₆ fluorinated saturated hydrocarbyl group,
Rf⁵ and Rf⁶ are each independently hydrogen, fluorine, or a C₁-C₆ fluorinated saturated hydrocarbyl group, it is excluded that all Rf⁵ and Rf⁶ are hydrogen at the same time,
Rf⁷ is fluorine, a C₁-C₆ fluorinated alkyl group, C₁-C₆ fluorinated alkoxy group or C₁-C₆ fluorinated alkylthio group,
R⁴¹ and R⁴² are each independently a C₁-C₂₀ hydrocarbyl group which may contain a heteroatom, R⁴¹ and R⁴² may bond together to form a ring with sulfur to which they are attached,
R⁴³ is halogen exclusive of iodine, or a C₁-C₂₀ hydrocarbyl group which may contain a heteroatom, a plurality of R⁴³ may be identical or different and a plurality of R⁴³ may bond together to form a ring with the carbon atoms to which they are attached, when h3 is 2, 3 or 4,
M⁻ is a non-nucleophilic counter ion, and
A⁺ is an onium cation.

In a preferred embodiment, repeat units having an aromatic ring structure account for at least 60 mol% of the overall repeat units of the polymer in the base polymer.

The resist composition may further comprise an organic solvent.

The resist composition may further comprise a photoacid generator.

The resist composition may further comprise a fluorinated polymer comprising repeat units of at least one type selected from repeat units having the formula (E1), repeat units having the formula (E2), repeat units having the formula (E3) and repeat units having the formula (E4) and optionally repeat units of at least one type selected from repeat units having the formula (E5) and repeat units having the formula (E6).

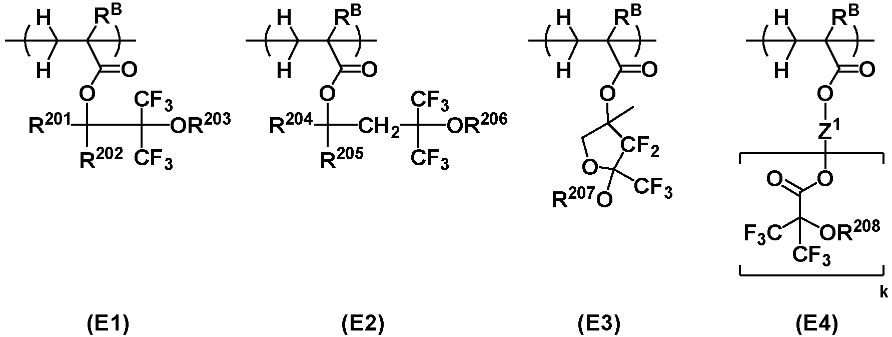

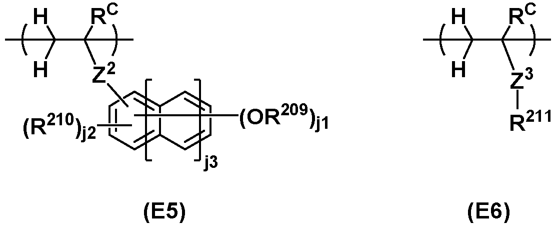

Herein j1 is 1, 2 or 3, j2 is an integer meeting 0 ≤ j2 ≤ 5+2(j3)-j1, j3 is 0 or 1, k is an integer of 1, 2 or 3,
R^{B} is each independently hydrogen, fluorine, methyl, or trifluoromethyl,
R^{C} is each independently hydrogen or methyl,
R²⁰¹, R²⁰², R²⁰⁴ and R²⁰⁵ are each independently hydrogen or a C₁-C₁₀ saturated hydrocarbyl group,
R²⁰³, R²⁰⁶, R²⁰⁷ and R²⁰¹ are each independently hydrogen, a C₁-C₁₅ hydrocarbyl group, C₁-C₁₅ fluorinated hydrocarbyl group, or acid labile group, and when R²⁰³, R²⁰⁶, R²⁰⁷ and R²⁰⁸ each are a hydrocarbyl or fluorinated hydrocarbyl group, an ether bond or carbonyl moiety may intervene in a carbon-carbon bond,
R²⁰⁹ is hydrogen or a C₁-C₅ straight or branched hydrocarbyl group in which a heteroatom-containing moiety may intervene in a carbon-carbon bond,
R²¹⁰ is a straight or branched C₁-C₅ hydrocarbyl group in which a group containing a heteroatom may intervene in a carbon-carbon bond,
R²¹¹ is a C₁-C₂₀ saturated hydrocarbyl group in which at least one hydrogen is substituted by fluorine, and in which some constituent -CH₂- may be replaced by an ester bond or ether bond,
Z¹ is a C₁-C₂₀ (k+1)-valent hydrocarbon group or C₁-C₂₀ (k+1)-valent fluorinated hydrocarbon group,
Z² is a single bond, *-C(=O)-O- or *-C(=O)-NH-, * designates a point of attachment to the carbon atom in the backbone,
Z³ is a single bond, -O-, *-C(=O)-O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond or sulfonamide bond, and * designates a point of attachment to the carbon atom in the backbone.

The chemically amplified positive resist composition may further comprise a quencher exclusive of the quencher defined above. In another aspect, the invention provides a resist pattern forming process comprising the steps of:
applying the chemically amplified positive resist composition defined herein onto a substrate to form a resist film thereon,
exposing the resist film patternwise to high-energy radiation, and
developing the exposed resist film in an alkaline developer.

Typically, the high-energy radiation is EUV of wavelength 3 to 15 nm or EB.

In an embodiment, the substrate has the outermost surface of a chromium containing material.

Typically, the substrate is a photomask blank.

### ADVANTAGEOUS EFFECTS OF THE INVENTION

When processed by the microfabrication technology, especially EB and EUV lithography processes, the inventive chemically amplified positive resist composition can form a resist pattern having a very high resolution and reduced LER. The chemically amplified positive resist composition provides a pattern of good rectangular profile.

### DESCRIPTION OF THE PREFERRED EMBODIMENT

Hereinafter, the present invention is described in detail. It is understood that for some structures represented by chemical formulae, there can exist enantiomers and diastereomers because of the presence of asymmetric carbon atoms. In such a case, a single formula collectively represents all such isomers. The isomers may be used alone or in admixture.

### [Sulfonium salt]

The inventive sulfonium salt has the formula (A).

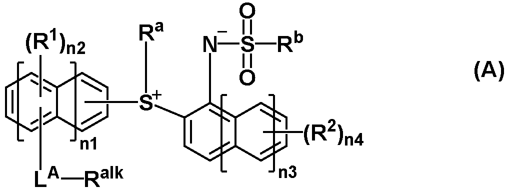

In formula (A), n1 is 0 or 1. The relevant structure is a benzene ring when n1=0, and a naphthalene ring when n1=1. The benzene ring corresponding to n1=0 is preferred from the aspect of solvent solubility. The subscript n2 is 0, 1, 2, 3 or 4, and n2 is preferably 0, 1 or 2 from the aspect of reactant availability. The subscript n3 is 0 or 1. The relevant structure is a benzene ring when n3=0, and a naphthalene ring when n3=1. The benzene ring corresponding to n3=0 is preferred from the aspect of solvent solubility. The subscript n4 is 0, 1, 2, 3 or 4, and n4 is preferably 0, 1 or 2 from the aspect of reactant availability.

In formula (A), L^{A} is a single bond, ether bond, ester bond, sulfonic ester bond, carbonate bond, or carbamate bond. Inter alia, L^{A} is preferably a single bond, ether bond, ester bond or sulfonic ester bond, more preferably ether bond or ester bond.

In formula (A), R^{alk} is a C₆-C₁₈ alkyl group or a C₄-C₁₈ fluorinated alkyl group.

When R^{alk} is a C₆-C₁₈ alkyl group, the alkyl group has at least one straight chain structure having 6 or more carbon atoms. Examples of the C₆-C₁₈ alkyl group R^{alk} include 1-hexyl, 1-heptyl, 1-octyl, 1-nonyl, 1-decyl, 1-undecyl, 1-dodecyl, 1-tridecyl, 1-tetradecyl, 1-hexadecyl, 1-octadecyl, octan-2-yl, decan-2-yl, decan-4-yl, octadecan-8-yl, 7,7-dimethyloctyl, 7,7-diethylnonyl, and 4-butyldodecyl groups. In the alkyl group, some constituent -CH₂- may be replaced by an ether bond or a carbonyl group, and a ring structure, for example, a cyclopentane ring, a cyclohexane ring, an adamantane ring, a norbornene ring or a benzene ring may be present at an end or in a carbon-carbon bond. R^{alk} is preferably a straight alkyl group or a straight glyme chain.

When R^{alk} is a C₄-C₁₈ fluorinated alkyl group, the fluorinated alkyl group has at least two groups selected from -CF₂- and -CF₃. Examples of the C₄-C₁₈ fluorinated alkyl group R^{alk} include groups obtained by replacing some or all hydrogen atoms of 1-butyl, 1-pentyl, 1-hexyl, 1-heptyl, 1-octyl, 1-nonyl, 1-decyl, 1-undecyl, 1-dodecyl, 1-tridecyl, 1-tetradecyl, 1-hexadecyl, 1-octadecyl, octan-2-yl, decan-2-yl, decan-4-yl, octadecan-8-yl, 7,7-dimethyloctyl, 7,7-diethylnonyl, 4-butyldodecyl groups or the like by fluorine. In the fluorinated alkyl group, some constituent -CH₂- may be replaced by an ether bond or a carbonyl group, and a ring structure, for example, a cyclopentane ring, a cyclohexane ring, an adamantane ring, a norbornyl ring or a benzene ring may be present at an end or in a carbon-carbon bond.

Preferred R^{alk} is shown below, but not limited thereto. The broken line designates a point of attachment.

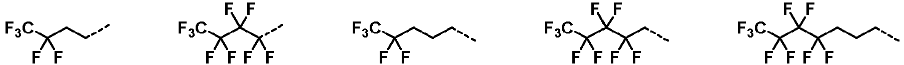

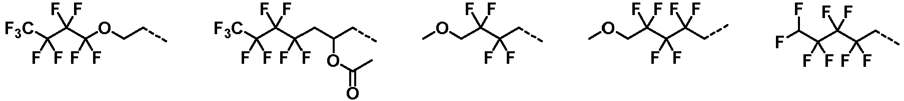

In formula (A), R^{a} is halogen, or a C₁-C₃₀ hydrocarbyl group which may contain a heteroatom.

Examples of the halogen R^{a} include fluorine, chlorine, bromine, and iodine.

The hydrocarbyl group R^{a} may be saturated or unsaturated, and may be straight, branched, or cyclic. Examples thereof include C₁-C₃₀ alkyl groups such as methyl, ethyl, n-propyl, isopropyl, n-butyl, sec-butyl and tert-butyl groups; C₃-C₃₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl groups; C₂-C₃₀ alkenyl groups such as vinyl, allyl, propenyl, butenyl and hexenyl groups; C₃-C₃₀ cyclic unsaturated hydrocarbyl groups such as a cyclohexenyl group; C₆-C₃₀ aryl groups such as phenyl, naphthyl and thienyl groups; C₇-C₃₀ aralkyl groups such as benzyl, 1-phenylethyl and 2-phenylethyl groups; and combinations thereof. The aryl groups are preferred. In the hydrocarbyl group, some or all hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain hydroxy, fluorine, chlorine, bromine, iodine, cyano, nitro, carbonyl, ether bond, ester bond, sulfonic ester bond, carbonate bond, lactone ring, sultone ring, carboxylic anhydride (-C(=O)-O-C(=O)-), or haloalkyl moiety.

Two of R^{a} and two aromatic rings bonded to S⁺ may bond together to form a ring with a sulfur atom to which they are attached. Examples of the structure of the ring include those represented by the formula.

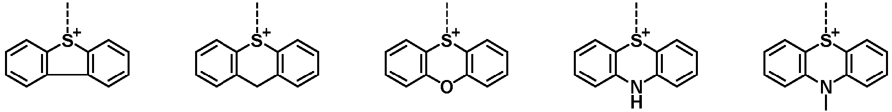

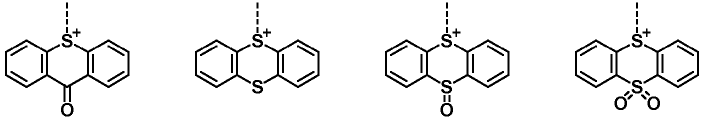

Herein the broken line designates a point of attachment to the remaining one substituent.

In formula (A), R^{b} is a C₁-C₁₈ hydrocarbyl group which may contain a heteroatom. The hydrocarbyl group may be saturated or unsaturated, and may be straight, branched, or cyclic. Examples thereof include C₁-C₁₈ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl and octadecyl groups; C₃-C₁₈ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl groups; C₂-C₁₈ alkenyl groups such as vinyl, allyl, propenyl, butenyl and hexenyl groups; C₃-C₁₈ cyclic unsaturated hydrocarbyl groups such as a cyclohexenyl group; C₆-C₁₈ aryl groups such as phenyl, methylphenyl, ethylphenyl, n-propylphenyl, isopropylphenyl, n-butylphenyl, isobutylphenyl, sec-butylphenyl, tert-butylphenyl and naphthyl groups; C₇-C₁₈ aralkyl groups such as benzyl, 1-phenylethyl, 2-phenylethyl, 1-naphthylethyl and 2-naphthylethyl groups; and combinations thereof. Of these, aryl groups are preferred. Some or all of hydrogen atoms of the hydrocarbyl group may be replaced by a group containing a heteroatom such as oxygen, sulfur, nitrogen or halogen, some constituent -CH₂-of the hydrocarbyl group may be replaced by a group containing a heteroatom such as oxygen, sulfur or nitrogen, and as a result, the hydrocarbyl group may contain a hydroxy group, a cyano group, fluorine, chlorine, bromine, iodine, a carbonyl group, an ether bond, an ester bond, a sulfonic ester bond, a carbonate bond, a lactone ring, a sultone ring, carboxylic anhydride (-C(=O)-O-C(=O)-), a haloalkyl group, or the like. R^{b} is preferably methyl, ethyl, phenyl, tolyl, trifluoromethyl or the like.

In formula (A), R¹ is halogen, nitro group, cyano group, hydroxy group, a C₁-C₂₀ hydrocarbyl group which may contain a heteroatom, a C₁-C₂₀ hydrocarbyloxy group which may contain a heteroatom, or a C₁-C₂₀ hydrocarbylthio group which may contain a heteroatom. The halogen is preferably fluorine, chlorine, bromine or iodine, more preferably fluorine or iodine. The hydrocarbyl group may be saturated or unsaturated, and may be straight, branched, or cyclic. Examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl and icocyl groups; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl groups; C₂-C₂₀ alkenyl groups such as vinyl, allyl, propenyl, butenyl and hexenyl groups; C₃-C₂₀ cyclic unsaturated hydrocarbyl groups such as a cyclohexenyl group; C₆-C₂₀ aryl groups such as phenyl and naphthyl groups; C₇-C₂₀ aralkyl groups such as benzyl, 1-phenylethyl and 2-phenylethyl groups; and combinations thereof. Of these, aryl groups are preferred. Some or all of hydrogen atoms of the hydrocarbyl group may be replaced by a group containing a heteroatom such as oxygen, sulfur, nitrogen or halogen, some constituent -CH₂- of the hydrocarbyl group may be replaced by a group containing a heteroatom such as oxygen, sulfur or nitrogen, and as a result, the hydrocarbyl group may contain a hydroxy group, a cyano group, fluorine, chlorine, bromine, iodine, a carbonyl group, an ether bond, an ester bond, a sulfonic ester bond, a carbonate bond, a lactone ring, a sultone ring, carboxylic anhydride (-C(=O)-O-C(=O)-), a haloalkyl group, or the like. A plurality of R¹ may be identical or different when n2 is 2, 3 or 4.

A plurality of R¹ may bond together to form a ring with the carbon atoms to which they are attached when n2 is 2, 3 or 4. Examples of the ring formed herein include cyclopropane, cyclobutane, cyclopentane, cyclohexane, norbornane and adamantane rings. Some or all of hydrogen atoms in the ring may be replaced by a group containing a heteroatom such as oxygen, sulfur, nitrogen or halogen, some constituent -CH₂- in the ring may be replaced by a group containing a heteroatom such as oxygen, sulfur or nitrogen, and as a result, the hydrocarbyl group may contain a hydroxy group, fluorine, chlorine, bromine, iodine, a cyano group, a carbonyl group, an ether bond, an ester bond, a sulfonic ester bond, a carbonate bond, a lactone ring, a sultone ring, carboxylic anhydride (-C(=O)-O-C(=O)-), a haloalkyl group, or the like.

In formula (A), R² is halogen, nitro group, cyano group, hydroxy group, a C₁-C₂₀ hydrocarbyl group which may contain a heteroatom, a C₁-C₂₀ hydrocarbyloxy group which may contain a heteroatom, or a C₁-C₂₀ hydrocarbylthio group which may contain a heteroatom. The halogen is preferably fluorine, chlorine, bromine or iodine, more preferably fluorine or iodine. The hydrocarbyl group may be saturated or unsaturated, and may be straight, branched, or cyclic. Examples of the hydrocarbyl group are as exemplified above as a hydrocarbyl group R¹, but not limited thereto. A plurality of R² may be identical or different when n4 is 2, 3 or 4.

A plurality of R² may bond together to form a ring with the carbon atoms to which they are attached when n4 is 2, 3 or 4. Examples of the ring formed herein are as exemplified above for the ring that a plurality of R¹ bonding together may form with the carbon atom to which they are attached, but the ring is not limited thereto.

Preferably, the sulfonium salt of formula (A) has the formula (A1).

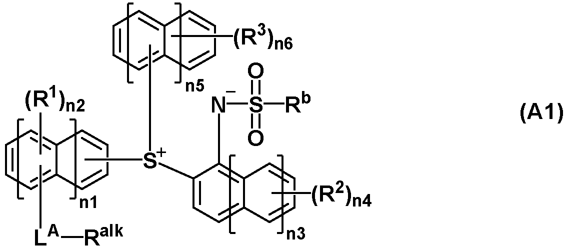

Herein n1 to n4, L^{A}, R^{alk}, R^{a}, R¹ and R² are as defined above.

In formula (A1), n5 is 0 or 1. The relevant structure is a benzene ring when n5=0, and a naphthalene ring when n5=1. The benzene ring corresponding to n5=0 is preferred from the aspect of solvent solubility. The subscript n6 is 0, 1, 2, 3 or 4, and n6 is preferably 0, 1 or 2 from the aspect of reactant availability.

In formula (A1), R³ is halogen, nitro group, cyano group, hydroxy group, a C₁-C₂₀ hydrocarbyl group which may contain a heteroatom, a C₁-C₂₀ hydrocarbyloxy group which may contain a heteroatom, or a C₁-C₂₀ hydrocarbylthio group which may contain a heteroatom. The halogen is preferably fluorine, chlorine, bromine or iodine, more preferably fluorine or iodine. The hydrocarbyl group may be saturated or unsaturated, and may be straight, branched, or cyclic. Examples thereof are as exemplified for the hydrocarbyl group R¹ in formula (A), but not limited thereto. A plurality of R³ may be identical or different when n6 is 2, 3 or 4.

A plurality of R³ may bond together to form a ring with the carbon atoms to which they are attached when n6 is 2, 3 or 4. Examples of the ring formed herein are as exemplified above for the ring that a plurality of R¹ bonding together may form with the carbon atom to which they are attached in formula (A), but the ring is not limited thereto.

Preferably, the sulfonium salt of formula (A1) has the formula (A2).

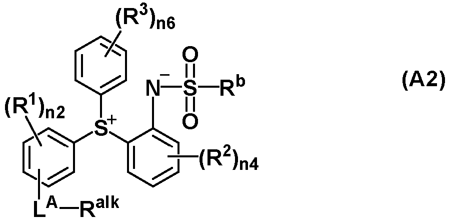

Herein n2, n4, n6, L^{A}, R^{alk}, R^{a}, R^{b}, R¹, R² and R³ are as defined above.

Examples of the sulfonium salt having the formula (A) are shown below, but not limited thereto.

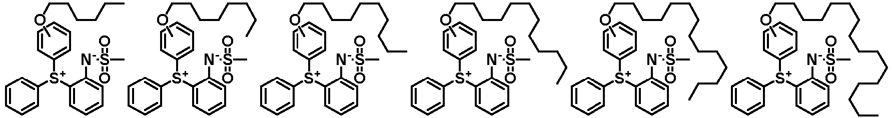

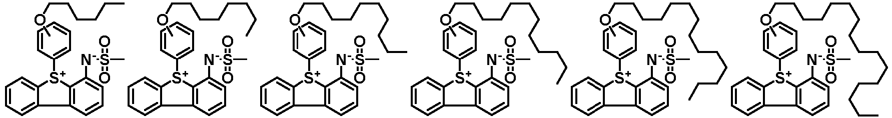

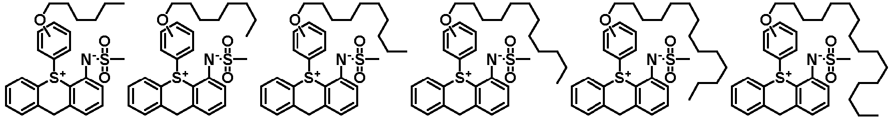

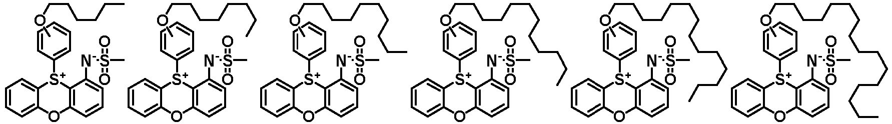

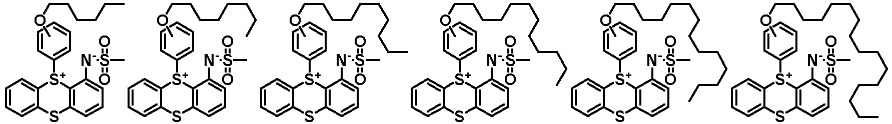

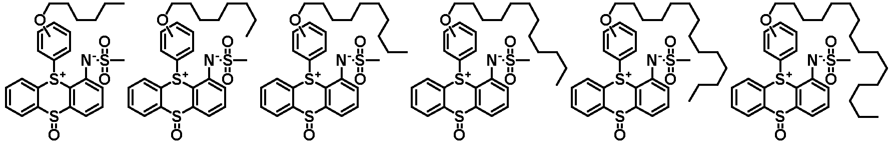

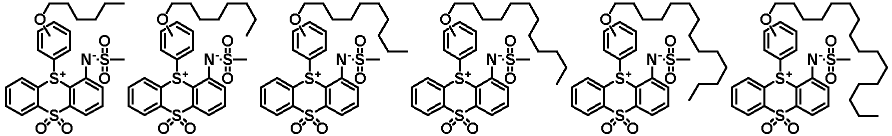

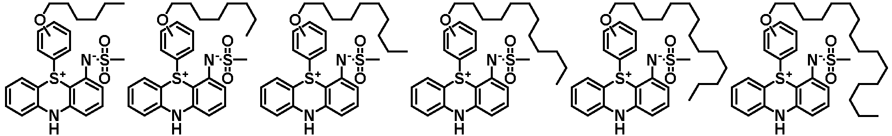

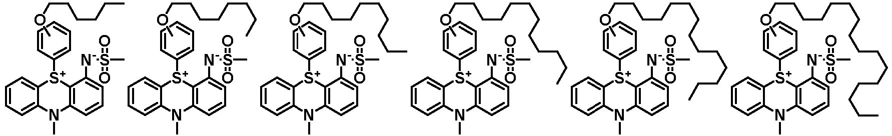

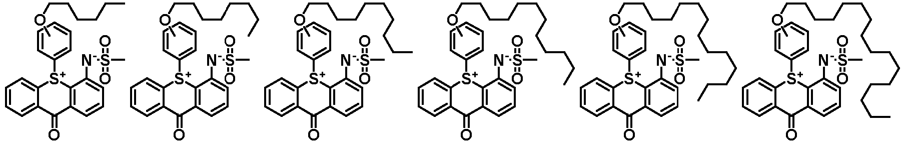

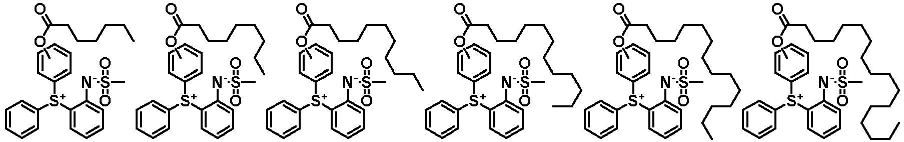

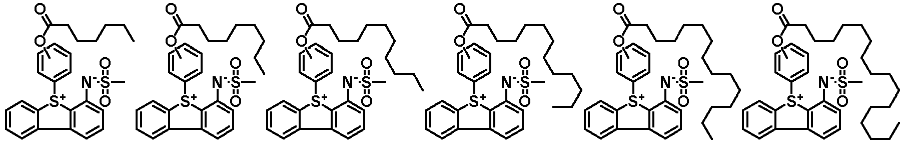

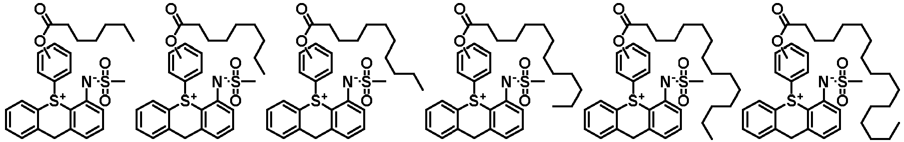

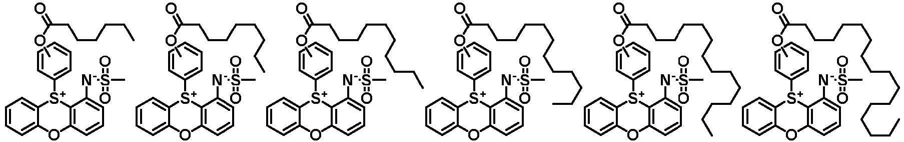

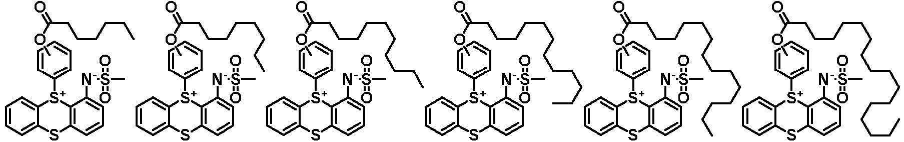

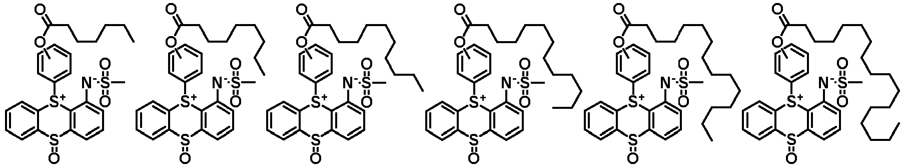

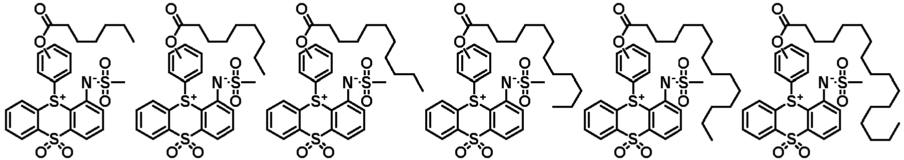

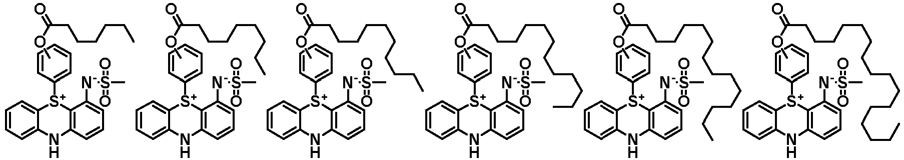

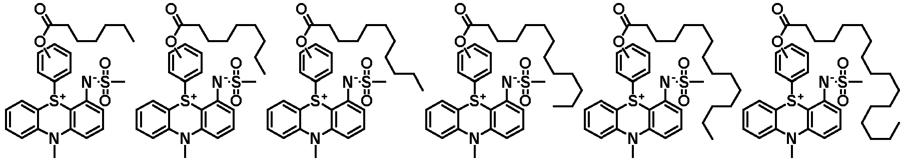

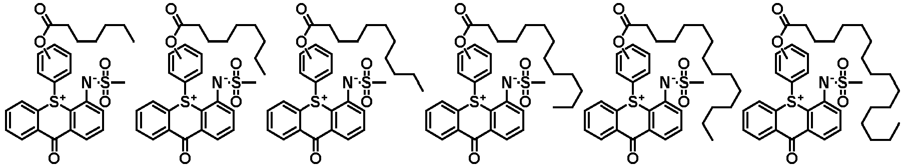

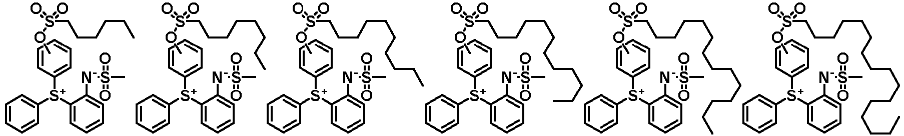

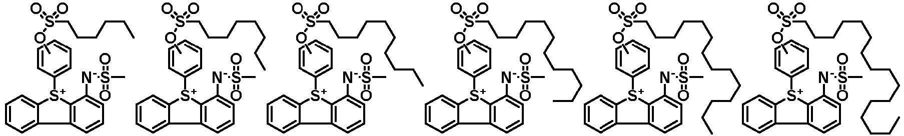

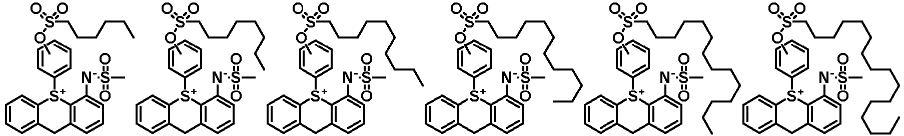

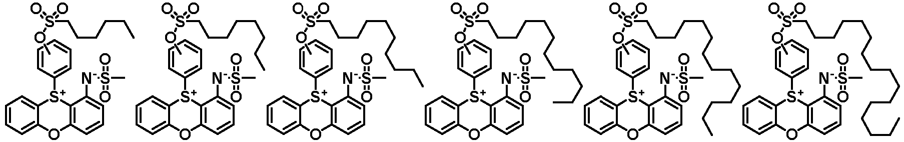

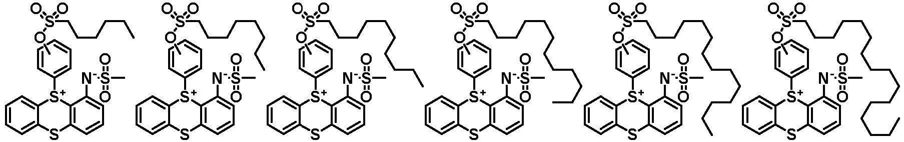

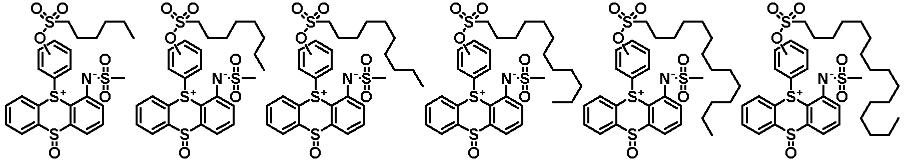

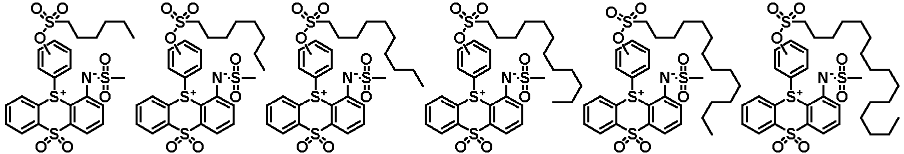

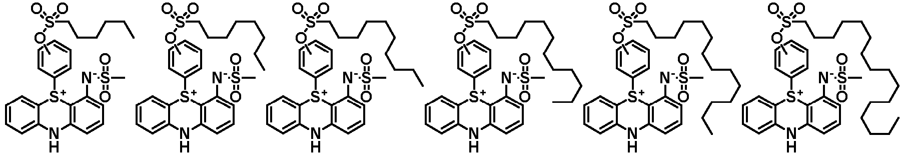

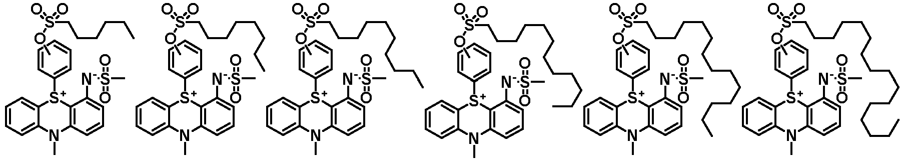

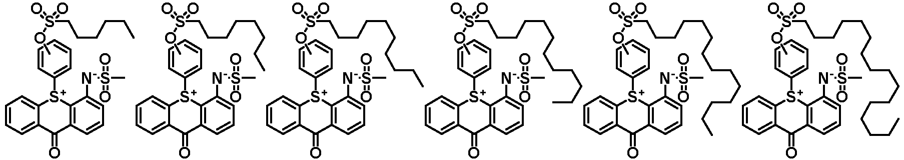

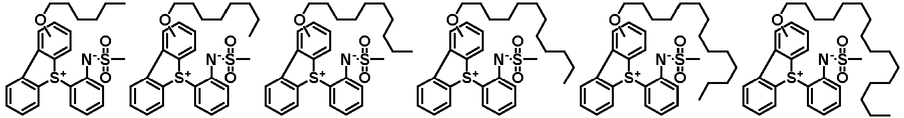

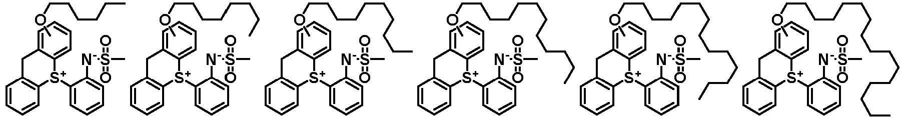

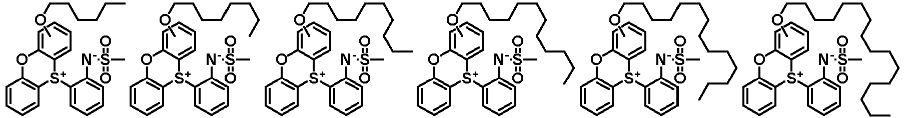

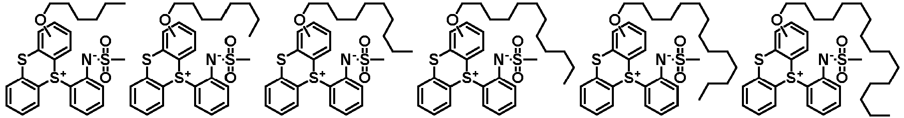

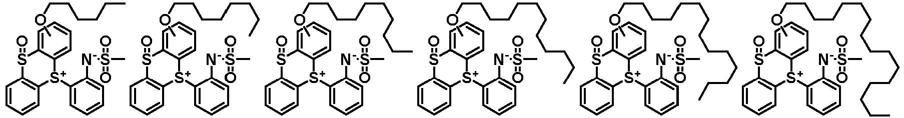

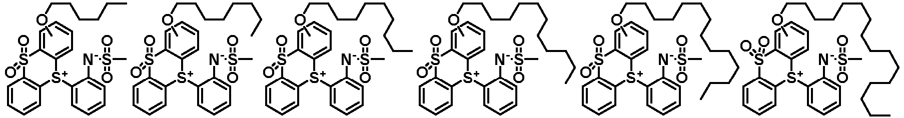

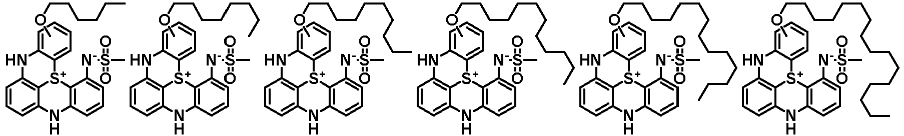

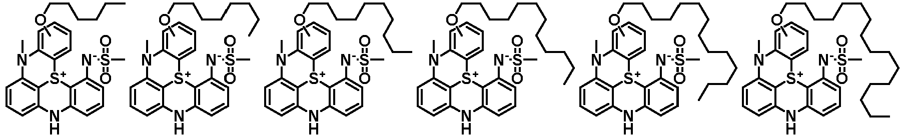

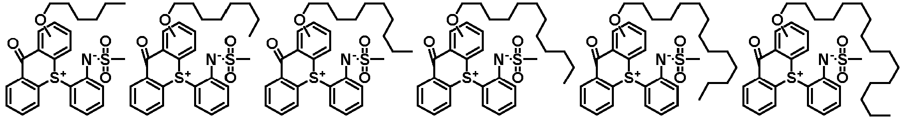

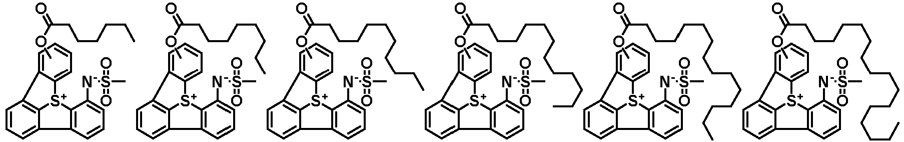

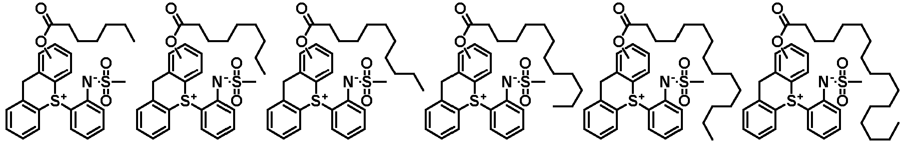

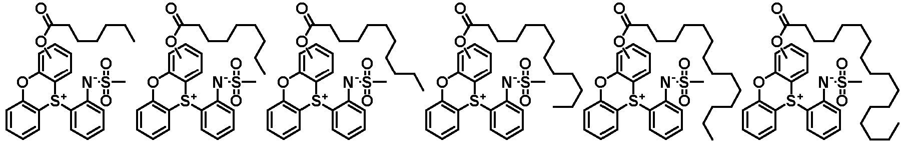

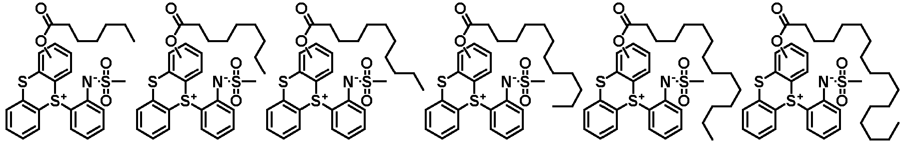

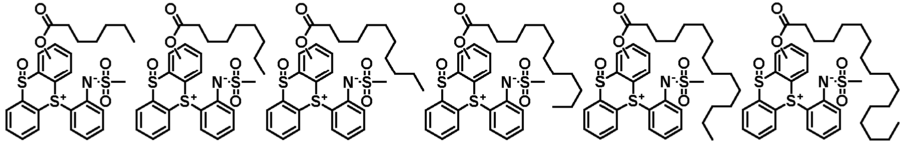

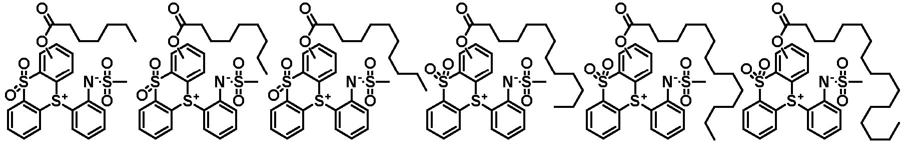

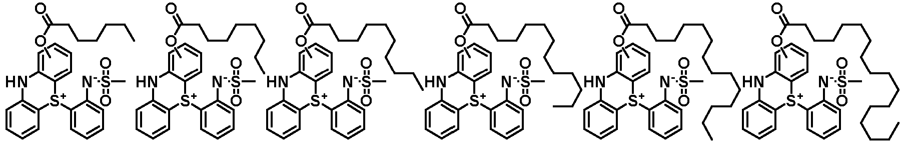

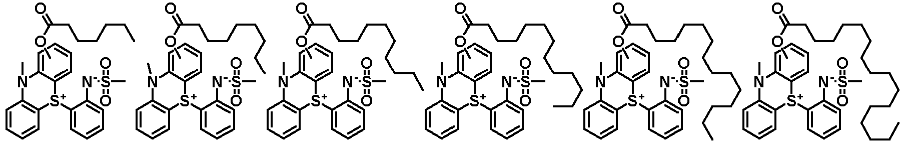

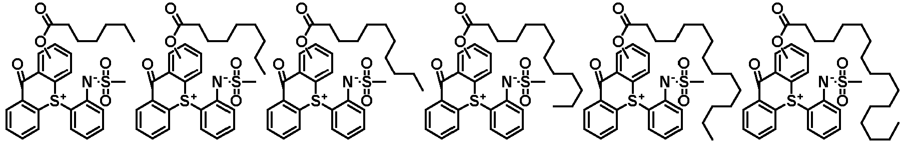

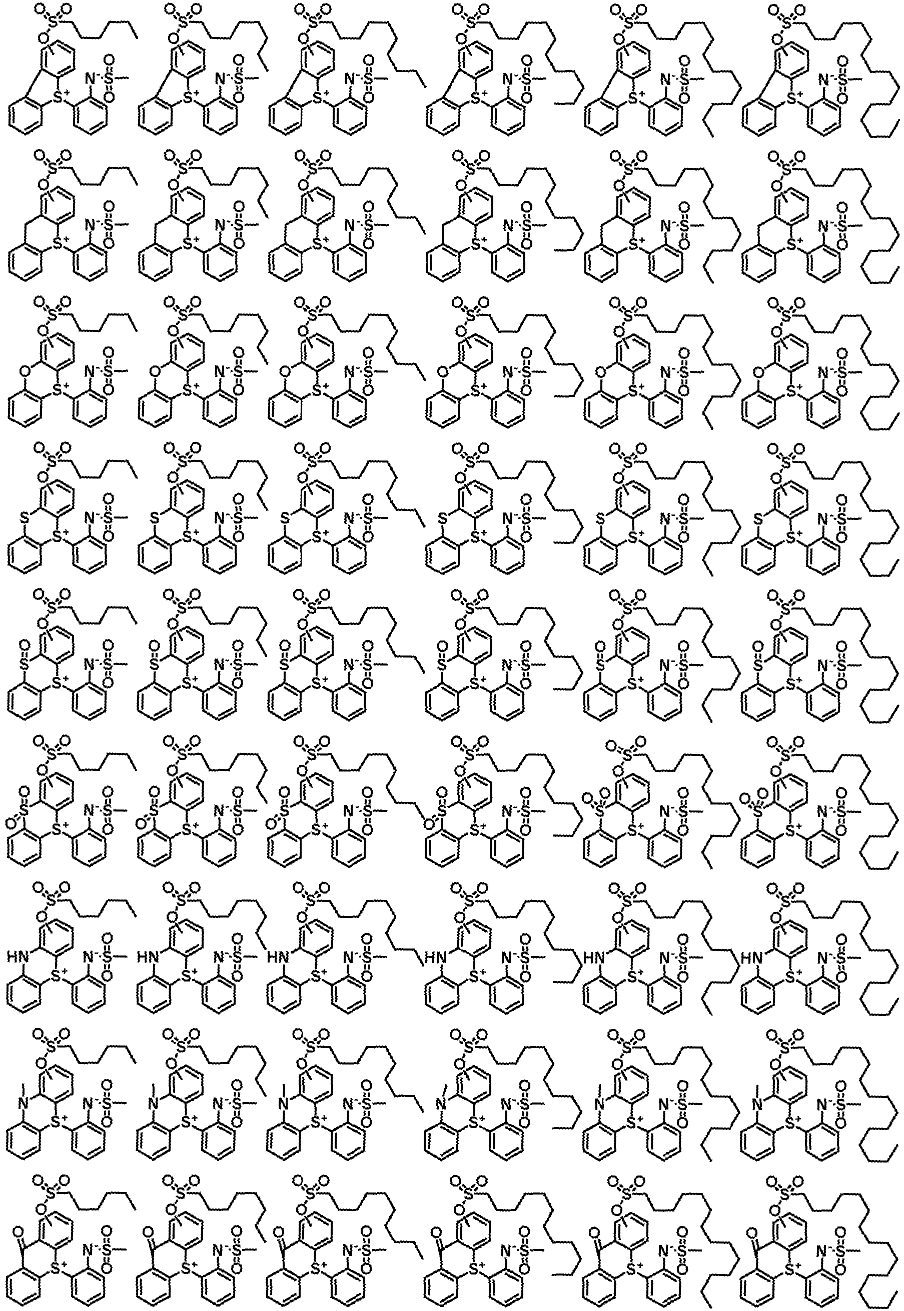

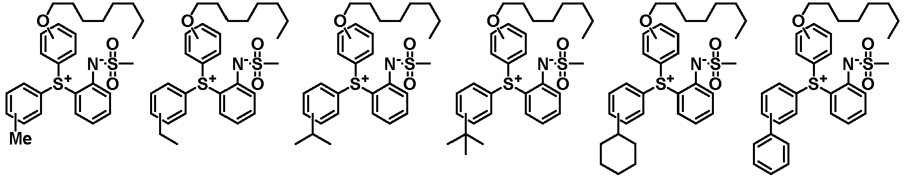

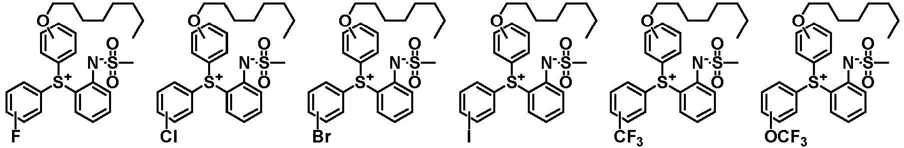

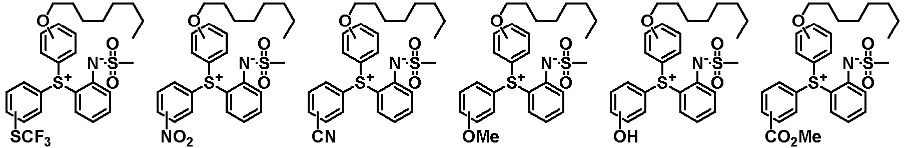

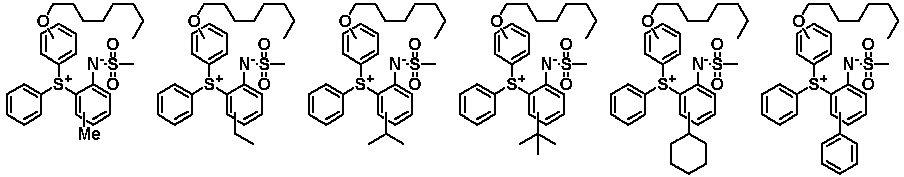

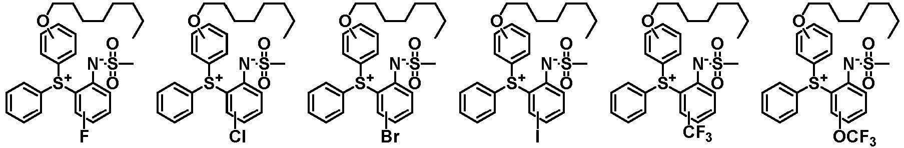

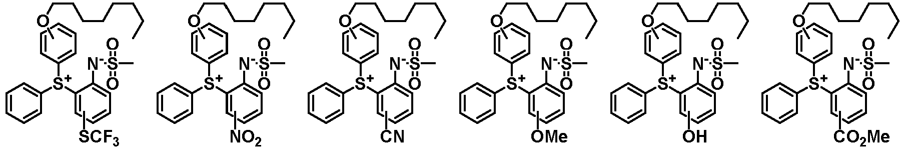

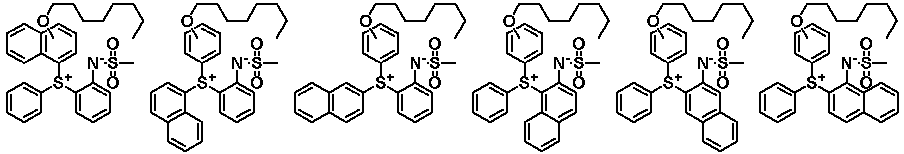

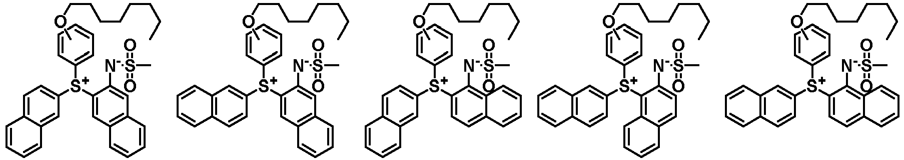

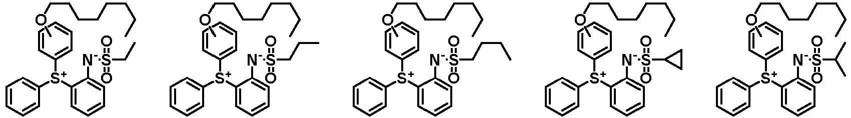

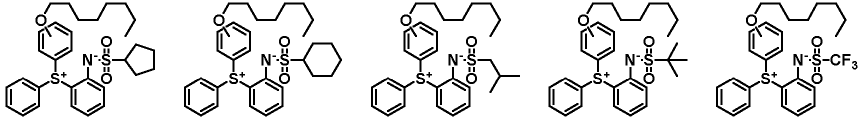

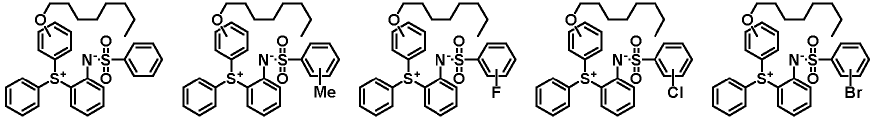

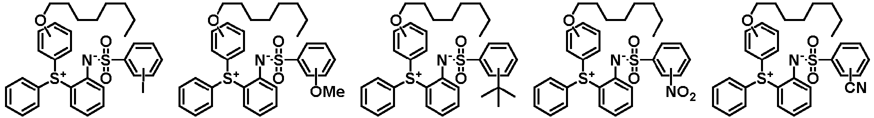

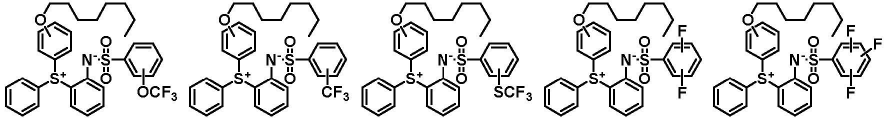

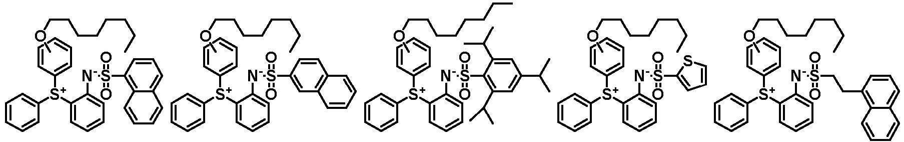

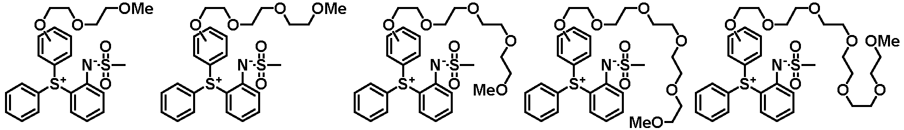

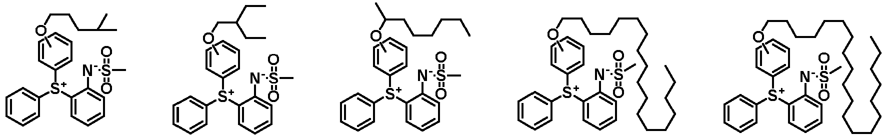

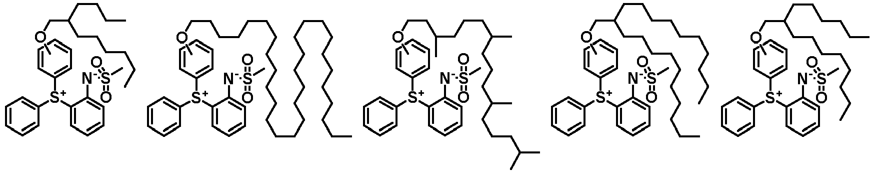

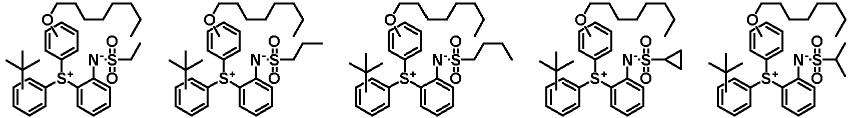

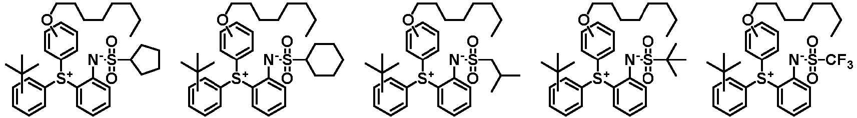

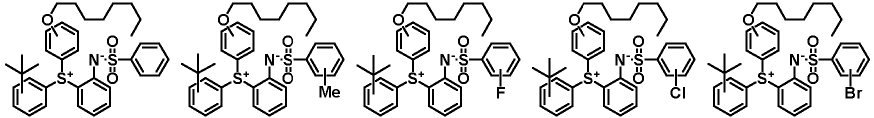

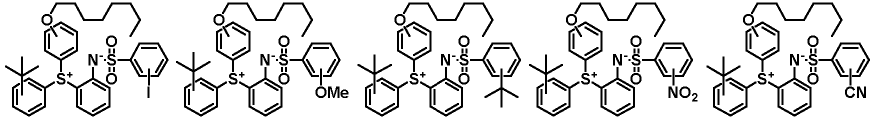

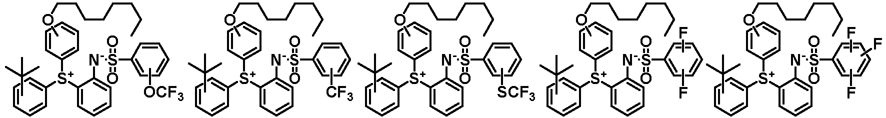

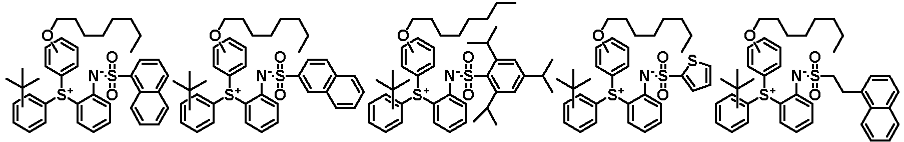

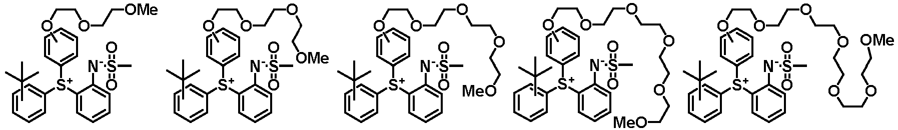

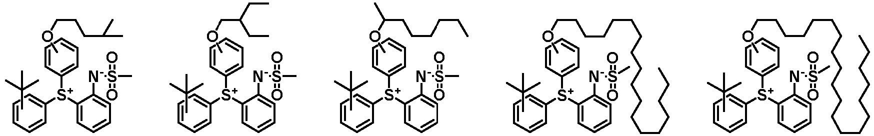

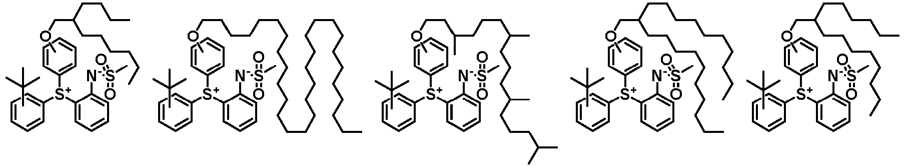

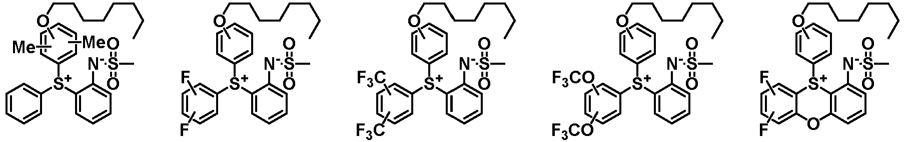

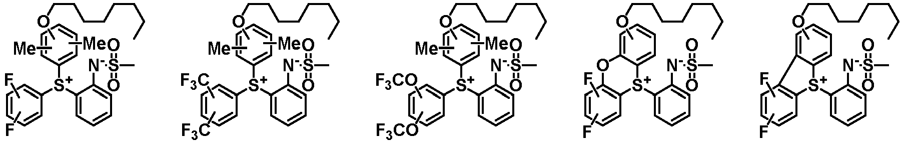

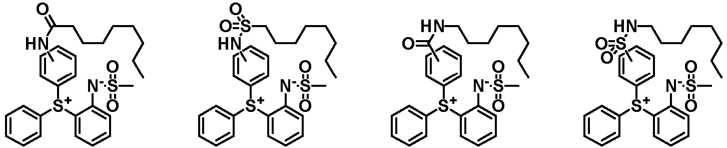

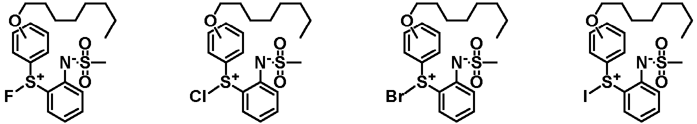

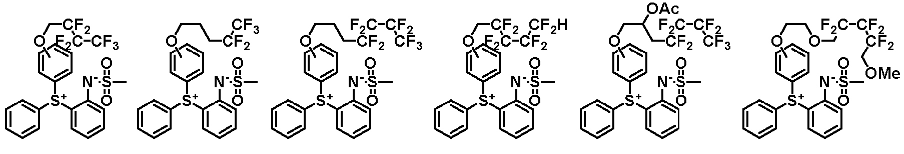

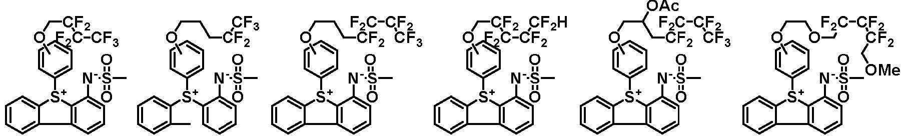

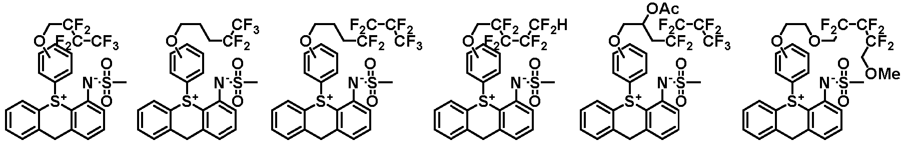

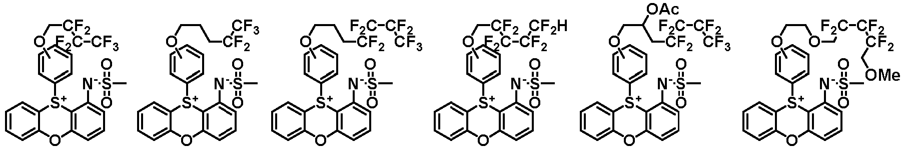

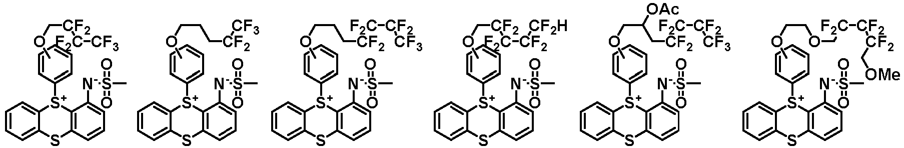

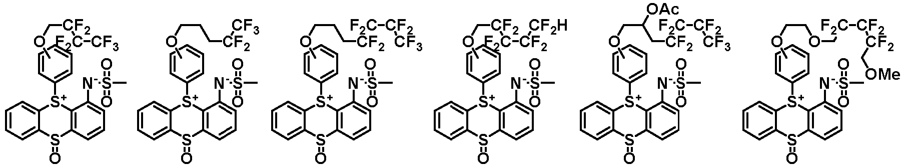

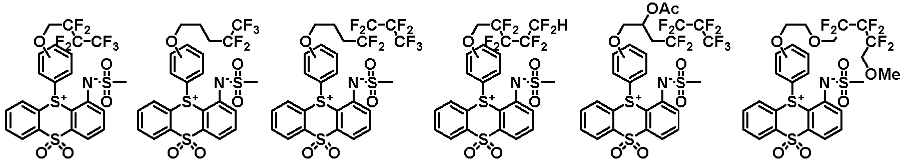

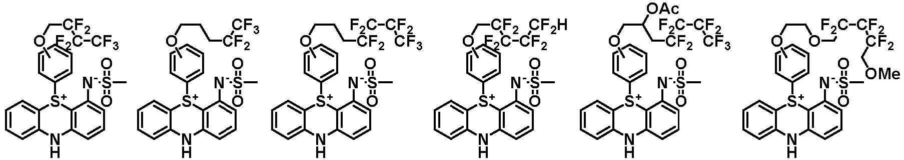

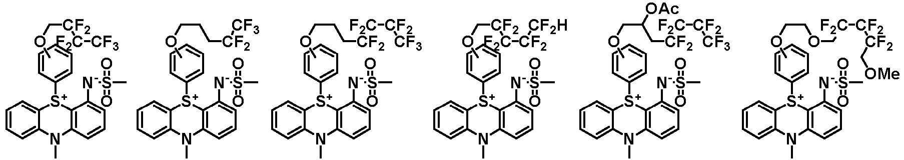

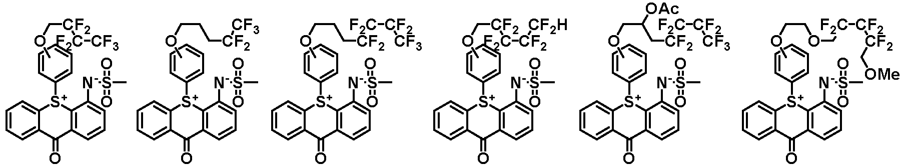

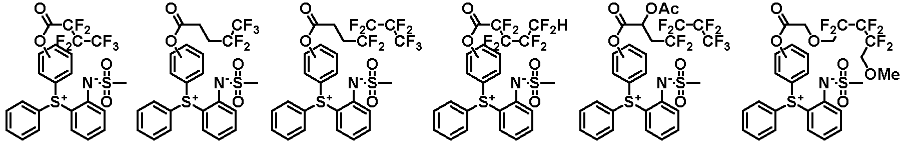

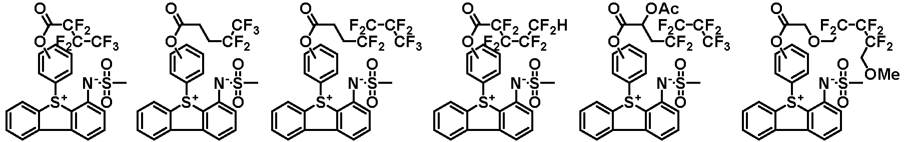

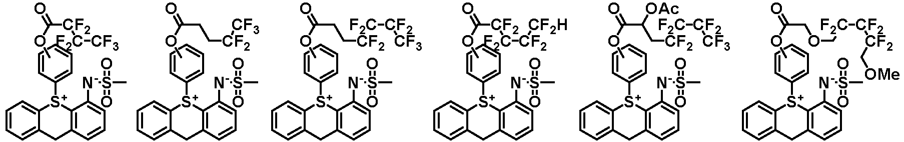

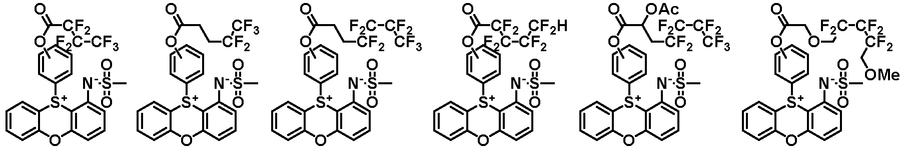

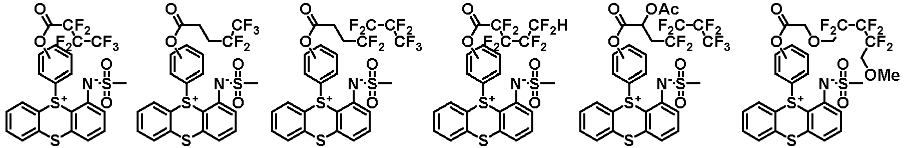

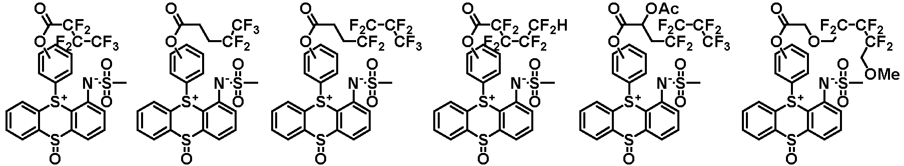

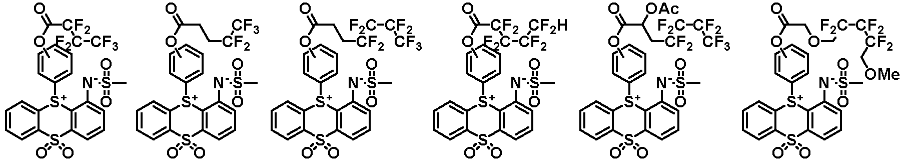

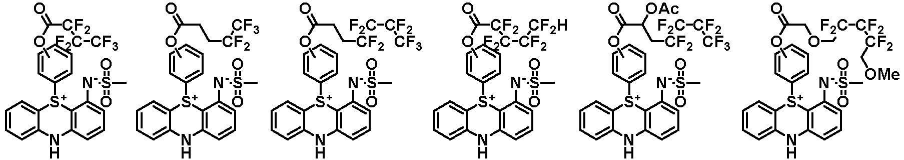

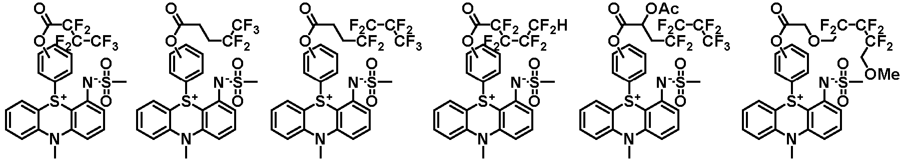

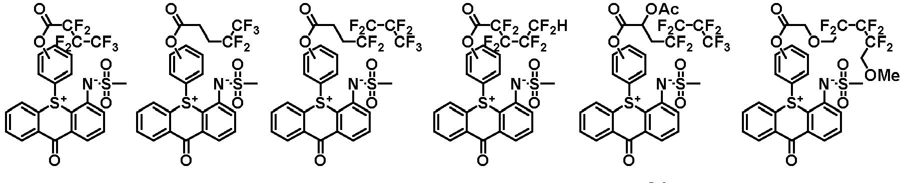

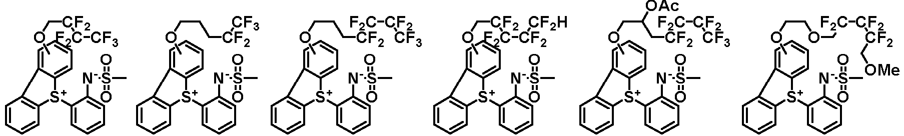

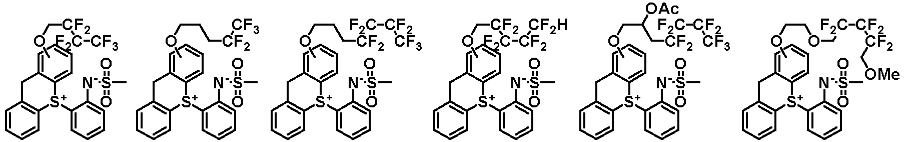

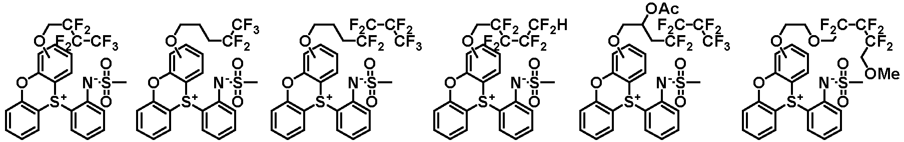

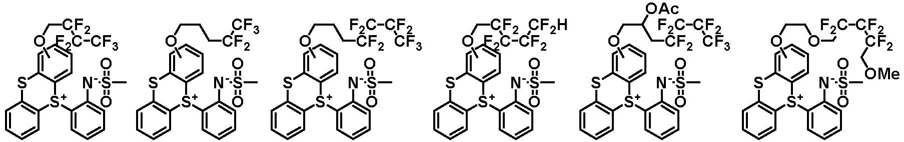

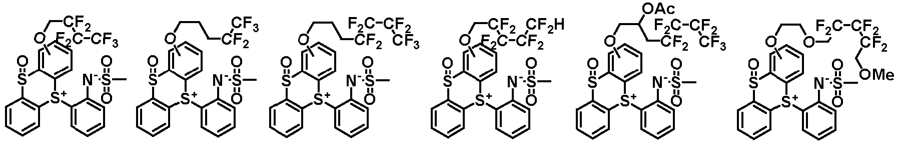

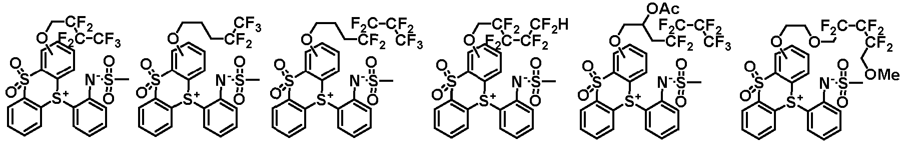

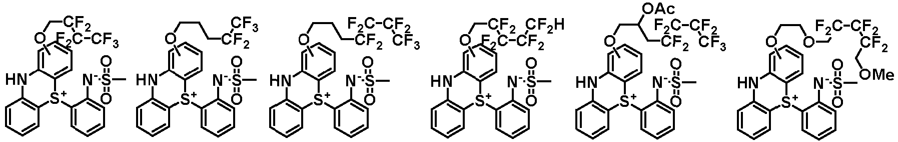

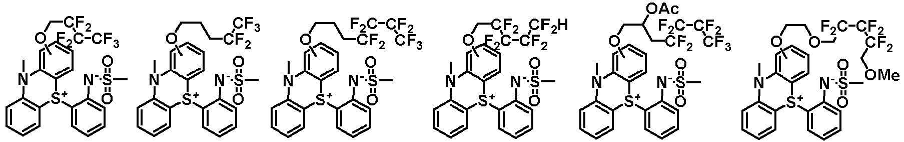

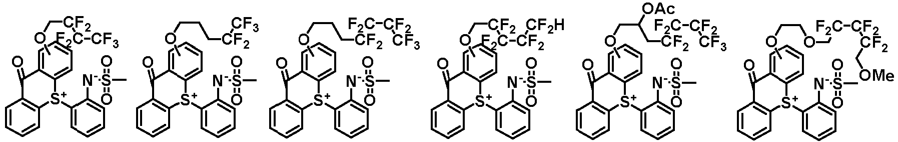

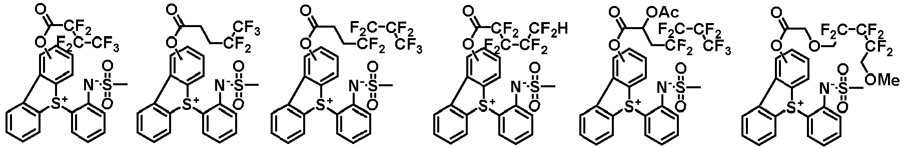

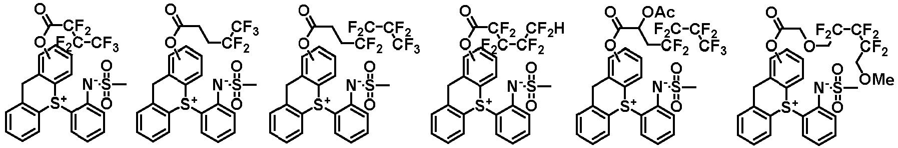

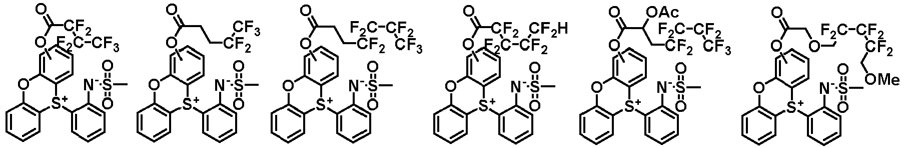

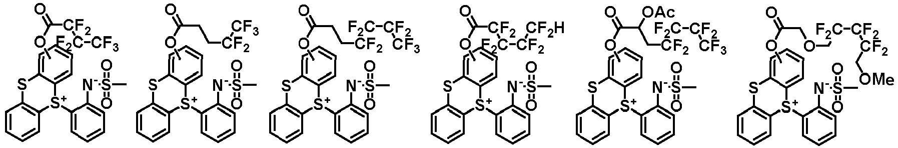

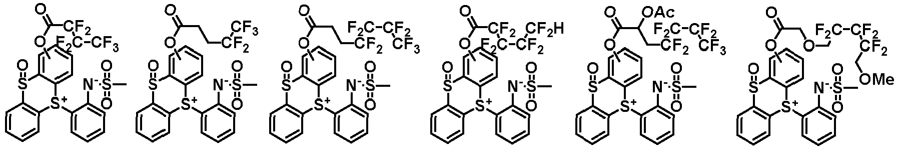

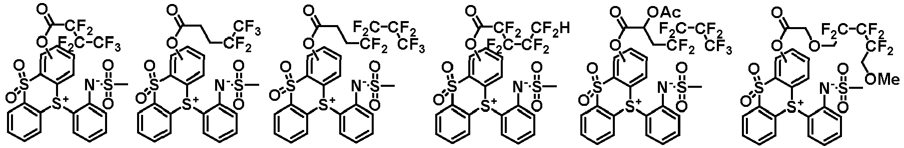

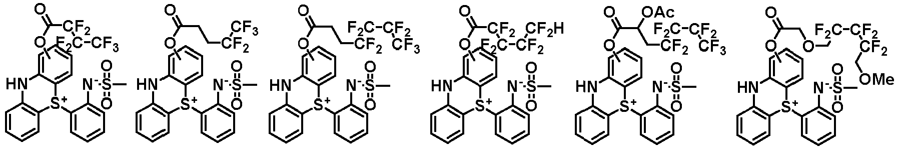

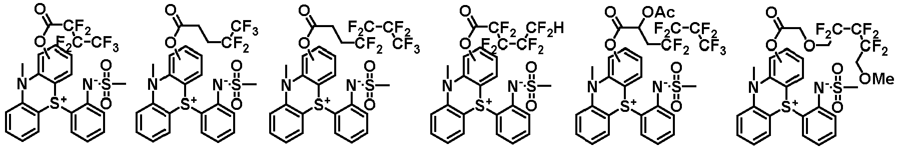

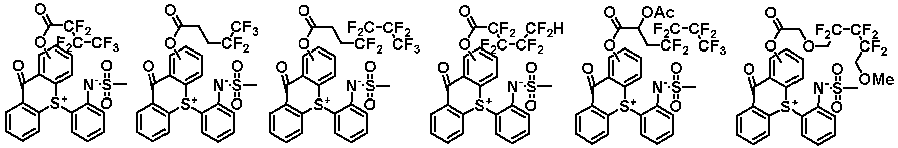

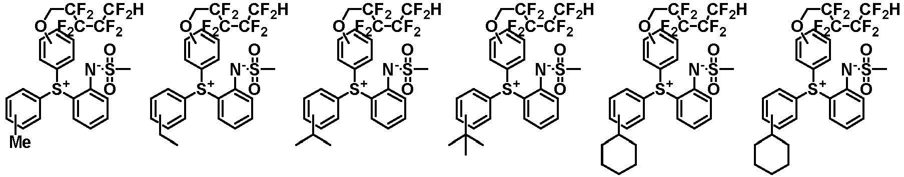

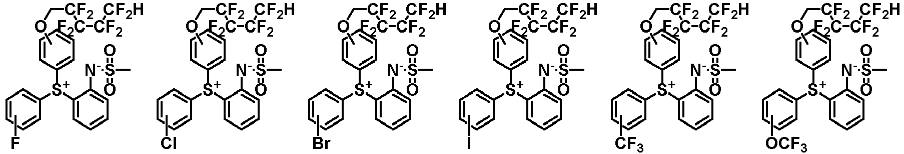

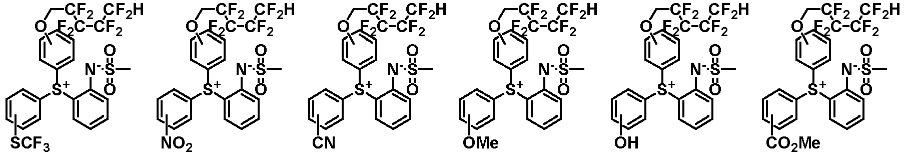

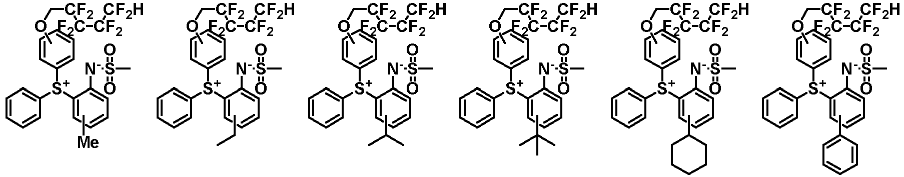

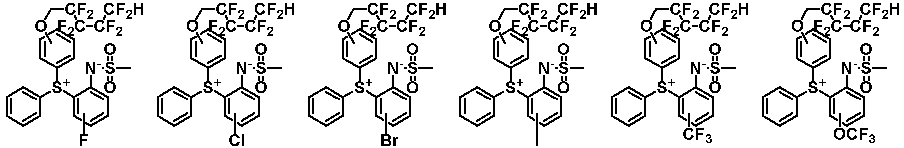

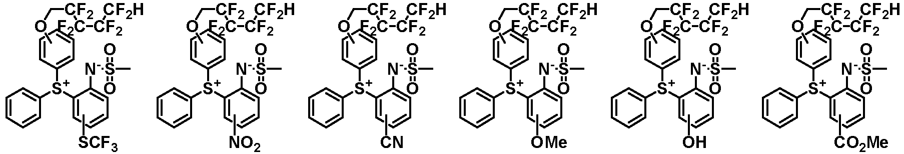

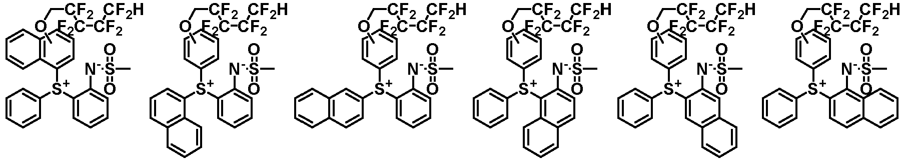

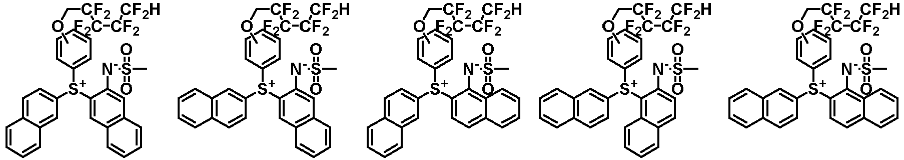

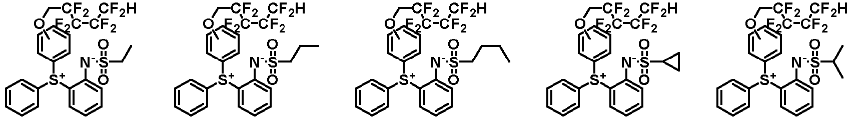

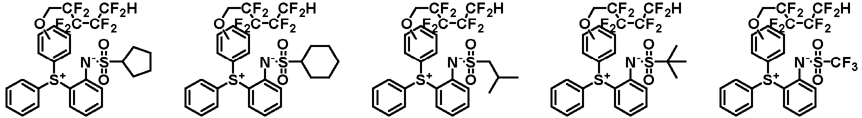

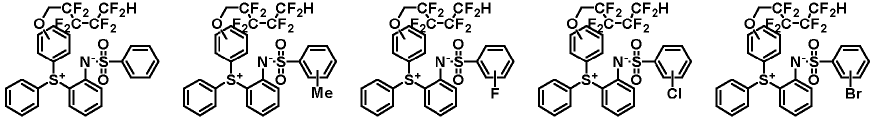

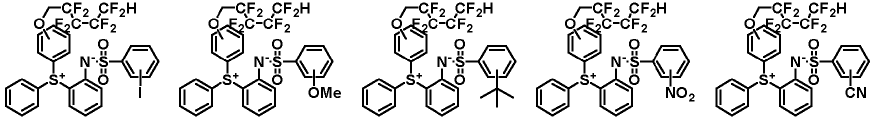

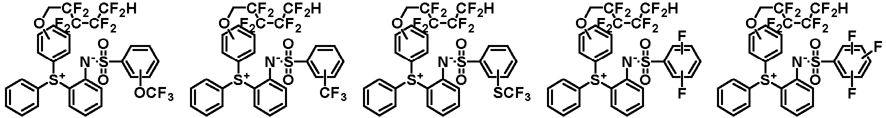

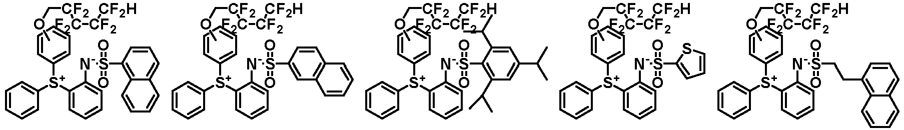

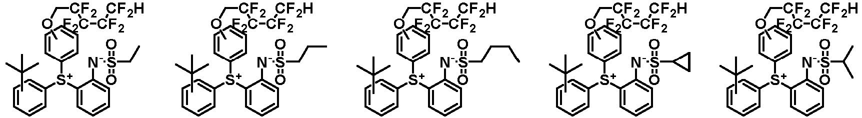

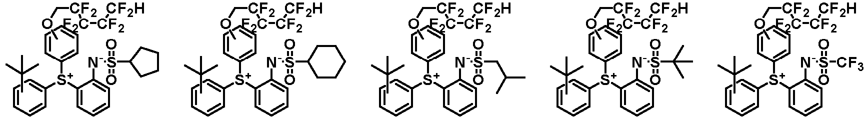

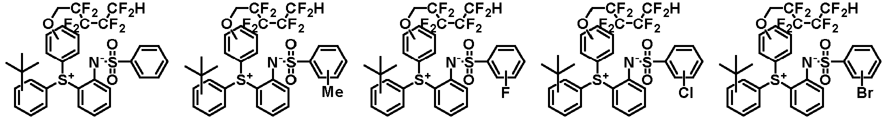

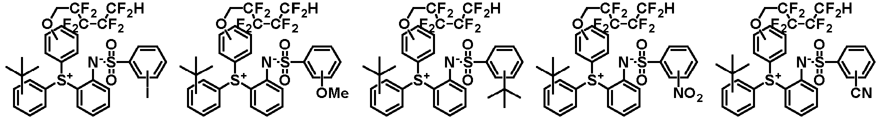

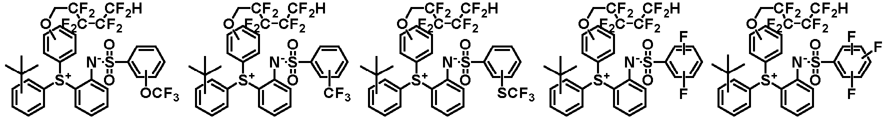

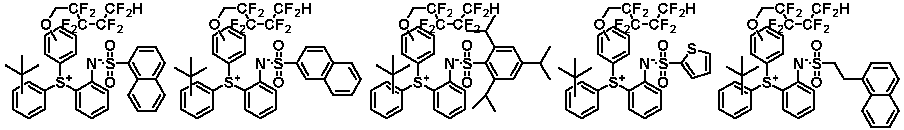

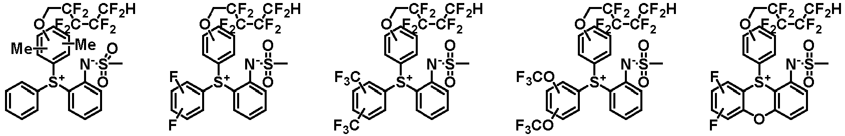

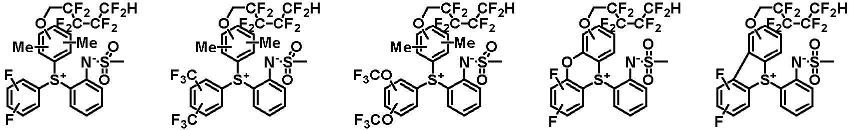

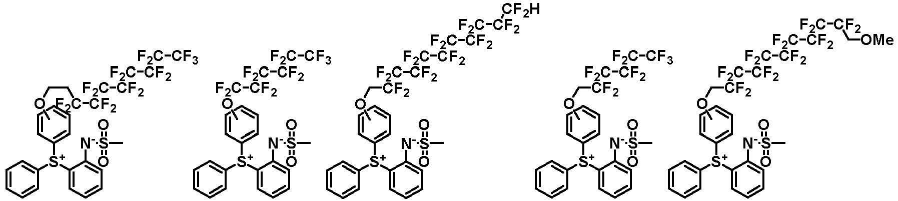

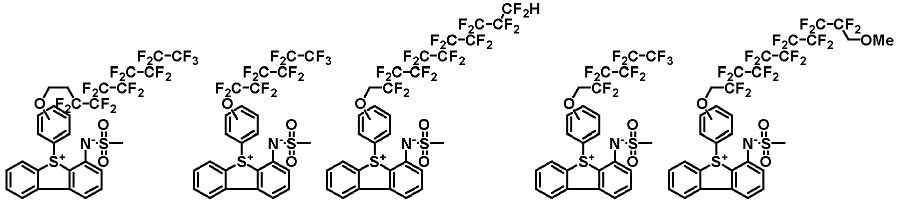

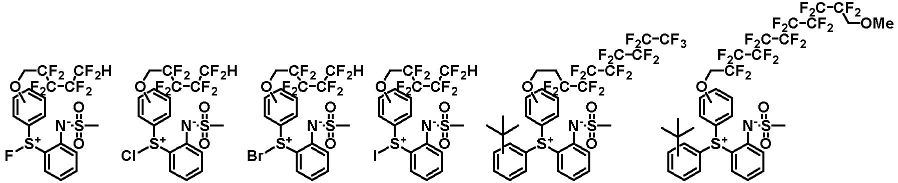

The inventive sulfonium salt can be synthesized by a known method. Examples thereof include methods described in JP 7031637, paragraphs [0026] and [0027], but are not limited thereto.

The sulfonium salt having the formula (A) is characterized by having a basic sulfoneamidate structure and a chain C₆-C₁₈ alkyl group or a chain C₄-C₁₈ fluorinated alkyl group on an aromatic ring that forms a sulfonium cation. The sulfonium salt having formula (A) may be distributed in the upper layer in the resist film because the alkyl group and the fluorinated alkyl group improve solubility in a solvent and have high lipid solubility. In particular, in EB lithography, there is a risk that in writing of an image with an electron beam, influences of forward scattering caused by the electron beam cannot be avoided in an exposed region, and when an ordinary quencher is used, the deprotection reaction in the upper region of the pattern may proceed to an excessive degree, so that the pattern has a top-rounded profile in which pattern features are rounded at their top. By using the inventive quencher, an excessive deprotection reaction in the upper region of the pattern can be effectively suppressed to correct the pattern to a rectangular profile. The sulfoneamidate anion in the same molecule is highly basic, and can control acid diffusion of an acid to perform quenching effectively. By virtue of the synergy of these effects, the use of the inventive sulfonium salt provides a pattern having good dissolution, a high contrast, good CDU, small LER, and a good rectangular profile.

The inventive sulfonium salt is advantageously used as a quencher.

### [Chemically amplified positive resist composition]

### (A) Quencher

Another embodiment of the invention is a chemically amplified positive resist composition essentially comprising (A) a quencher in the form of the sulfonium salt having formula. As used herein, the "quencher" refers to a compound capable of trapping an acid generated from an acid generator. This enables reduction of the diffusion rate at which the acid generated from the acid generator diffuses into the resist film, so that even when a substrate whose outermost surface is made from a material containing chromium is used, the material containing chromium is hardly influenced by the acid generated in the resist film.

In the chemically amplified positive resist composition, the content of the quencher (A) is preferably 0 to 50 parts by weight, more preferably 0.1 to 40 parts by weight per 80 parts by weight of a base polymer (B) described later. When the content of the quencher is in the above range, an acid generated is effectively trapped, and a good pattern profile is obtained. Also, storage stability is high. The quencher (A) may be used alone or in admixture.

### (B) Base polymer

The inventive resist composition comprises (B) a base polymer as a component. The base polymer comprises a polymer adapted to increase its solubility in an aqueous alkaline by degrading under the action of an acid.

The polymer may further comprise repeat units having the formula (B1) (hereinafter also referred to as repeat units B1).

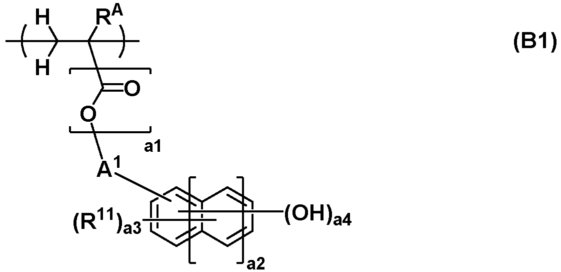

In formula (B1), a1 is 0 or 1. The subscript a2 is 0, 1 or 2. The relevant structure is a benzene skeleton when a2=0, a naphthalene ring when a2=1, and an anthracene skeleton when a2=2. The subscript a3 is an integer which satisfies 0 ≤ a3 ≤ 5+2(a2)-a4. The subscript a4 is 1, 2 or 3. Preferably, a3 is 0, 1, 2 or 3 and a4 is 1, 2 or 3, when a2=0. Preferably, a3 is 0, 1, 2, 3 or 4, and a4 is 1, 2 or 3, when a2 is 1 or 2.

In formula (B1), R^{A} is hydrogen, fluorine, methyl, or trifluoromethyl.

In formula (B 1), R¹¹ is a halogen atom, nitro group, carboxy group, a C₁-C₆ saturated hydrocarbyl group which may be substituted with a halogen atom, a C₁-C₆ saturated hydrocarbyloxy group which may be substituted with a halogen atom, or a C₂-C₈ saturated hydrocarbylcarbonyloxy group which may be substituted with a halogen atom. The saturated hydrocarbyl group and the saturated hydrocarbyl moieties of the saturated hydrocarbyloxy group and saturated hydrocarbylcarbonyloxy group may be straight, branched or cyclic. Examples thereof include alkyl groups such as a methyl group, an ethyl group, a n-propyl group, an isopropyl group, a butyl group, a pentyl group, a hexyl group and structural isomers thereof; cycloalkyl groups such as a cyclopropyl group, a cyclobutyl group, a cyclopentyl group and a cyclohexyl group; and combinations thereof. A carbon count below the upper limit ensures a satisfactory solubility in alkaline developer. Groups R¹¹ may be the same or different when a3 is 2 or more.

In formula (B1), A¹ is a single bond or C₁-C₁₀ saturated hydrocarbylene group in which some constituent -CH₂- may be replaced by -O-. The saturated hydrocarbylene group may be straight, branched or cyclic. Examples thereof include C₁-C₁₀ alkanediyl groups such as a methyl group, an ethane-1,2-diyl group, a propane-1,3-diyl group, a butane-1,4-diyl group, a pentane-1,5-diyl group, a hexane-1,6-diyl group and structural isomers thereof; C₃-C₁₀ cyclic saturated hydrocarbylene groups such as a cyclopropanediyl group, a cyclobutanediyl group, a cyclopentanediyl group and a cyclohexanediyl group; and combinations thereof. For the saturated hydrocarbylene group containing an ether bond, when a1=1 in formula (B1), the ether bond may be incorporated at any position excluding the position between the α-carbon and β-carbon relative to the ester oxygen atom. When a1=0, the atom that bonds with the backbone becomes an ethereal oxygen atom, and a second ether bond may be incorporated at any position excluding the position between the α-carbon and β-carbon relative to the ethereal oxygen atom. Saturated hydrocarbylene groups having no more than 10 carbon atoms are desirable because of a sufficient solubility in alkaline developer.

When a1 is 0 and A¹ is a single bond, in other words, the aromatic ring is directly bonded to the backbone of the polymer (i.e., there is no linker (-C(=O)-O-A¹-)), preferred examples of the repeat unit B1 include units 3-hydroxystyrene, 4-hydroxystyrene, 5-hydroxy-2-vinylnaphthalene, and 6-hydroxy-2-vinylnaphthalene. Examples thereof are shown below, but not limited thereto. R^{A} is as defined above.

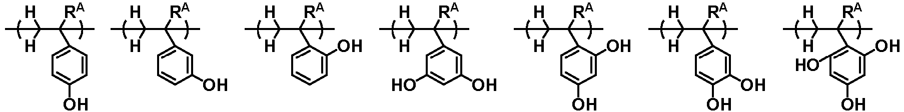

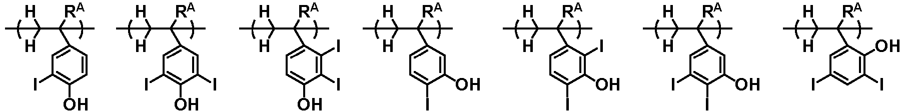

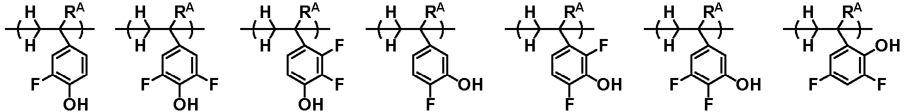

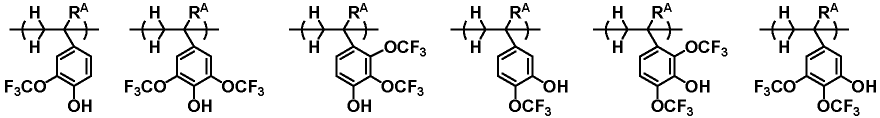

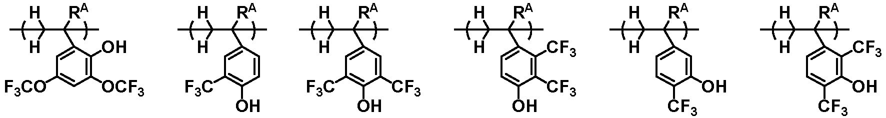

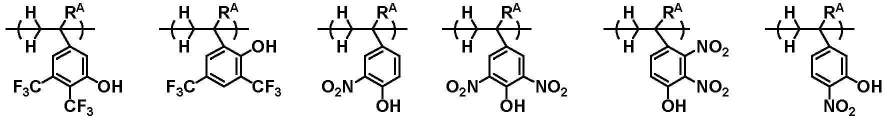

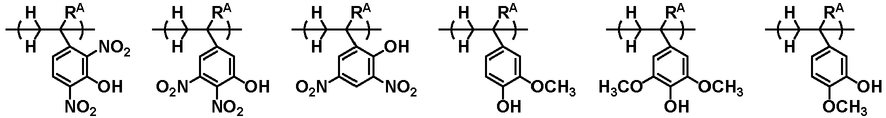

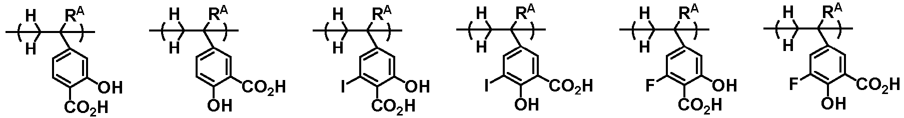

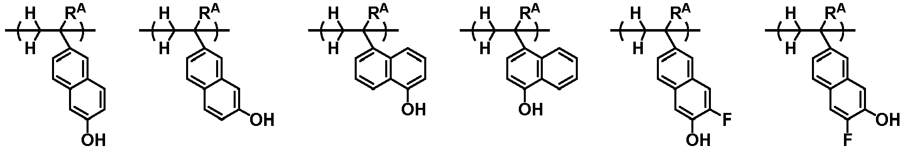

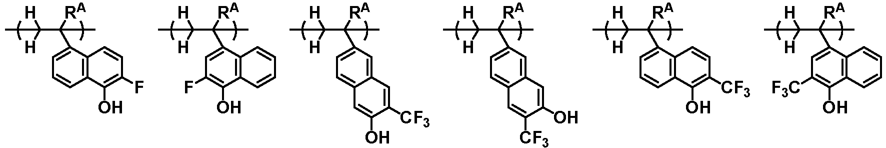

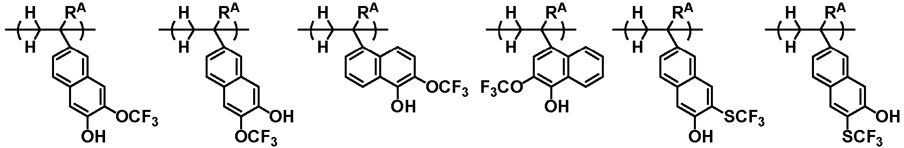

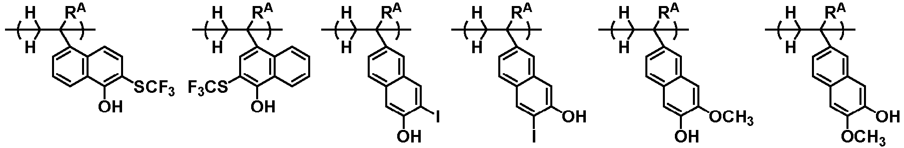

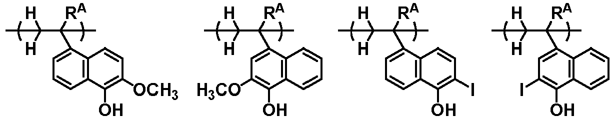

When a1 is 1 (i.e., there is a linker (-C(=O)-O-A¹-)), preferred examples of the repeat unit B1 are shown below, but not limited thereto. R^{A} is as defined above.

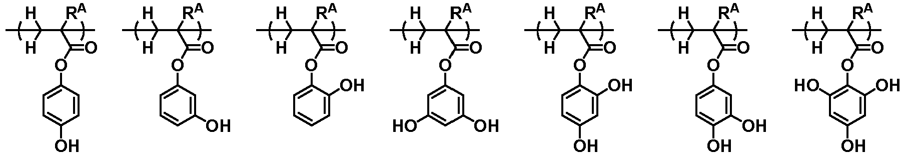

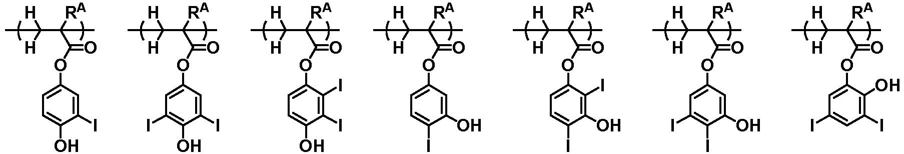

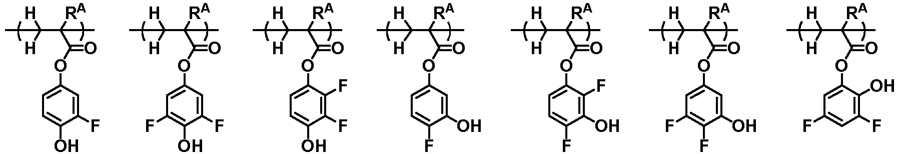

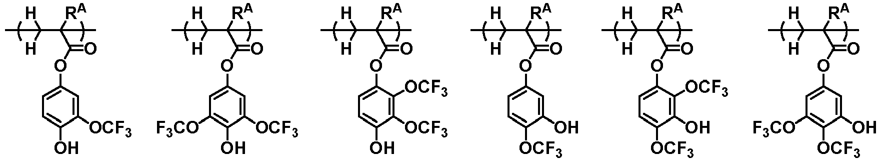

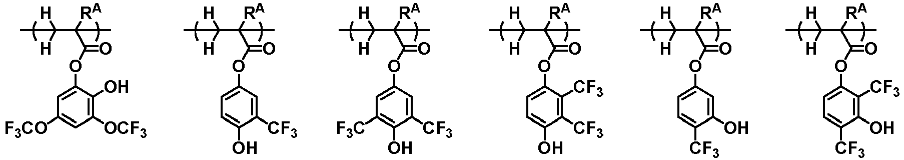

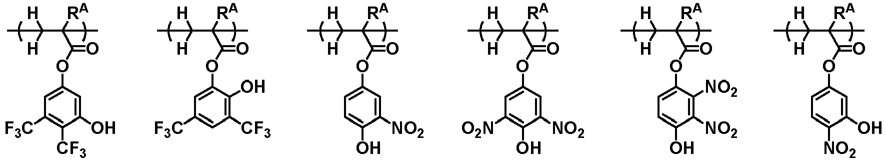

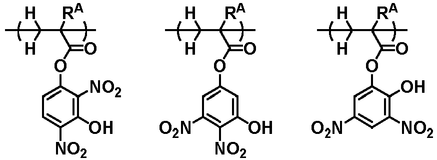

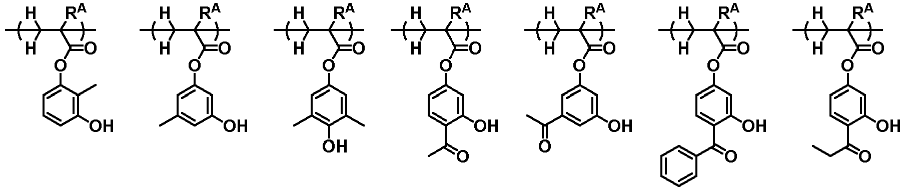

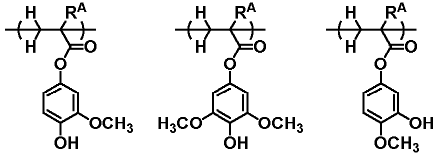

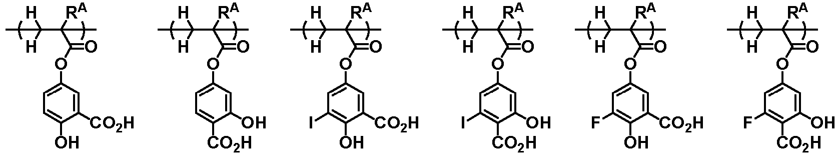

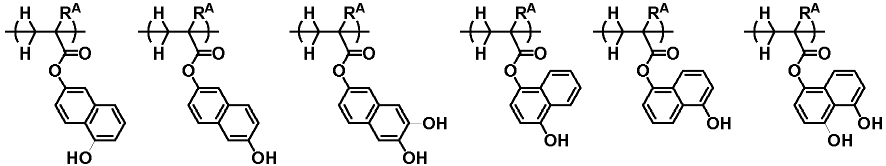

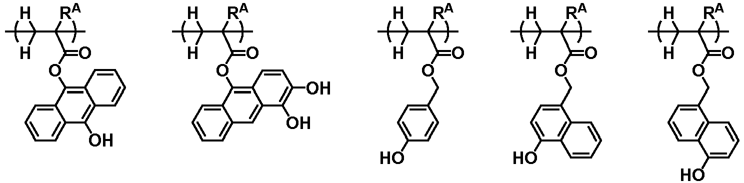

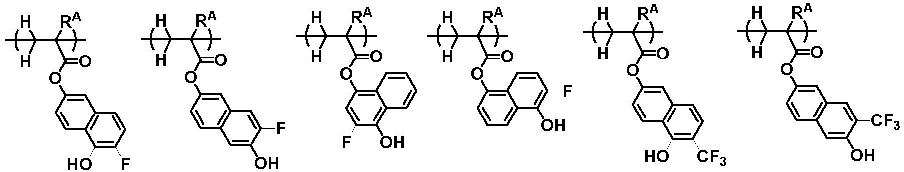

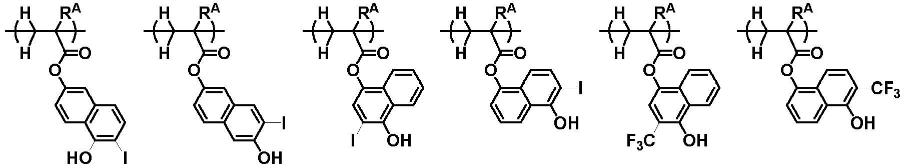

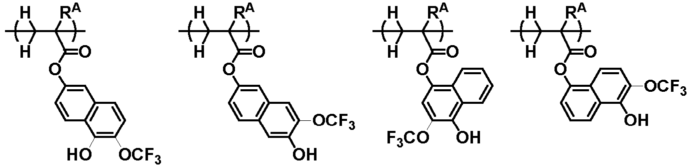

The content of repeat units B1 is preferably 15 to 90 mol%, more preferably 15 to 80 mol% of the overall repeat units of the polymer. It is noted that when the polymer further contains repeat units of at least one type selected from repeat units having formulae (B3) and (B4) and imparting higher etch resistance to the polymer, preferably the repeat units of at least one type containing a phenolic hydroxy group as a substituent, the content of repeat units B1 plus repeat units B3 and/or B4 falls in the above range. Each of the repeat units B1 may be of one type or a combination of plural types.

For the polymer to have a property as a positive resist composition in which exposed regions are soluble in an alkaline developer, the polymer preferably contains repeat units having a functional group protected by an acid labile group, i.e., repeat units protected by an acid labile group and becoming alkali-soluble under the action of an acid, which are also referred to as repeat units B2, hereinafter.

Examples of the most preferred repeat unit B2 include those having the formula (B2-1), which are also referred to repeat units B2-1, hereinafter.

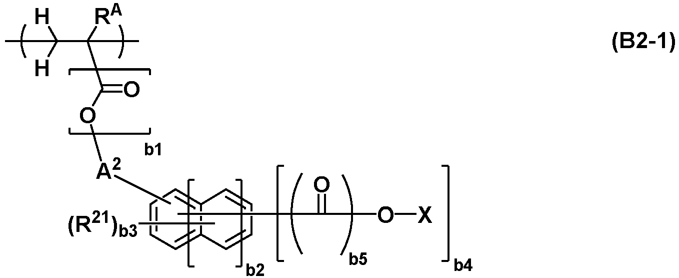

In formula (B2-1), b1 is 0 or 1. The subscript b2 is 0, 1 or 2. The relevant structure is benzene when a2=0, a naphthalene ring when a2=1, and anthracene when a2=2. The subscript b3 is an integer which satisfies 0 ≤ b3 ≤ 5+2(b2)-b4. The subscript b4 is 1, 2 or 3. The subscript b5 is 0 or 1. Preferably, b3 is 0, 1, 2 or 3 and b4 is 1, 2 or 3, when b2=0. Preferably, b3 is 0, 1, 2, 3 or 4, and b4 is 1, 2 or 3, when b2 is 1 or 2.

In formula (B2-1), R^{A} is hydrogen, fluorine, methyl, or trifluoromethyl.

In formula (B2-1), R²¹ is a halogen atom, C₁-C₆ saturated hydrocarbyl group which may be substituted with a halogen atom, C₁-C₆ saturated hydrocarbyloxy group which may be substituted with a halogen atom, or C₂-C₈ saturated hydrocarbylcarbonyloxy group which may be substituted with a halogen atom. The saturated hydrocarbylcarbonyloxy group and the saturated hydrocarbyl moieties of the saturated hydrocarbyloxy group and saturated hydrocarbyloxy group may be straight, branched or cyclic. Examples thereof include alkyl groups such as a methyl group, an ethyl group, a n-propyl group, an isopropyl group, a butyl group, a pentyl group, a hexyl group and structural isomers thereof; cycloalkyl groups such as a cyclopropyl group, a cyclobutyl group, a cyclopentyl group and a cyclohexyl group; and combinations thereof. A carbon count below the upper limit ensures a satisfactory solubility in alkaline developer. A plurality of R²¹ may be identical or different when b3 is 2 or more.

In formula (B2-1), A² is a single bond or C₁-C₁₀ saturated hydrocarbylene group in which some constituent -CH₂- may be replaced by -O-. The saturated hydrocarbylene group may be straight, branched or cyclic. Examples thereof include C₁-C₁₀ alkanediyl groups such as a methyl group, an ethane-1,2-diyl group, a propane-1,3-diyl group, a butane-1,4-diyl group, a pentane-1,5-diyl group, a hexane-1,6-diyl group and structural isomers thereof; C₃-C₁₀ cyclic saturated hydrocarbylene groups such as a cyclopropanediyl group, a cyclobutanediyl group, a cyclopentanediyl group and a cyclohexanediyl group; and combinations thereof. For the saturated hydrocarbylene group containing an ether bond, when b1=1 in formula (B2-1), the ether bond may be incorporated at any position excluding the position between the α-carbon and β-carbon relative to the ester oxygen atom. When b1=0, the atom that bonds with the backbone becomes an ethereal oxygen atom, and a second ether bond may be incorporated at any position excluding the position between the α-carbon and β-carbon relative to the ethereal oxygen atom. Saturated hydrocarbylene groups having no more than 10 carbon atoms are desirable because of a sufficient solubility in alkaline developer.

In formula (B2-1), X is an acid labile group when b4 is 1. X is each independently hydrogen or an acid labile group, at least one being an acid labile group, in case of b4 is 2 or 3. That is, repeat units B2-1 have phenolic hydroxy groups bonded to an aromatic ring, at least one of which is protected with an acid labile group, or repeat units B2-1 have a carboxy group bonded to an aromatic ring, which is protected with an acid labile group. The acid labile group used herein is not particularly limited as long as it is commonly used in a number of well-known chemically amplified positive resist compositions and eliminated under the action of acid to release an acidic group.

Examples of the acid labile group include a tertiary saturated hydrocarbyl group. The tertiary saturated hydrocarbyl groups of 4 to 18 carbon atoms are preferred because the corresponding monomer for use in polymerization is available through distillation.

The saturated hydrocarbyl group attached to the tertiary carbon atom in the tertiary saturated hydrocarbyl group is preferably a C₁-C₁₅ saturated hydrocarbyl group. The C₁-C₁₅ saturated hydrocarbyl group may be straight, branched or cyclic, and an oxygen atom-containing functional group such as an ether group or carbonyl group may be incorporated in a carbon-carbon bond. The saturated hydrocarbyl groups attached to the tertiary carbon atom may bond together to form a ring with the tertiary carbon atom to which they are attached.

Examples of the alkyl substituent include methyl, ethyl, propyl, adamantyl, norbornyl, tetrahydrofuran-2-yl, 7-oxanorbornan-2-yl, cyclopentyl, 2-tetrahydrofuryl, tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, tetracyclo[4.4.0.1^{2,5}.1^{7,10}]dodecyl and 3-oxo-1-cyclohexyl groups.

Examples of the tertiary saturated hydrocarbyl group include, but are not limited to, tert-butyl, tert-pentyl, 1-ethyl-1-methylpropyl, 1,1-diethylpropyl, 1,1,2-trimethylpropyl, 1-adamantyl-1-methylethyl, 1-methyl-1-(2-norbornyl)ethyl, 1-methyl-1-(tetrahydrofuran-2-yl)ethyl, 1-methyl-1-(7-oxanorbornan-2-yl)ethyl, 1-methylcyclopentyl, 1-ethylcyclopentyl, 1-propylcyclopentyl, 1-cyclopentylcyclopentyl, 1-cyclohexylcyclopentyl, 1-(2-tetrahydrofuryl)cyclopentyl, 1-(7-oxanorbornan-2-yl)cyclopentyl, 1-methylcyclohexyl, 1-ethylcyclohexyl, 1-cyclopentylcyclohexyl, 1-cyclohexylcyclohexyl, 2-methyl-2-norbornyl, 2-ethyl-2-norbornyl, 8-methyl-8-tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, 3-ethyl-3-tetracyclo[4.4.0.1^{2,5}.1^{7,10}]dodecyl, 2-methyl-2-adamantyl, 2-ethyl-2-adamantyl, 1-methyl-3-oxo-1-cyclohexyl, 1-methyl-1-(tetrahydrofuran-2-yl)ethyl, 5-hydroxy-2-methyl-2-adamantyl and 5-hydroxy-2-ethyl-2-adamantyl groups.

A group having the formula (B2-1-1) is also suitable as the acid labile group. The group having formula (B2-1-1) is often used as the acid labile group. It is a good choice of the acid labile group that ensures to form a pattern having a relatively rectangular pattern-substrate interface in a consistent manner. An acetal structure is formed when X is a group having formula (B2-1-1).

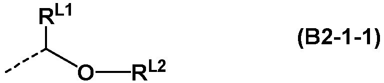

Herein the broken line designates a point of attachment.

In formula (B2-1-1), R^{L1} is hydrogen or a C₁-C₁₀ saturated hydrocarbyl group. The saturated hydrocarbyl group may be straight, branched or cyclic.

A choice of R^{L1} may depend on the designed sensitivity of decomposable group to acid. For example, hydrogen or a group having a tertiary carbon atom as a carbon atom bonded to acetal carbon is preferred when the acid decomposable group is designed to ensure relatively high stability and to be decomposed with strong acid. Examples of R^{L1} bonded to acetal carbon via the tertiary carbon atom include, but are not limited to, tert-butyl, tert-pentyl and 1-adamantyl groups. A straight alkyl group is preferred when the acid decomposable group is designed to have relatively high reactivity and high sensitivity to pH changes. Although the choice varies with a particular combination of acid generator and quencher in the resist composition, R^{L1} is preferably a group in which the carbon in bond with acetal carbon is a secondary carbon atom, when R^{L2} is a relatively large alkyl group substituted at the end and the acid decomposable group is designed to undergo a substantial change of solubility by decomposition. Examples of R^{L1} bonded to acetal carbon via the secondary carbon atom include, but are not limited to, sec-butyl, cyclopentyl and cyclohexyl groups.

In formula (B2-1-1), R^{L2} is a C₁-C₃₀ hydrocarbyl group. The hydrocarbyl group may be saturated or unsaturated, and may be straight, branched or cyclic. The hydrocarbyl group may substituted at some constituent -CH₂- with heteroatoms such as an oxygen atom or a sulfur atom, and resultantly contain an ether bond, a sulfide bond or the like. Examples of the hydrocarbyl group include C₁-C₃₀ saturated hydrocarbyl and C₆-C₃₀ aryl groups. Particularly in fine pattern formation, R^{L2} is preferably a C₁-C₆ hydrocarbyl group for acquiring a higher resolution. When R^{L2} is a C₁-C₆ hydrocarbyl group, an alcohol, which is formed after a deprotection reaction by an acid proceeds, is soluble in water. Therefore, in formation of positive tone pattern with an alkaline developer, the alcohol is dissolved in the developer, so that residue defects on exposed regions can be reduced.

Preferred examples of the group having formula (B2-1-1) are shown below, but not limited thereto. In the formulae, R^{L1} is as defined above, and the broken line designates a point of attachment.

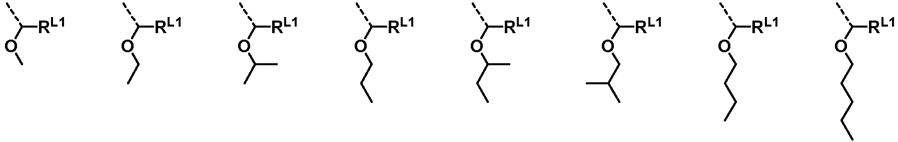

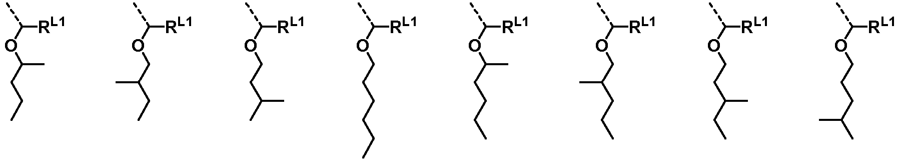

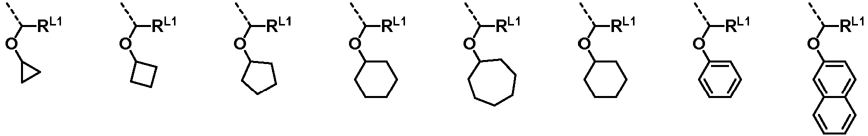

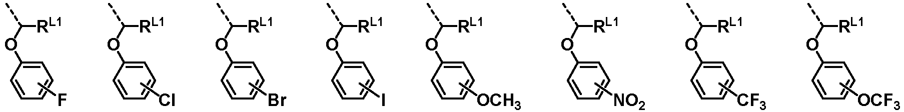

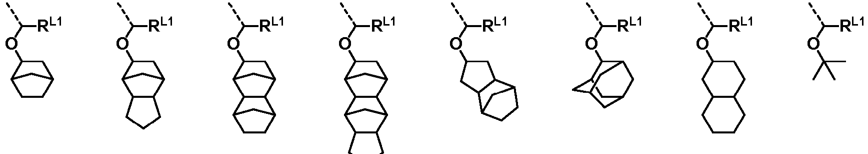

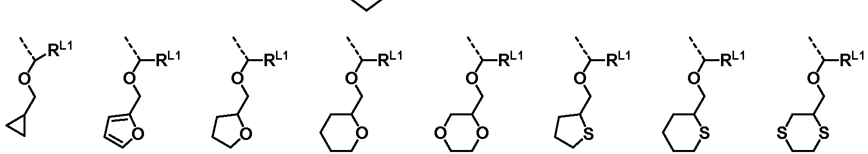

Another choice of acid labile group which can be used herein is a phenolic hydroxy group whose hydrogen is substituted by a tertiary saturated hydrocarbyl moiety: -CH₂COO-. Examples of the tertiary saturated hydrocarbyl moiety are as exemplified above for the tertiary saturated hydrocarbyl group used for the protection of phenolic hydroxy group.

Examples of the repeat unit B2 include repeat units having the formula (B2-2), which are also referred to repeat units B2-2, hereinafter. The repeat unit B2-2 is a good choice as an acid labile group-containing unit which enhances the dissolution rate of exposed regions to improve performance against the variation of line width in develop loading.

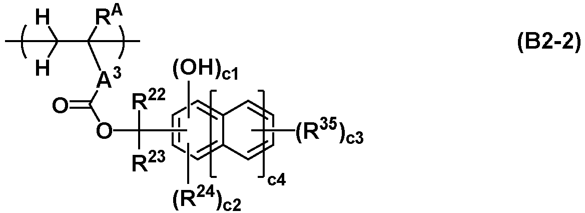

In formula (B2-2), c1 is 0, 1 or 2. The subscript c2 is 0, 1 or 2. The c3 is 0, 1, 2, 3, 4 or 5. c4 is 0, 1 or 2.

In formula (B2-2), R^{A} is hydrogen, fluorine, methyl, or trifluoromethyl.

In formula (B2-2), R²² and R²³ are each independently a C₁-C₁₀ hydrocarbyl group which may contain a heteroatom, and R²² and R²³ may bond together to form a ring with the carbon atom to which they are attached.

In formula (B2-2), R²⁴ is each independently a fluorine atom, C₁-C₅ fluorinated alkyl group or C₁-C₅ fluorinated alkoxy group.

In formula (B2-2), R²⁵ is each independently a C₁-C₁₀ hydrocarbyl group which may contain a heteroatom.

In formula (B2-2), A³ is a single bond, phenylene group, naphthylene group or *-C(=O)-O-A³¹-. A³¹ is a C₁-C₂₀ aliphatic hydrocarbylene group which may contain a hydroxy group, ether bond, ester bond or lactone ring, or a phenylene or naphthylene group. * designates a point of attachment to the carbon atom in the backbone.

Examples of repeat unit B2-2 are shown below, but not limited thereto. R^{A} is as defined above.

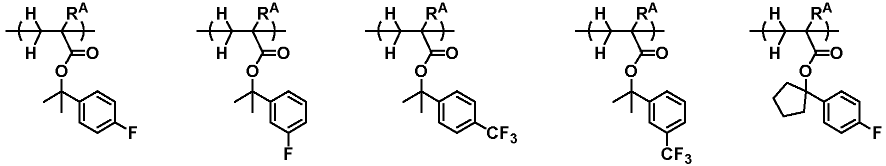

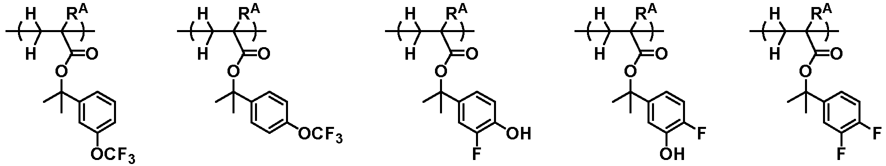

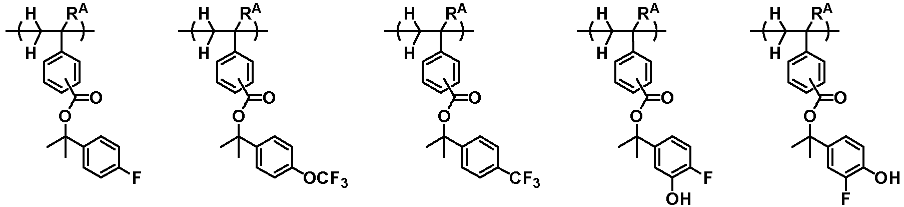

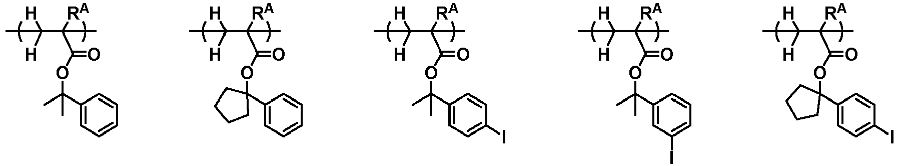

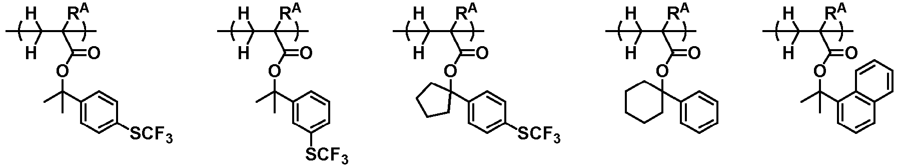

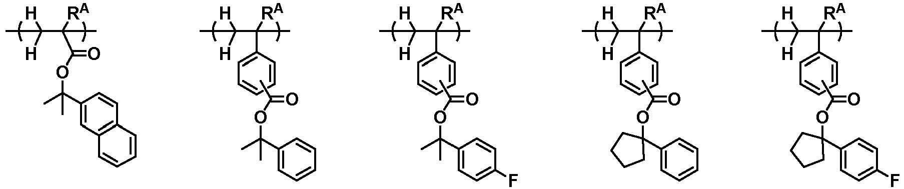

The content of repeat units B2 is preferably 5 to 95 mol%, more preferably 20 to 80 mol% of the overall repeat units of the polymer. Each of the repeat units B2 may be of one type or a combination of plural types.

The polymer may contain repeat units of at least one type selected from repeat units having the formula (B3), repeat units having the formula (B4) and repeat units having the formula (B5). It is noted that the repeat units having the formulae (B3), (B4) and (B5) are also referred to as units B3, B4 and B5, respectively, hereinafter.

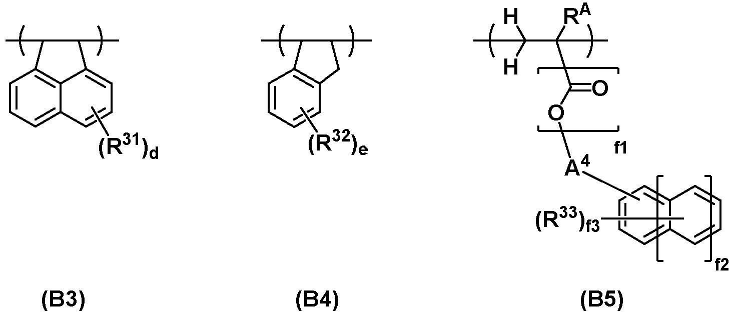

In formulae (B3) and (B4), d is 0, 1, 2, 3, 4, 5 or 6. The subscript e is 0, 1, 2, 3 or 4.

In formulae (B3) and (B4), R³¹ and R³² are each independently a hydroxy group, halogen atom, C₁-C₆ saturated hydrocarbyl group which may be substituted with a halogen atom, C₁-C₆ saturated hydrocarbyloxy group which may be substituted with a halogen atom, or C₂-C₈ saturated hydrocarbylcarbonyloxy group which may be substituted with a halogen atom. The saturated hydrocarbyl, saturated hydrocarbyloxy and saturated hydrocarbylcarbonyloxy groups may be straight, branched or cyclic. A plurality of R³¹ may be identical or different when d is 2 or more. A plurality of R³² may be identical or different when e is 2 or more.

In formula (B5), f1 is 0 or 1. The subscript f2 is 0, 1 or 2. The relevant structure is a benzene skeleton when a2=0, a naphthalene ring when a2=1, and an anthracene skeleton when a2=2. The subscript f3 is 0, 1, 2, 3, 4 or 5. Preferably, f3 is 0, 1, 2 or 3 when f2 is 0. Preferably, f3 is 0, 1, 2, 3 or 4 when f2 is 1 or 2.

In formula (B5), R^{A} is hydrogen, fluorine, methyl, or trifluoromethyl.

In formula (B5), R³³ is a C₁-C₂₀ saturated hydrocarbyl group, a C₁-C₂₀ saturated hydrocarbyloxy group, a C₂-C₂₀ saturated hydrocarbylcarbonyloxy group, a C₂-C₂₀ saturated hydrocarbyloxyhydrocarbyl group, a C₂-C₂₀ saturated hydrocarbylthiohydrocarbyl group, halogen atom, nitro group, or cyano group, and may be a hydroxy group when f2 is 1 or 2. The saturated hydrocarbyl, saturated hydrocarbyloxy, saturated hydrocarbylcarbonyloxy, saturated hydrocarbyloxyhydrocarbyl and saturated hydrocarbylthiohydrocarbyl groups may be straight, branched or cyclic. A plurality of R³³ may be identical or different when f3 is 2 or more.

In formula (B5), A⁴ is a single bond or C₁-C₁₀ saturated hydrocarbylene group in which some constituent -CH₂- may be replaced by -O-. The saturated hydrocarbylene group may be straight, branched or cyclic, and examples thereof are as exemplified for A¹ in formula (B 1).

When repeat units of at least one type selected from repeat units B3 to B5 are incorporated, better performance is obtained because not only the aromatic ring possesses etch resistance, but the cyclic structure incorporated into the main chain also exerts the effect of improving resistance to etching and EB irradiation during pattern inspection step.

The content of repeat units B3 to B5 is preferably 5 mol% or more of the overall repeat units of the polymer for obtaining the effect of improving etch resistance. The content of repeat units B3 to B5 is preferably 25 mol% or less, more preferably 20 mol% or less, of the overall repeat units of the polymer. When the relevant units are free of functional groups or have a functional group other than hydroxy, their content of up to 25 mol% is preferred because the risk of forming development defects is eliminated. Each of the repeat units B3 to B5 may be of one type or a combination of plural types.

It is preferred that the polymer comprise repeat units B 1, repeat units B2, and repeat units of at least one type selected from repeat units B3 to B5, because both etch resistance and high resolution are achievable. The total content of these repeat units is preferably at least 60 mol%, more preferably at least 70 mol%, even more preferably at least 80 mol%, most preferably at least 90 mol% based on the overall repeat units of the polymer.

The polymer further comprises repeat units of at least one type selected from repeat units having the formula (B6) (hereinafter, also referred to as repeat units B6), repeat units having the formula (B7) (hereinafter, also referred to as repeat units B7), repeat units having the formula (B8) (hereinafter, also referred to as repeat units B8), repeat units having the formula (B9) (hereinafter, also referred to as repeat units B9), and repeat units having the formula (B10) (hereinafter, also referred to as repeat units B10), shown below.

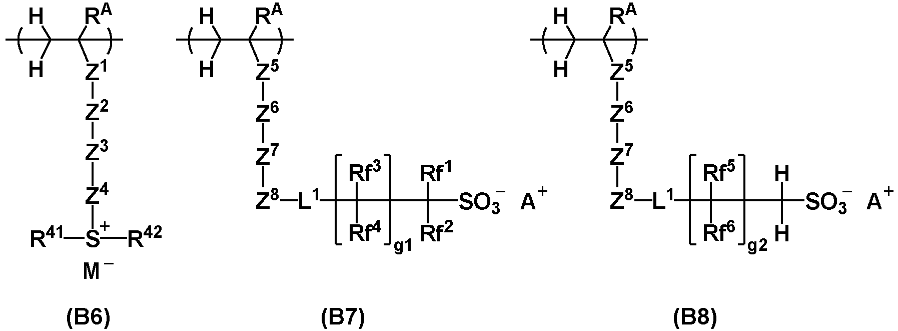

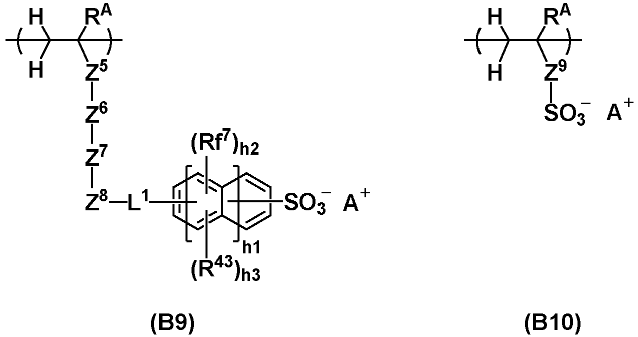

In formulae (B6) and (B10), R^{A} is each independently hydrogen, fluorine, methyl, or trifluoromethyl group. Z¹ is a single bond or optionally substituted phenylene group. Z² is a single bond, *-C(=O)-O-Z²¹-, **-C(=O)-NH-Z²¹- or **-O-Z²¹-. Z²¹ is a C₁-C₆ aliphatic hydrocarbylene group, a phenylene group or a divalent group obtained by combining the foregoing, which may contain halogen, carbonyl moiety, ester bond, ether bond or hydroxy group. Z³ is a single bond, ether bond, ester bond, amide bond, sulfonate ester bond, sulfonamide bond, carbonate bond or carbamate bond. Z⁴ is a single bond, or a C₁-C₆ aliphatic hydrocarbylene group, phenylene group or a divalent group obtained by combining the foregoing, which may contain halogen, carbonyl group, ester bond, ether bond or hydroxy group. Z⁵ is each independently a single bond, optionally substituted phenylene group, naphthylene group, or *-C(=O)-O-Z⁵¹-. Z⁵¹ is a C₁-C₁₀ aliphatic hydrocarbylene group which may contain halogen, hydroxy moiety, ether bond, ester bond or lactone ring, or phenylene or naphthylene group. Z⁶ is a single bond, ether bond, ester bond, amide bond, sulfonate ester bond, sulfonamide bond, carbonate bond or carbamate bond. Z⁷ is each independently a single bond, ***-Z⁷¹-C(=O)-O-, ***-C(=O)-NH-Z⁷¹-, or ***-O-Z⁷¹-. Z⁷¹ is a C₁-C₂₀ hydrocarbylene group which may contain a heteroatom. Z⁸ is each independently a single bond, ****-Z⁸¹-C(=O)-O-, ****-C(=O)-NH-Z⁸¹-, or ****-O-Z⁸¹-. Z⁸¹ is a C₁-C₂₀ hydrocarbylene group which may contain a heteroatom. Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-. Z⁹¹ is a C₁-C₆ aliphatic hydrocarbylene group, phenylene group, fluorinated phenylene group or trifluoromethyl substituted phenylene group, which may contain a carbonyl moiety, ester bond, ether bond or hydroxy group. * designates a point of attachment to the carbon atom in the backbone. ** designates a point of attachment to Z¹. *** designates a point of attachment to Z⁶. **** designates a point of attachment to Z⁷.

The aliphatic hydrocarbylene groups Z²¹, Z⁵¹ and Z⁹¹ may be straight, branched or cyclic. Examples thereof include alkanediyl groups such as methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, 1,1-dimethylethane-1,2-diyl, pentane-1,5-diyl, 2-methylbutane-1,2-diyl, and hexane-1,6-diyl; cycloalkanediyl groups such as cyclopropanediyl, cyclobutanediyl, cyclopentanediyl and cyclohexanediyl, and combinations thereof.

The hydrocarbylene groups Z⁷¹ and Z⁸¹ which may contain a heteroatom may be saturated or unsaturated and straight, branched or cyclic. Examples of the hydrocarbylene group are shown below, but not limited thereto.

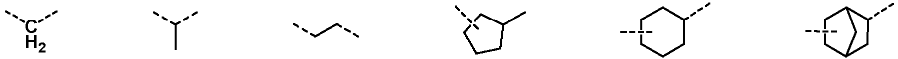

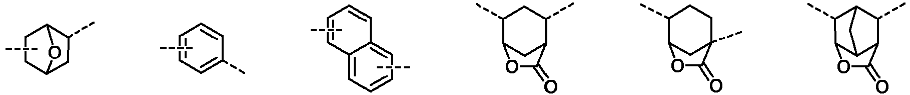

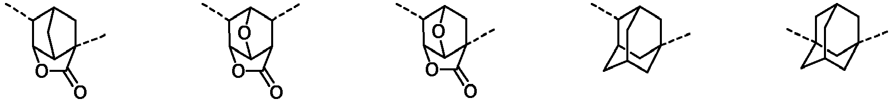

Herein the broken line designates a point of attachment.

In formula (B6), R⁴¹ to R⁴² are each independently a C₁-C₂₀ hydrocarbyl group which may contain a heteroatom. The hydrocarbyl group may be saturated or unsaturated, and may be straight, branched, or cyclic. Examples thereof include C₁-C₂₀ alkyl groups such as methyl, ethyl, n-propyl, isopropyl, n-butyl, sec-butyl and tert-butyl groups; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl groups; C₂-C₂₀ alkenyl groups such as vinyl, 1-propenyl, 2-propenyl, butenyl and hexenyl groups; C₃-C₂₀ cyclic unsaturated hydrocarbyl groups such as a cyclohexenyl group; C₆-C₂₀ aryl groups such as phenyl, naphthyl and thienyl groups; C₇-C₂₀ aralkyl groups such as benzyl, 1-phenylethyl and 2-phenylethyl groups; and combinations thereof. The aryl groups are preferred. In the hydrocarbyl group, some or all hydrogen may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-) or haloalkyl moiety.

R⁴¹ and R⁴² may bond together to form a ring with sulfur to which they are attached. Examples of the ring are shown below.

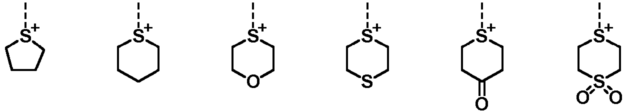

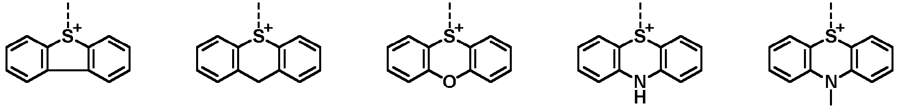

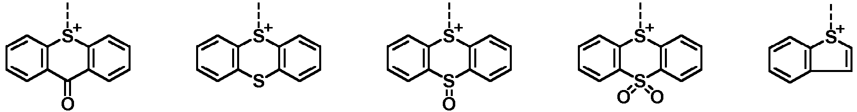

Herein the broken line designates a point of attachment to Z⁴.

Examples of the cation in repeat units B6 are given below, but not limited thereto. R^{A} is as defined above.

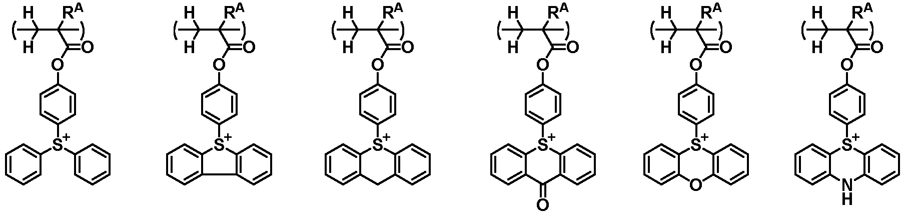

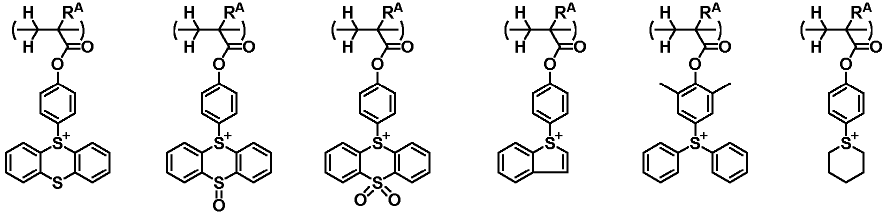

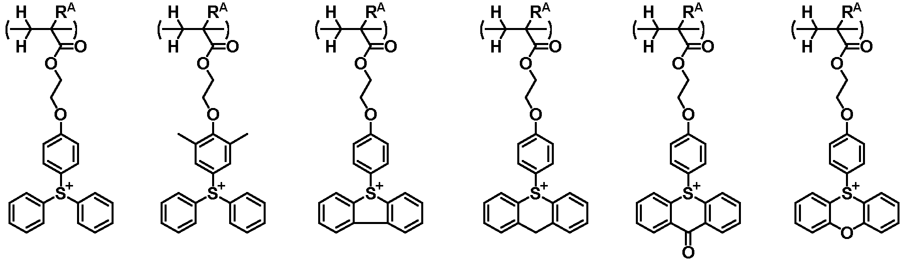

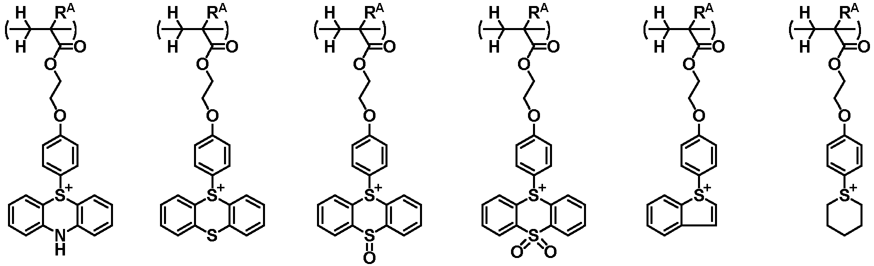

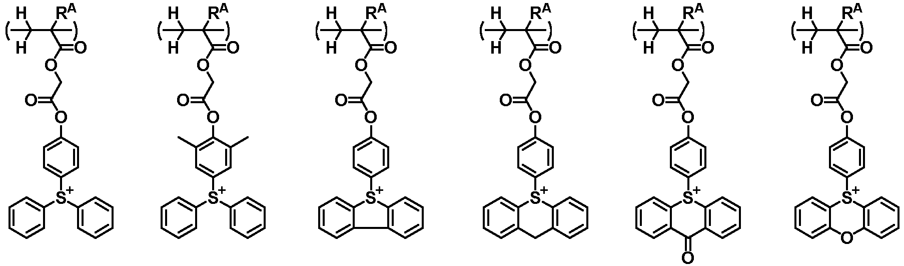

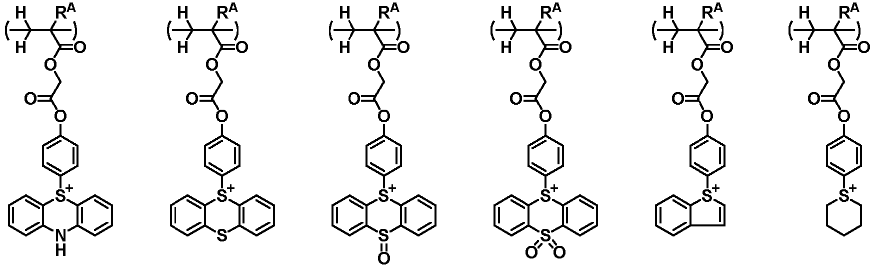

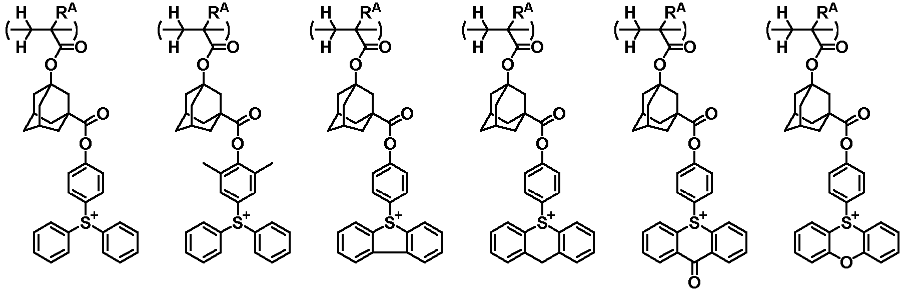

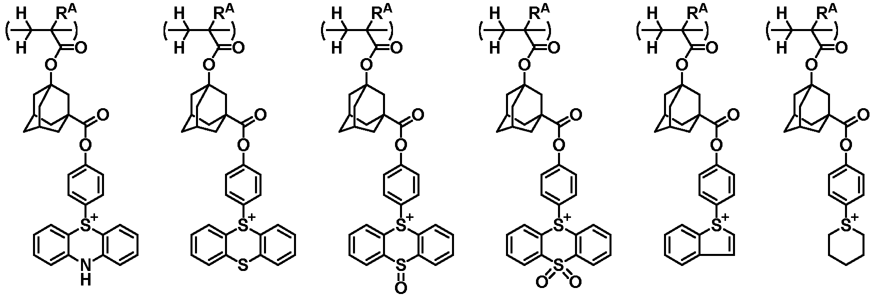

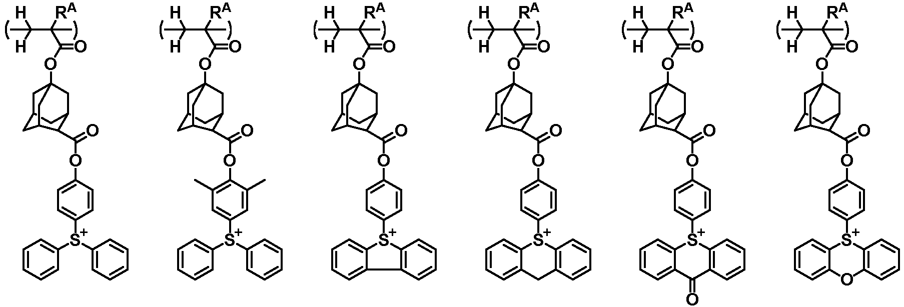

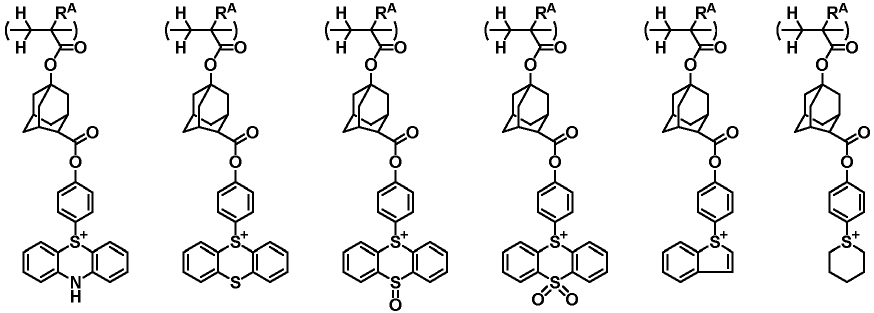

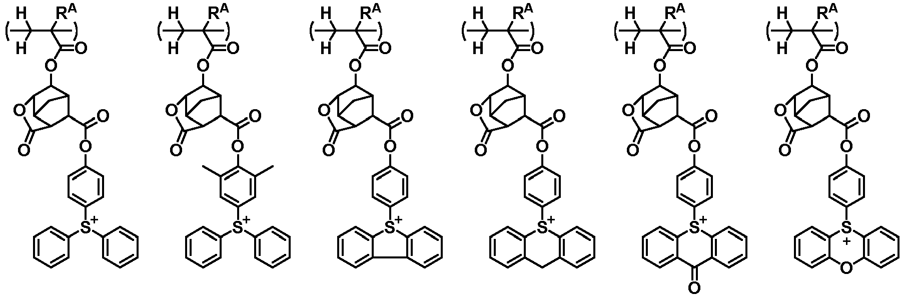

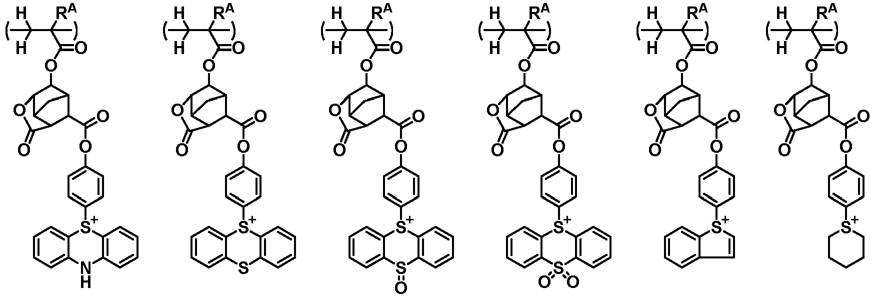

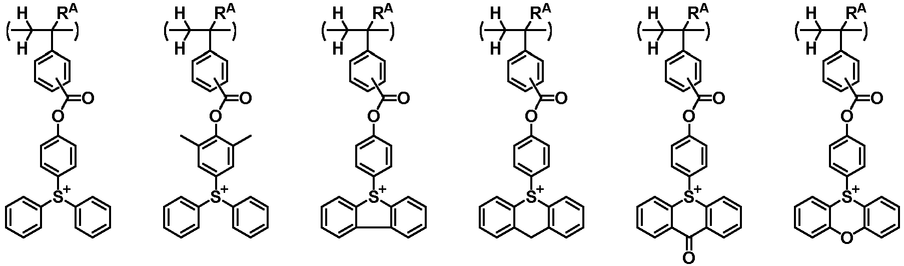

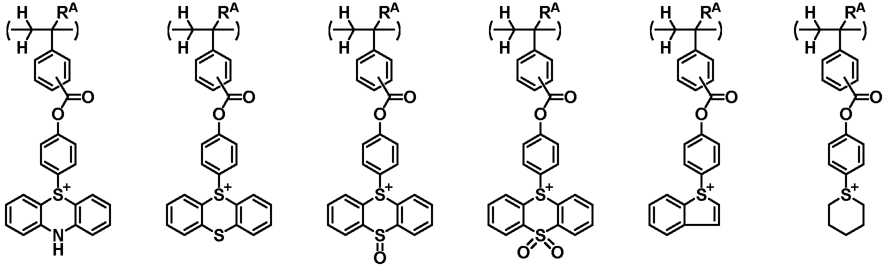

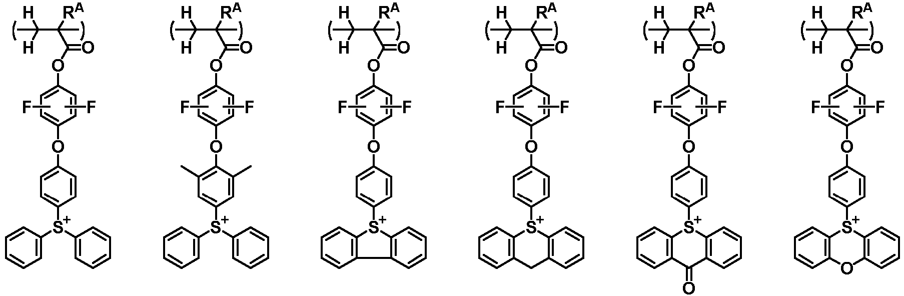

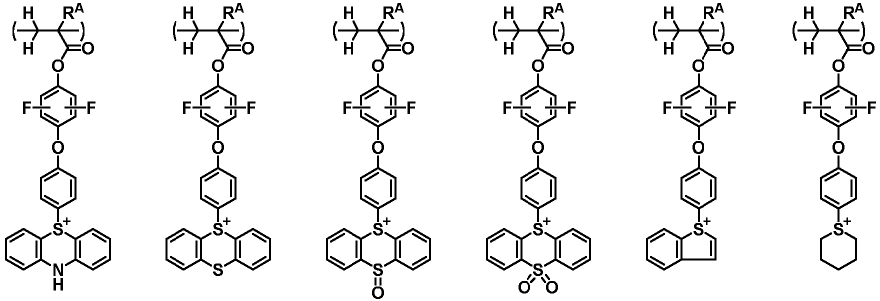

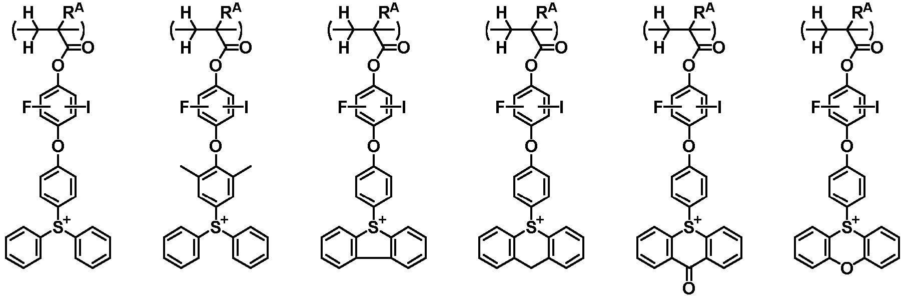

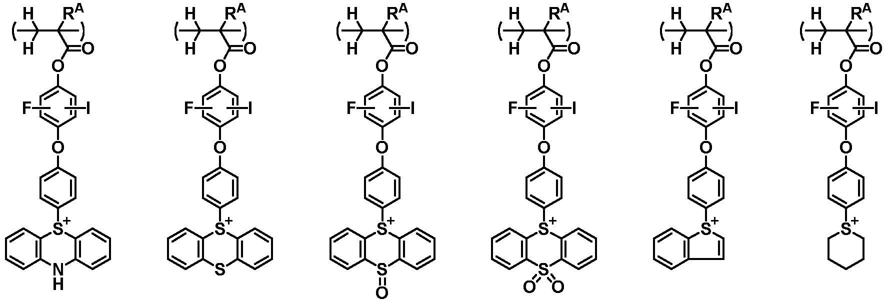

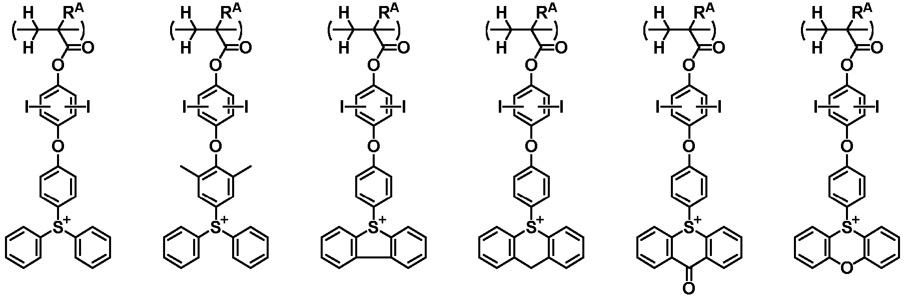

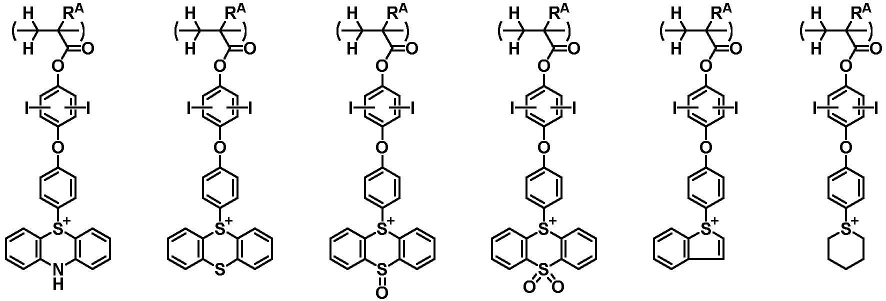

In formula (B6), M⁻ is a non-nucleophilic counter ion. Halide ions, sulfonate anions, imide anions, and methide anions are preferred as the non-nucleophilic counter ion. Examples of the halide ions include chloride and bromide ions; sulfonate anions, specifically fluoroalkylsulfonate ions such as triflate, 1,1,1-trifluoroethanesulfonate, and nonafluorobutanesulfonate, arylsulfonate ions such as tosylate, benzenesulfonate, 4-fluorobenzenesulfonate, and 1,2,3,4,5-pentafluorobenzenesulfonate, alkylsulfonate ions such as mesylate and butanesulfonate; imide ions such as bis(trifluoromethylsulfonyl)imide, bis(perfluoroethylsulfonyl)imide and bis(perfluorobutylsulfonyl)imide; and methide ions such as tris(trifluoromethylsulfonyl)methide and tris(perfluoroethylsulfonyl)methide.

Anions having the following formulae (B6-1) to (B6-4) are also useful as the non-nucleophilic counter ion.

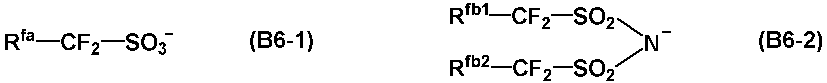

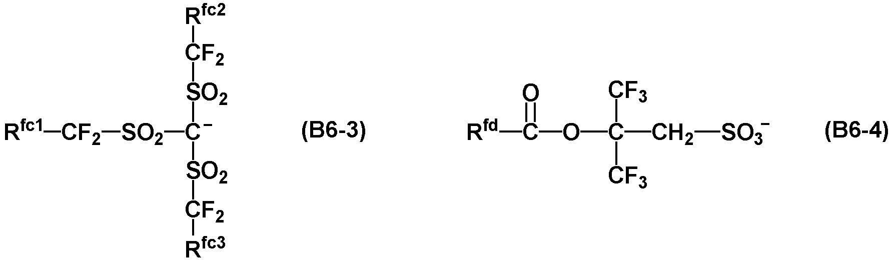

In formula (B6-1), R^{fa} is fluorine or a C₁-C₄₀ hydrocarbyl group which may contain a heteroatom. The hydrocarbyl group may be saturated or unsaturated, and may be straight, branched, or cyclic. Examples thereof are as will be exemplified below for the hydrocarbyl group R^{fa1} in formula (B6-1-1).

Preferably, the anion of formula (B6-1) has the formula (B6-1-1).

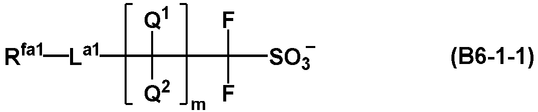

In formula (B6-1-1), Q¹ and Q² are each independently hydrogen, fluorine or a C₁-C₆ fluorinated saturated hydrocarbyl group. Preferably, at least one of Q¹ and Q² is a trifluoromethyl group. The subscript m is 0, 1, 2, 3 or 4, preferably 1. R^{fa1} is a C₁-C₃₅ hydrocarbyl group which may contain a heteroatom. As the heteroatom, oxygen, nitrogen, sulfur and halogen atoms are preferred, with oxygen being more preferred. Of the hydrocarbyl groups, those groups of 6 to 30 carbon atoms are preferred from the aspect of achieving a high resolution in forming patterns of small feature size.

In formula (B6-1-1), the C₁-C₃₅ hydrocarbyl group R^{fa1} may be saturated or unsaturated and straight, branched or cyclic. Examples thereof include C₁-C₃₅ alkyl groups such as methyl, ethyl, n-propyl, isopropyl, n-butyl, isobutyl, sec-butyl, tert-butyl, pentyl, neopentyl, hexyl, heptyl, 2-ethylhexyl, nonyl, undecyl, tridecyl, pentadecyl, heptadecyl and icocyl groups; C₃-C₃₅ cyclic saturated hydrocarbyl groups such as cyclopentyl, cyclohexyl, 1-adamantyl, 2-adamantyl, 1-adamantylmethyl, norbornyl, norbornylmethyl, tricyclodecyl, tetracyclodecyl, tetracyclodecylmethyl and dicyclohexylmethyl groups; C₂-C₃₅ unsaturated aliphatic hydrocarbyl groups such as 2-propenyl and 3-cyclohexenyl groups; C₆-C₃₅ aryl groups such as phenyl, 1-naphthyl, 2-naphthyl and 9-fluorenyl groups; C₇-C₃₅ aralkyl groups such as benzyl and diphenylmethyl groups; and combinations thereof.

In the hydrocarbyl group, some or all hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain hydroxy, fluorine, chlorine, bromine, iodine, cyano, nitro, carbonyl, ether bond, ester bond, sulfonic ester bond, carbonate bond, lactone ring, sultone ring, carboxylic anhydride (-C(=O)-O-C(=O)-), or haloalkyl moiety. Examples of the heteroatom-containing hydrocarbyl group include tetrahydrofuryl, methoxymethyl, ethoxymethyl, methylthiomethyl, acetamidomethyl, trifluoroethyl, (2-methoxyethoxy)methyl, acetoxymethyl, 2-carboxy-1-cyclohexyl, 2-oxopropyl, 4-oxo-1-adamantyl, and 3-oxocyclohexyl groups.

In formula (B6-1-1), L^{a1} is a single bond, ether bond, ester bond or sulfonic ester bond. From the aspect of synthesis, an ether bond or ester bond is preferred, with the ester bond being more preferred.

Examples of the anion having formula (B6-1) are shown below, but not limited thereto. In the following formulae, Q¹ is as defined above, and Ac is an acetyl group.

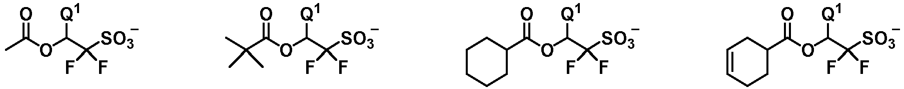

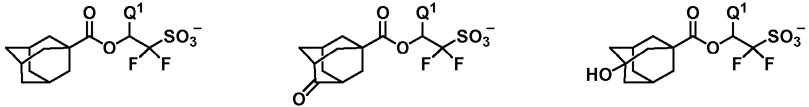

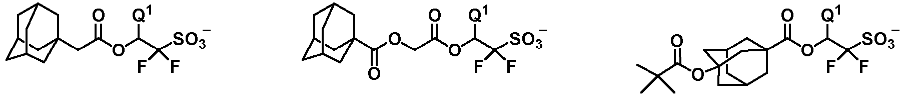

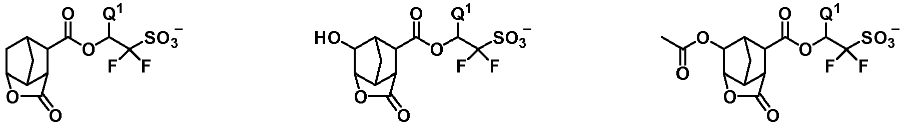

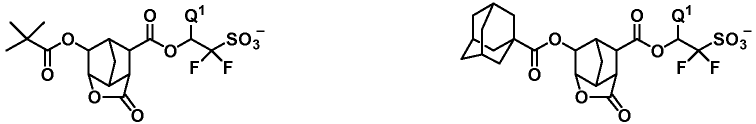

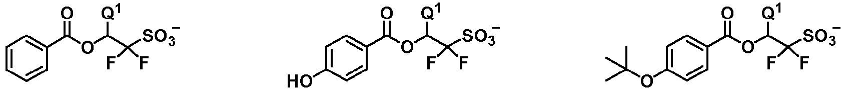

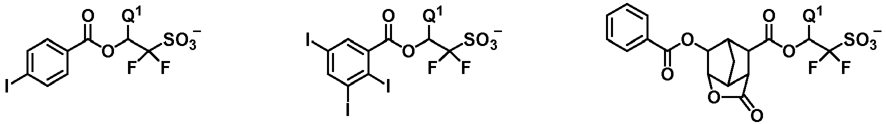

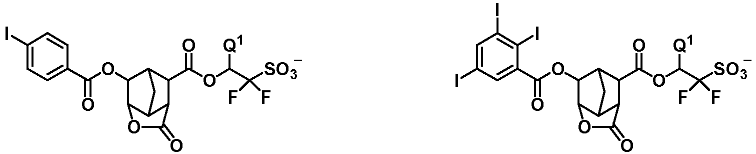

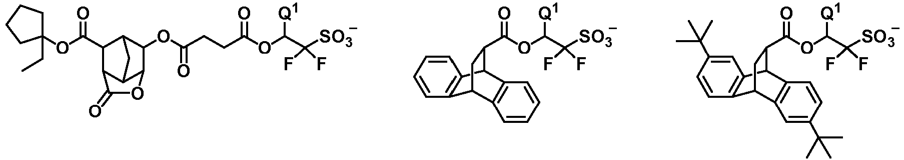

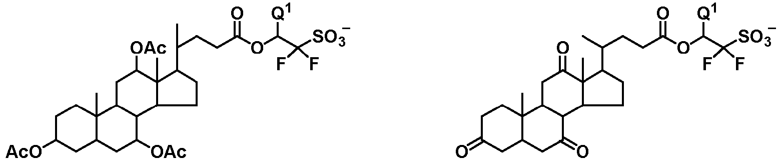

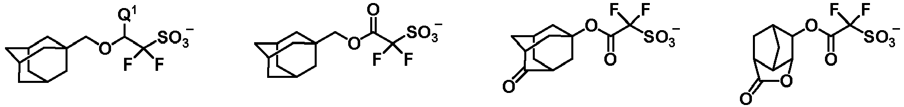

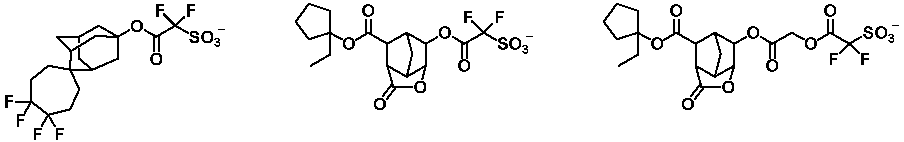

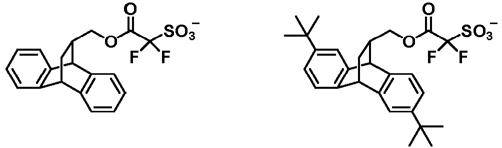

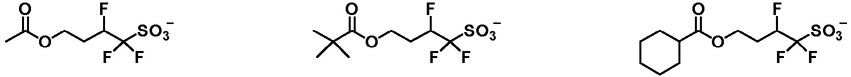

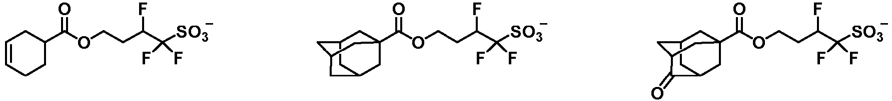

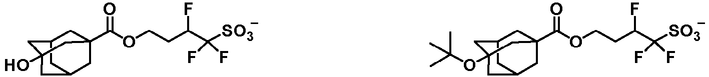

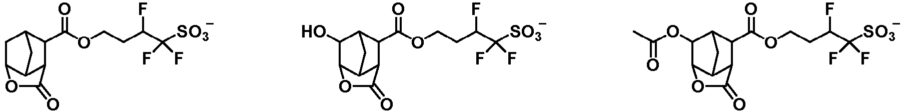

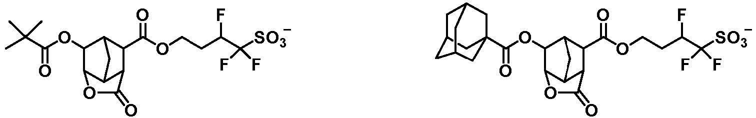

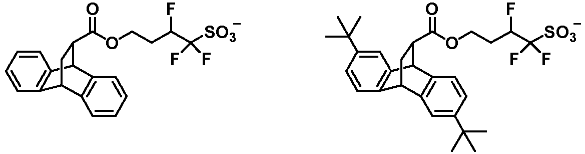

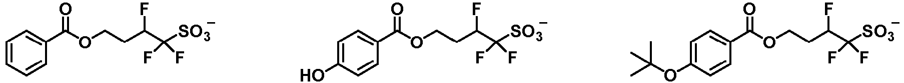

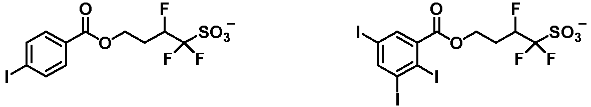

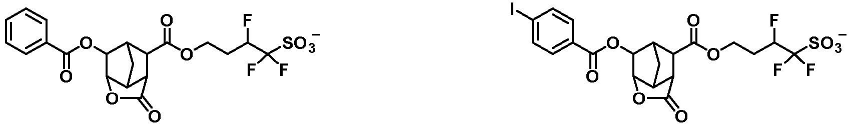

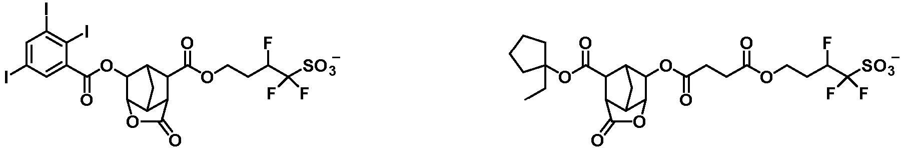

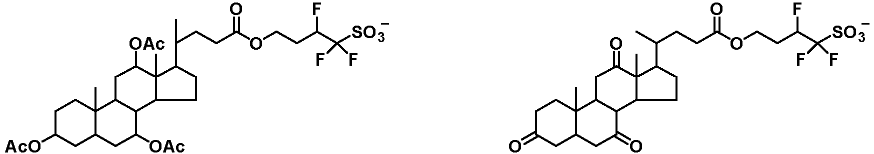

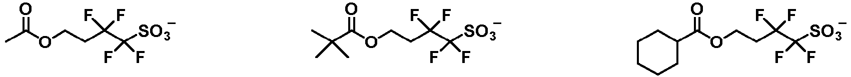

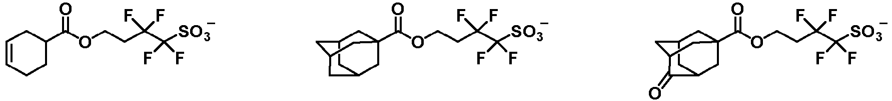

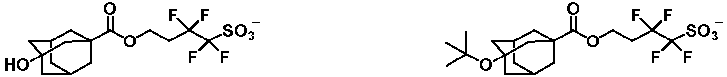

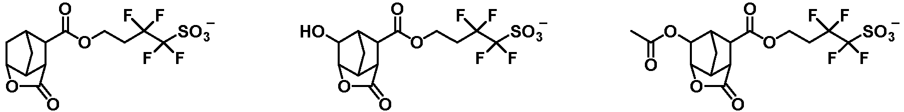

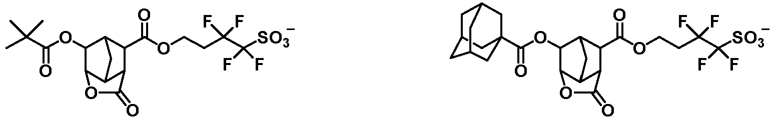

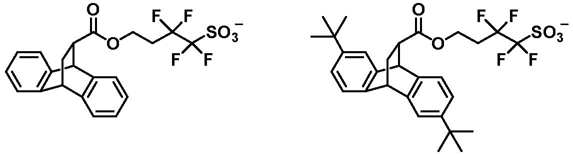

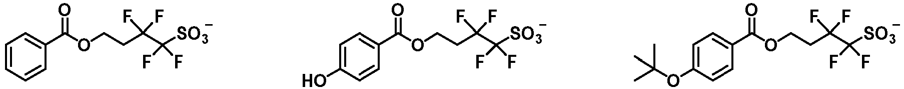

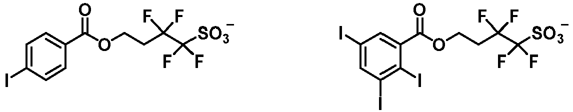

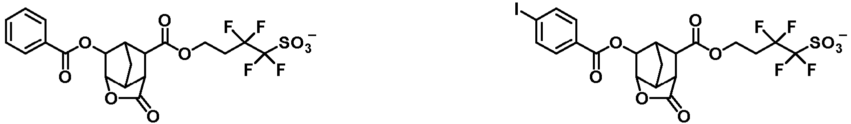

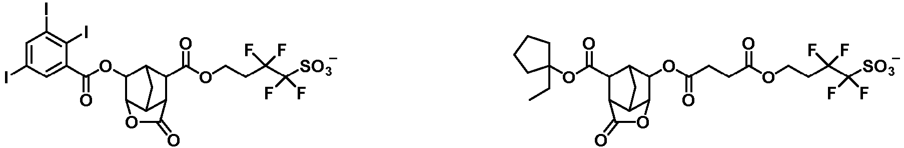

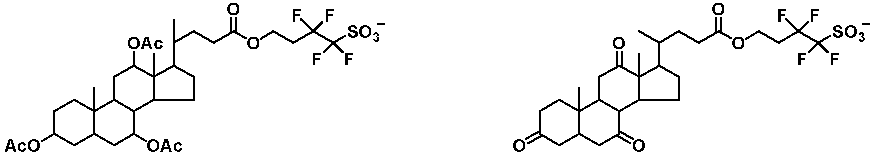

In formula (B6-2), R^{fb1} and R^{fb2} are each independently fluorine, or a C₁-C₄₀ hydrocarbyl group which may contain a heteroatom. The hydrocarbyl group may be saturated or unsaturated, and may be straight, branched, or cyclic. Examples thereof are as exemplified above for the hydrocarbyl group R^{fa1} in formula (B6-1-1). Preferably R^{fb1} and R^{fb2} are fluorine or C₁-C₄ straight fluorinated alkyl groups. Also, R^{fb1} and R^{fb2} may bond together to form a ring with the linkage: -CF₂-SO₂-N⁻-SO₂-CF₂- to which they are attached. It is preferred that a combination of R^{fb1} and R^{fb2} be a fluorinated ethylene or fluorinated propylene group.

In formula (B6-3), R^{fc1}, R^{fc2} and R^{fc3} are each independently fluorine or a C₁-C₄₀ hydrocarbyl group which may contain a heteroatom. The hydrocarbyl group may be saturated or unsaturated, and may be straight, branched, or cyclic. Examples thereof are as exemplified above for the hydrocarbyl group R^{fa1} in formula (B6-1-1). Preferably R^{fc1}, R^{fc2} and R^{fc3} are fluorine or C₁-C₄ straight fluorinated alkyl groups. Also, R^{fc1} and R^{fc2} may bond together to form a ring with the linkage: -CF₂-SO₂-C⁻-SO₂-CF₂- to which they are attached. It is preferred that a combination of R^{fc1} and R^{fc2} be a fluorinated ethylene or fluorinated propylene group.

In formula (B6-4), R^{fd} is a C₁-C₄₀ hydrocarbyl group which may contain a heteroatom. The hydrocarbyl group may be saturated or unsaturated, and may be straight, branched, or cyclic. Examples thereof are as exemplified above for the hydrocarbyl group R^{fa1} in formula (B6-1-1).

Examples of the anion having formula (B6-4) are shown below, but not limited thereto.

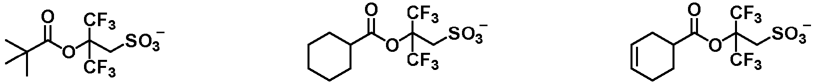

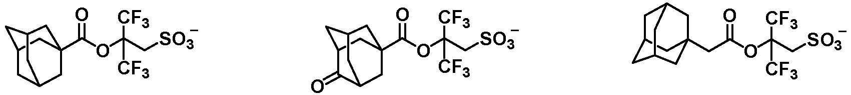

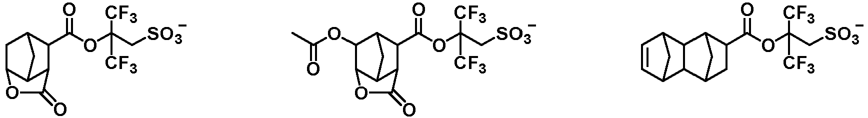

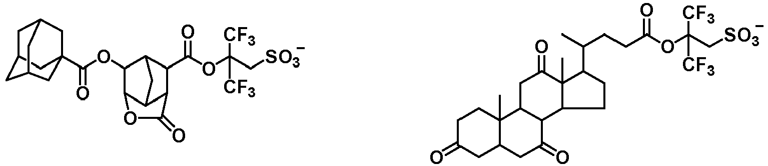

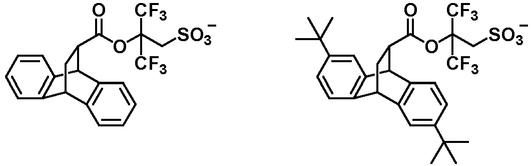

Anions having an iodized or brominated aromatic ring are also useful as the non-nucleophilic counter ion. Examples of the anion include anions of the formula (B6-5).

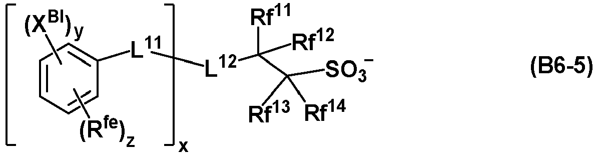

In formula (B6-5), x is 1, 2 or 3. The subscript y is 1, 2, 3, 4 or 5. The subscript z is 0, 1, 2 or 3. The sum y+z is from 1 to 5. The subscript y is preferably 1, 2 or 3, more preferably 2 or 3. The subscript z is preferably 0, 1 or 2.

In formula (B6-5), X^{BI} is iodine or bromine, and may be different or identical when x and/or y are 2 or more.

In formula (B6-5), L¹¹ is a single bond, an ether bond, an ester bond, or a C₁-C₆ saturated hydrocarbylene group which may contain an ether bond or an ester bond. The saturated hydrocarbylene group may be straight, branched or cyclic.

In formula (B6-5), L¹² is a single bond or a C₁-C₂₀ divalent linking group when x=1, or a C₁-C₂₀ (x+1)-valent linking group when x=2 or 3. The linking group may contain an oxygen, sulfur or nitrogen atom.

In formula (B6-5), R^{fe} is hydroxy, carboxy, fluorine, chlorine, bromine, amino group, or a C₁-C₂₀ hydrocarbyl, C₁-C₂₀ hydrocarbyloxy, C₂-C₂₀ hydrocarbylcarbonyl, C₂-C₂₀ hydrocarbyloxycarbonyl, C₂-C₂₀ hydrocarbylcarbonyloxy, or C₁-C₂₀ hydrocarbylsulfonyloxy group, which may contain fluorine, chlorine, bromine, hydroxy, amino or ether bond, or -N(R^{feA})(R^{feB}), -N(R^{feC})-C(=O)-R^{feD} or -N(R^{feC})-C(=O)-O-R^{feD}. R^{feA} and R^{feB} are each independently hydrogen or a C₁-C₆ saturated hydrocarbyl group. R^{feC} is hydrogen, or a C₁-C₆ saturated hydrocarbyl group which may contain halogen, hydroxy, a C₁-C₆ saturated hydrocarbyloxy, C₂-C₆ saturated hydrocarbylcarbonyl or C₂-C₆ saturated hydrocarbylcarbonyloxy group. R^{feD} is a C₁-C₁₆ aliphatic hydrocarbyl group, a C₆-C₁₂ aryl group, or a C₇-C₁₅ aralkyl group, halogen, hydroxy group, a C₁-C₆ saturated hydrocarbyloxy group, C₂-C₆ saturated hydrocarbylcarbonyl group, or C₂-C₆ saturated hydrocarbylcarbonyloxy group. The aliphatic hydrocarbyl group may be saturated or unsaturated and straight, branched or cyclic. The hydrocarbyl, hydrocarbyloxy, hydrocarbylcarbonyl, hydrocarbyloxycarbonyl, hydrocarbylcarbonyloxy and hydrocarbylsulfonyloxy groups may be straight, branched or cyclic. A plurality of R^{fe} may be identical or different when x and/or z is 2 or more.

Of these, R^{fe} is preferably a hydroxy group, -N(R^{feC})-C(=O)-R^{feD}, -N(R^{feC})-C(=O)-O-R^{feD}, fluorine, chlorine, bromine, a methyl group, or a methoxy group.

In formula (B6-5), Rf¹¹ to Rf¹⁴ are each independently hydrogen, fluorine or trifluoromethyl, at least one of Rf¹¹ to Rf¹⁴ is fluorine or trifluoromethyl. Rf¹¹ and Rf¹², taken together, may form a carbonyl group. More preferably, both Rf¹³ and Rf¹⁴ are fluorine.

Examples of the anion having formula (B6-5) are shown below, but not limited thereto. X^{BI} is as defined above.

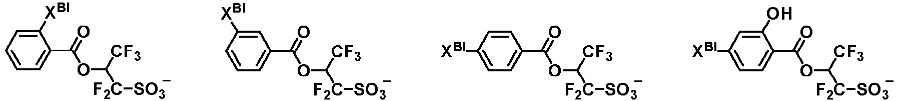

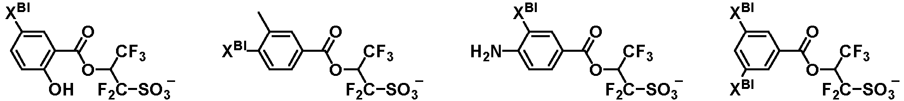

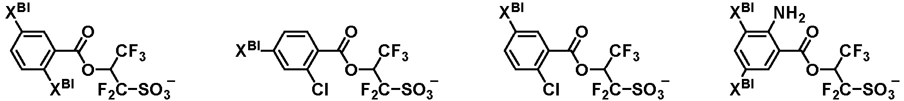

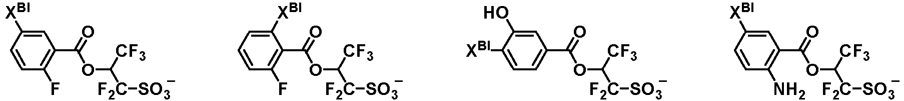

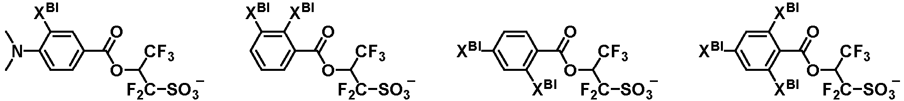

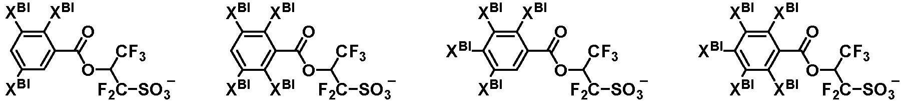

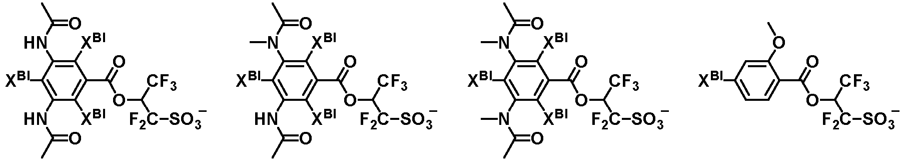

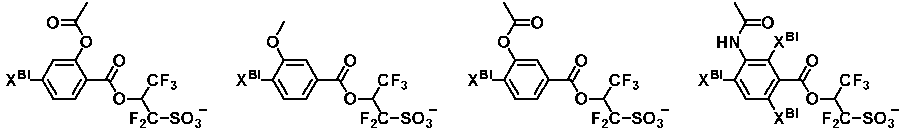

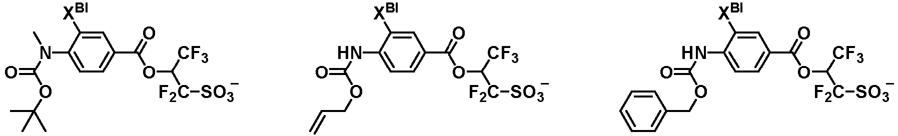

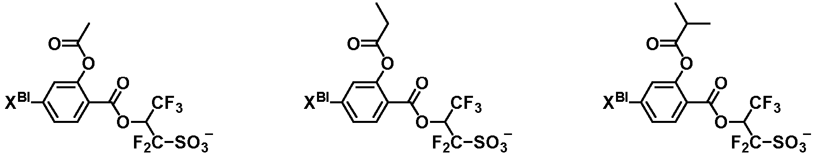

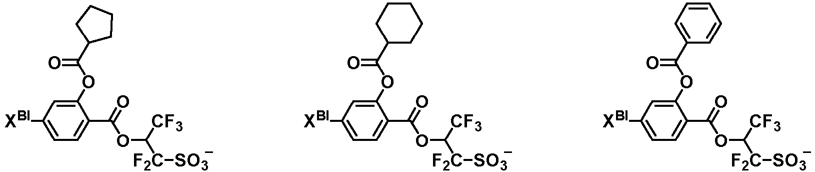

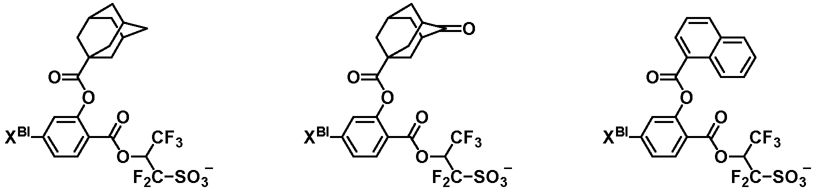

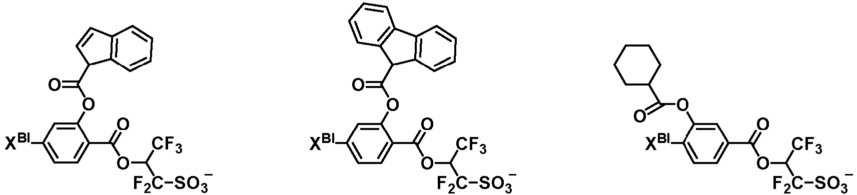

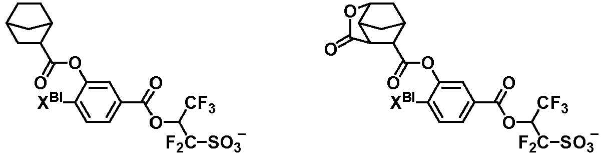

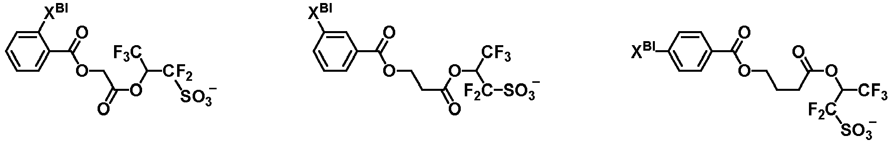

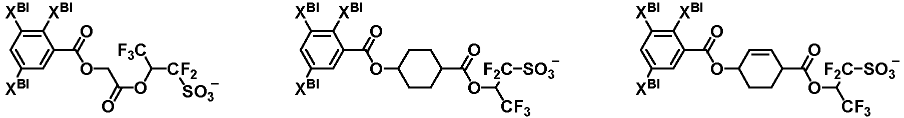

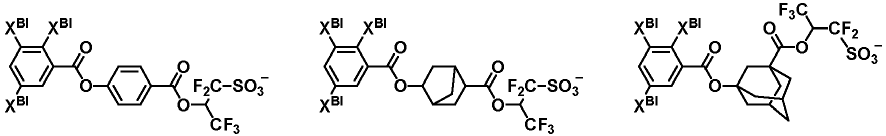

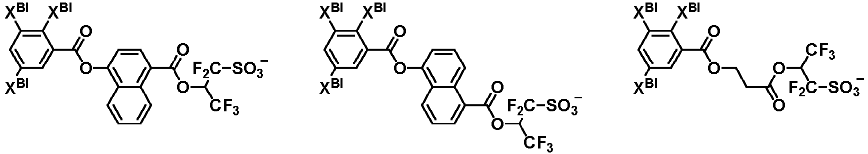

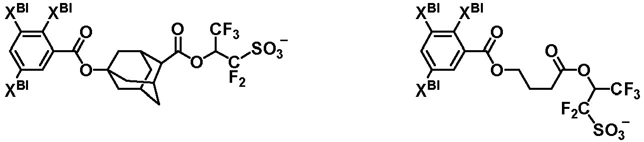

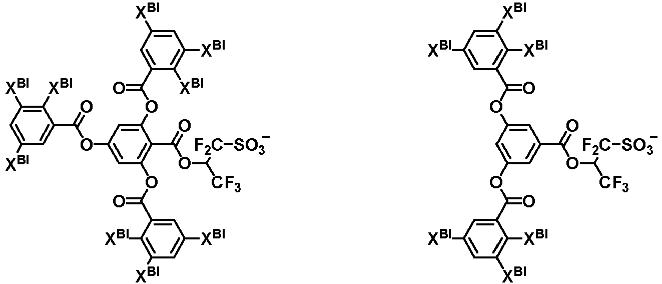

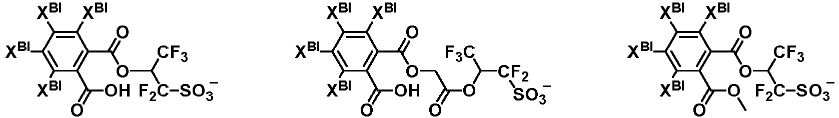

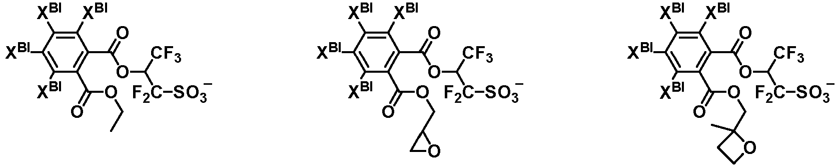

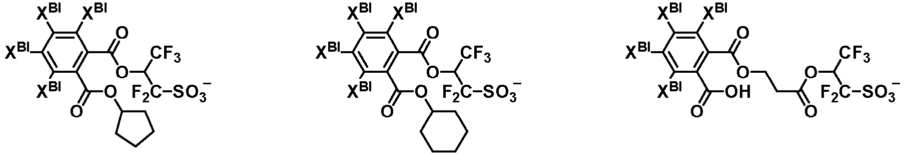

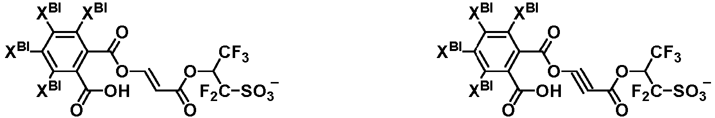

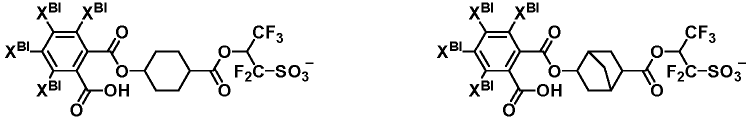

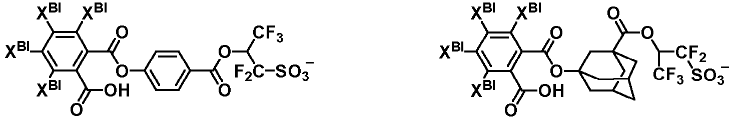

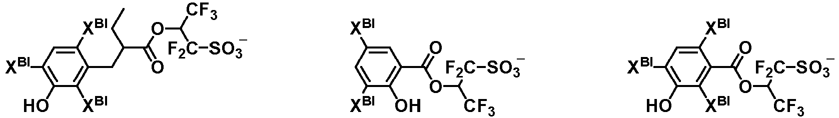

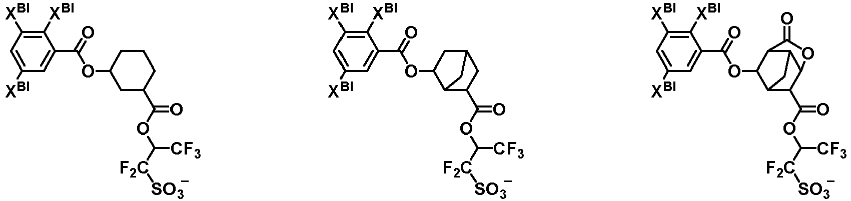

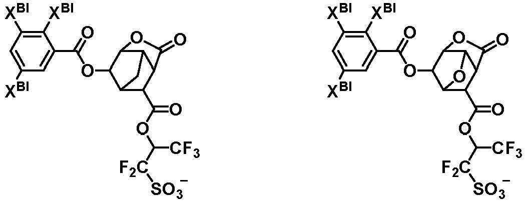

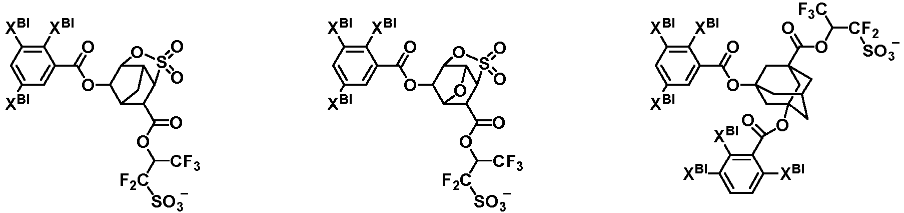

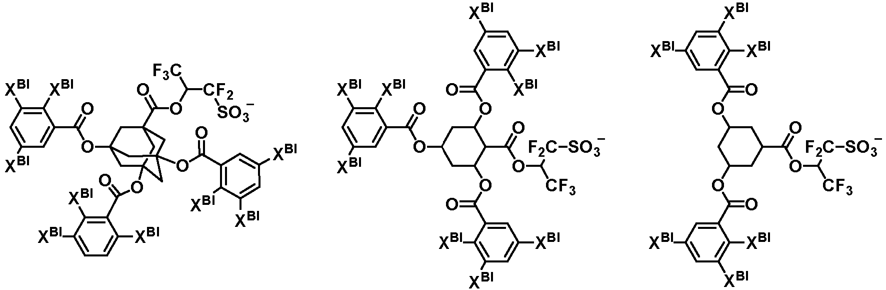

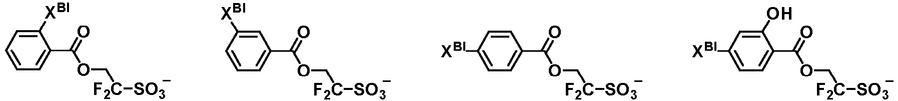

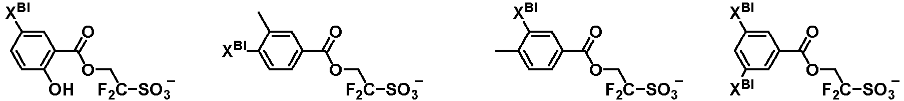

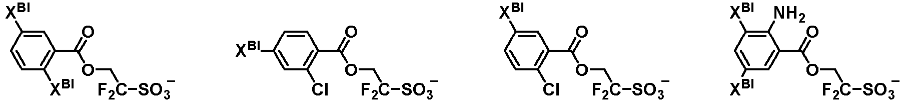

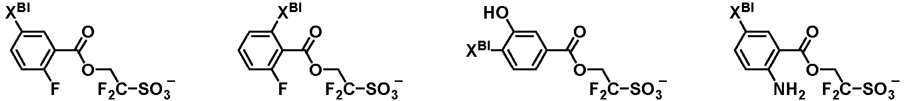

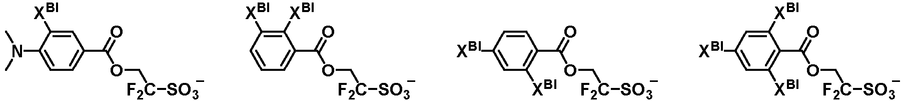

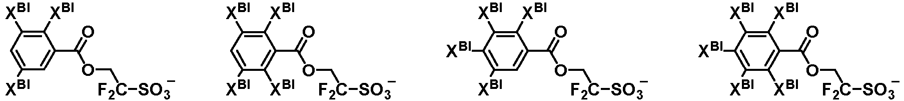

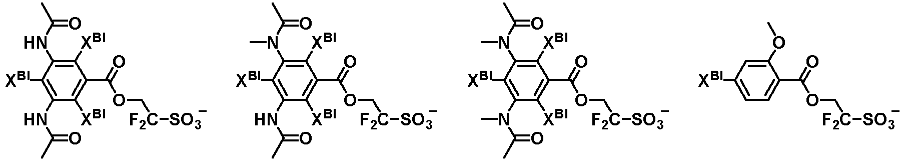

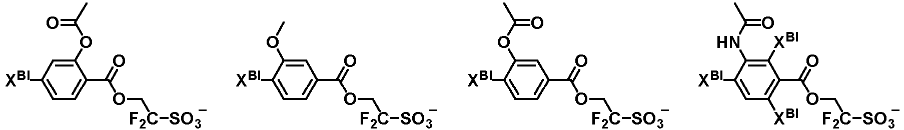

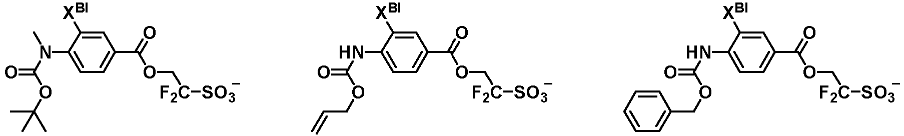

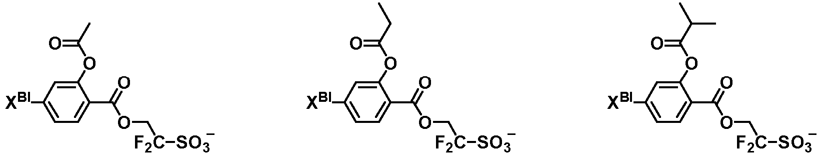

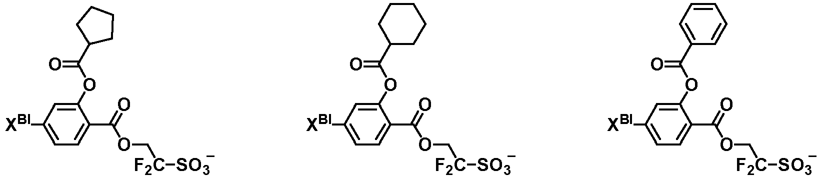

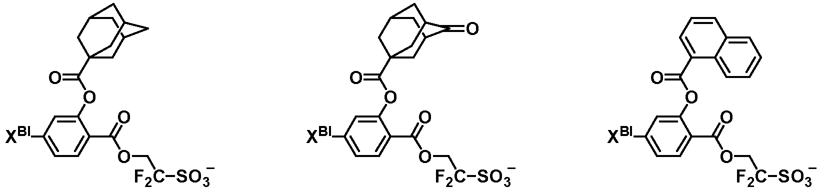

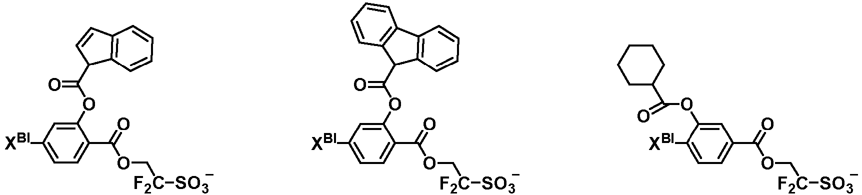

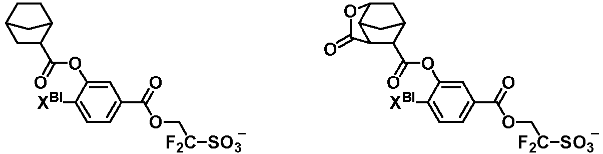

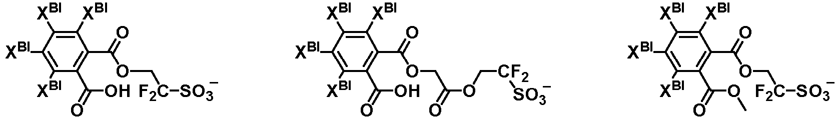

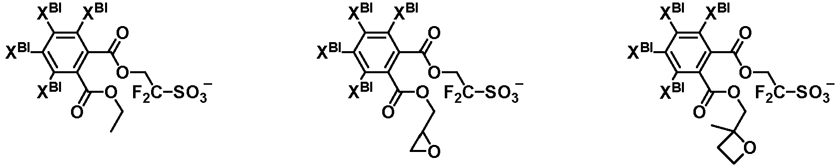

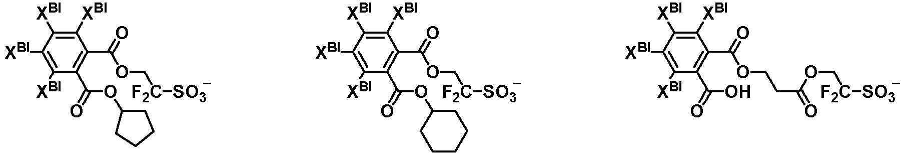

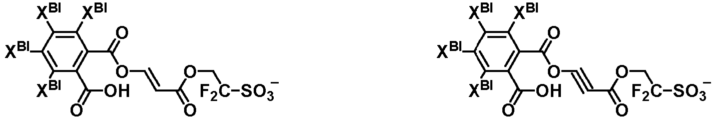

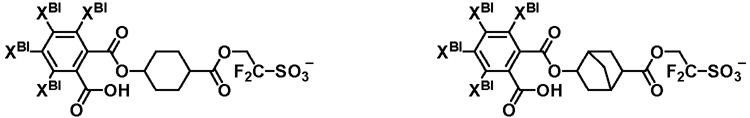

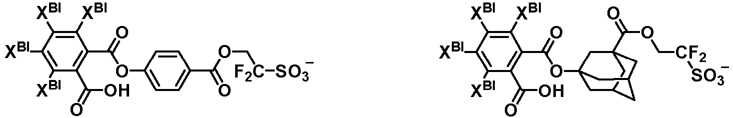

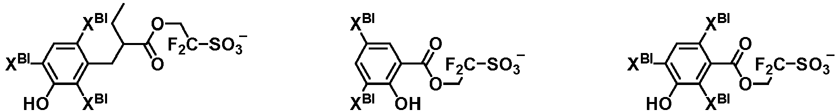

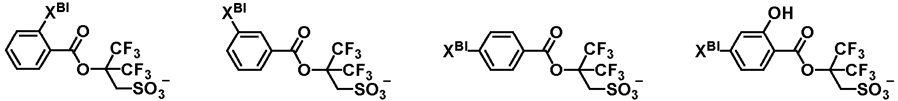

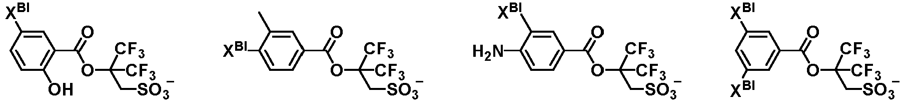

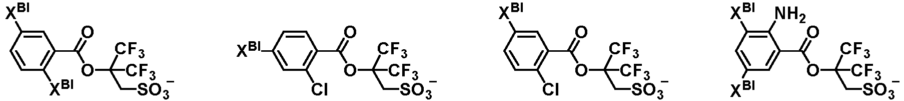

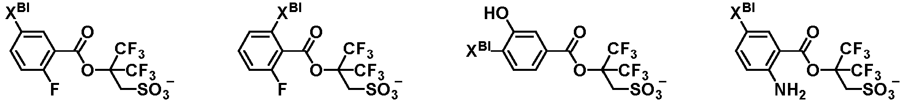

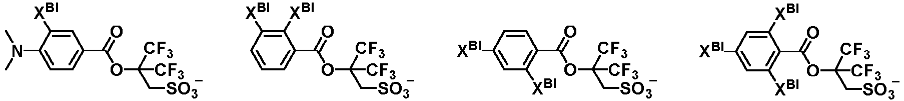

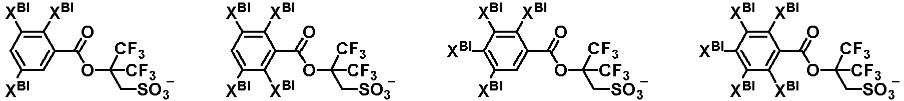

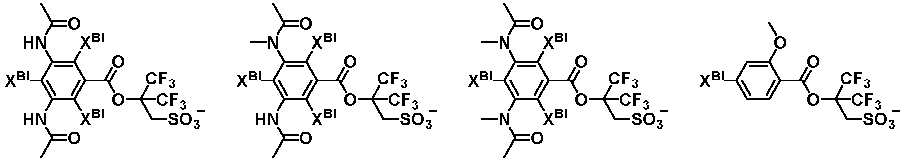

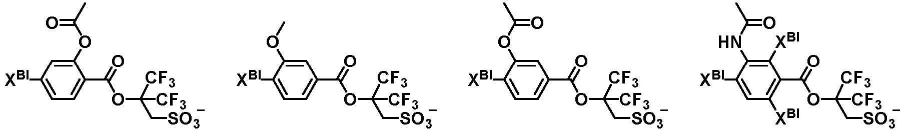

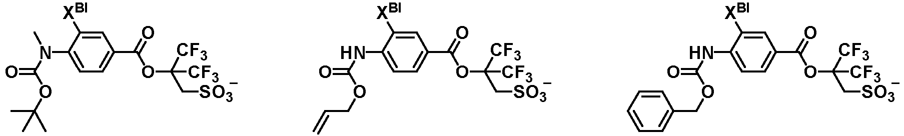

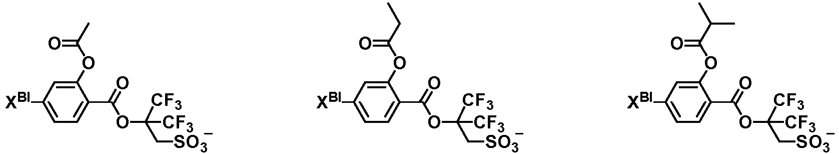

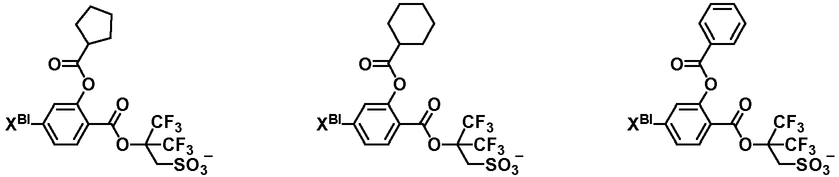

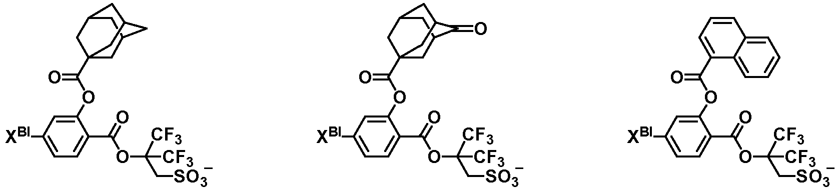

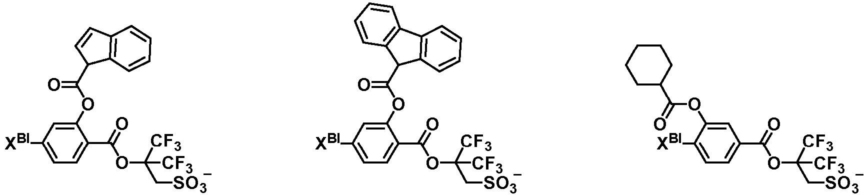

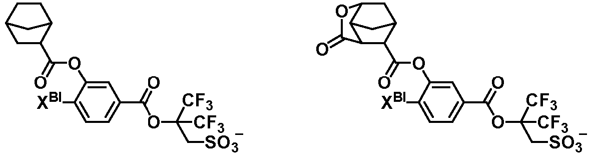

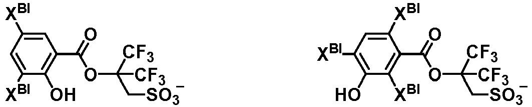

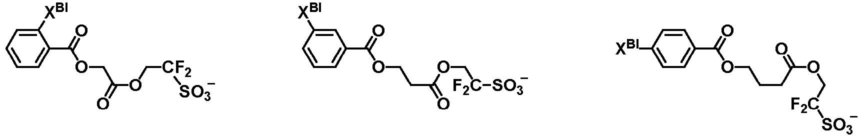

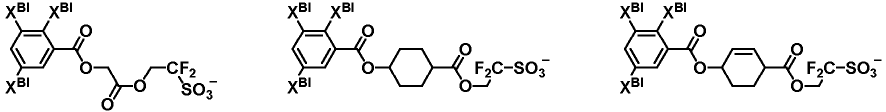

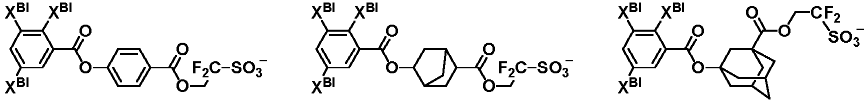

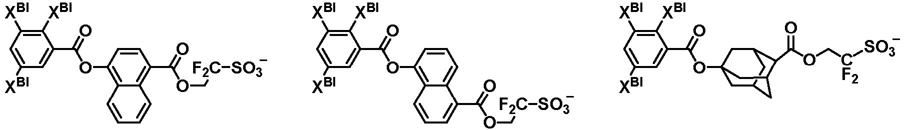

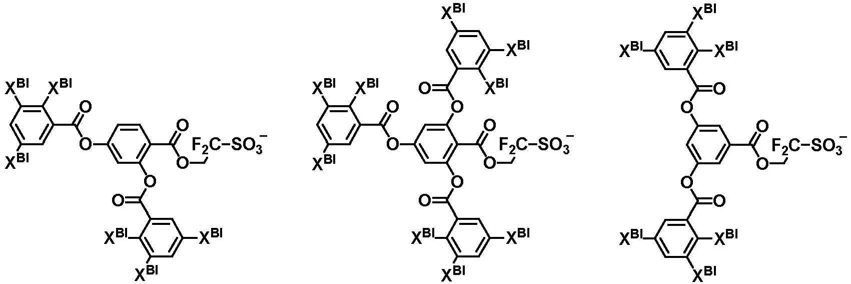

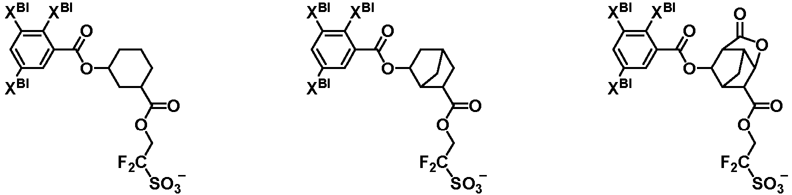

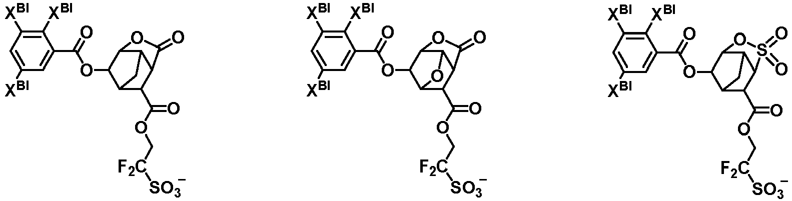

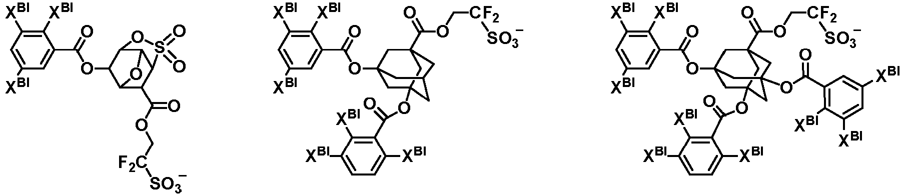

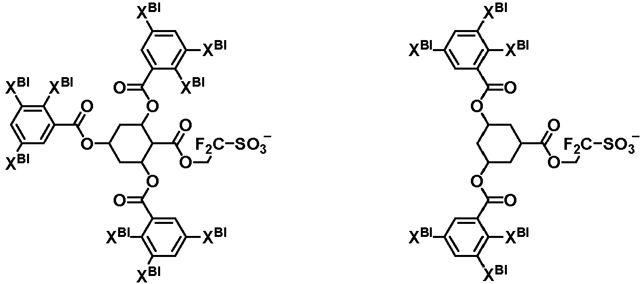

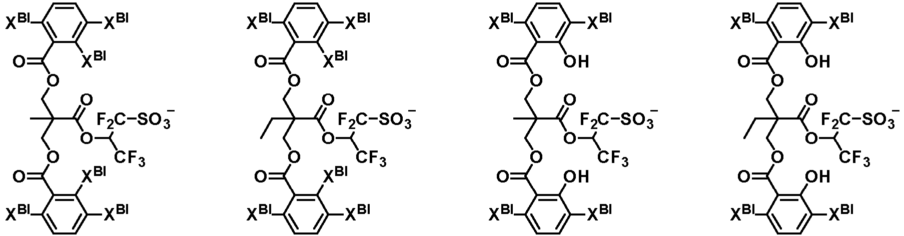

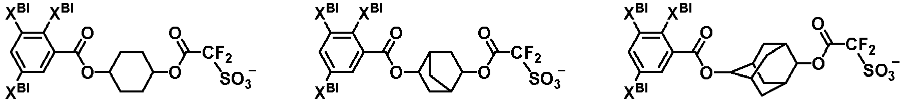

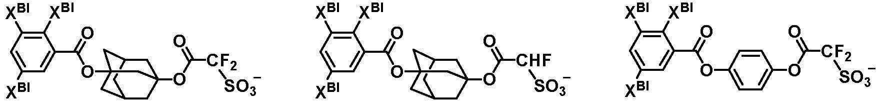

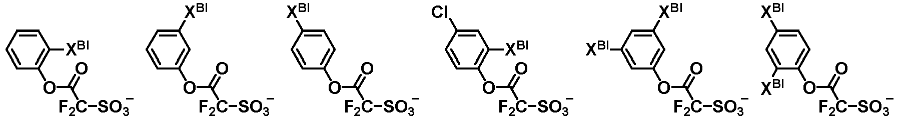

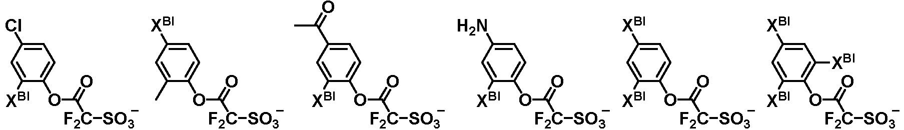

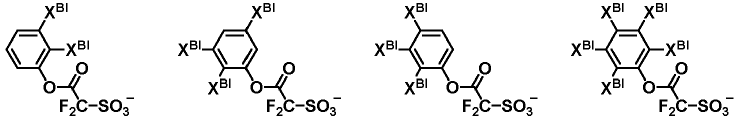

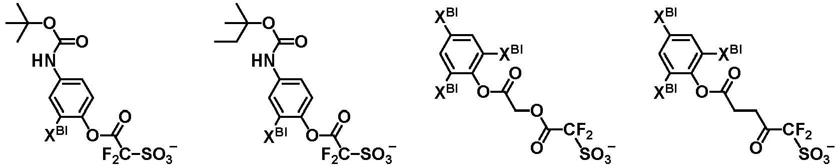

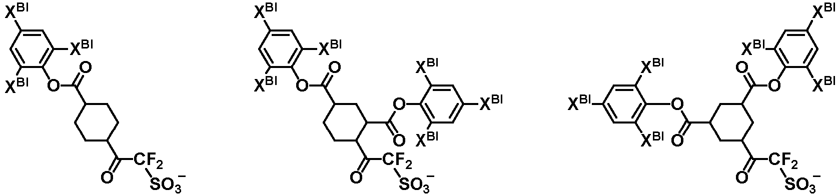

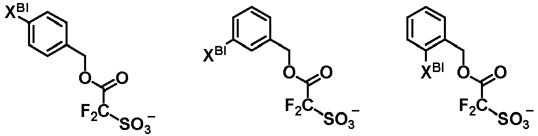

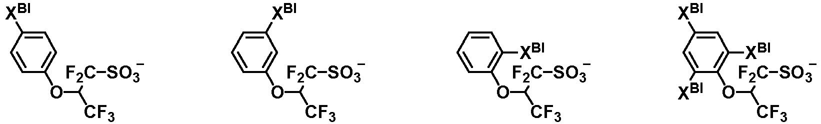

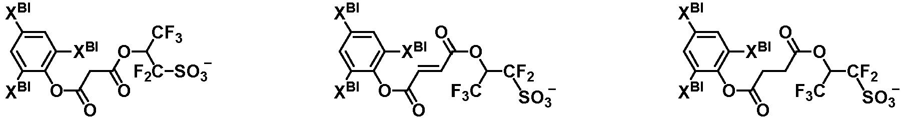

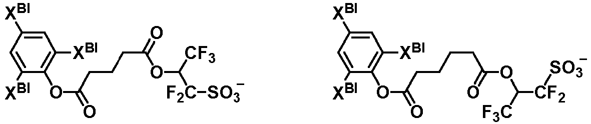

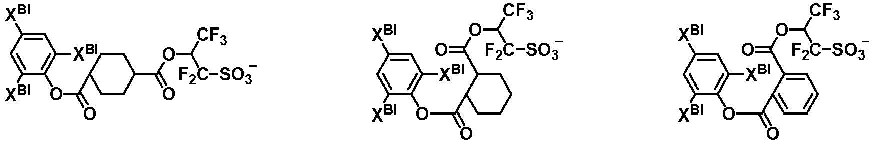

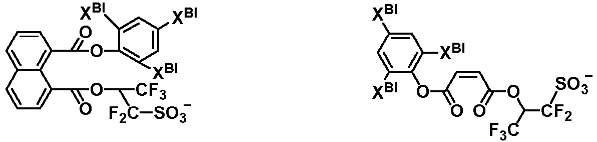

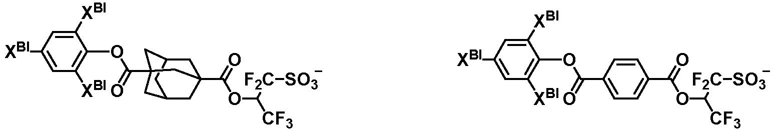

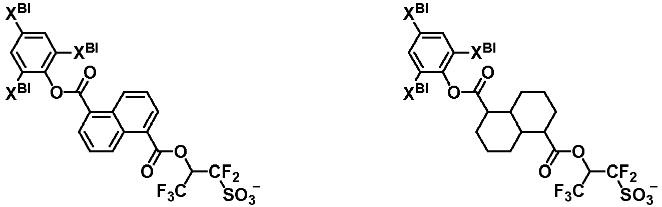

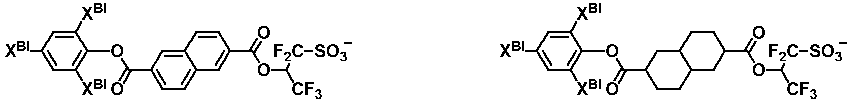

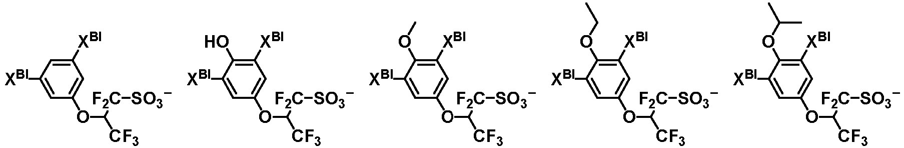

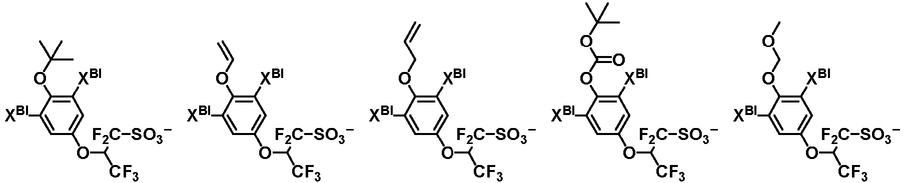

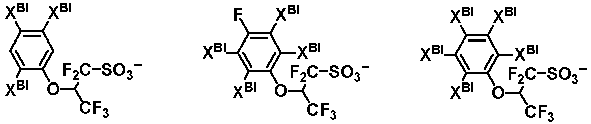

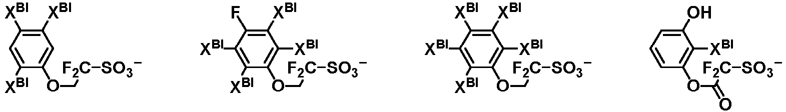

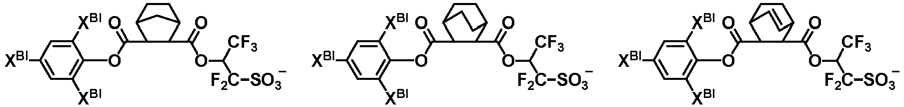

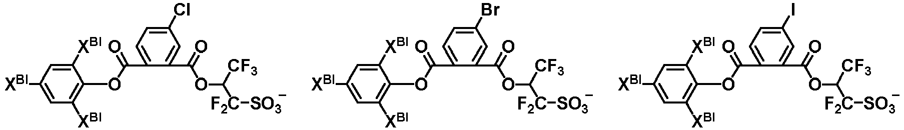

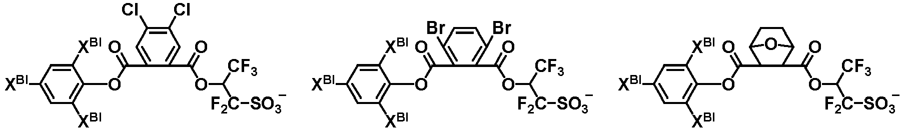

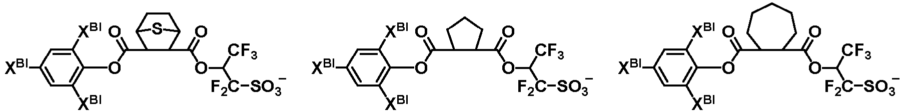

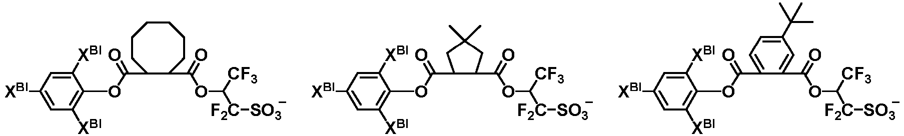

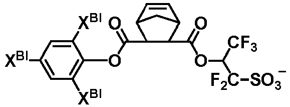

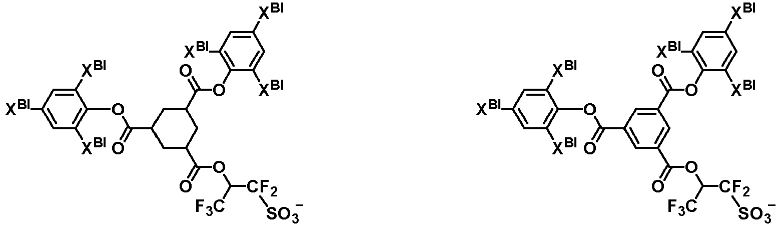

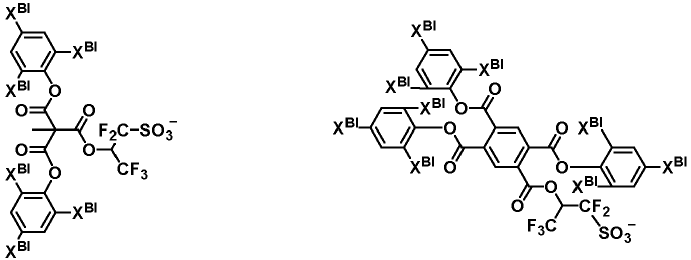

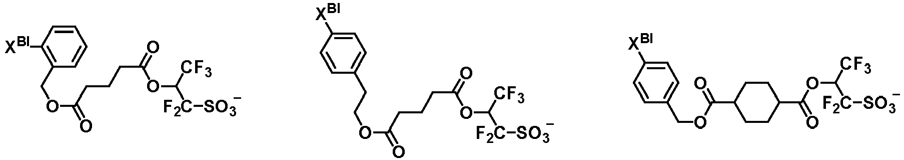

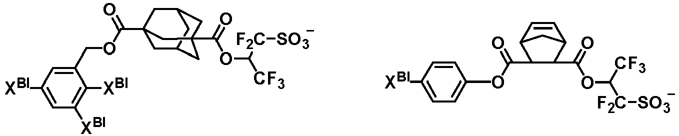

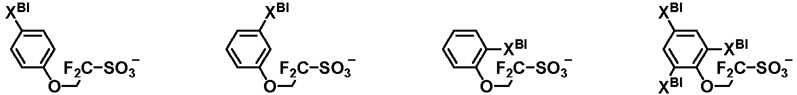

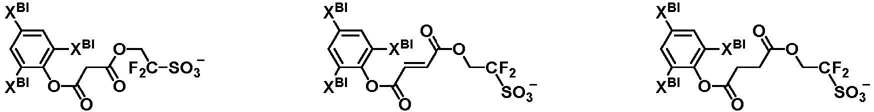

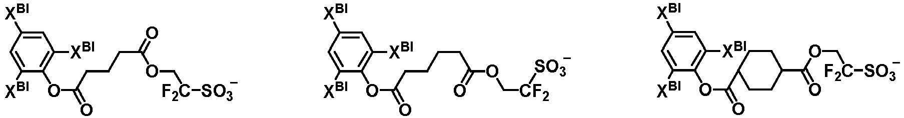

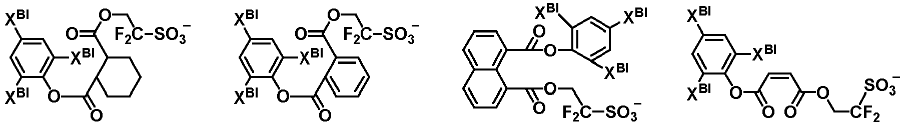

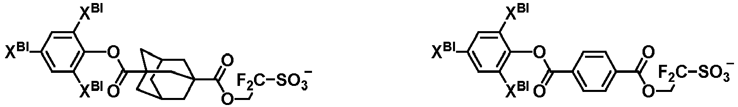

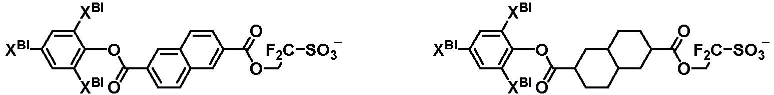

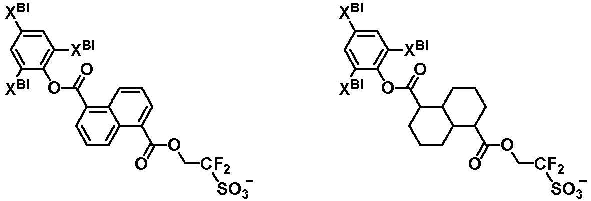

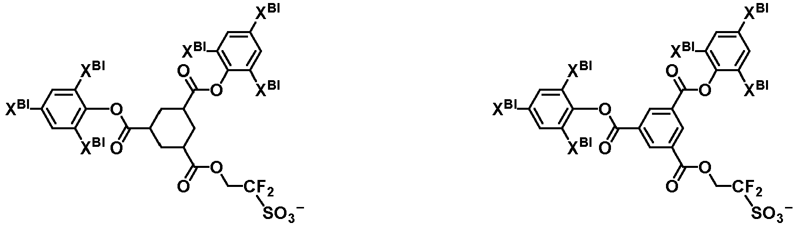

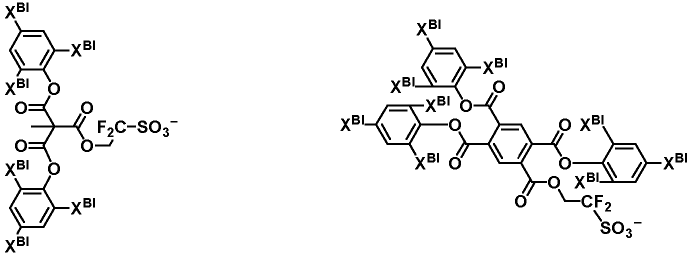

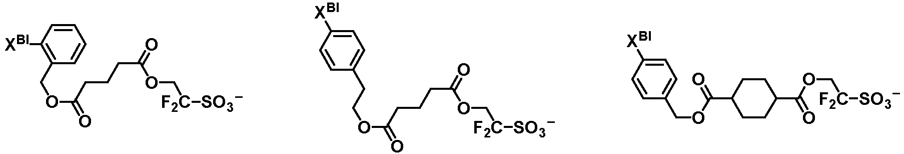

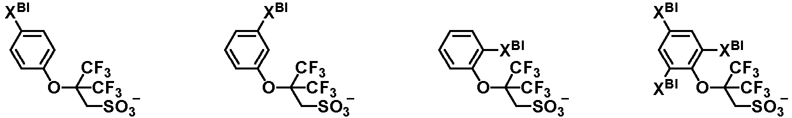

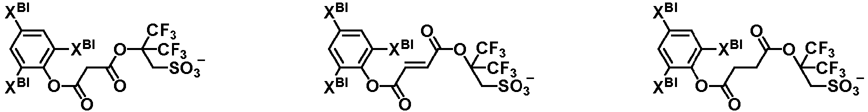

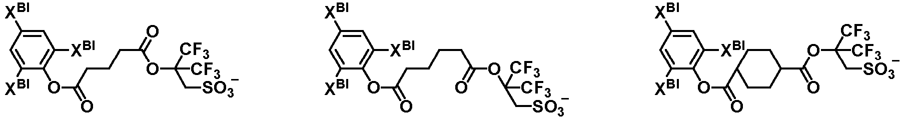

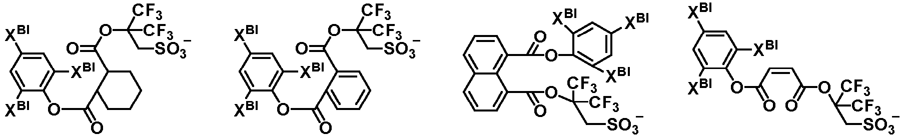

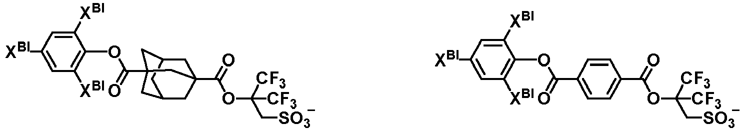

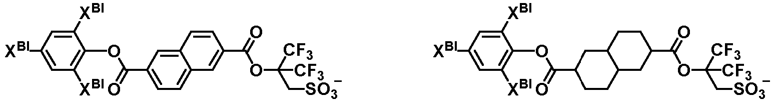

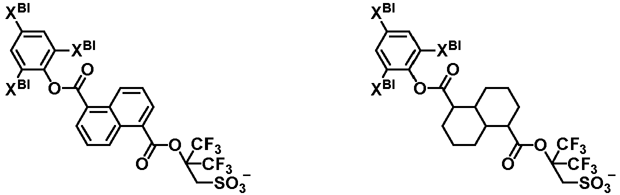

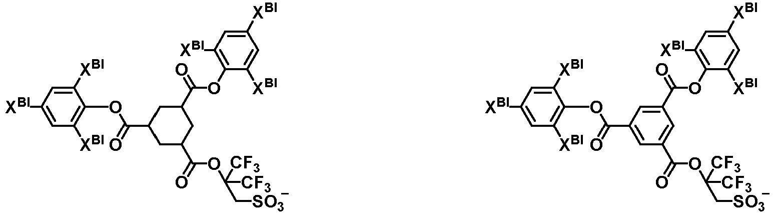

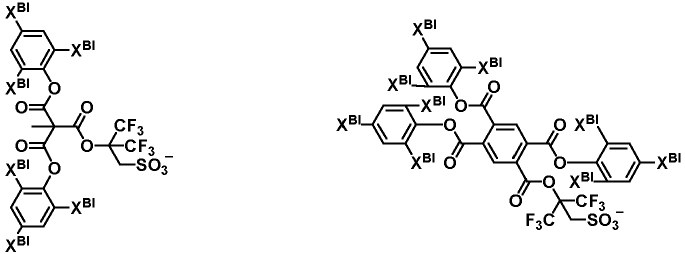

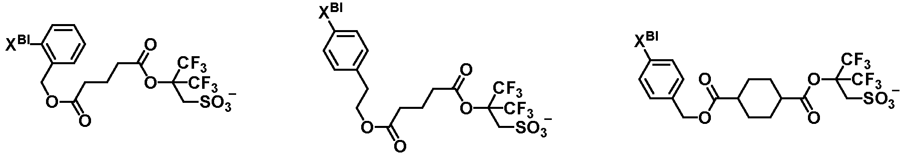

As the non-nucleophilic counter ion, fluorobenzenesulfonic acid anions having an iodized aromatic ring bonded thereto as described in JP 6648726, anions having an acid-catalyzed decomposition mechanism as described in WO 2021/200056 and JP-A 2021-70692, anions having a cyclic ether group as described in JP-A 2018-180525 and JP-A 2021-35935, and anions as described in JP A 2018-92159 may be used.

As the non-nucleophilic counter ion, fluorine-free bulky benzenesulfonic acid anions as described in JP-A 2006-276759, JP-A 2015-117200, JP-A 2016-65016, and JP-A 2019-202974; fluorine-free benzenesulfonic acid or alkylsulfonic acid anions having an iodized aromatic group bonded thereto as described in JP 6645464 may be used.

As the non-nucleophilic counter ion, bissulfonic acid anions as described in JP-A 2015-206932, sulfonamide or sulfonimide anions having sulfonic acid side and different side as described in WO 2020/158366, and anions having a sulfonic acid side and a carboxylic acid side as described in JP-A 2015-24989 may be used.

In formulae (B7) and (B8), g1 and g2 are each independently 0, 1, 2 or 3, preferably 1.

In formula (B9), h1 is 0 or 1. The subscript h2 is 0, 1, 2, 3 or 4. The h3 is 0, 1, 2, 3 or 4. The sum h2+h3 is from 0 to 4 when h1 is 0, and h2+h3 is from 0 to 6 when h1 is 1.

In formulae (B7), (B8) and (B9), L¹ is a single bond, ether bond, ester bond, carbonyl group, sulfonic ester bond, sulfonamide bond, carbonate bond, or carbamate bond. From the aspect of synthesis, an ether bond, ester bond or carbonyl group is preferred, with the ester bond or carbonyl being more preferred.

In formula (B7), Rf¹ and Rf² are each independently fluorine or a C₁-C₆ fluorinated saturated hydrocarbyl group. It is preferred that both Rf¹ and Rf² be fluorine because the generated acid has a higher acid strength. Rf³ and Rf⁴ are each independently hydrogen, fluorine, or a C₁-C₆ fluorinated saturated hydrocarbyl group. It is preferred for enhancing the solvent solubility that at least one of Rf³ and Rf⁴ be trifluoromethyl.

In formula (B8), Rf⁵ and Rf⁶ are each independently hydrogen, fluorine or a C₁-C₆ fluorinated saturated hydrocarbyl group. It is excluded that all Rf⁵ and Rf⁶ are hydrogen at the same time. It is preferred for enhancing the solvent solubility that at least one of Rf⁵ and Rf⁶ be trifluoromethyl.

In formula (B9), Rf⁷ is fluorine, a C₁-C₆ fluorinated alkyl group, C₁-C₆ fluorinated alkoxy group or C₁-C₆ fluorinated alkylthio group. Rf⁷ is preferably fluorine, a trifluoromethyl, difluoromethyl, trifluoromethoxy, difluoromethoxy, trifluoromethylthio or difluoromethylthio, more preferably fluorine, a trifluoromethyl or trifluoromethoxy group. A plurality of Rf⁷ may be identical or different when h2 is 2, 3 or 4.

In formula (B9), R⁴³ is halogen exclusive of iodine, or a C₁-C₂₀ hydrocarbyl group which may contain a heteroatom. The hydrocarbyl group may be saturated or unsaturated, and may be straight, branched, or cyclic. Examples thereof are as exemplified for the hydrocarbyl group R¹ in formula (A), but not limited thereto. A plurality of R⁴³ may be identical or different when h3 is 2, 3 or 4.

A plurality of R⁴³ may bond together to form a ring with the carbon atoms to which they are attached when h3 is 2, 3 or 4. Examples of the ring formed herein include cyclopropane, cyclobutane, cyclopentane, cyclohexane, norbornane and adamantane rings. Some or all of hydrogen atoms in the ring may be replaced by a group containing a heteroatom such as oxygen, sulfur, nitrogen or halogen, some constituent -CH₂- in the ring may be replaced by a group containing a heteroatom such as oxygen, sulfur or nitrogen, and as a result, the hydrocarbyl group may contain a hydroxy group, fluorine, chlorine, bromine, iodine, a cyano group, a carbonyl group, an ether bond, an ester bond, a sulfonic ester bond, a carbonate bond, a lactone ring, a sultone ring, carboxylic anhydride (-C(=O)-O-C(=O)-), a haloalkyl group, or the like.

Examples of the anion in repeat unit B7 are shown below, but not limited thereto. In the following formulae, R^{A} is as defined above, and Me is a methyl group.

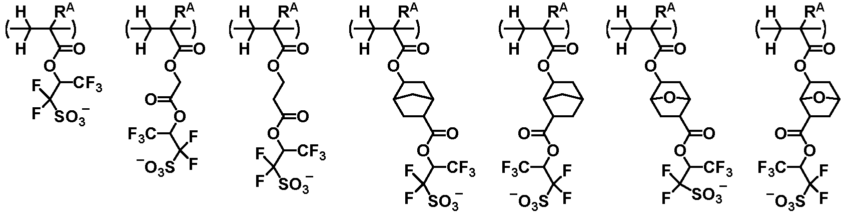

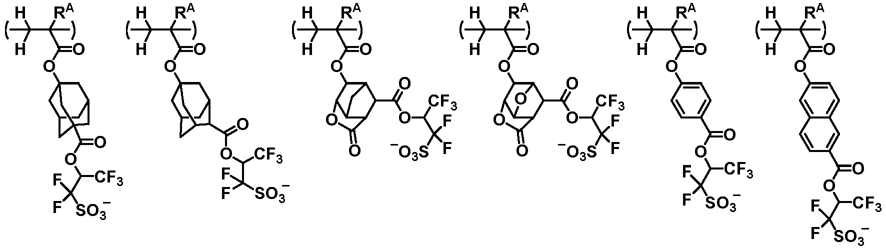

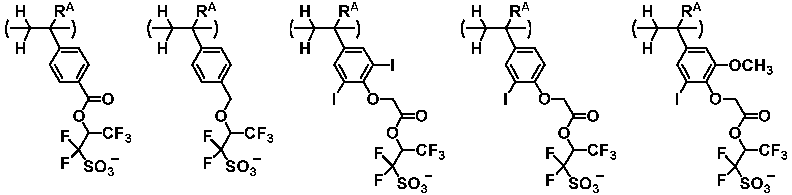

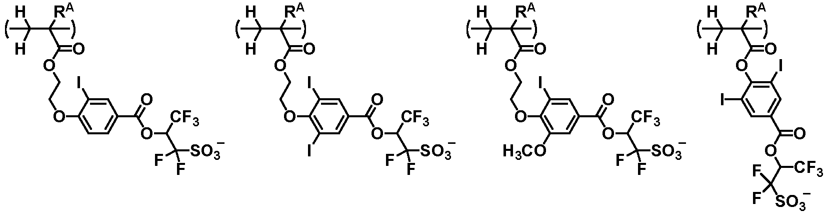

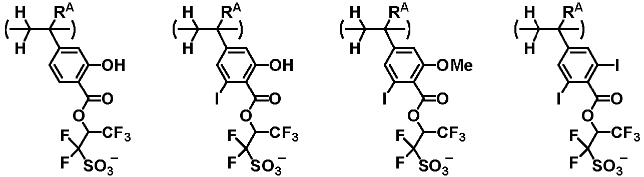

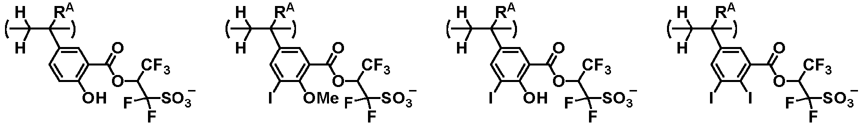

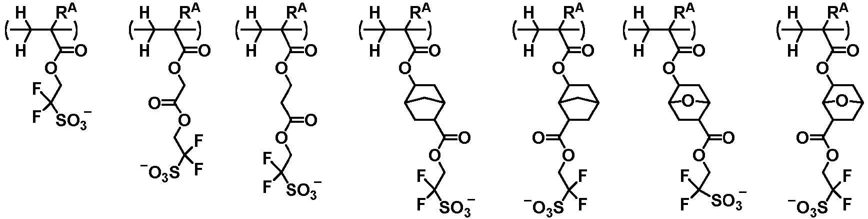

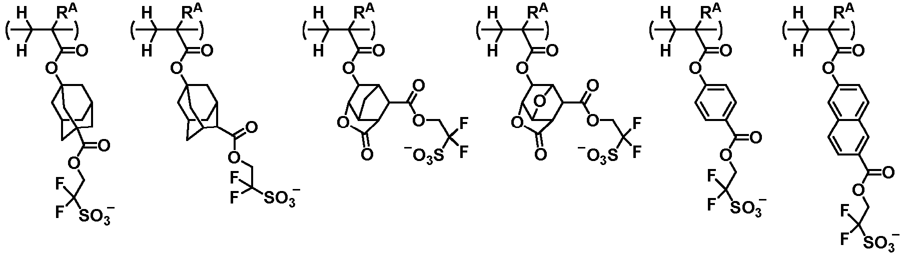

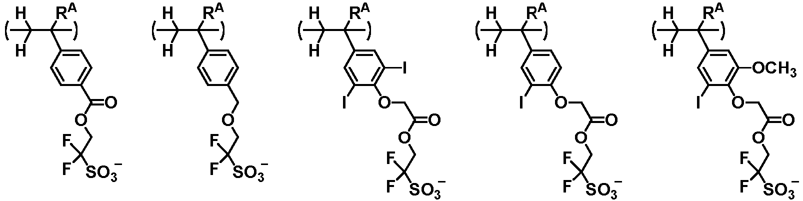

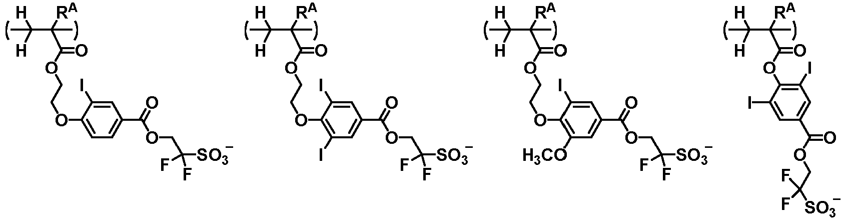

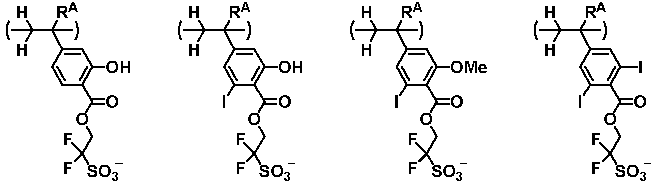

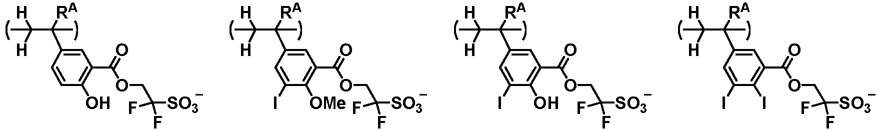

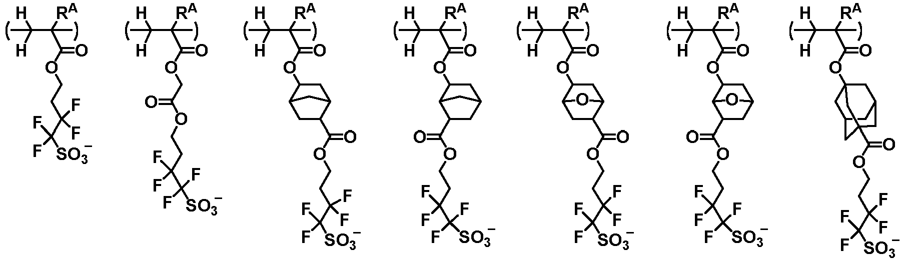

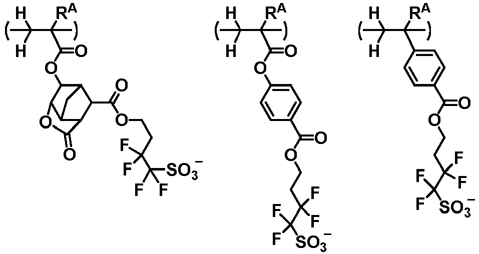

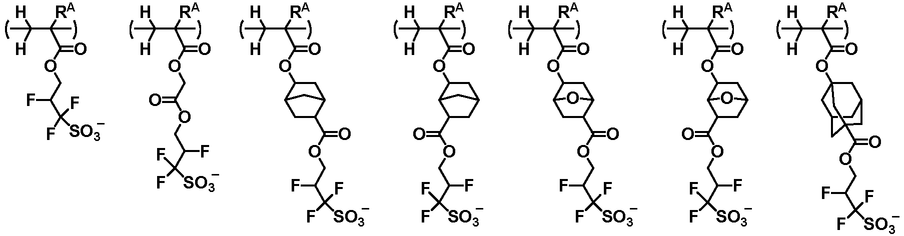

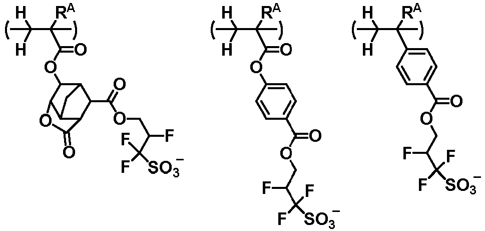

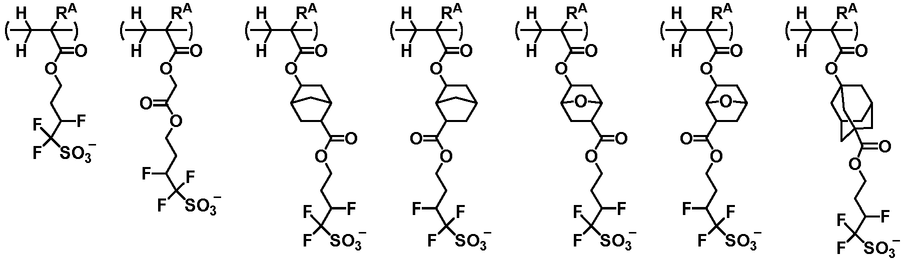

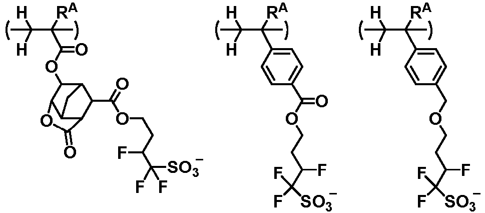

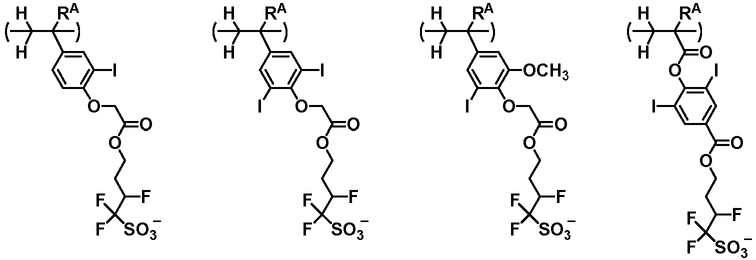

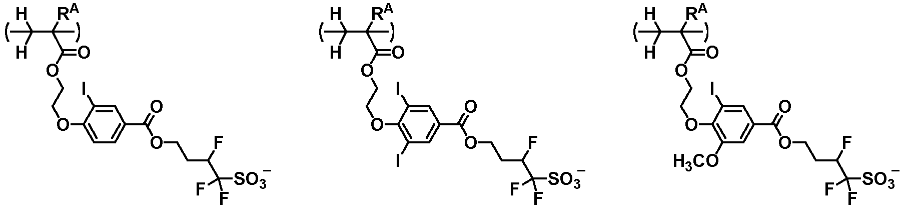

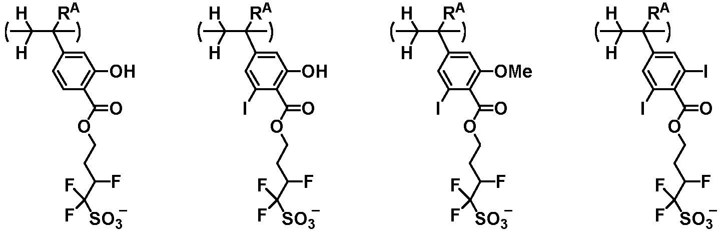

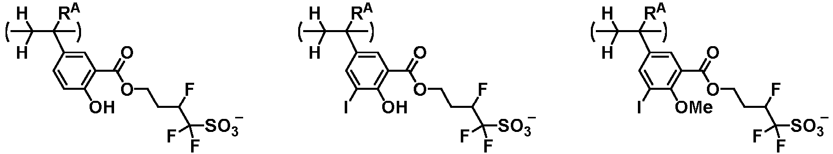

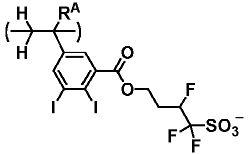

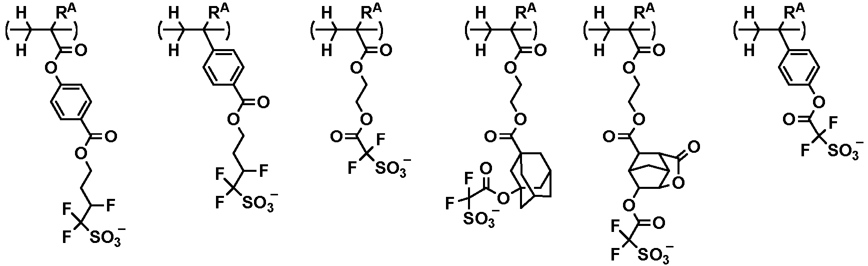

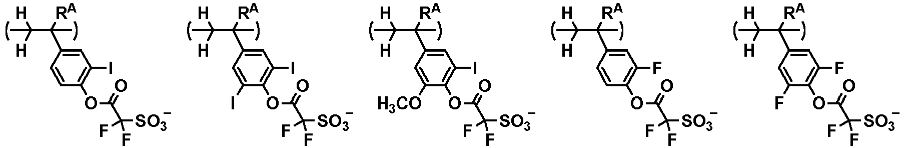

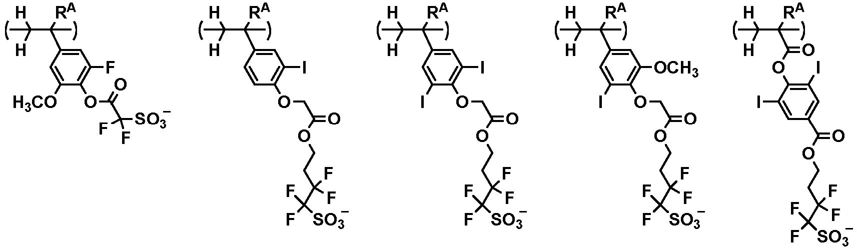

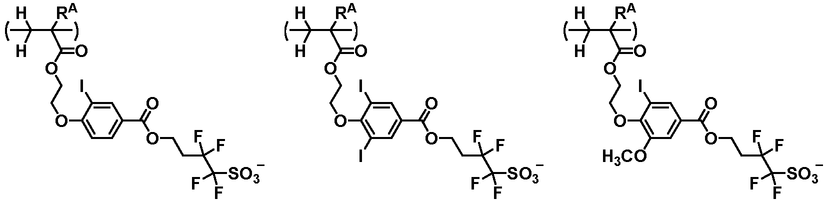

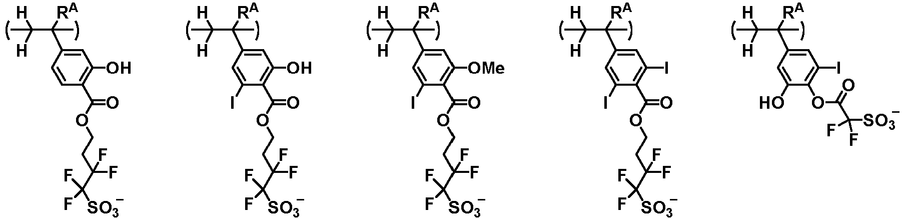

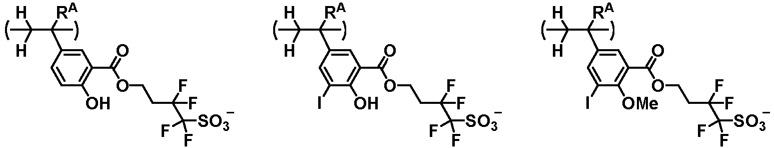

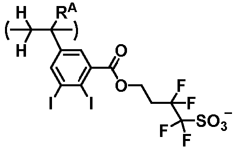

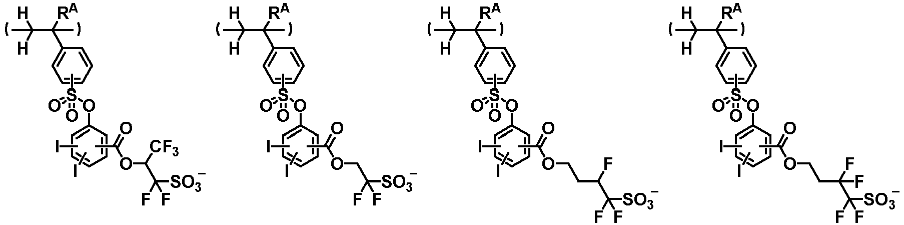

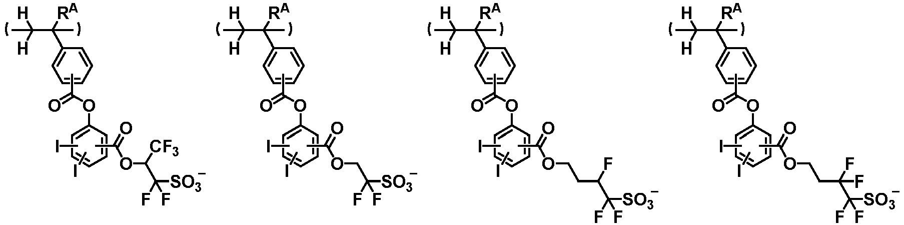

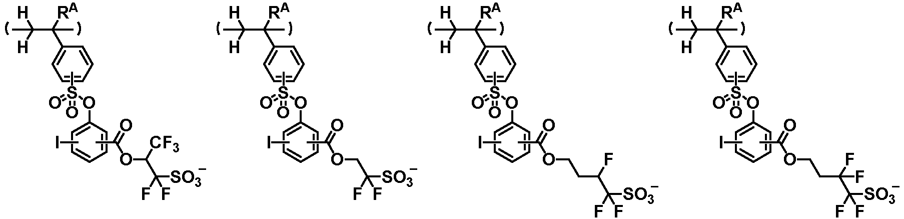

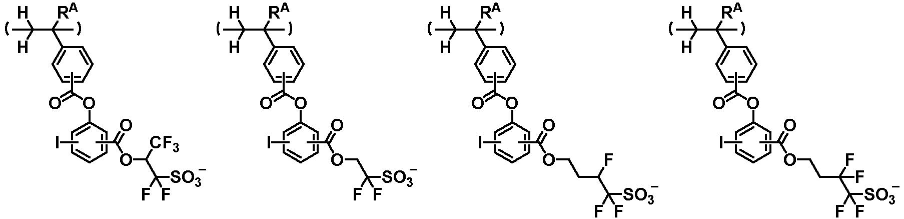

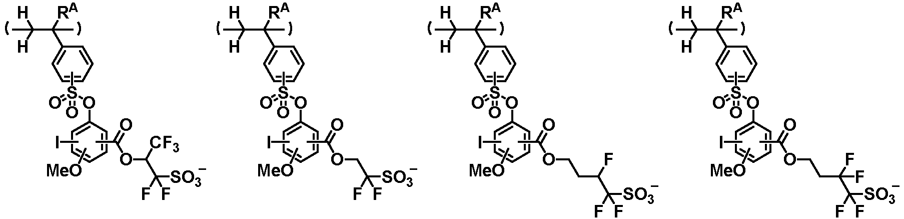

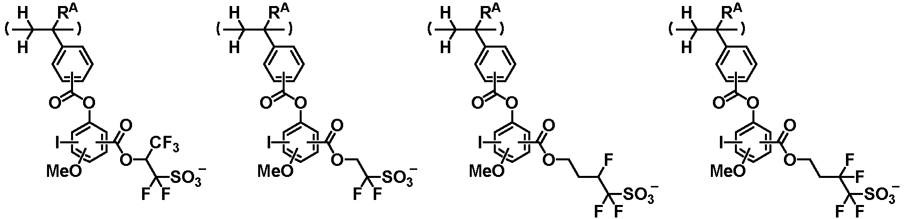

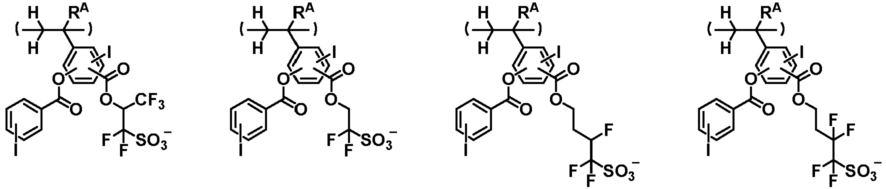

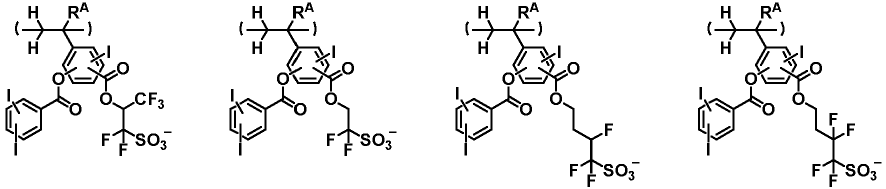

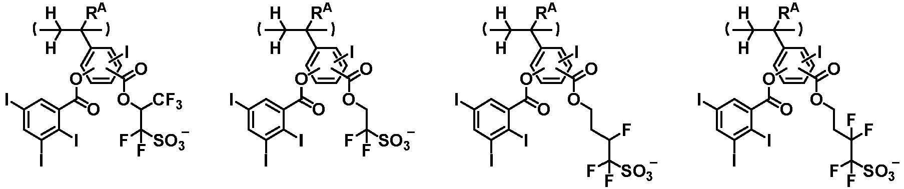

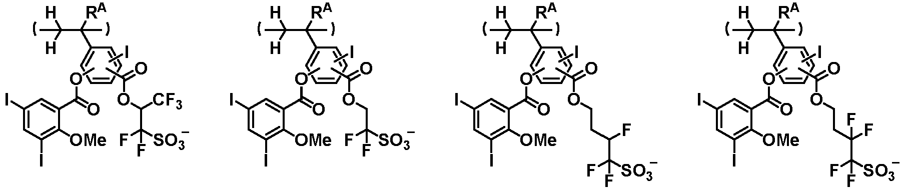

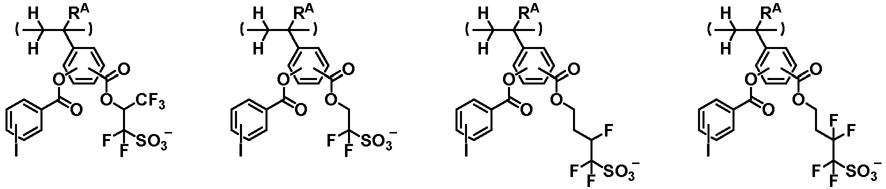

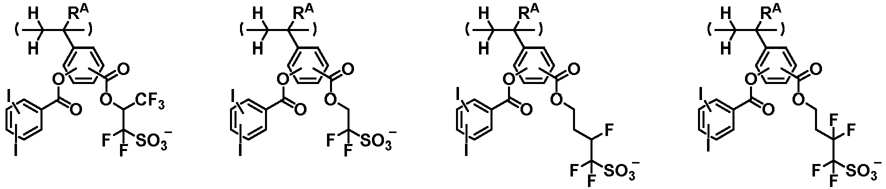

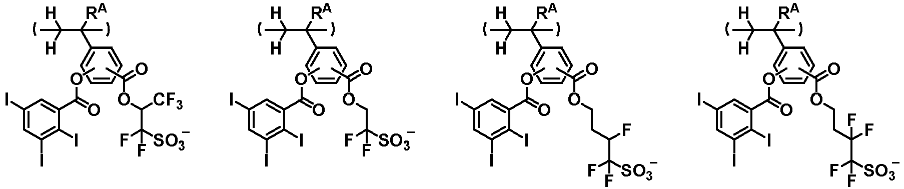

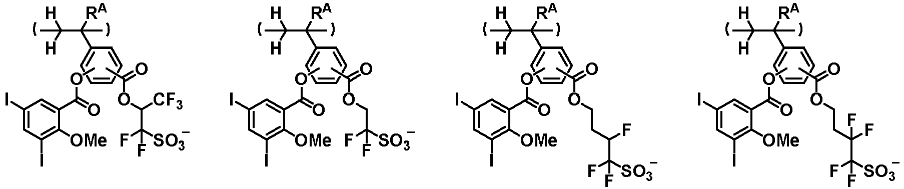

Examples of the anion in repeat unit B8 are shown below, but not limited thereto. R^{A} is as defined above.

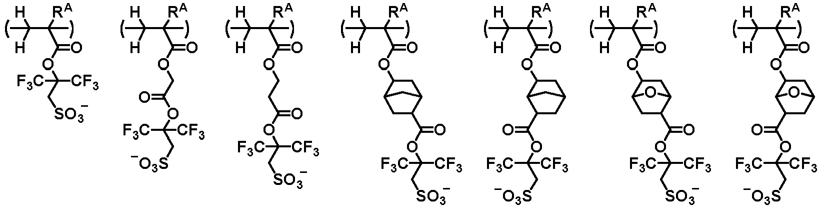

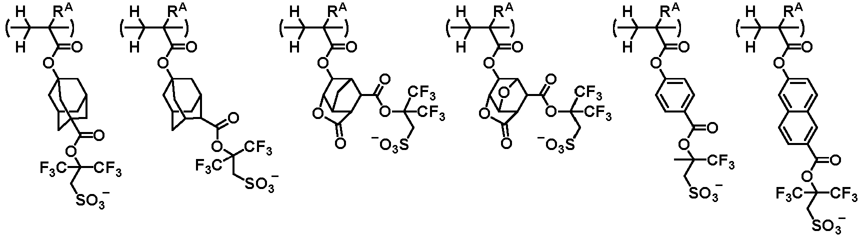

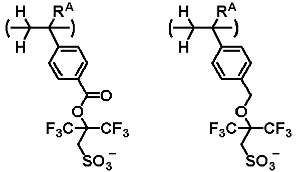

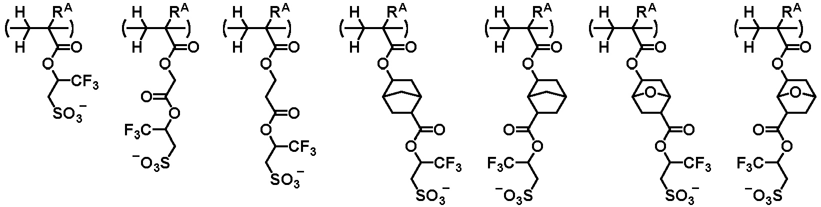

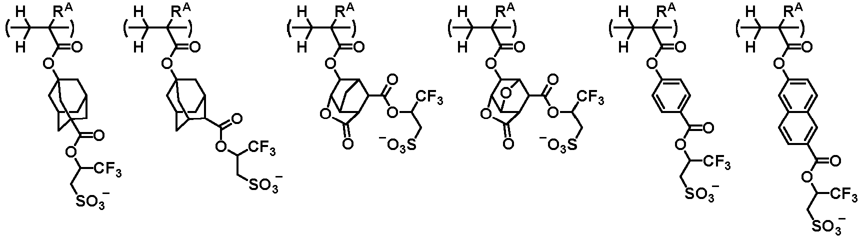

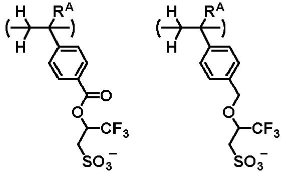

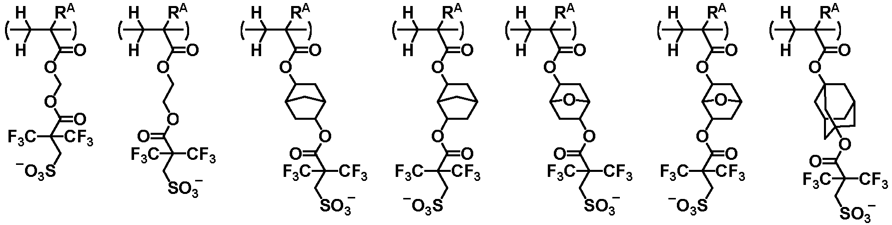

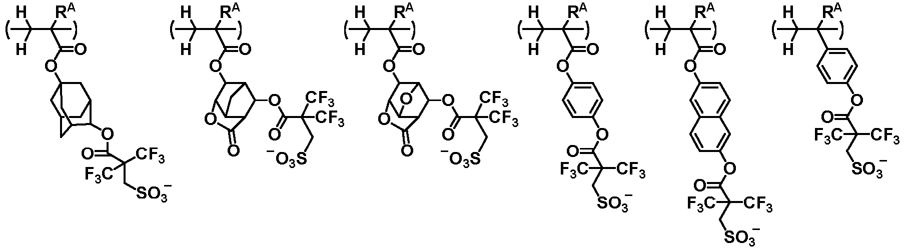

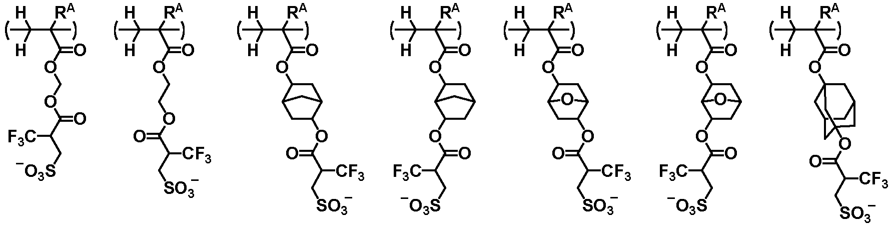

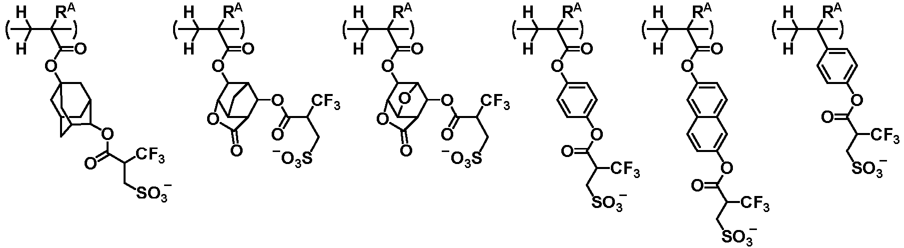

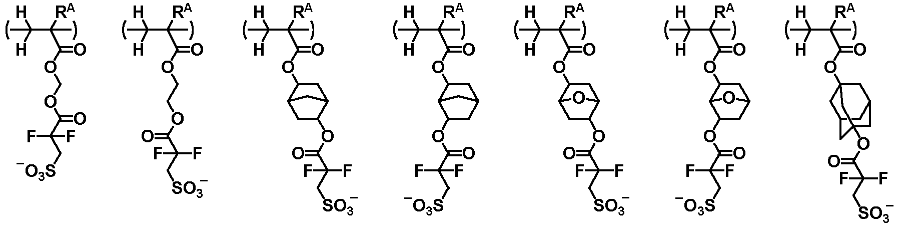

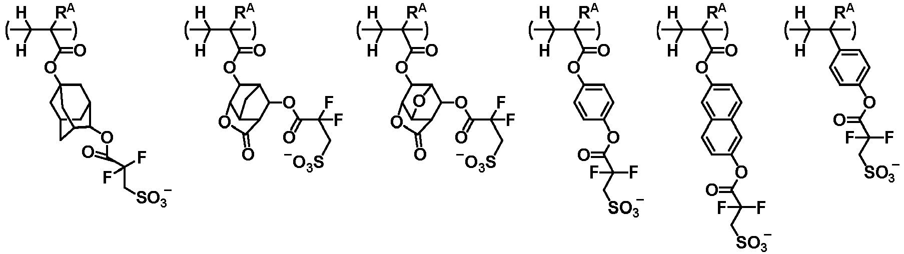

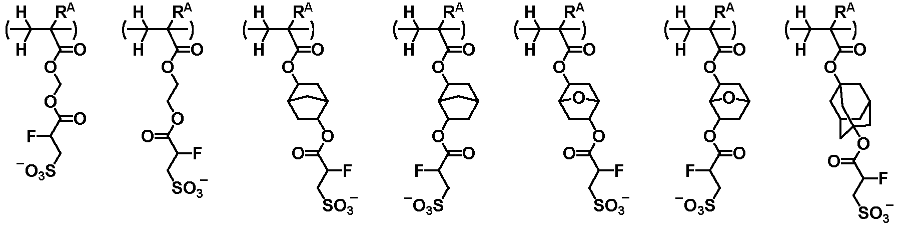

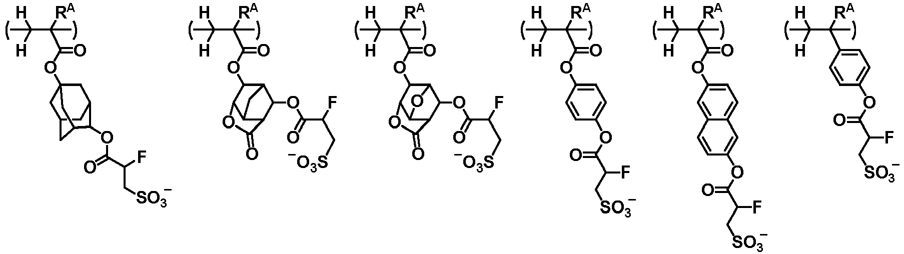

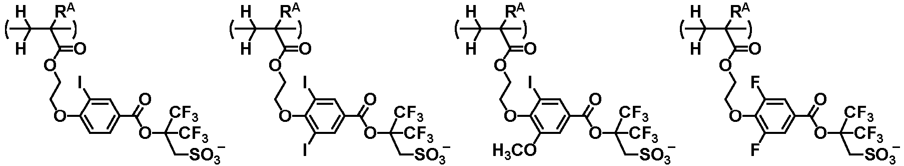

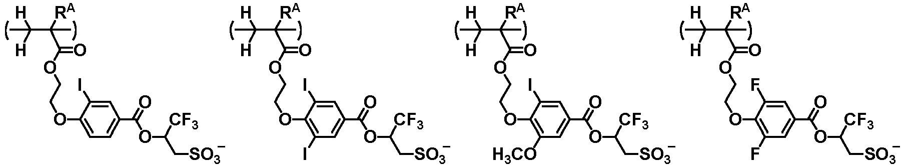

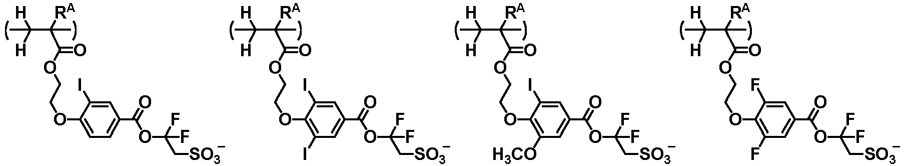

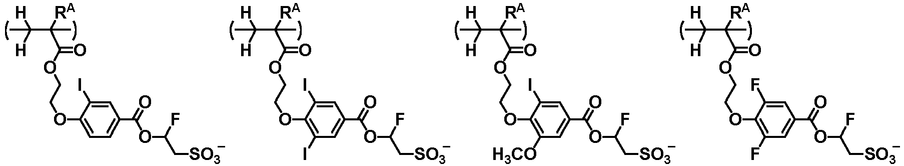

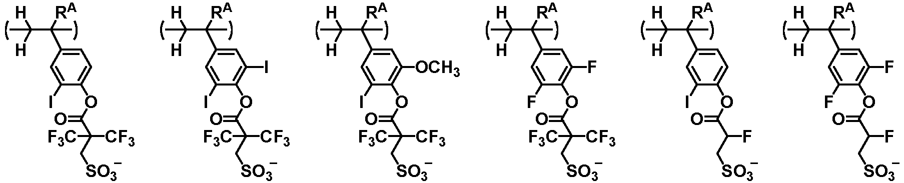

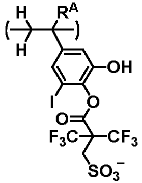

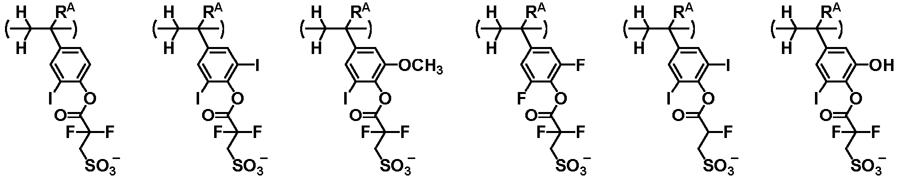

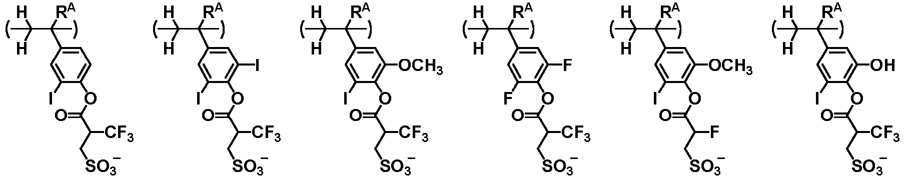

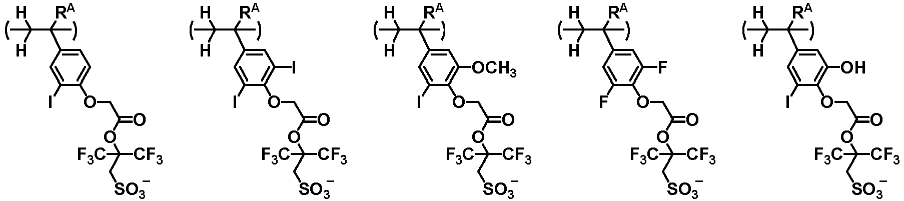

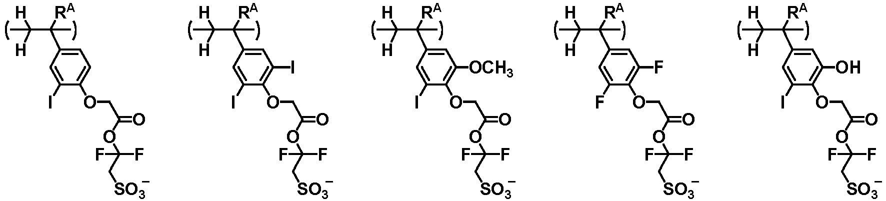

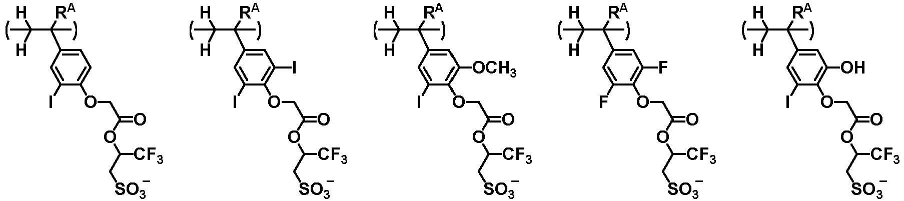

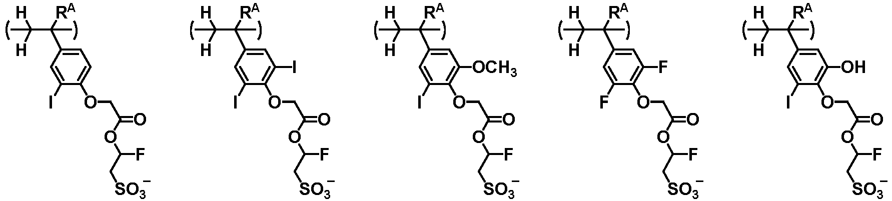

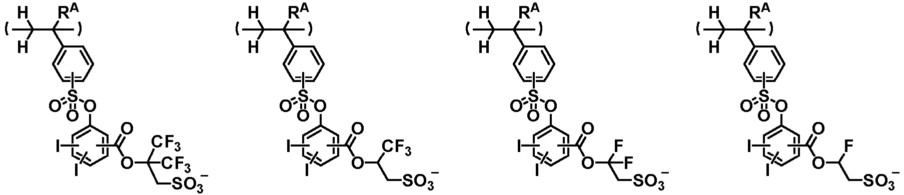

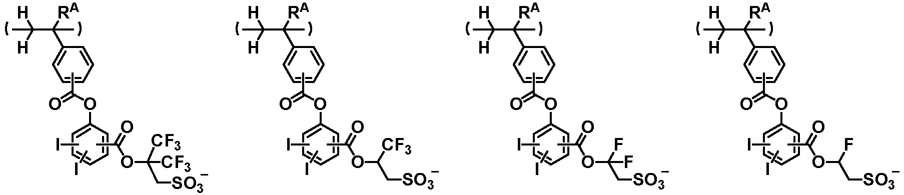

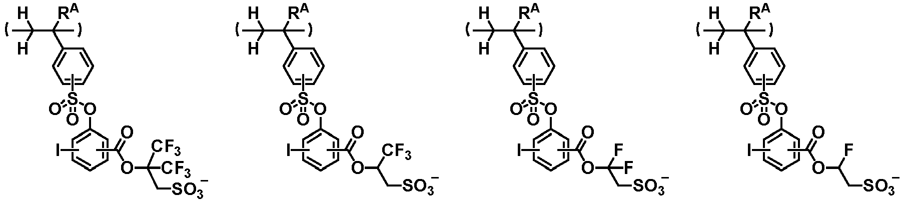

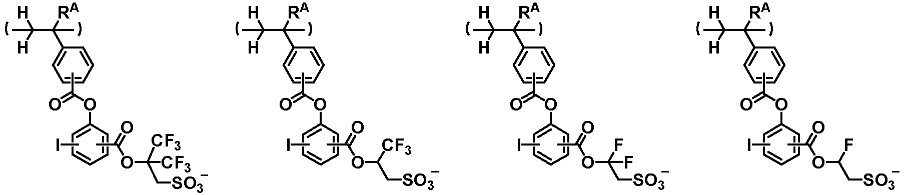

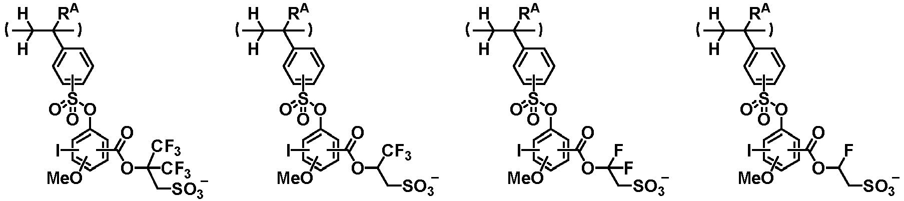

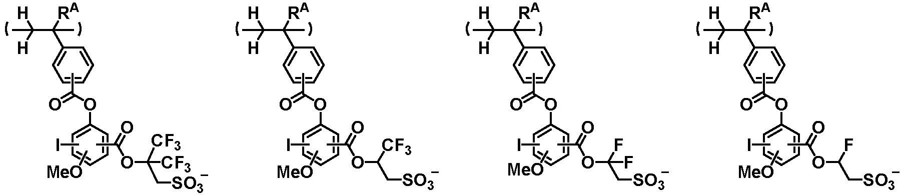

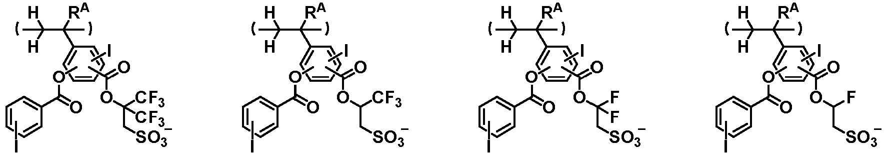

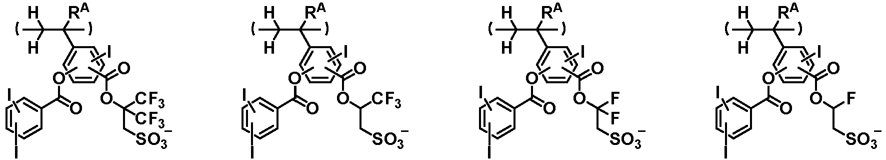

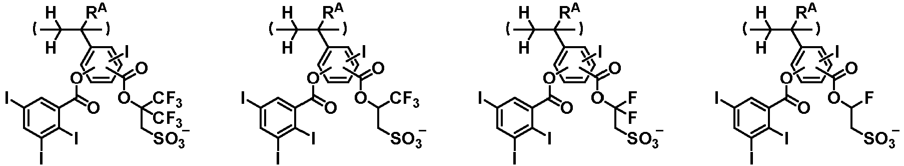

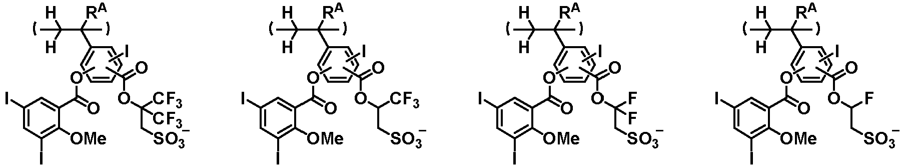

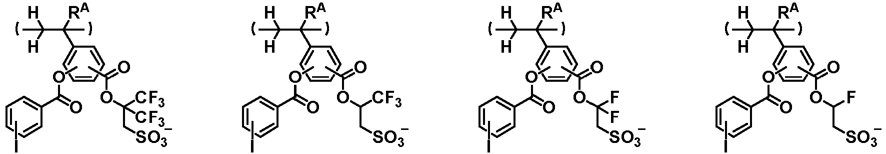

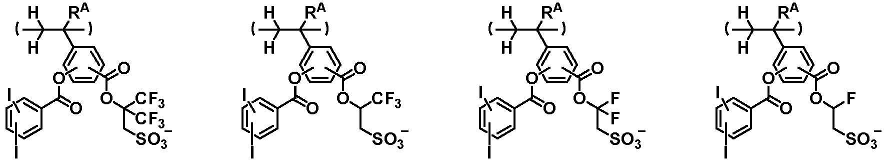

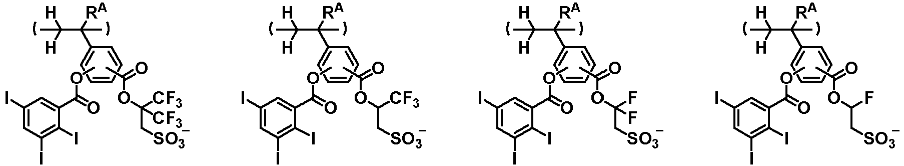

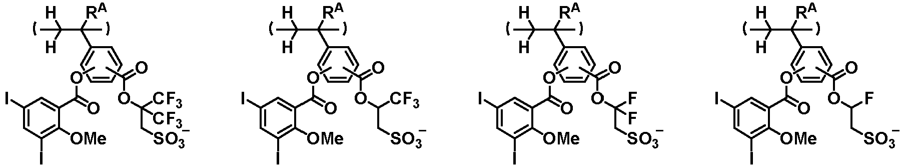

Examples of the anion in repeat unit B9 are shown below, but not limited thereto. R^{A} is as defined above.

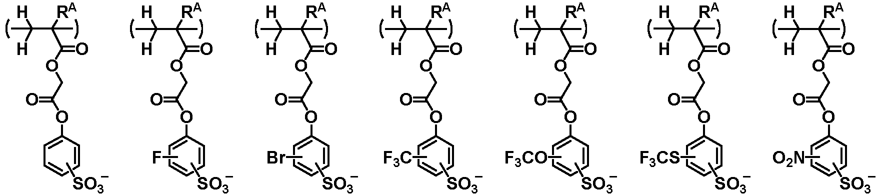

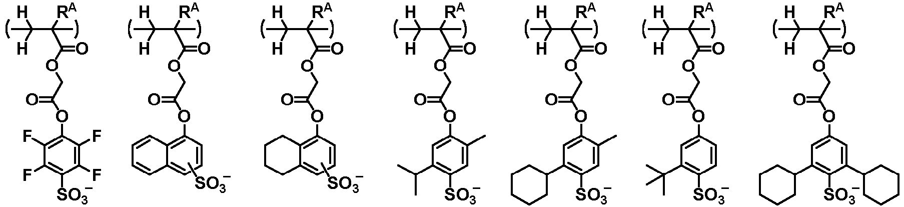

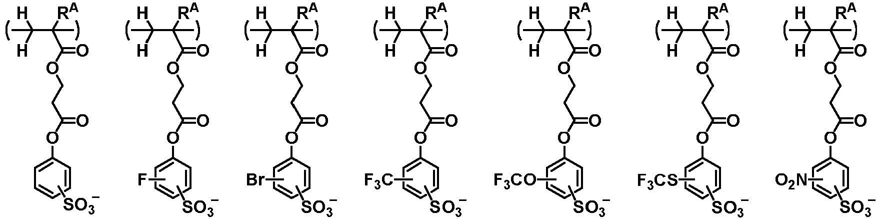

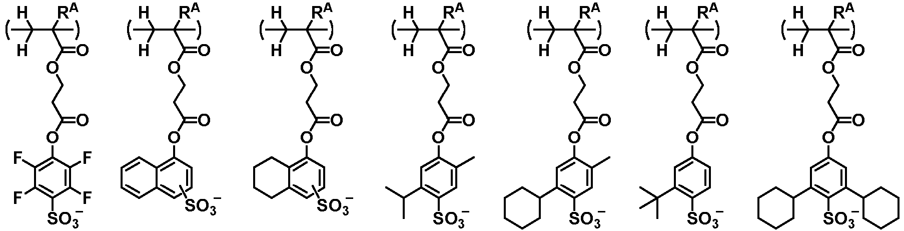

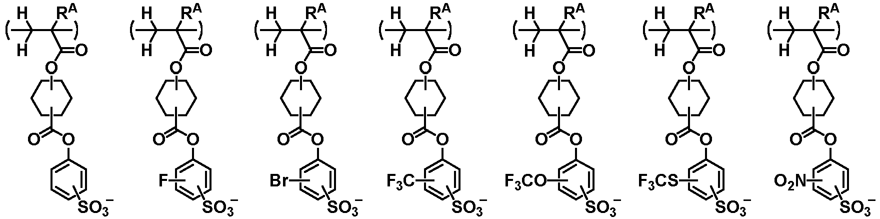

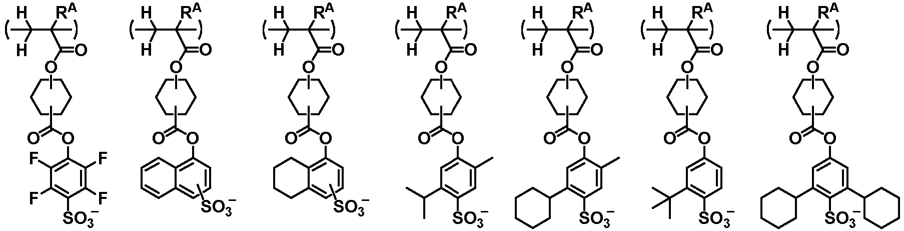

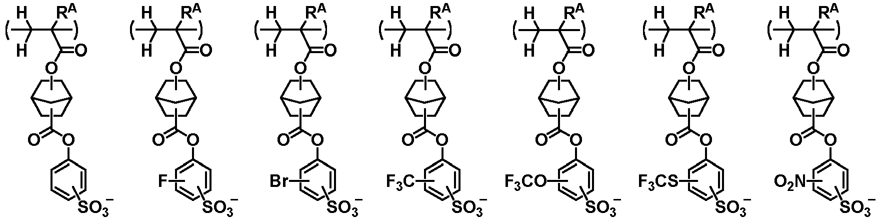

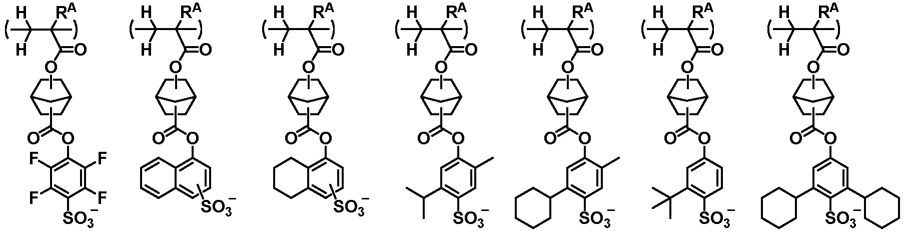

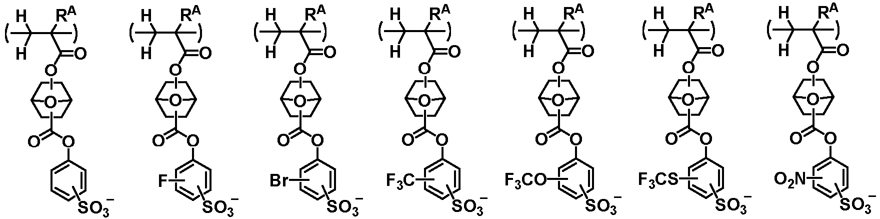

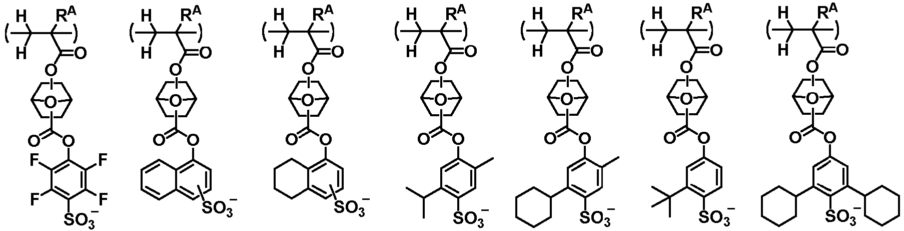

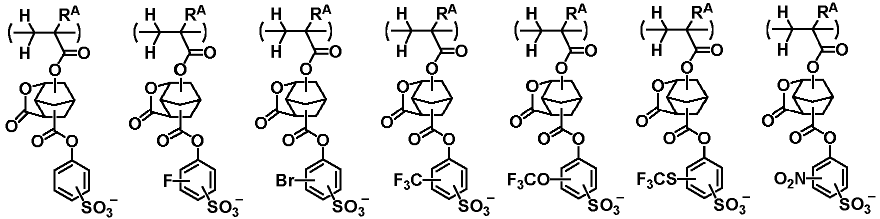

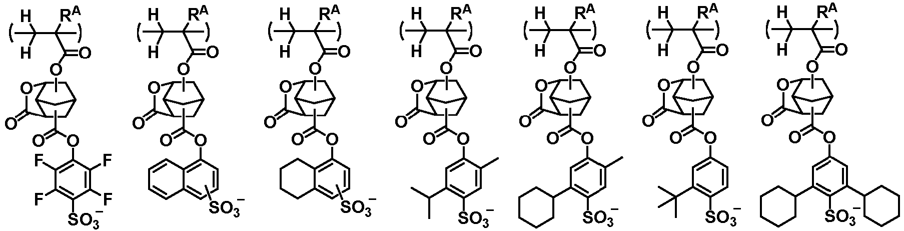

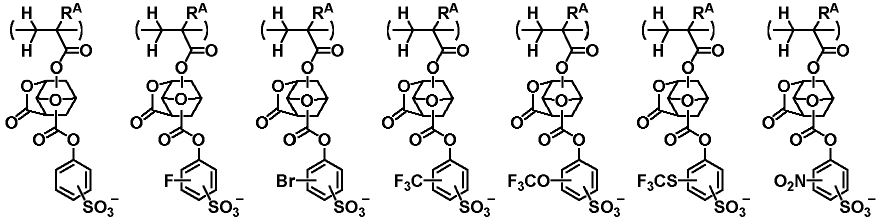

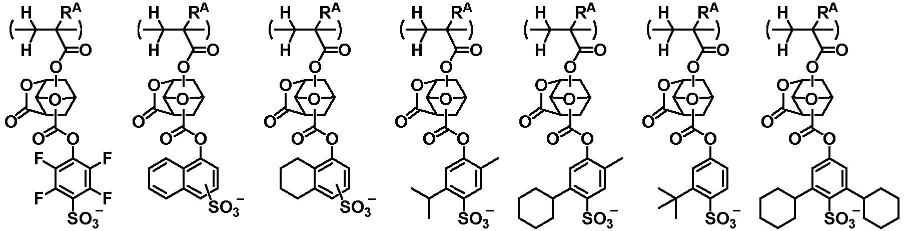

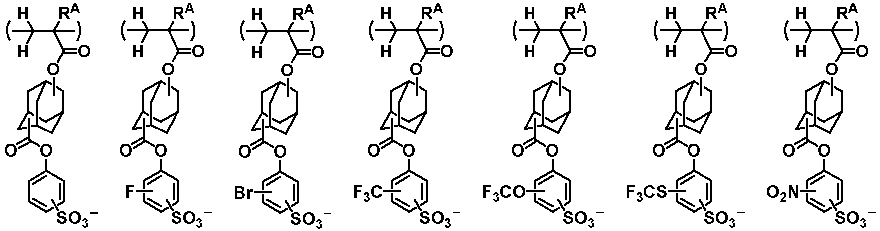

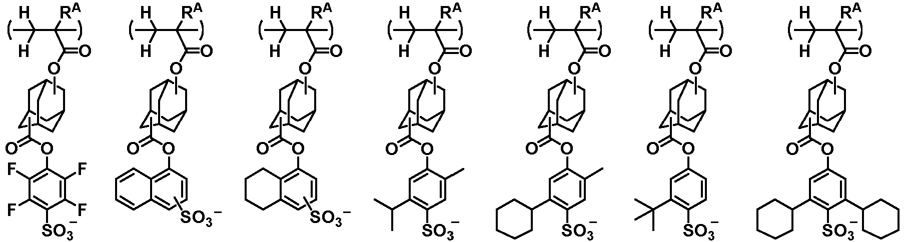

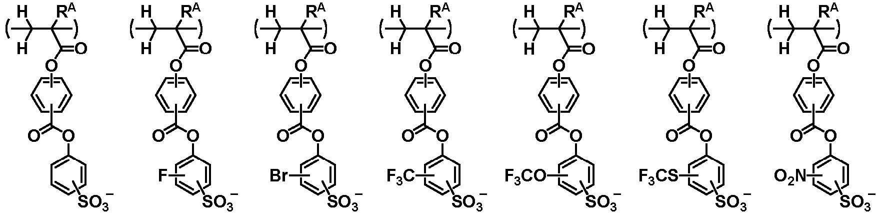

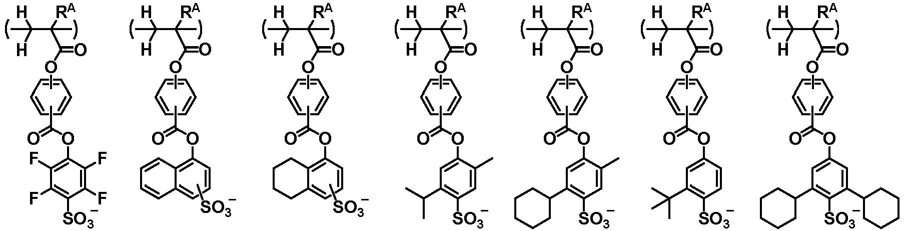

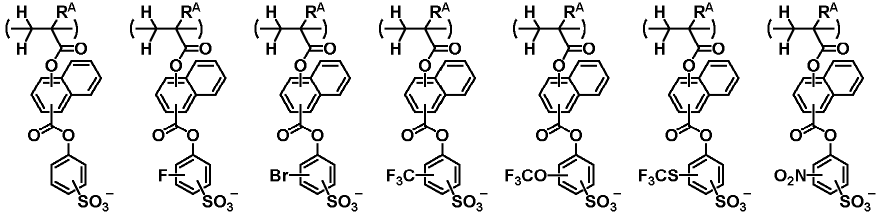

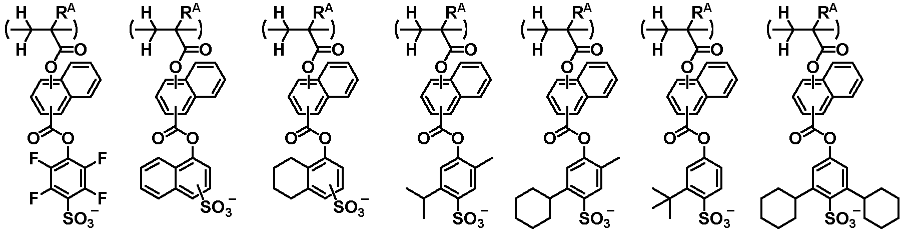

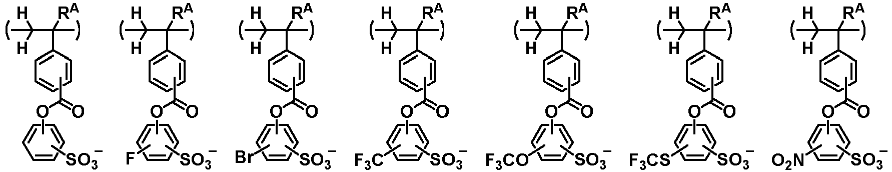

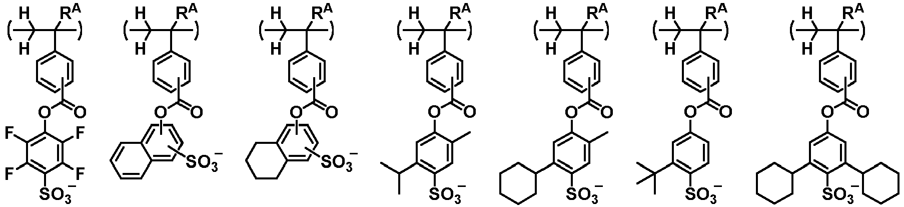

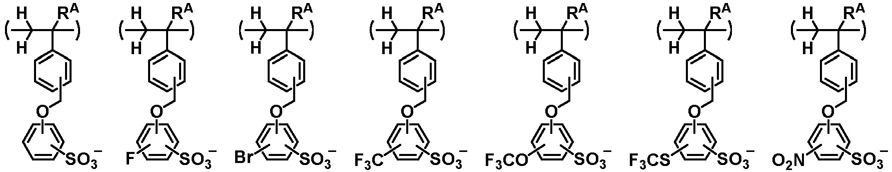

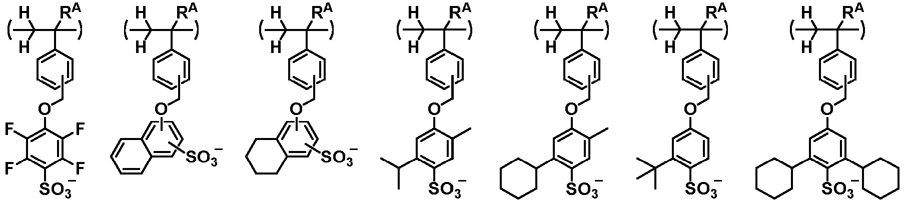

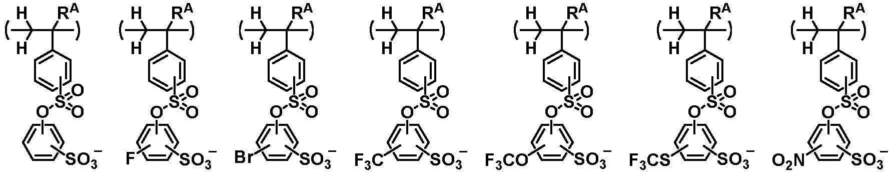

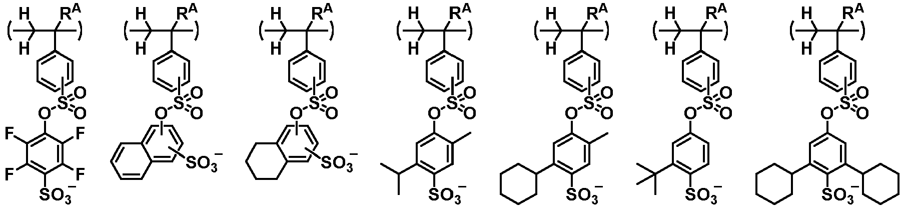

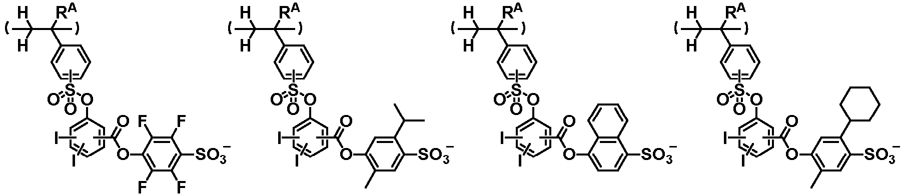

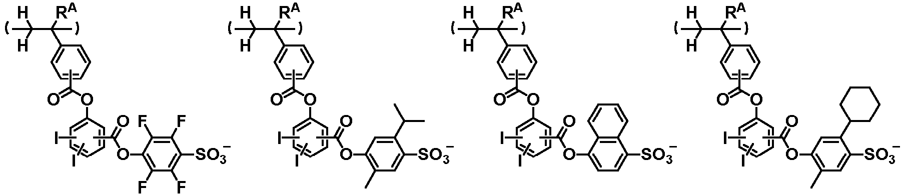

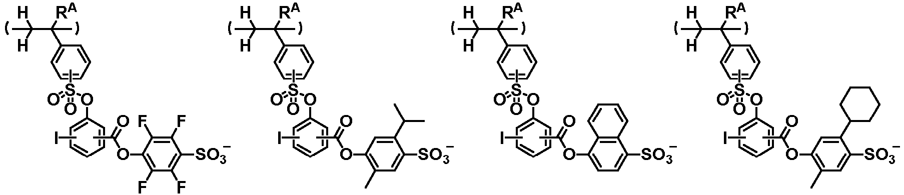

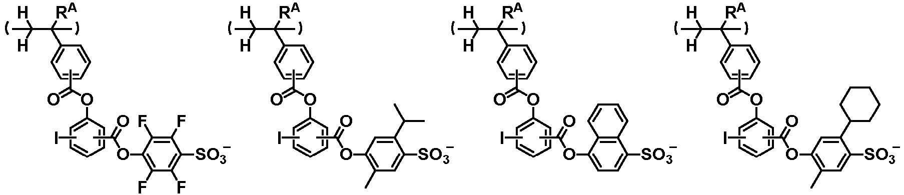

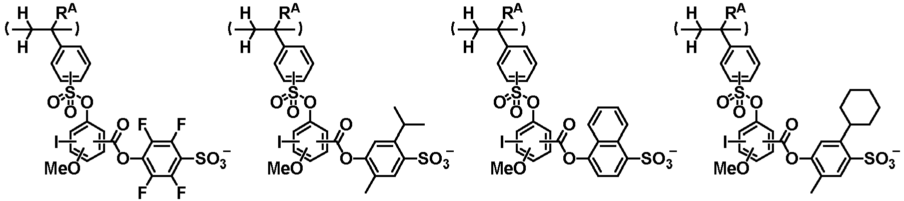

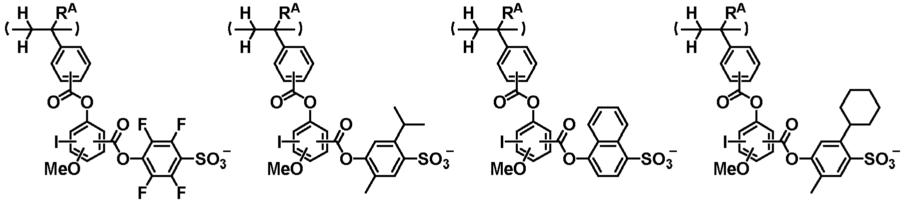

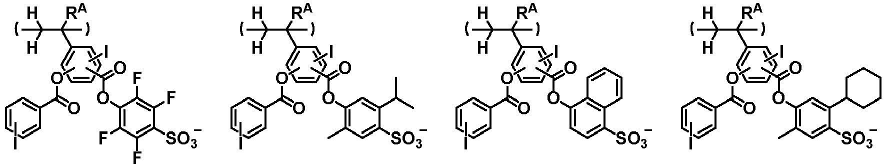

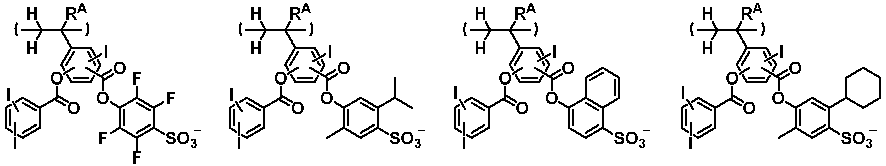

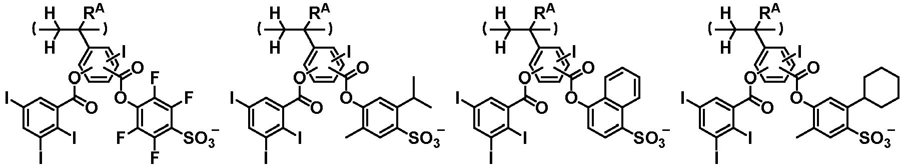

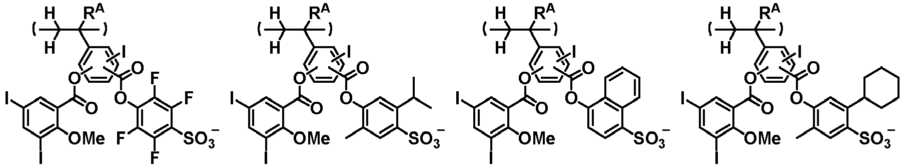

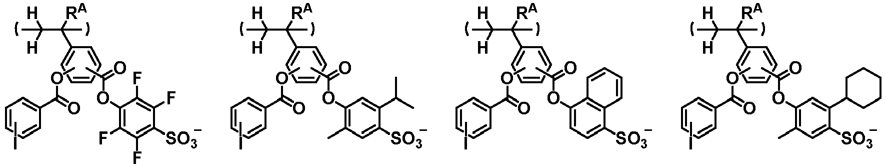

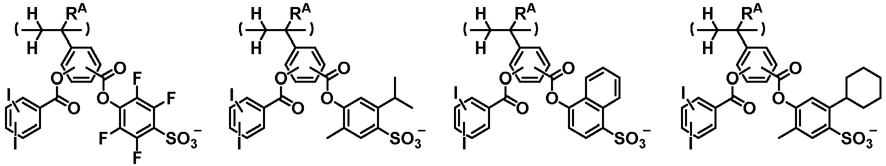

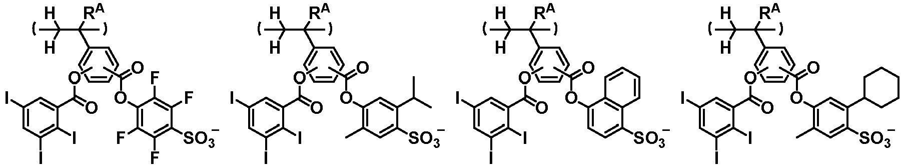

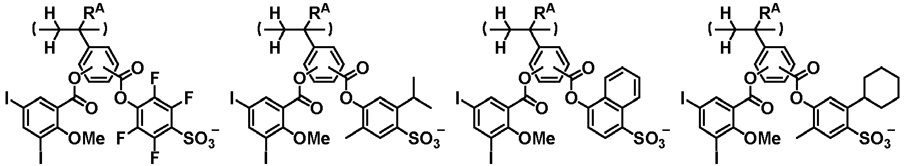

Examples of the anion in repeat unit B10 are shown below, but not limited thereto. R^{A} is as defined above.

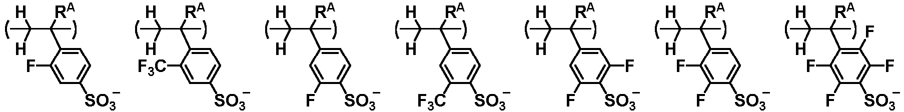

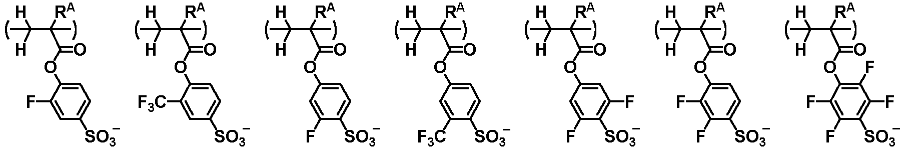

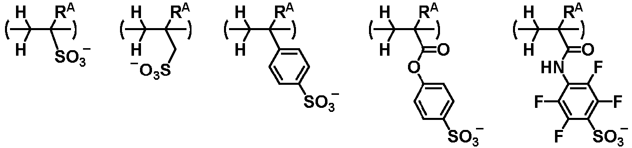

In formulae (B7) to (B10), A⁺ is an onium cation. The onium cation is preferably a sulfonium cation having the formula (Z-1) or iodonium cation having the formula (Z-2).

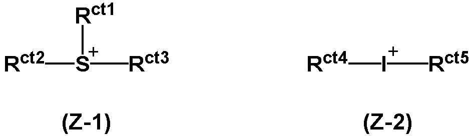

In formulae (Z-1) and (Z-2), R^{ct1} to R^{ct5} are each independently halogen, or a C₁-C₃₀ hydrocarbyl group which may contain a heteroatom.

Specific examples of the halogen atom represented by R^{ct1} to R^{ct5} include a fluorine atom, a chlorine atom, a bromine atom, and an iodine atom.

The hydrocarbyl group represented by R^{ct1} to R^{ct5} may be saturated or unsaturated, and may be straight, branched, or cyclic. Examples thereof include C₁-C₃₀ alkyl groups such as methyl, ethyl, n-propyl, isopropyl, n-butyl, sec-butyl and tert-butyl groups; C₃-C₃₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl groups; C₂-C₃₀ alkenyl groups such as vinyl, 1-propenyl, 2-propenyl, butenyl and hexenyl groups; C₃-C₃₀ cyclic unsaturated hydrocarbyl groups such as a cyclohexenyl group; C₆-C₃₀ aryl groups such as phenyl, naphthyl and thienyl groups; C₇-C₃₀ aralkyl groups such as benzyl, 1-phenylethyl and 2-phenylethyl groups; and combinations thereof. The aryl groups are preferred. In the hydrocarbyl group, some or all hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain hydroxy, fluorine, chlorine, bromine, iodine, cyano, nitro, carbonyl, ether bond, ester bond, sulfonic ester bond, carbonate bond, lactone ring, sultone ring, carboxylic anhydride (-C(=O)-O-C(=O)-), or haloalkyl moiety.

R^{ct1} and R^{ct2} may bond together to form a ring with the sulfur atom to which they are attached. Exemplary structures of the ring are shown below.

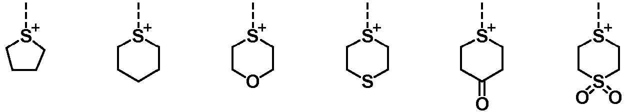

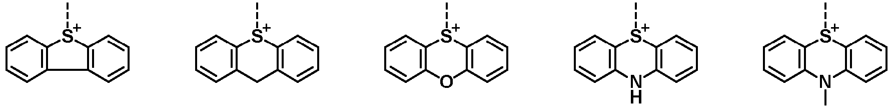

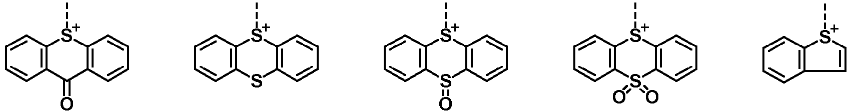

Herein the broken line designates a point of attachment to R^{ct3}.

Examples of the sulfonium cation having formula (Z-1) are as described in JP-A 2024-003744, paragraphs [0102]-[0125] and JP-A 2023-169812, paragraphs [0070]-[0085], but not limited thereto.

Examples of the iodonium cation having formula (Z-2) are as described in JP-A 2024-000259, paragraph [0181], but not limited thereto.

Also preferred as the onium cation Z⁺ is a sulfonium cation having the formula (Z-3).

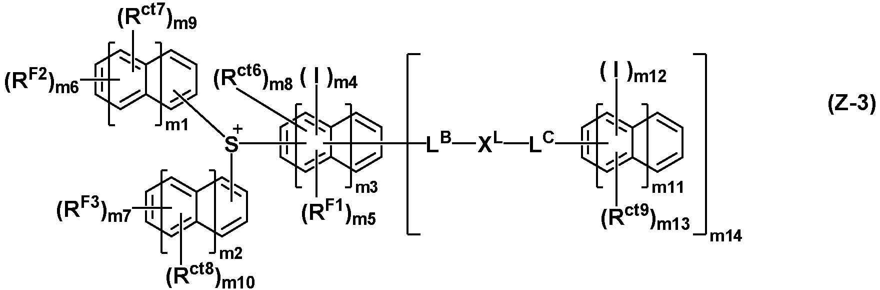

In formula (Z-3), m1 is 0 or 1. The relevant structure is a benzene ring in case of m1=0, and a naphthalene ring in case of m1=1. From the aspect of solvent solubility, the benzene ring corresponding to m1=0 is preferred. The subscript m2 is 0 or 1. The relevant structure is a benzene ring in case of m2=0, and a naphthalene ring in case of m2=1. From the aspect of solvent solubility, the benzene ring corresponding to m2=0 is preferred. The subscript m3 is 0 or 1. The relevant structure is a benzene ring in case of m3=0, and a naphthalene ring in case of m3=1. From the aspect of solvent solubility, the benzene ring corresponding to m3=0 is preferred.

In formula (Z-3), m4 is 0, 1, 2, 3 or 4. As the number of iodine atoms in the cationic structure becomes larger, the amount of absorption of EUV increases, but precipitation in the resist composition may occur due to reduced solvent solubility. Therefore, m4 is preferably 0, 1, 2 or 3, more preferably, 0, 1 or 2.

In formula (Z-3), m5 is 0, 1, 2, 3 or 4. From the aspect of reactant availability, m5 is preferably 0, 1, 2 or 3, more preferably 0, 1 or 2. The subscript m6 is 0, 1, 2, 3, 4, 5 or 6. It is preferred from the aspect of reactant availability that m6 be 0, 1, 2 or 3, more preferably 0, 1 or 2. The subscript m7 is 0, 1, 2, 3, 4, 5 or 6. It is preferred from the aspect of reactant availability that m7 be 0, 1, 2 or 3, more preferably 0, 1 or 2.

In formula (Z-3), m8 is 0, 1 or 2. It is preferred from the aspect of reactant availability that m8 be 0 or 1. The subscript m9 is 0, 1, or 2. It is preferred from the aspect of reactant availability that m9 be 0 or 1. The subscript m10 is 0, 1 or 2. It is preferred from the aspect of reactant availability that m10 be 0 or 1.

In formula (Z-3), m11 is 0 or 1. The relevant structure is a benzene ring in case of m11=0 and a naphthalene ring in case of m11=1. From the aspect of solvent solubility, the benzene ring corresponding to m11=0 is preferred.

In formula (Z-3), m12 is 0, 1, 2, 3 or 4. As the number of iodine atoms in the cationic structure becomes larger, the amount of absorption of EUV increases, but precipitation in the resist composition may occur due to reduced solvent solubility. Therefore, m12 is preferably 0, 1, 2 or 3, more preferably, 0, 1 or 2.

In formula (Z-3), m13 is 0, 1 or 2. It is preferred from the aspect of reactant availability that m13 be 0 or 1. The subscript m14 is 0, 1 or 2. From the aspect of synthesis, m14 is preferably 0 or 1.

The sum m6+m9 is from 0 to 4 when m1=0, and the sum m6+m9 is from 0 to 6 when m1=1. The sum m7+m10 is from 0 to 4 when m2=0, and the sum m7+m10 is from 0 to 6 when m2=1. The sum m4+m5+m8+m14 is from 1 to 4 when m3=0, and the sum m4+m5+m8+m14 is from 1 to 6 when m3=1. The sum m12+m13 is from 0 to 4 when m11=0, and the sum m12+m13 is from 0 to 6 when m11=1. The sum m4+m12 is 1 or more.

In formula (Z-3), R^{F1} to R^{F3} are each independently fluorine, a C₁-C₆ fluorinated saturated hydrocarbyl group, C₁-C₆ fluorinated saturated hydrocarbyloxy group, or C₁-C₆ fluorinated saturated hydrocarbylthio group. Of these, trifluoromethyl, trifluoromethoxy, and trifluorothiomethoxy are preferred. A plurality of R^{F1} may be identical or different when m6 is 2 or more, a plurality of R^{F2} may be identical or different when m7 is 2 or more, and a plurality of R^{F3} may be identical or different when m5 is 2 or more.

In formula (Z-3), R^{ct6} to R^{ct9} are each independently halogen exclusive of iodine and fluorine, nitro, cyano, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom. The hydrocarbyl group and hydrocarbyl moiety in the hydrocarbyloxy and hydrocarbylthio groups may be saturated or unsaturated and straight, branched or cyclic. Examples thereof are as exemplified above for the hydrocarbyl group R¹ in formula (A). In the hydrocarbyl group, and hydrocarbyl moieties of the hydrocarbyloxy and hydrocarbylthio groups, some or all hydrogen may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy, cyano, fluorine, chlorine, bromine, iodine, carbonyl, ether bond, ester bond, sulfonate ester bond, carbonate bond, lactone ring, sultone ring, carboxylic anhydride (-C(=O)-O-C(=O)-) or haloalkyl moiety.

Two R^{ct6} may be identical or different and the two R^{ct6} may bond together to form a ring with the carbon atoms to which they are attached when m8 is 2, two R^{ct7} may be identical or different and the two R^{ct7} may bond together to form a ring with the carbon atoms to which they are attached when m9 is 2, two R^{ct8} may be identical or different and the two R^{ct8} may bond together to form a ring with the carbon atoms to which they are attached when m10 is 2, and two R^{ct9} may be identical or different and the two R^{ct9} may bond together to form a ring with the carbon atoms to which they are attached when m13 is 2. Examples of the ring formed herein include cyclopropane, cyclobutane, cyclopentane, cyclohexane, norbornane and adamantane rings. Some or all of hydrogen atoms in the ring may be replaced by a group containing a heteroatom such as oxygen, sulfur, nitrogen or halogen, some constituent -CH₂- in the ring may be replaced by a group containing a heteroatom such as oxygen, sulfur or nitrogen, and as a result, the hydrocarbyl group may contain a hydroxy group, fluorine, chlorine, bromine, iodine, a cyano group, a carbonyl group, an ether bond, an ester bond, a sulfonic ester bond, a carbonate bond, a lactone ring, a sultone ring, carboxylic anhydride (-C(=O)-O-C(=O)-), a haloalkyl group, or the like.

The aromatic rings directly bonded to S⁺ in the sulfonium cation having formula (Z-3) may bond together to form a ring with S⁺. Exemplary structures of the ring are shown below.

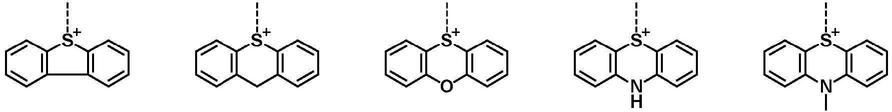

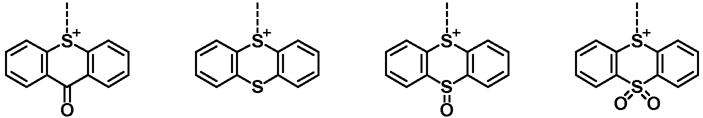

Herein the broken line designates a point of attachment.

In formula (Z-3), L^{B} and L^{C} are each independently a single bond, ether bond, ester bond, amide bond, sulfonate ester bond, sulfonic acid amide amide bond, carbonate bond or carbamate bond. L^{B} is preferably a single bond, ether bond, ester bond or sulfonate ester bond, more preferably an ester bond or sulfonate ester bond. L^{C} is preferably a single bond, ether bond or ester bond, more preferably a single bond.

In formula (Z-3), X^{L} is a single bond or a C₁-C₄₀ hydrocarbylene group which may contain a heteroatom. The hydrocarbylene group may be straight, branched or cyclic. Suitable hydrocarbylene groups include alkanediyl, cyclic saturated hydrocarbylene and arylene groups. Suitable heteroatoms include oxygen, nitrogen and sulfur atoms.

Examples of the optionally heteroatom-containing C₁-C₄₀ hydrocarbylene group X^{L} are shown below, but not limited thereto. In the formulae, * is a point of attachment to L^{B} and L^{C}.

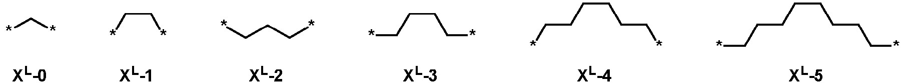

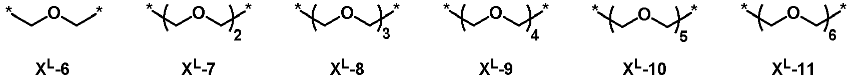

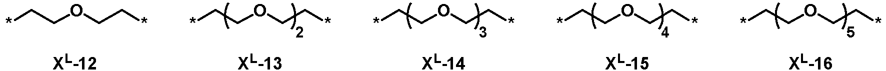

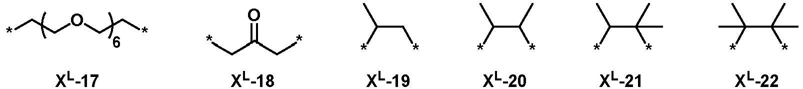

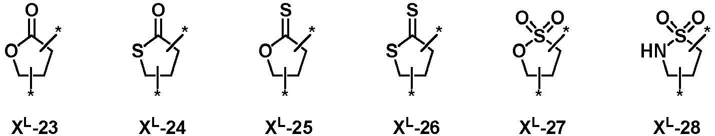

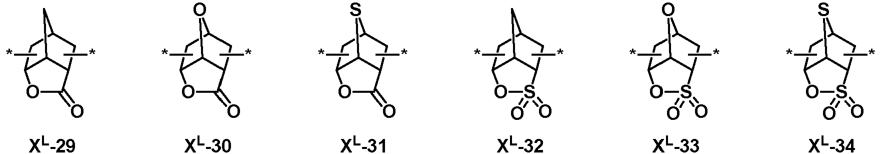

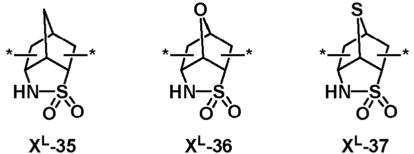

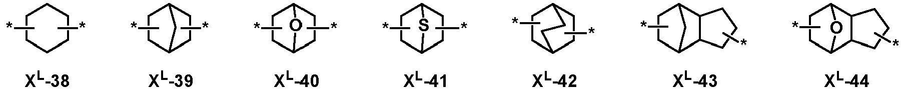

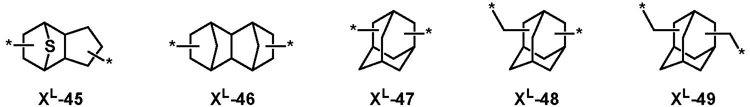

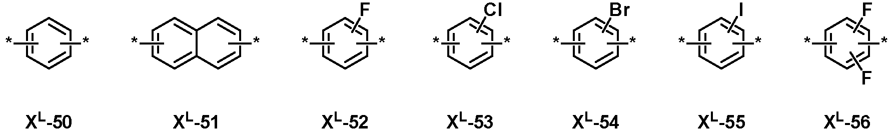

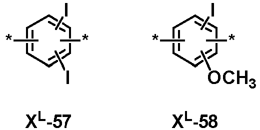

Of these, X^{L}-0 to X^{L}-22, X^{L}-29 to X^{L}-34, and X^{L}-47 to X^{L}-58 are preferred.

Preferably, the sulfonium cation of formula (Z-3) has the formula (Z-3-1).

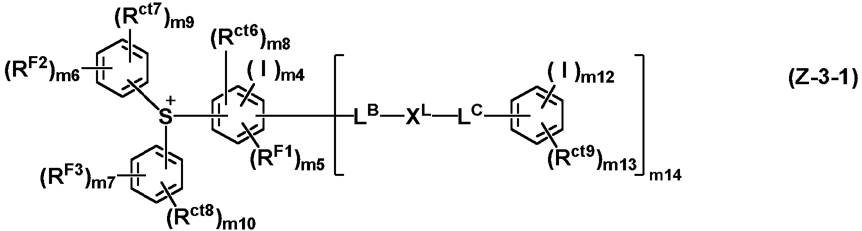

Herein m4 to m10, m12 to m14, R^{F1} to R^{F3}, R^{ct6} to R^{ct9}, L^{B}, L^{C} and X^{L} are as defined above.

Preferably, the sulfonium cation of formula (Z-3-1) has the formula (Z-3-2).

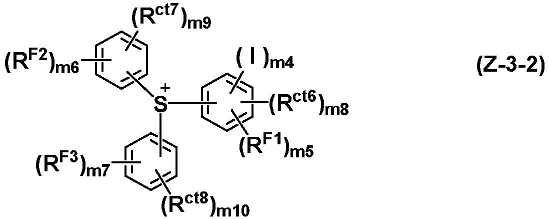

Herein m4 to m10, R^{F1} to R^{F3} and R^{ct6} to R^{ct8} are as defined above.

Examples of the sulfonium cation of formula (Z-3) are shown below, but not limited thereto. In the following formula, Me is a methyl group.

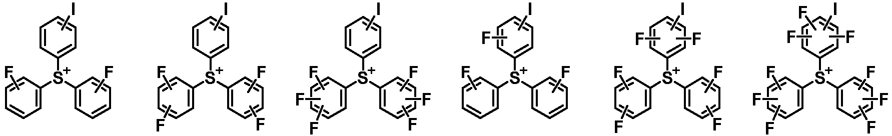

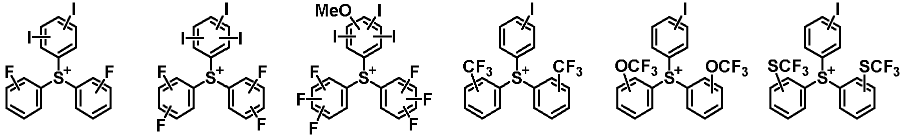

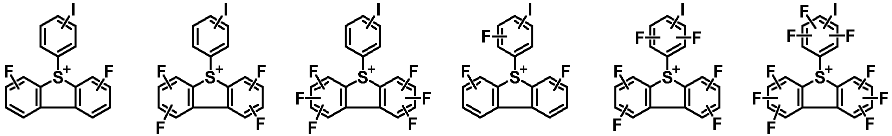

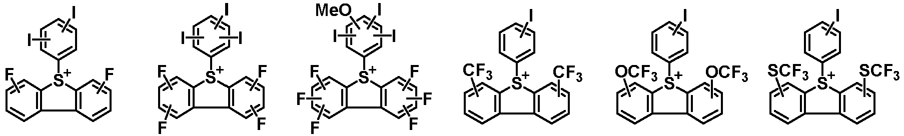

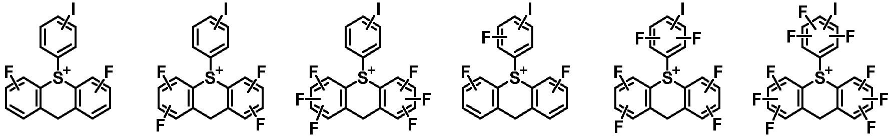

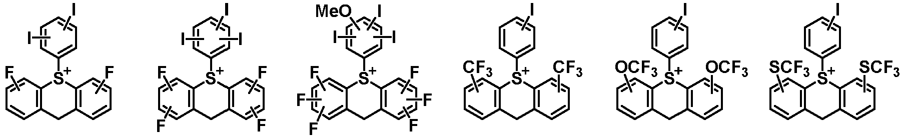

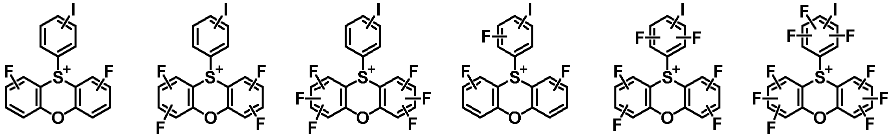

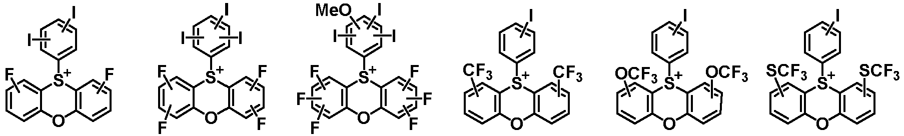

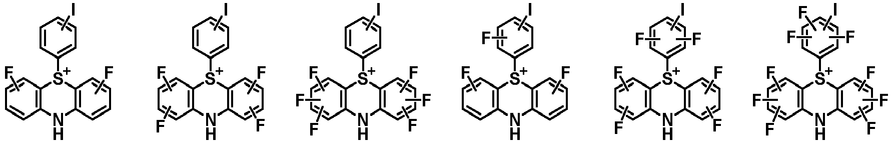

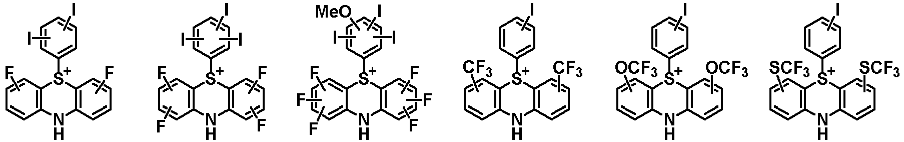

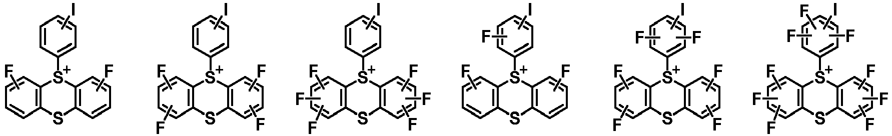

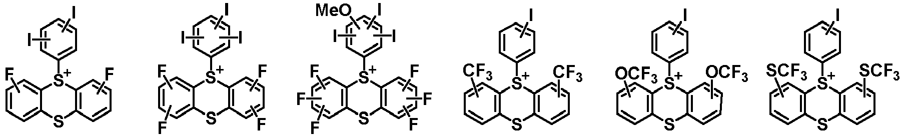

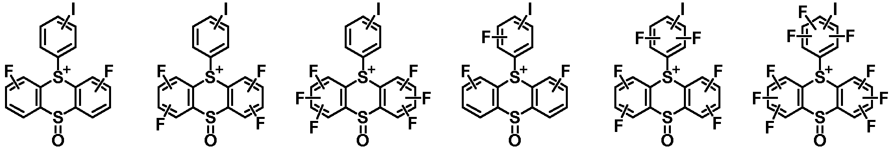

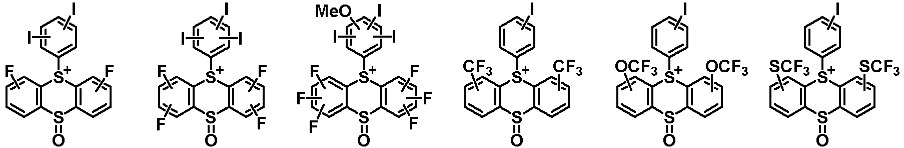

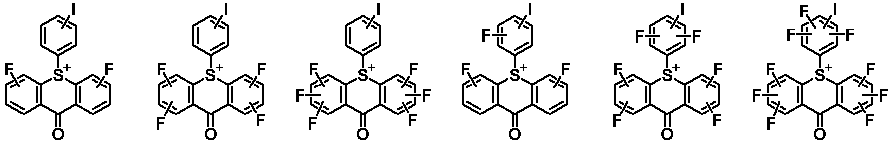

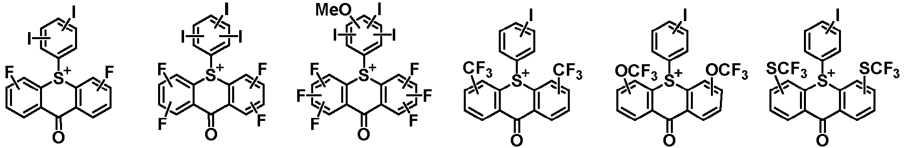

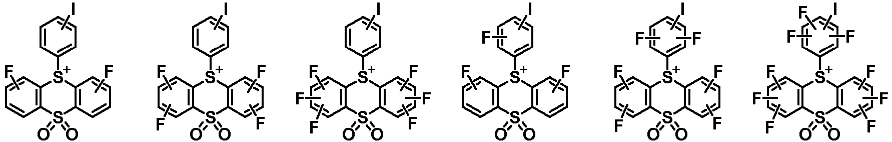

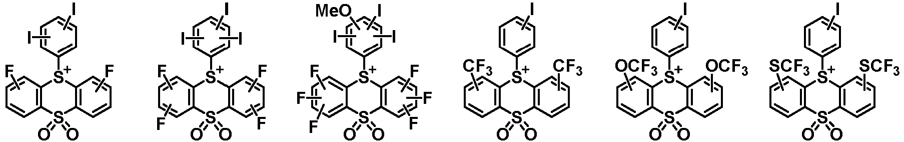

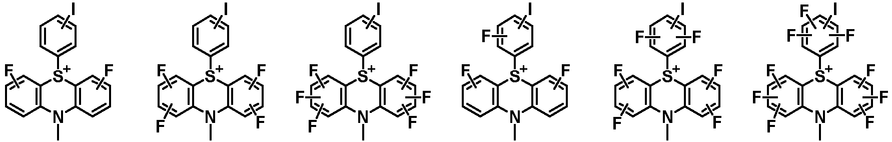

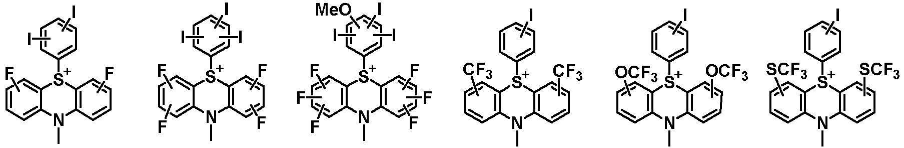

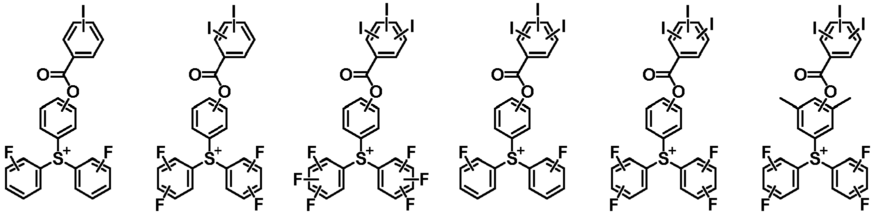

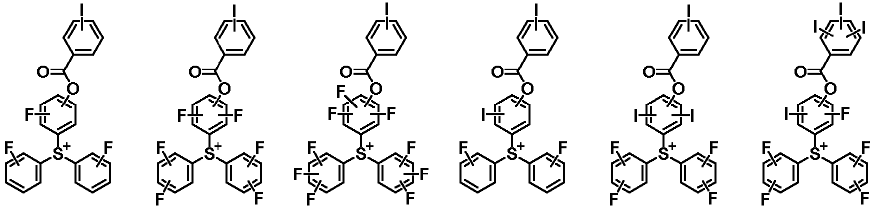

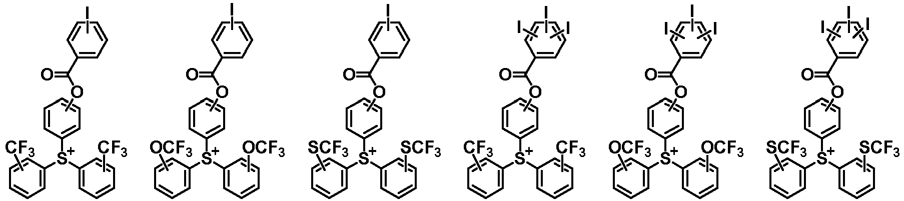

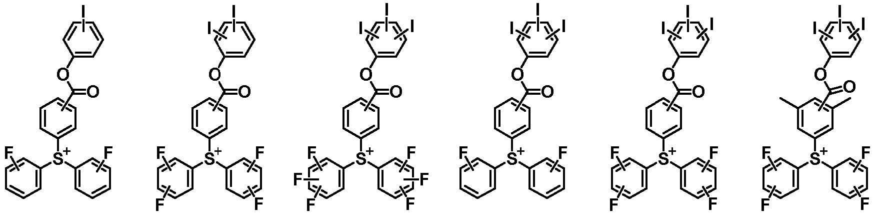

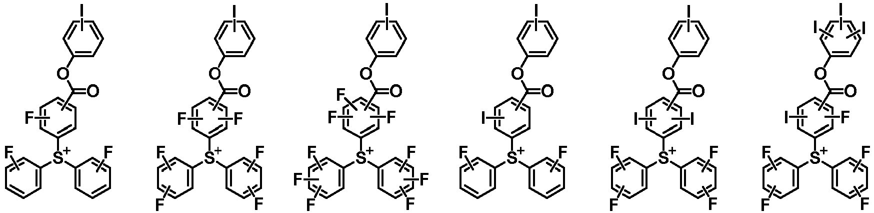

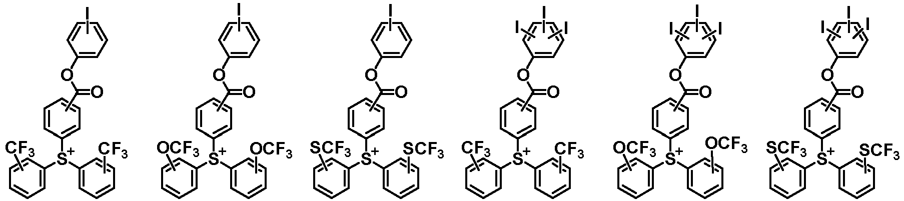

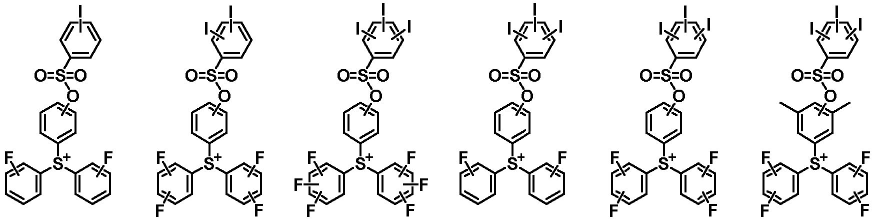

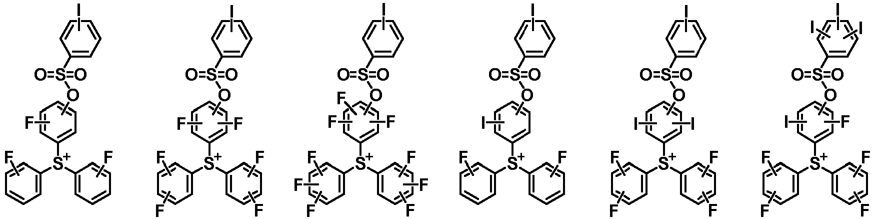

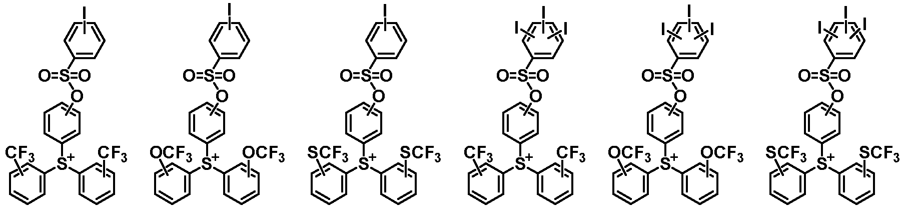

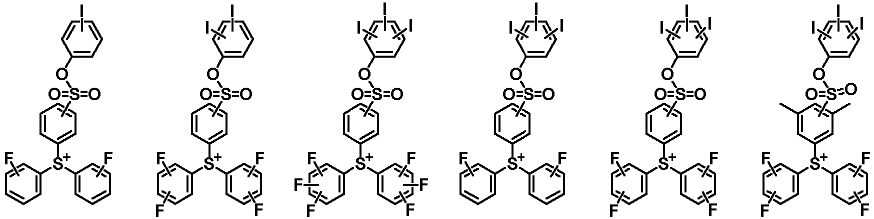

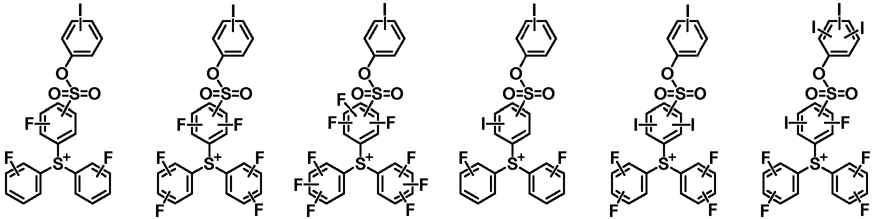

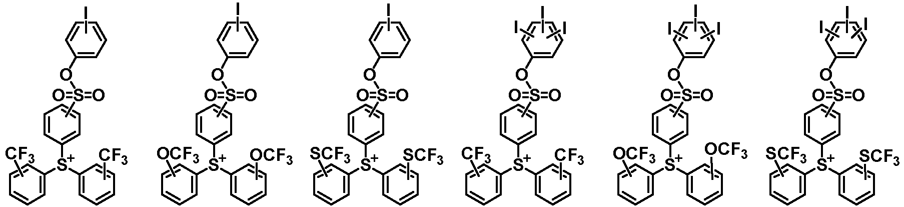

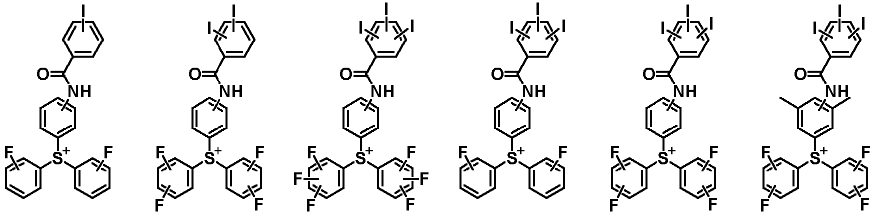

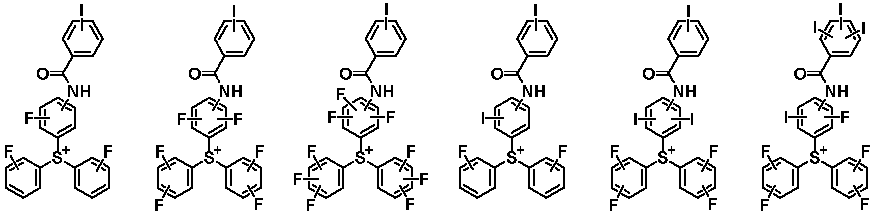

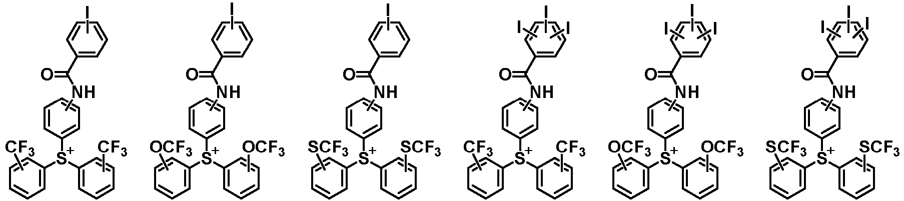

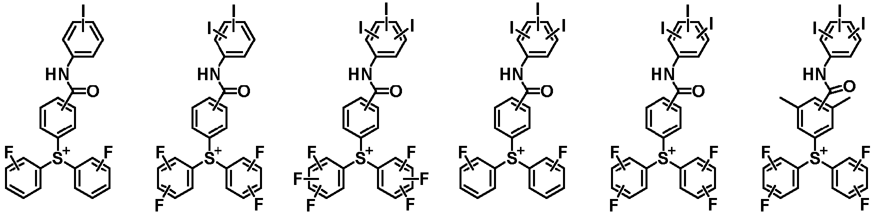

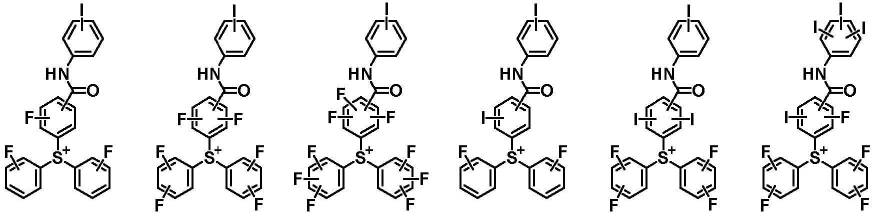

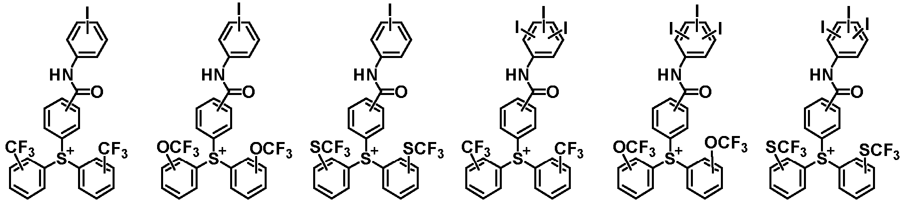

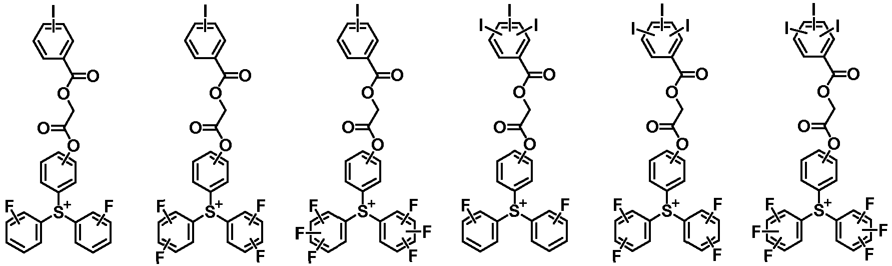

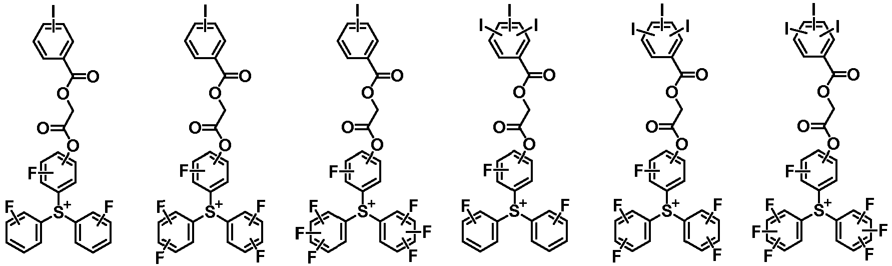

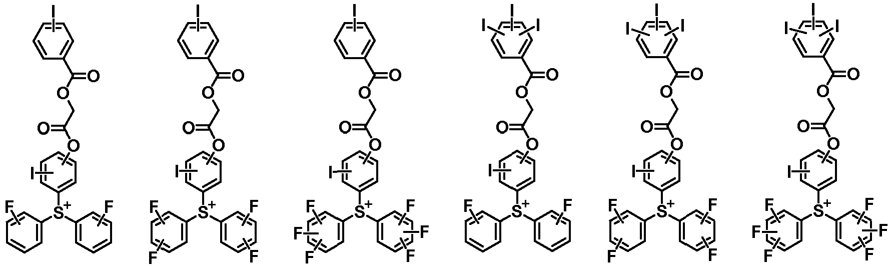

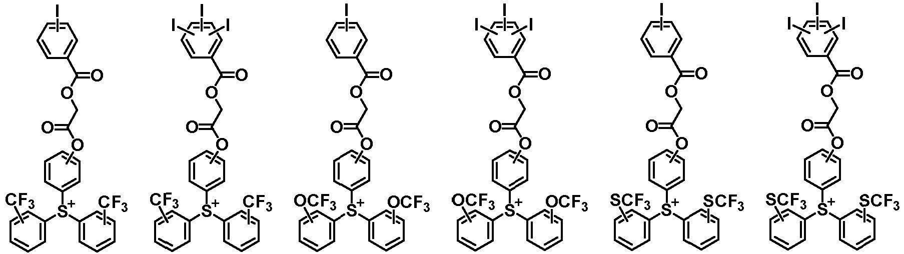

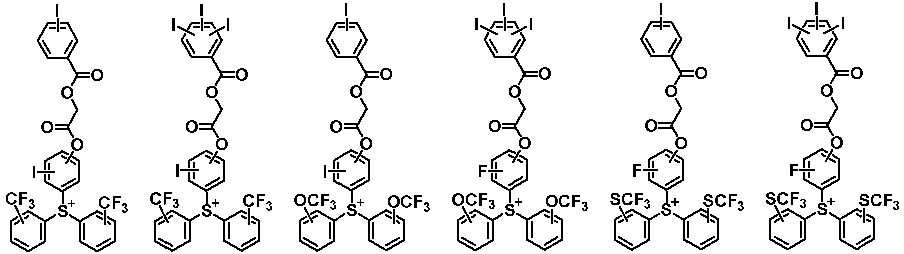

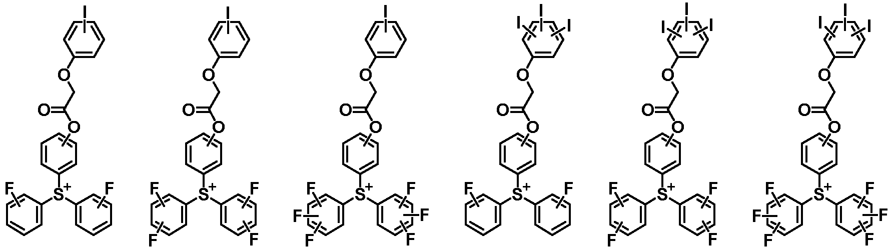

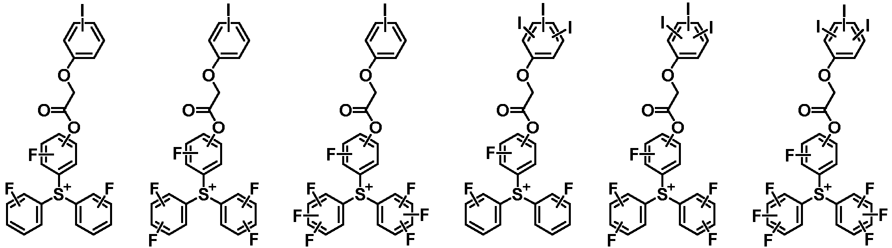

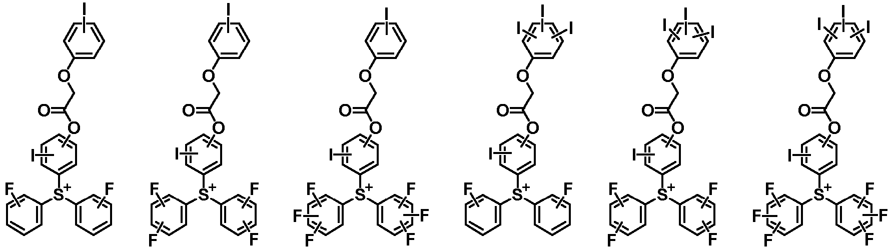

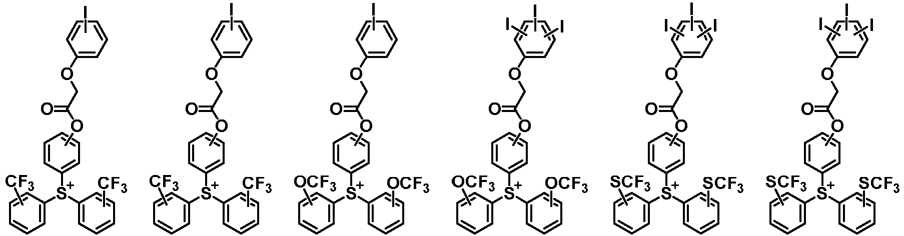

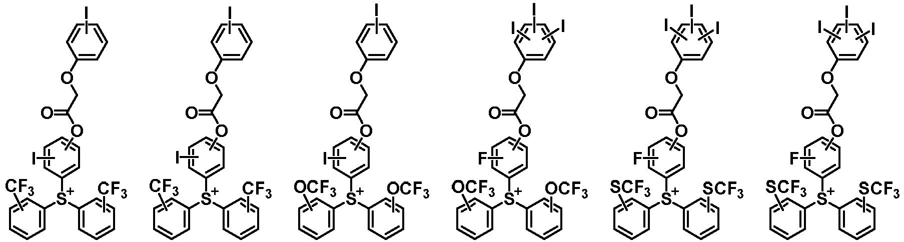

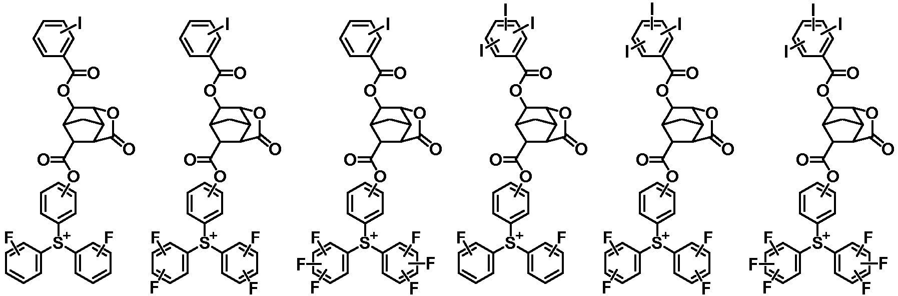

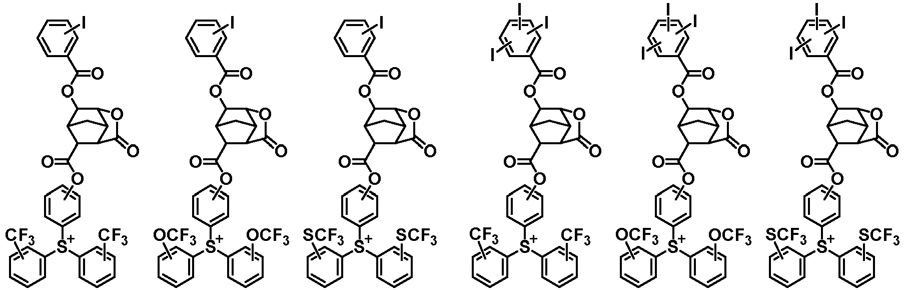

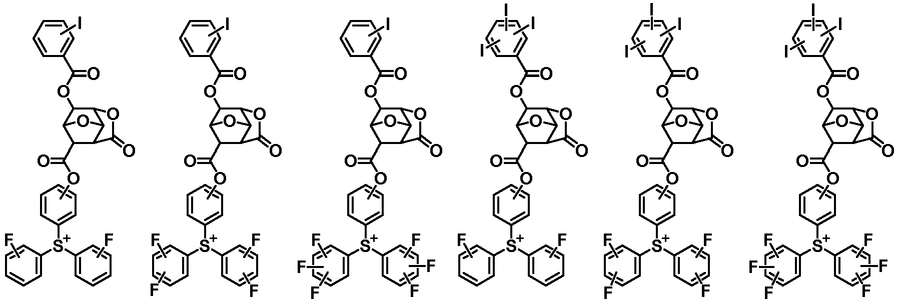

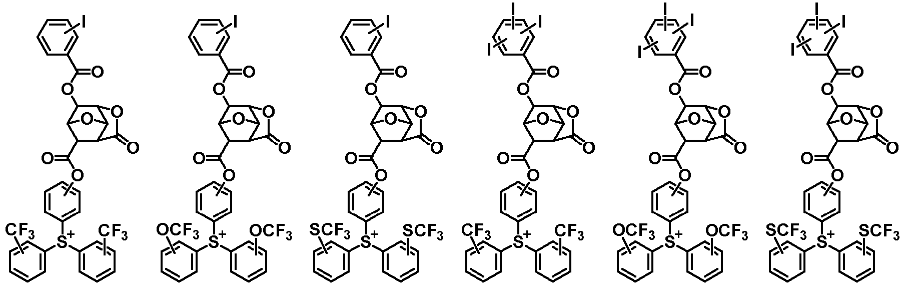

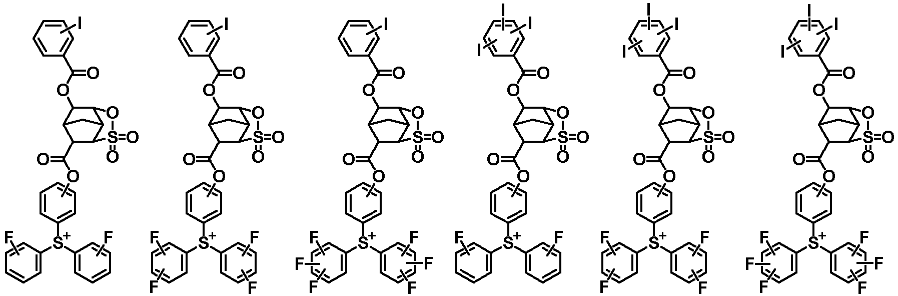

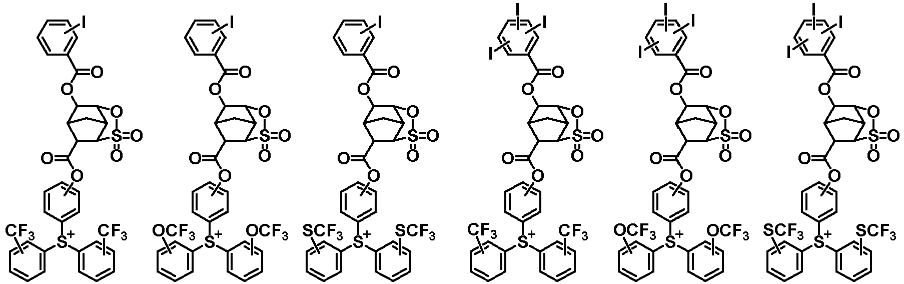

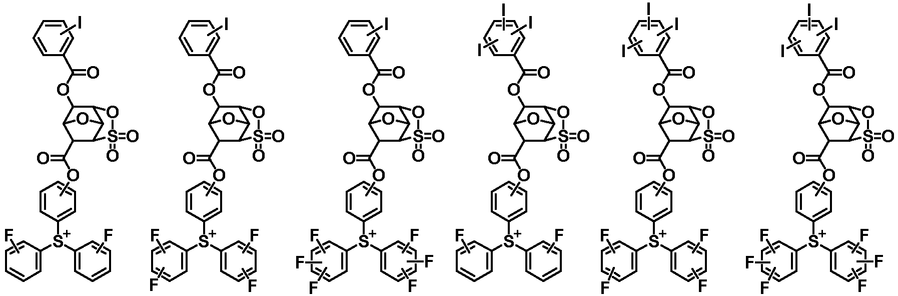

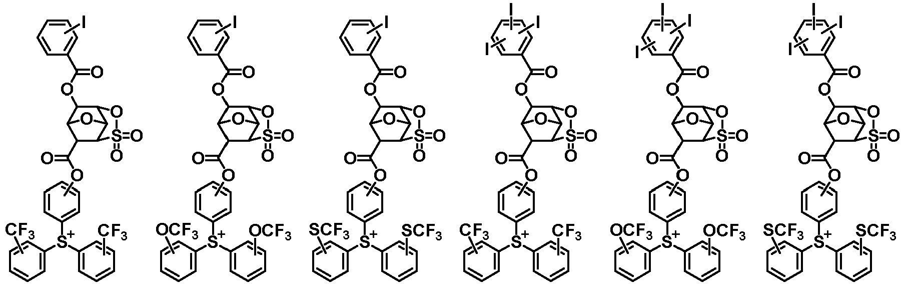

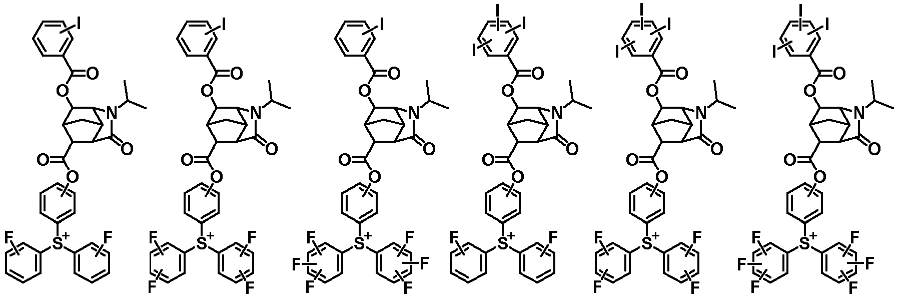

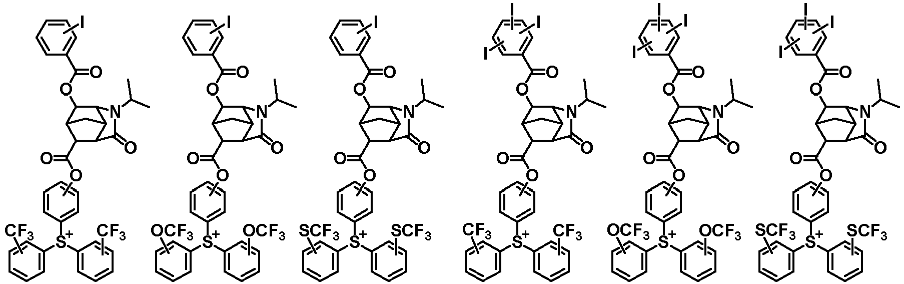

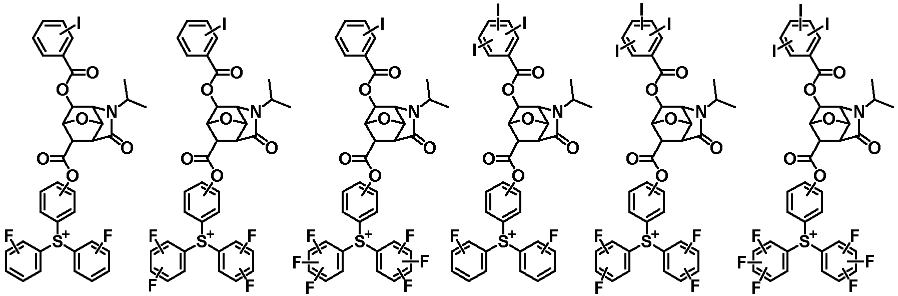

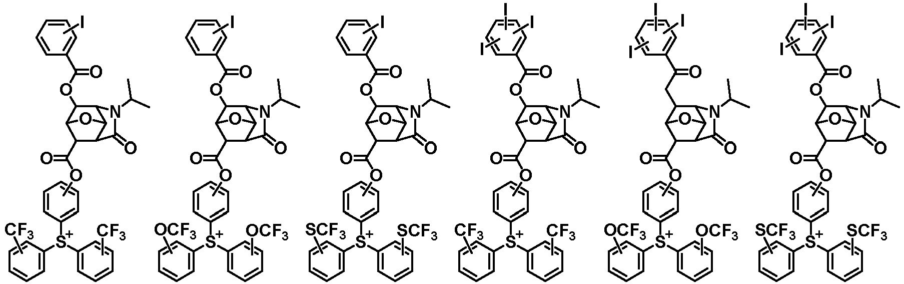

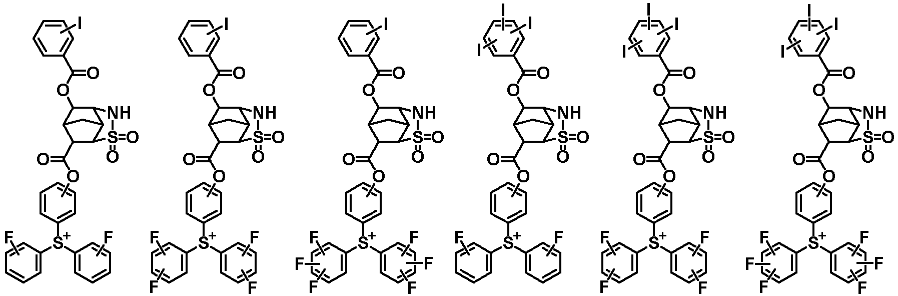

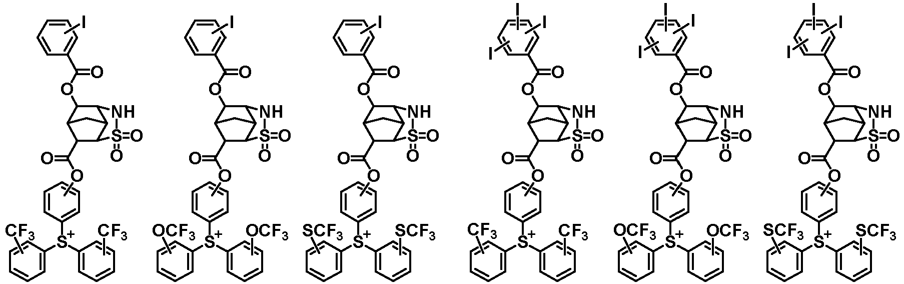

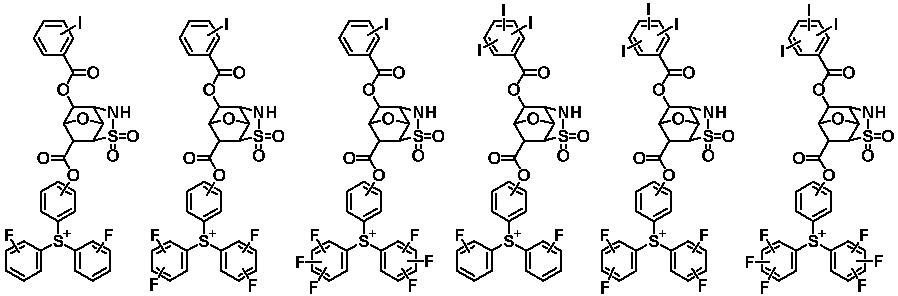

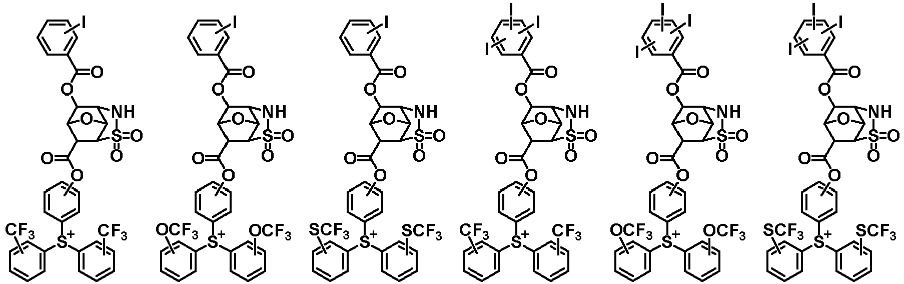

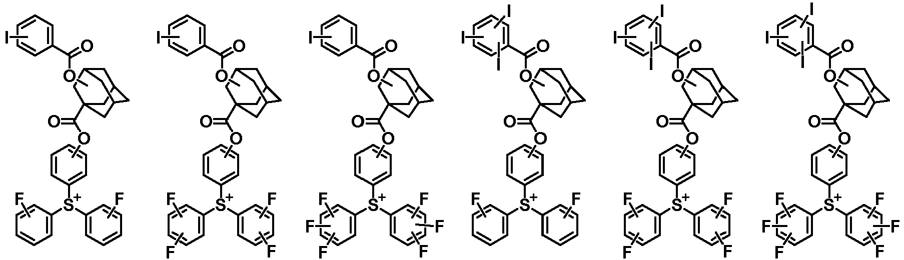

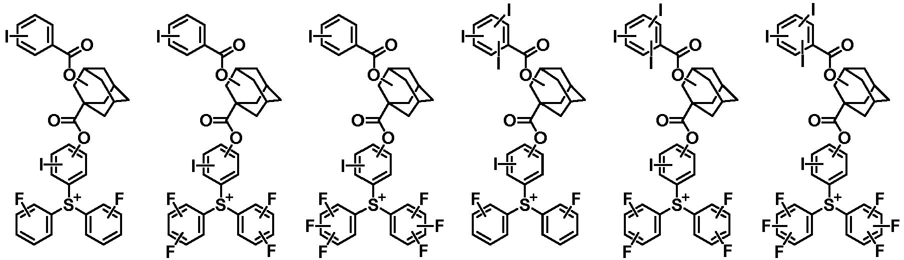

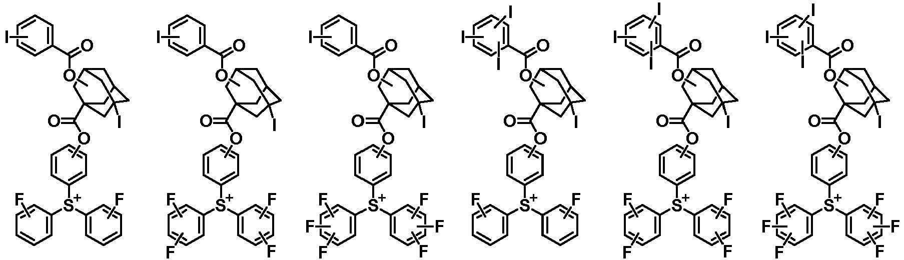

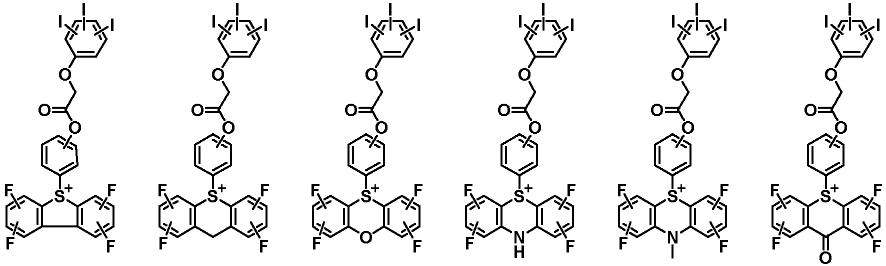

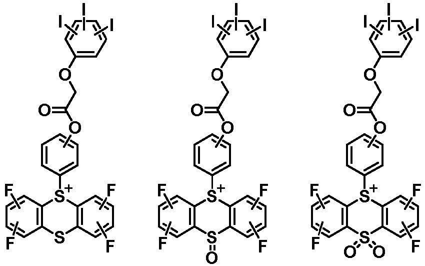

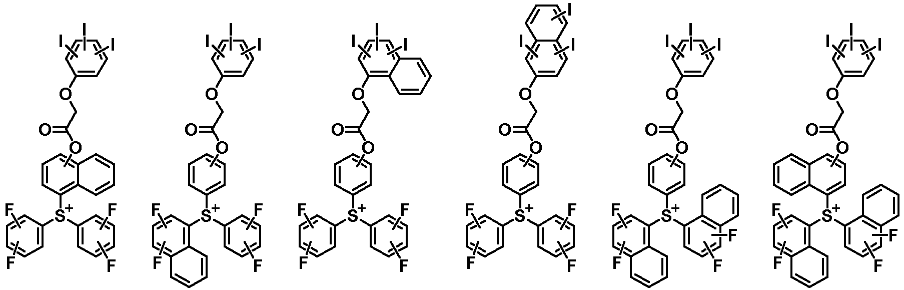

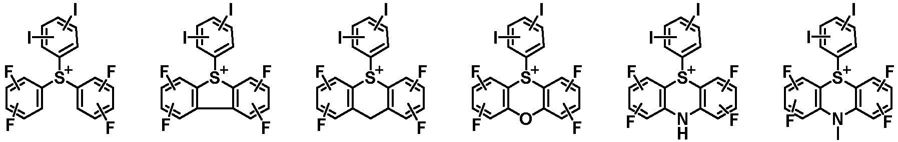

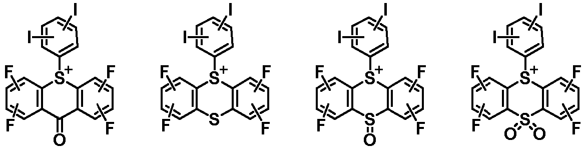

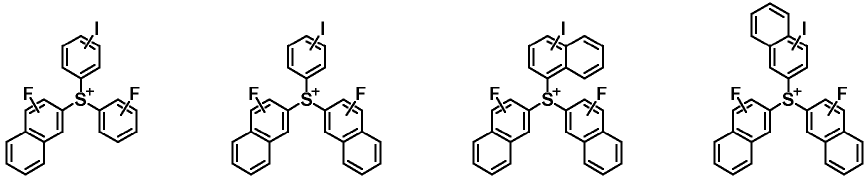

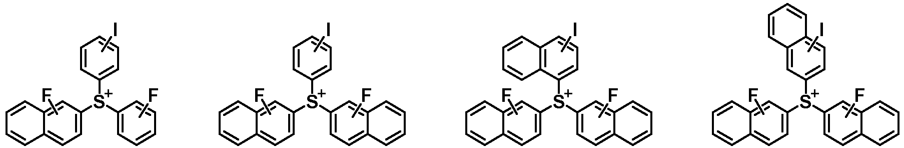

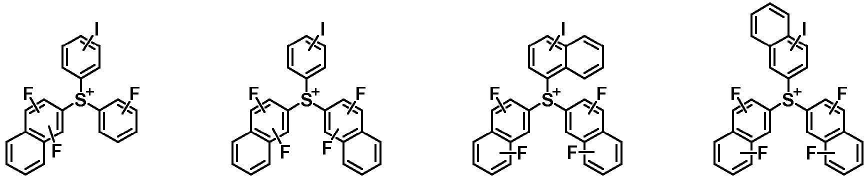

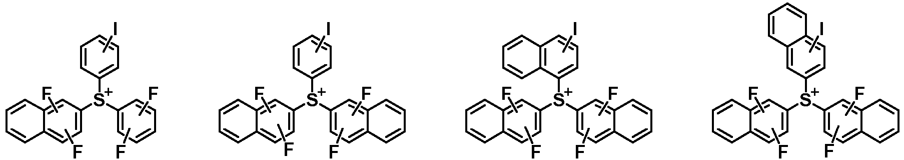

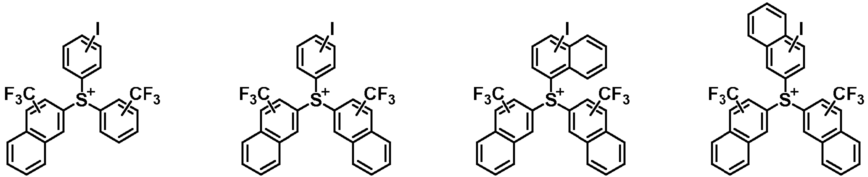

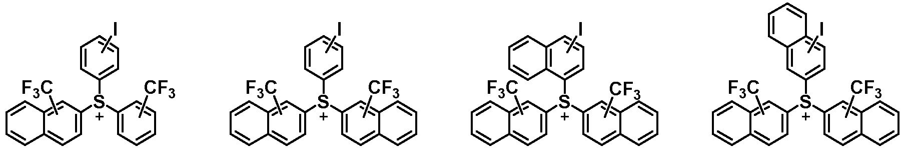

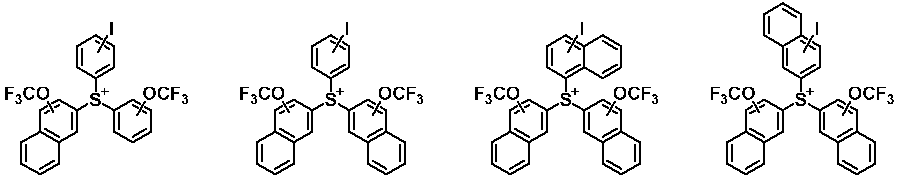

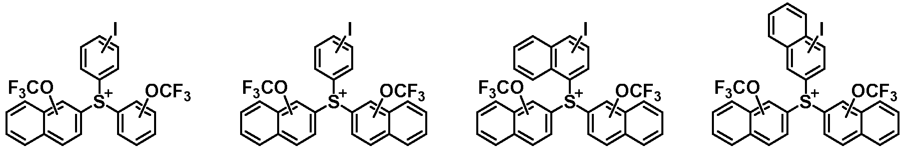

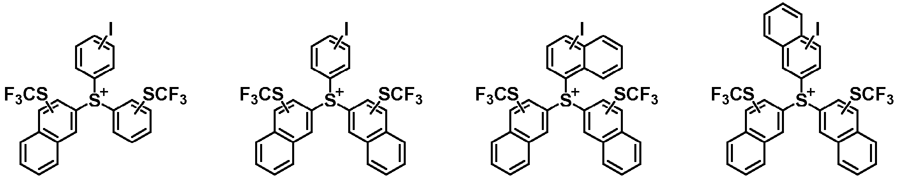

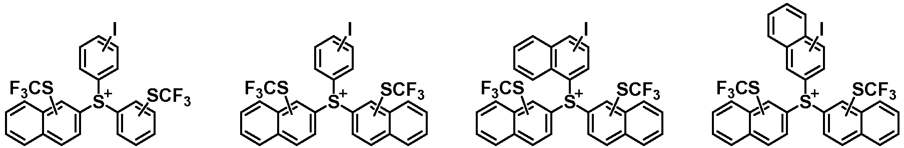

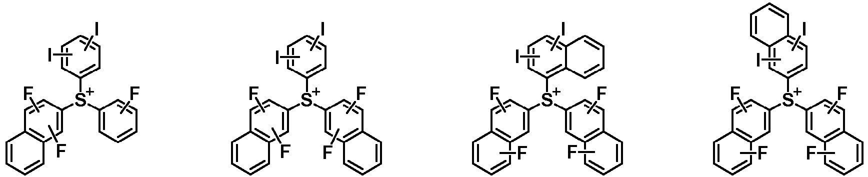

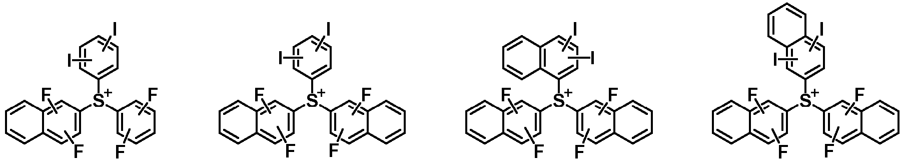

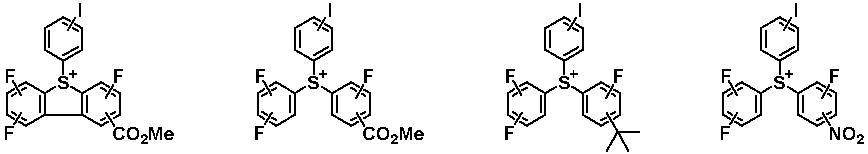

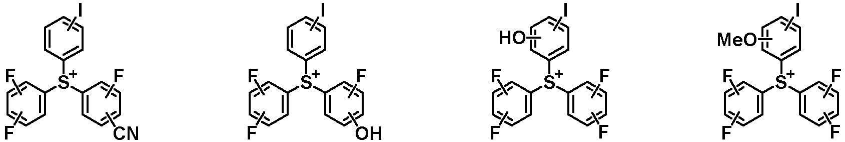

Examples of repeat units B6 to B10 include arbitrary combinations of the anion with the cation, both as exemplified above.

The repeat units B6 to B10 are capable of generating an acid upon exposure to high energy radiation. It is believed that binding of the relevant units to a polymer enables to appropriately control acid diffusion and to form a pattern with reduced LER. Since the acid-generating unit is bound to a polymer, the phenomenon that acid volatilizes from the exposed region and re-deposits on the unexposed region during bake in vacuum is suppressed. This is effective for reducing LER and for suppressing profile degradation due to unwanted film thickness loss in the unexposed region.

Of repeat units B6 to B10, repeat units B7 to B10 are preferred for the processing of photomask blanks because an optimum acid strength is available for the suppression of acid diffusion and the design of an acid labile group on the polymer. The repeat units B8, B9 and B10 are more preferred.

When repeat units B6 to B10 are included, their content is preferably 0.1 to 30 mol%, more preferably 0.5 to 20 mol% based on the overall repeat units of the polymer. Each of repeat units B6 to B10 may be of one type or a combination of plural types.

The content of repeat units having an aromatic ring structure is preferably at least 65 mol%, more preferably at least 75 mol%, even more preferably at least 85 mol% based on the overall repeat units of the polymer. When the polymer does not contain repeat units B6 to B 10, it is preferred that all units have an aromatic ring structure.

The polymer may further comprise (meth)acrylate units protected with an acid labile group or (meth)acrylate units having an adhesive group such as lactone structure or hydroxy group other than phenolic hydroxy as commonly used in the art. These repeat units are effective for fine adjustment of properties of a resist film, but not essential.

Examples of the (meth)acrylate unit having an adhesive group include repeat units having the formula (B11), repeat units having the formula (B12), and repeat units having the formula (B13), which are also referred to as repeat units B11, B12, and B13, respectively. While these units do not exhibit acidity, they may be used as auxiliary units for providing adhesion to substrates or adjusting solubility.

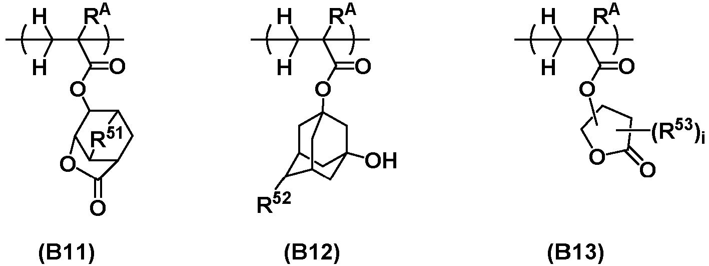

In formulae (B11) and (B13), R^{A} is each independently hydrogen, fluorine, methyl, or trifluoromethyl group. R⁵¹ is -O- or methylene. R⁵² is hydrogen or hydroxy group. R⁵³ is a C₁-C₄ saturated hydrocarbyl group. The subscript i is 0, 1, 2 or 3.

When the repeat units B11 to B13 are included, their content is preferably 0 to 20 mol%, more preferably 0 to 10 mol% based on the overall repeat units of the polymer. Each of the repeat units B11 to B13 may be of one type or a combination of plural types.

The polymer can be synthesized by copolymerizing monomers protected with a protective group if necessary, by a known method, and then carrying out a deprotection reaction if necessary. The copolymerization reaction is not limited, and is preferably radical polymerization, or anionic polymerization. For these methods, reference may be made to JP-A 2004-115630.

The polymer should preferably have a weight average molecular weight (Mw) of 1,000 to 50,000, and more preferably 2,000 to 20,000. A Mw of at least 1,000 eliminates the risk that pattern features are rounded at their top to invite degradations of resolution and LER. A Mw of up to 50,000 eliminates the risk that LER is degraded when a pattern with a line width of up to 100 nm is formed. As used herein, Mw is measured by gel permeation chromatography (GPC) versus polystyrene standards using THF or DMF solvent.

The polymer preferably has a narrow molecular weight distribution or dispersity (Mw/Mn) of 1.0 to 2.0, more preferably 1.0 to 1.9, even more preferably 1.0 to 1.8. A polymer with such a narrow dispersity eliminates the risk that foreign matter are left on the pattern after development and the pattern profile is aggravated.

The base polymer is designed such that the dissolution rate in alkaline developer is preferably up to 10 nm/min, more preferably up to 7 nm/min, even more preferably up to 5 nm/min. In the lithography of advanced generation wherein the coating film on the substrate is in a thin film range of up to 100 nm, the influence of pattern film thickness loss during alkaline development becomes strong. When the polymer has an alkaline dissolution rate in excess of 10 nm/min, pattern collapse occurs, meaning a failure to form small-size patterns. The problem becomes outstanding in the fabrication of photomasks requiring to be defectless and having a tendency of strong development process. It is noted that the dissolution rate of a base polymer in alkaline developer is computed by spin coating a 16.7 wt% solution of a polymer in propylene glycol monomethyl ether acetate (PGMEA) solvent onto an 8-inch silicon wafer, baking at 100°C for 90 seconds to form a film of 1,000 nm thick, developing the film in a 2.38 wt% aqueous solution of tetramethylammonium hydroxide (TMAH) at 23°C for 100 seconds, and measuring a loss of film thickness.

The base polymer as component (B) may comprise additional polymers as well as the polymers described above. As the additional polymers, polymers that have been heretofore known as base polymers for resist compositions can be used. The content of the other polymer is not limited as long as the effect of the present invention is not impaired.

### (C) Organic solvent

The inventive resist composition may comprise (C) an organic solvent as a component. The organic solvent is not particularly limited as long as the components are soluble therein. Examples of the organic solvent are described in JP-A 2008-111103, paragraphs [0144]-[0145] (USP 7,537,880). Exemplary solvents include ketones such as cyclohexanone, cyclopentanone, methyl-2-n-pentyl ketone and 2-heptanone; alcohols such as 3-methoxybutanol, 3-methyl-3-methoxybutanol, 1-methoxy-2-propanol, 1-ethoxy-2-propanol and diacetone alcohol (DAA); ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as PGMEA, propylene glycol monoethyl ether acetate, ethyl lactate (EL), ethyl pyruvate, butyl acetate, methyl 3-methoxypropionate, ethyl 3-ethoxypropionate, tert-butyl acetate, tert-butyl propionate and propylene glycol mono-tert-butyl ether acetate; and lactones such as γ-butyrolactone; and mixtures thereof. When a polymer containing an acid labile group of acetal form is used, a high-boiling alcohol solvent may be added for accelerating the deprotection reaction of acetal, for example, diethylene glycol, propylene glycol, glycerol, 1,4-butanediol or 1,3-butanediol.

Of the foregoing organic solvents, 1-ethoxy-2-propanol, PGMEA, PGME, cyclohexanone, EL, γ-butyrolactone and mixtures thereof are preferred.

When the inventive chemically amplified positive resist composition comprises the organic solvent (C), the content of the organic solvent (C) is preferably 200 to 10,000 parts by weight, more preferably 400 to 5,000 parts by weight per 80 parts by weight of the base polymer (B). The organic solvent (C) may be used alone or in admixture.

### (D) Photoacid generator

The inventive chemically amplified positive resist composition may further comprise (D) a photoacid generator (PAG) as a component. The PAG used herein may be any compound capable of generating an acid upon exposure to high-energy radiation. Suitable PAGs include sulfonium salts, iodonium salts, sulfonyldiazomethane, N-sulfonyloxyimide, and oxime-O-sulfonate acid generators.

Suitable PAGs include nonafluorobutane sulfonate, partially fluorinated sulfonates described in JP-A 2012-189977, paragraphs [0247]-[0251], partially fluorinated sulfonates described in JP-A 2013-101271, paragraphs [0261]-[0265], and those described in JP-A 2008-111103, paragraphs [0122]-[0142] and JP-A 2010-215608, paragraphs [0080]-[0081]. Among others, arenesulfonate and alkanesulfonate type PAGs are preferred because they generate acids having an appropriate strength to deprotect the acid labile group in repeat unit B2.

The preferred PAGs are salt compounds having a sulfonium anion of the structure shown below.

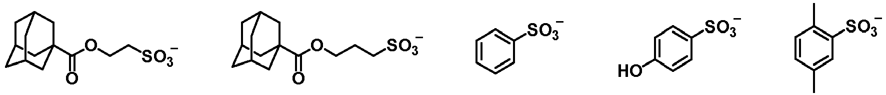

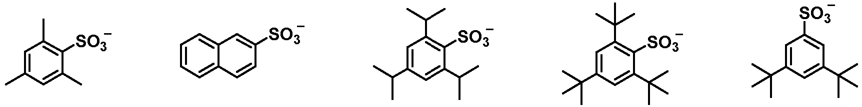

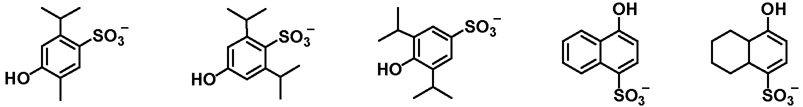

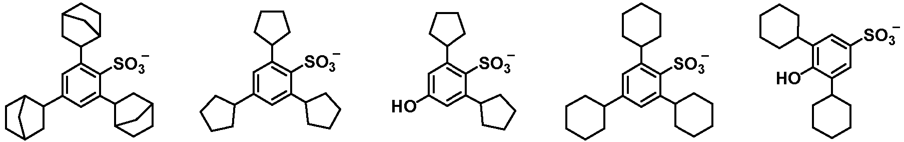

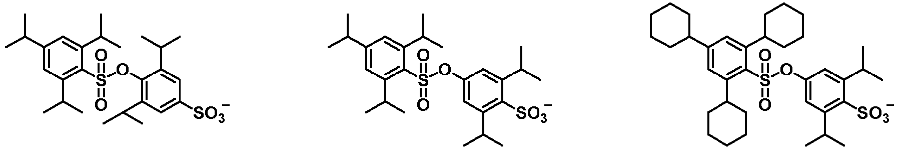

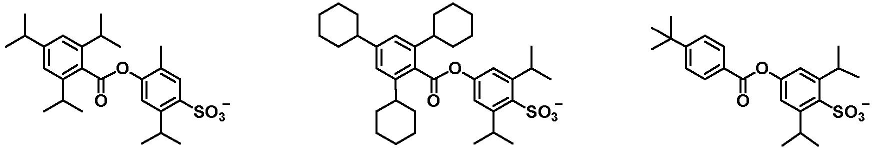

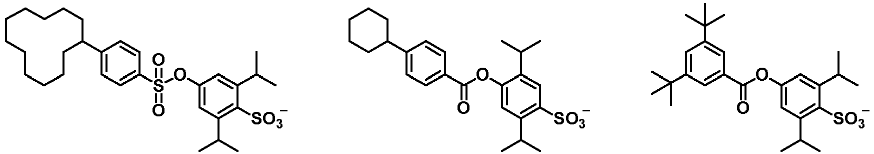

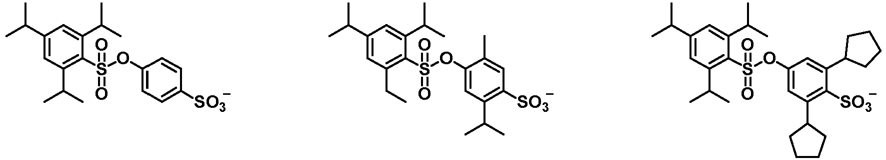

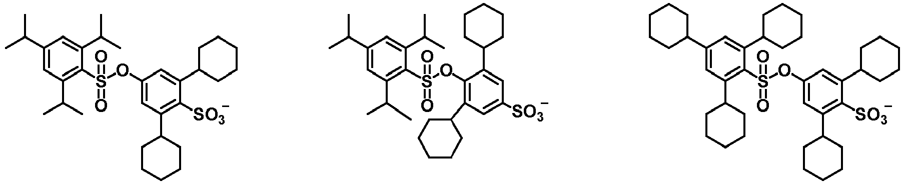

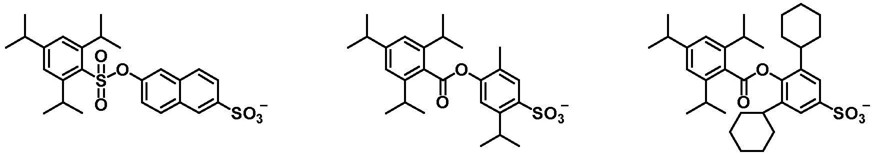

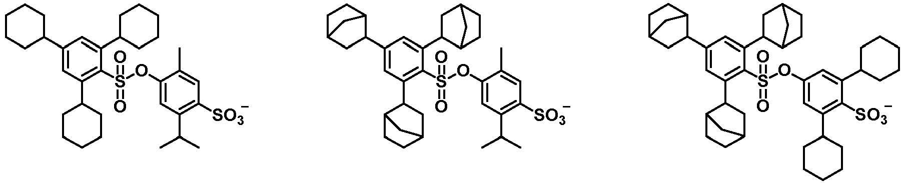

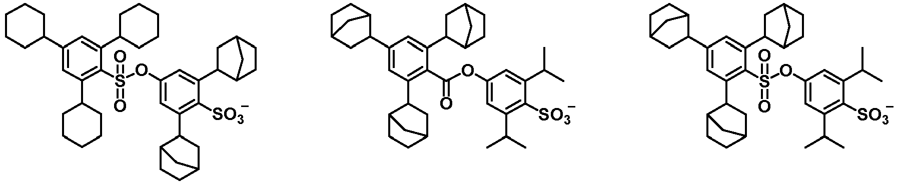

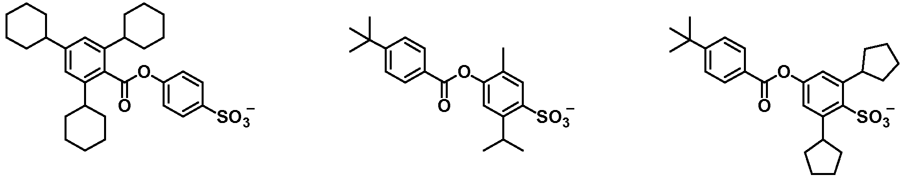

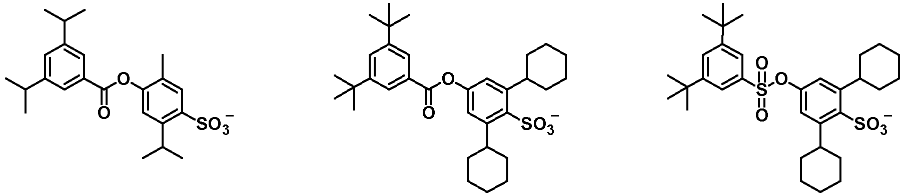

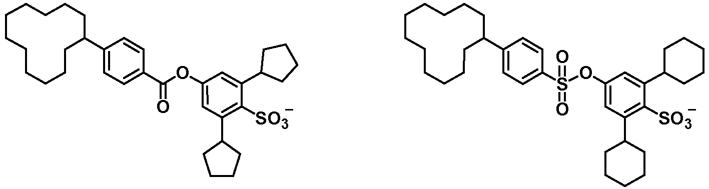

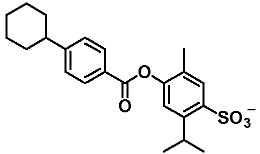

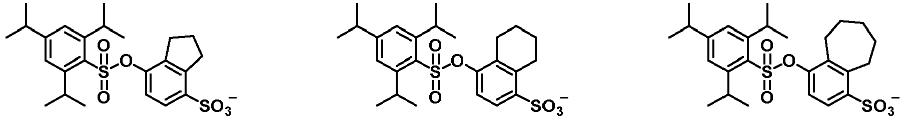

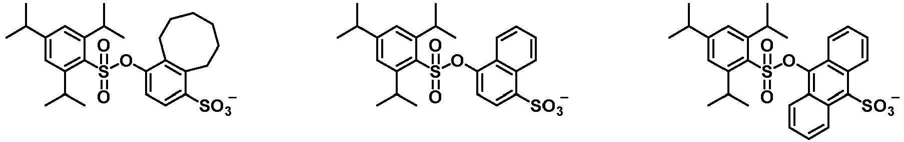

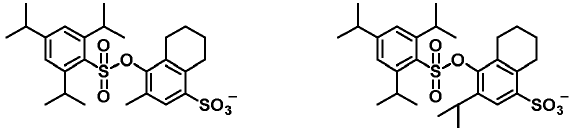

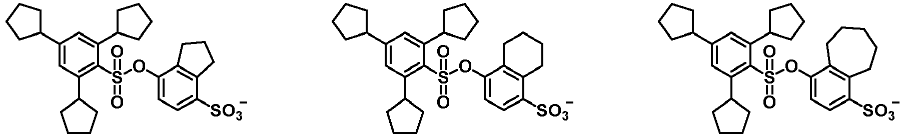

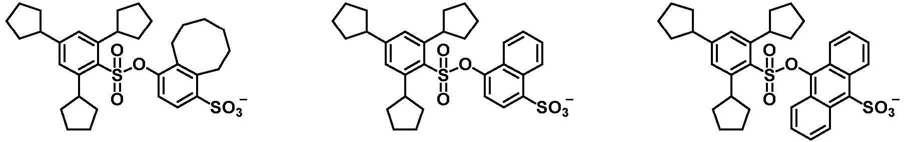

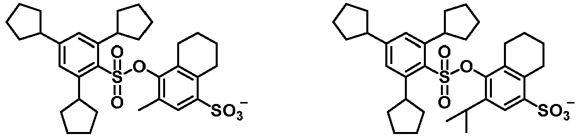

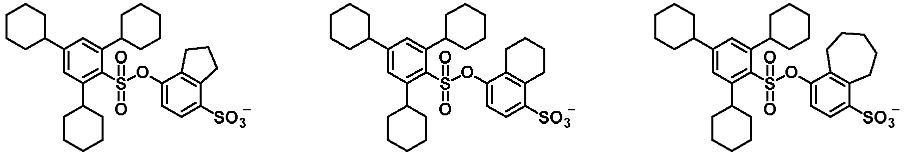

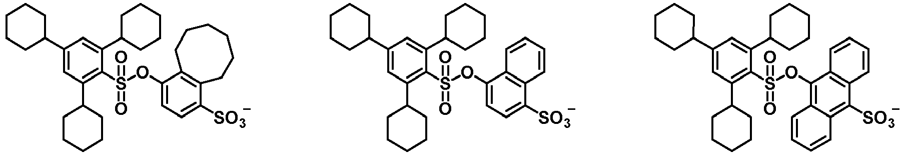

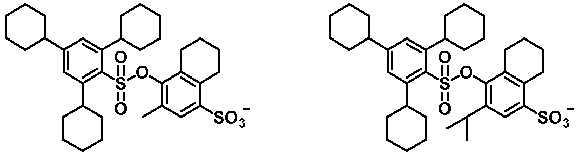

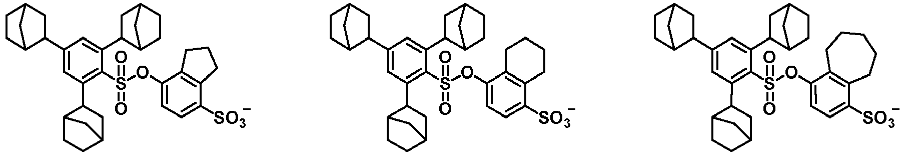

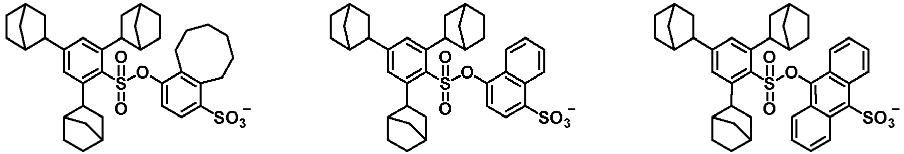

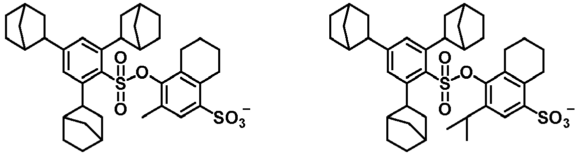

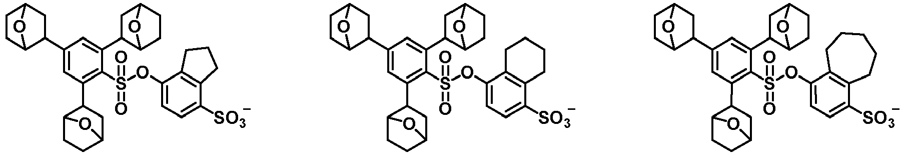

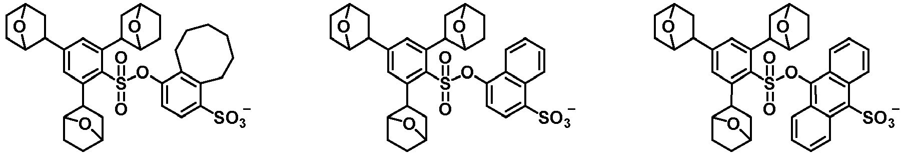

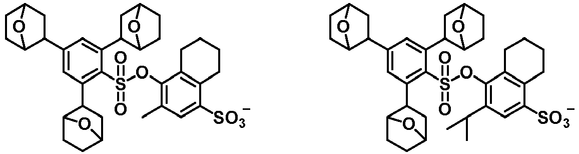

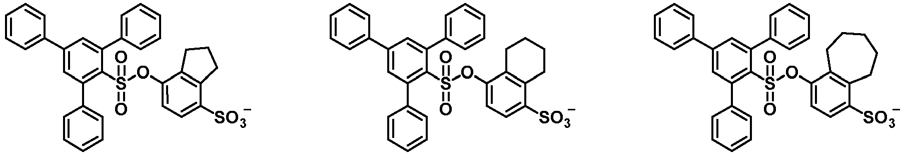

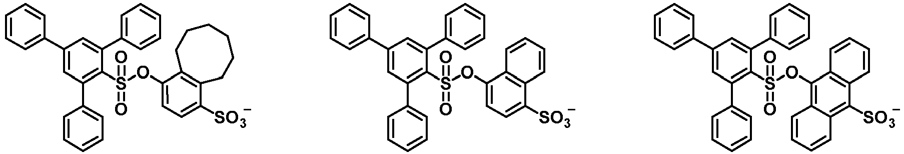

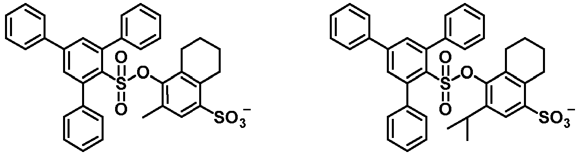

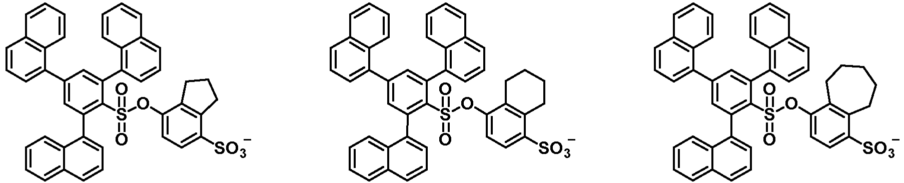

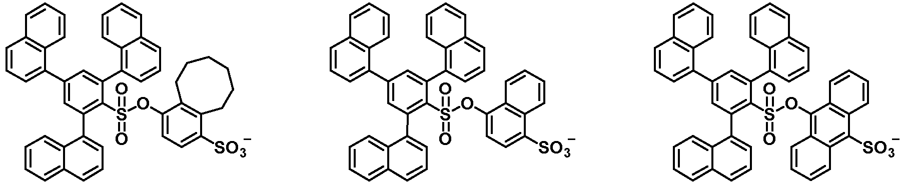

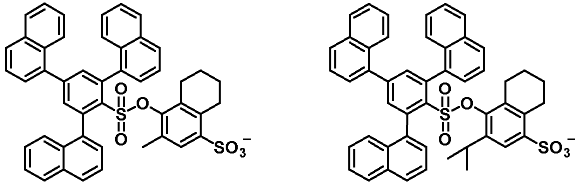

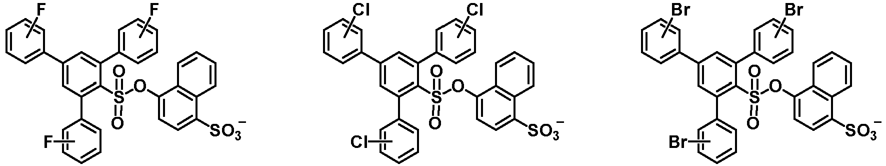

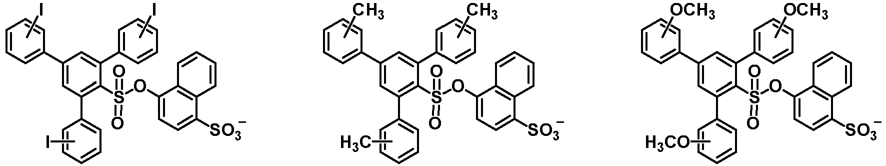

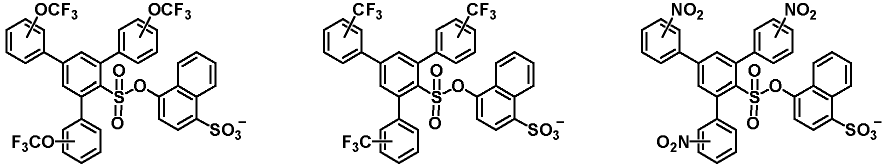

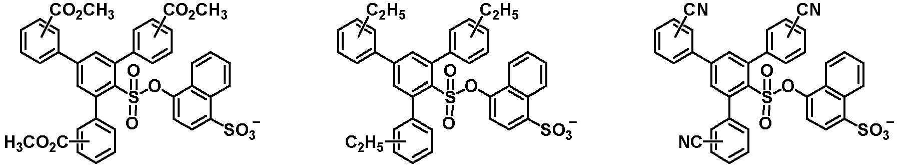

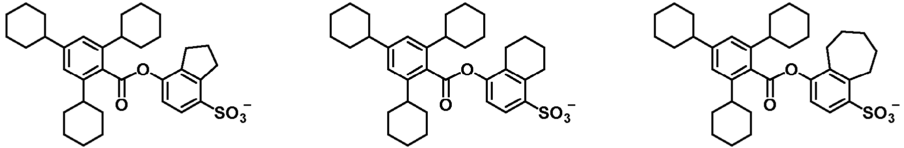

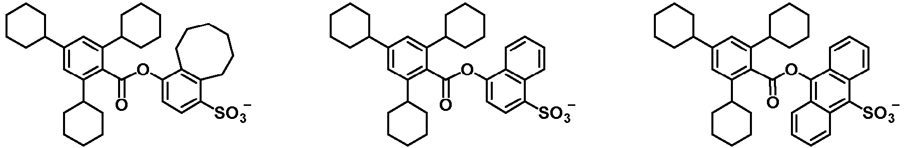

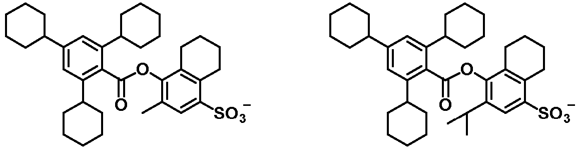

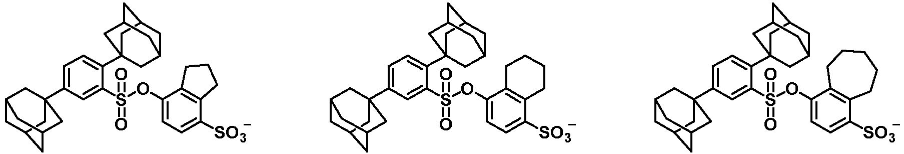

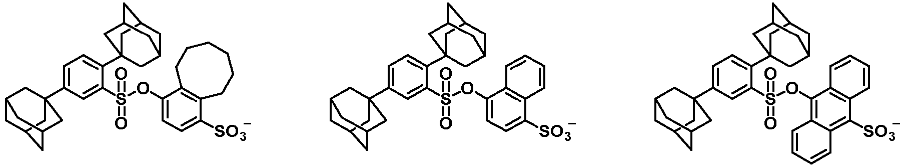

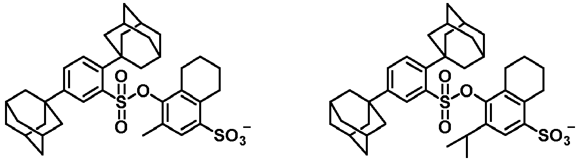

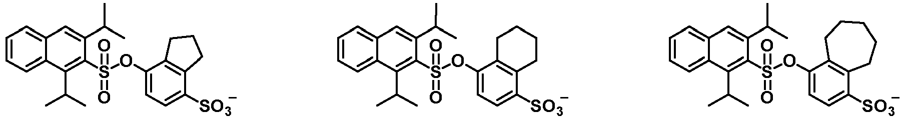

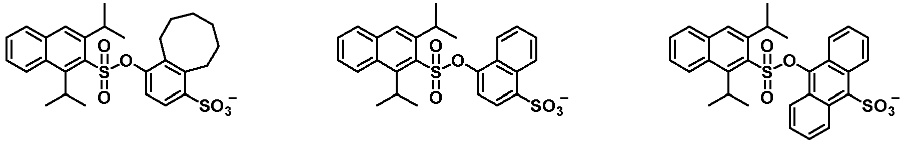

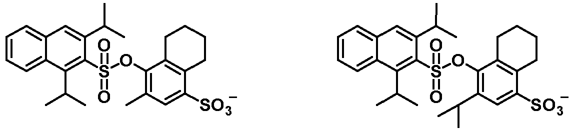

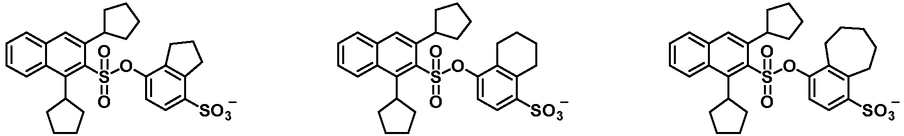

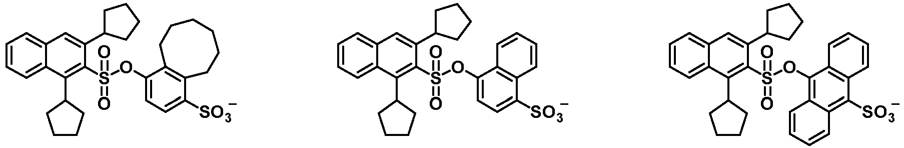

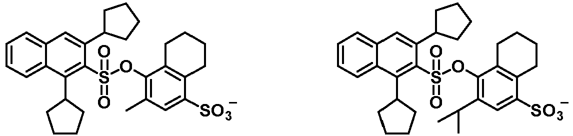

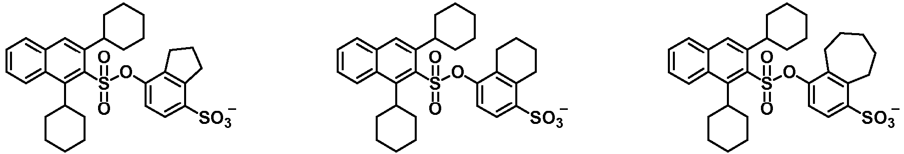

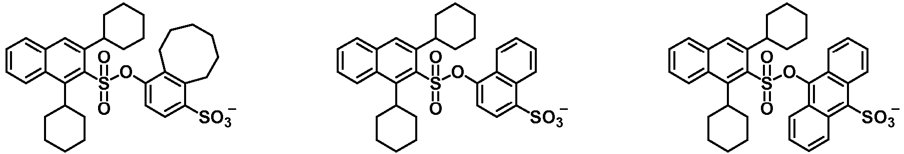

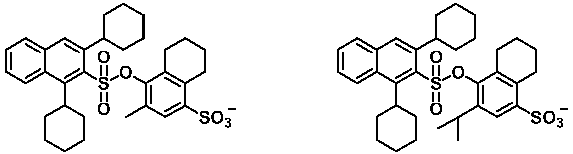

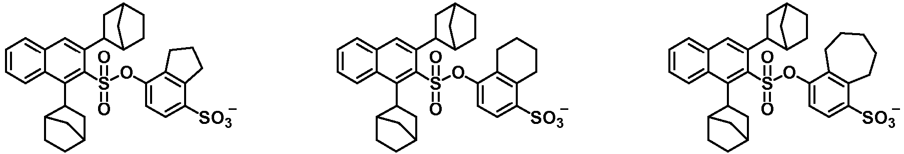

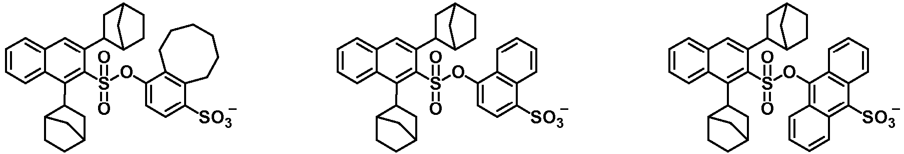

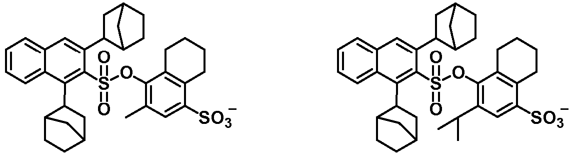

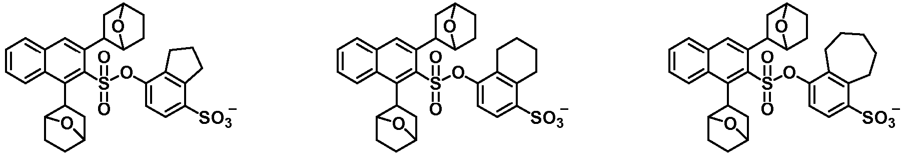

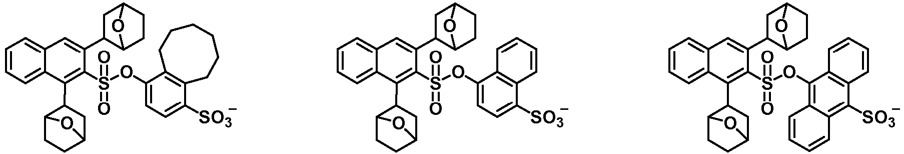

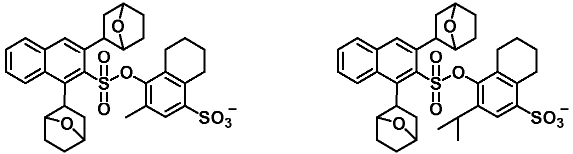

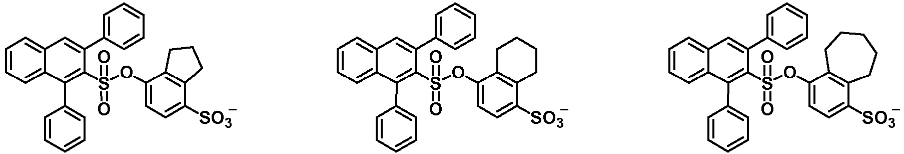

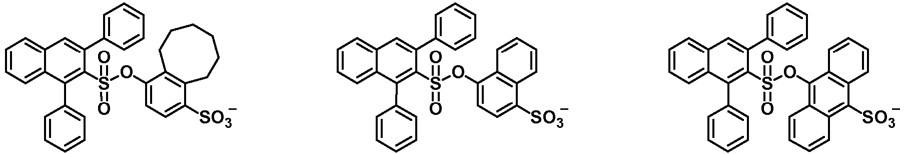

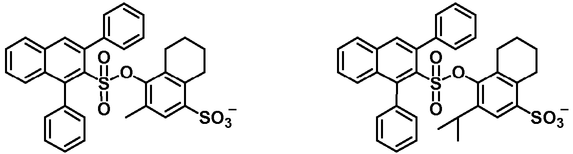

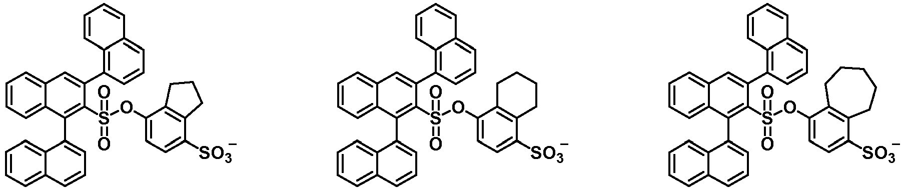

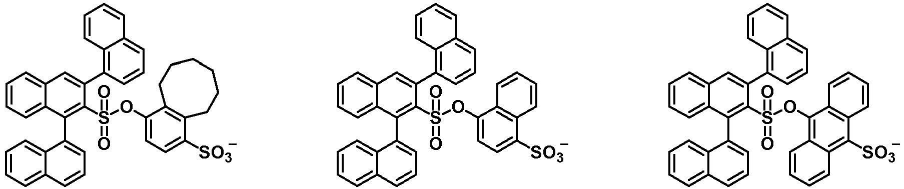

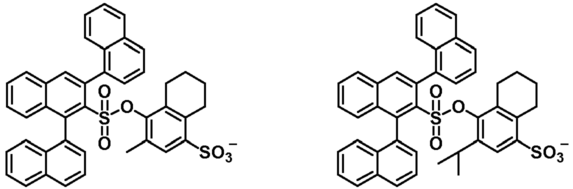

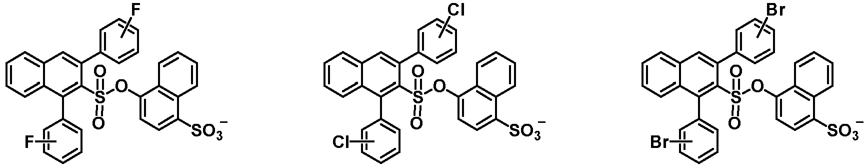

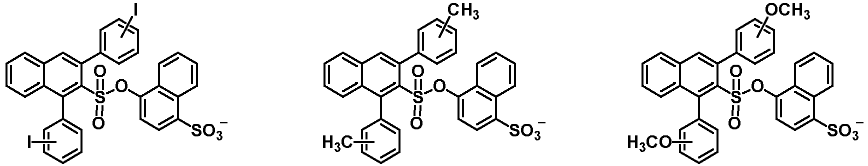

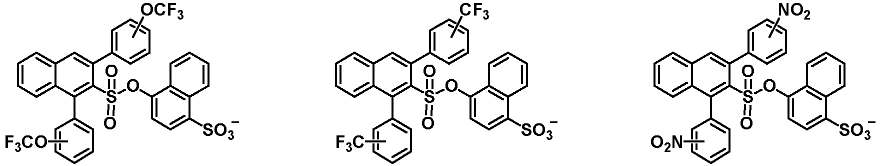

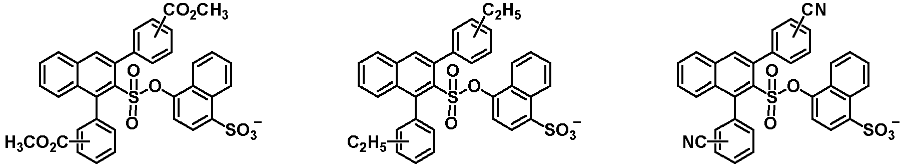

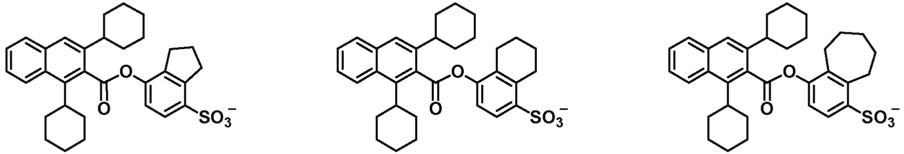

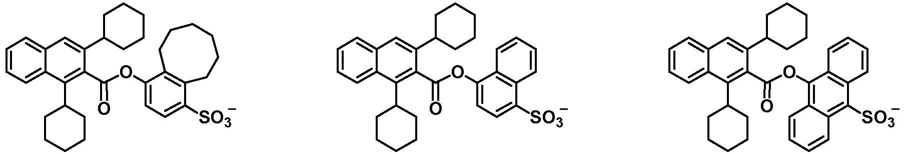

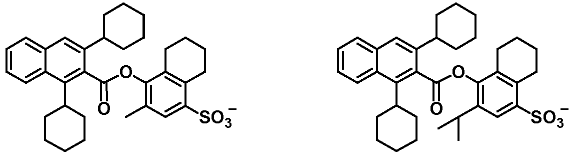

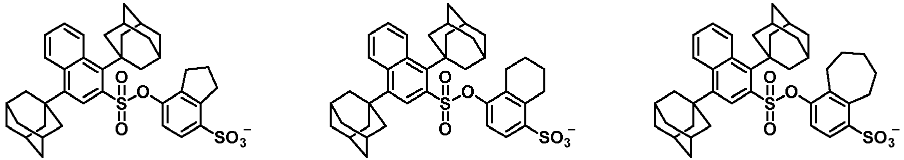

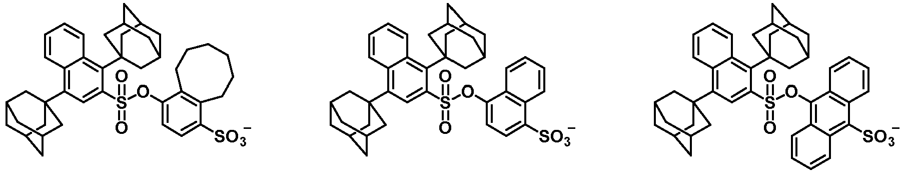

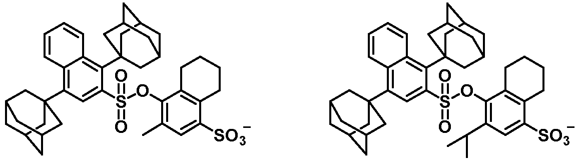

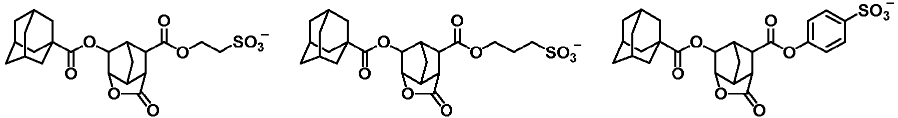

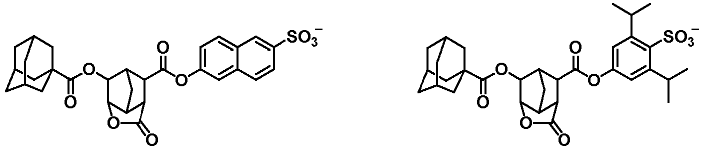

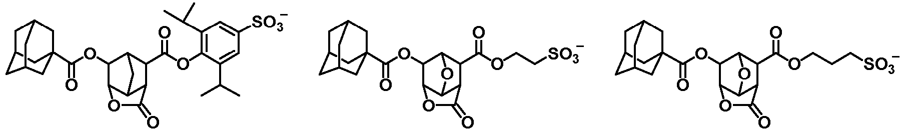

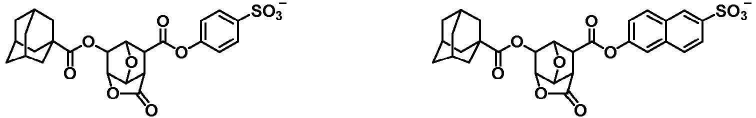

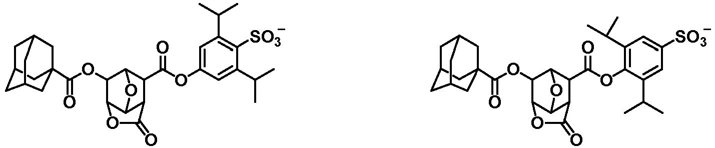

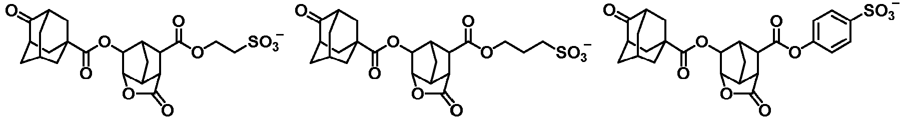

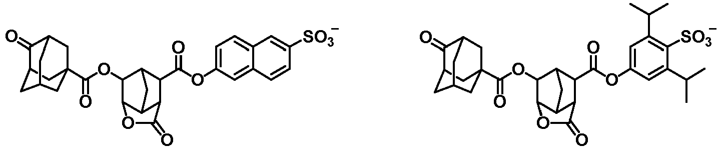

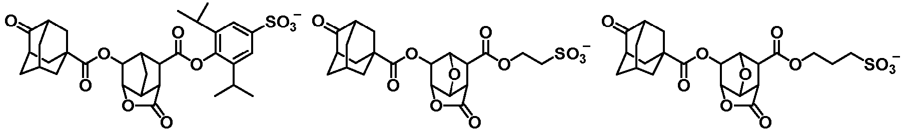

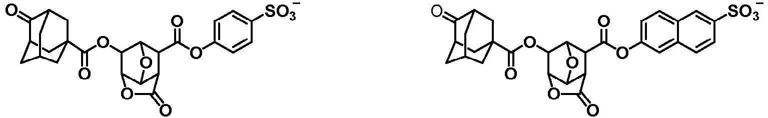

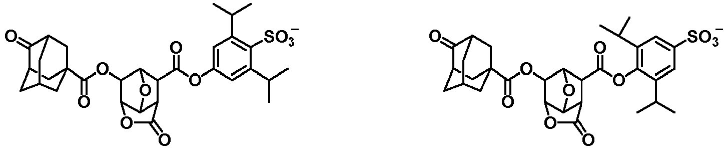

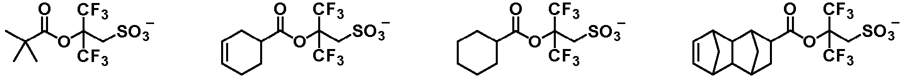

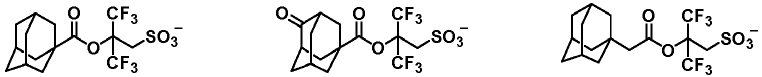

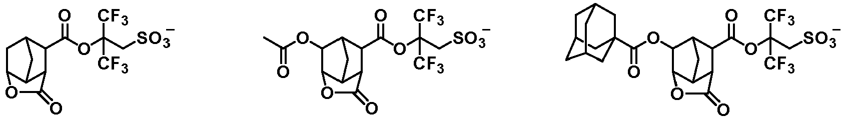

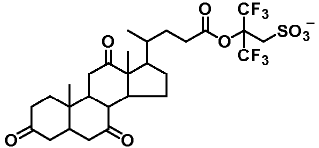

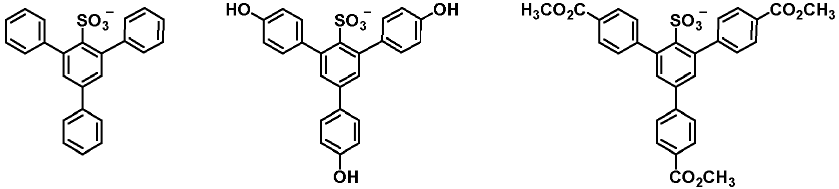

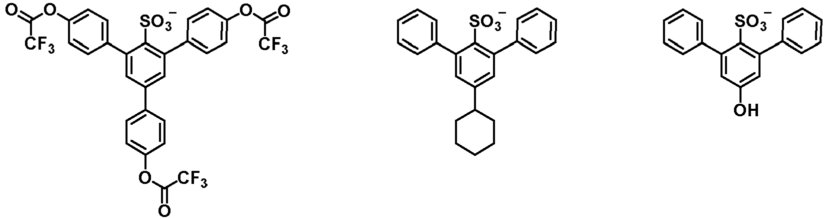

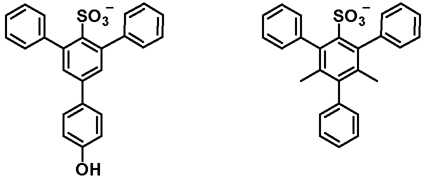

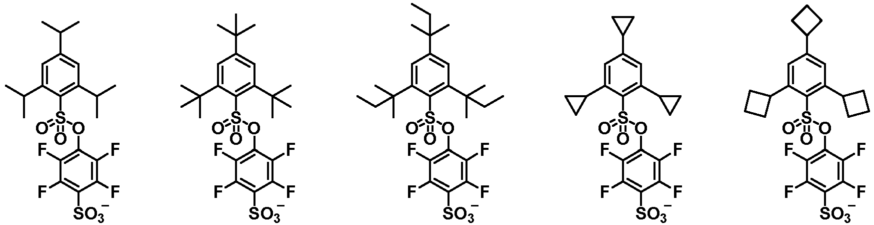

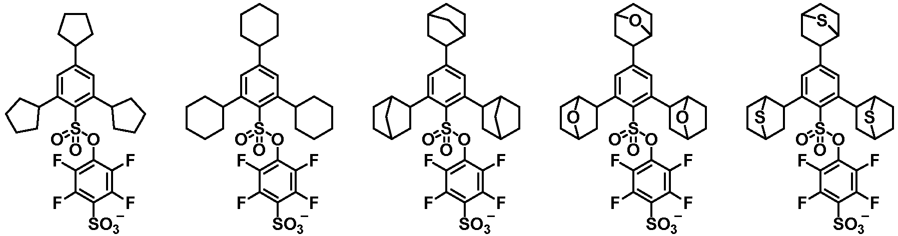

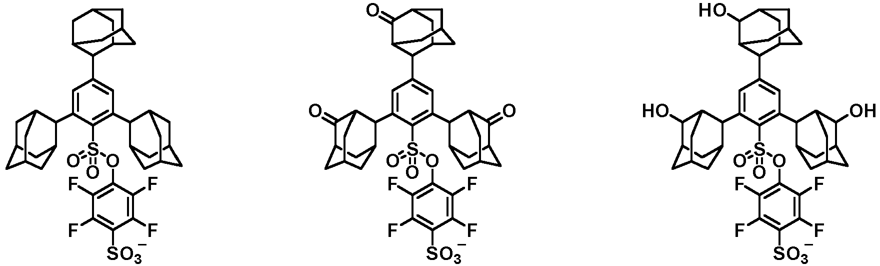

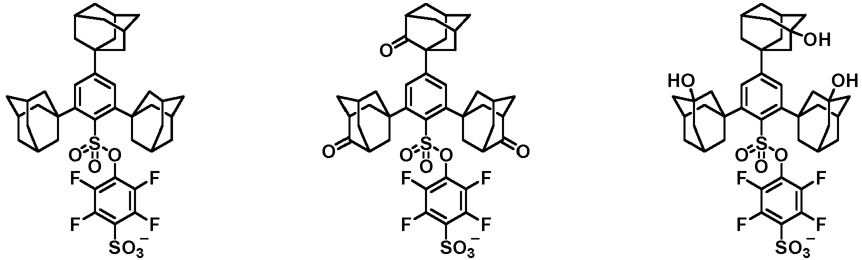

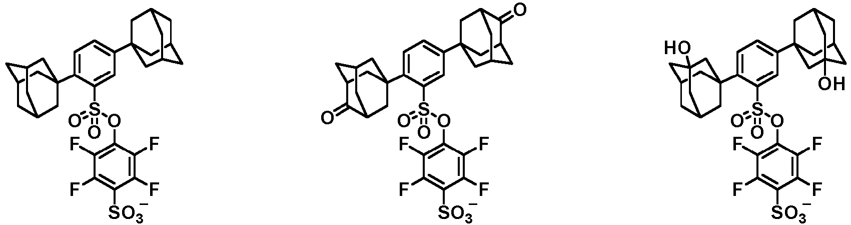

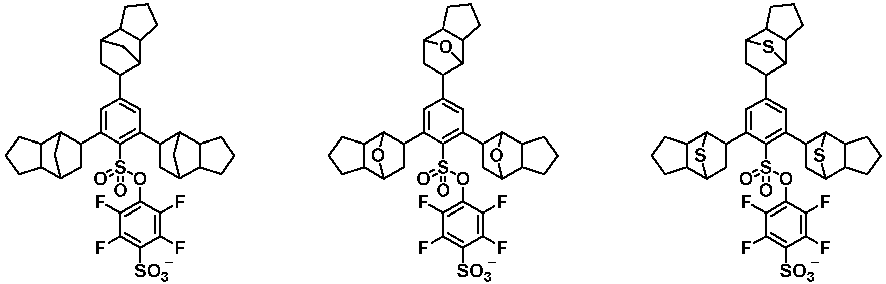

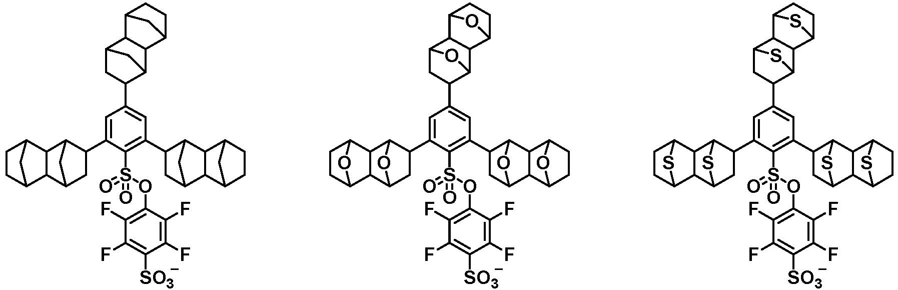

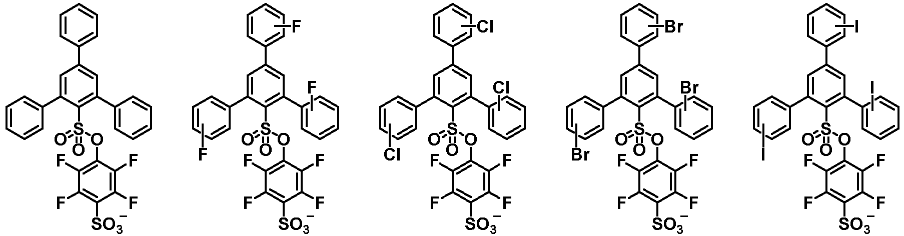

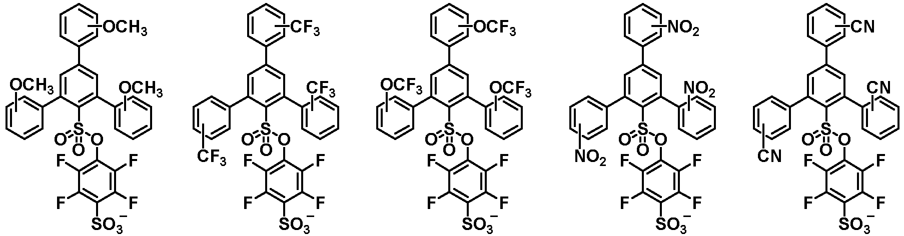

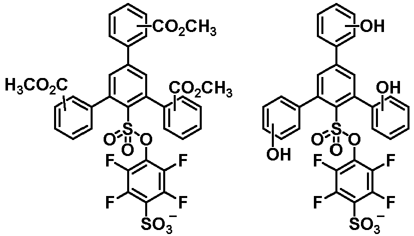

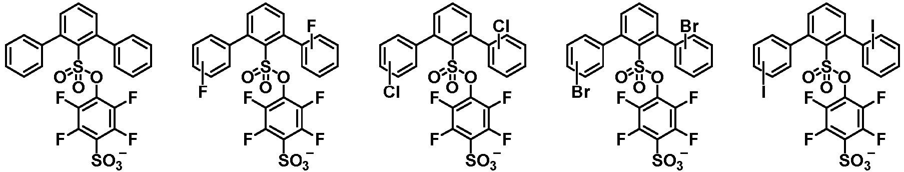

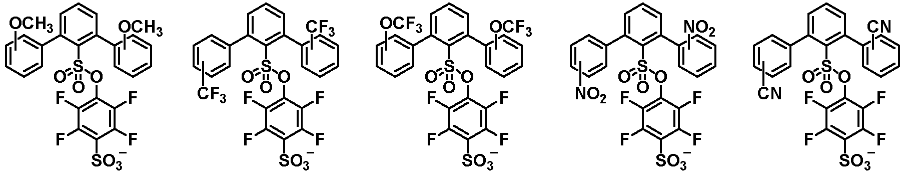

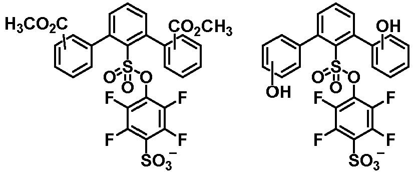

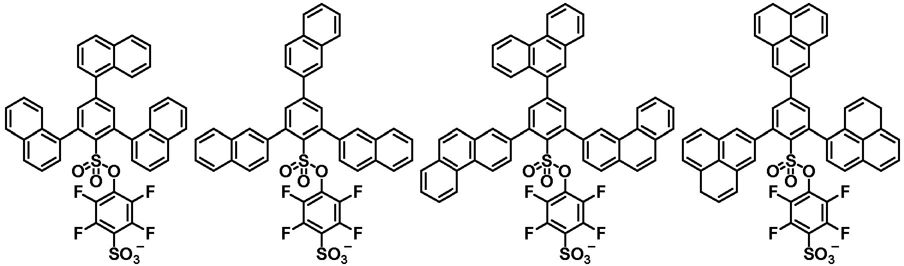

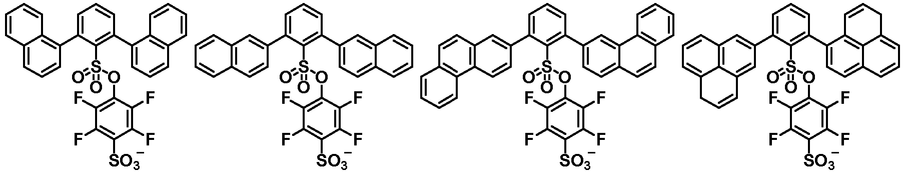

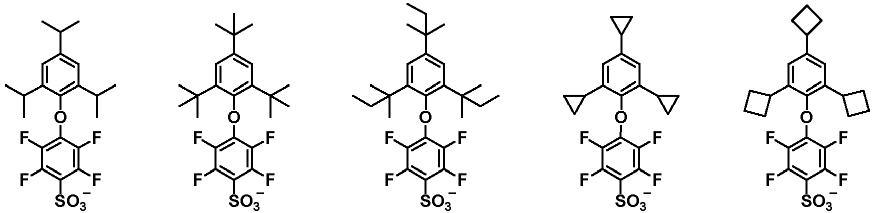

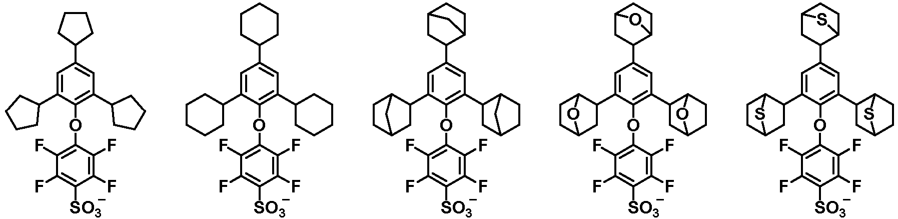

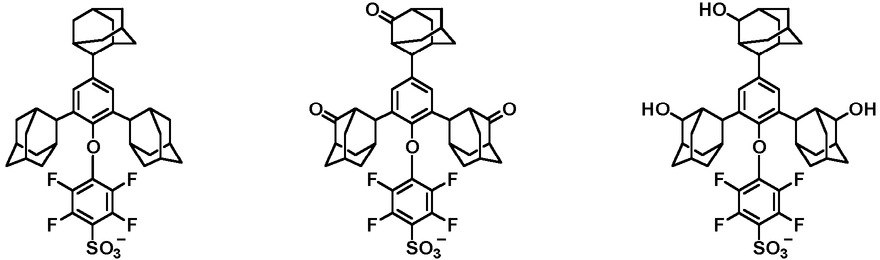

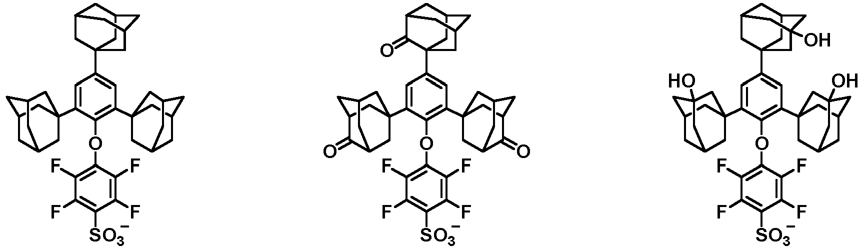

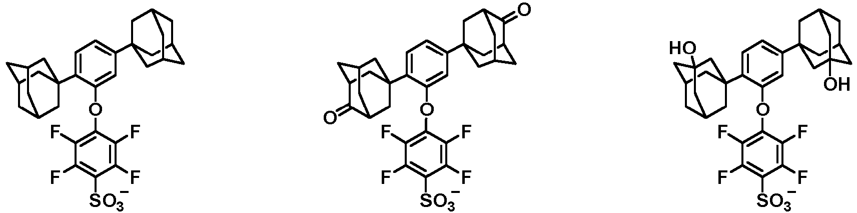

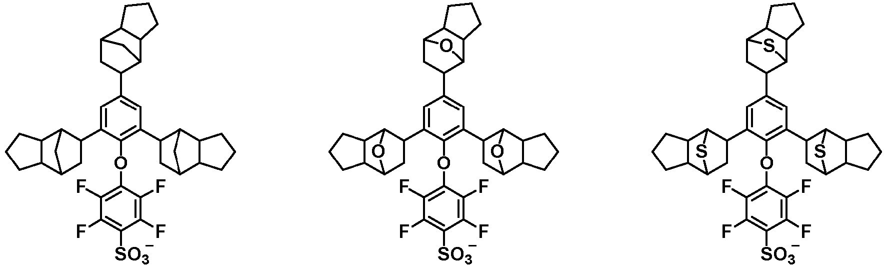

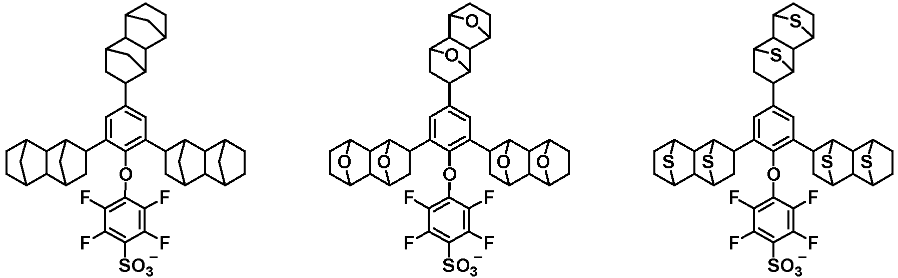

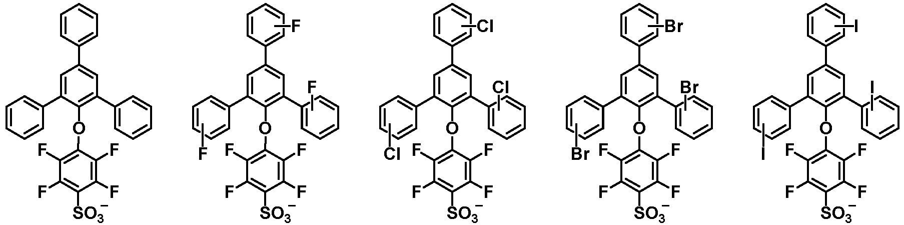

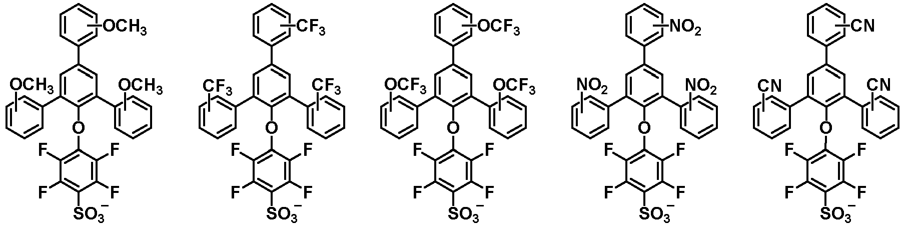

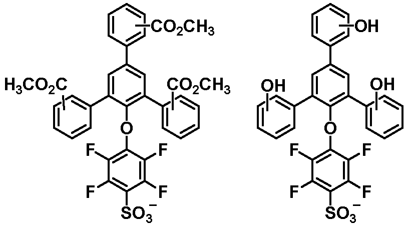

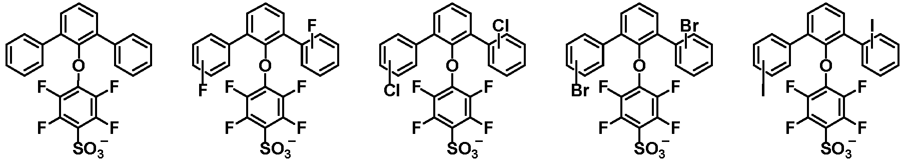

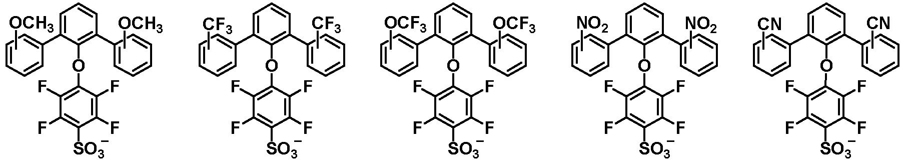

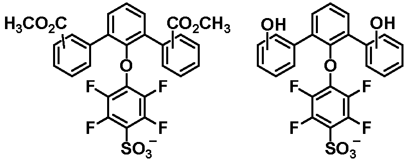

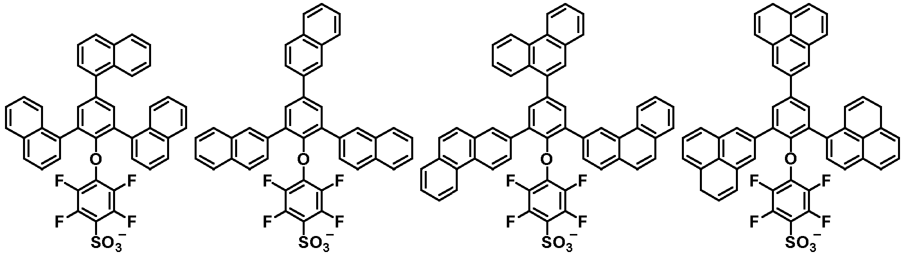

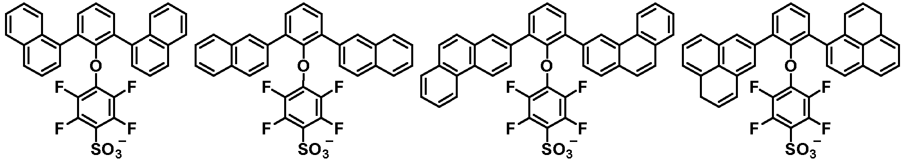

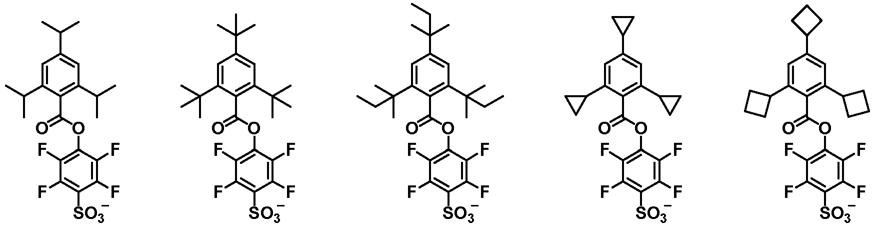

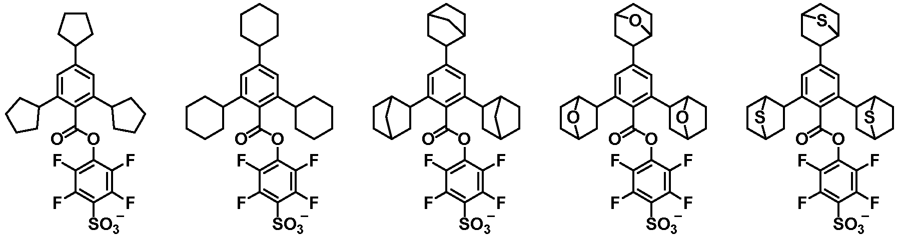

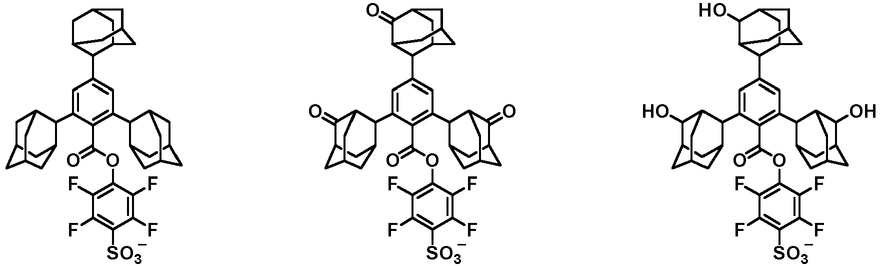

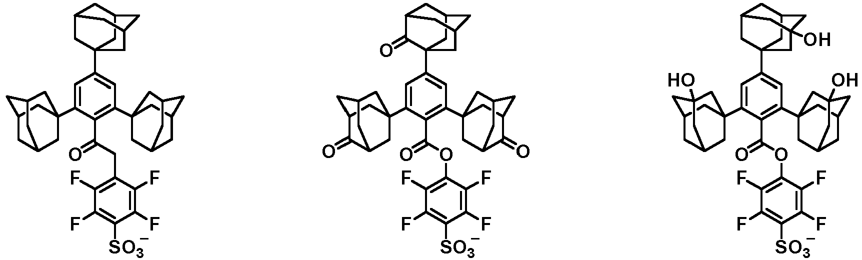

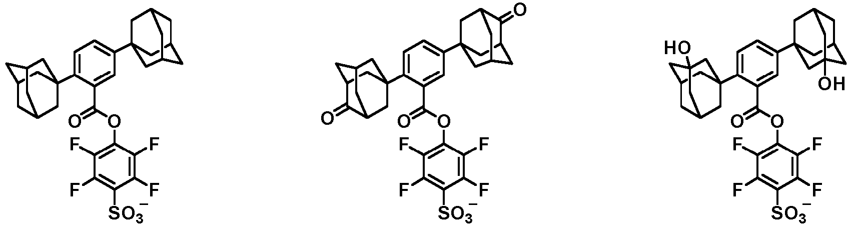

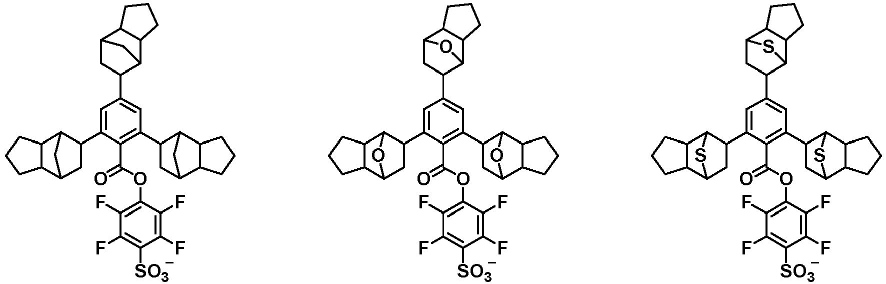

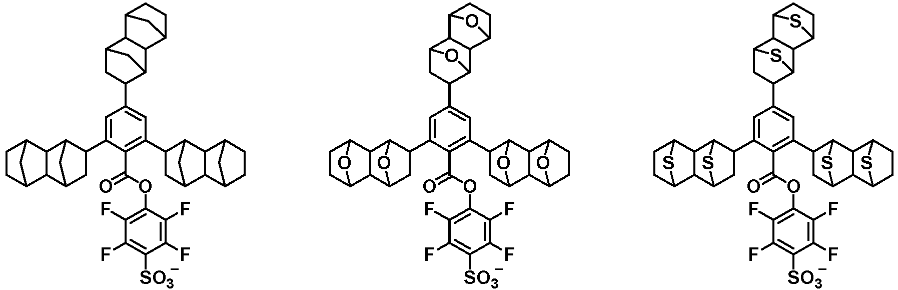

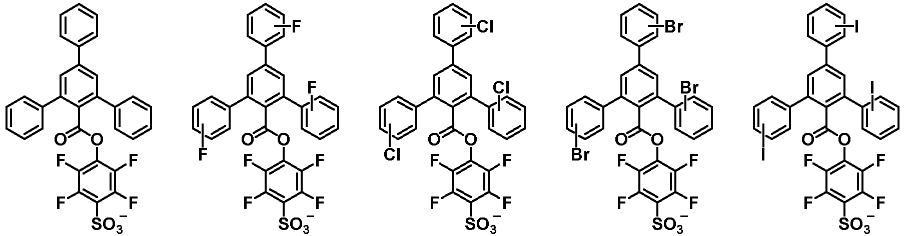

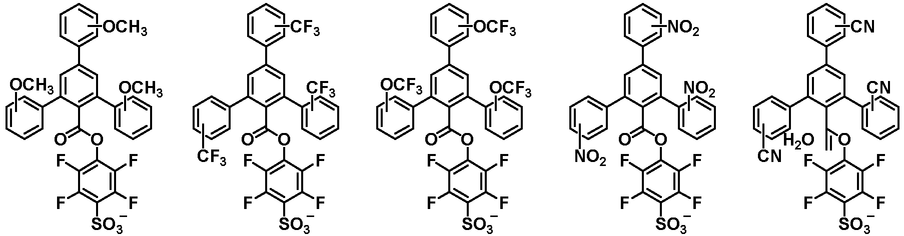

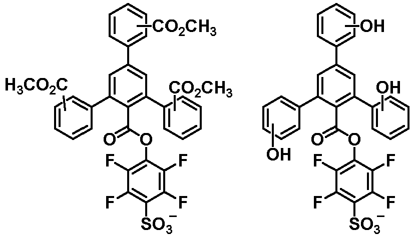

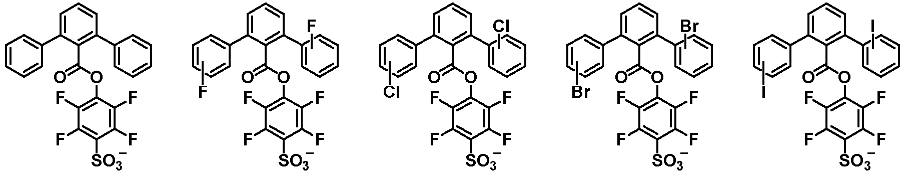

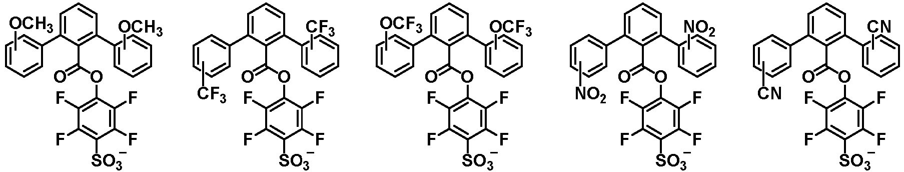

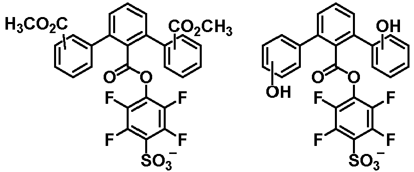

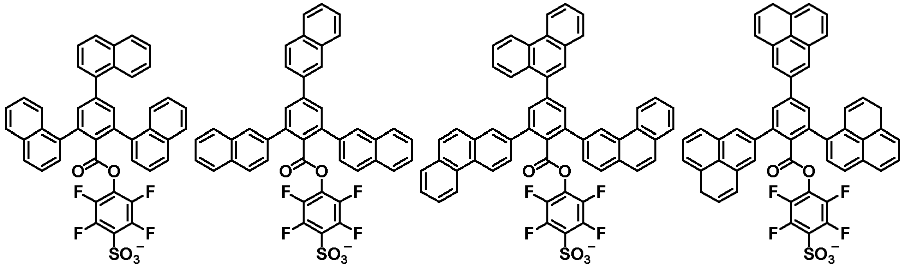

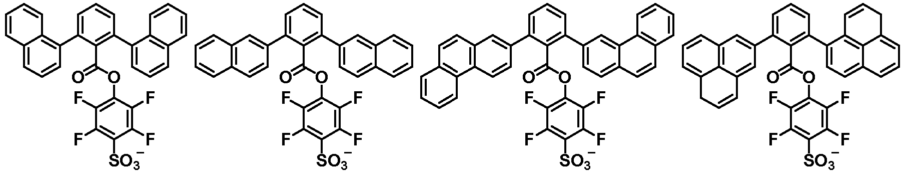

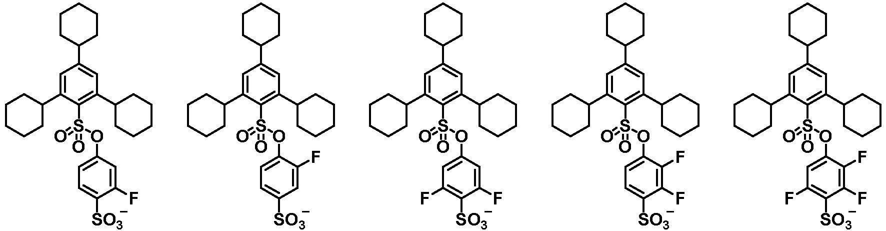

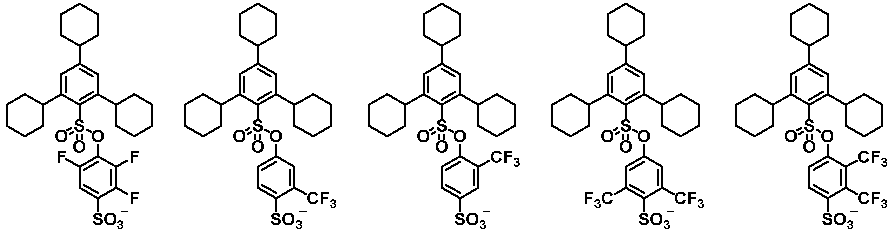

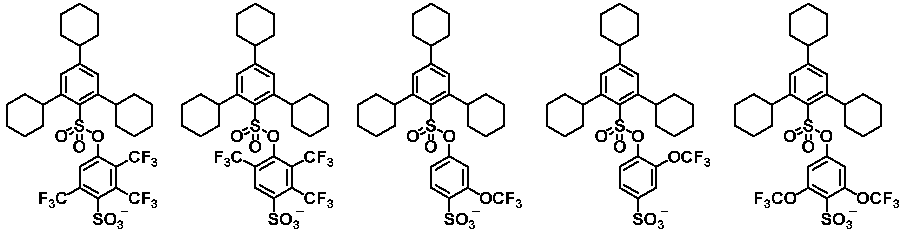

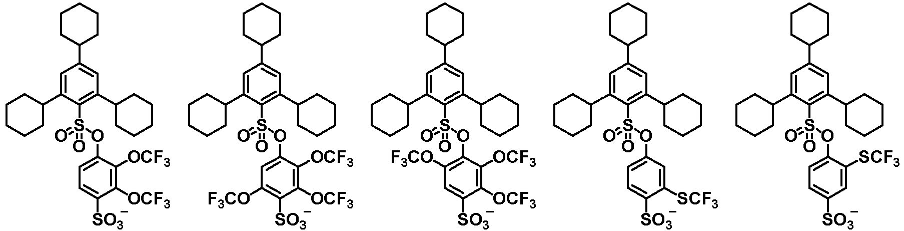

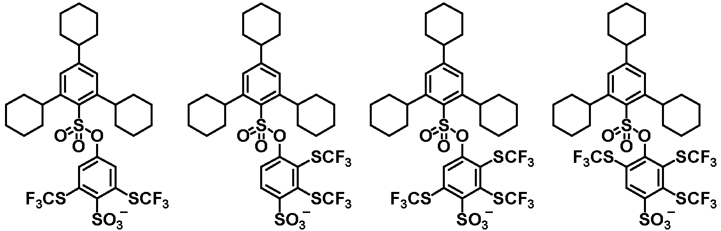

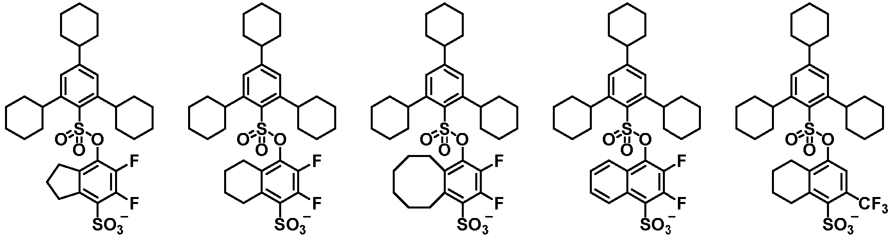

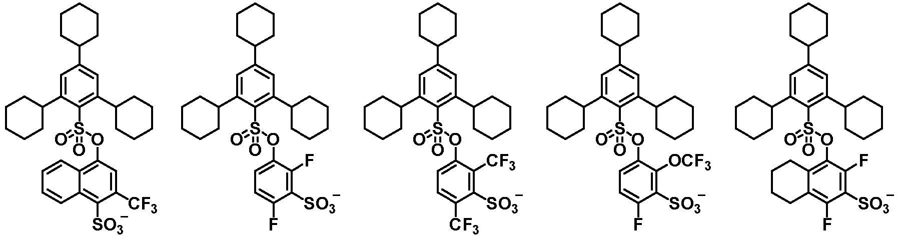

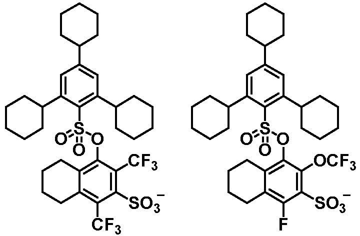

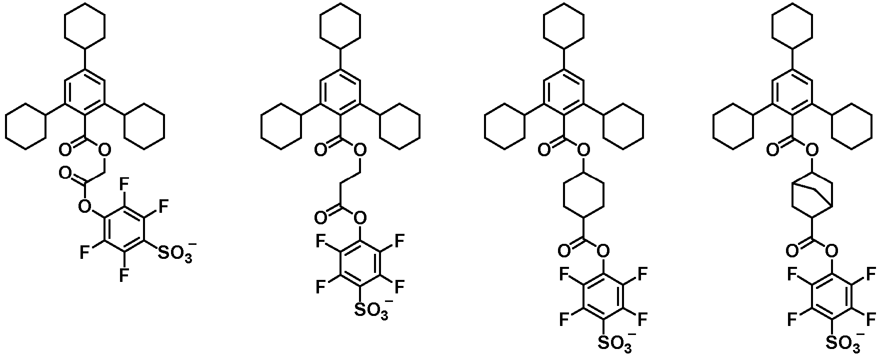

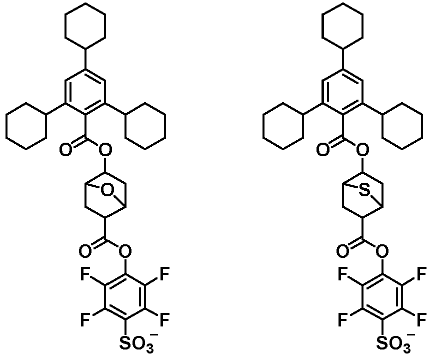

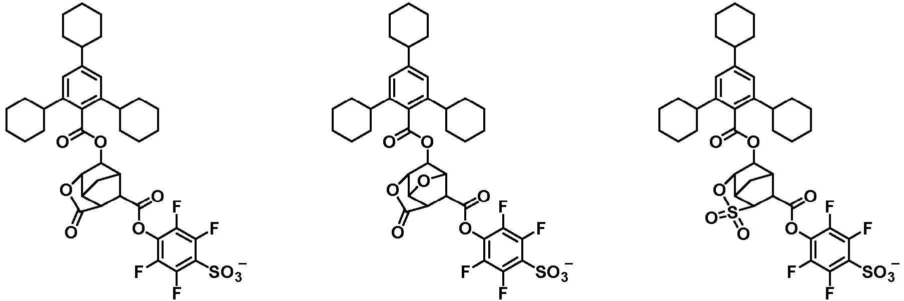

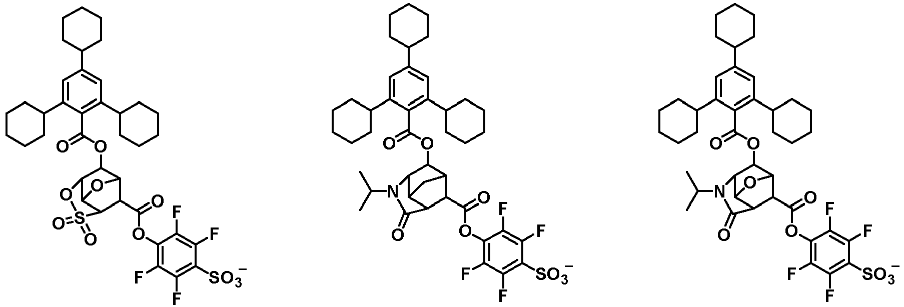

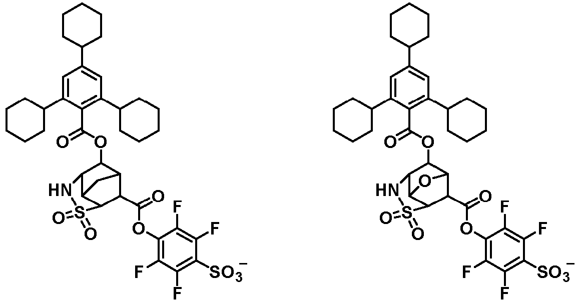

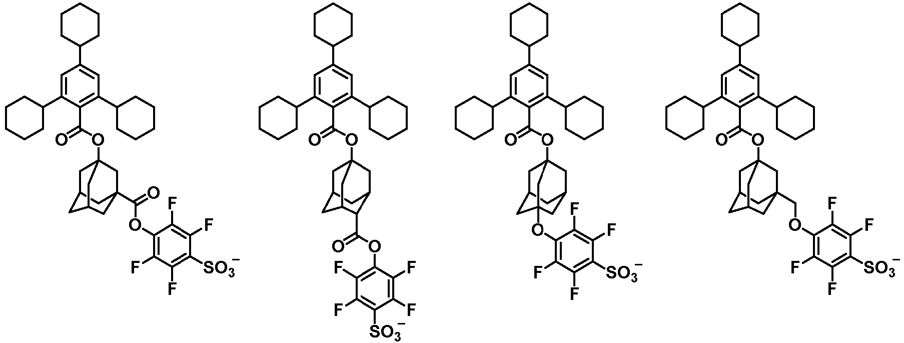

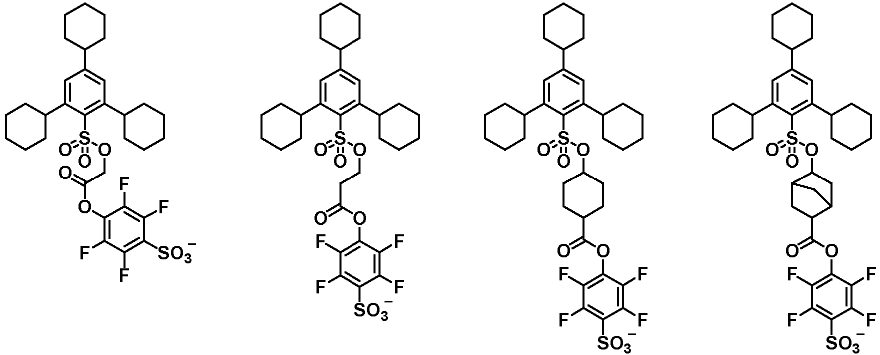

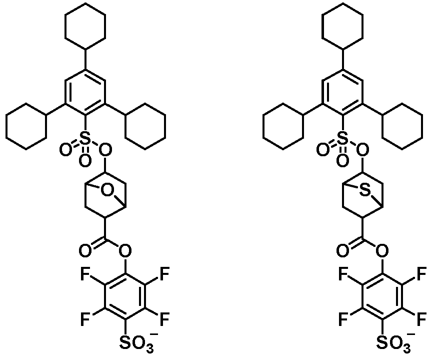

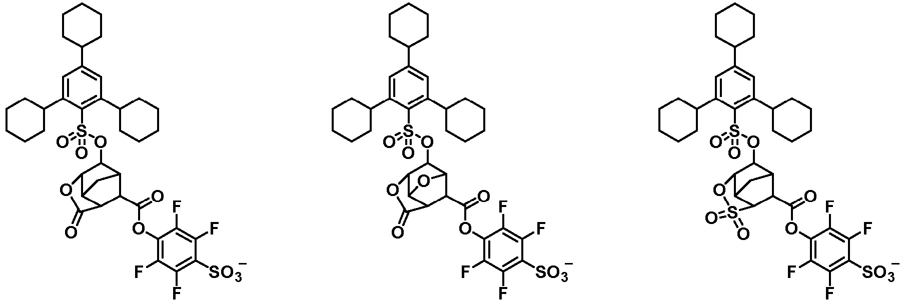

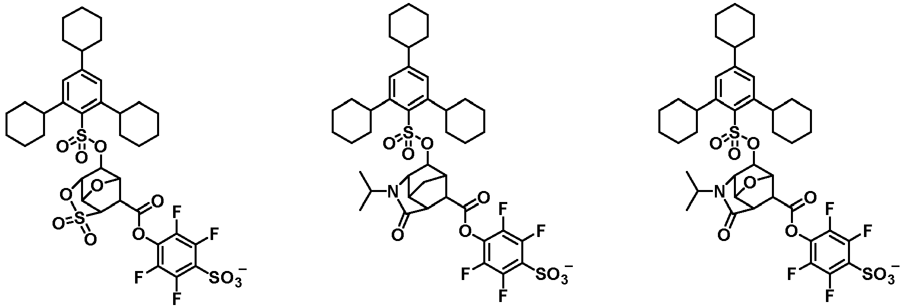

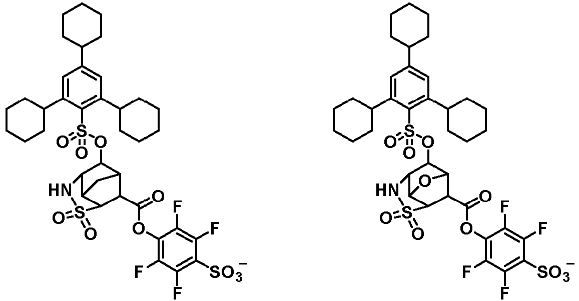

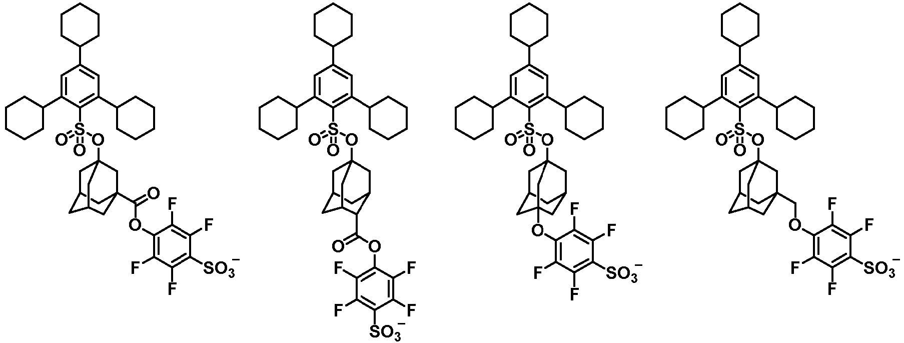

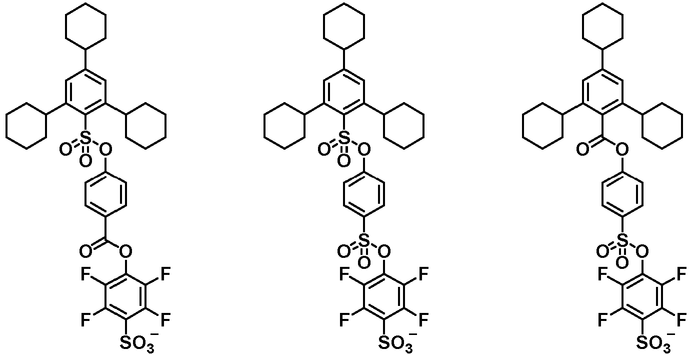

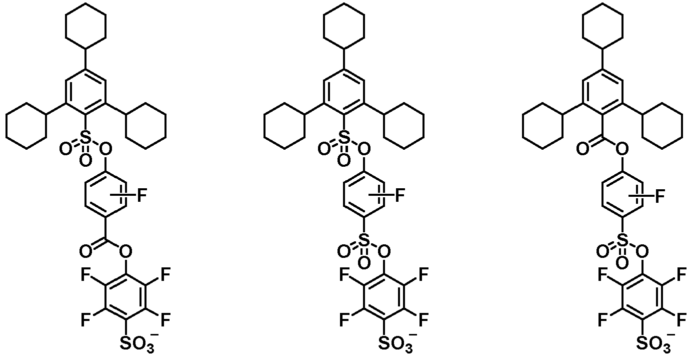

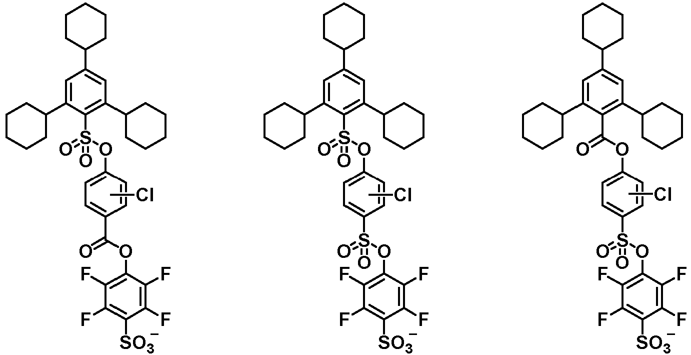

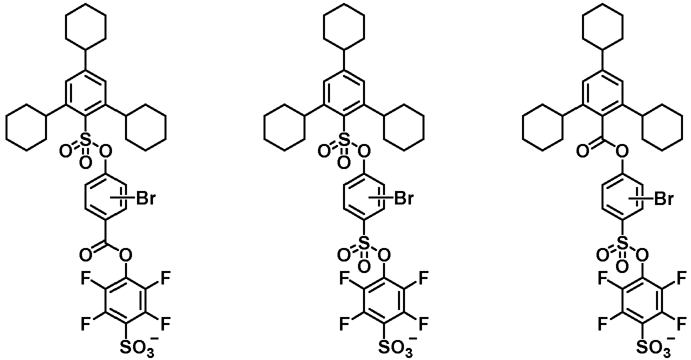

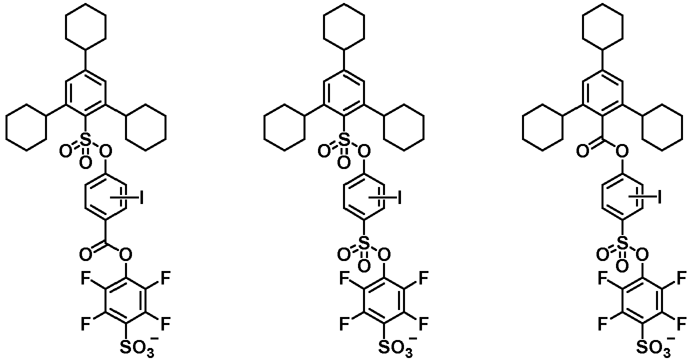

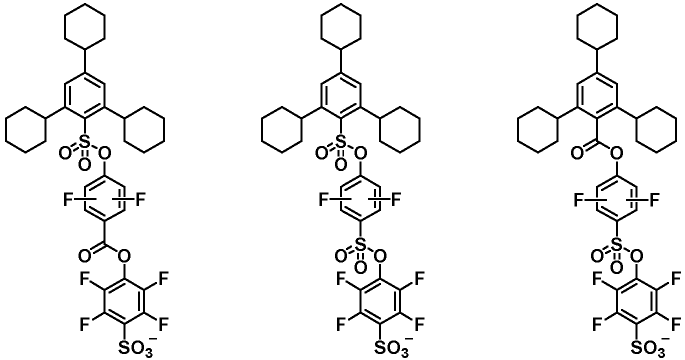

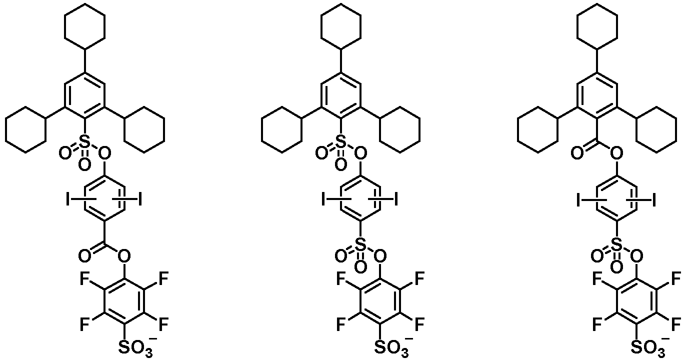

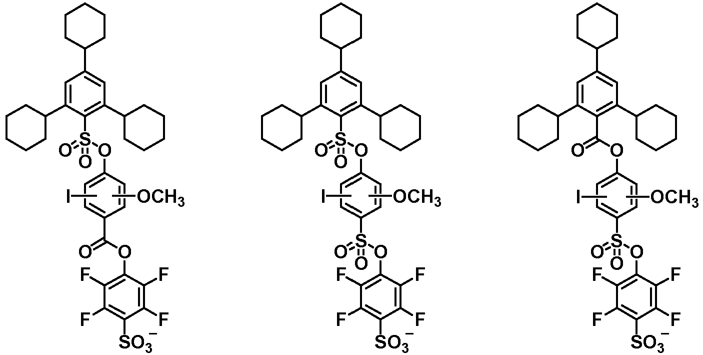

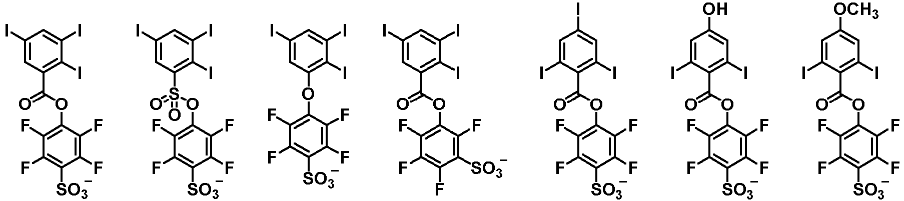

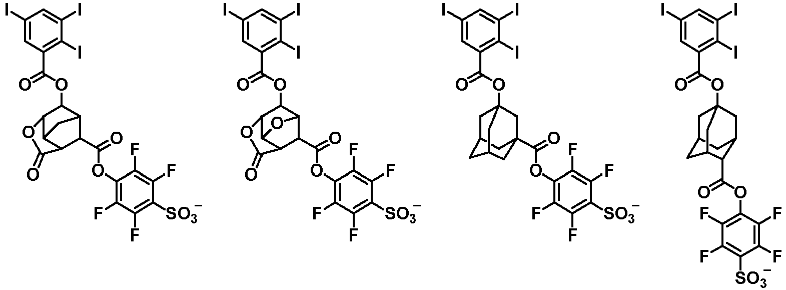

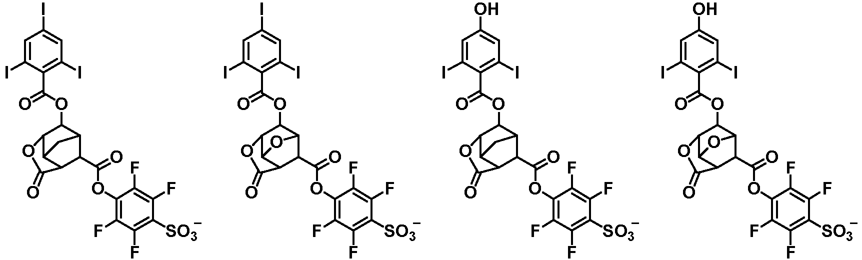

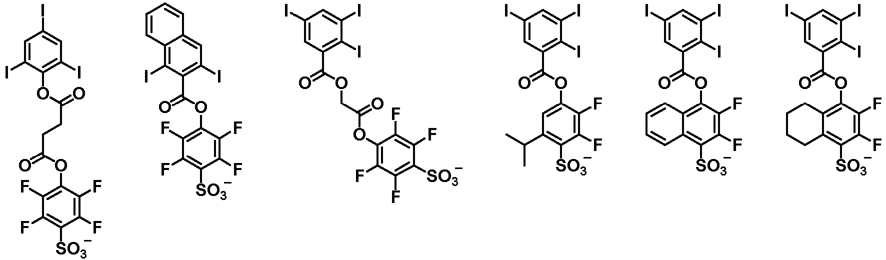

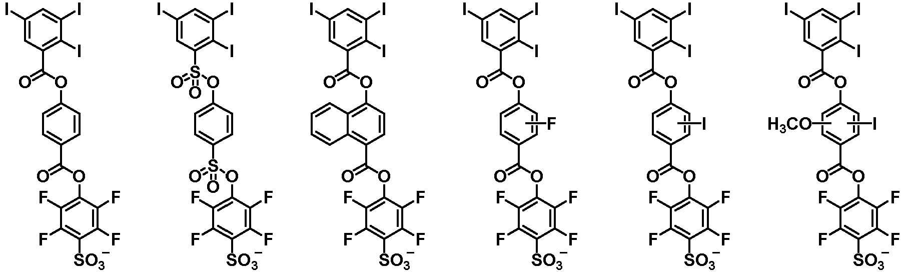

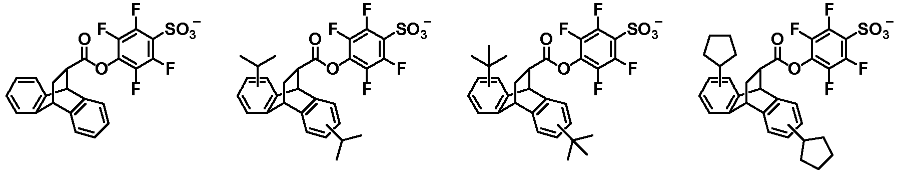

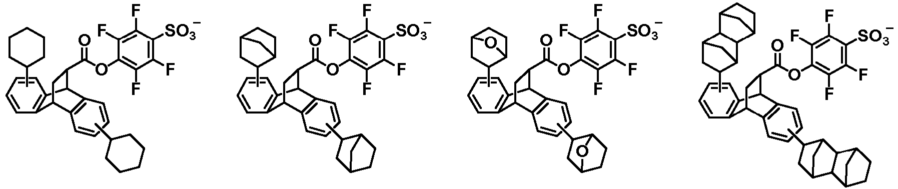

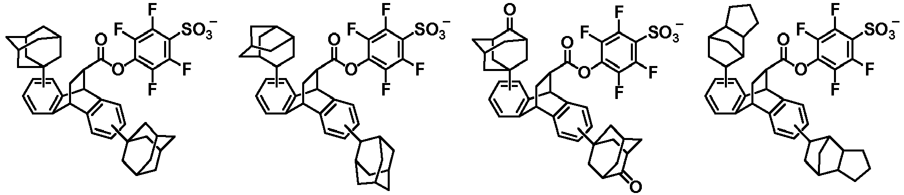

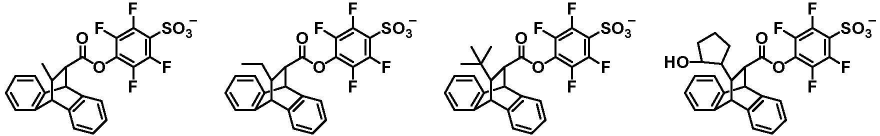

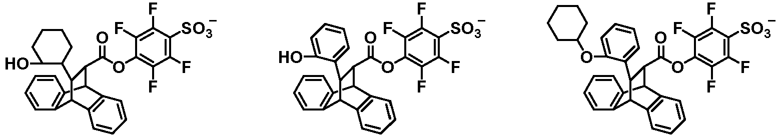

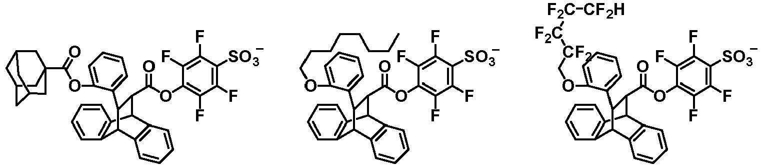

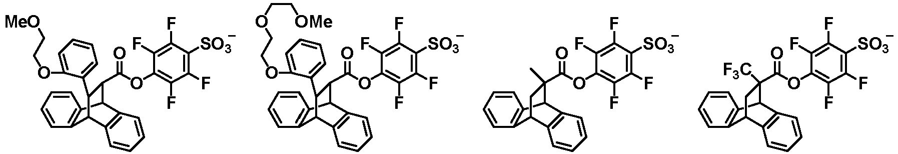

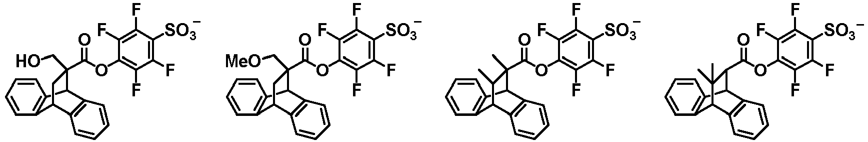

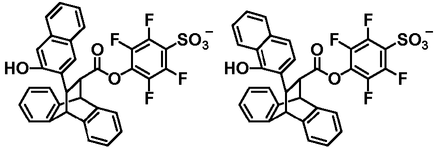

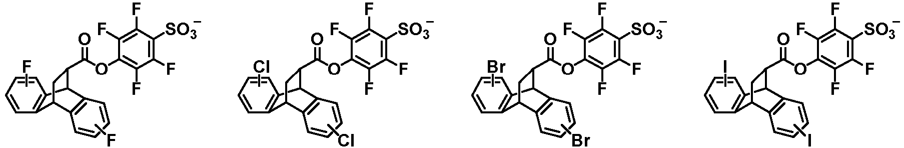

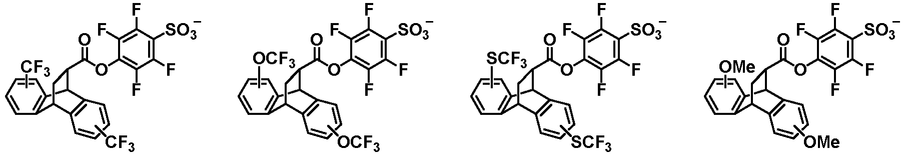

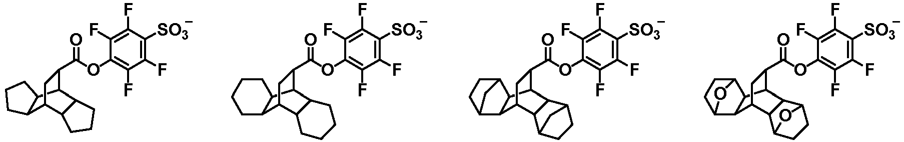

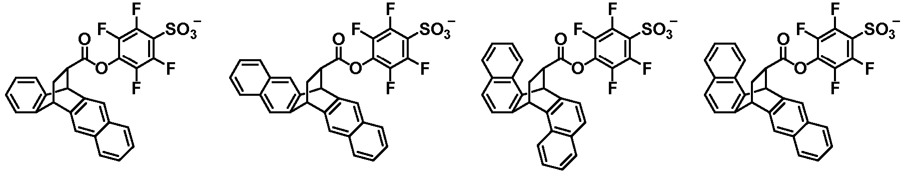

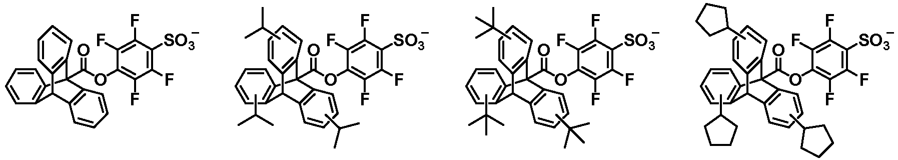

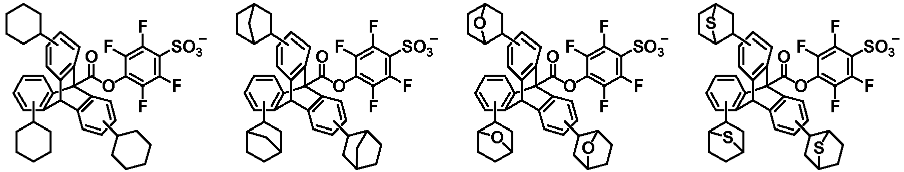

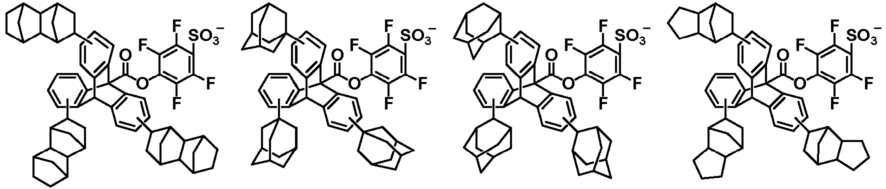

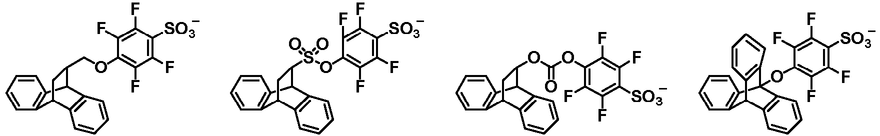

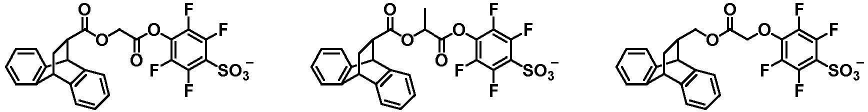

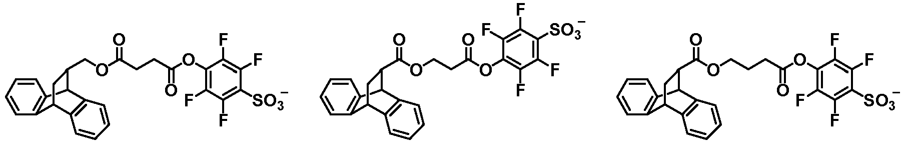

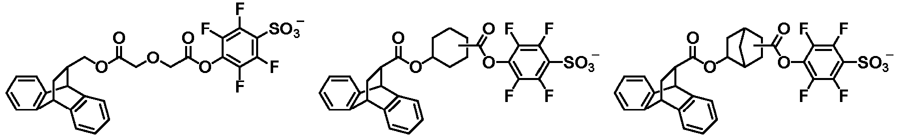

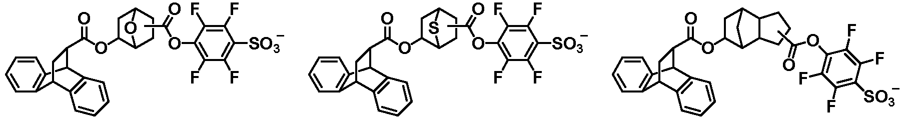

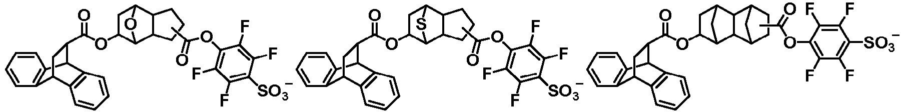

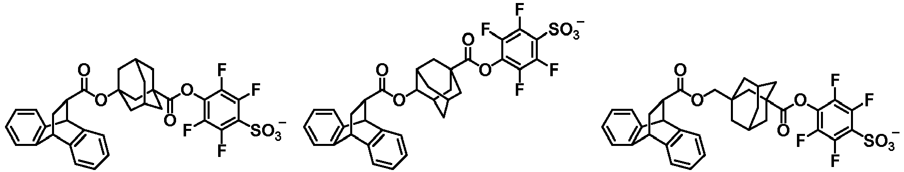

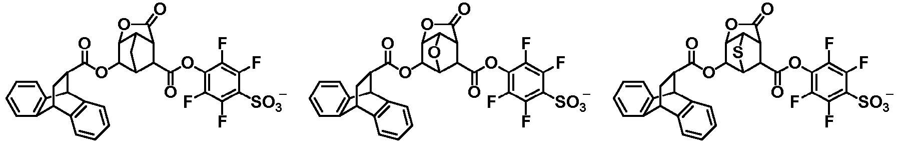

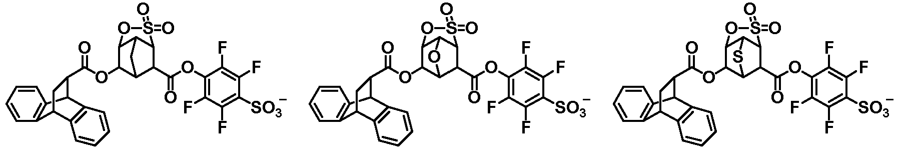

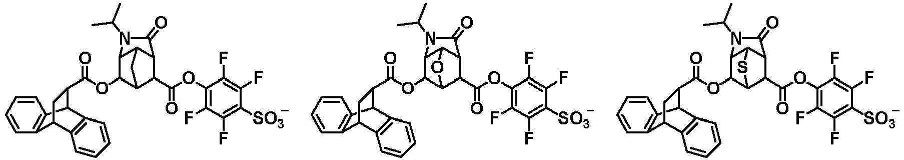

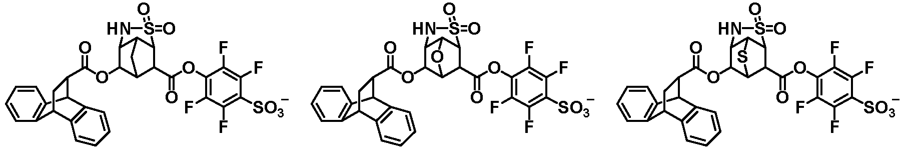

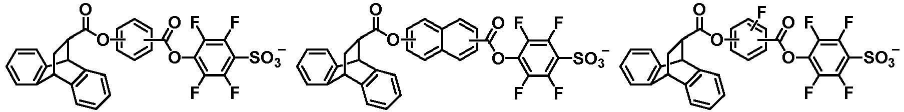

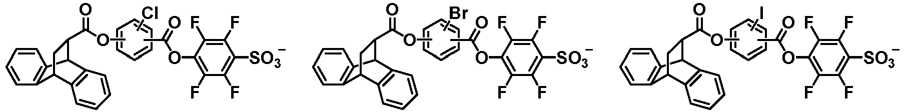

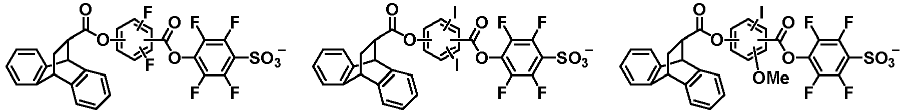

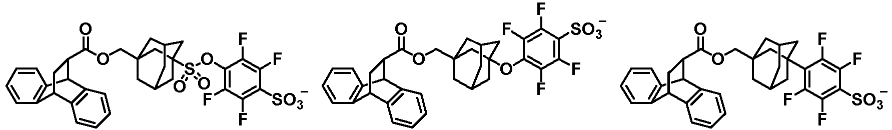

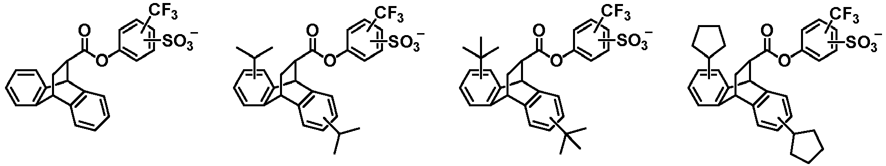

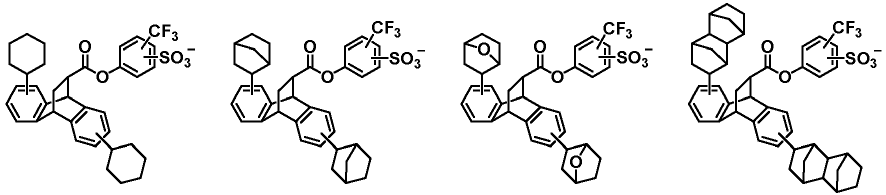

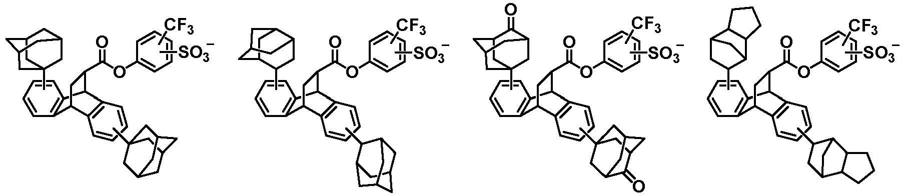

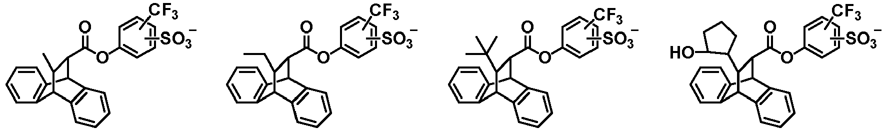

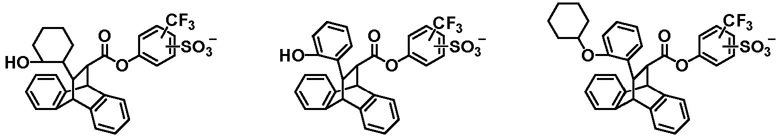

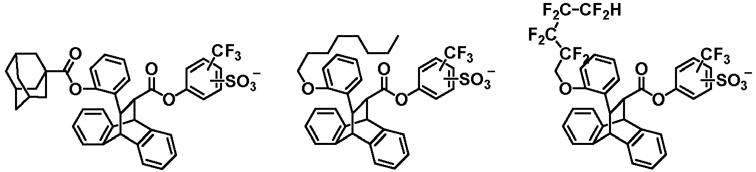

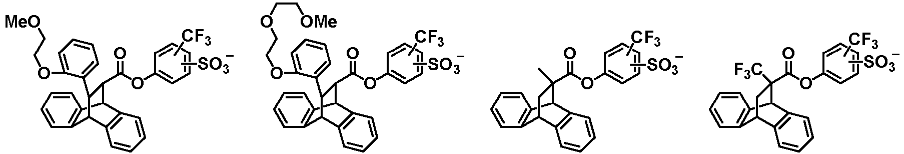

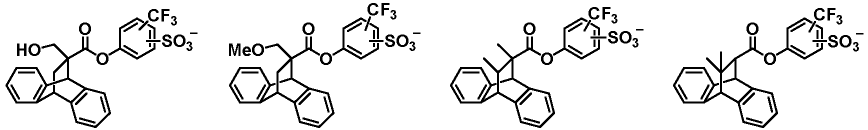

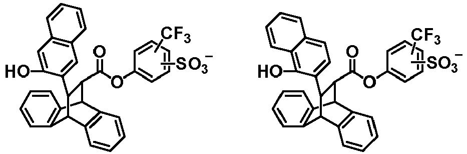

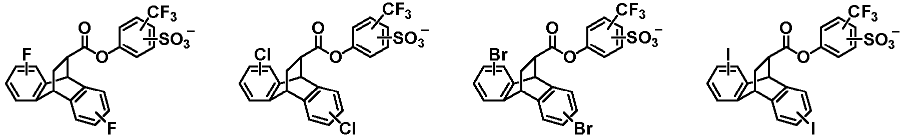

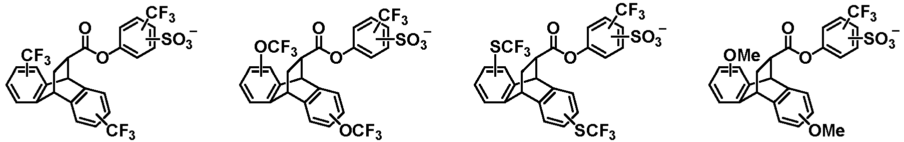

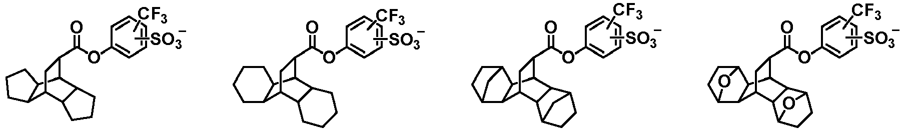

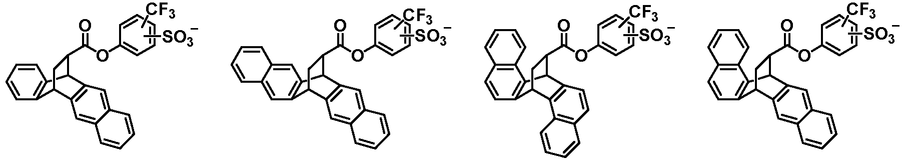

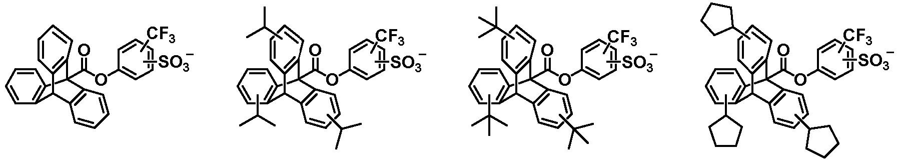

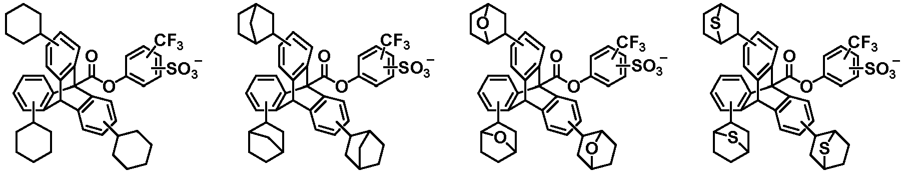

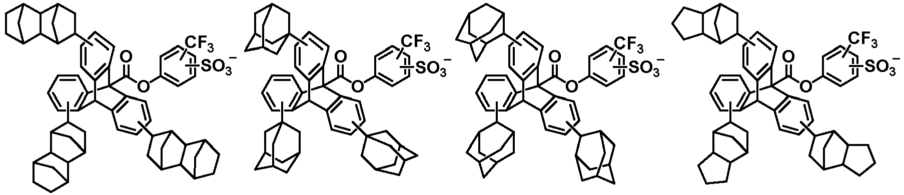

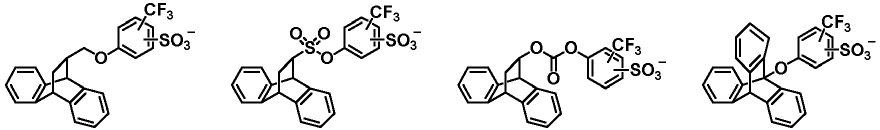

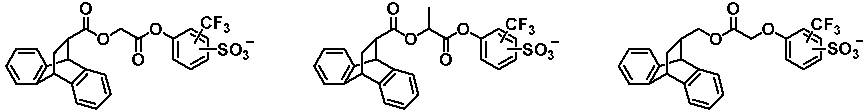

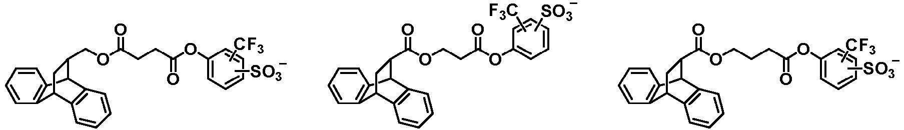

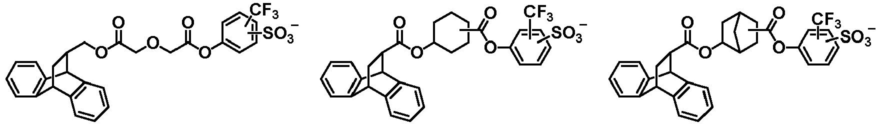

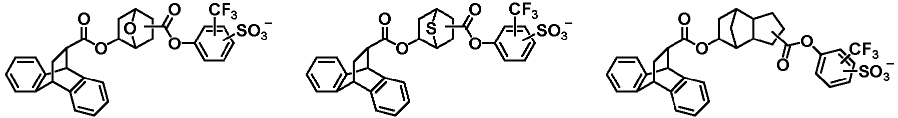

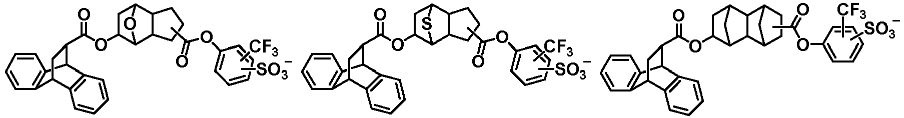

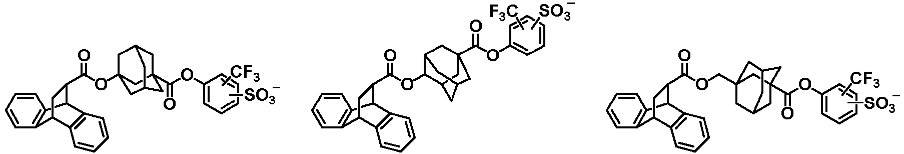

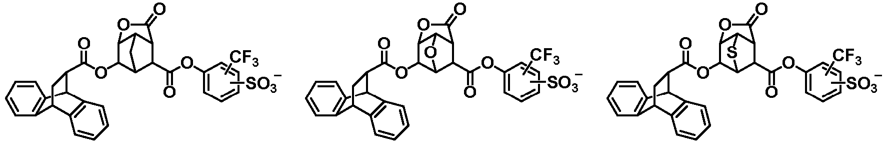

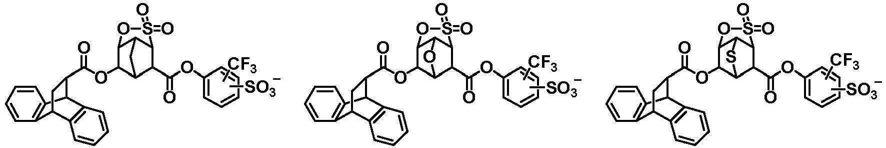

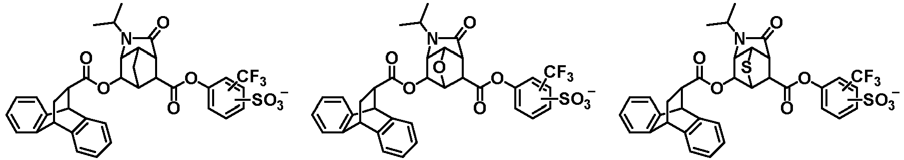

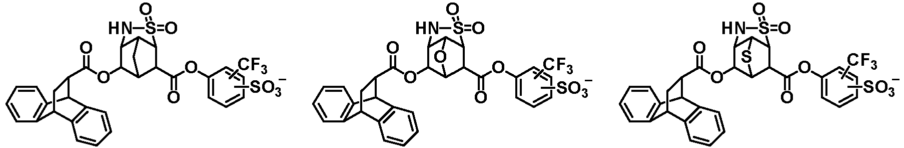

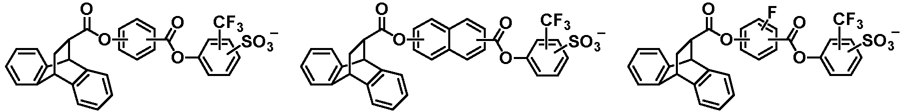

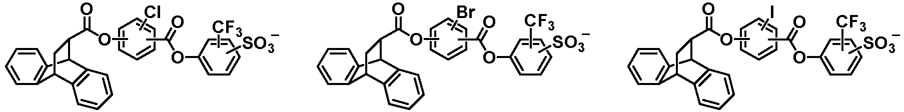

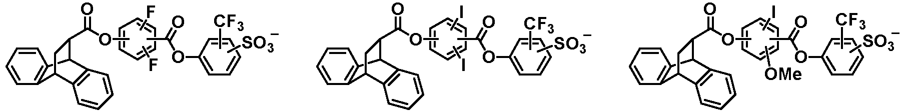

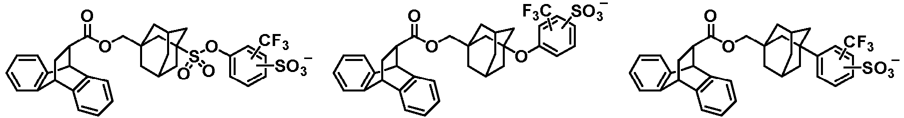

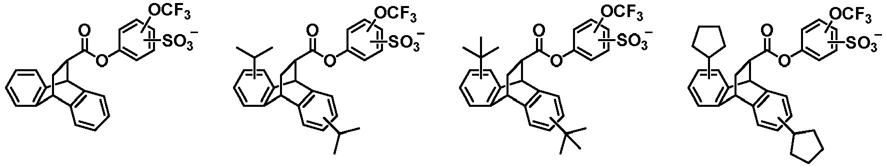

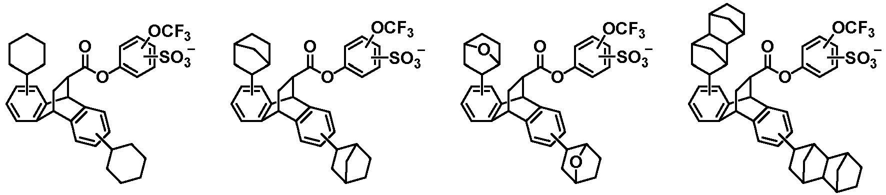

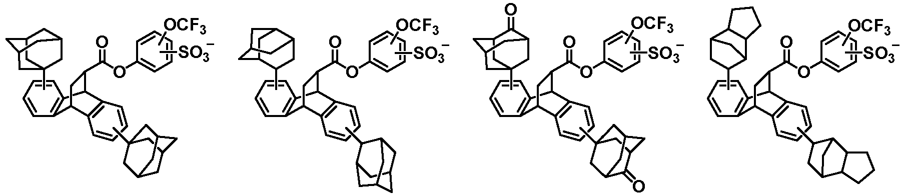

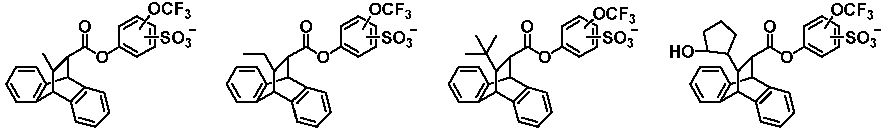

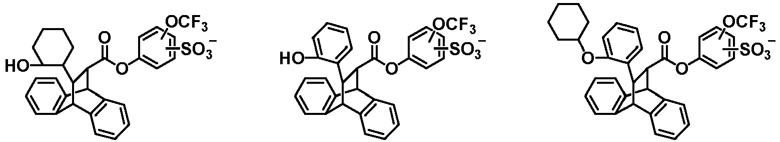

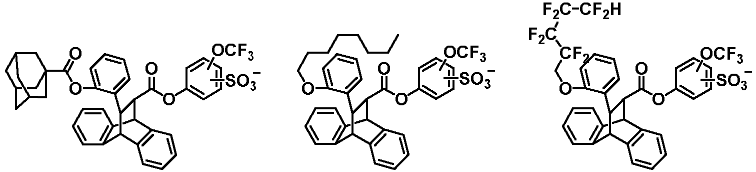

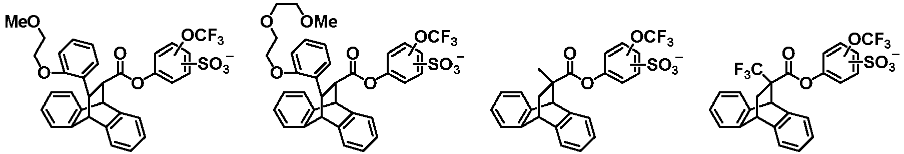

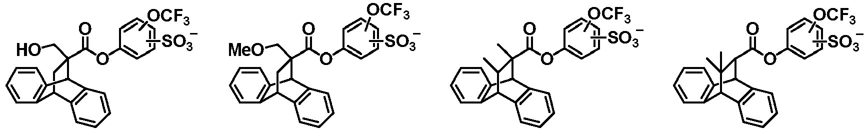

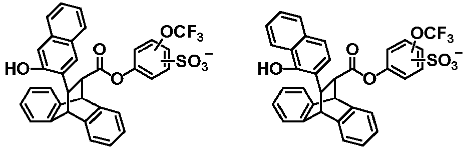

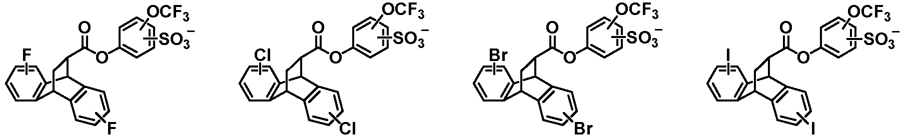

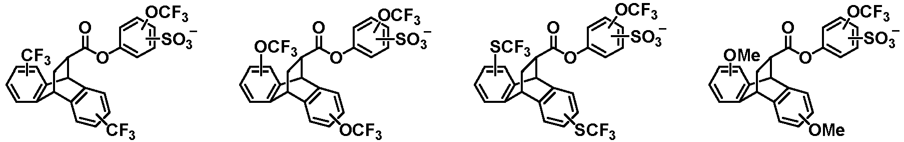

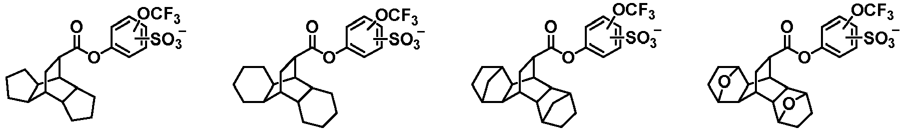

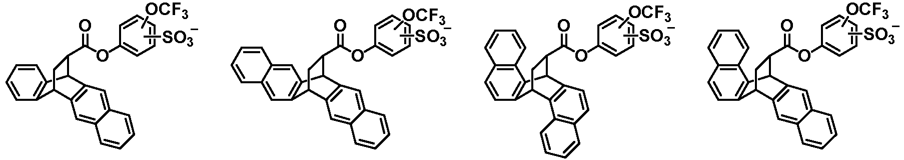

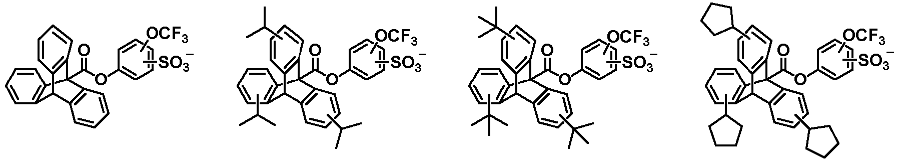

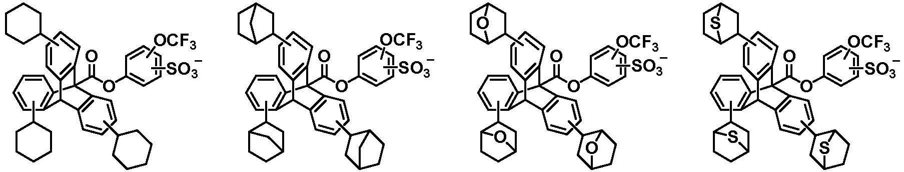

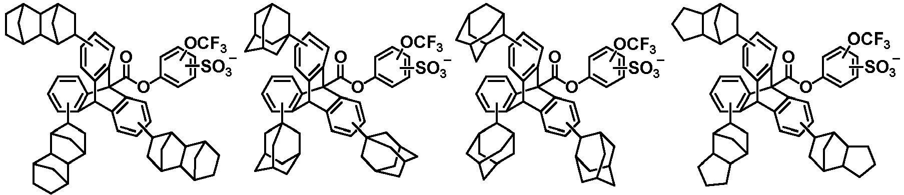

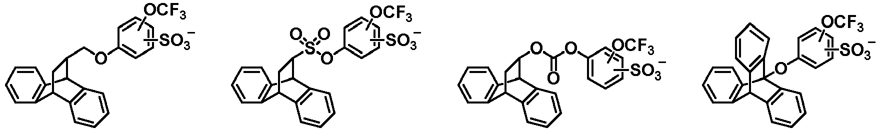

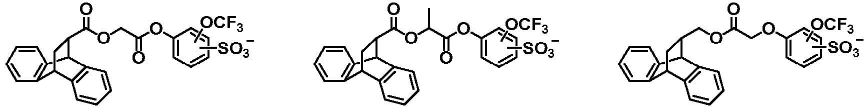

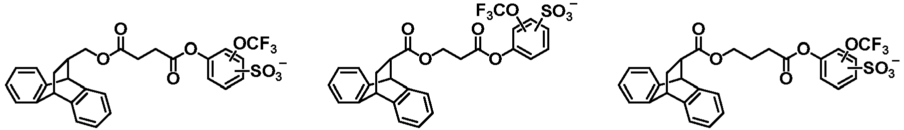

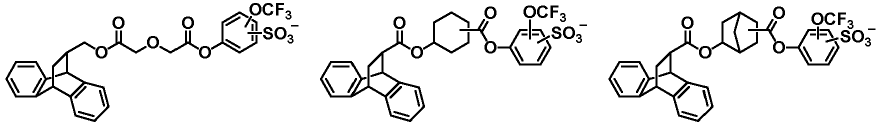

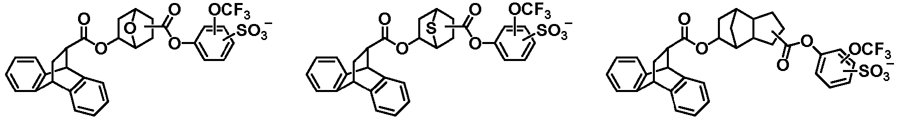

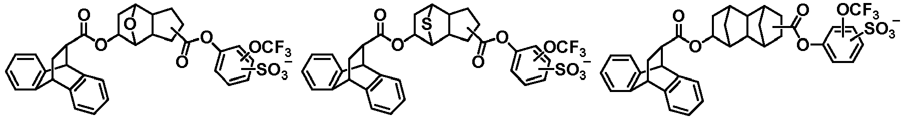

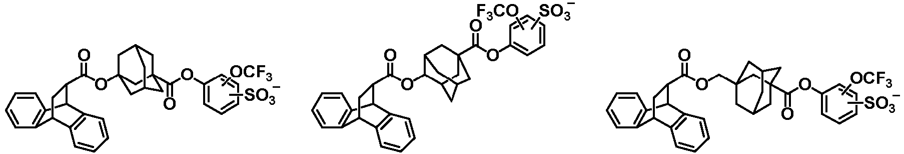

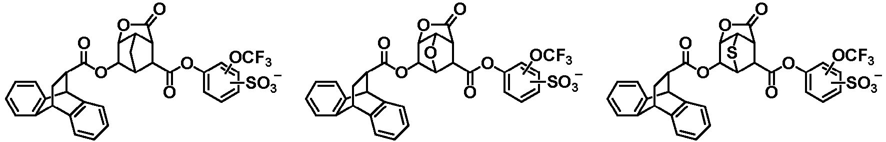

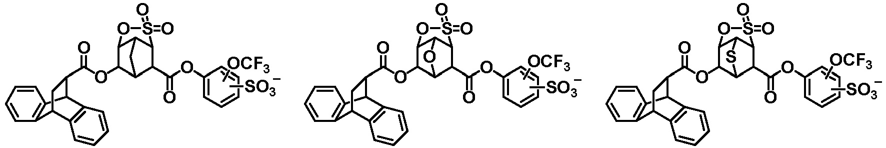

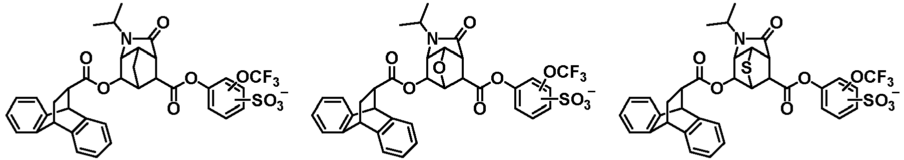

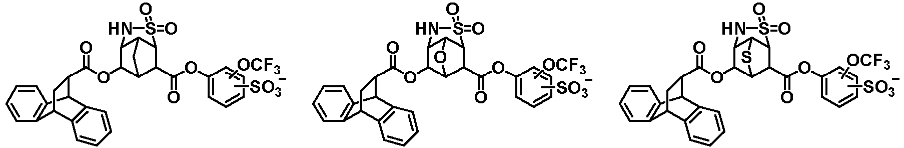

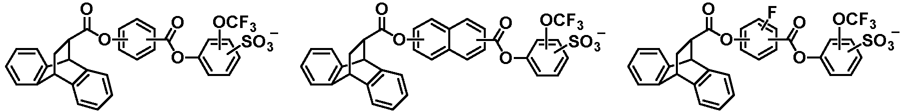

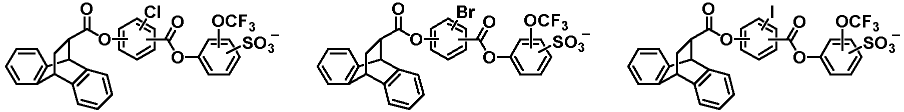

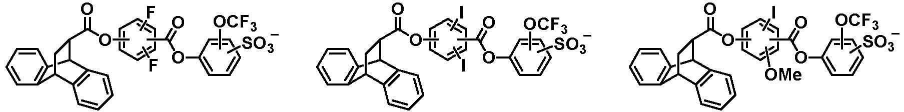

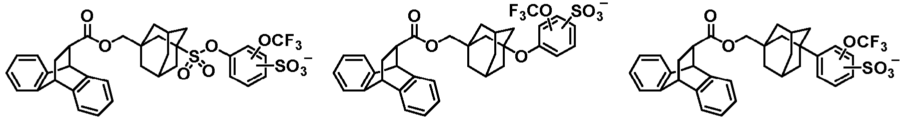

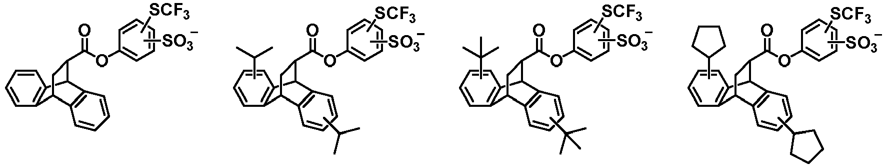

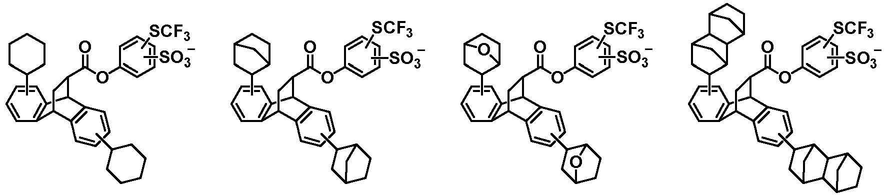

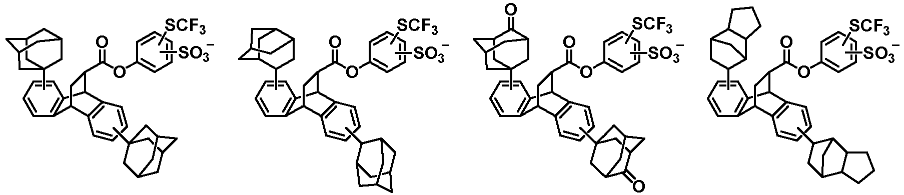

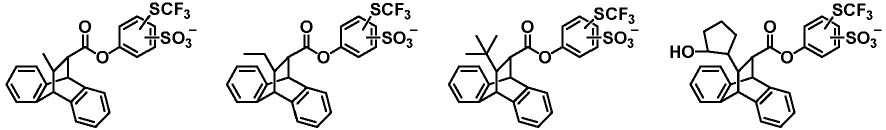

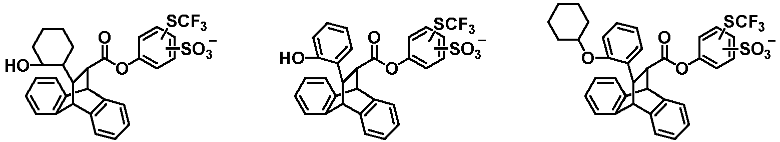

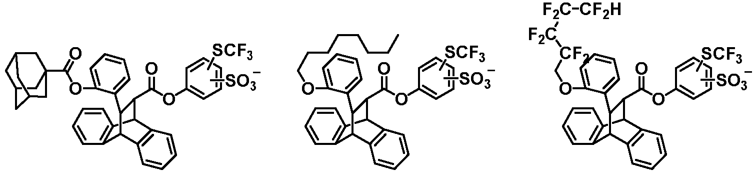

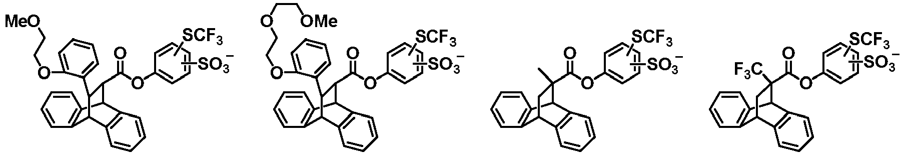

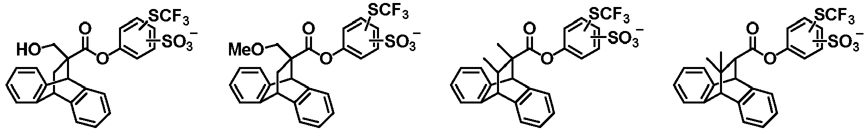

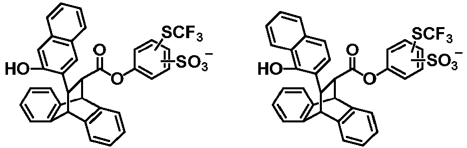

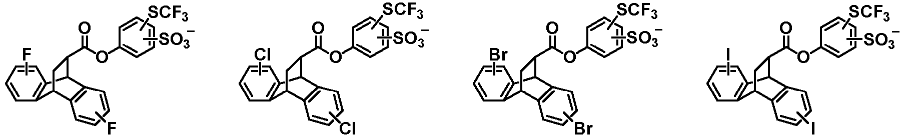

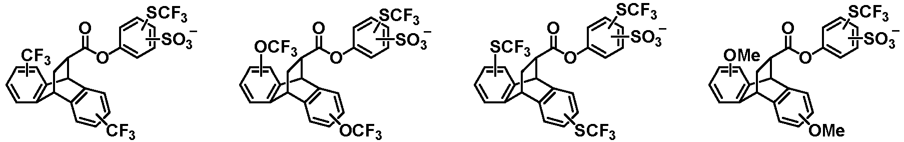

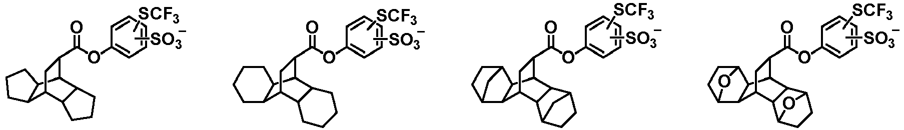

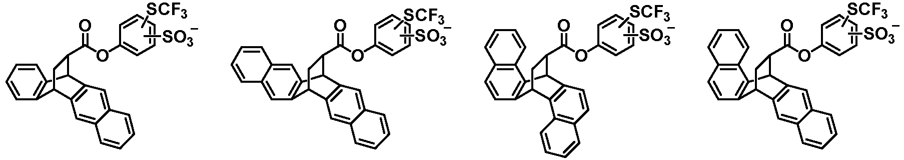

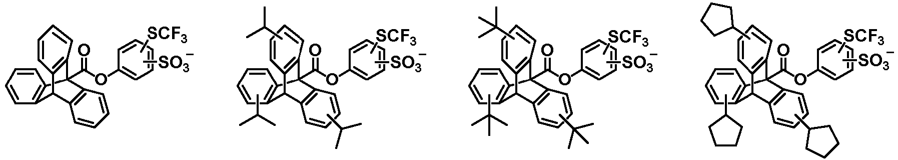

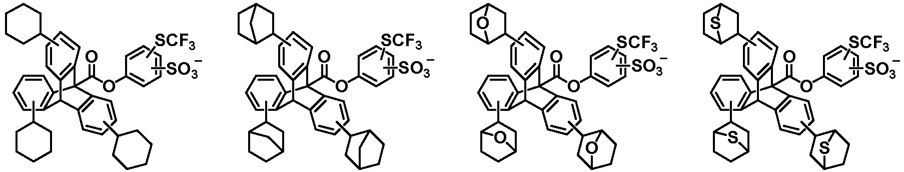

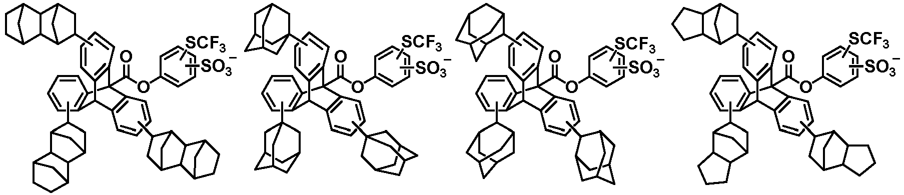

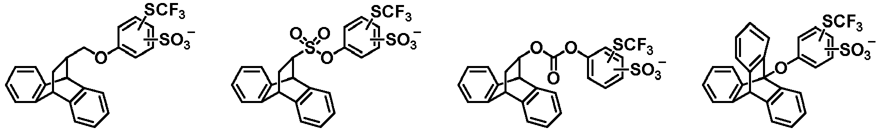

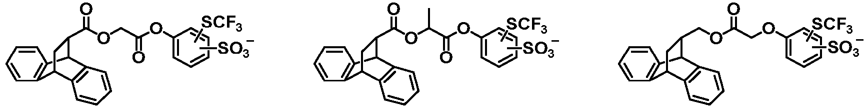

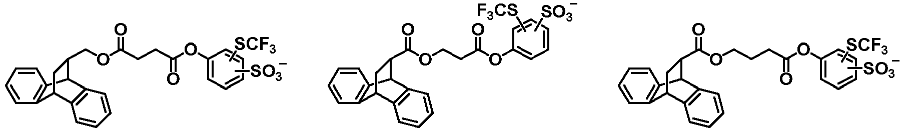

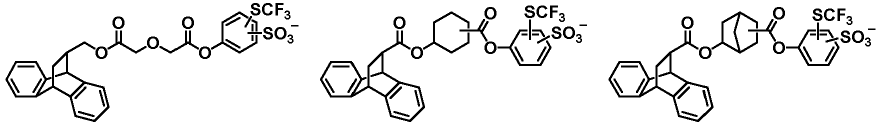

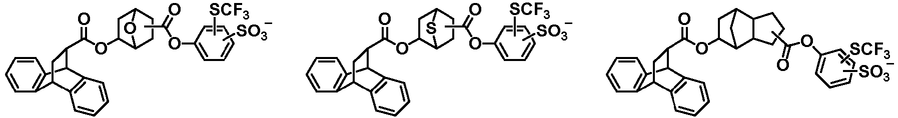

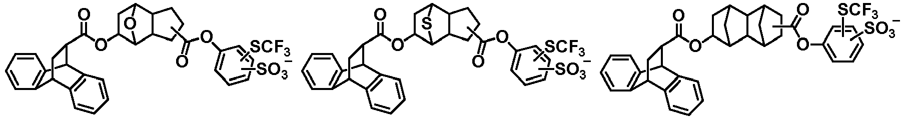

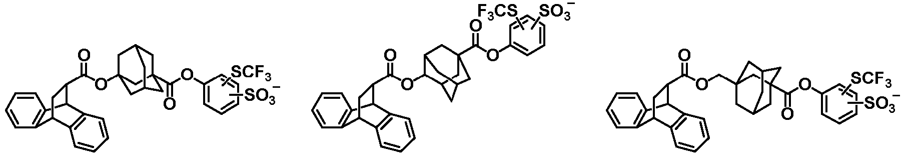

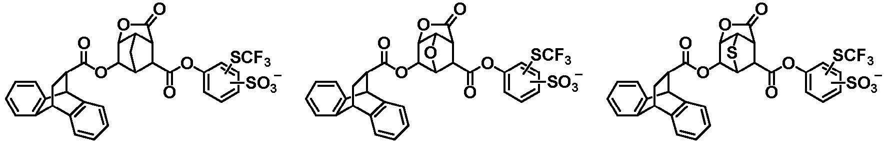

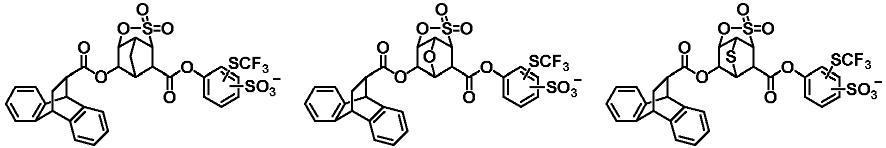

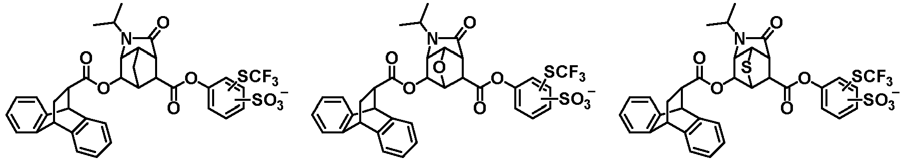

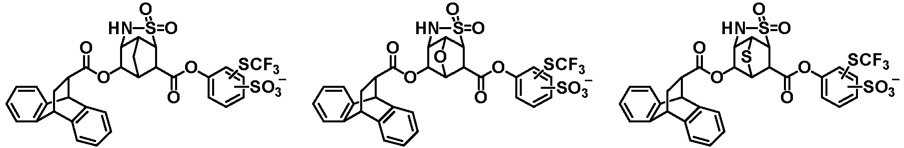

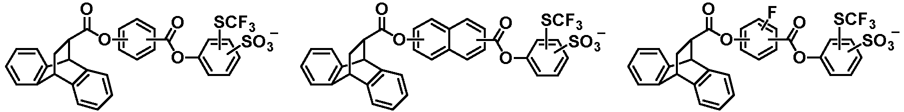

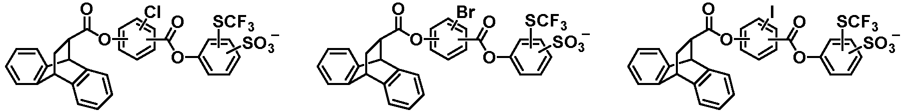

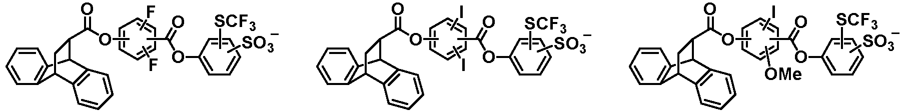

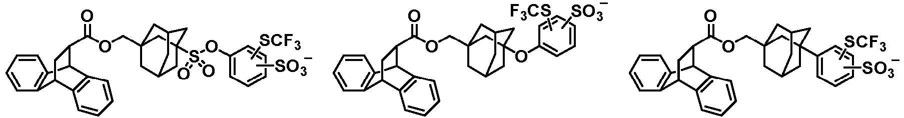

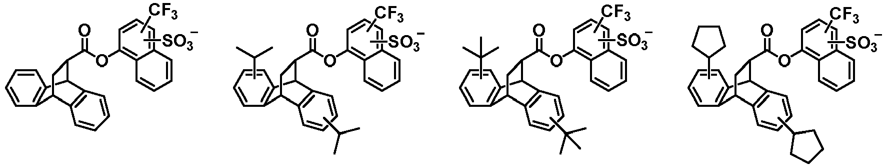

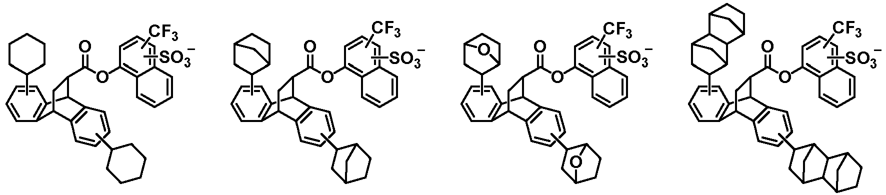

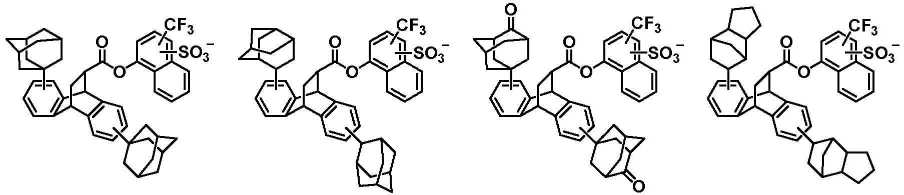

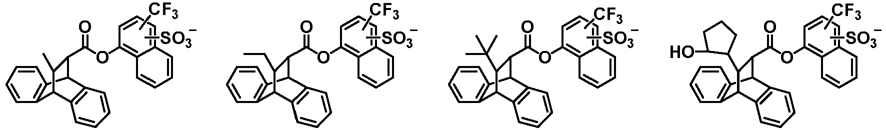

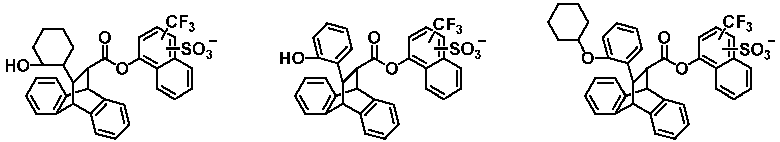

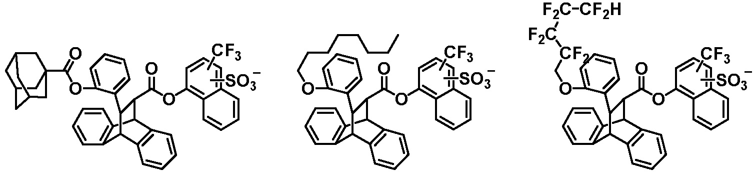

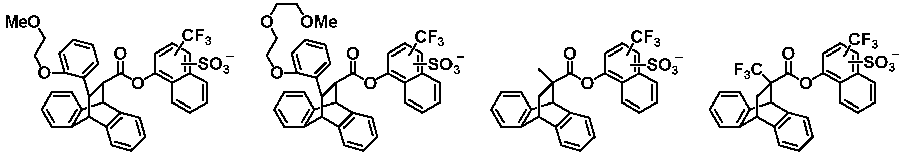

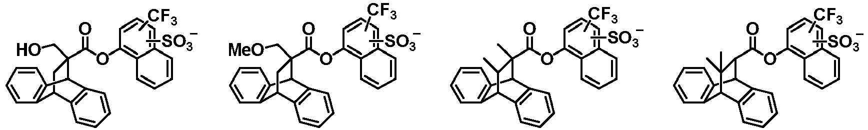

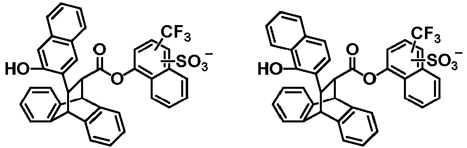

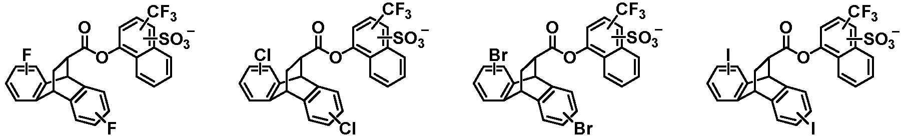

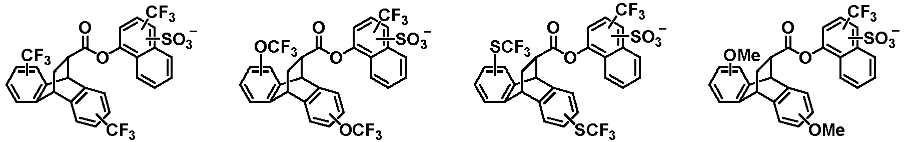

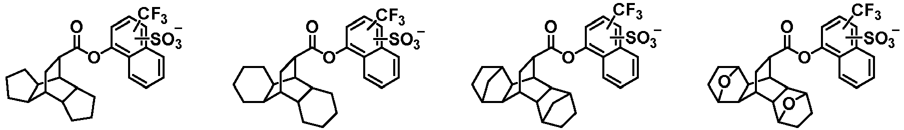

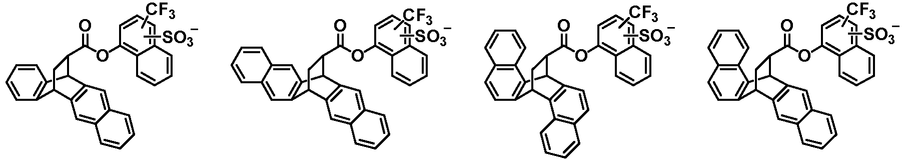

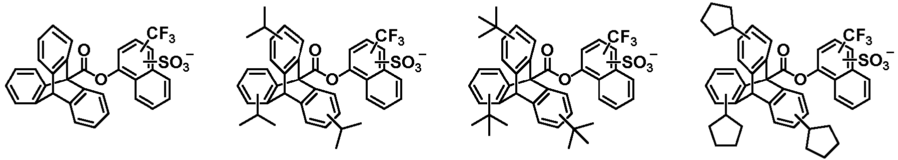

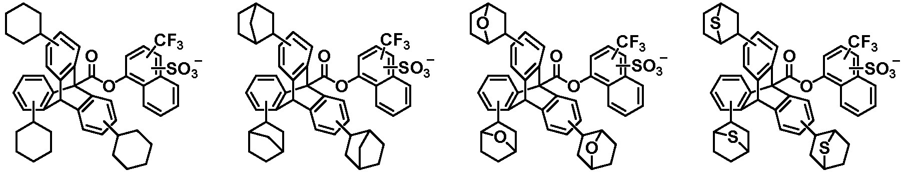

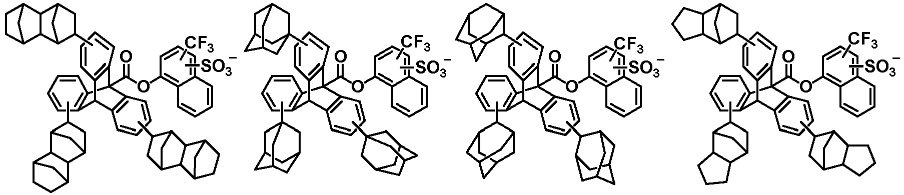

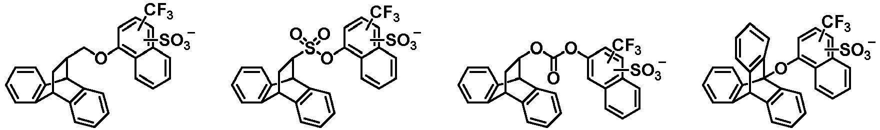

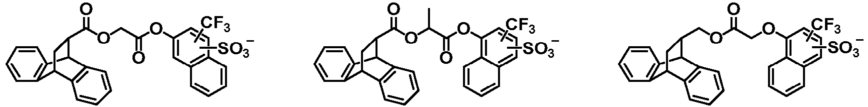

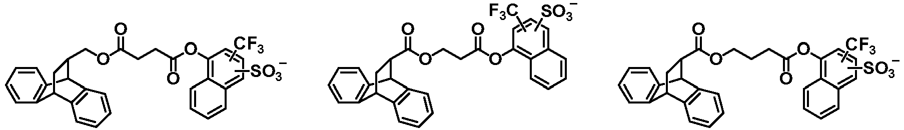

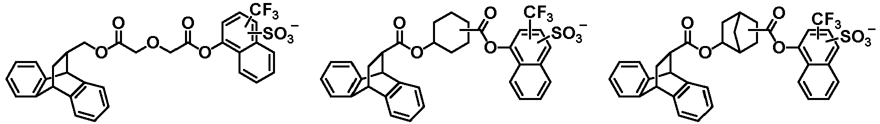

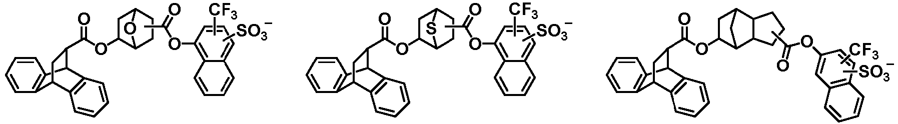

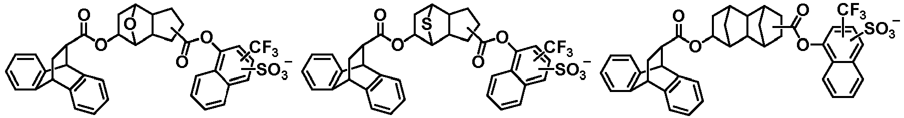

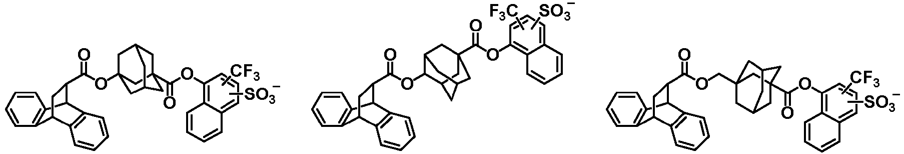

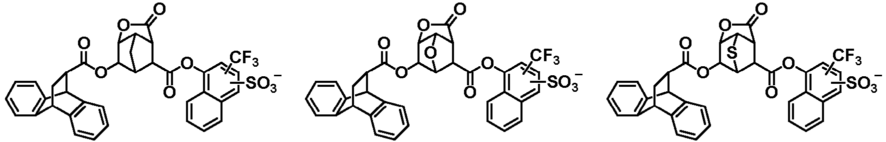

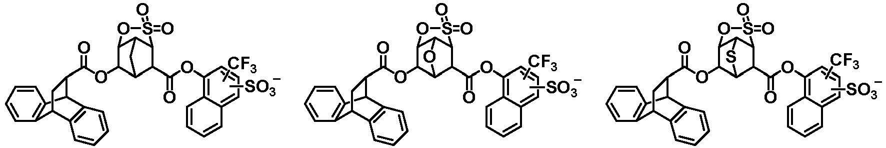

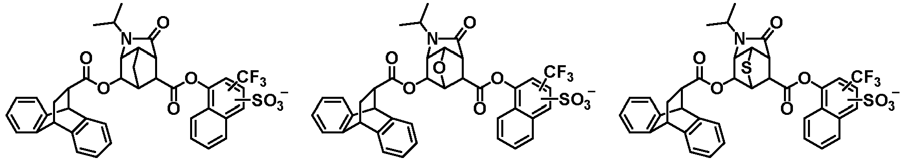

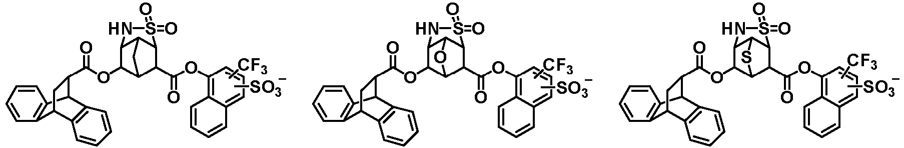

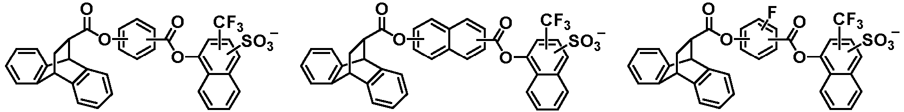

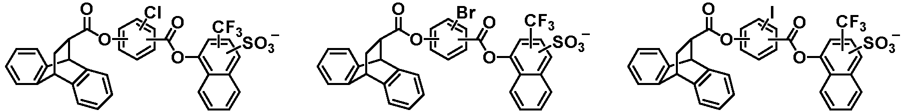

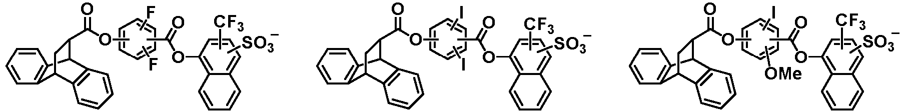

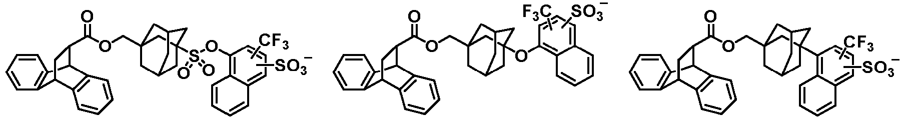

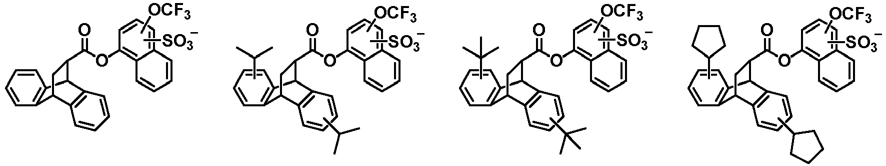

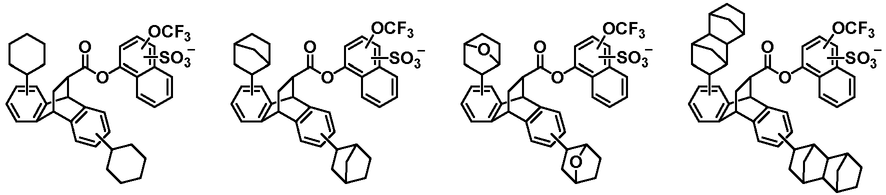

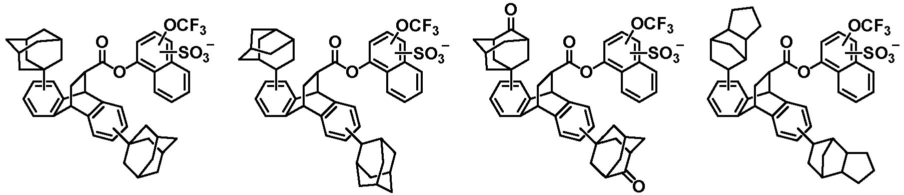

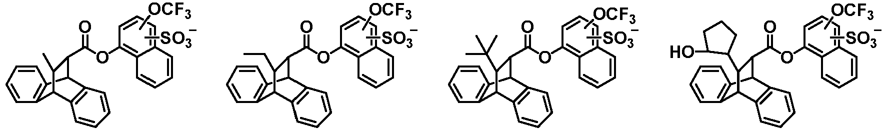

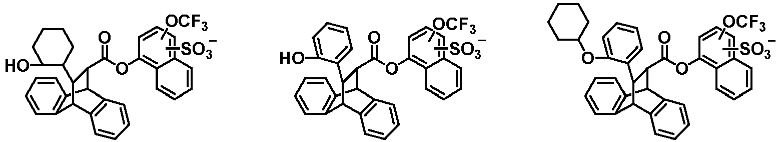

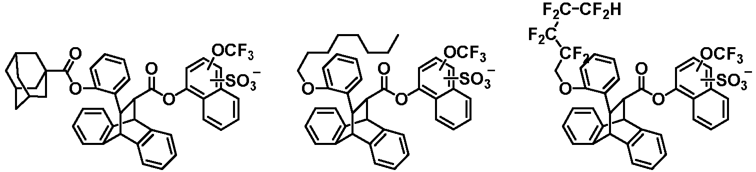

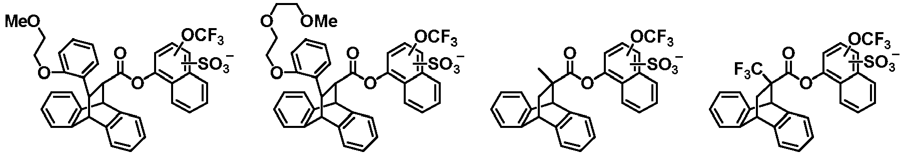

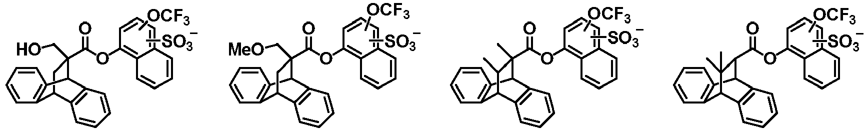

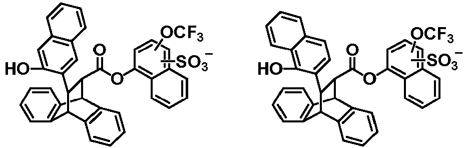

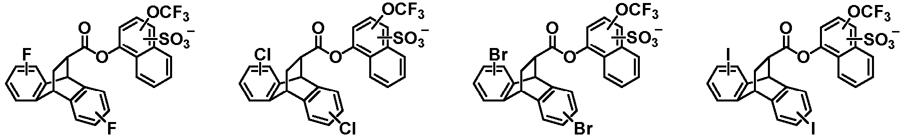

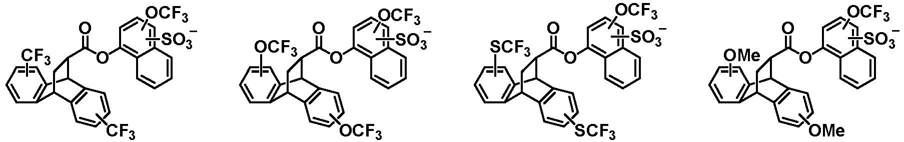

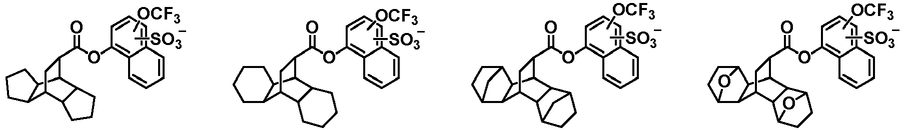

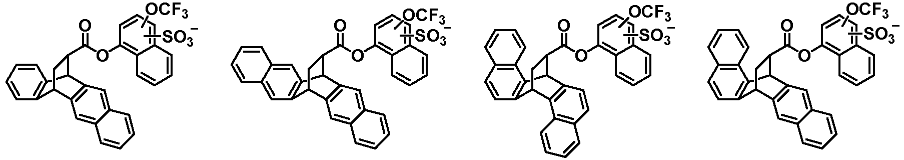

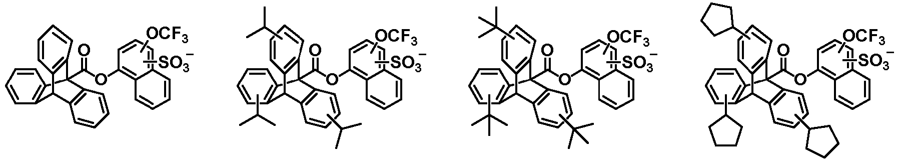

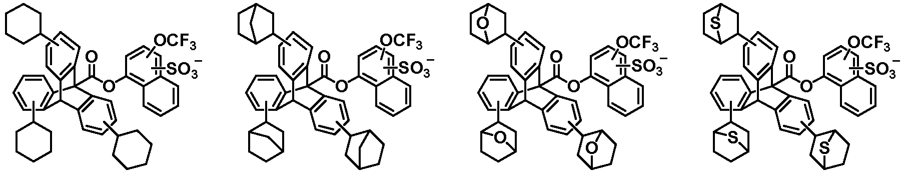

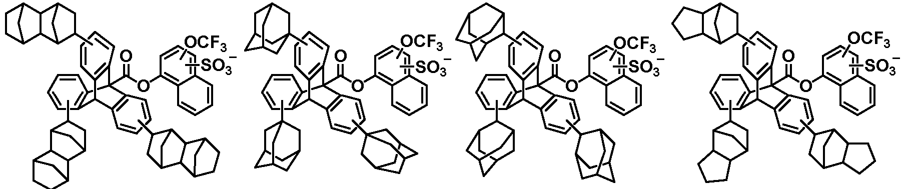

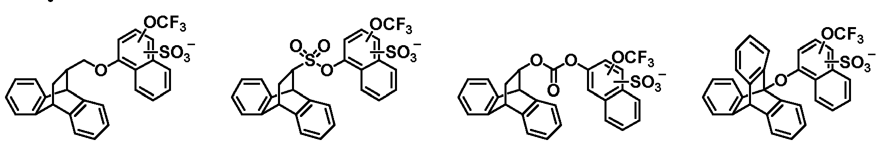

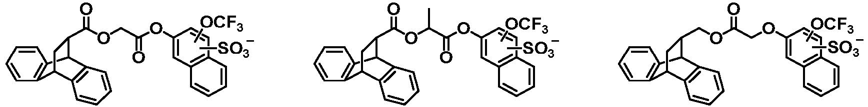

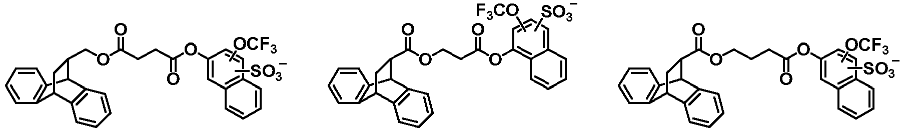

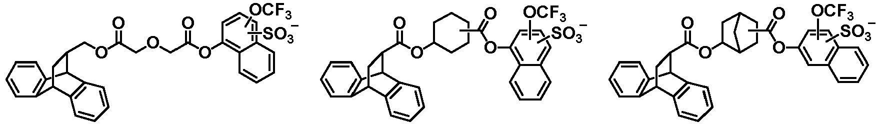

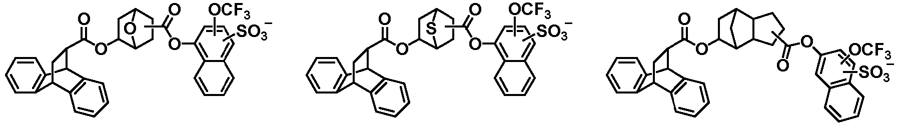

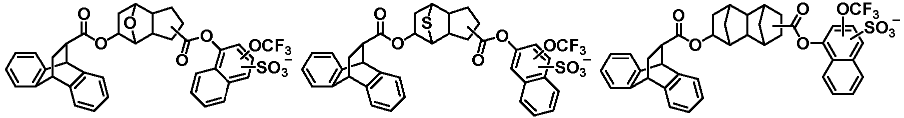

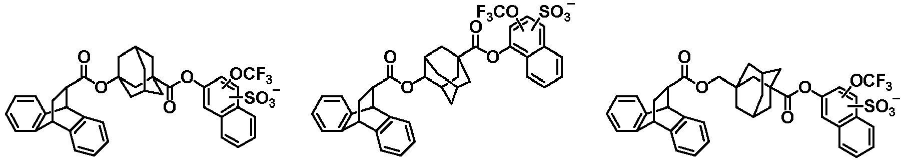

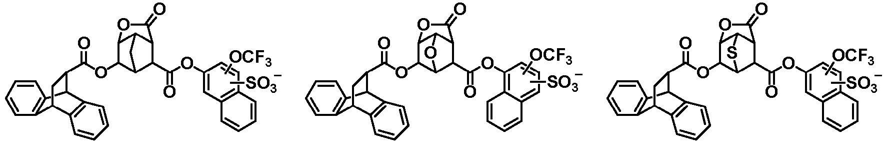

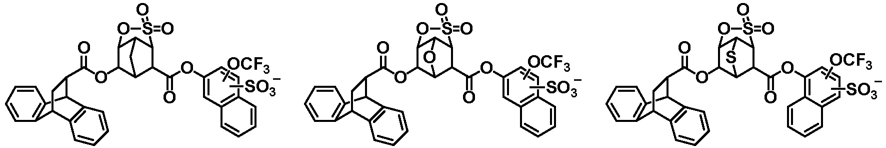

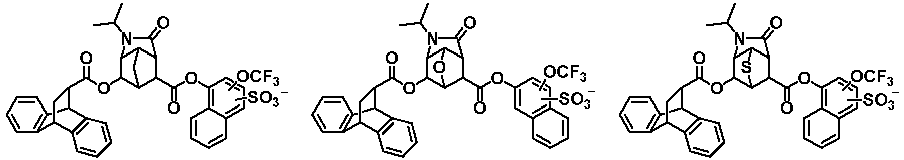

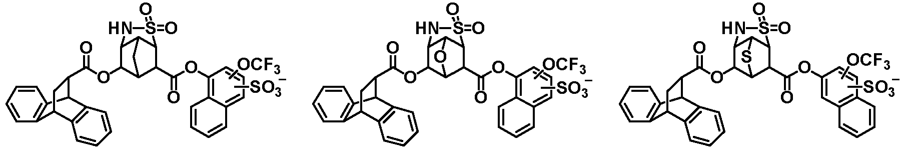

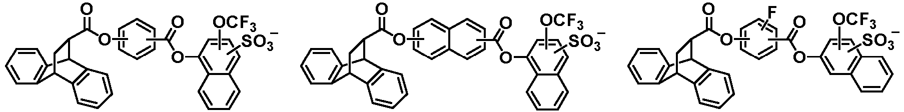

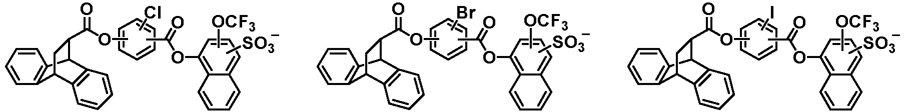

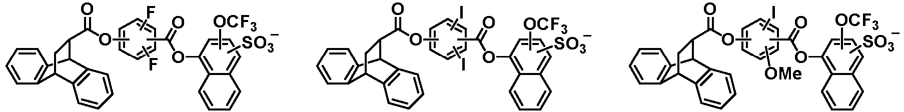

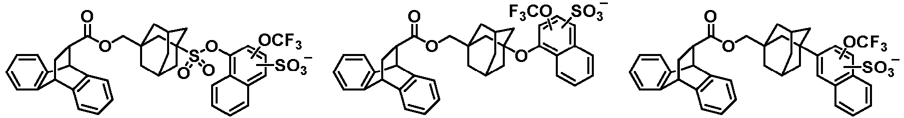

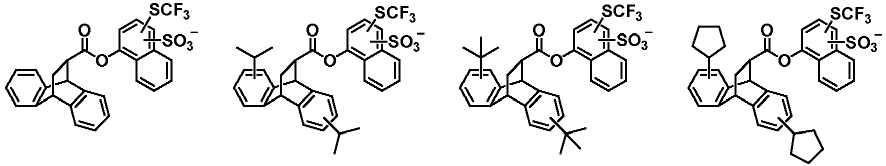

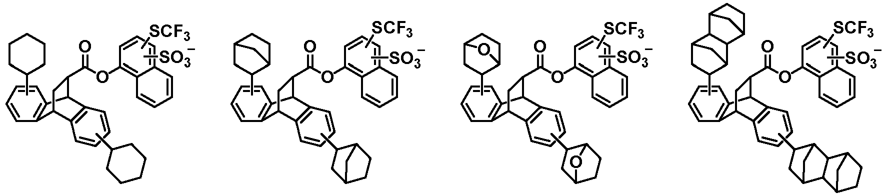

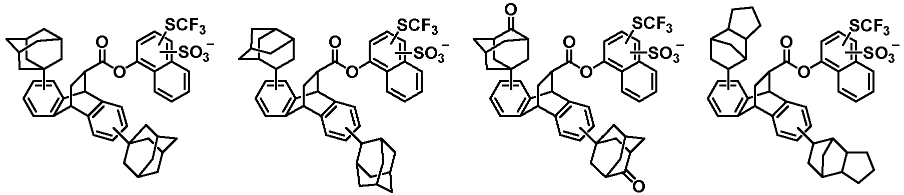

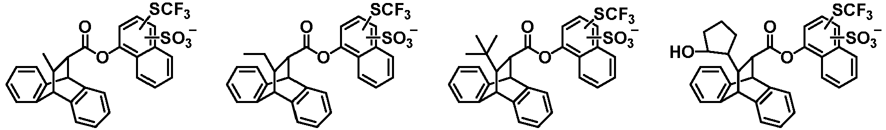

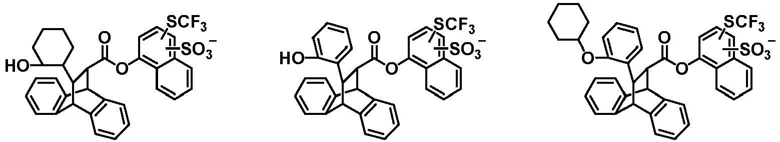

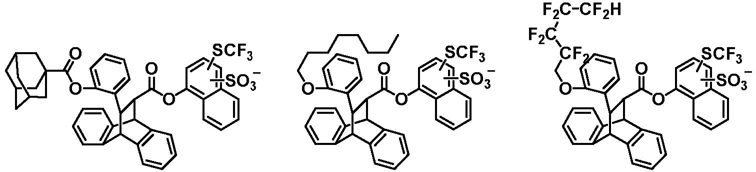

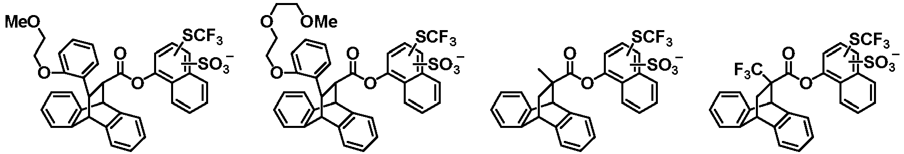

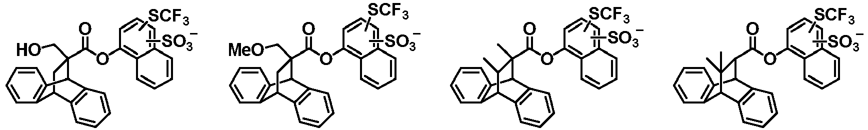

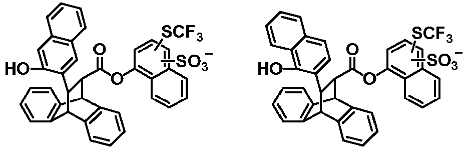

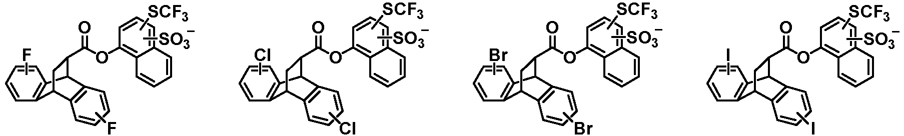

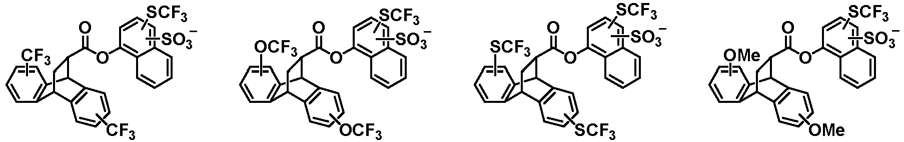

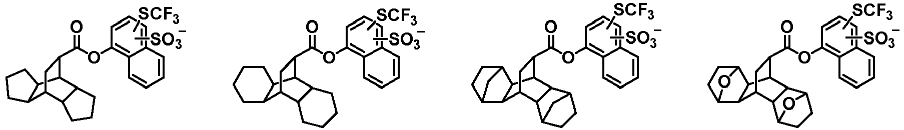

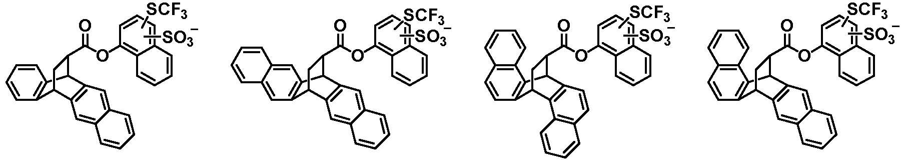

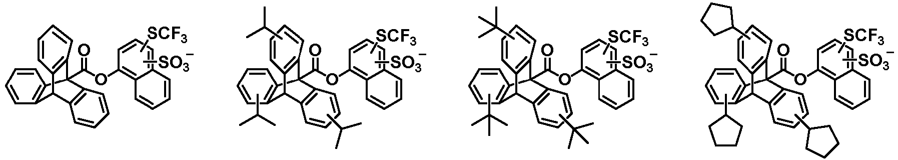

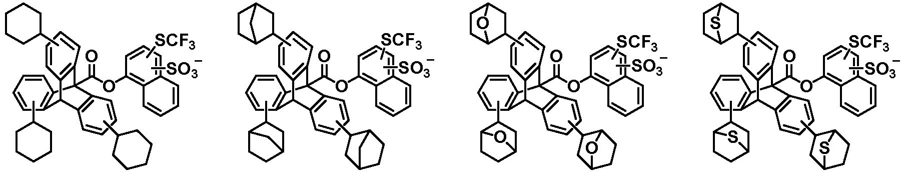

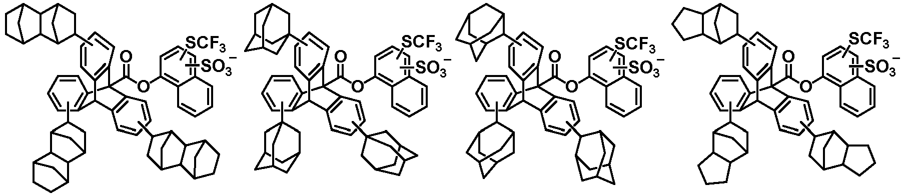

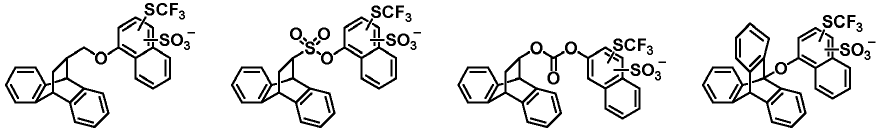

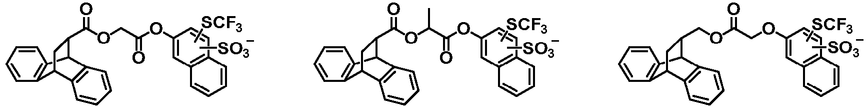

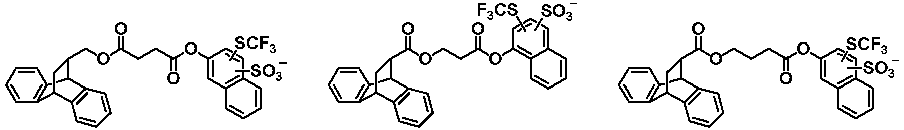

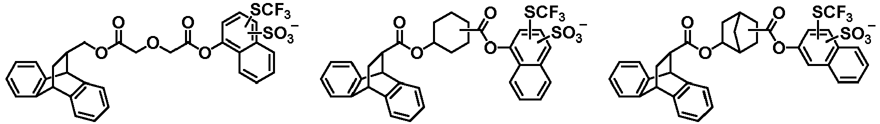

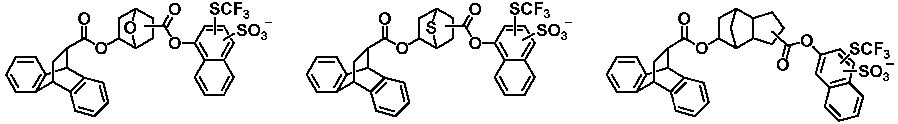

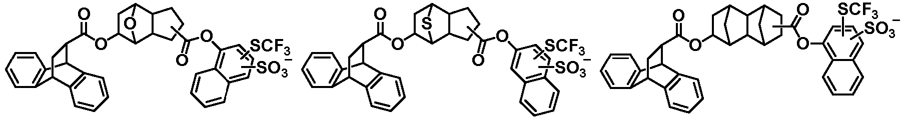

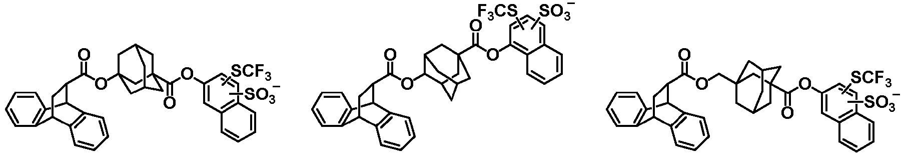

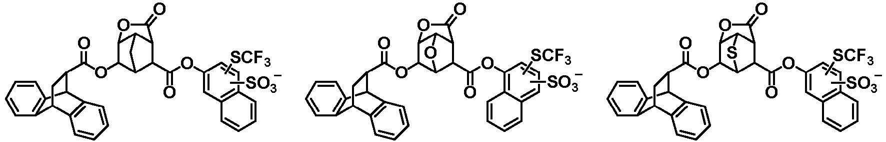

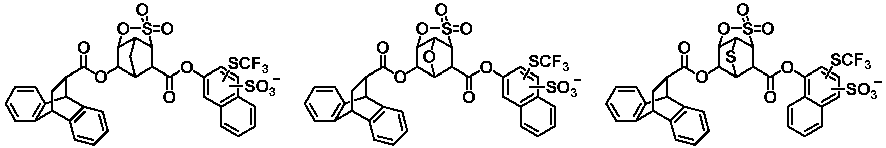

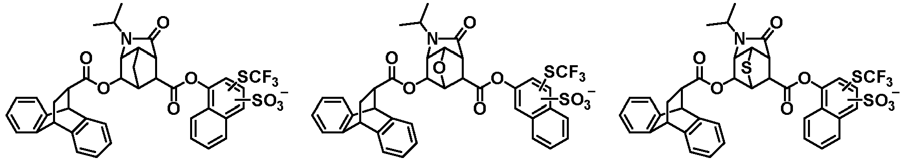

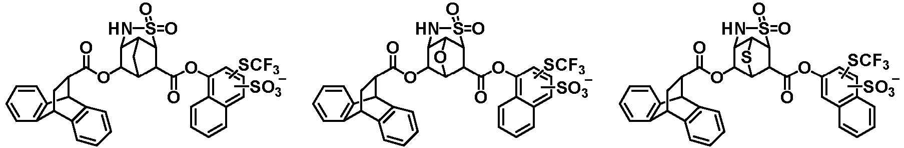

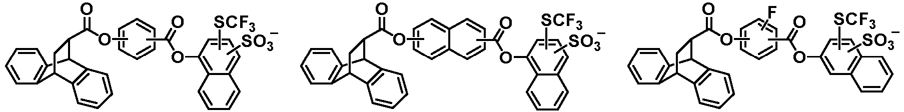

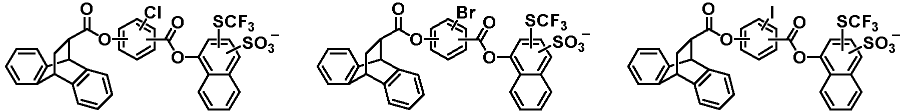

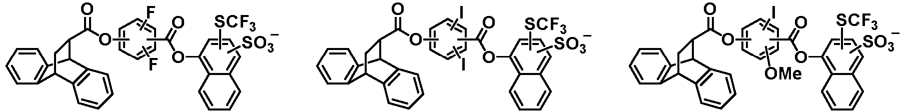

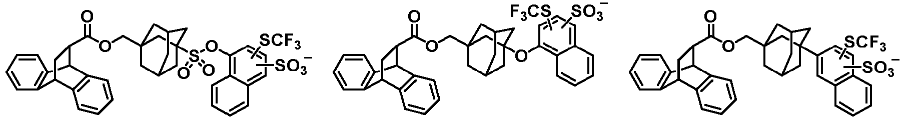

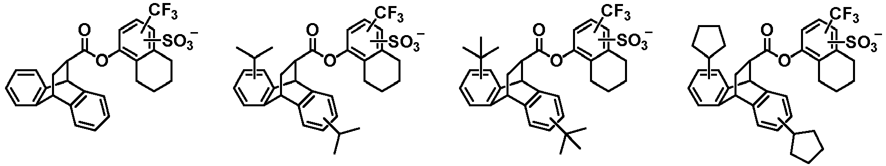

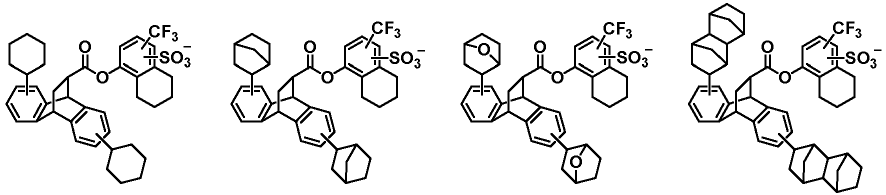

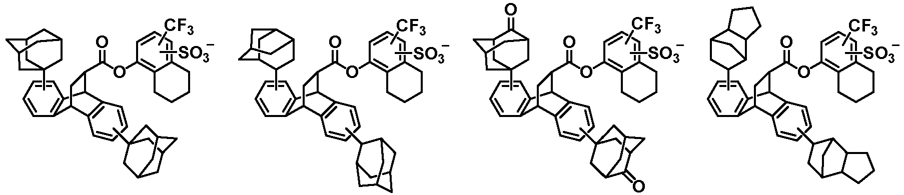

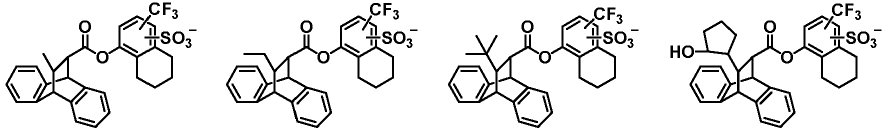

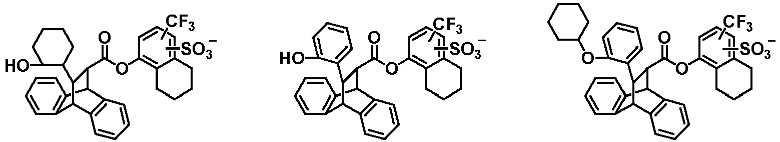

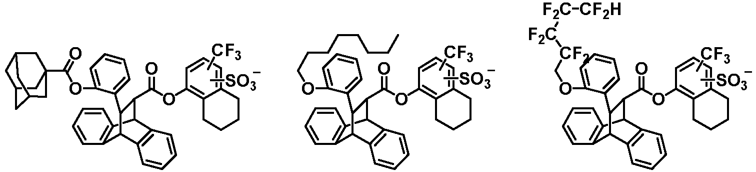

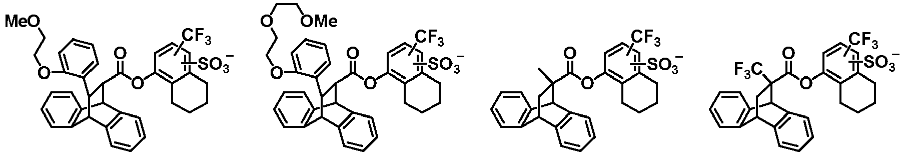

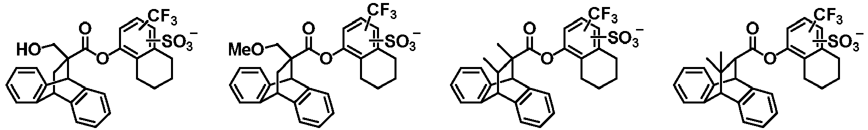

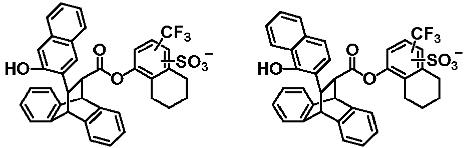

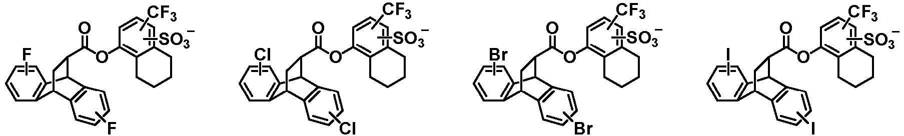

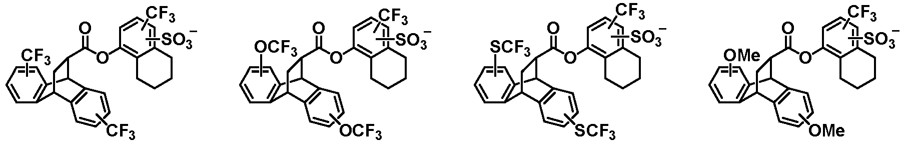

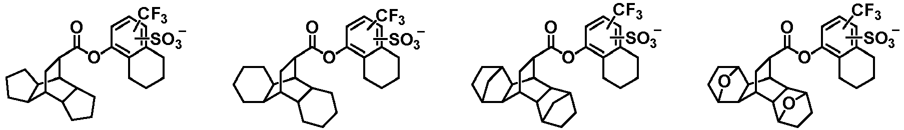

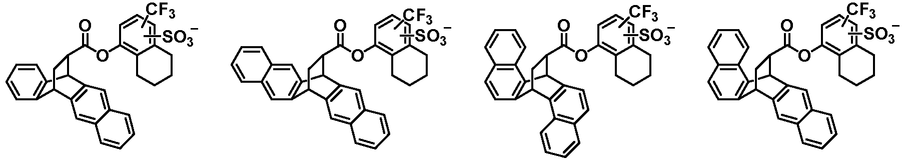

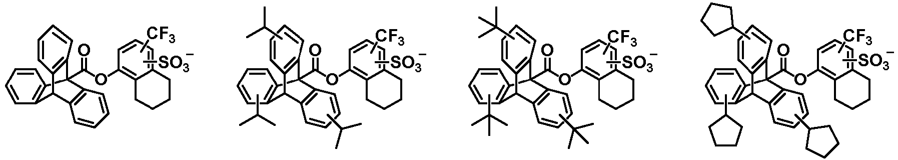

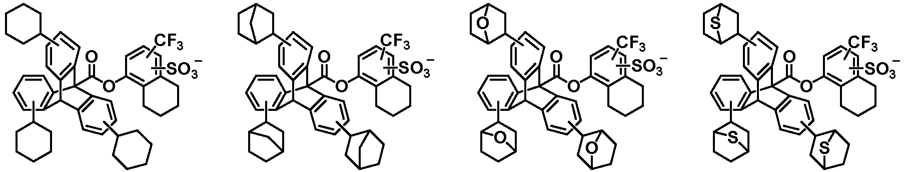

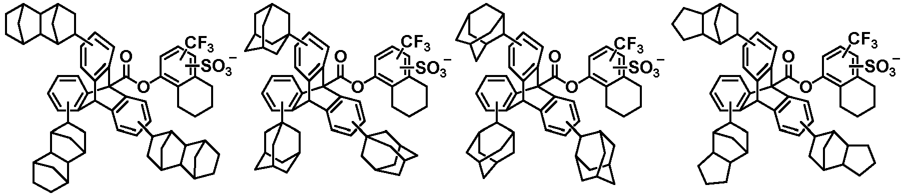

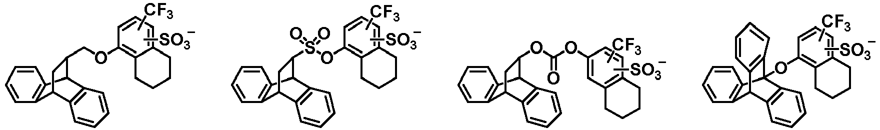

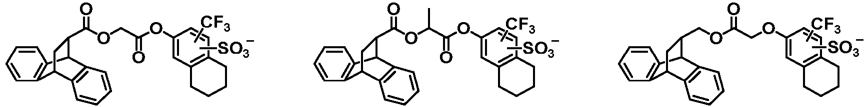

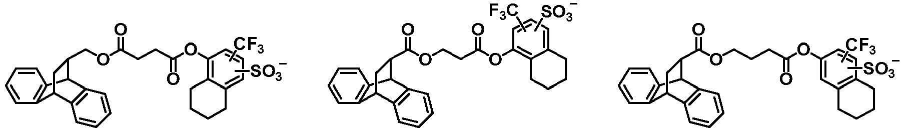

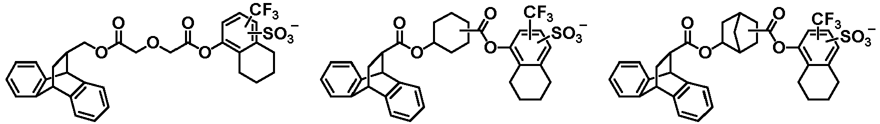

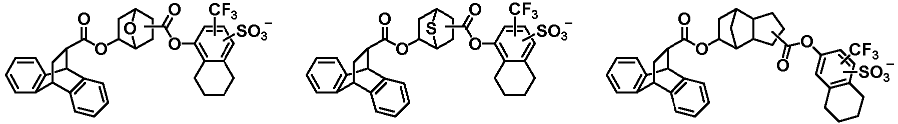

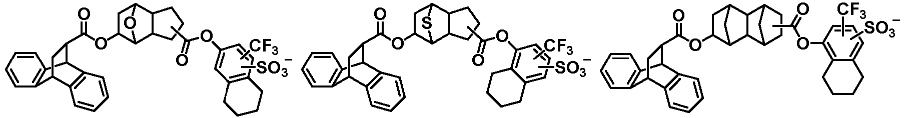

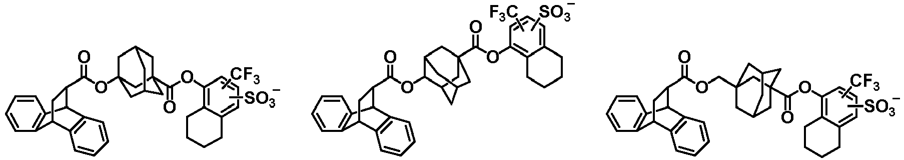

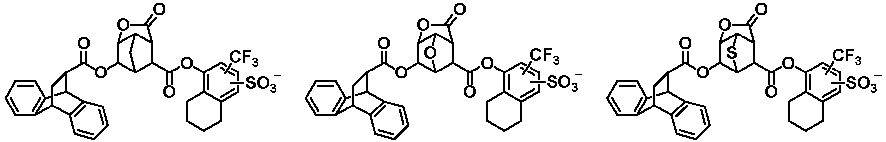

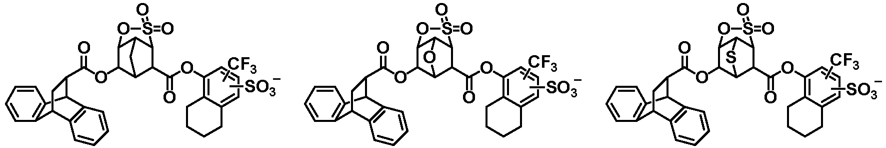

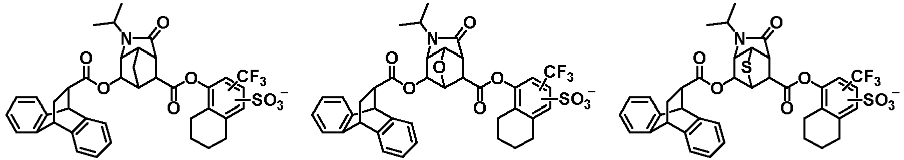

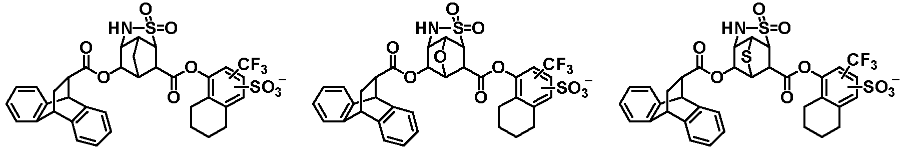

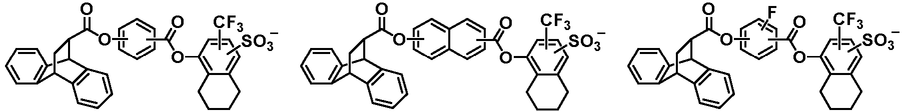

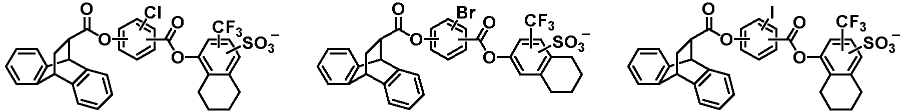

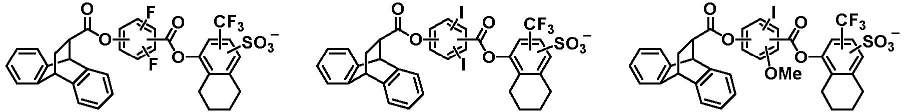

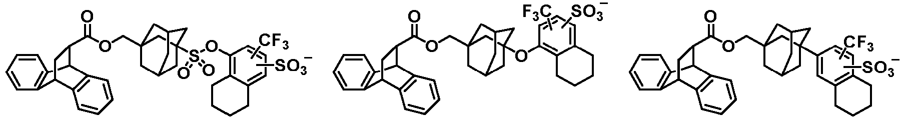

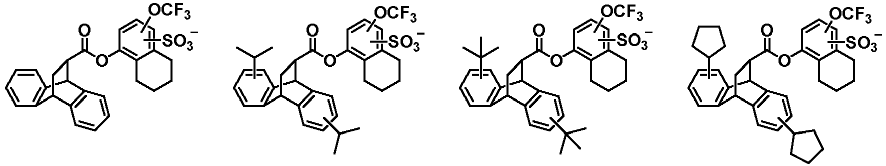

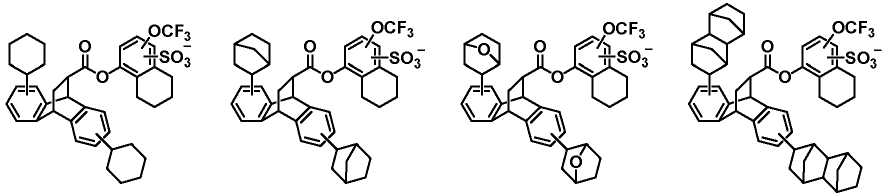

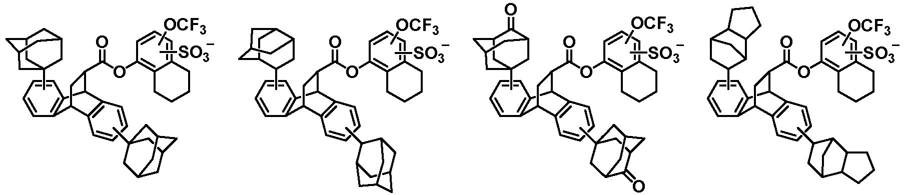

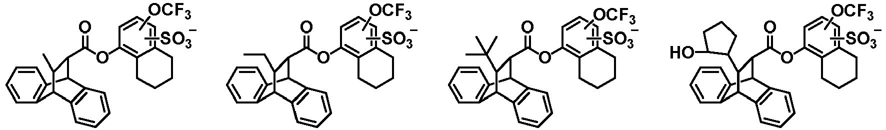

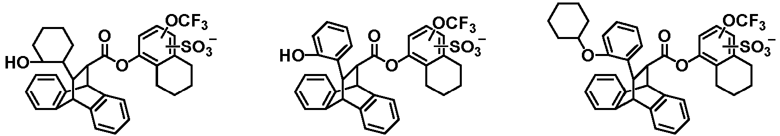

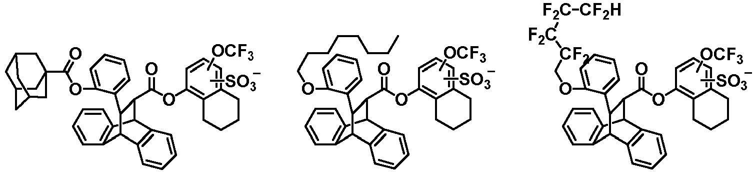

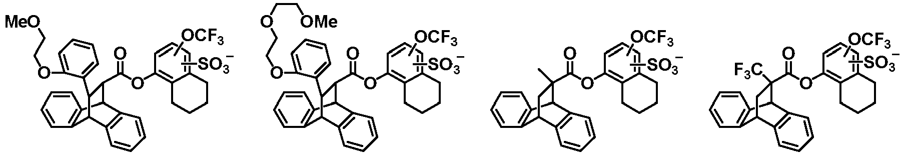

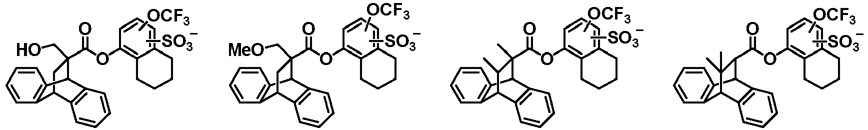

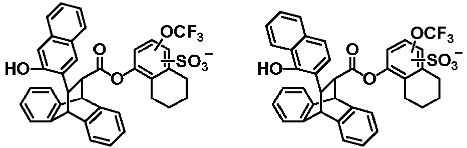

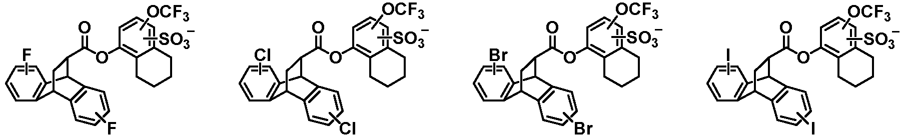

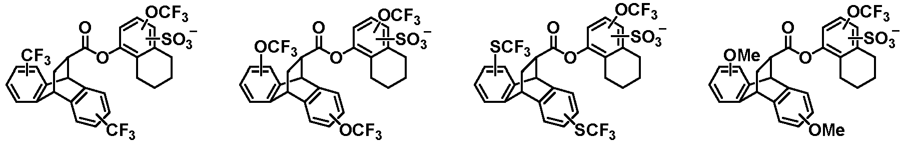

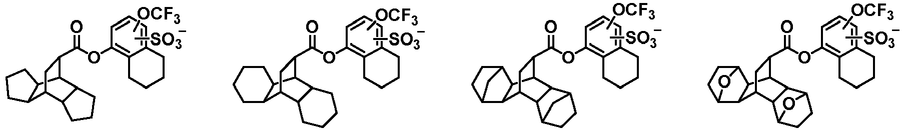

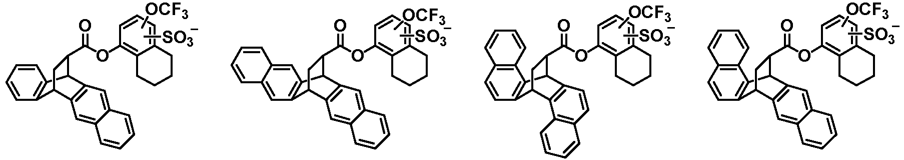

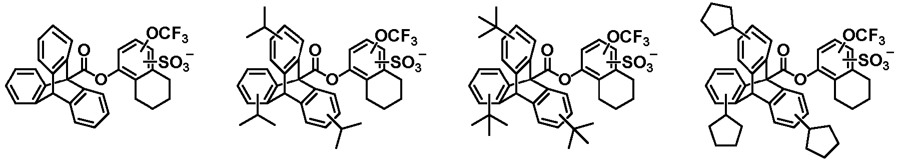

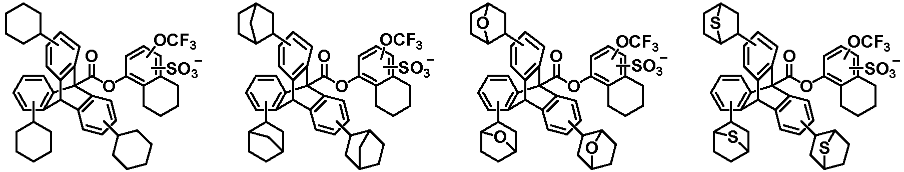

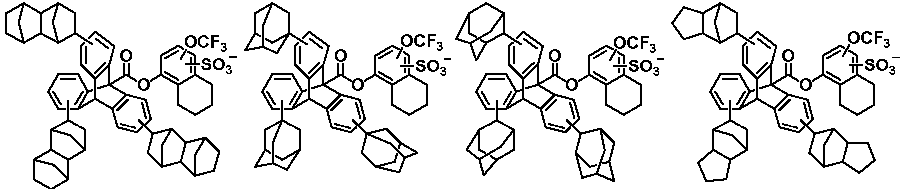

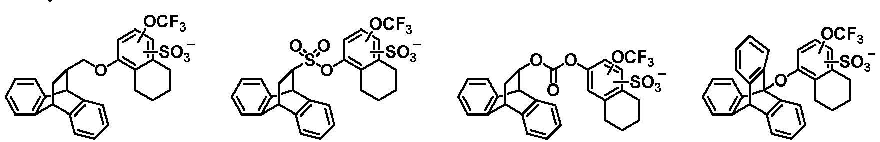

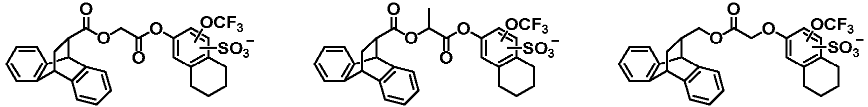

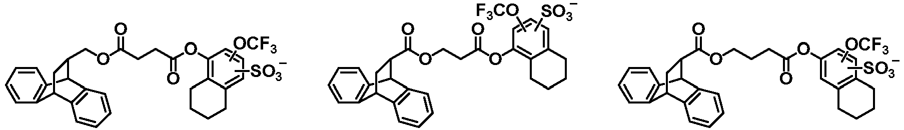

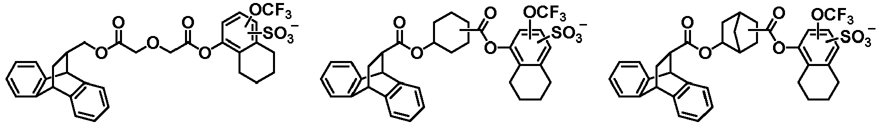

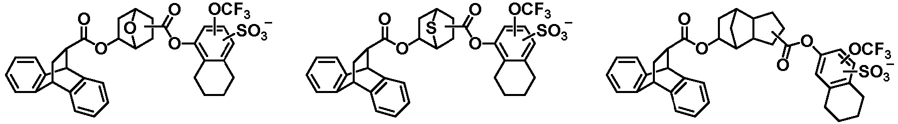

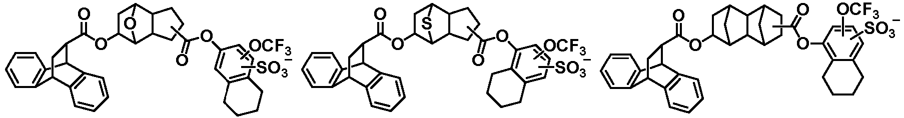

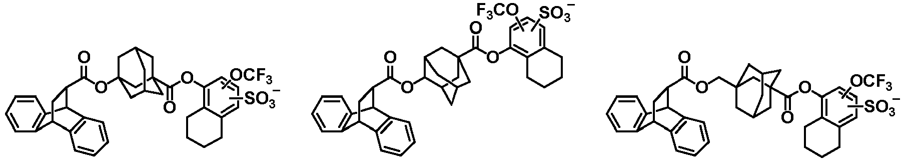

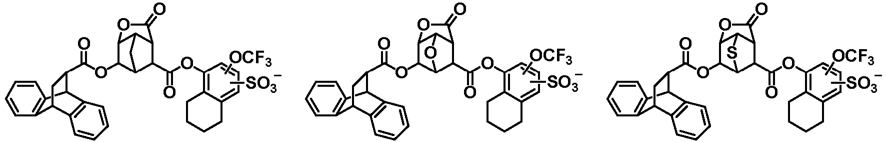

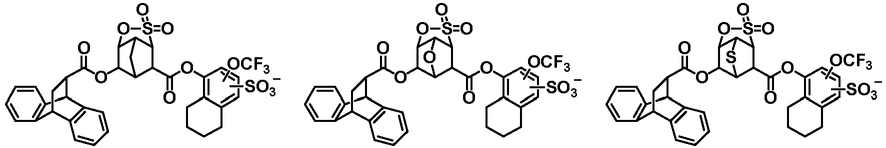

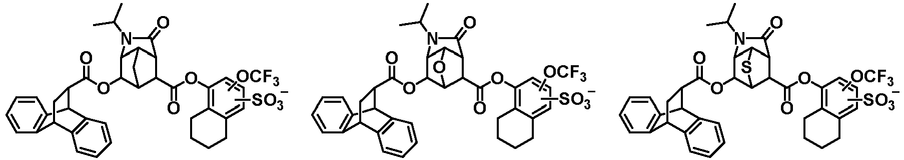

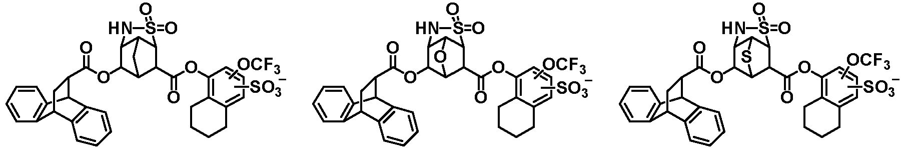

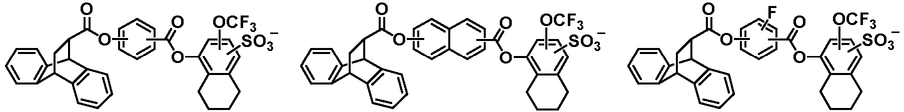

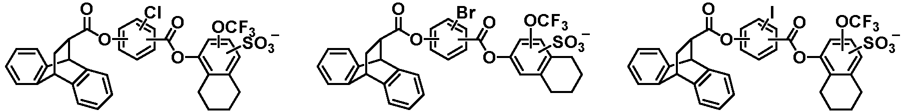

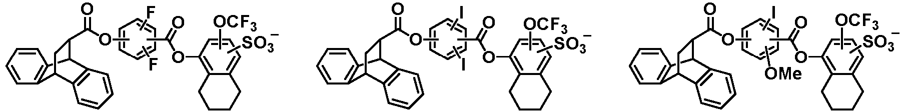

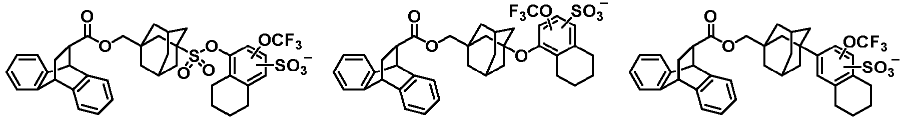

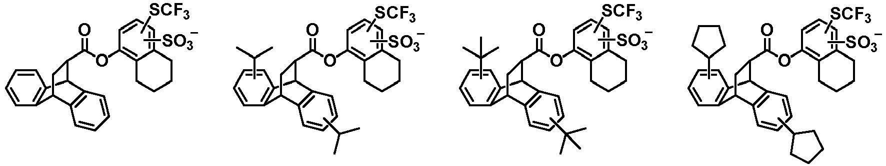

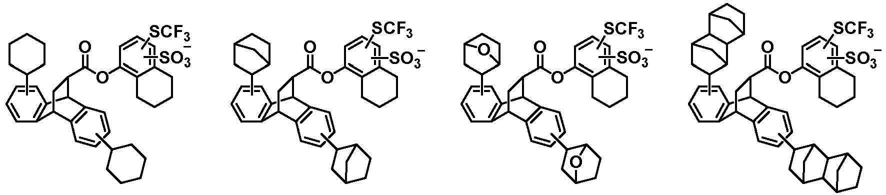

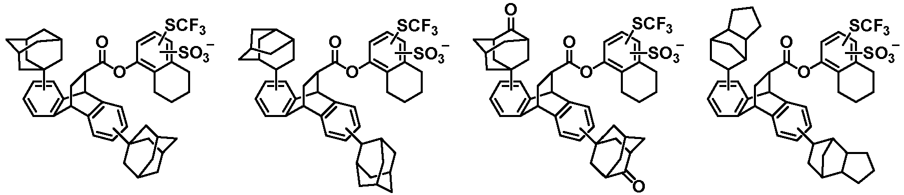

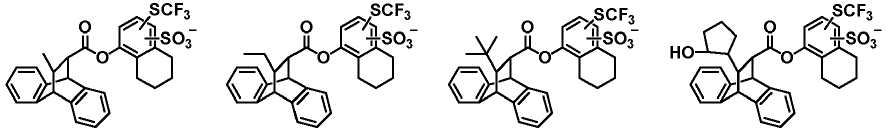

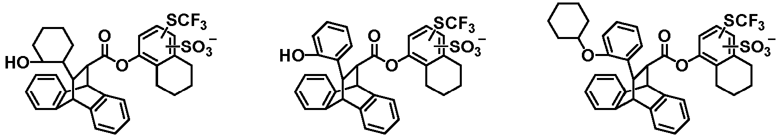

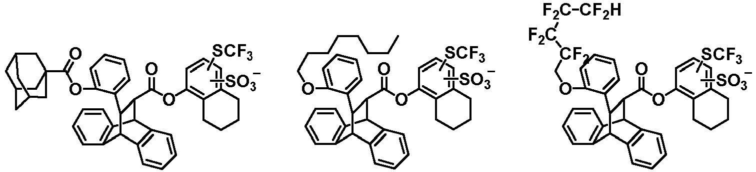

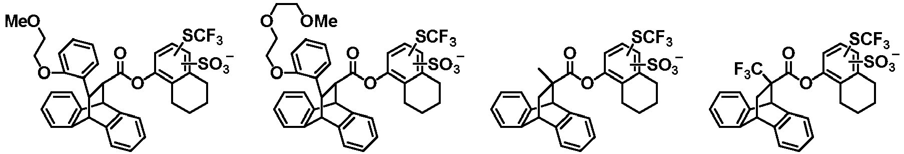

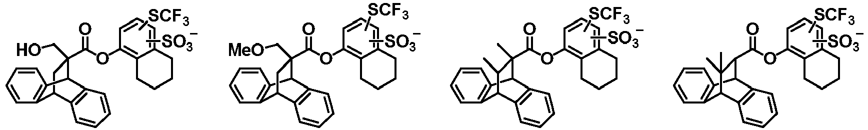

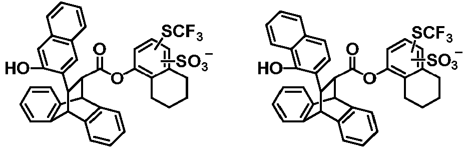

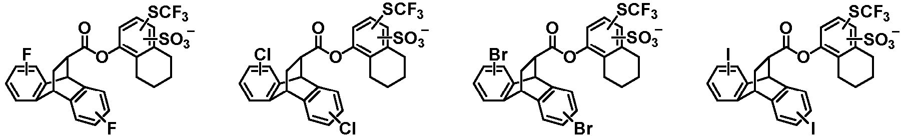

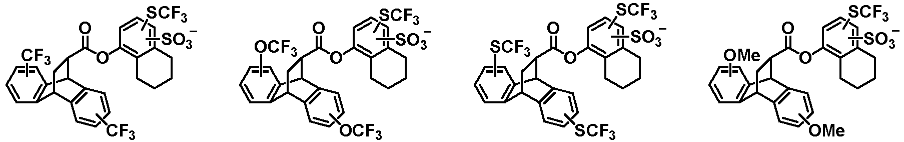

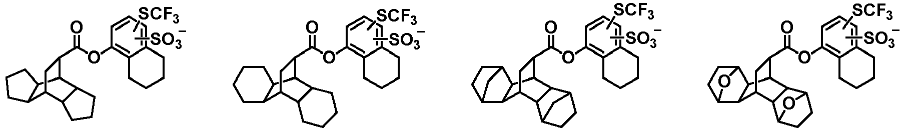

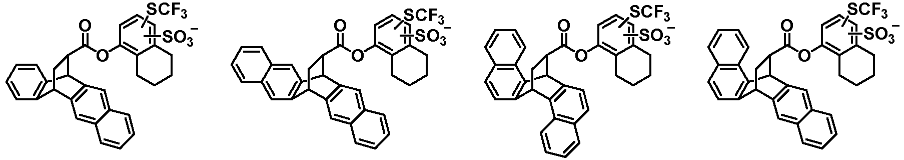

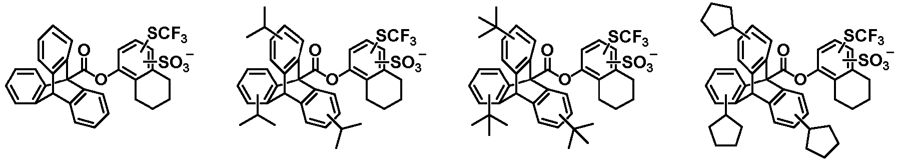

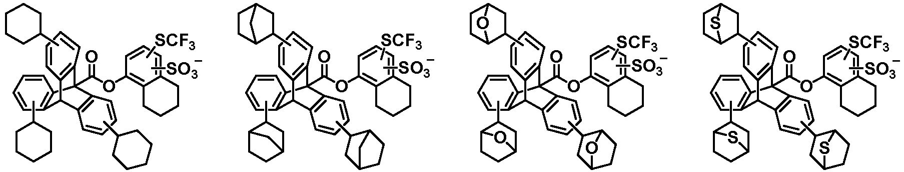

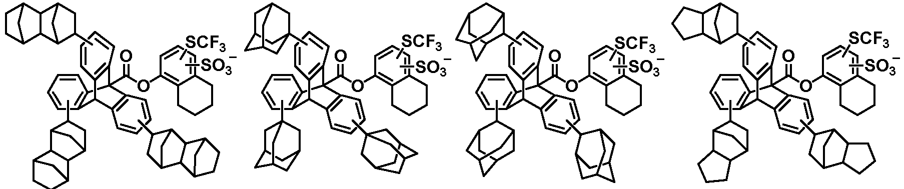

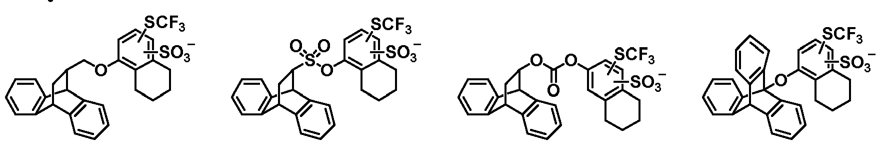

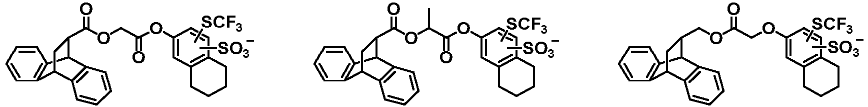

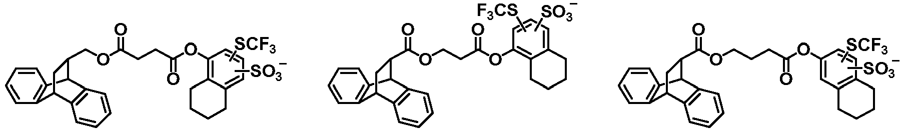

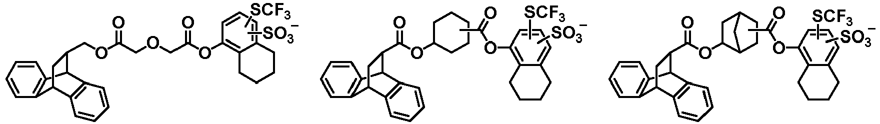

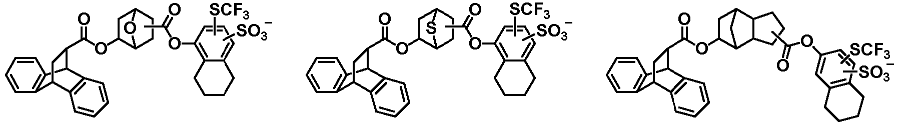

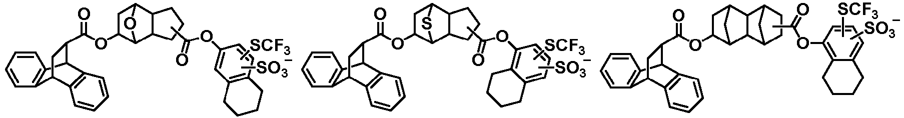

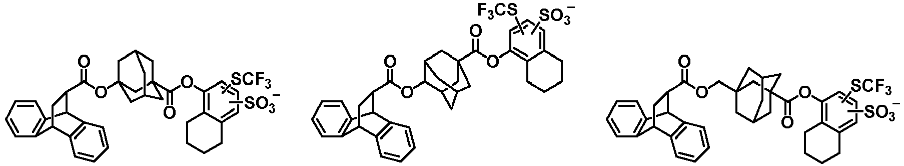

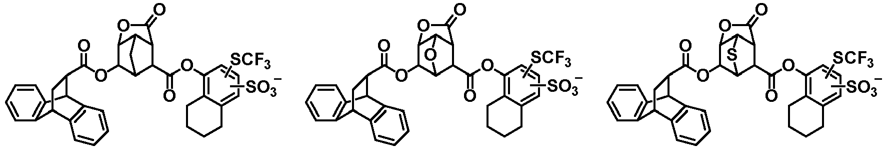

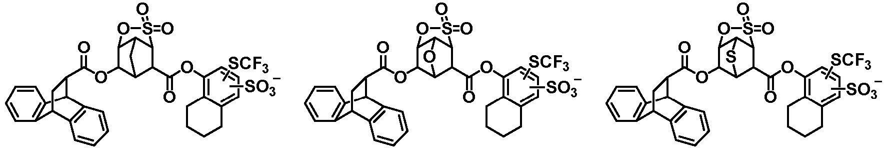

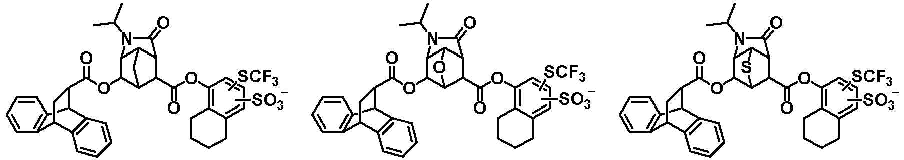

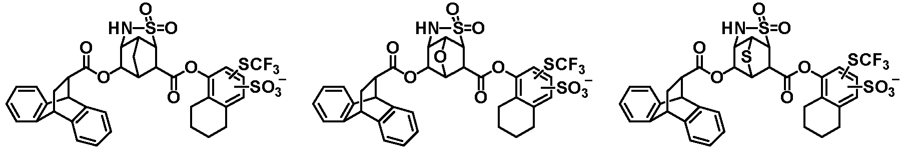

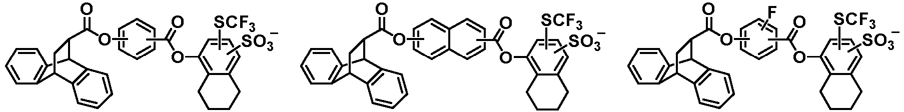

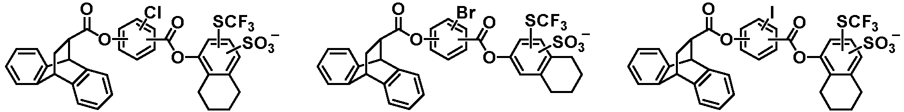

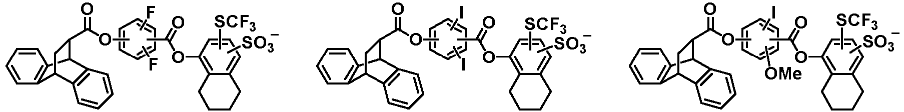

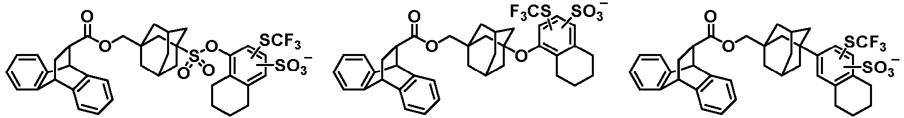

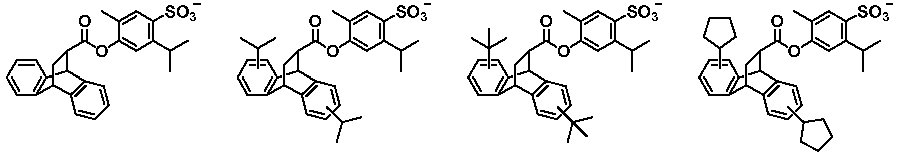

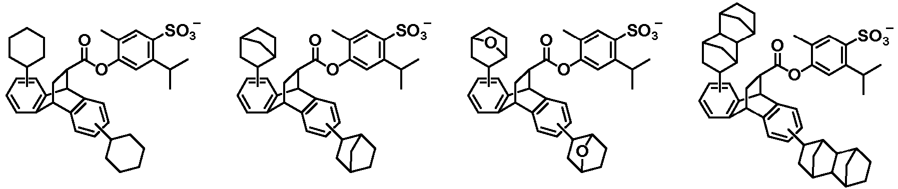

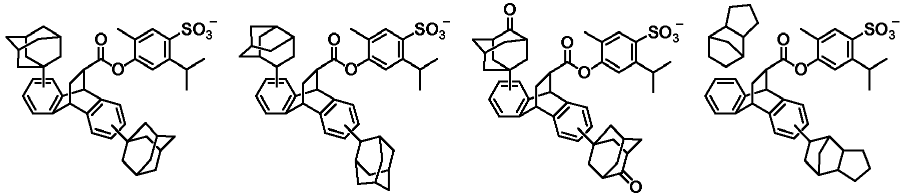

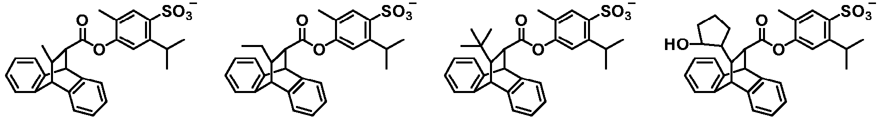

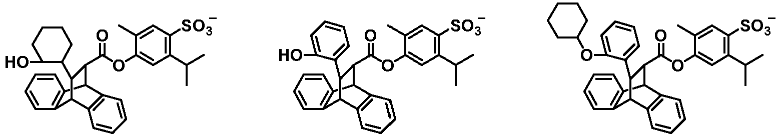

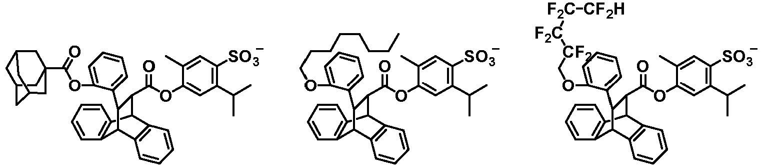

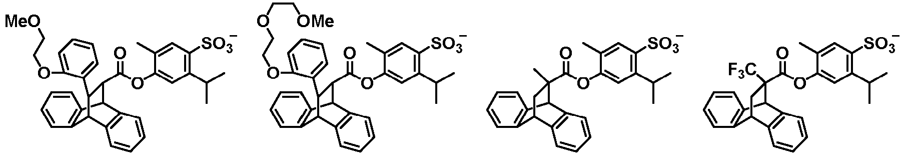

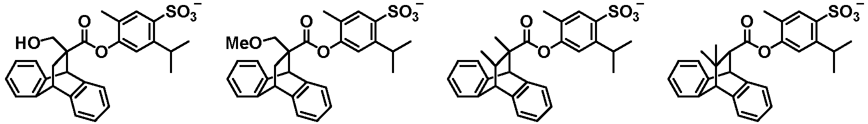

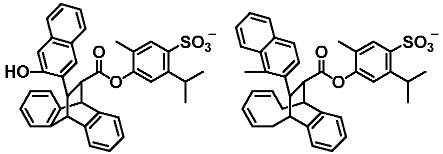

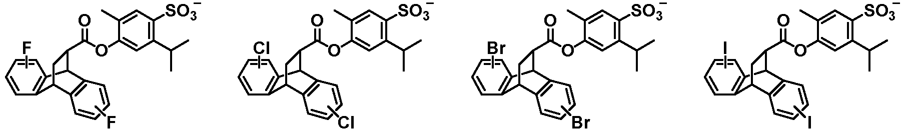

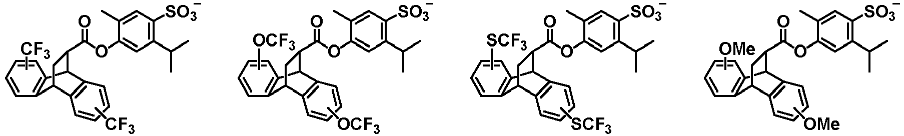

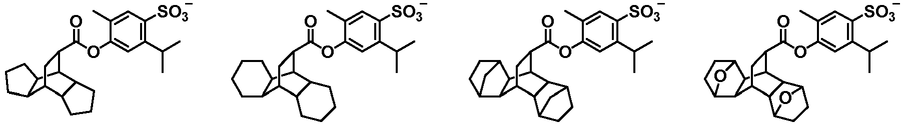

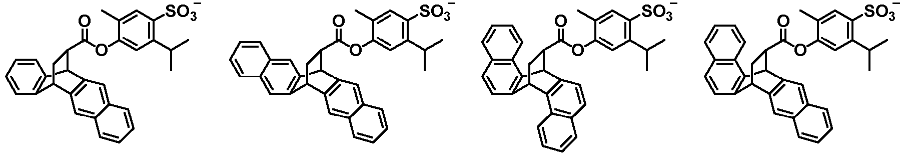

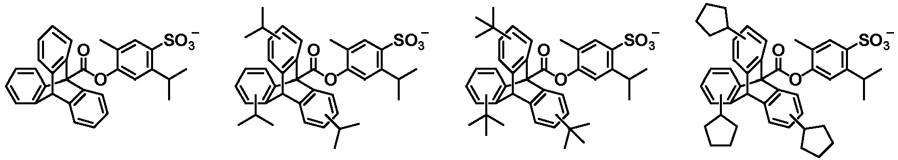

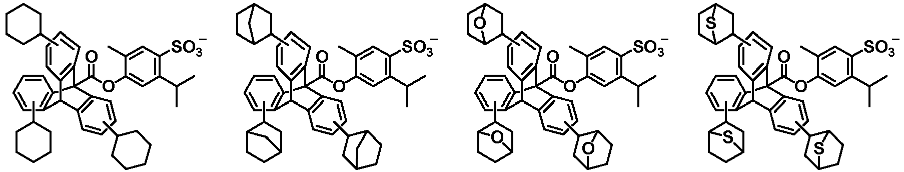

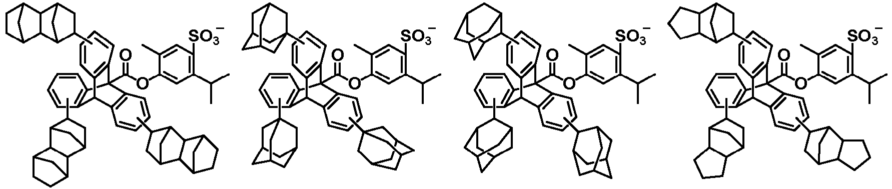

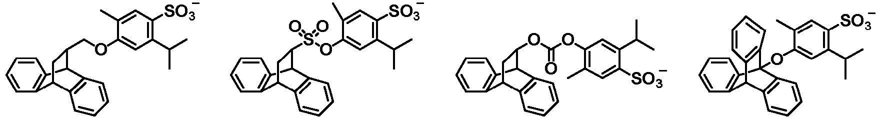

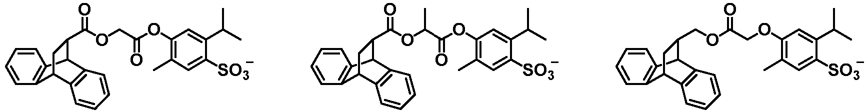

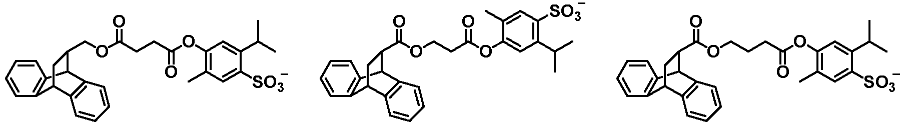

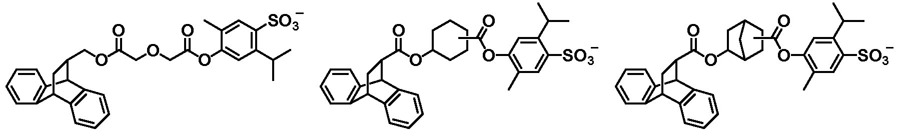

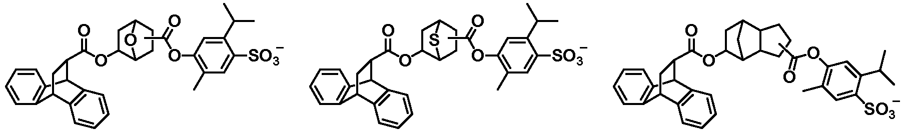

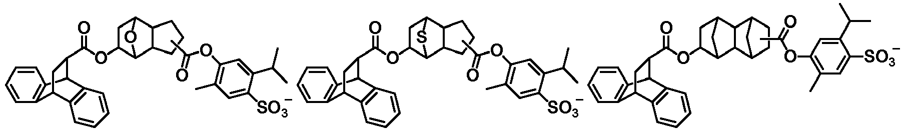

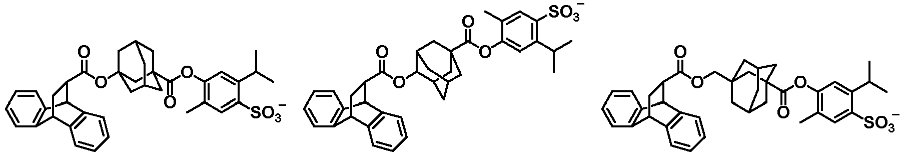

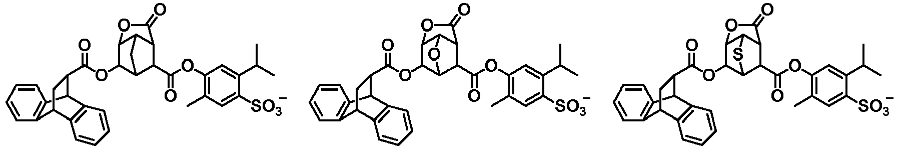

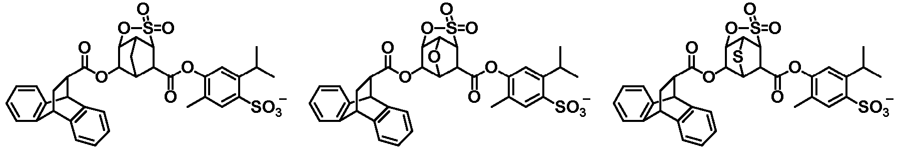

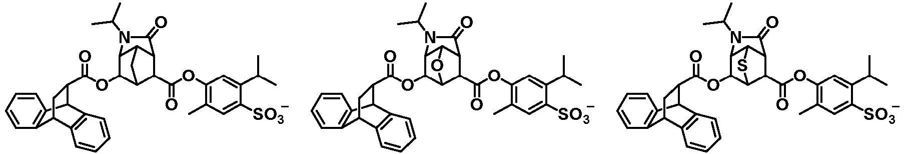

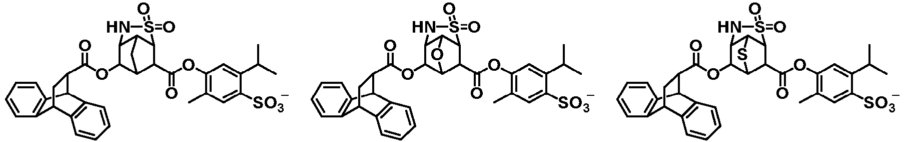

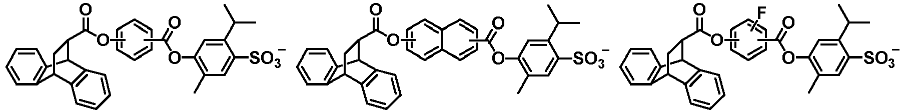

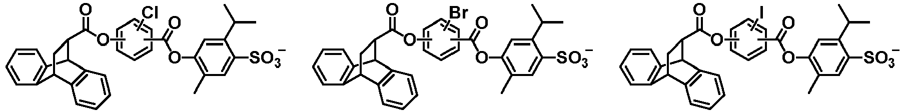

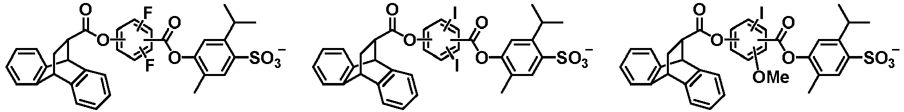

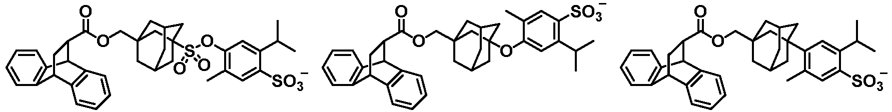

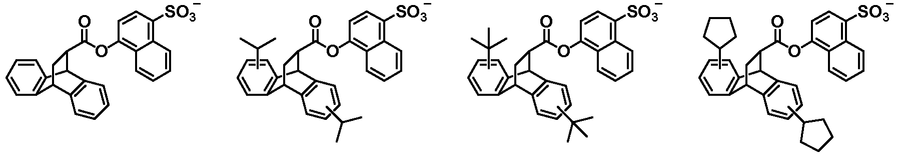

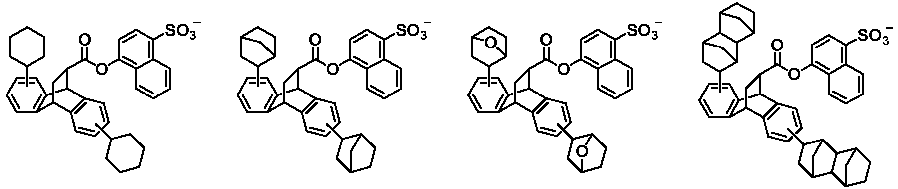

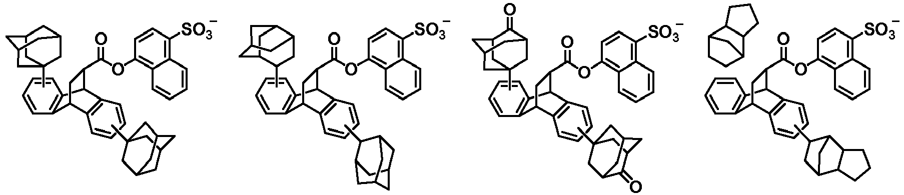

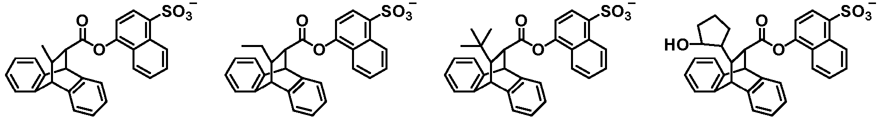

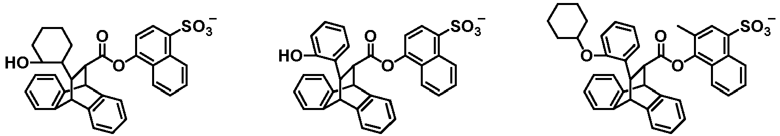

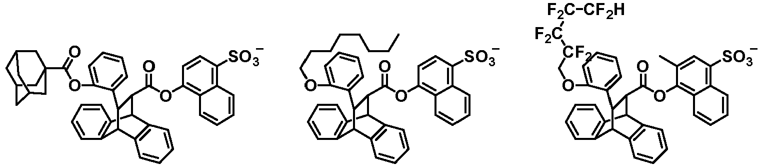

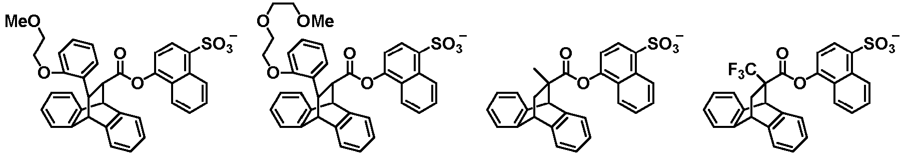

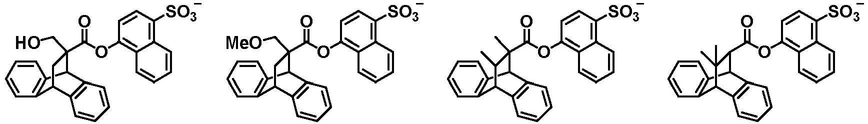

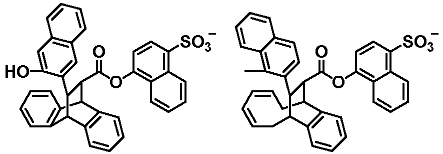

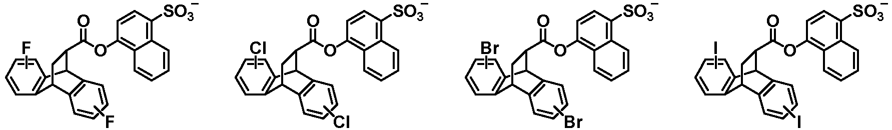

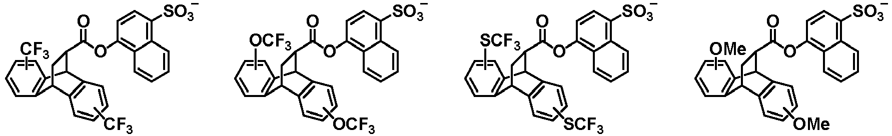

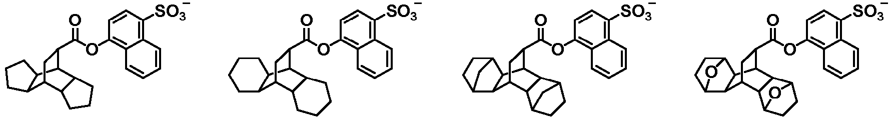

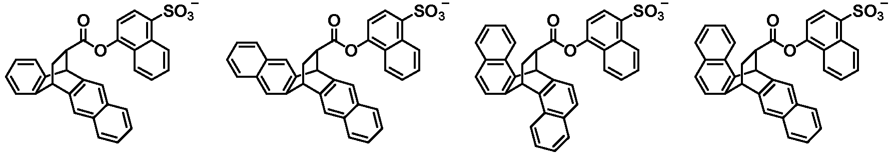

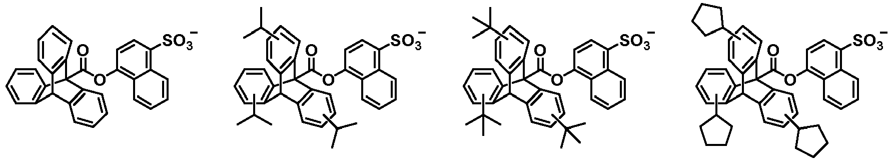

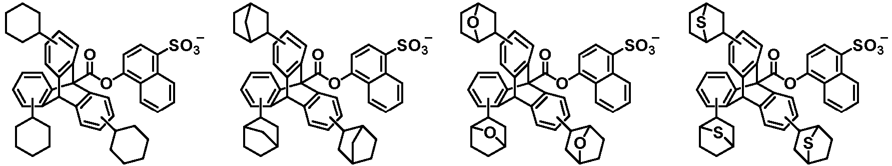

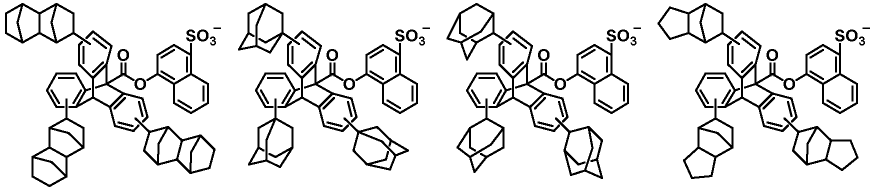

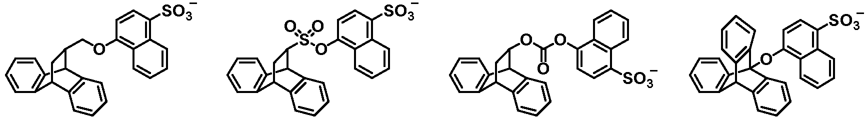

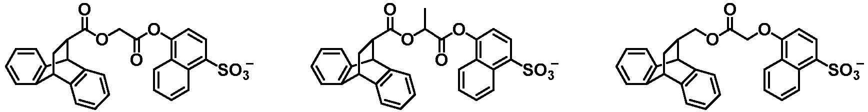

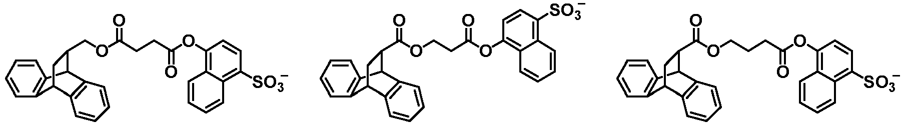

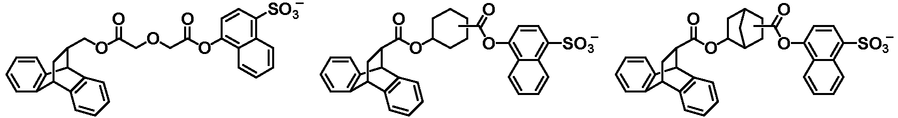

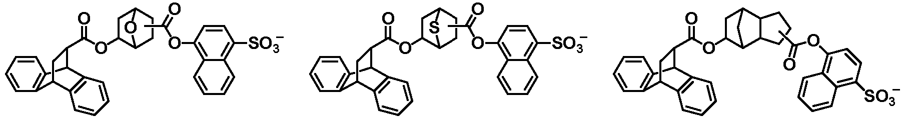

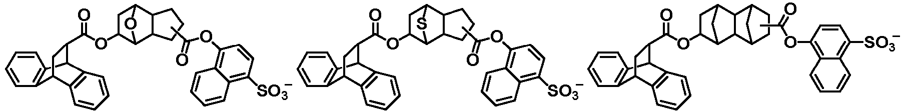

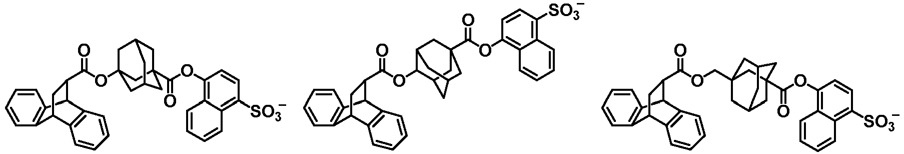

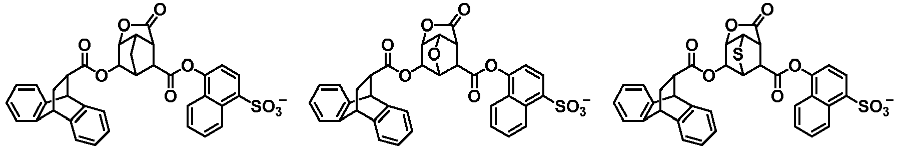

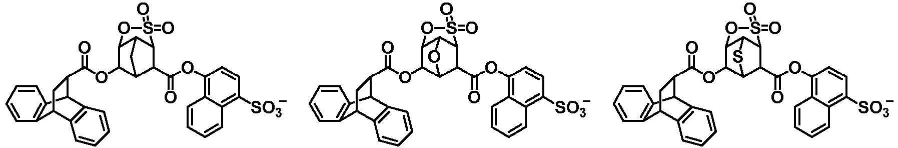

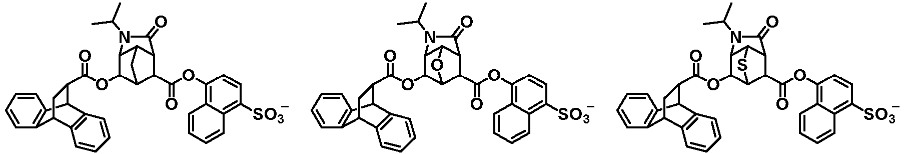

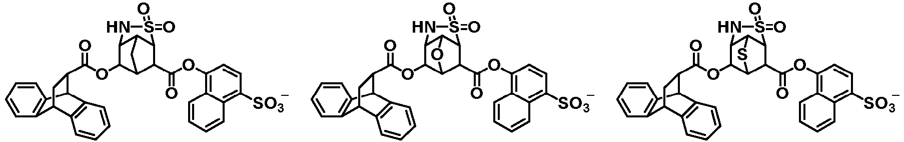

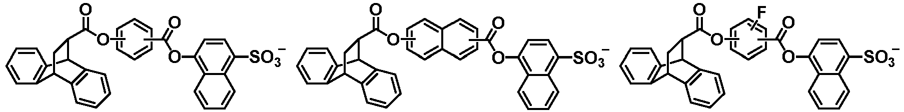

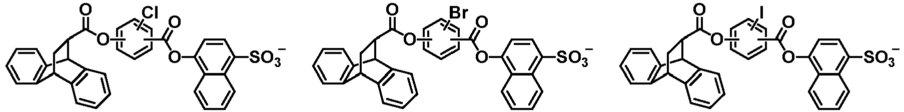

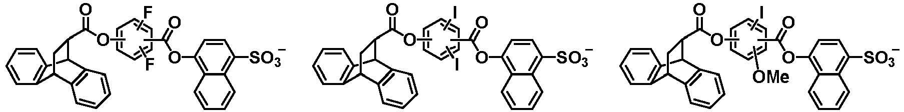

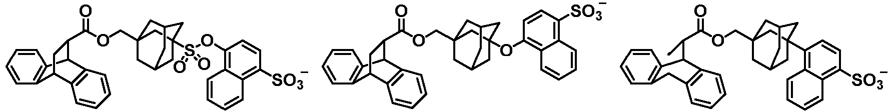

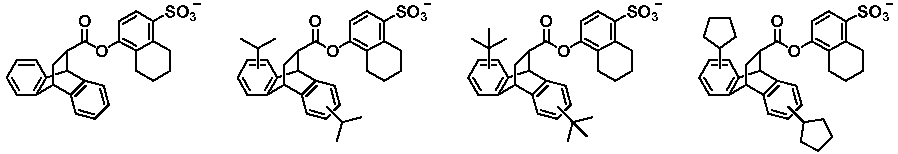

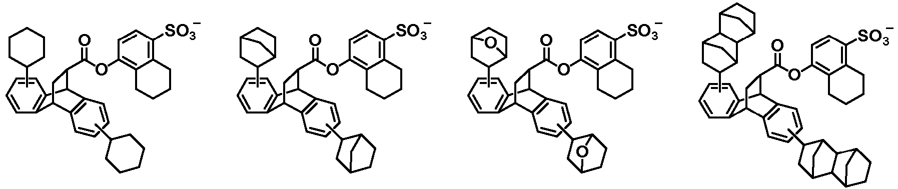

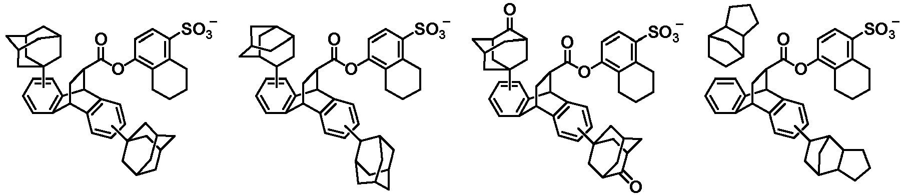

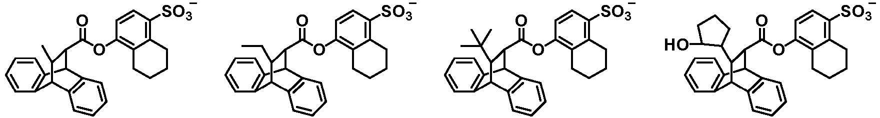

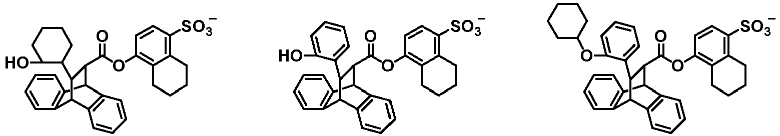

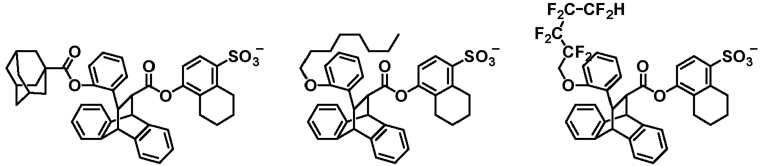

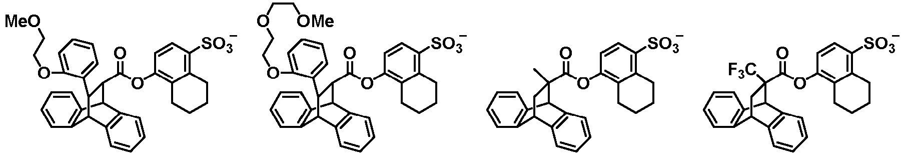

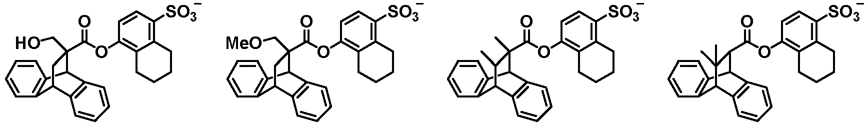

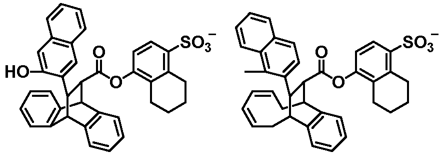

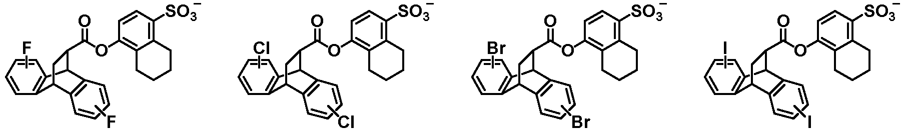

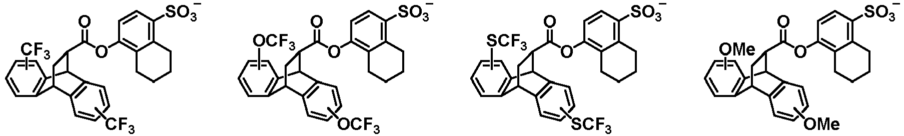

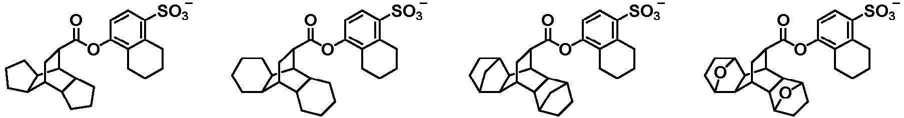

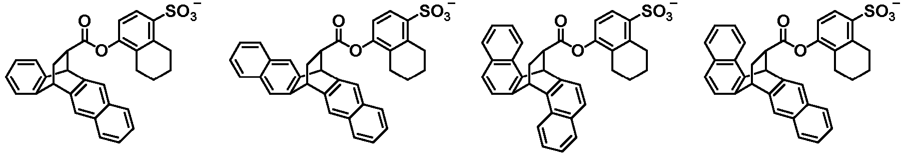

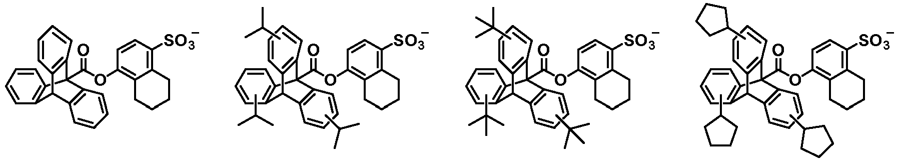

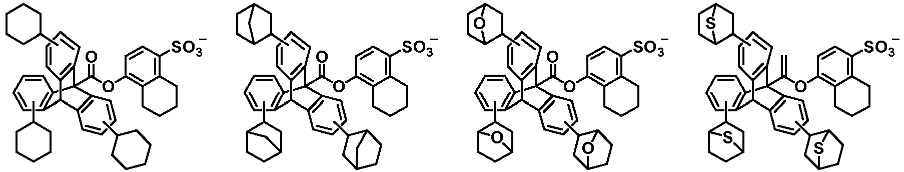

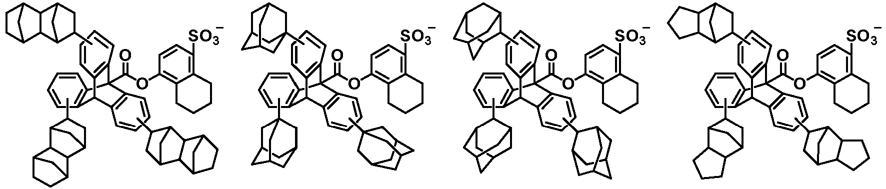

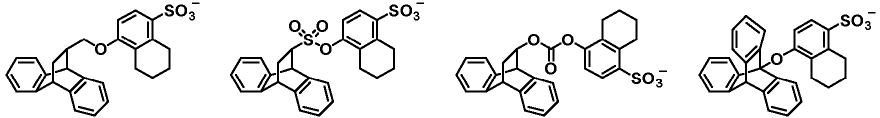

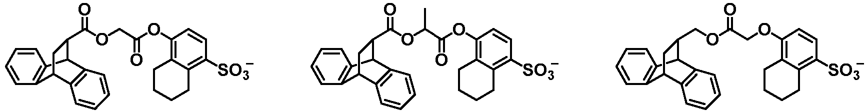

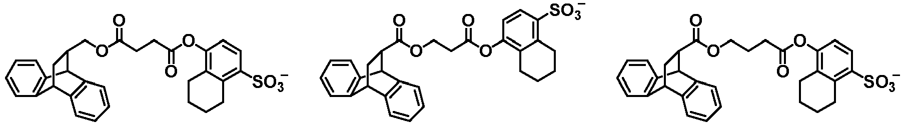

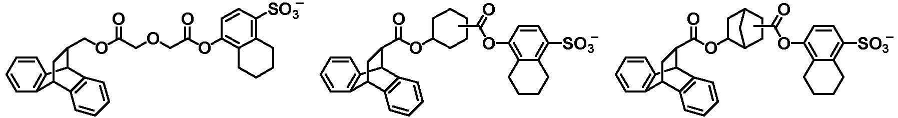

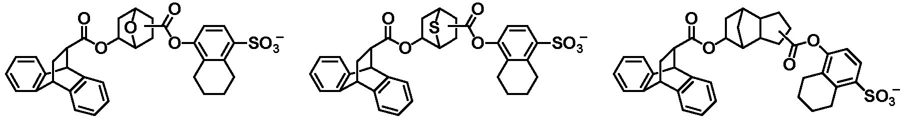

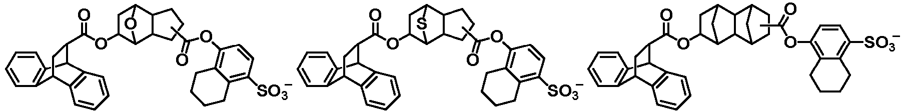

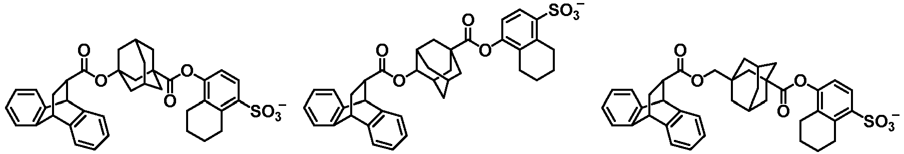

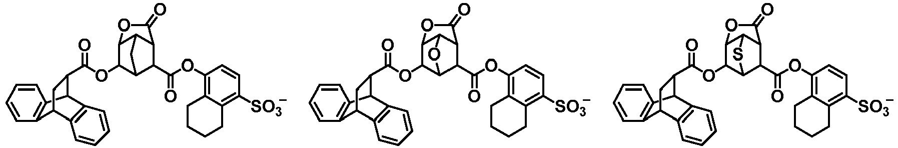

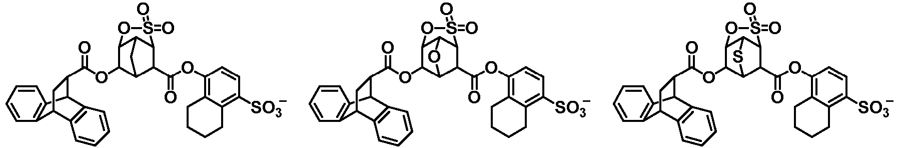

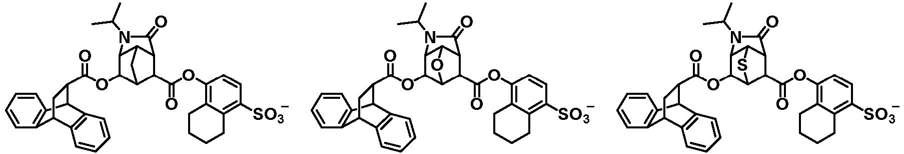

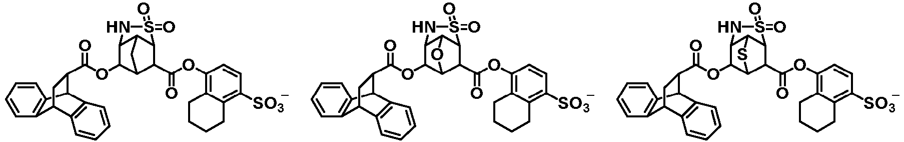

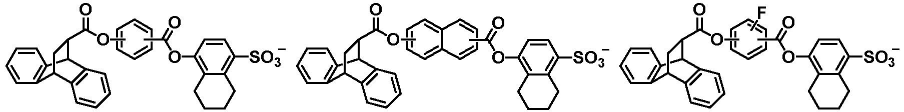

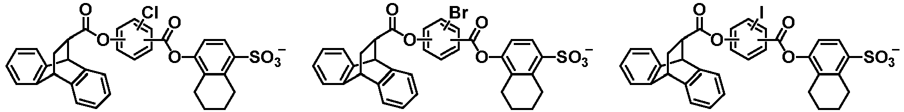

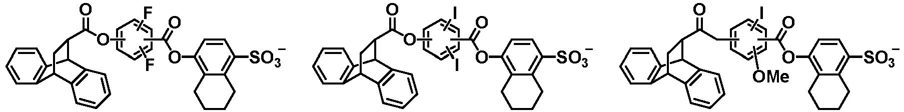

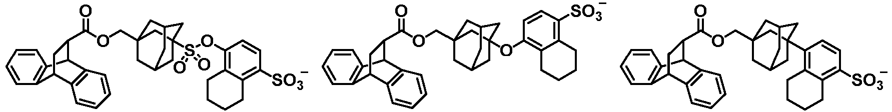

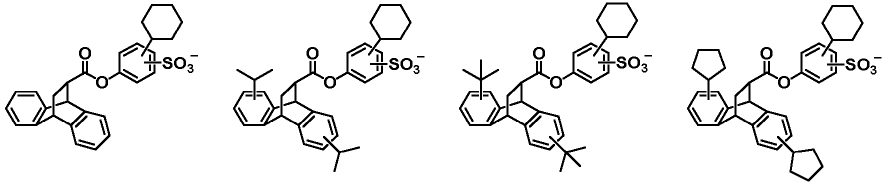

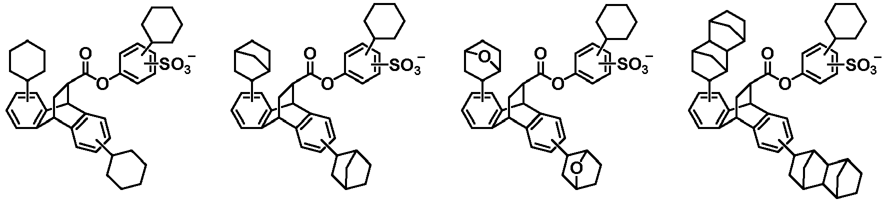

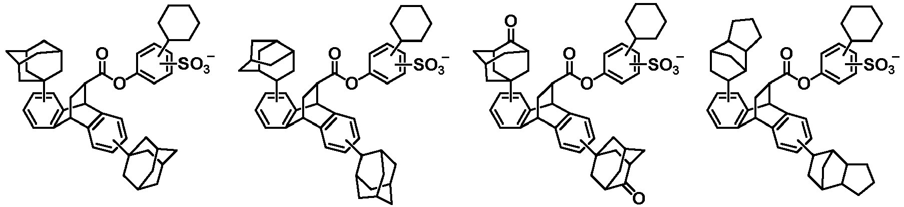

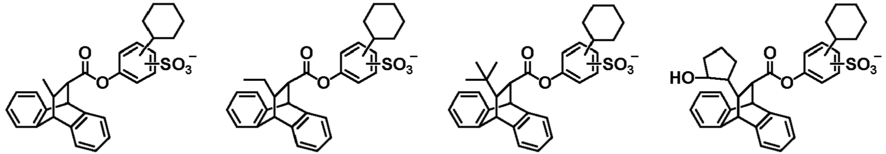

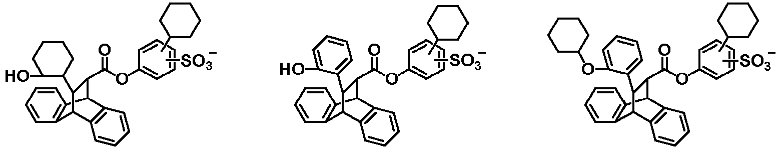

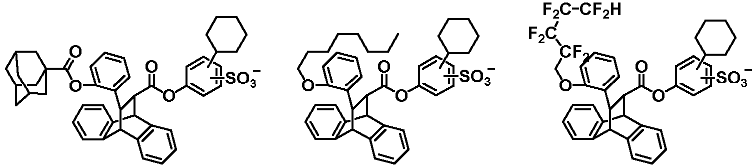

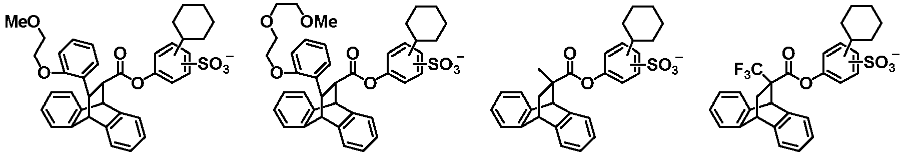

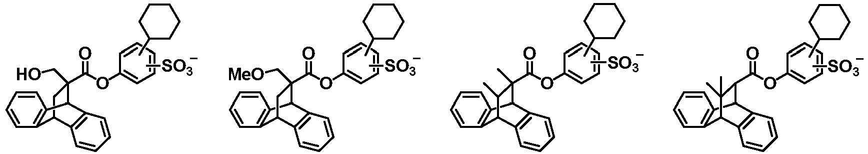

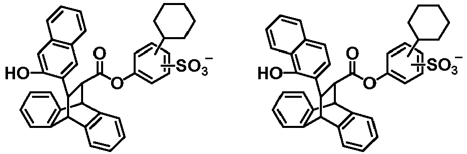

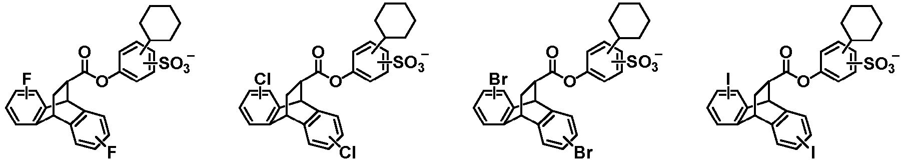

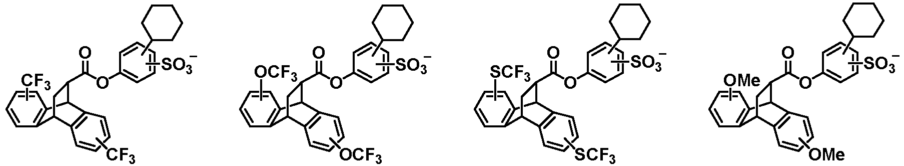

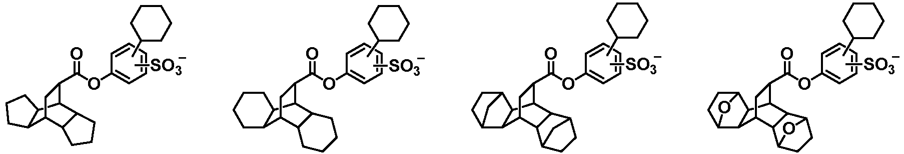

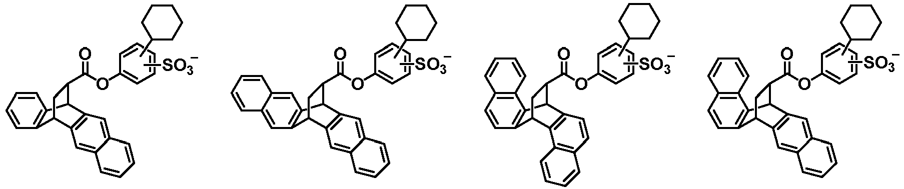

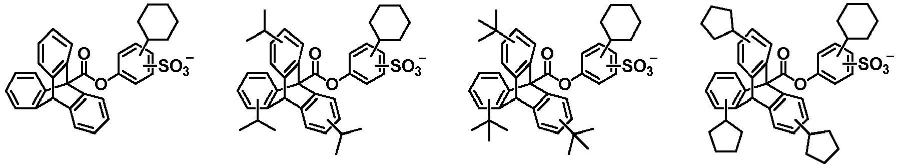

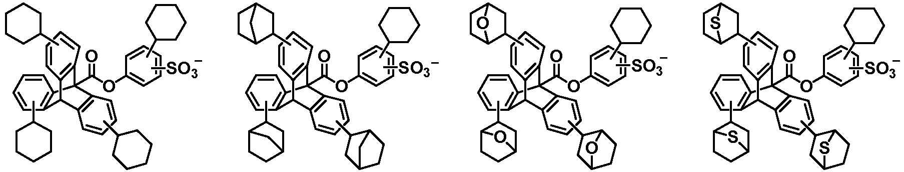

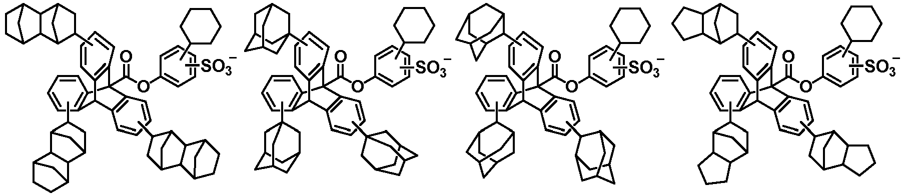

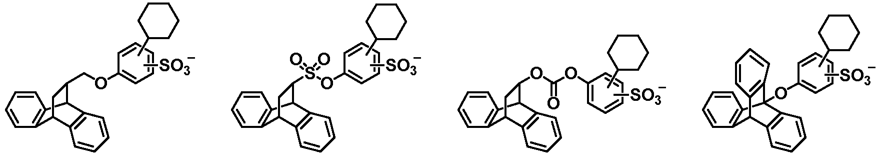

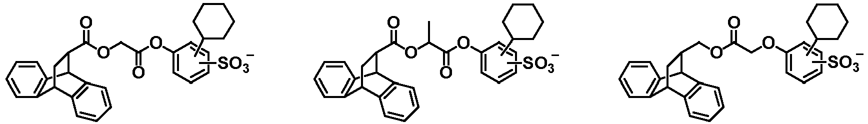

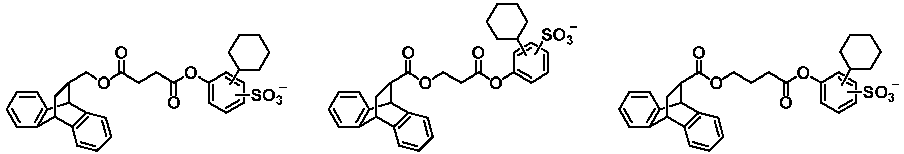

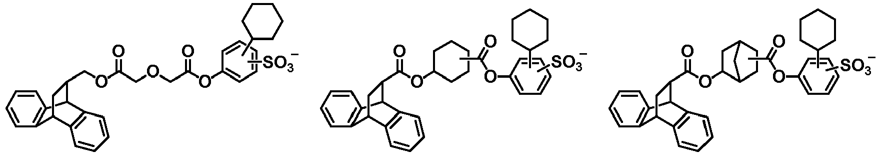

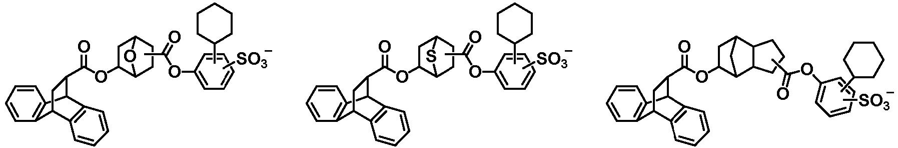

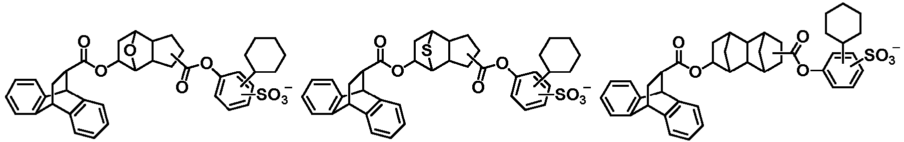

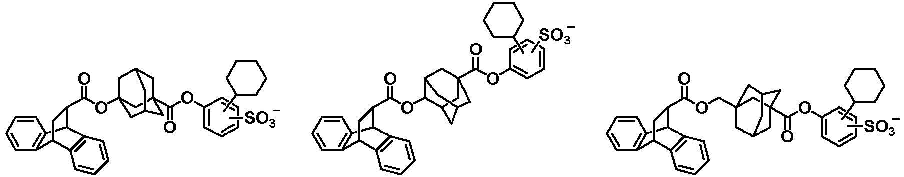

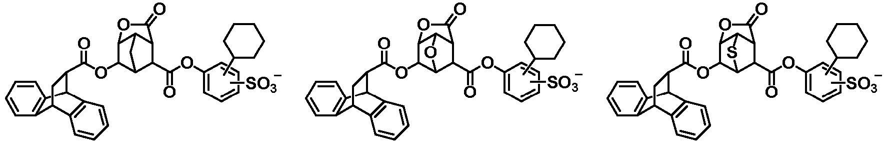

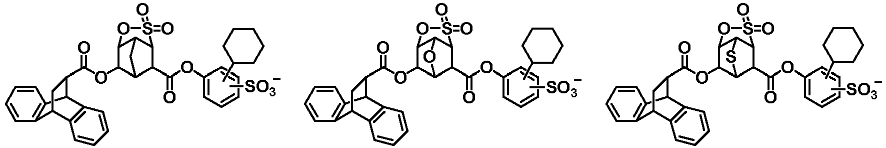

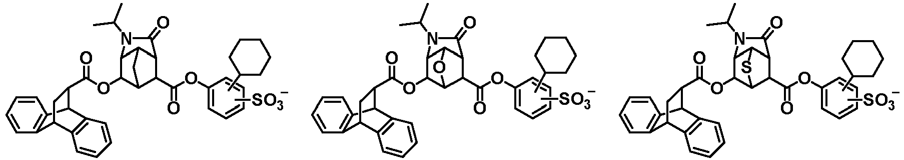

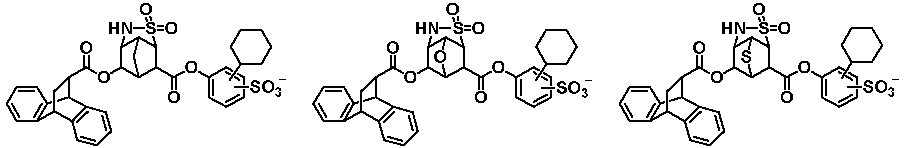

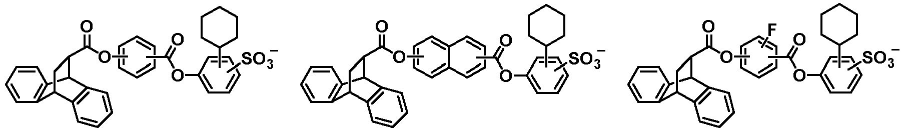

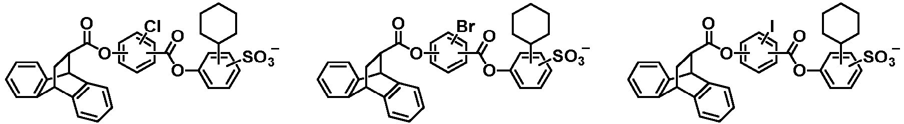

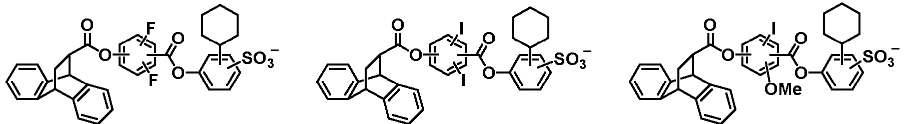

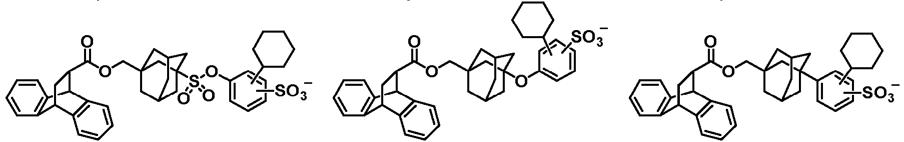

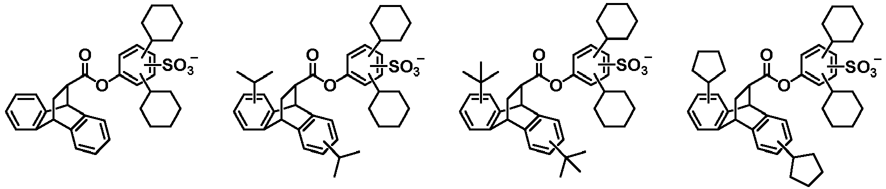

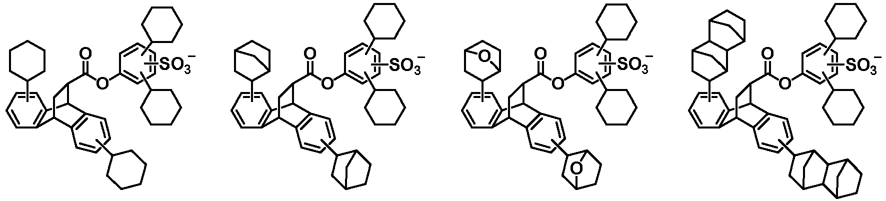

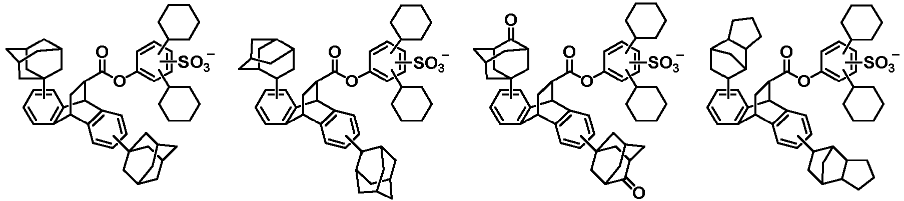

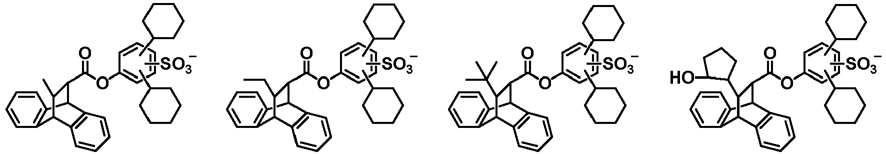

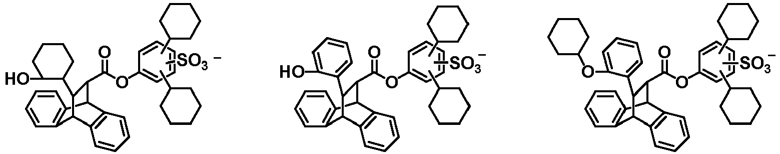

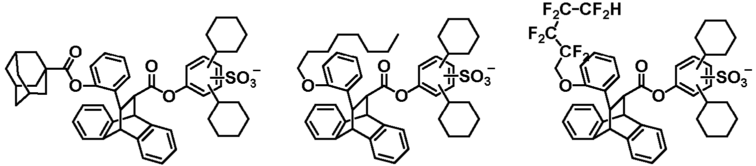

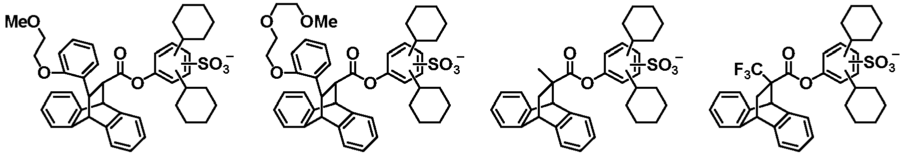

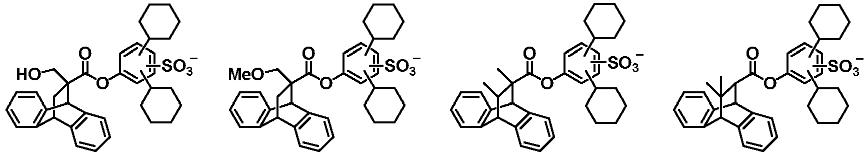

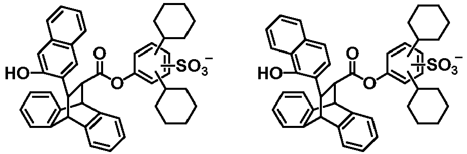

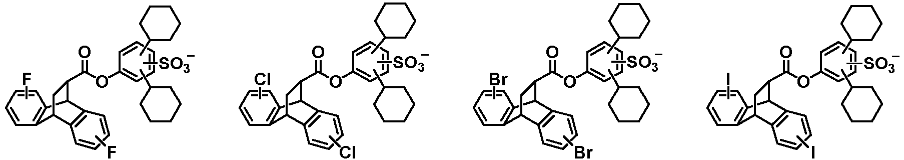

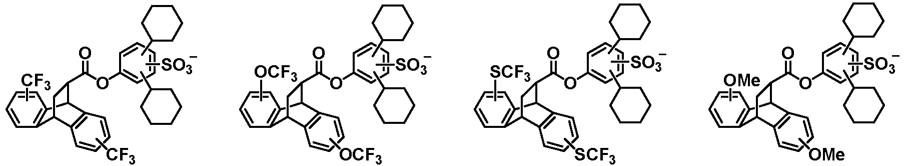

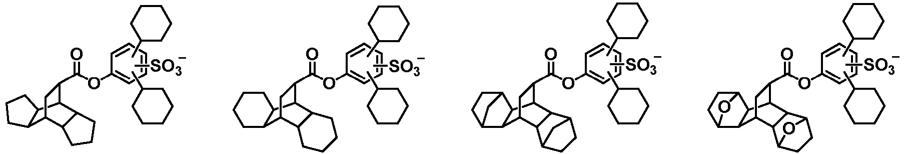

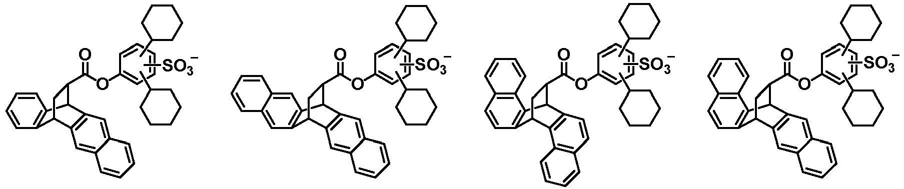

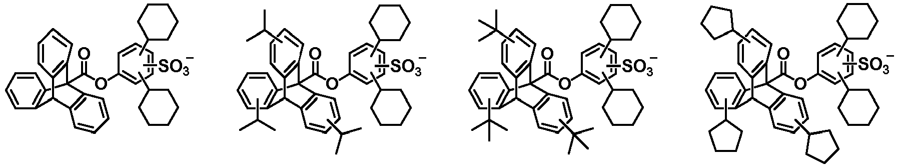

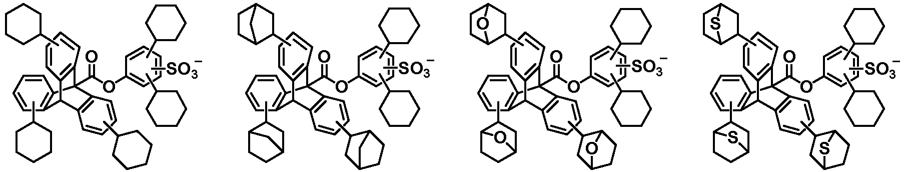

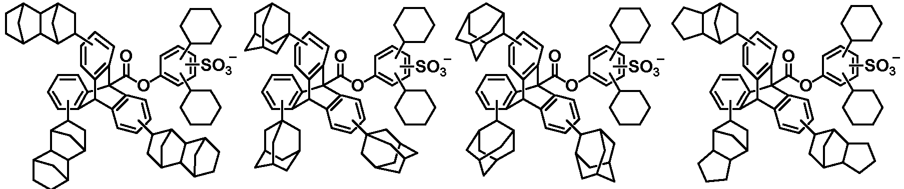

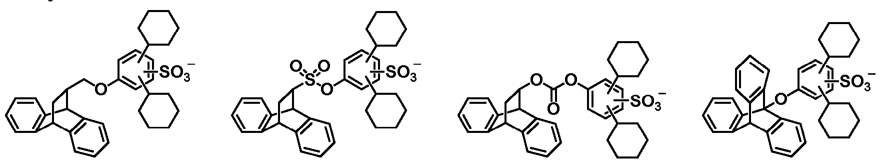

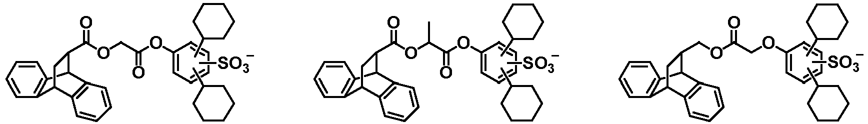

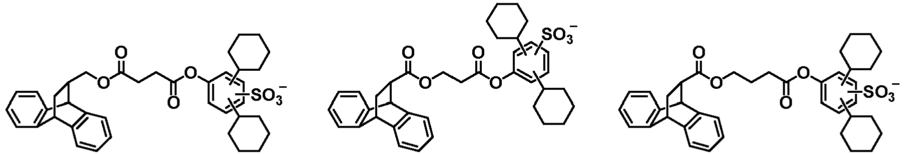

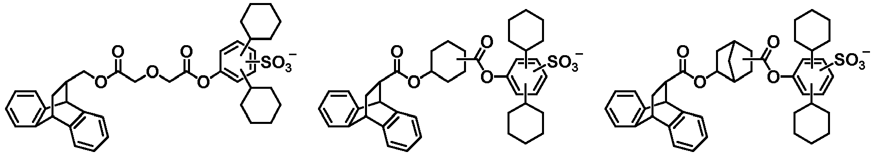

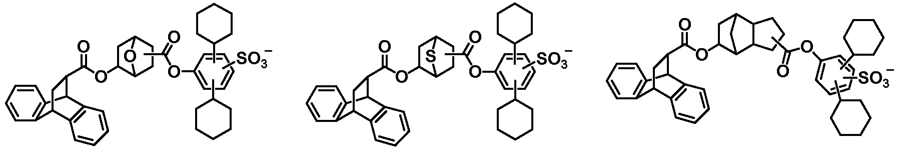

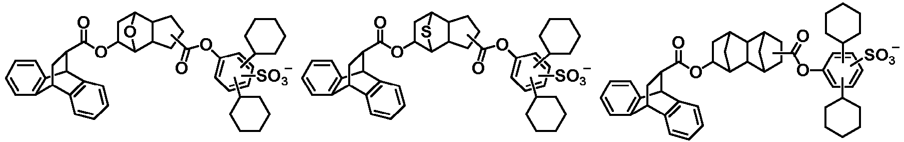

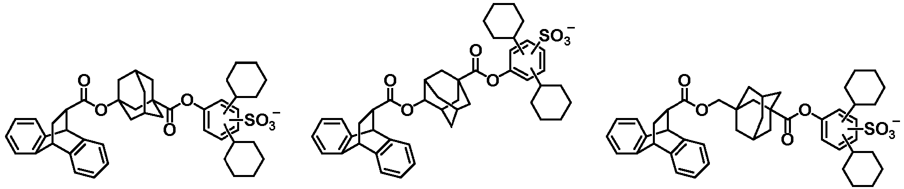

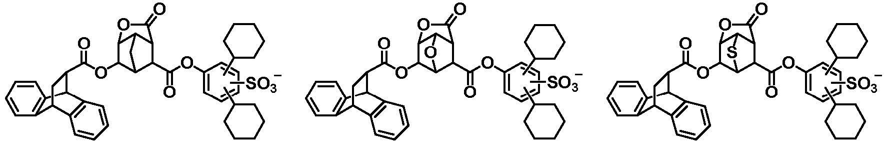

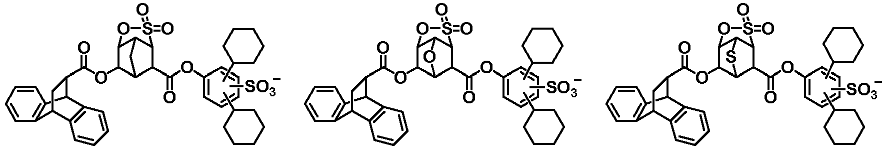

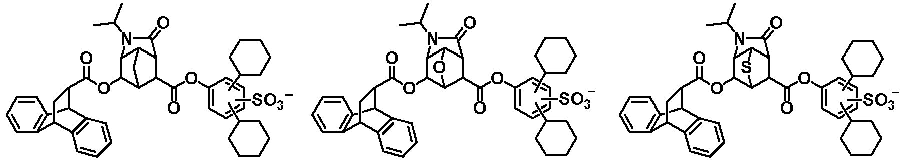

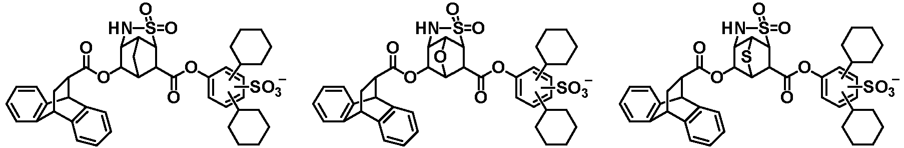

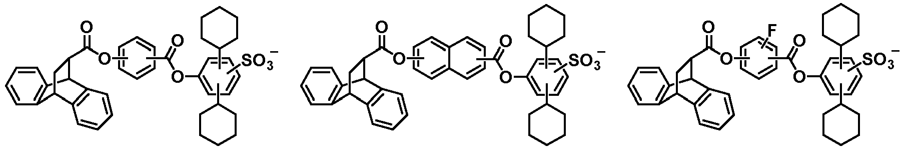

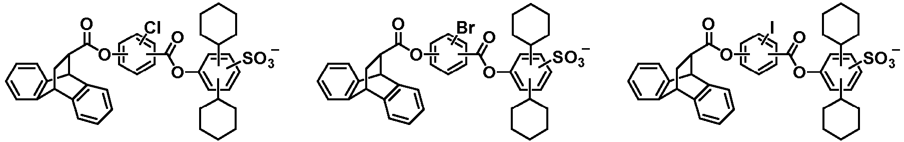

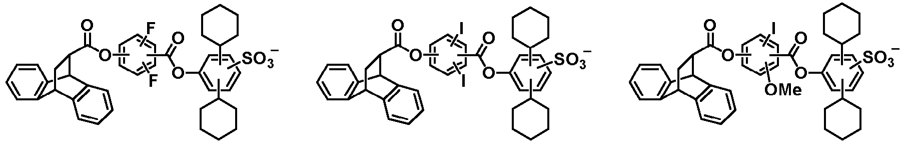

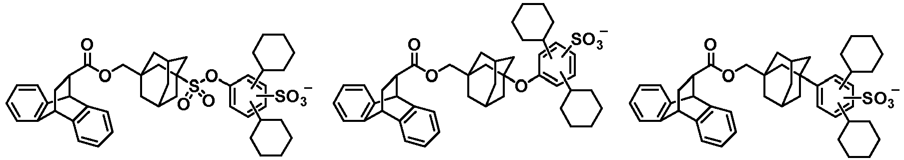

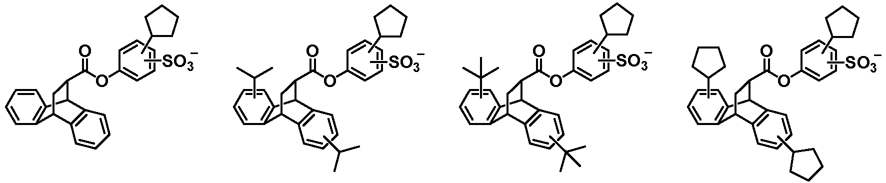

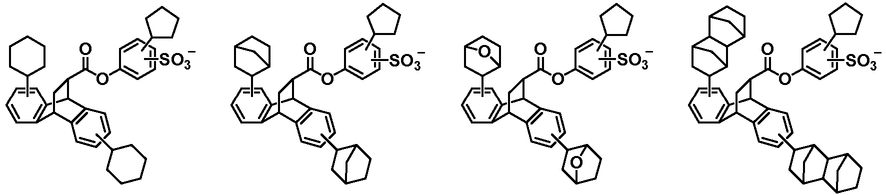

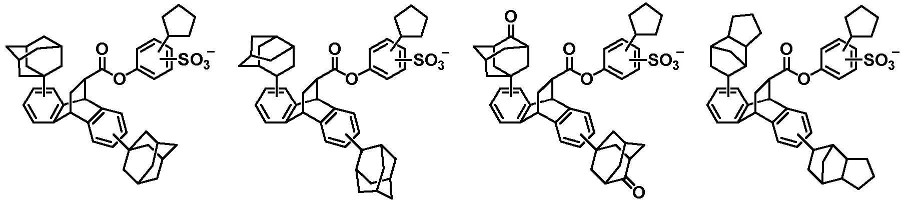

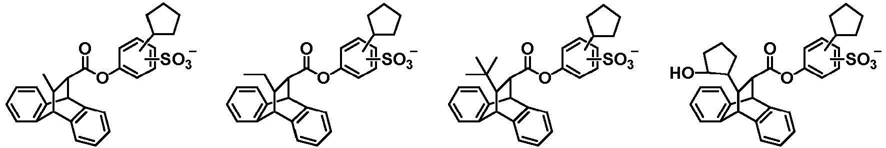

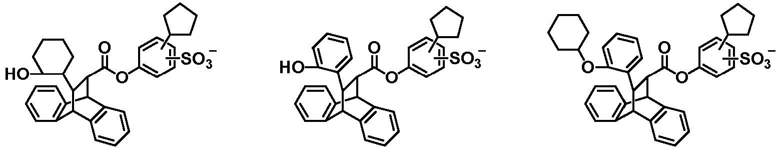

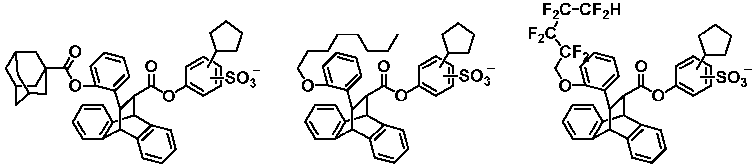

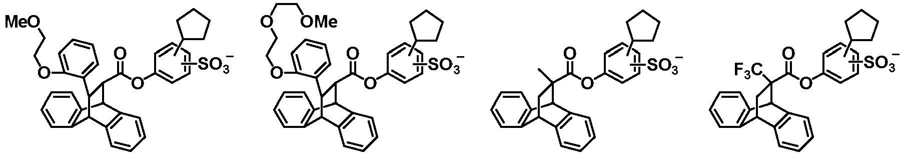

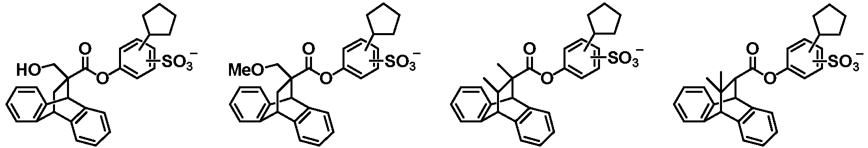

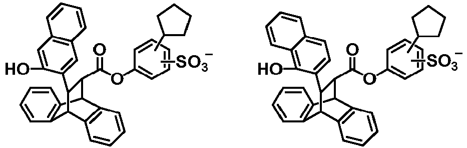

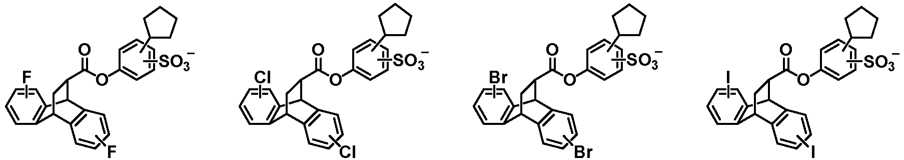

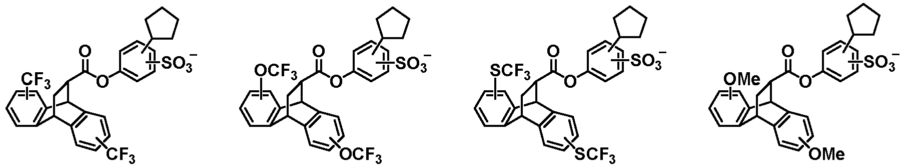

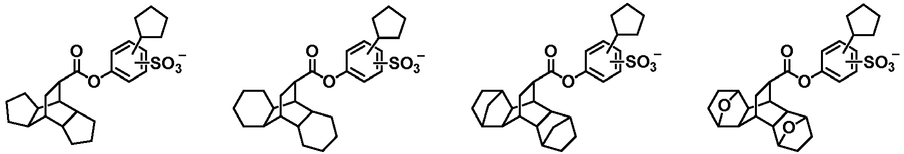

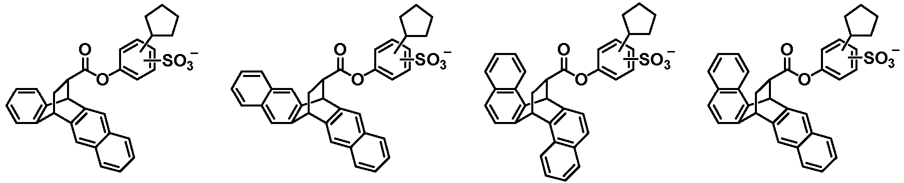

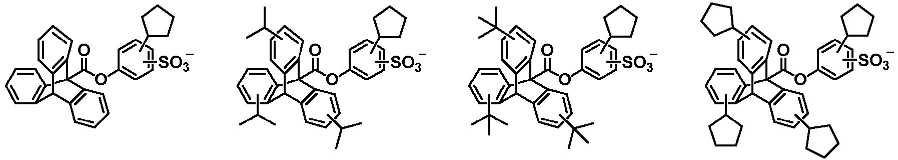

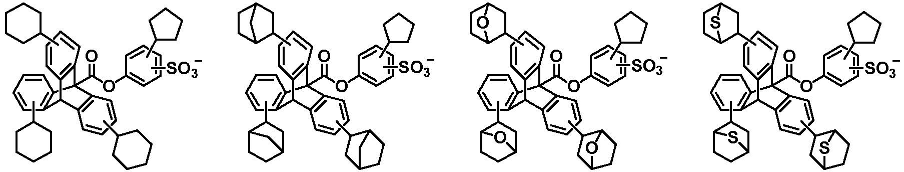

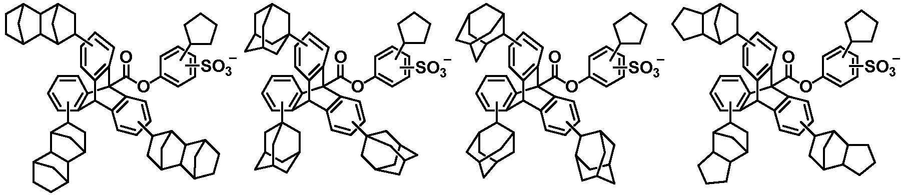

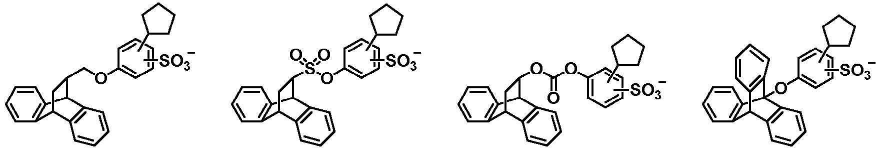

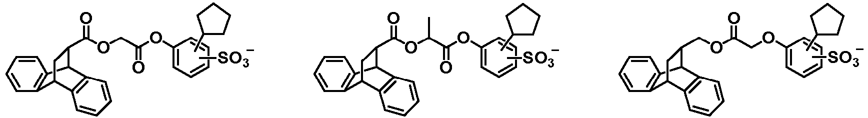

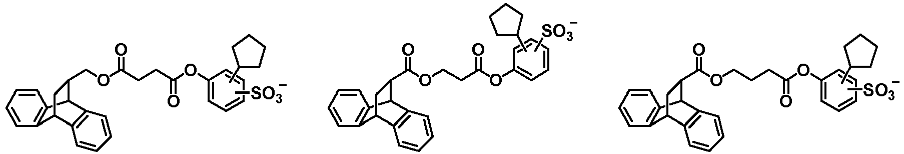

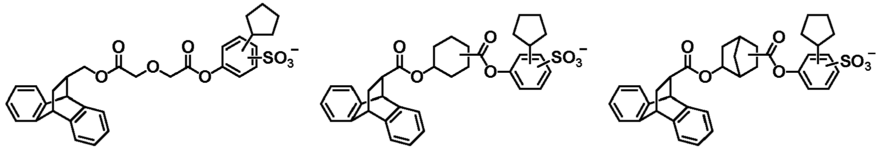

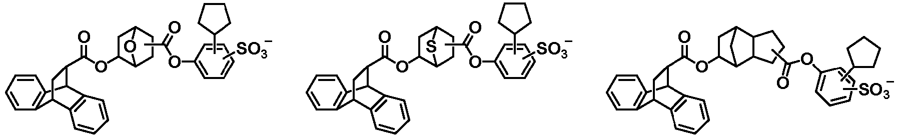

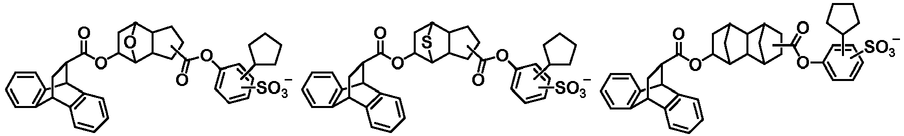

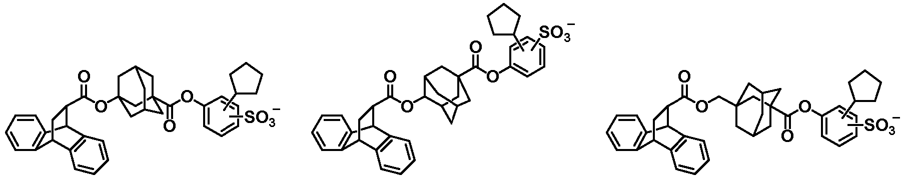

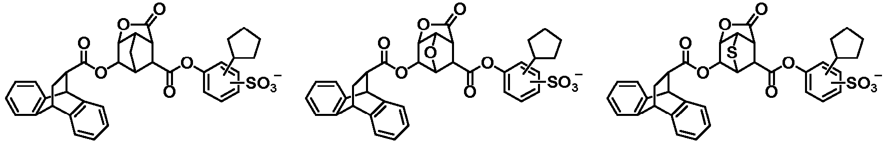

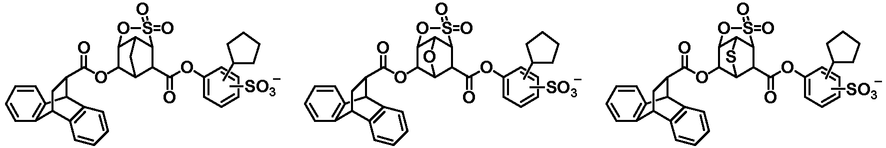

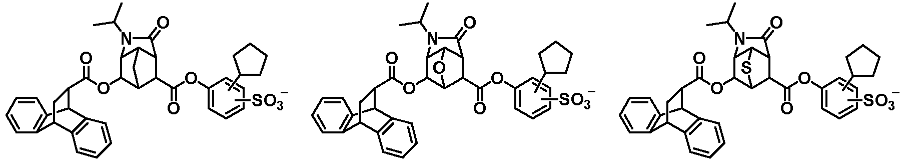

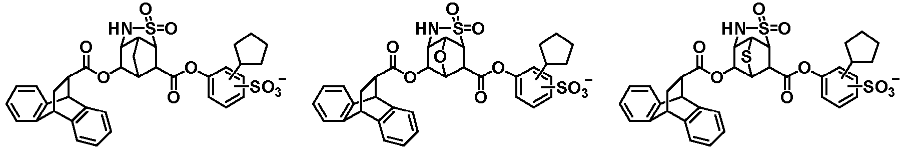

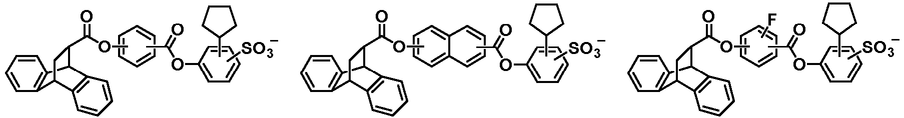

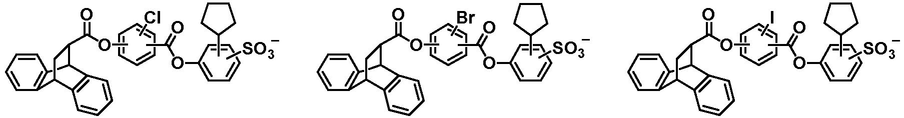

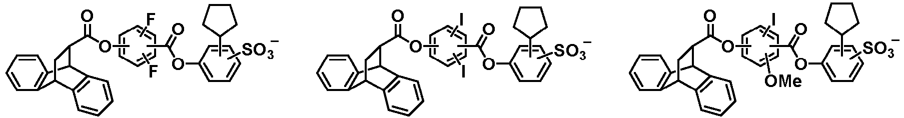

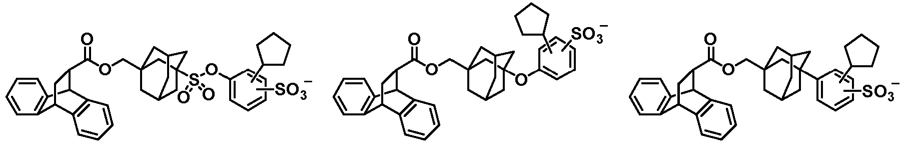

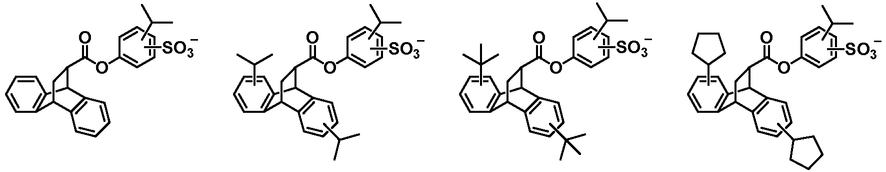

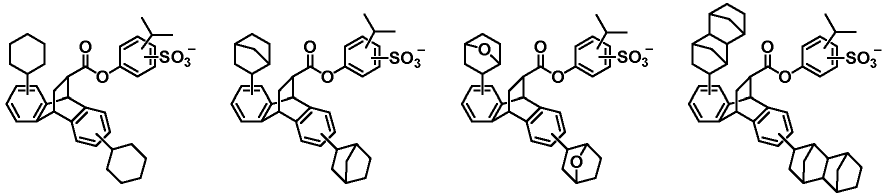

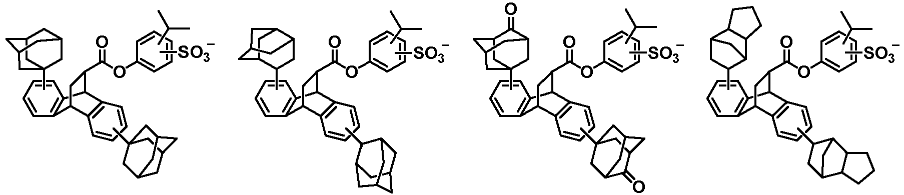

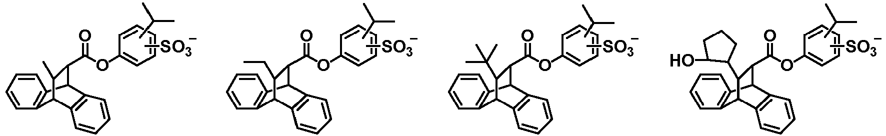

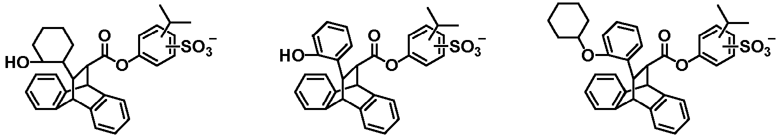

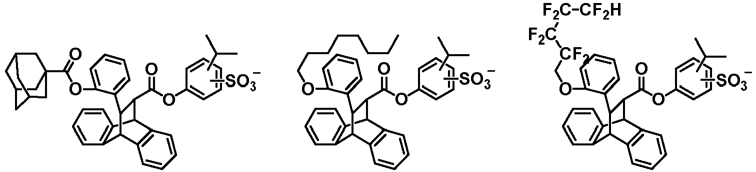

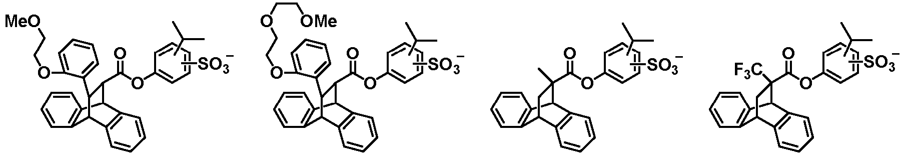

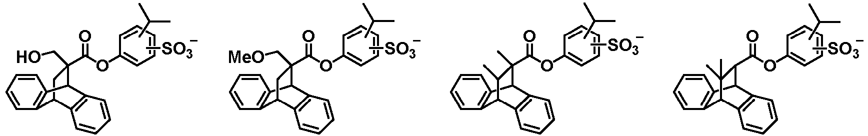

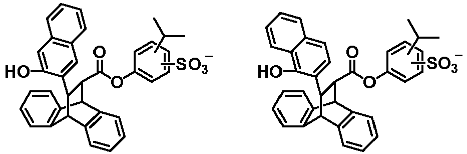

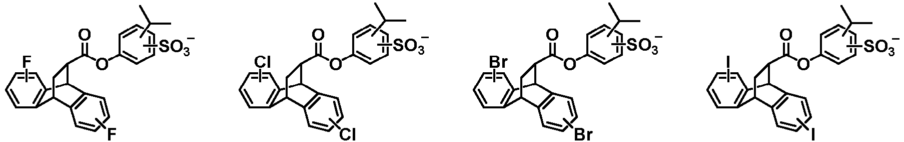

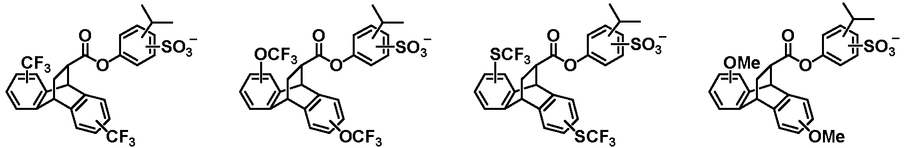

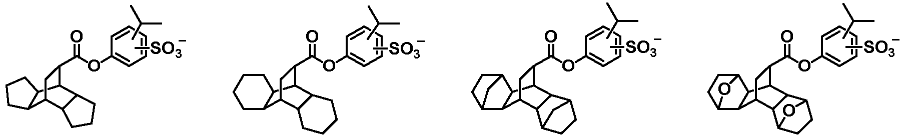

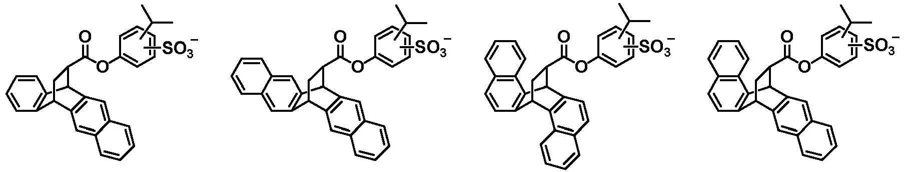

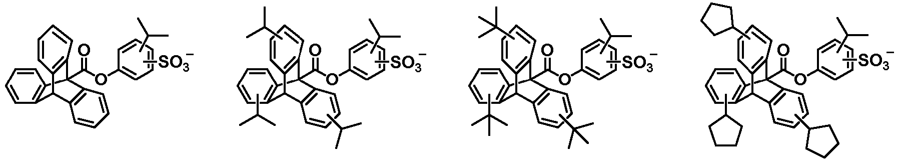

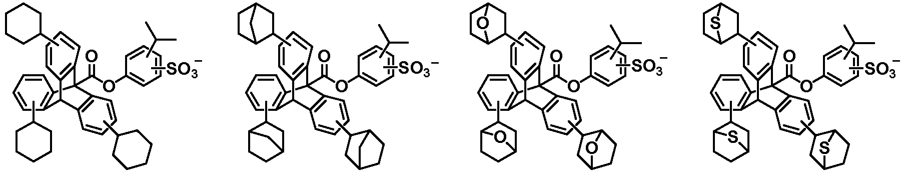

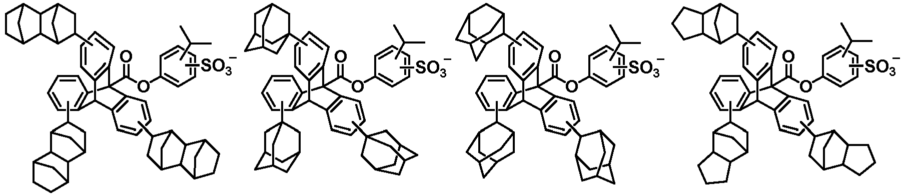

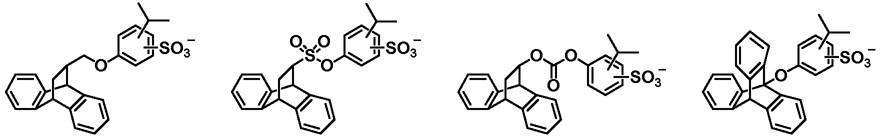

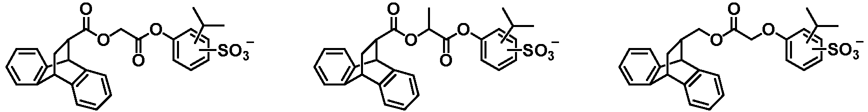

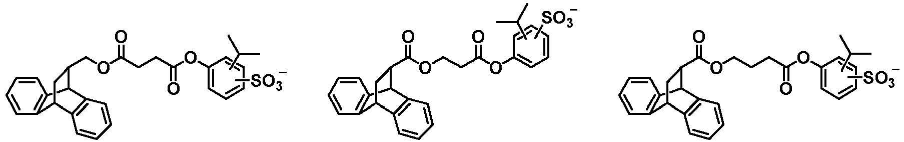

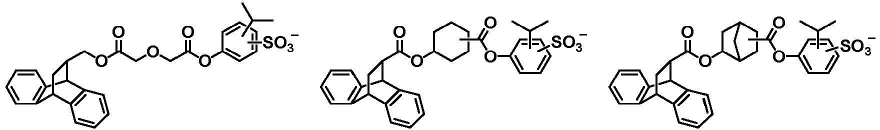

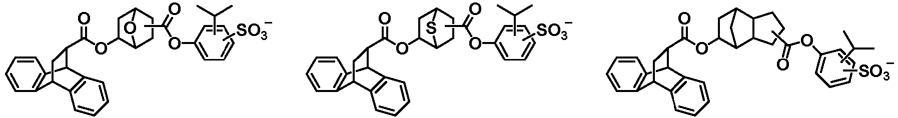

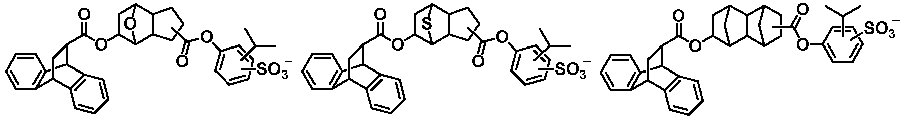

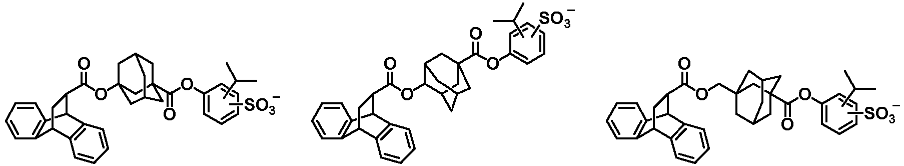

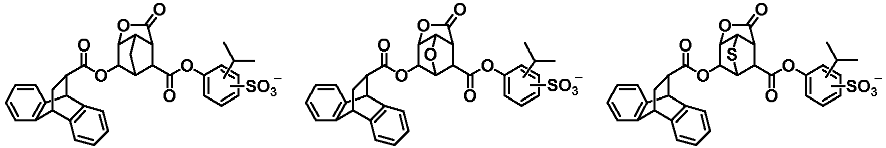

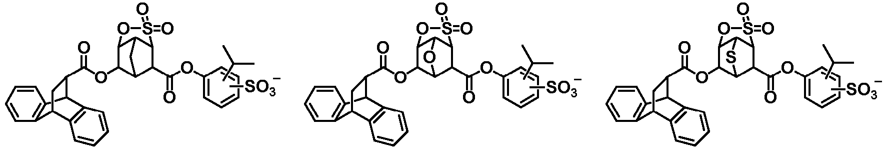

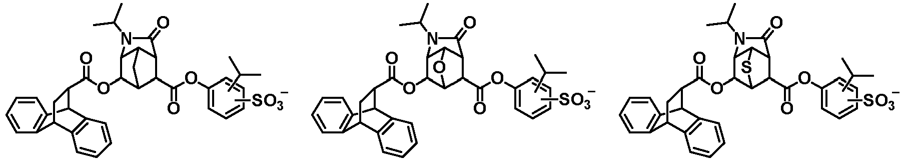

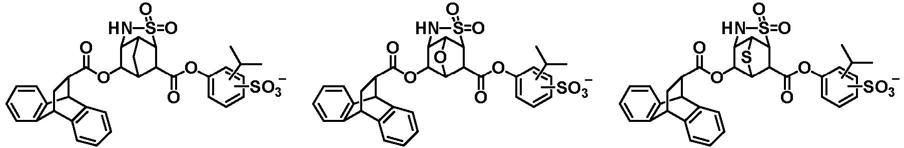

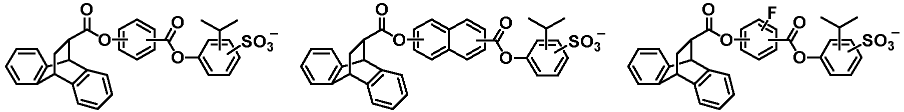

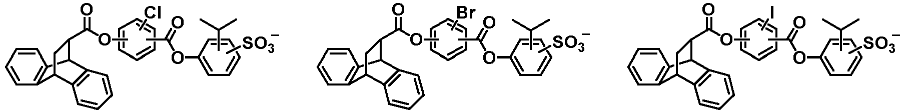

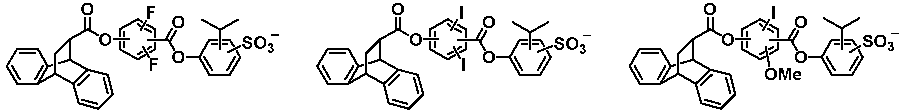

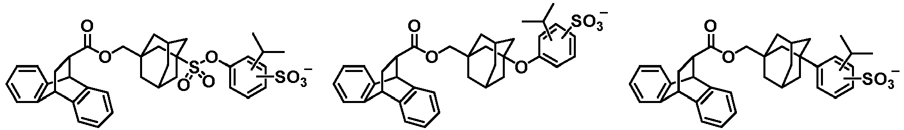

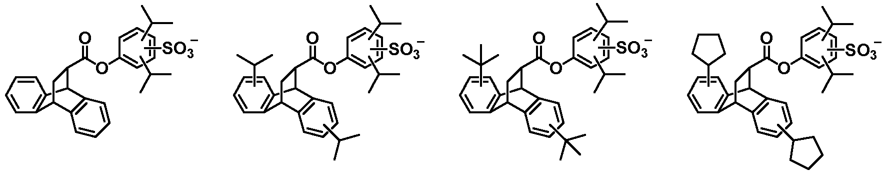

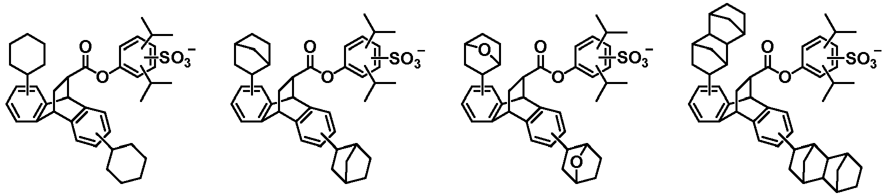

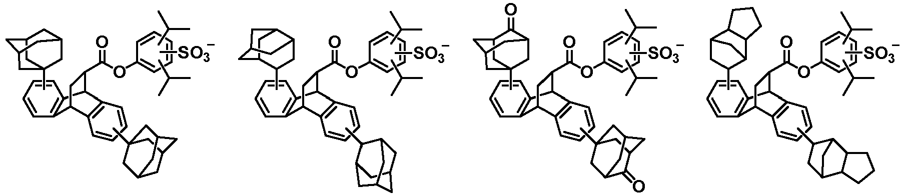

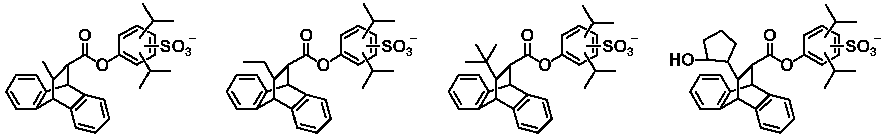

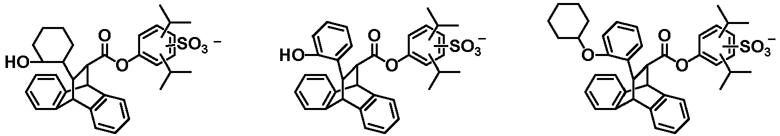

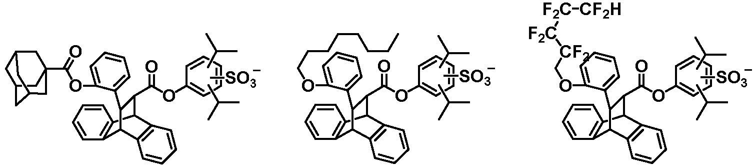

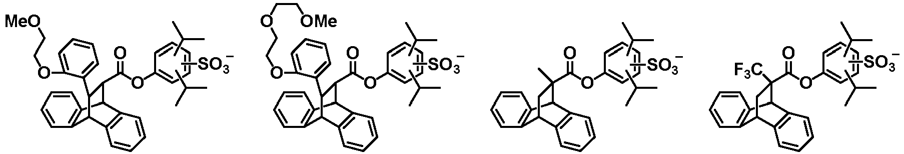

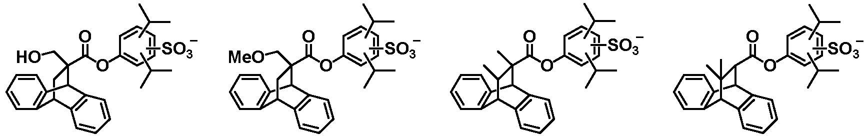

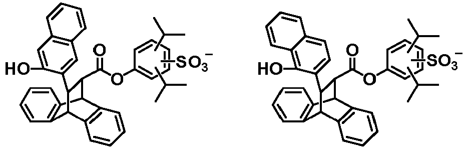

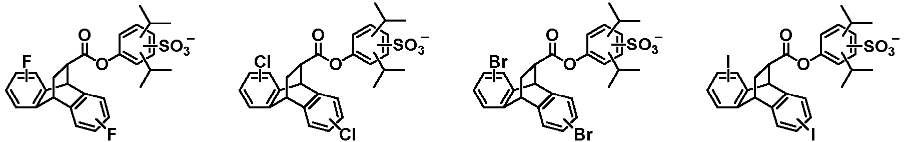

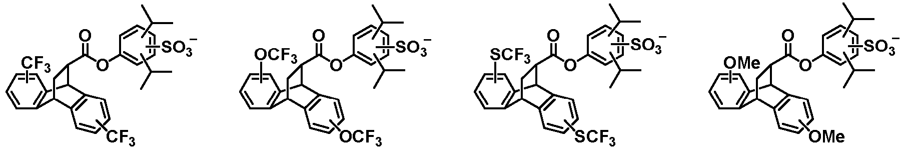

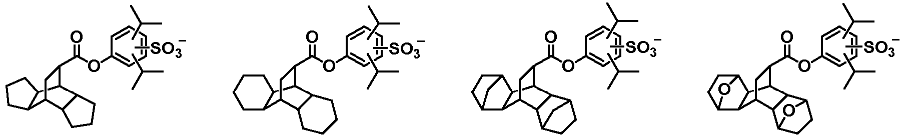

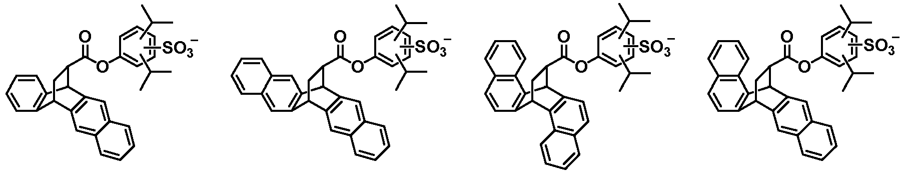

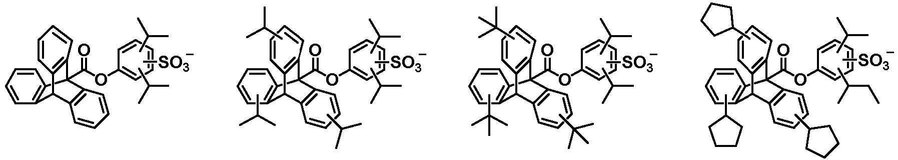

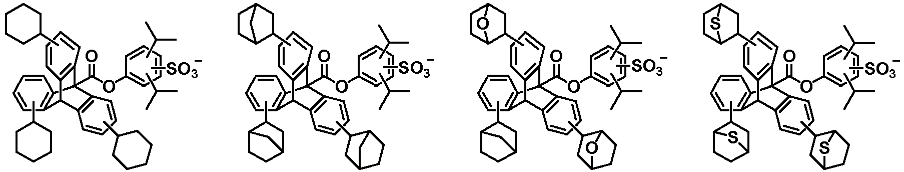

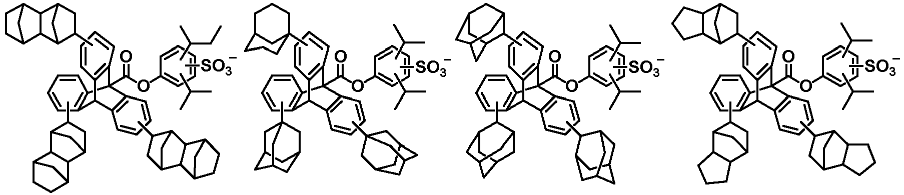

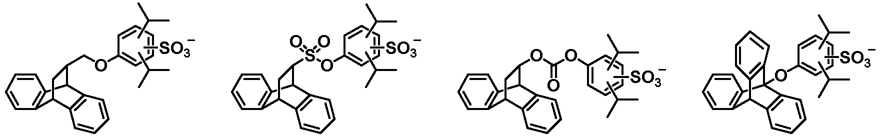

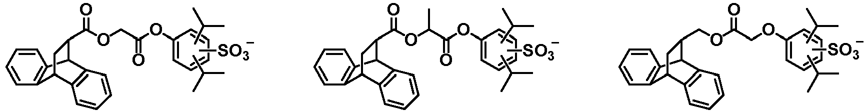

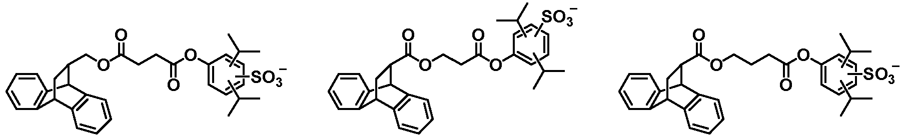

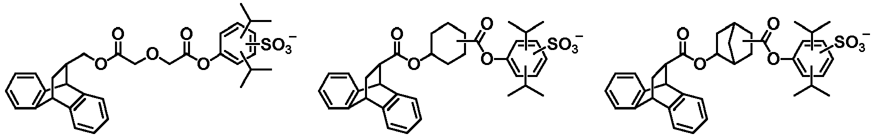

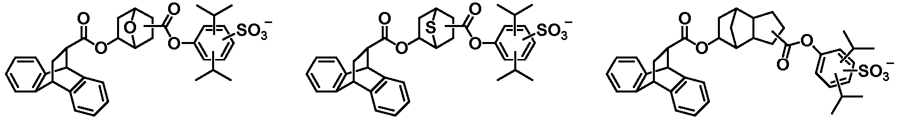

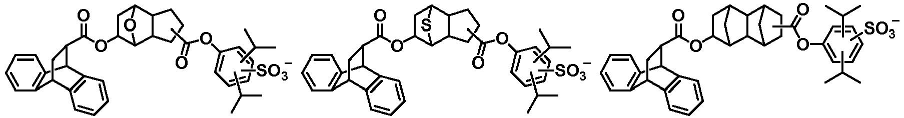

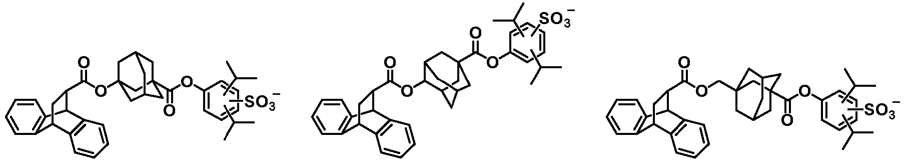

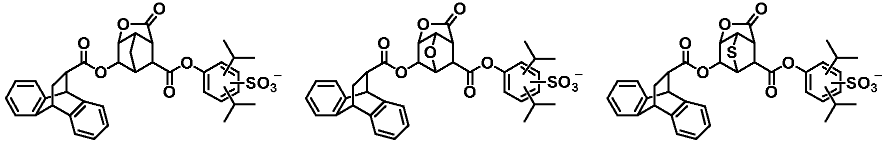

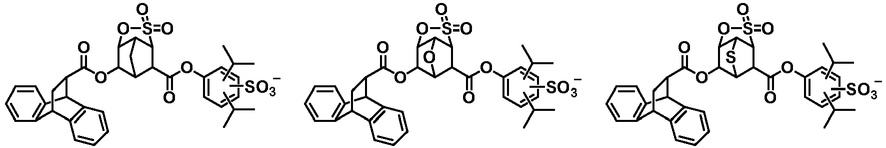

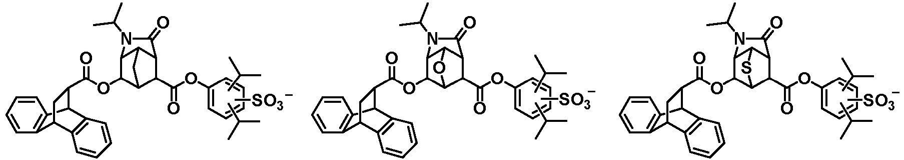

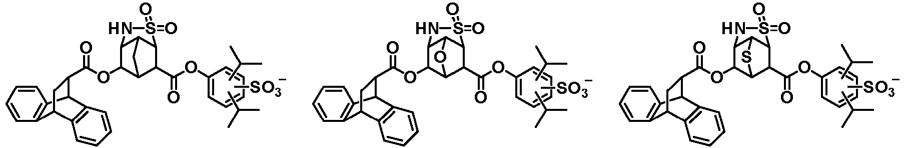

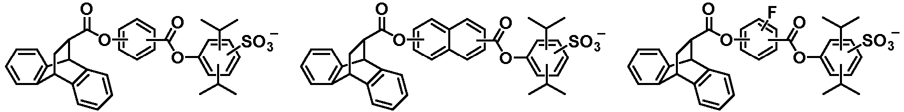

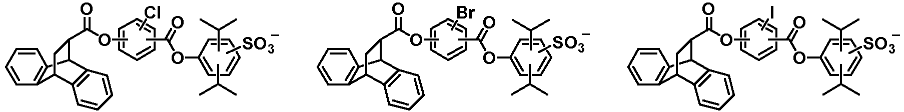

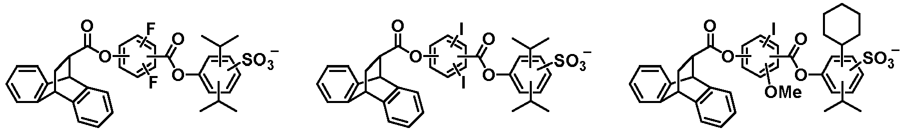

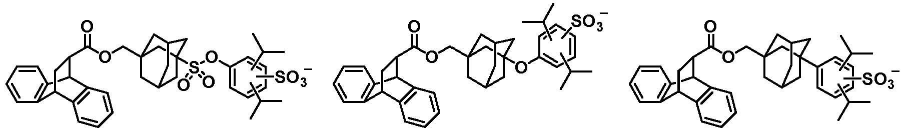

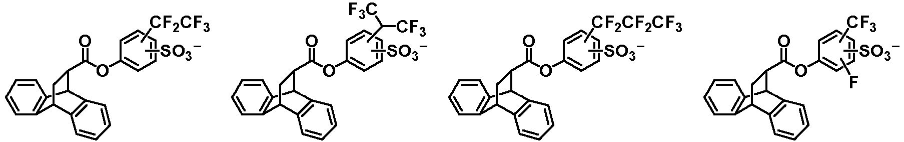

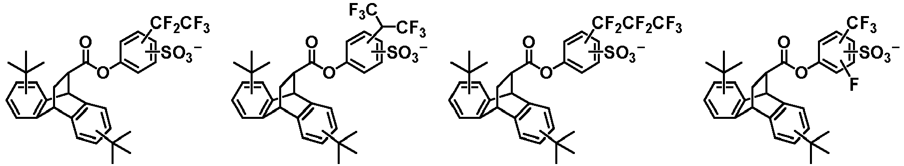

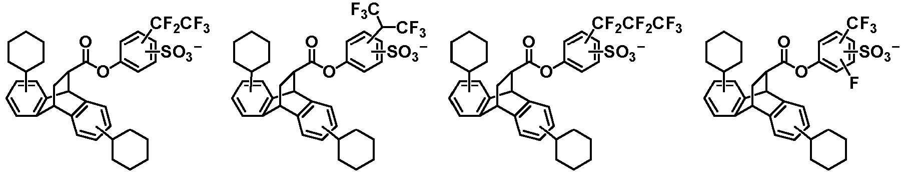

Also preferred as the PAG is a salt compound containing an anion having the formula (D1).

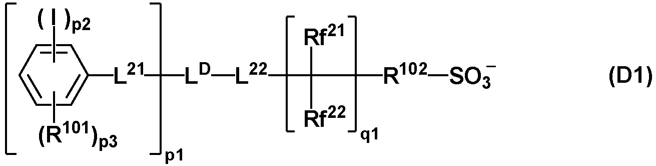

In formula (D1), p1 is 1, 2 or 3. The subscript p2 is 1, 2, 3, 4 or 5. The subscript p3 is 0, 1, 2 or 3. The sum p2+p3 is 1 or more and 5 or less. The subscript q1 is 0 or 1.

In formula (D1), L²¹ is a single bond, ether bond, ester bond, carbonyl group, sulfonic ester bond, carbonate bond, or carbamate bond.

In formula (D1), L²² is an ether bond, ester bond, carbonyl group, sulfonic ester bond, carbonate bond, or carbamate bond.

In formula (D1), L^{D} is a single bond or a C₁-C₂₀ hydrocarbylene group when p1 is 1, and a C₁-C₂₀ (p1+1)-valent hydrocarbon group when p1 is 2 or 3. The hydrocarbylene group and (p1+1)-valent hydrocarbon group may contain at least one moiety selected from an ether bond, a carbonyl group, an ester bond, an amide bond, a sultone ring, a lactam ring, a carbonate bond, a halogen atom, a hydroxy group and a carboxy group.

The C₁-C₂₀ hydrocarbylene group L^{D} may be saturated or unsaturated and straight, branched or cyclic. Specific examples thereof include C₁-C₂₀ alkanediyl groups such as methanediyl, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl and dodecane-1,12-diyl; C₃-C₂₀ cyclic saturated hydrocarbylene groups such as cyclopentanediyl, cyclohexanediyl, norbornanediyl and adamantanediyl; C₂-C₂₀ unsaturated aliphatic hydrocarbylene groups such as vinylene and propene-1,3-diyl; C₆-C₂₀ arylene groups such as phenylene and naphthylene; and groups obtained by combining the foregoing. The C₁-C₂₀ (p1+1)-valent hydrocarbon group represented by L^{D} may be saturated or unsaturated, and may be straight, branched, or cyclic. Examples thereof include those exemplified above for the C₁-C₂₀ hydrocarbylene group, with one or two hydrogen atoms being eliminated.

In formula (D1), Rf²¹ and Rf²² are each independently hydrogen, fluorine or trifluoromethyl, at least one of Rf²¹ and Rf²² is fluorine or trifluoromethyl.

In formula (D1), R¹⁰¹ is hydroxy, carboxy, a C₁-C₆ saturated hydrocarbyl group, C₁-C₆ saturated hydrocarbyloxy group, C₂-C₆ saturated hydrocarbylcarbonyloxy group, fluorine, chlorine, bromine, -N(R^{101A})(R^{101B}), -N(R^{101C})-C(=O)-R^{101D} or -N(R^{101C})-C(=O)-O-R^{101D}. R^{101A} and R^{101B} are each independently hydrogen, or a C₁-C₆ saturated hydrocarbyl group. R^{101C} is hydrogen or a C₁-C₆ saturated hydrocarbyl group. R^{101D} is a C₁-C₆ saturated hydrocarbyl group or C₂-C₈ unsaturated hydrocarbyl group.

The C₁-C₆ saturated hydrocarbyl group represented by R¹⁰¹, R^{101A}, R^{101B} and R^{101C} may be straight, branched or cyclic. Examples thereof include C₁-C₆ alkyl groups such as methyl, ethyl, n-propyl, isopropyl, n-butyl, isobutyl, sec-butyl, tert-butyl, n-pentyl, and n-hexyl; and C₃-C₆ cyclic saturated hydrocarbyl groups such as cyclopropyl, cyclobutyl, cyclopentyl, and cyclohexyl. Examples of the saturated hydrocarbyl moiety in the C₁-C₆ saturated hydrocarbyloxy group represented by R¹⁰¹ are as exemplified above for the saturated hydrocarbyl group. Examples of the saturated hydrocarbyl moiety in the C₂-C₆ saturated hydrocarbylcarbonyloxy group represented by R¹⁰¹ are as exemplified above for the C₁-C₆ saturated hydrocarbyl group, but of 1 to 5 carbon atoms.

The C₂-C₈ unsaturated hydrocarbyl group represented by R^{101D} may be straight, branched or cyclic. Examples thereof include C₂-C₈ alkenyl groups such as vinyl, propenyl, butenyl, and hexenyl; C₂-C₈ alkynyl groups such as ethynyl, propynyl, and butynyl; and C₃-C₈ cyclic unsaturated aliphatic hydrocarbyl groups such as cyclohexenyl and norbornenyl.

In formula (D1), R¹⁰² is a C₁-C₂₀ saturated hydrocarbylene group or C₆-C₂₀ arylene group. Some or all of the hydrogen atoms in the saturated hydrocarbylene group may be substituted by halogen exclusive of fluorine. Some or all of the hydrogen atoms in the arylene group may be substituted by a substituent selected from C₁-C₂₀ saturated hydrocarbyl groups, C₁-C₂₀ saturated hydrocarbyloxy groups, C₆-C₂₀ aryl groups, halogen, and hydroxy.

The C₁-C₂₀ hydrocarbylene group R¹⁰² may be saturated or unsaturated and straight, branched or cyclic. Examples thereof are as exemplified above as a C₁-C₂₀ hydrocarbylene group L^{B}.

Examples of the C₆-C₂₀ arylene group represented by R¹⁰² include phenylene, naphthylene, phenanthrenediyl, and anthracenediyl. The C₁-C₂₀ saturated hydrocarbyl moiety and hydrocarbyl moiety in the C₁-C₂₀ hydrocarbyloxy moiety, which are substituents on the arylene group, may be straight, branched or cyclic and examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl, and icosyl; and C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl, and adamantyl. Examples of the C₆-C₁₄ arylene moiety which is a substituent on the arylene group include phenylene, naphthylene, phenanthrenediyl and anthracenediyl.

The anion of formula (D1) is preferably an anion having the formula (D2).

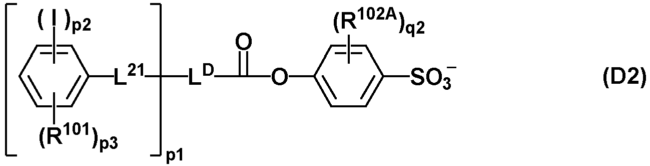

In formula (D2), p1, p2, p3, L²¹, L^{D}, and R¹⁰¹ are as defined above. The subscript q2 is 1, 2, 3 or 4. R^{102A} is a C₁-C₂₀ saturated hydrocarbyl group, C₁-C₂₀ saturated hydrocarbyloxy group, C₆-C₁₄ aryl group, halogen or hydroxy group. A plurality of R^{102A} may be identical or different when q2 is 2, 3 or 4.

Examples of the anion having formula (D1) are shown below, but not limited thereto.

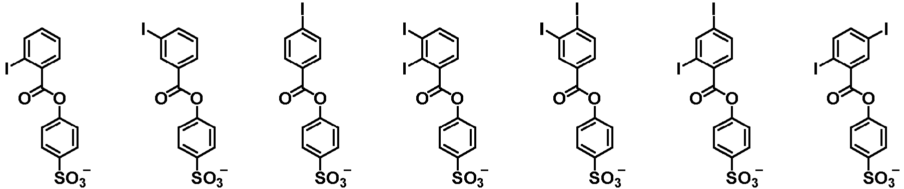

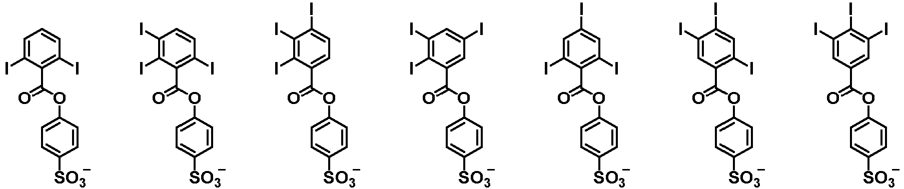

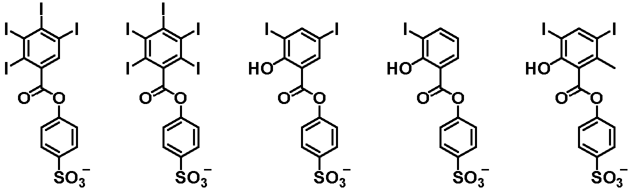

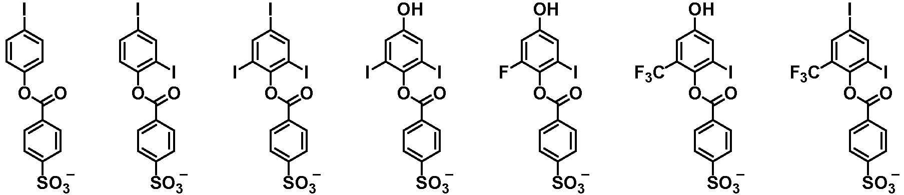

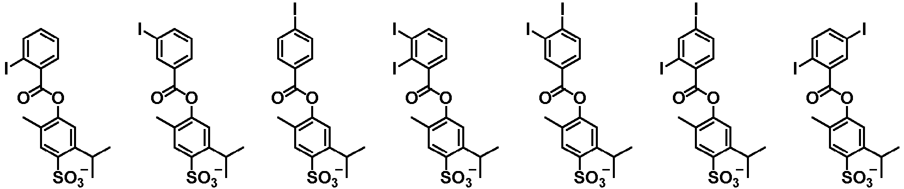

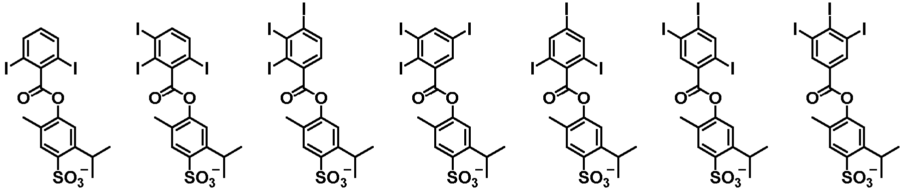

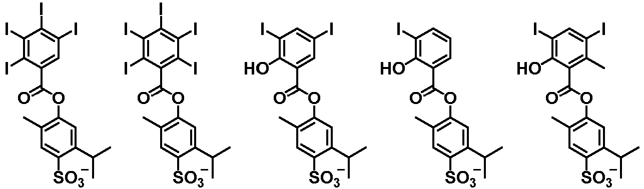

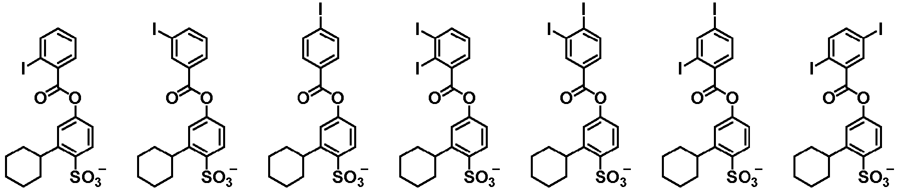

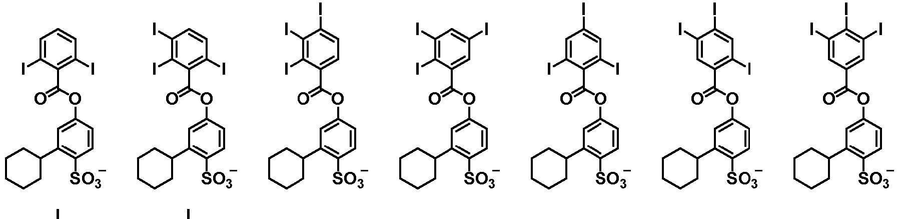

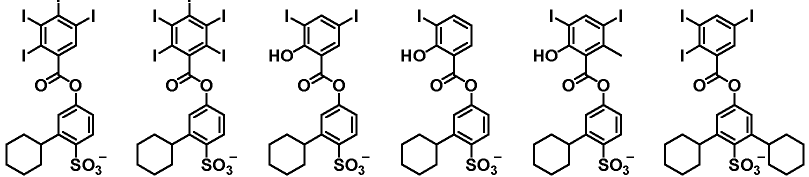

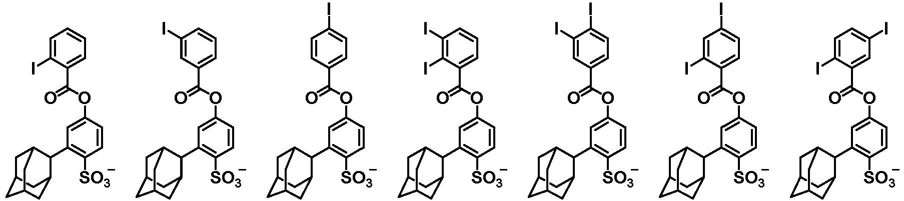

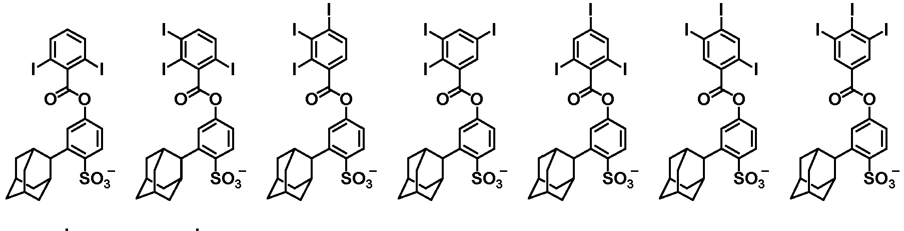

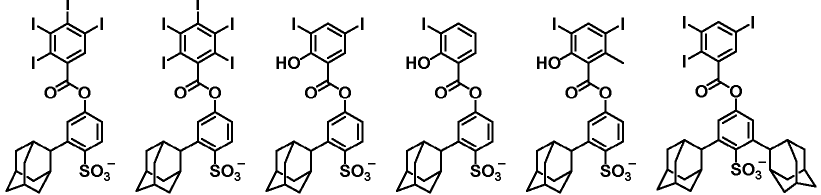

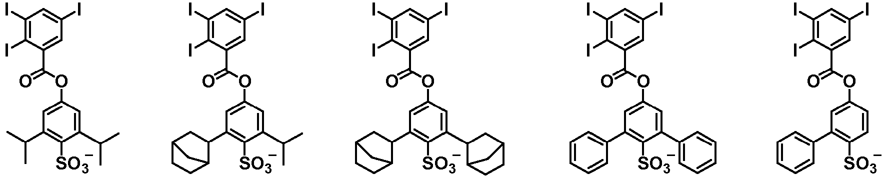

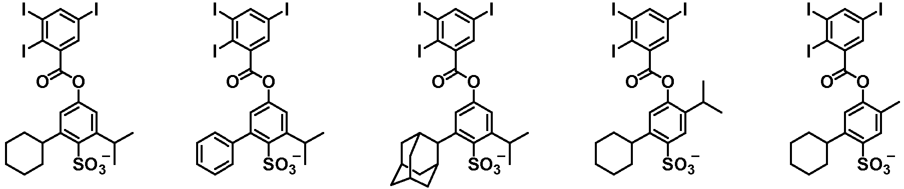

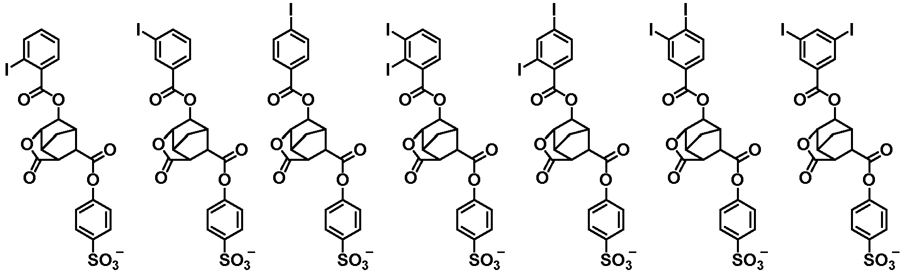

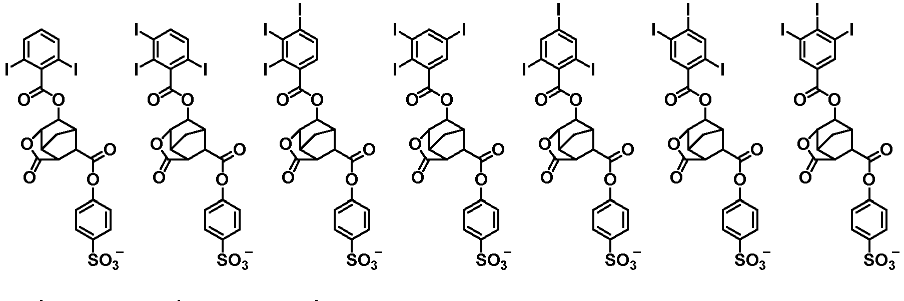

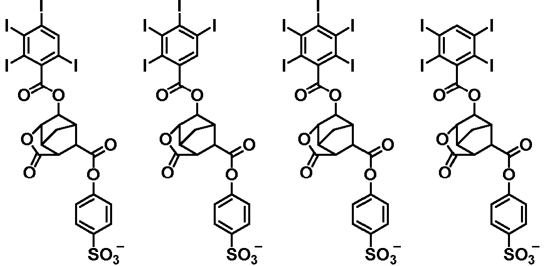

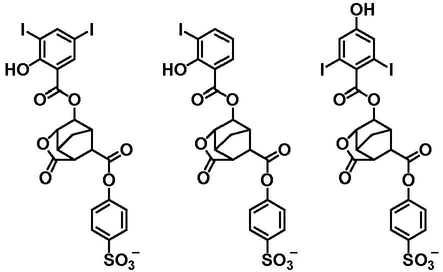

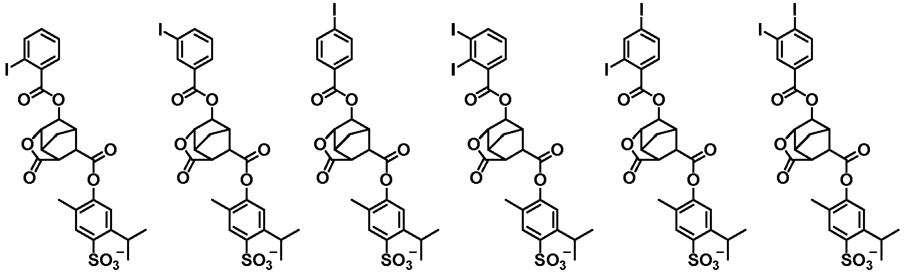

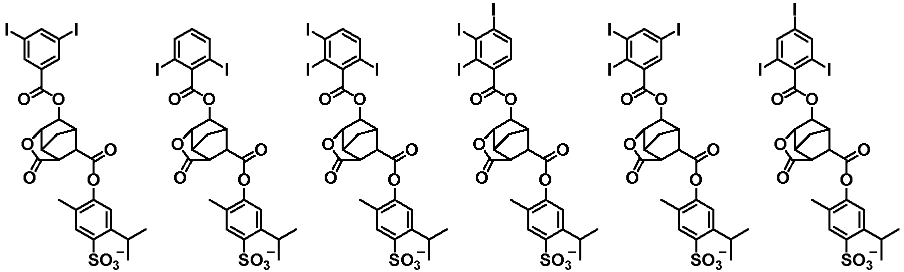

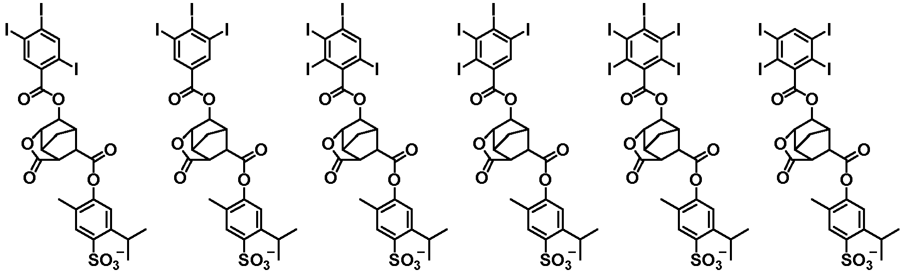

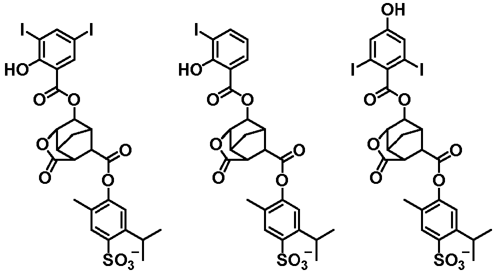

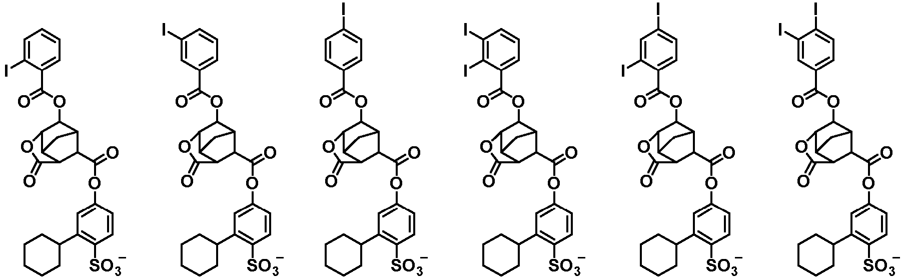

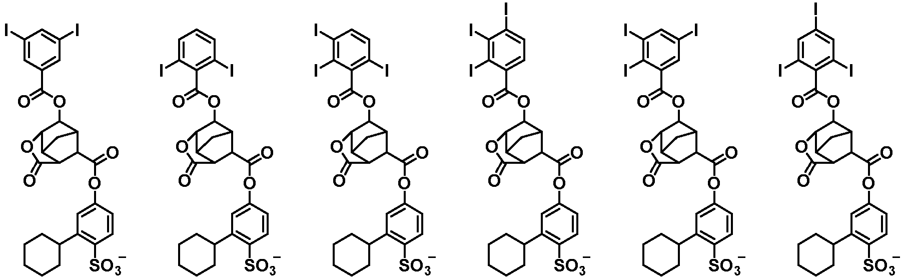

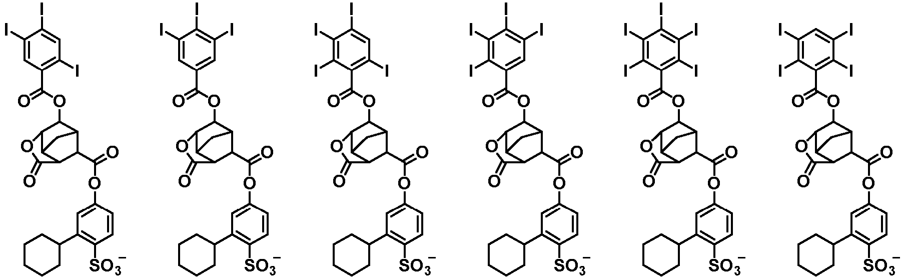

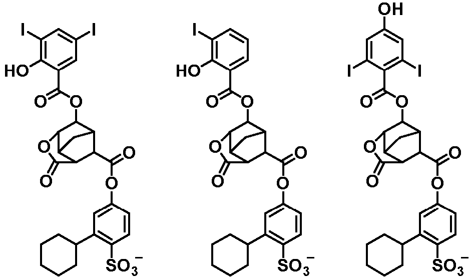

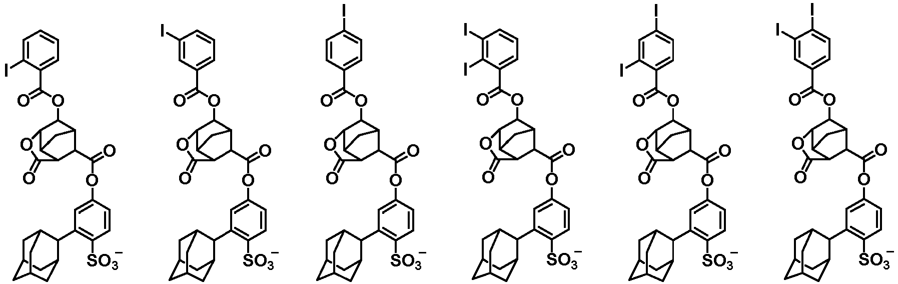

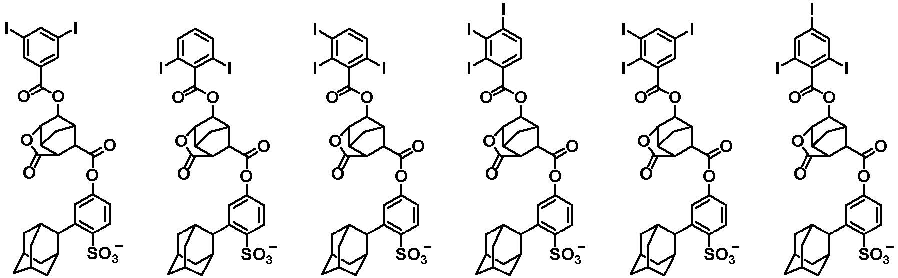

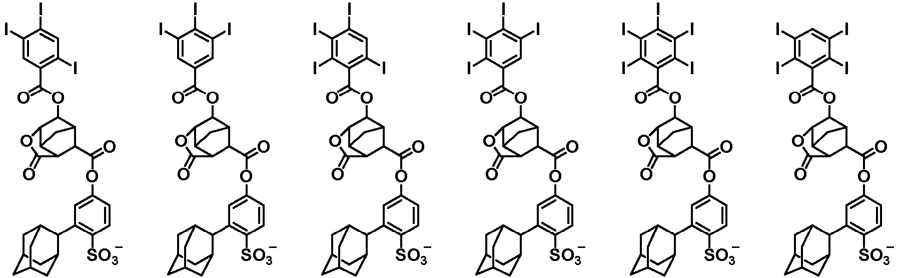

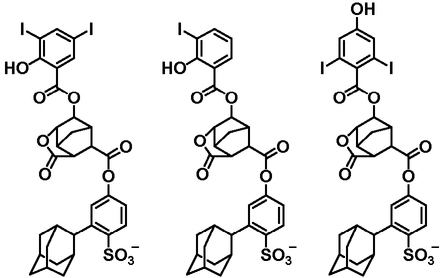

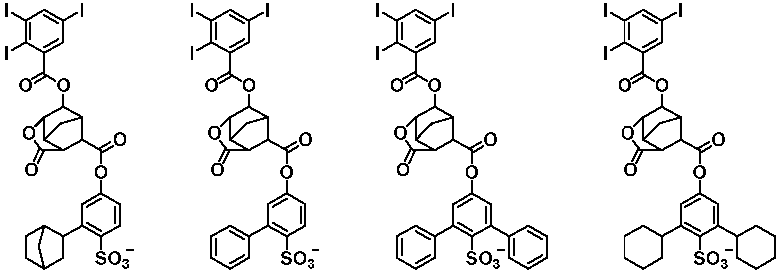

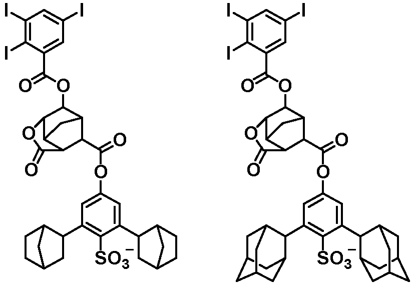

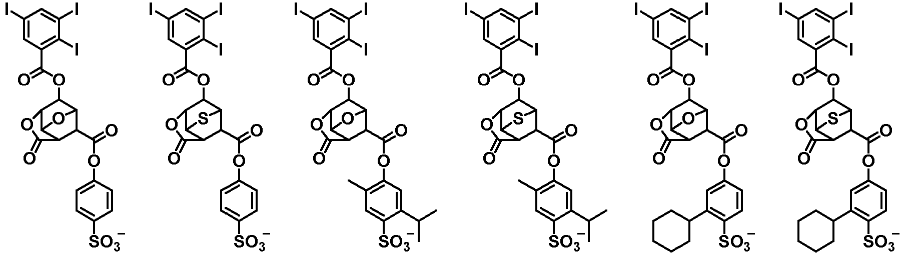

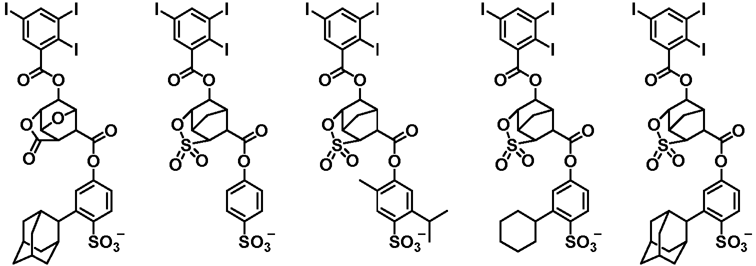

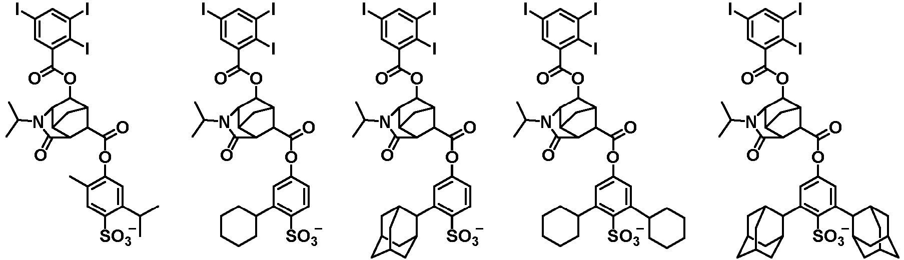

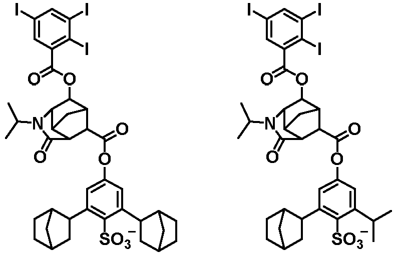

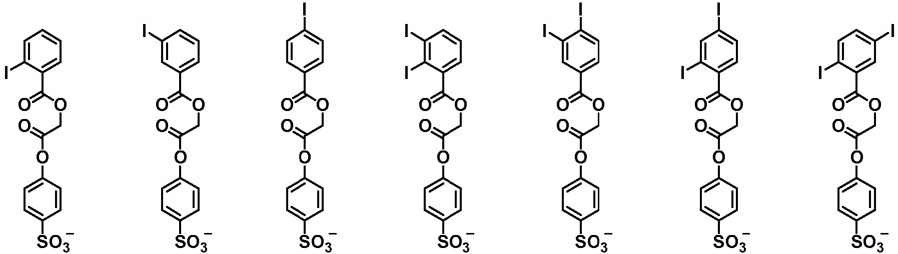

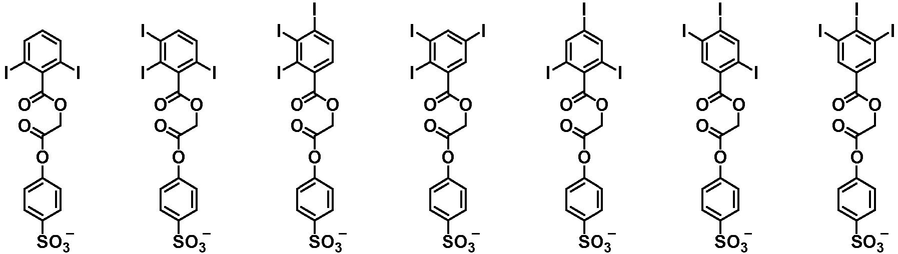

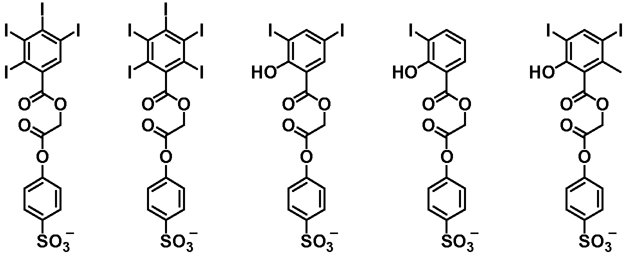

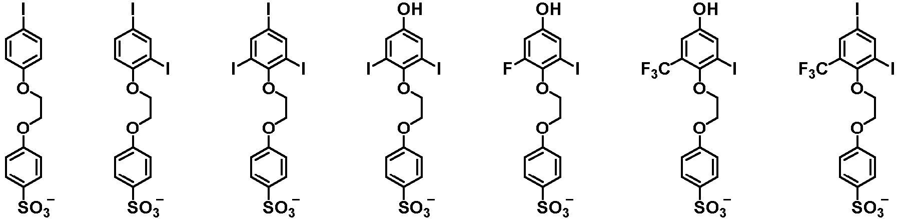

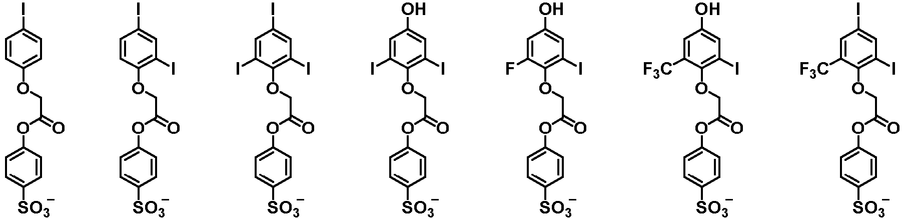

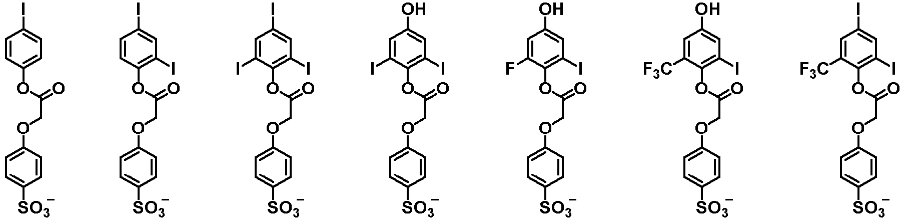

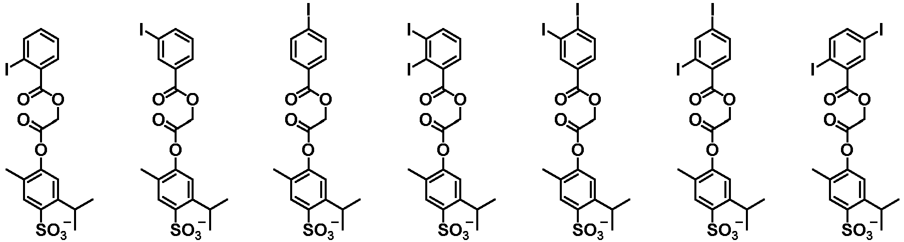

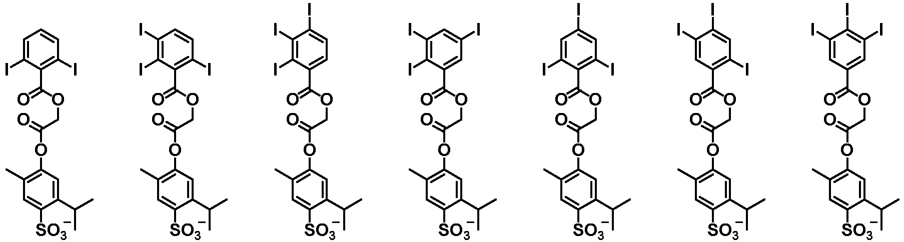

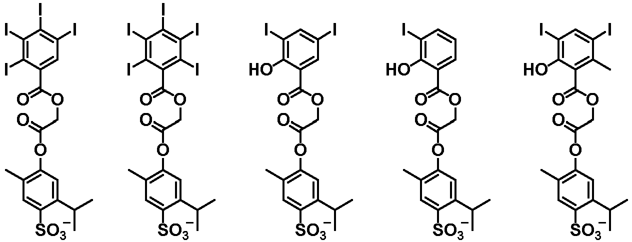

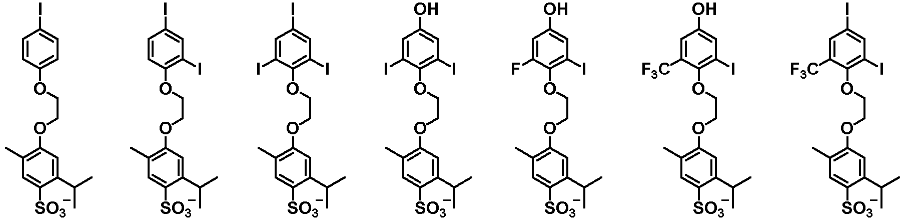

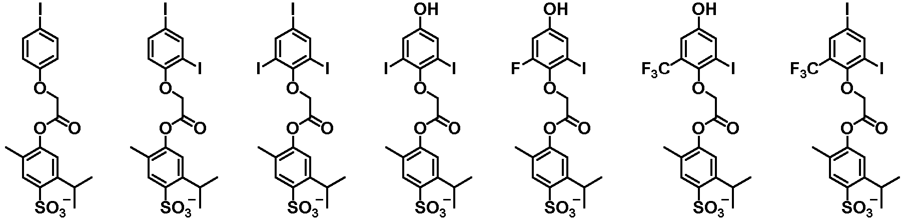

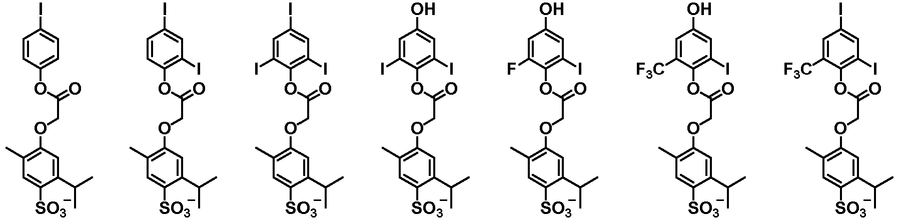

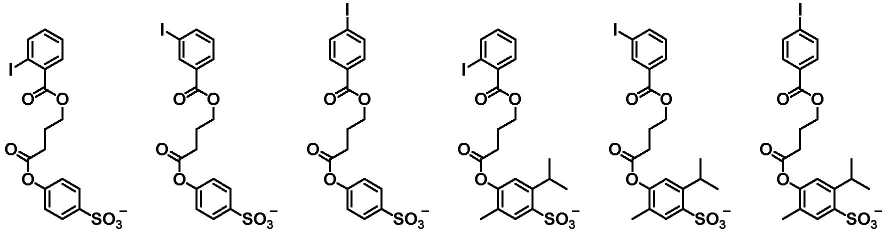

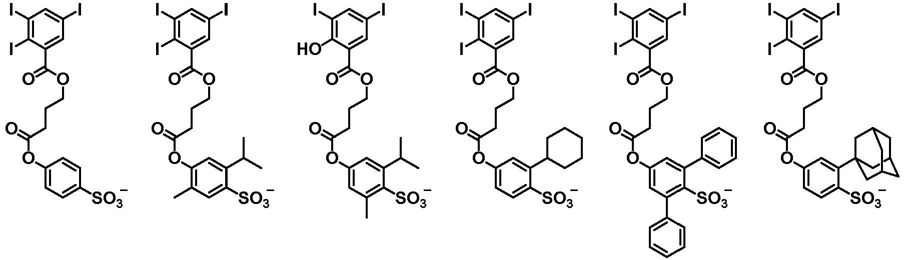

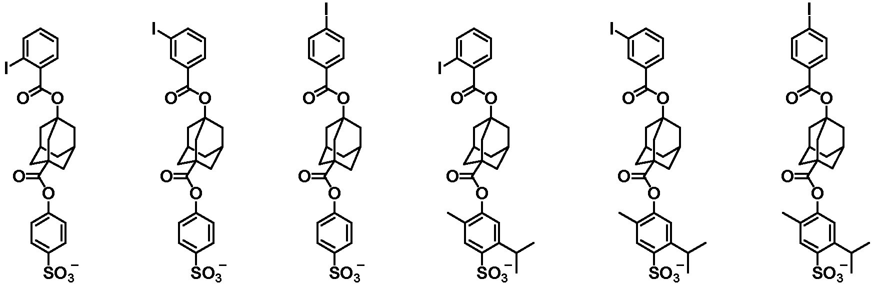

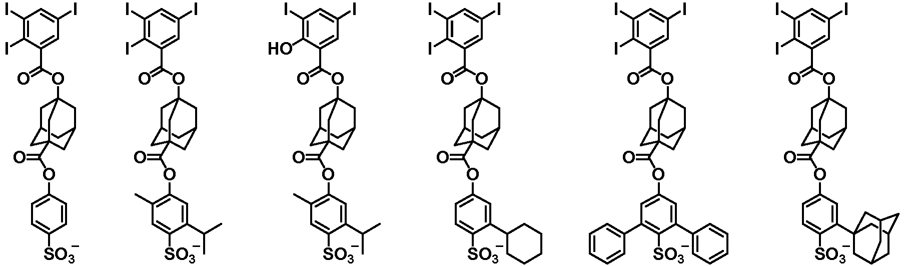

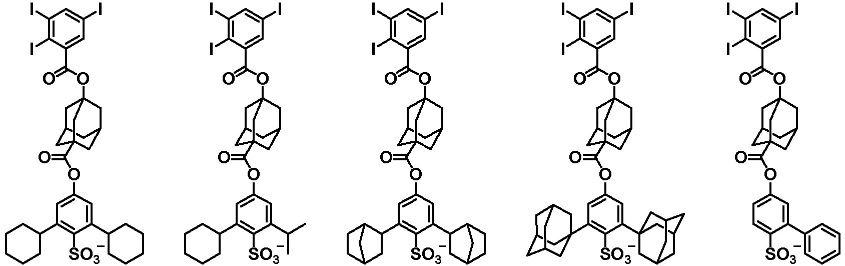

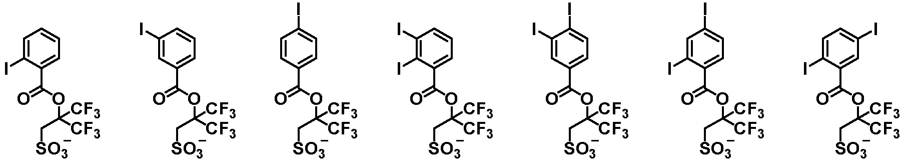

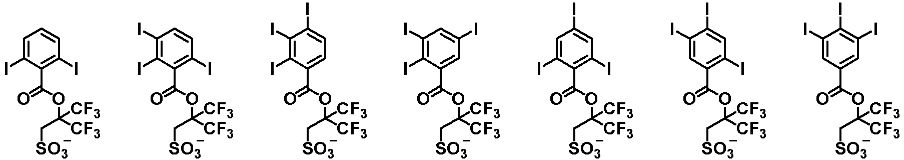

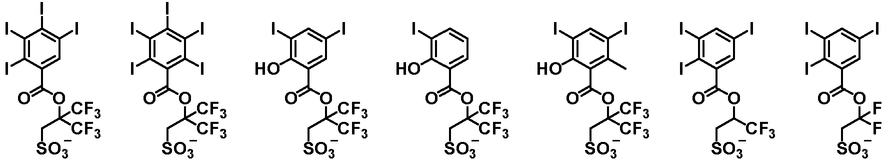

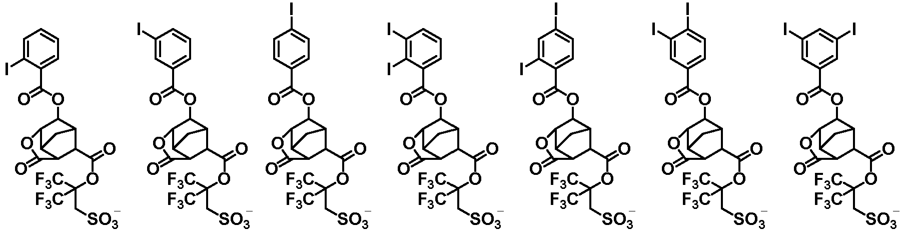

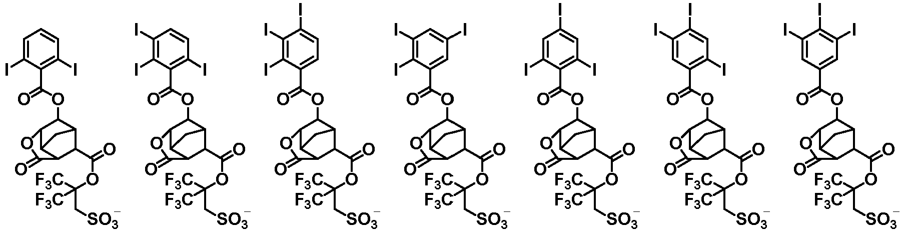

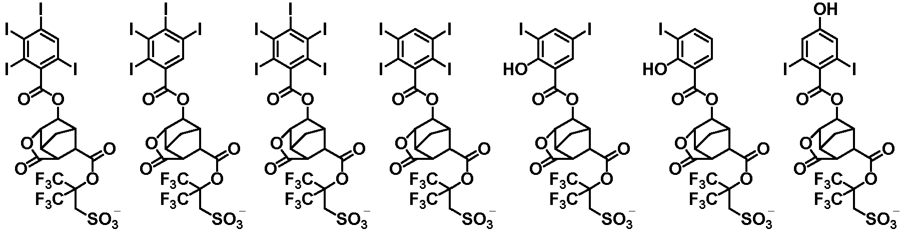

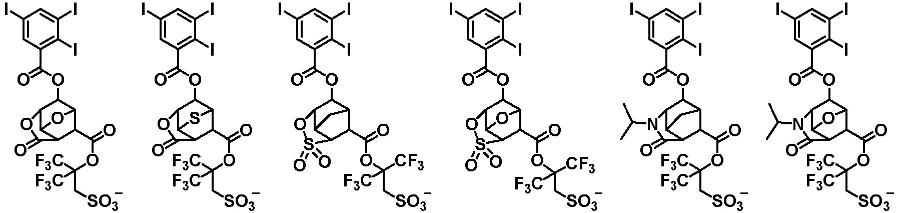

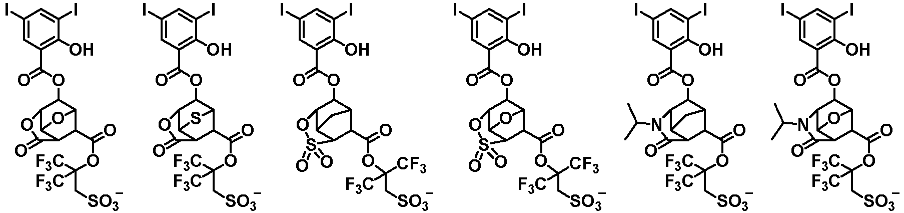

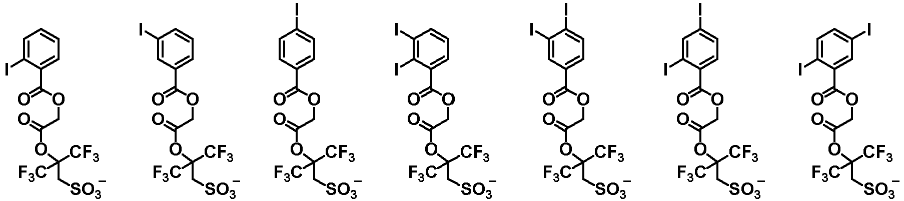

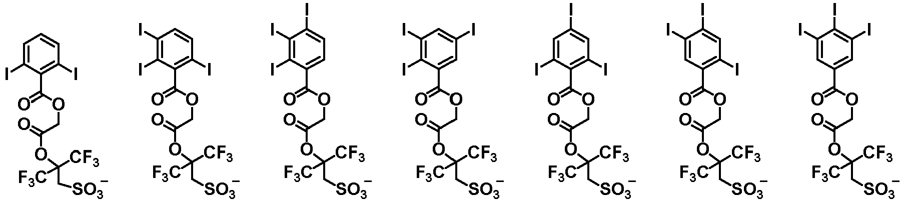

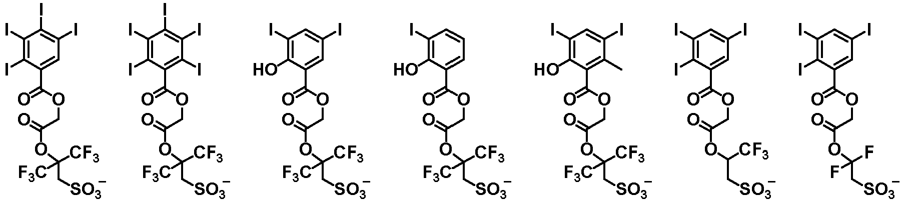

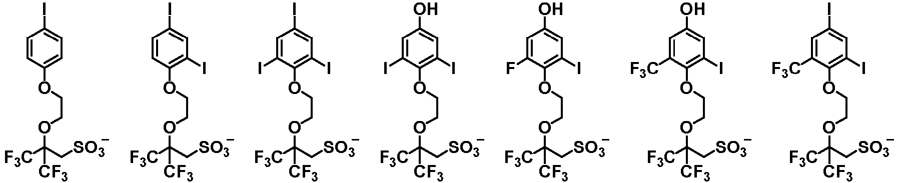

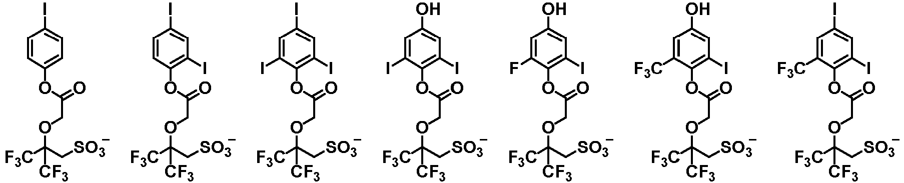

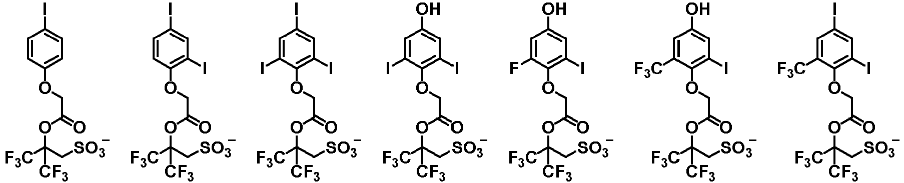

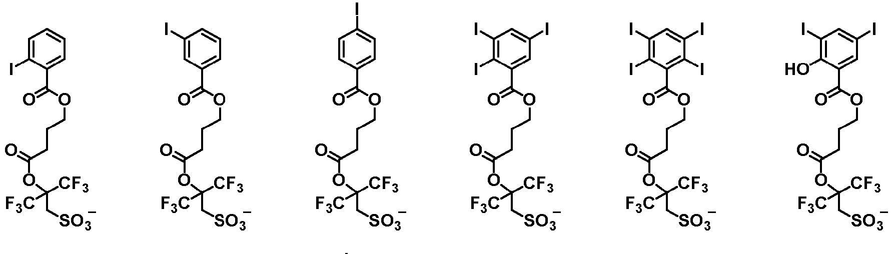

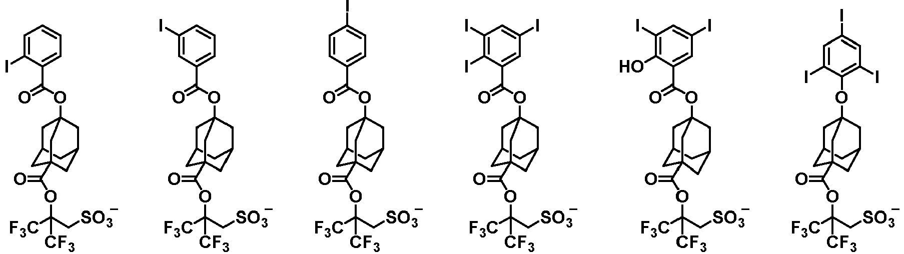

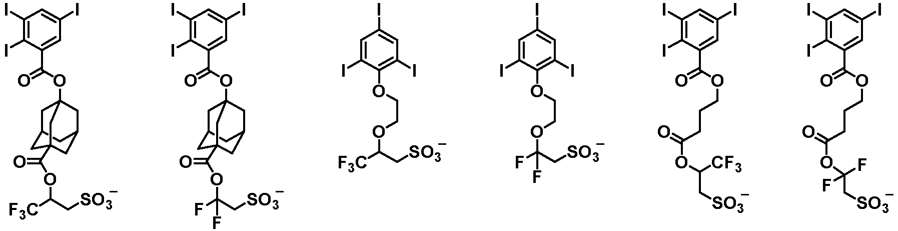

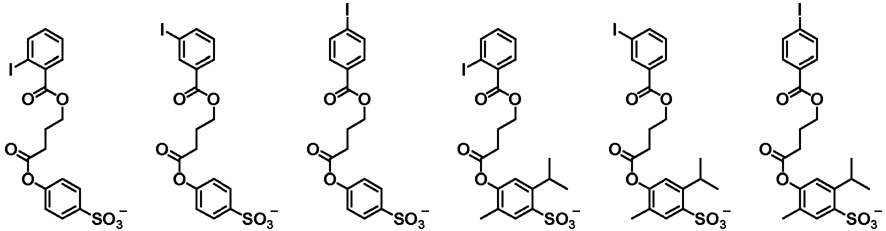

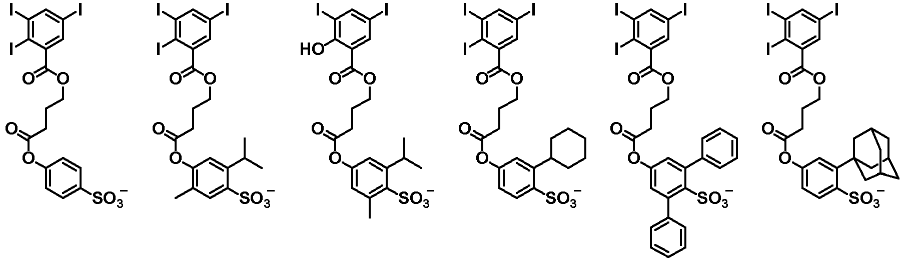

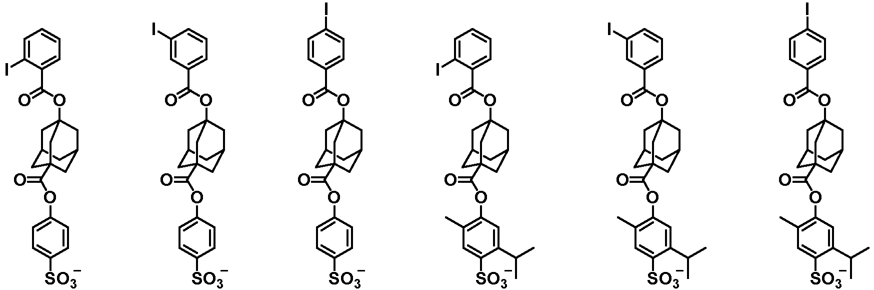

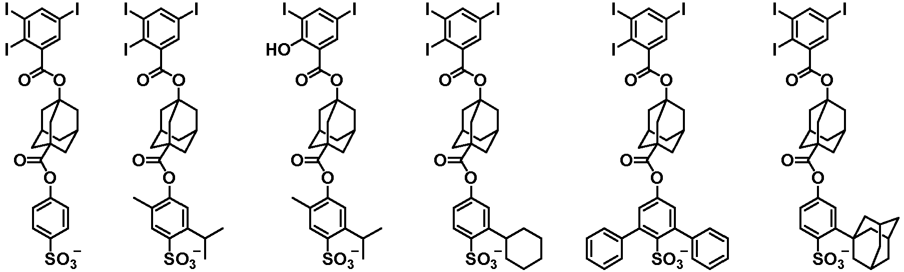

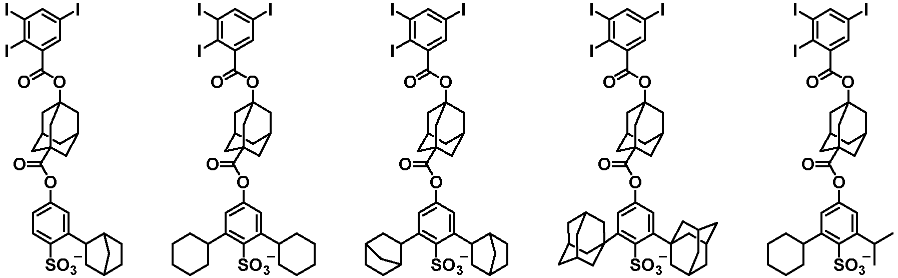

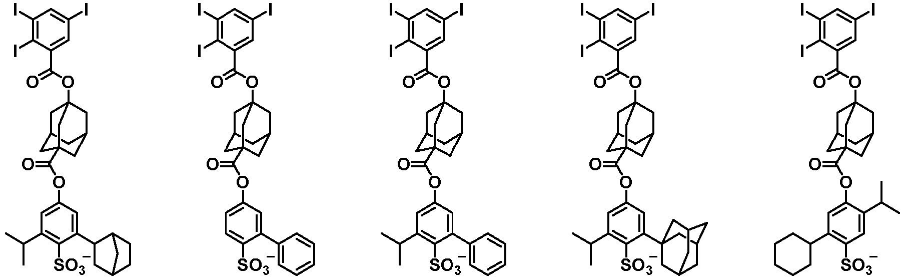

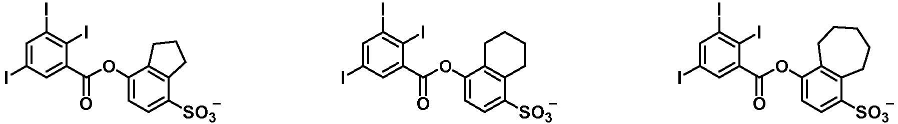

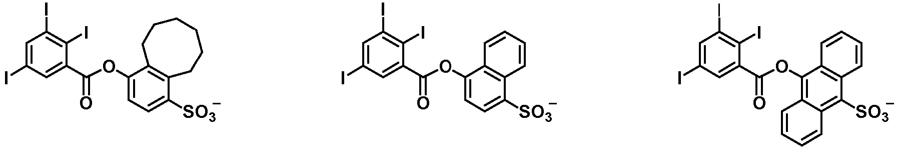

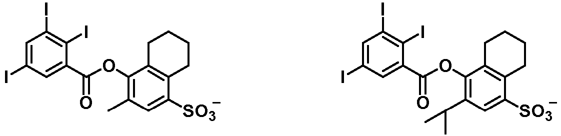

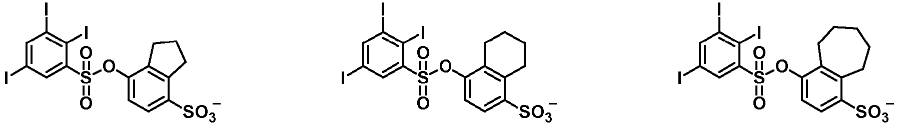

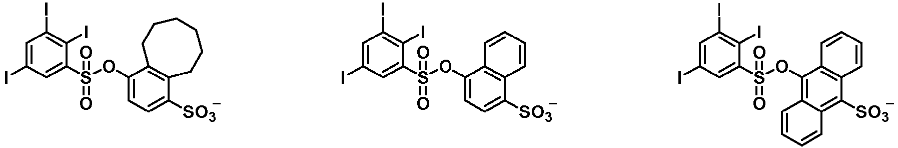

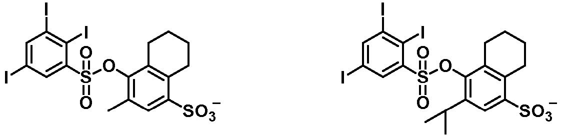

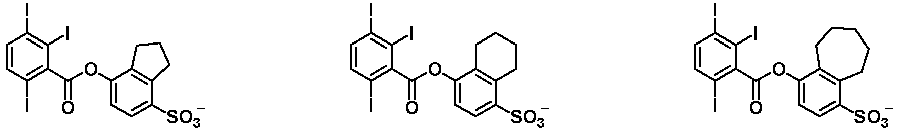

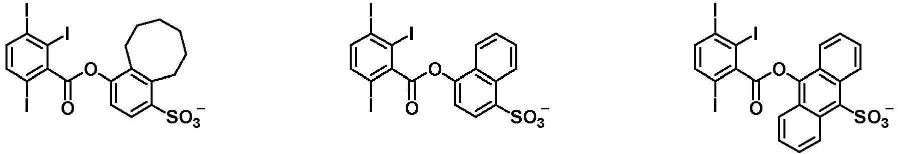

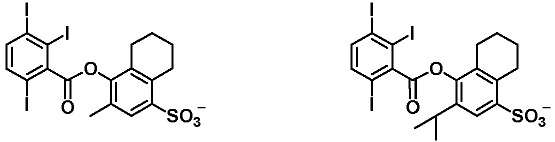

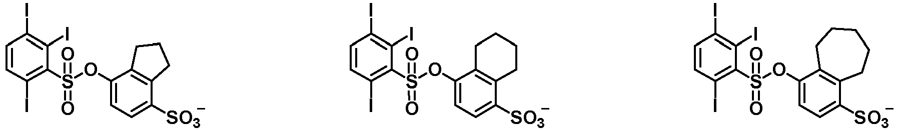

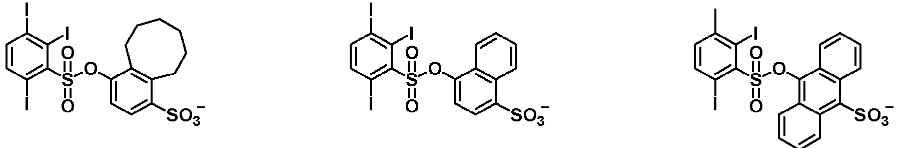

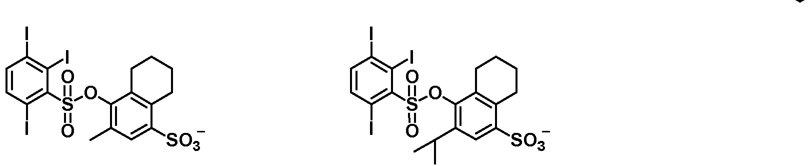

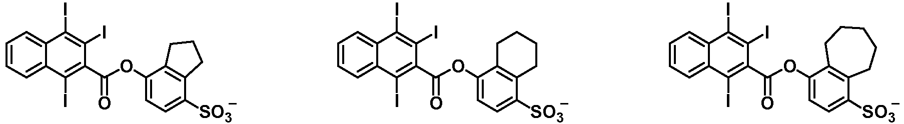

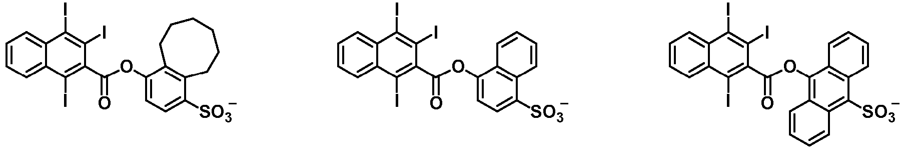

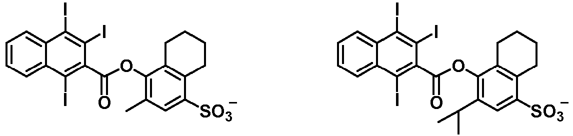

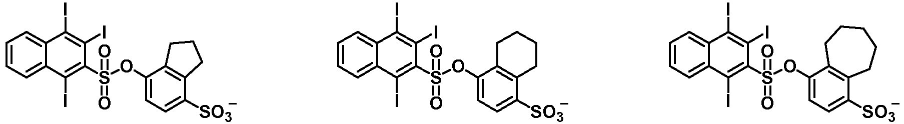

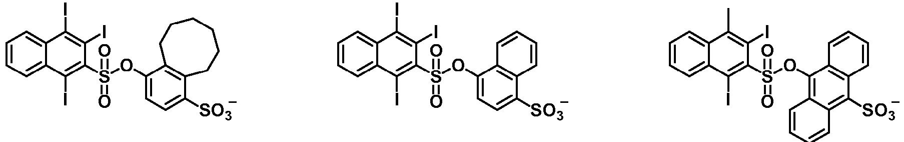

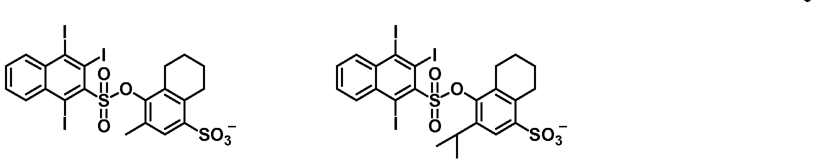

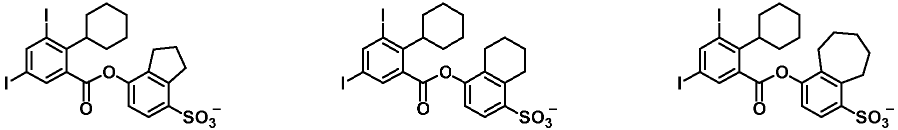

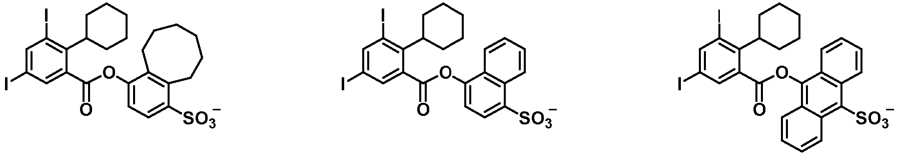

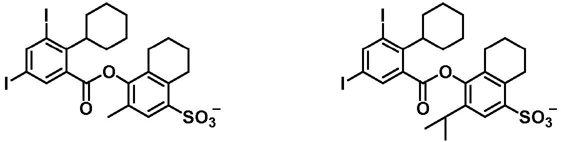

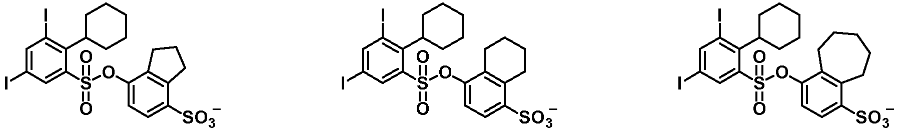

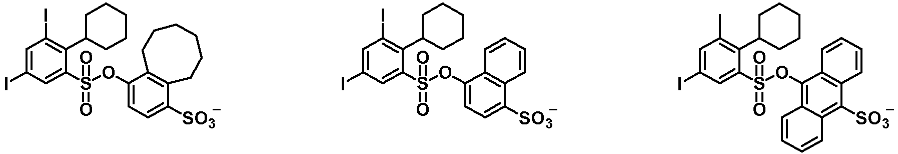

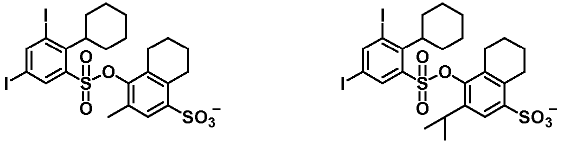

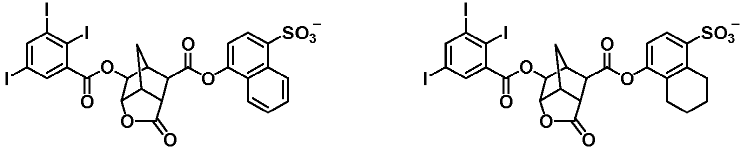

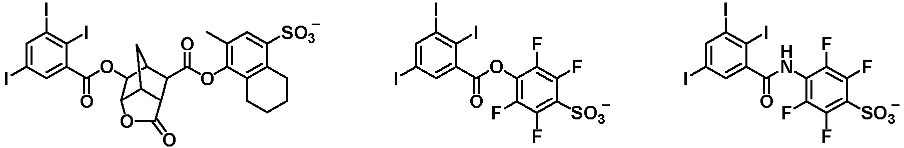

Preferred examples of the cation that pairs with the anion in the photoacid generator (D) include sulfonium cations and iodonium cations. Examples of the sulfonium cation are as exemplified for the sulfonium cations of formulae (Z-1) and (Z-3), but not limited thereto. Examples of the iodonium cation are as exemplified for the iodonium cation of formula (Z-2), but not limited thereto.

The PAG generates an acid having a pKa value of preferably -2.0 or larger, more preferably -1.0 or larger. The upper limit of pKa is preferably 2.0. Notably, the pKa value is computed using pKa DB in software ACD/Chemsketch ver: 9.04 of Advanced Chemistry Development Inc.

When the inventive chemically amplified positive resist composition comprises photoacid generator (D), the content of the photoacid generator (D) is preferably 1 to 10 parts by weight, more preferably 1 to 5 parts by weight per 80 parts by weight of the base polymer. The incorporation of the photoacid generator (D) can appropriately adjust the amount of an acid generated in the exposed regions and the ability to inhibit dissolution of the unexposed regions. The PAG (D) may be used alone or in admixture.

When the inventive chemically amplified positive resist composition contains both the photoacid generator (D) and the quencher (A), the weight ratio of the photoacid generator to the quencher (photoacid generator/quencher) is preferably less than 3/1, more preferably less than 2.5/1, even more preferably less than 2/1. As long as the weight ratio of the PAG to the quencher in the chemically amplified positive resist composition is in the range, the resist composition is able to fully suppress acid diffusion, leading to improved resolution and dimensional uniformity.

### (E) Fluorinated polymer

The chemically amplified positive resist composition may further comprise a fluorinated polymer for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. (E) The fluorinated polymer contains repeat units of at least one type selected from repeat units having the formula (E1), repeat units having the formula (E2), repeat units having the formula (E3), and repeat units having the formula (E4), as components. It is noted that repeat units having formulae (E1), (E2), (E3), and (E4) are also referred to as repeat units E1, E2, E3, and E4, respectively, hereinafter. Since the fluorinated polymer also has a surface active function, it can prevent insoluble residues from re-depositing onto the substrate during the development step and is thus effective for preventing development defects.

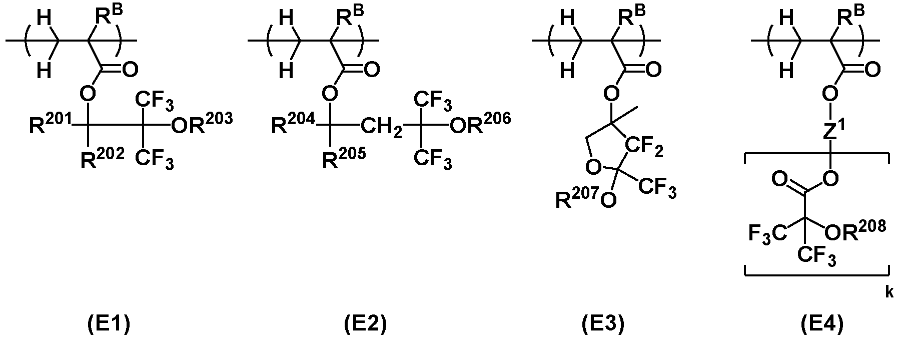

In formulae (E1) to (E4), k is 1, 2 or 3. R^{B} is each independently a hydrogen atom, fluorine atom, methyl, or trifluoromethyl. R²⁰¹, R²⁰², R²⁰⁴ and R²⁰⁵ are each independently hydrogen or a C₁-C₁₀ saturated hydrocarbyl group. R²⁰³, R²⁰⁶, R²⁰⁷ and R²⁰⁸ are each independently a hydrogen atom, C₁-C₁₅ hydrocarbyl group, C₁-C₁₅ fluorinated hydrocarbyl group or acid labile group, and an ether bond or carbonyl group may intervene in a carbon-carbon bond when each of R²⁰¹, R²⁰⁶, R²⁰⁷ and R²⁰⁸ is a hydrocarbyl group or fluorinated hydrocarbyl group. Z¹ is a C₁-C₂₀ (k+1)-valent hydrocarbon group or C₁-C₂₀ (k+1)-valent fluorinated hydrocarbon group.

The C₁-C₁₀ saturated hydrocarbyl groups represented by R²⁰¹, R²⁰², R²⁰⁴ and R²⁰⁵ may be straight, branched or cyclic. Examples thereof include C₁-C₁₀ alkyl groups such as methyl, ethyl, n-propyl, isopropyl, n-butyl, isobutyl, sec-butyl, tert-butyl, n-pentyl, n-hexyl, n-heptyl, n-octyl, n-nonyl and n-decyl, and C₃-C₁₀ cyclic saturated hydrocarbyl groups such as cyclopropyl, cyclobutyl, cyclopentyl, cyclohexyl, adamantyl and norbornyl. Of these, C₁-C₆ saturated hydrocarbyl groups are preferred.

The C₁-C₁₃ saturated hydrocarbyl groups represented by R²⁰³, R²⁰⁶, R²⁰⁷ and R²⁰⁸ may be straight, branched or cyclic. Examples thereof include C₁-C₁₅ alkyl, C₂-C₁₃ alkenyl and C₂-C₁₅ alkynyl groups, with the C₁-C₁₅ alkyl groups being preferred. Suitable alkyl groups include methyl, ethyl, n-propyl, isopropyl, n-butyl, isobutyl, sec-butyl, n-pentyl, n-hexyl, n-heptyl, n-octyl, n-nonyl, n-decyl, n-undecyl, n-dodecyl, n-tridecyl, n-tetradecyl and n-pentadecyl groups. Examples of the fluorinated hydrocarbyl group include the foregoing hydrocarbyl groups in which some or all carbon-bonded hydrogen atoms are substituted by fluorine.

Examples of the C₁-C₂₀ (k+1)-valent hydrocarbon group Z¹ include the foregoing C₁-C₂₀ alkyl groups and C₃-C₂₀ cyclic saturated hydrocarbyl groups, with k number of hydrogen atoms being eliminated. Examples of the C₁-C₂₀ (h+1)-valent fluorinated hydrocarbon group Z¹ include the foregoing (h+1)-valent hydrocarbon groups in which one or more hydrogen atoms are substituted by fluorine.

Examples of repeat units E1 to E4 are shown below, but not limited thereto. Herein R^{B} is as defined above.

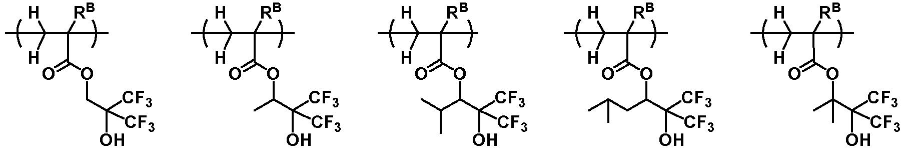

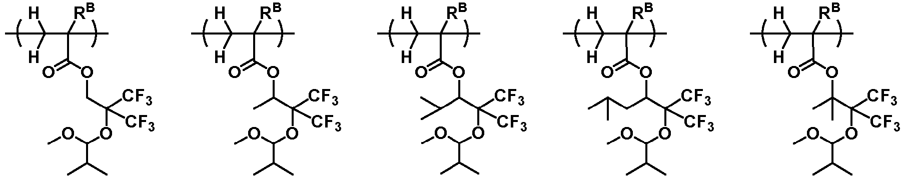

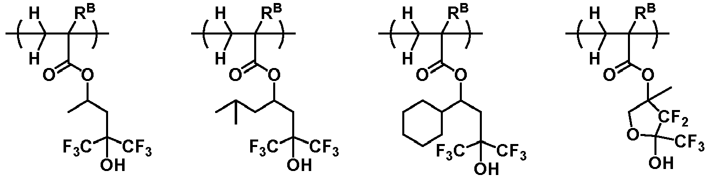

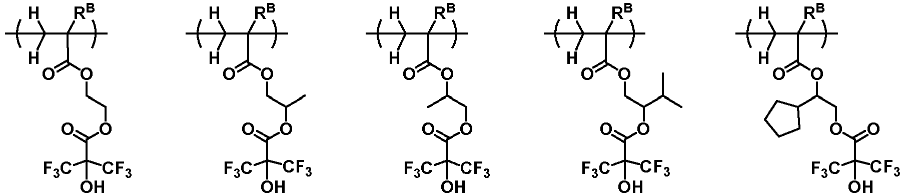

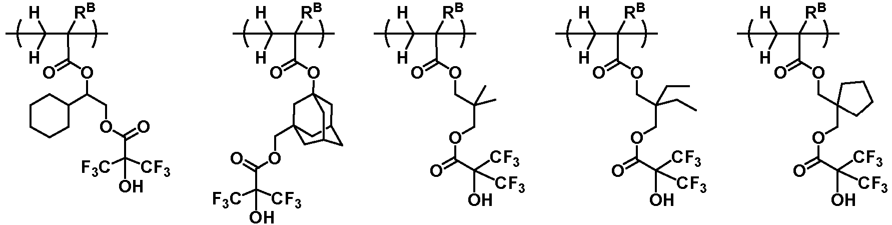

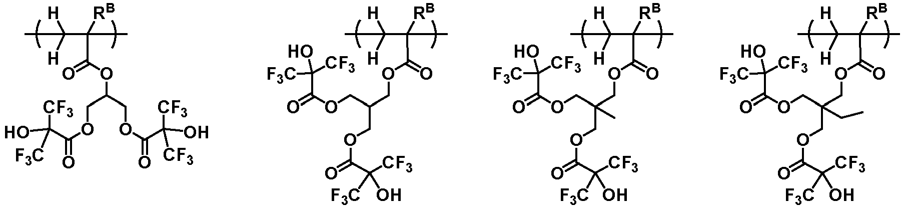

Preferably the fluorinated polymer further contains repeat units of at least one type selected from repeat units having the formula (E5) and repeat units having the formula (E6). It is noted that repeat units having formulae (E5) and (E6) are also referred to as repeat units E5 and E6, respectively, hereinafter.

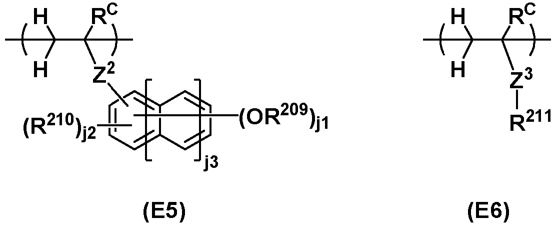

In formulae (E5) to (E6), j1 is 1, 2 or 3. The subscript j2 is an integer meeting 0 ≤ j2 ≤ 5+2(j3)-j1. The subscript j3 is 0 or 1. R^{C} is each independently hydrogen or methyl. R²⁰⁹ is hydrogen or a straight or branched C₁-C₅ hydrocarbyl group in which a group containing a heteroatom may intervene in a carbon-carbon bond. R²¹⁰ is a straight or branched C₁-C₅ hydrocarbyl group in which a group containing a heteroatom may intervene in a carbon-carbon bond. R²¹¹ is a C₁-C₂₀ saturated hydrocarbyl group in which at least one hydrogen is substituted by a fluorine atom, and some constituent -CH₂- of the saturated hydrocarbyl group may be replaced by an ester bond or ether bond. Z² is a single bond, *-C(=O)-O- or *-C(=O)-NH-. Z³ is a single bond, -O-, *-C(=O)-O-Z³¹-Z³²- or *-C(=O)-NH-Z³¹-Z³²-. Z³¹ is a single bond or C₁-C₁₀ saturated hydrocarbylene group. Z³² is a single bond, ester bond, ether bond or sulfonamide bond. * designates a point of attachment to the carbon atom in the backbone.

Examples of the C₁-C₅ hydrocarbyl groups R²⁰⁹ and R²¹⁰ include alkyl, alkenyl and alkynyl groups, with the alkyl groups being preferred. Examples of the alkyl group include methyl, ethyl, n-propyl, isopropyl, n-butyl, isobutyl, sec-butyl and n-pentyl groups. In the hydrocarbyl groups, a moiety containing a heteroatom such as oxygen, sulfur or nitrogen may intervene in a carbon-carbon bond.

Preferably, -OR²⁰⁹ in formula (E5) is a hydrophilic group. In this case, R²⁰⁹ is preferably hydrogen or a C₁-C₅ alkyl group in which oxygen intervenes in a carbon-carbon bond.

The C₁-C₂₀ saturated hydrocarbyl group R²¹¹ in which at least one hydrogen is substituted by fluorine may be straight, branched or cyclic, and examples thereof include C₁-C₂₀ alkyl groups or C₃-C₂₀ cyclic saturated hydrocarbyl groups in which at least one hydrogen is substituted by a fluorine atom.

Z² is preferably *-C(=O)-O- or *-C(=O)-NH-. Also preferably R^{C} is methyl. The inclusion of carbonyl in Z² enhances the ability to trap the acid originating from the anti-charging film. A polymer wherein R^{C} is methyl is a robust polymer having a high glass transition temperature (Tg) which is effective for suppressing acid diffusion. As a result, the resist film is improved in stability with time, and neither resolution nor pattern profile is degraded.

The C₁-C₁₀ saturated hydrocarbylene group Z³ may be straight, branched or cyclic, and examples thereof include methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, and 1,1-dimethylethane-1,2-diyl groups.

Examples of the repeat unit E5 are shown below, but not limited thereto. Herein, R^{C} is as defined above.

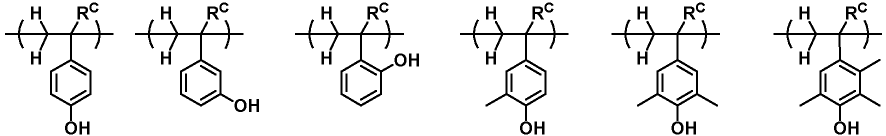

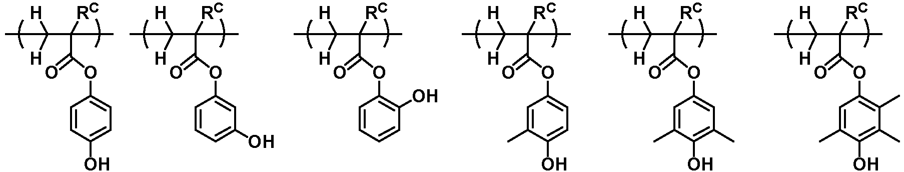

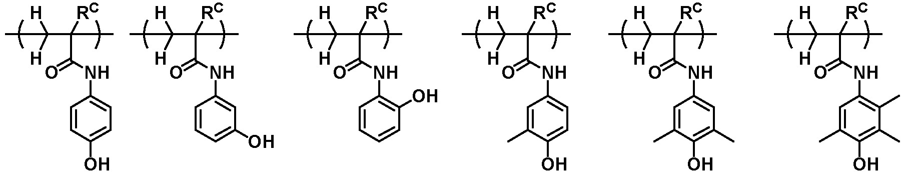

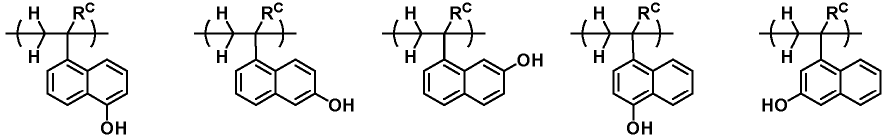

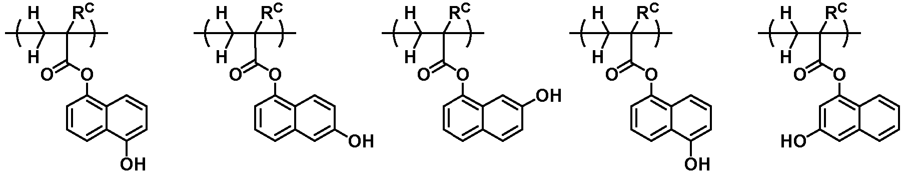

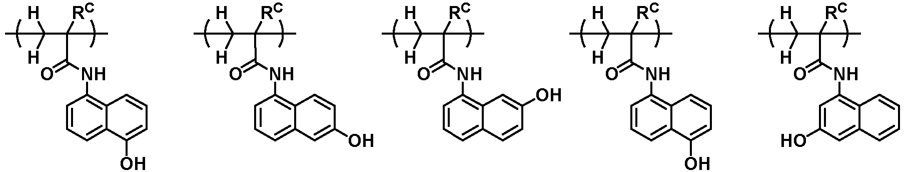

Examples of the repeat unit E6 are shown below, but not limited thereto. Herein, R^{C} is as defined above.

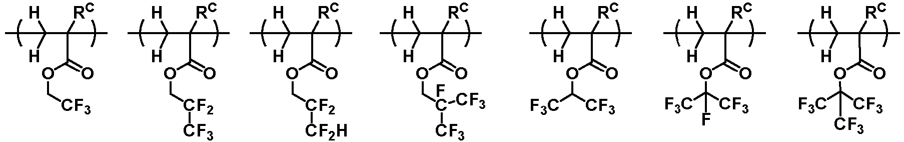

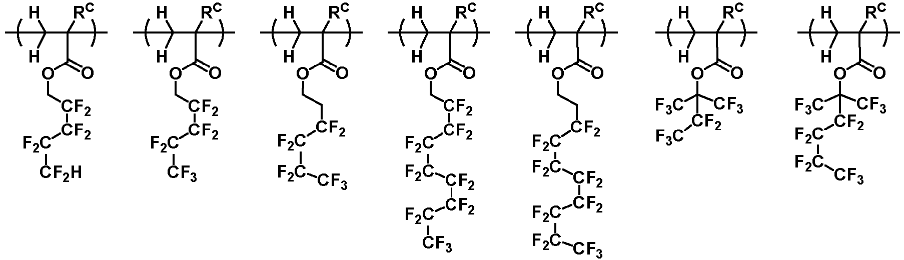

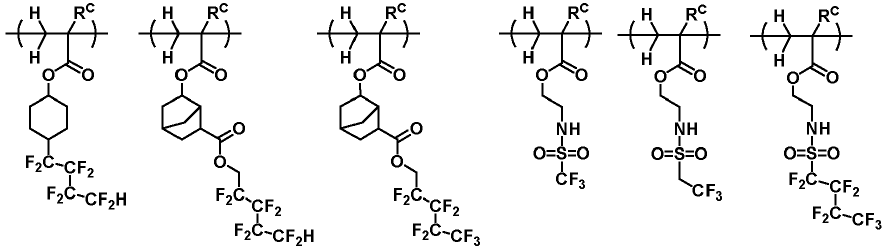

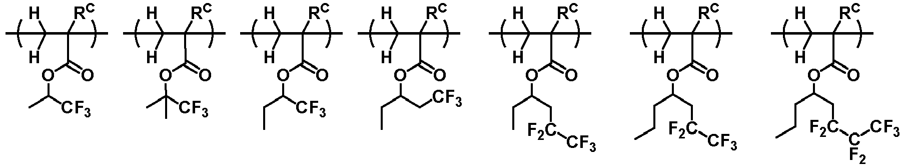

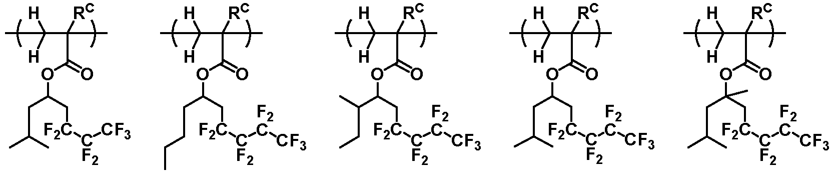

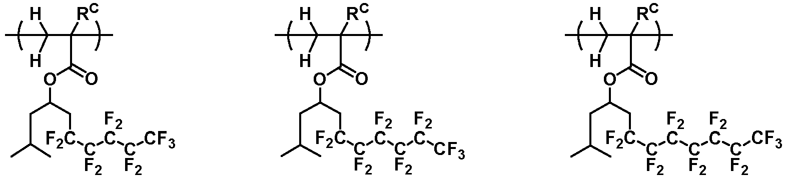

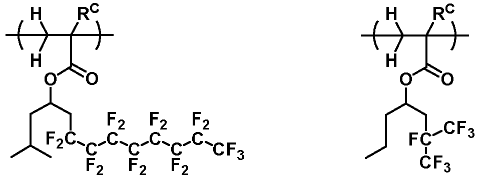

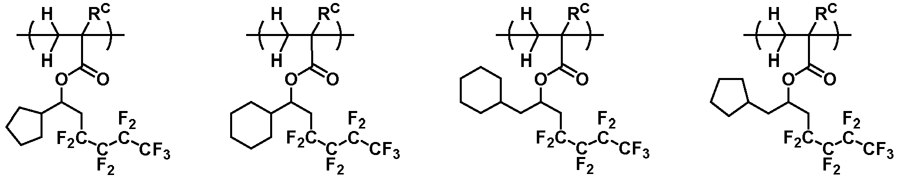

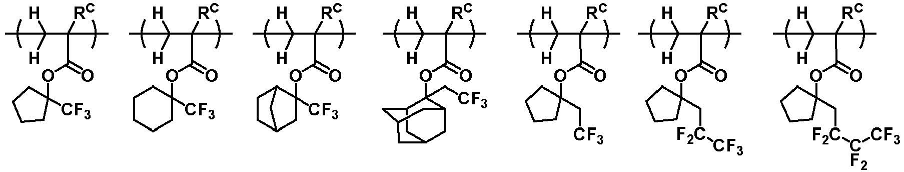

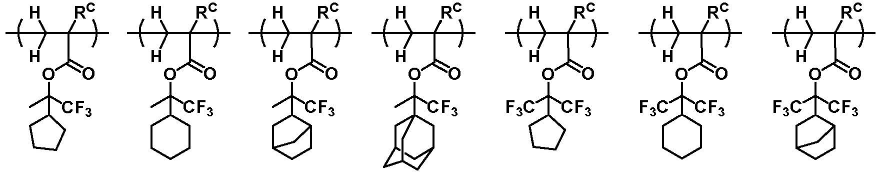

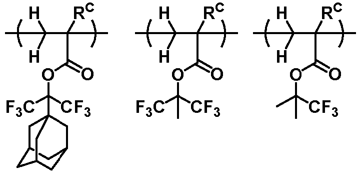

The content of repeat units E1 to E4 is preferably 15 to 95 mol%, more preferably 20 to 85 mol%, of the overall repeat units of the fluorinated polymer. The content of repeat units E5 and/or E6 is preferably 5 to 85 mol%, more preferably 15 to 80 mol%, of the overall repeat units of the fluorinated polymer. Each of the repeat units E1 to E6 may be of one type or a combination of plural types.

The fluorinated polymer (E) may comprise additional repeat units as well as the repeat units D1 to D6. Suitable additional repeat units include those described in USP 9,091,918 (JP-A 2014-177407, paragraphs [0046]-[0078]). When the fluorinated polymer (E) comprises additional repeat units, their content is preferably up to 50 mol% based on the overall repeat units of the fluorinated polymer.

The fluorinated polymer (E) may be synthesized by combining suitable monomers optionally protected with a protective group, copolymerizing them in the standard way, and effecting deprotection reaction if necessary. The copolymerization reaction is not limited, and is preferably radical polymerization, or anionic polymerization. For these methods, reference may be made to JP-A 2004-115630.

The fluorinated polymer (E) should preferably have a Mw of 2,000 to 50,000, and more preferably 3,000 to 20,000. A fluorinated polymer with a Mw of at least 2,000 prevents extra acid diffusion and degradations of resolution and age stability. A polymer with a Mw of up to 50,000 has a sufficient solubility in solvent and leaves no coating defects. The fluorinated polymer (E) preferably has a dispersity (Mw/Mn) of 1.0 to 2.2, more preferably 1.0 to 1.7.

When the inventive chemically amplified positive resist composition comprises fluorinated polymer (E), the content of the fluorinated polymer (E) is preferably 0.01 to 30 parts by weight, more preferably 0.1 to 20 parts by weight per 80 parts by weight of the base polymer (B). The fluorinated polymer (E) may be used alone or in admixture.

### (F) Other quencher

The chemically amplified positive resist composition of the invention may further comprise (F) a quencher other than component (A) (hereinafter, also referred to as the other quencher) as necessary.

The other quencher is typically selected from conventional basic compounds. Examples of the conventional basic compound include primary, secondary, and tertiary aliphatic amines, mixed amines, aromatic amines, heterocyclic amines, nitrogen-containing compounds having a carboxy group, nitrogen-containing compounds having a sulfonyl group, nitrogen-containing compounds having a hydroxy group, nitrogen-containing compounds having a hydroxyphenyl group, alcoholic nitrogen-containing compounds, amide derivatives, imide derivatives, and carbamate derivatives. In particular, primary, secondary, and tertiary amine compounds described in paragraphs [0146] to [0164] of JP-A 2008-111103, particularly amine compounds having a hydroxy group, an ether bond, an ester bond, a lactone ring, a cyano group, or a sulfonic acid ester bond, and compounds having a carbamate group described in JP 3790649 are preferred. Inter alia, tris[2-(methoxymethoxy)ethyl]amine, tris[2-(methoxymethoxy)ethyl]amine-N-oxide, dibutylaminobenzoic acid, morpholine derivatives, and imidazole derivatives are preferred. Addition of a basic compound may be effective for further suppressing the diffusion rate of acid in the resist film or correcting the pattern profile.

Onium salts such as sulfonium, iodonium and ammonium salts of carboxylic acids which are not fluorinated at α-position as described in USP 8,795,942 (JP-A 2008-158339) may also be used as the other quencher. While an α-fluorinated sulfonic acid, imide acid, and methide acid are necessary to deprotect the acid labile group, an α-non-fluorinated carboxylic acid is released by salt exchange with an α-non-fluorinated onium salt. An α-non-fluorinated carboxylic acid functions as a quencher because it does not induce substantial deprotection reaction.

Examples of the onium salt of α-non-fluorinated carboxylic acid include compounds having the formula (F1).

**R³⁰¹-CO₂⁻ Mq_{A}⁺** **(F1)**

In formula (F1), R³⁰¹ is hydrogen or a C₁-C₄₀ hydrocarbyl group which may contain a heteroatom, exclusive of the hydrocarbyl group in which the hydrogen bonded to the carbon atom at α-position of the carboxy group is substituted by fluorine or fluoroalkyl moiety.

The hydrocarbyl group represented by R³⁰¹ may be saturated or unsaturated, and may be straight, branched, or cyclic. Examples thereof include C₁-C₄₀ alkyl groups such as methyl, ethyl, n-propyl, isopropyl, n-butyl, sec-butyl, tert-butyl, n-pentyl, tert-pentyl, n-hexyl, n-octyl, 2-ethylhexyl, n-nonyl, n-decyl; C₃-C₄₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; C₂-C₄₀ alkenyl groups such as vinyl, allyl, propenyl, butenyl and hexenyl; C₃-C₄₀ cyclic unsaturated aliphatic hydrocarbyl groups such as cyclohexenyl; C₆-C₄₀ aryl groups such as phenyl, naphthyl, alkylphenyl groups (e.g., 2-methylphenyl, 3-methylphenyl, 4-methylphenyl, 4-ethylphenyl, 4-tert-butylphenyl, 4-n-butylphenyl), di- or tri-alkylphenyl groups (e.g., 2,4-dimethylphenyl and 2,4,6-triisopropylphenyl), alkylnaphthyl groups (e.g., methylnaphthyl and ethylnaphthyl), dialkylnaphthyl groups (e.g., dimethylnaphthyl and diethylnaphthyl); and C₇-C₄₀ aralkyl groups such as benzyl, 1-phenylethyl and 2-phenylethyl.

In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, carbonyl moiety, ether bond, thioether bond, ester bond, sulfonic ester bond, carbonate bond, lactone ring, sultone ring, carboxylic anhydride (-C(=O)-O-C(=O)-), or haloalkyl moiety. Examples of heteroatom-containing hydrocarbyl groups include heteroaryl groups such as thienyl, 4-hydroxyphenyl, alkoxyphenyl groups such as 4-methoxyphenyl, 3-methoxyphenyl, 2-methoxyphenyl, 4-ethoxyphenyl, 4-tert-butoxyphenyl, 3-tert-butoxyphenyl; alkoxynaphthyl groups such as methoxynaphthyl, ethoxynaphthyl, n-propoxynaphthyl and n-butoxynaphthyl; dialkoxynaphthyl groups such as dimethoxynaphthyl and diethoxynaphthyl; and aryloxoalkyl groups, typically 2-aryl-2-oxoethyl groups such as 2-phenyl-2-oxoethyl, 2-(1-naphthyl)-2-oxoethyl and 2-(2-naphthyl)-2-oxoethyl.

In formula (F1), Mq_{A}⁺ is an onium cation. The onium cation is preferably a sulfonium, iodonium or ammonium cation, with the sulfonium cation or iodonium cation being more preferred. Examples of the sulfonium cation are as exemplified above for the sulfonium cations of formulae (Z-1) and (Z-3). Examples of the iodonium cation are as exemplified for the iodonium cation of formula (Z-2).

Examples of the anion in the onium salt having formula (F1) are shown below, but not limited thereto.

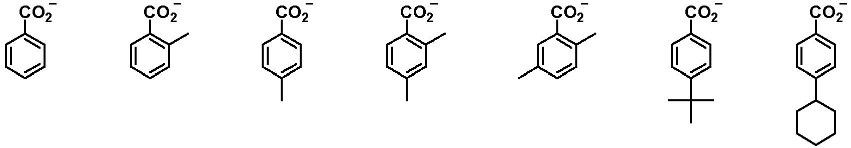

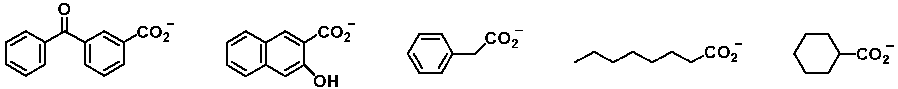

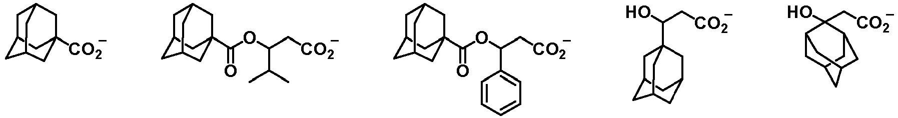

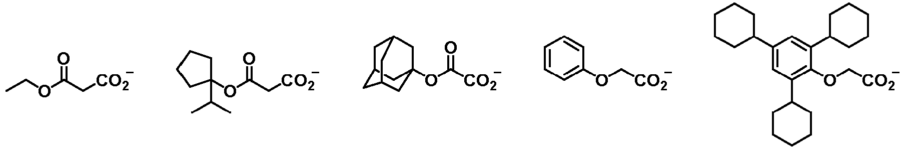

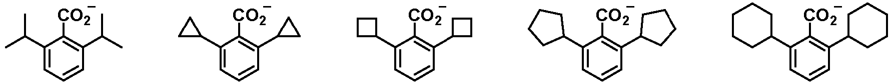

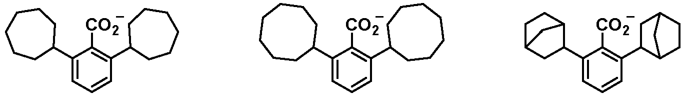

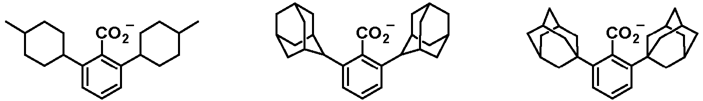

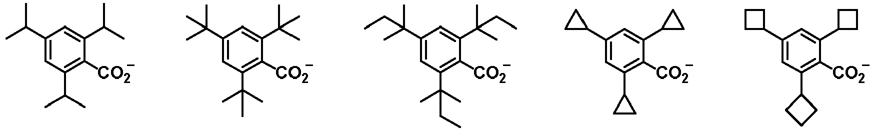

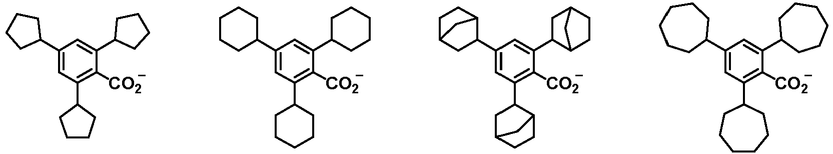

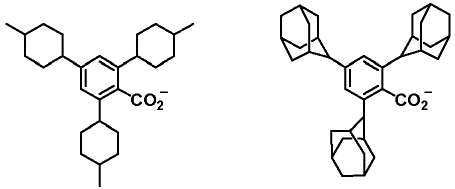

A sulfonium salt of iodized benzene ring-containing carboxylic acid having the formula (F2) is also useful as the quencher.

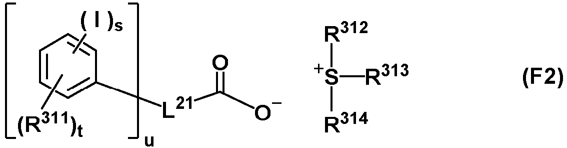

In formula (F2), s is 1, 2, 3, 4 or 5. The subscript t is 0, 1, 2 or 3. The sum s+t is 1 or more and 5 or less. The subscript u is 1, 2 or 3.

In formula (F2), R³¹¹ is hydroxy, fluorine, chlorine, bromine, amino, nitro, cyano, or a C₁-C₆ saturated hydrocarbyl, C₁-C₆ saturated hydrocarbyloxy, C₂-C₆ saturated hydrocarbylcarbonyloxy, or C₁-C₄ saturated hydrocarbylsulfonyloxy group, in which some or all hydrogen atoms may be substituted by halogen atom, or -N(R^{311A})-C(=O)-R^{311B} or -N(R^{311A})-C(=O)-O-R^{311B}. R^{311A} is hydrogen or a C₁-C₆ saturated hydrocarbyl group. R^{311B} is a C₁-C₆ saturated hydrocarbyl group, or a C₂-C₈ unsaturated hydrocarbyl group. A plurality of R³¹¹ may be identical or different when t and/or u are 2 or more.

In formula (F2), L²¹ is a single bond, or a C₁-C₂₀ (u+1)-valent linking group which may contain at least one moiety selected from an ether bond, a carbonyl group, an ester bond, an amide bond, a sultone ring, a lactam ring, a carbonate bond, a halogen atom, a hydroxy group and a carboxy group. The saturated hydrocarbyl, saturated hydrocarbyloxy, saturated hydrocarbylcarbonyloxy and saturated hydrocarbylsulfonyloxy groups may be straight, branched or cyclic.

In formula (F2), R³¹², R³¹³ and R³¹⁴ are each independently halogen atom or a C₁-C₂₀ hydrocarbyl group which may contain a heteroatom. The hydrocarbyl group may be saturated or unsaturated, and may be straight, branched, or cyclic. Examples thereof include C₁-C₂₀ alkyl, C₂-C₂₀ alkenyl, C₆-C₂₀ aryl and C₇-C₂₀ aralkyl groups. In these groups, some or all hydrogen may be substituted by hydroxy, carboxy, halogen, oxo, cyano, nitro, sultone ring, sulfo, or sulfonium salt-containing moiety, or some -CH₂- may be replaced by an ether bond, ester bond, carbonyl moiety, amide bond, carbonate bond or sulfonate ester bond. R³¹² and R³¹³ may bond together to form a ring with a sulfur atom to which they are attached.

Examples of the compound having formula (F2) include those described in USP 10,295,904 (JP-A 2017-219836). The compounds (F2) exert a sensitizing effect due to remarkable absorption and an acid diffusion-controlling effect.

A nitrogen-containing carboxylic acid salt compound having the formula (F3) is also useful as the quencher.

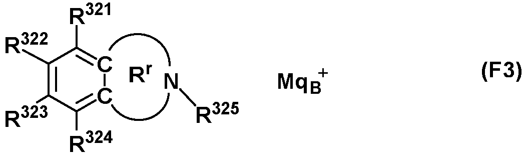

In formula (F3), R³²¹ to R³²⁴ are each independently a hydrogen atom, -L³¹-CO₂⁻, or a C₁-C₂₀ hydrocarbyl group which may contain a heteroatom. R³²¹ and R³²², R³²² and R³²³, or R³²³ and R³²⁴ may bond together to form a ring with the carbon atom to which they are attached. L³¹ is a single bond or C₁-C₂₀ hydrocarbylene group which may contain a heteroatom. R³²⁵ is hydrogen or a C₁-C₂₀ hydrocarbyl group which may contain a heteroatom.

In formula (F3), the ring R^{r} is a C₂-C₆ ring containing the carbon and nitrogen atoms in the formula, in which some or all of the carbon-bonded hydrogen atoms may be substituted by a C₁-C₂₀ hydrocarbyl group or -L³¹-CO₂⁻ and in which some carbon may be replaced by sulfur, oxygen or nitrogen. The ring may be alicyclic or aromatic and is preferably a 5- or 6-membered ring. Suitable rings include pyridine, pyrrole, pyrrolidine, piperidine, pyrazole, imidazoline, pyridazine, pyrimidine, pyrazine, imidazoline, oxazole, thiazole, morpholine, thiazine, and triazole rings.

The carboxylic onium salt having formula (F3) has at least one -L³¹-CO₂⁻ group. That is, at least one of R³²¹ to R³²⁴ is -L³¹-CO₂⁻, and/or at least one of hydrogen atoms bonded to carbon atoms in the ring R^{r} is substituted by -L³¹-CO₂⁻.

In formula (F3), Mq_{B}⁺ is a sulfonium, iodonium or ammonium cation, with the sulfonium cation being preferred. Examples of the sulfonium cation are as exemplified above for the sulfonium cations of formulae (Z-1) and (Z-3).

Examples of the anion in the compound having formula (F3) are shown below, but not limited thereto.

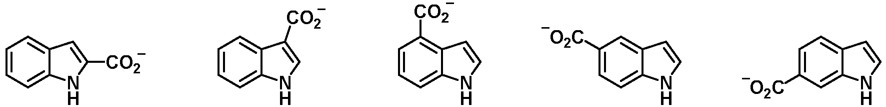

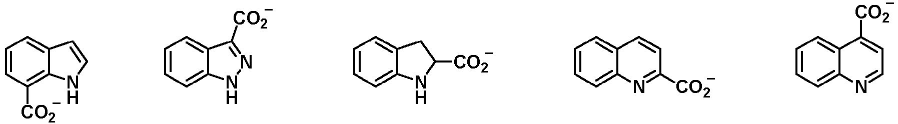

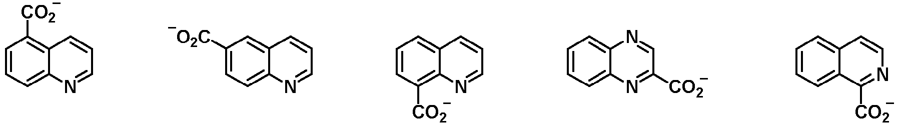

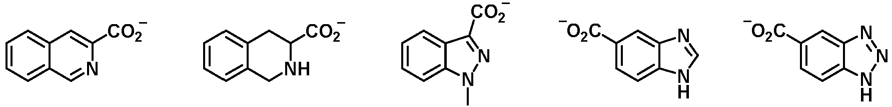

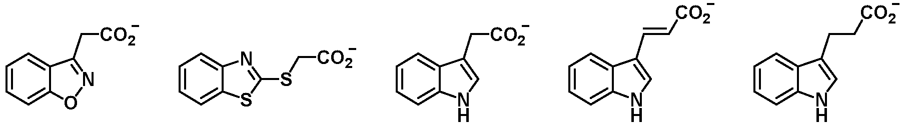

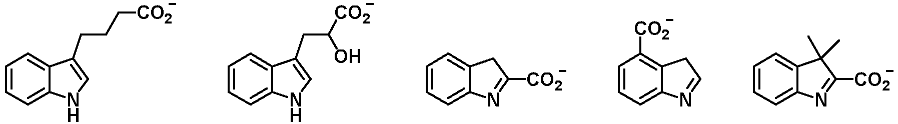

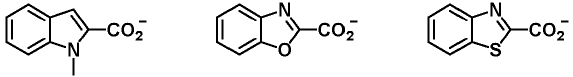

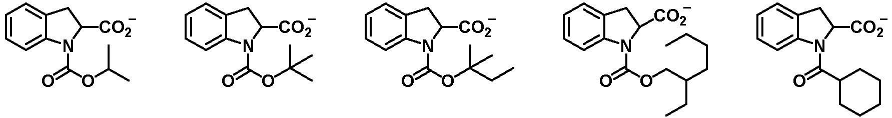

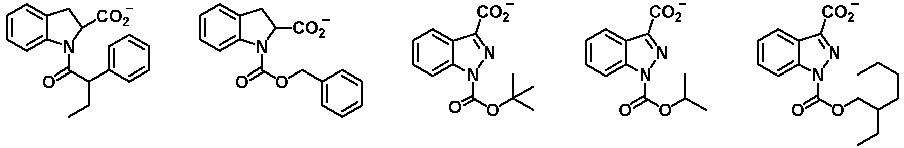

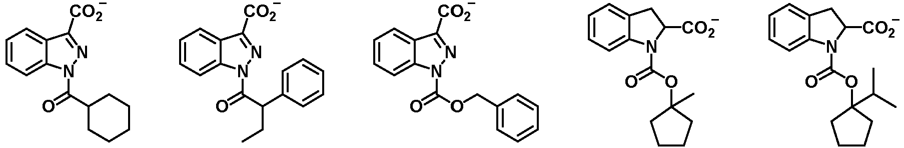

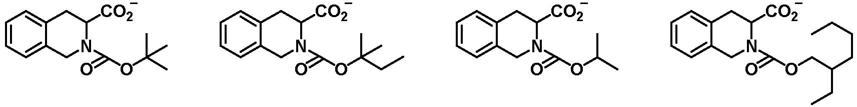

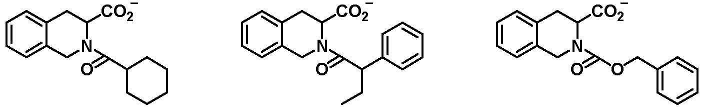

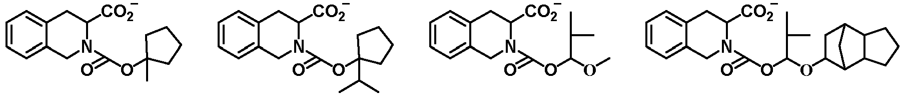

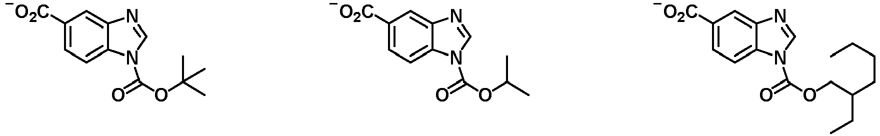

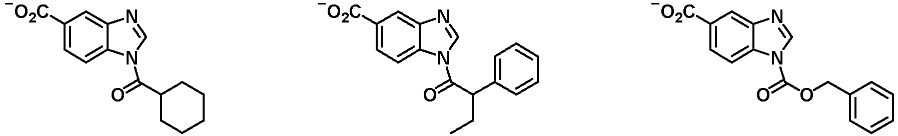

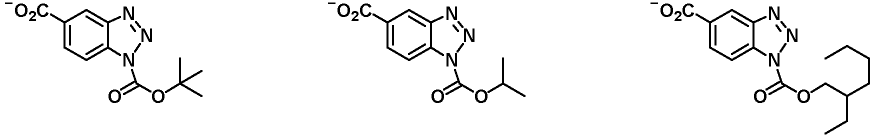

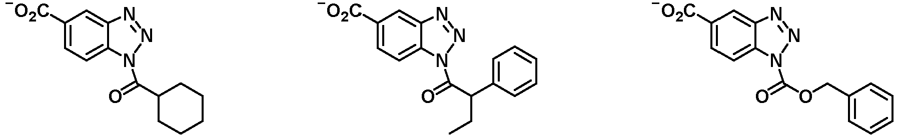

Weak acid betaine compounds are also useful as the quencher. Examples of the hydrocarbylene group are shown below, but not limited thereto.

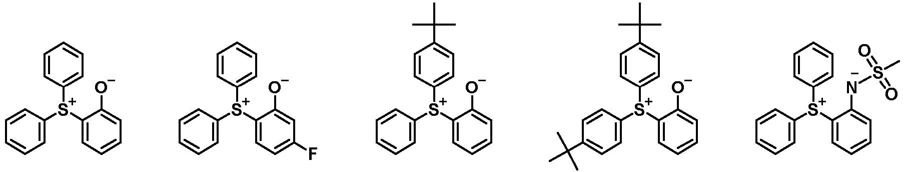

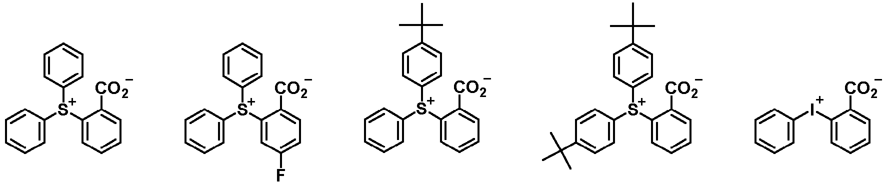

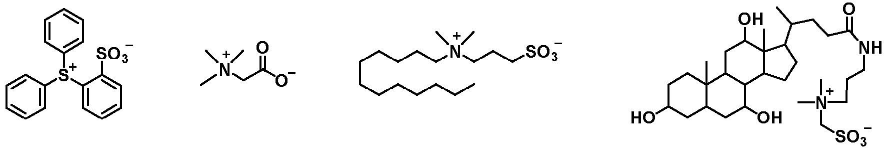

Also useful are quenchers of polymer type as described in USP 7,598,016 (JP-A 2008-239918). The polymeric quencher segregates at the resist surface after coating and thus enhances the rectangularity of resist pattern. When a protective film is applied as is often the case in the immersion lithography, the polymeric quencher is also effective for preventing a film thickness loss of resist pattern or rounding of pattern top.

When the inventive chemically amplified positive resist composition comprises the other quencher (F), the content of the quencher is preferably 0 to 50 parts by weight, more preferably 0.1 to 40 parts by weight per 80 parts by weight of the base polymer (B). The quencher may be used alone or in admixture.

### (G) Surfactant

The inventive chemically amplified positive resist composition may contain (G) any conventional surfactants as component for facilitating to coat the composition to the substrate. A number of surfactants are known in the art as described in WO 2006/121096, JP-A 2008-102383, JP-A 2008-304590, JP-A 2004-115630 and JP-A 2005-8766, and any suitable one may be chosen therefrom.

When the inventive chemically amplified positive resist composition comprises surfactant (G), the content of the surfactant (G) is preferably 2 parts by weight or less, more preferably 1 part by weight or less per 80 parts by weight of the base polymer (B). The lower limit thereof is preferably 0.01 parts by weight or more. The surfactant (G) may be used alone or in admixture.

### [Resist pattern forming process]

The inventive resist pattern forming process comprises the steps of: applying the chemically amplified positive resist composition defined herein onto a substrate to form a resist film thereon, exposing the resist film to a pattern of high-energy radiation, and developing the exposed resist film in an alkaline developer.

The substrate used herein may be a substrate for integrated circuitry fabrication, e.g., Si, SiO, SiO₂, SiN, SiON, TiN, WSi, BPSG, SOG, organic antireflective film, etc. or a substrate for mask circuitry fabrication, e.g., Cr, CrO, CrON, MoSi₂, Si, SiO, SiO₂, etc. The chemically amplified positive resist composition is coated on the substrate by a method such as spin coating so as to have a film thickness of 0.03 to 2 µm, and is prebaked on a hot plate at 60 to 150°C for 1 to 20 minutes, more preferably at 80 to 140°C for 1 to 10 minutes to form a resist film.

The resist film is then exposed to a desired pattern of high-energy radiation. Examples of the high-energy radiation include UV, deep-UV, excimer laser radiation (e.g., KrF or ArF), EB, EUV, X-rays, γ-rays and synchrotron radiation.

When UV, deep-UV, excimer laser, EUV, X-rays, γ-rays, or synchrotron radiation is used as the high-energy radiation, the resist film is exposed thereto through a mask having a desired pattern preferably at a dose of about 1 to 300 mJ/cm², more preferably 10 to 200 mJ/cm². On use of EB as the high-energy radiation, a pattern is written directly preferably in a dose of 1 to 300 µC/cm², more preferably 10 to 200 µC/cm². The inventive chemically amplified positive resist composition is particularly for EUV or EB lithography.

The exposure may be performed by conventional lithography whereas the immersion lithography of holding a liquid, typically water between the resist film and the mask may be employed if desired. In the case of immersion lithography, a protective film which is insoluble in water may be formed on the resist film.

After the exposure, the resist film may be baked (PEB) on a hotplate preferably at 60 to 150°C for 1 to 20 minutes, more preferably at 80 to 140°C for 1 to 10 minutes.

After the exposure or PEB, the resist film is developed in a developer in the form of an aqueous alkaline solution for preferably 0.1 to 3 minutes, more preferably 0.5 to 2 minutes by conventional techniques such as dip, puddle and spray techniques. A preferable developer is a 0.1 to 5 wt%, more preferably 2 to 3 wt% aqueous solution of tetramethylammonium hydroxide (TMAH) or another alkali. In this way, the desired pattern is formed on the substrate.

The inventive chemically amplified positive resist composition has particularly high etch resistance, and is effectively used under conditions required to ensure that even when the time until PEB after exposure is extended, the change of the pattern line width as well as LER is small. The resist composition is effectively applicable to a substrate, specifically a substrate having a surface layer of material to which a resist film is less adherent and which is likely to invite pattern stripping or pattern collapse. A typical substrate has sputter deposited on their outermost surface metallic chromium or a chromium compound containing at least one light element selected from oxygen, nitrogen and carbon. The inventive chemically amplified positive resist composition is effective for pattern formation using a photomask blank as a substrate.

### EXAMPLES

Synthesis Examples, Examples, and Comparative Examples of the invention are given below by way of illustration and not by way of limitation. The apparatuses used are as follows.
MALDI TOF-MS: S3000 manufactured by JEOL Ltd.

### [1] Synthesis of sulfonium salts

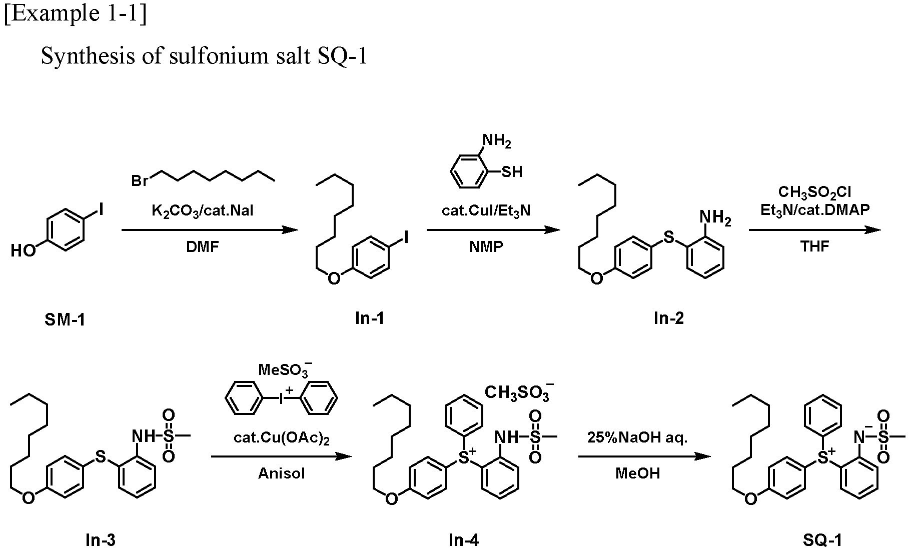

### (1) Synthesis of Intermediate In-1

In a reactor under nitrogen atmosphere, 110.0 g of 4-iodophenol, 82.9 g of potassium carbonate and 7.5 g of sodium iodide were dissolved in 400 g of DMF. The reactor was warmed up to an internal temperature of 70°C, and 106.2 g of n-octyl bromide was added. After addition, the reaction mixture was heated and aged at a reactor internal temperature of 80°C for 12 hours. At the end of aging, the reaction solution was cooled, and 500 g of water was added to quench the reaction, followed by extraction of a target with 500 g of hexane. This was followed by ordinary aqueous work-up, solvent stripping, and purification with a silica gel column. There was obtained Intermediate In-1 was obtained as oily matter (amount 162.8 g, yield 98%).

### (2) Synthesis of Intermediate In-2

In a reactor under nitrogen atmosphere, 132.9 g of Intermediate In-1, 55.1 g of 2-aminobenzenethiol and 3.8 g of copper iodide were dissolved in 400 g of N-methylpyrrolidone. The reactor was warmed up to an internal temperature of 70°C, and 48.6 g of triethylamine was added. After addition, the reaction mixture was heated and aged at a reactor internal temperature of 80°C for 12 hours. At the end of aging, the reaction solution was cooled, and 300 g of water was added to quench the reaction, followed by extraction of a target with 500 g of toluene. This was followed by ordinary aqueous work-up, solvent stripping, and purification with a silica gel column. Intermediate In-2 was obtained as oily matter (amount 110.7 g, yield 84%).

### (3) Synthesis of Intermediate In-3

In a reactor under nitrogen atmosphere, 98.9 g of Intermediate In-2, 36.4 g of triethylamine and 3.7 g of 4-dimethylaminopyridine were dissolved in 300 g of THF. The reactor was cooled to an internal temperature of 10°C or lower, and 37.8 g of methanesulfonyl chloride was added dropwise. After addition, the reaction mixture was aged at a reactor internal temperature of 40°C for 12 hours. At the end of aging, the reaction solution was cooled, and 500 g of water was added to quench the reaction, followed by extraction of a target with 500 g of hexane. This was followed by ordinary aqueous work-up, solvent stripping, and purification with a silica gel column. Intermediate In-3 was obtained as oily matter (amount 117.4 g, yield 96%).

### (4) Synthesis of Intermediate In-4

In a reactor under nitrogen atmosphere, 40.8 g of Intermediate In-3 and 41.4 g of diphenyliodonium=methane sulfonate were dissolved in 150 g of anisole. This was followed by addition of 0.9 g of copper acetate. The reactor was warmed up to an internal temperature of 100°C, and the reaction mixture was heated and aged for 12 hours. At the end of aging, the reaction solution was cooled, and 200 g of water was added to quench the reaction, followed by extraction of a target with 300 g of methylene chloride. This was followed by ordinary aqueous work-up, solvent stripping, and purification with a silica gel column. Intermediate In-4 was obtained as oily matter (amount 50.4 g, yield 87%).

### (5) Synthesis of sulfonium salt SQ-1

In a reactor under nitrogen atmosphere, 14.5 g of Intermediate In-4 was dissolved in 40 g of methanol. 4.8 g of a 25 wt% sodium hydroxide aqueous solution was added, and the reaction mixture was aged for 4 hours. At the end of aging, 30 g of water was added to quench the reaction, and 80 g of methylene chloride was added to extract a target. This was followed by ordinary aqueous work-up, and solvent stripping. This was followed by washing of the residue with diisopropyl ether. Target SQ-1 was obtained as oily matter (amount 10.2 g, yield 84%).
SQ-1 was analyzed by TOF-MS, with the data shown below.
MALDI TOF-MS: POSITIVE M⁺484 (corresponding to C₂₇H₃₄NO₃S₂⁺)

### [Examples 1-2 to 1-10]

### Synthesis of sulfonium salts SQ-2 to SQ-10

Sulfonium Salt SQ-2 to SQ-10 of the following formulae were synthesized using the corresponding reactants and well-known organic chemistry reaction.

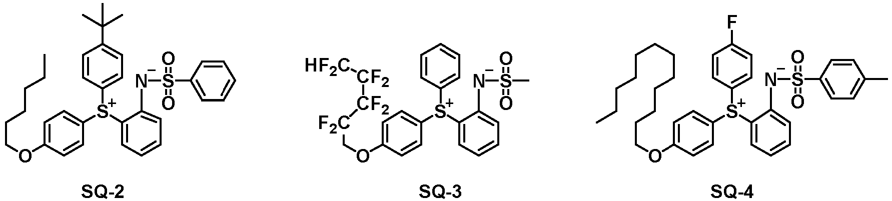

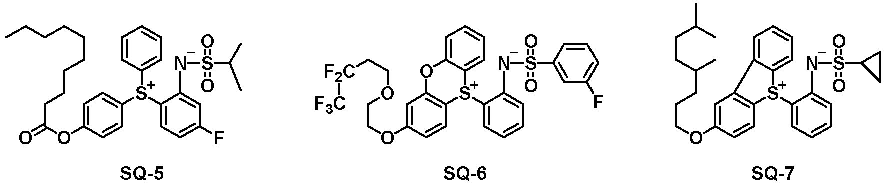

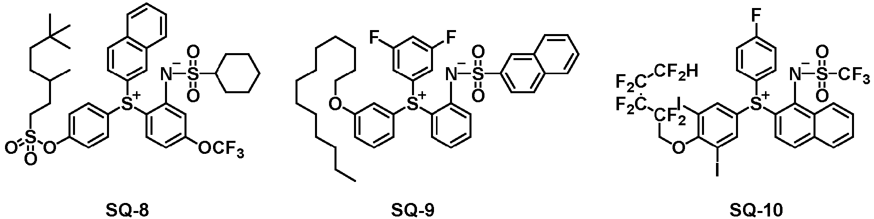

### [2] Synthesis of base polymers

### [Synthesis Example]

### Synthesis of base polymers (polymers P-1 to P-6)

Base polymers P-1 to P-6 were synthesized by combining monomers in accordance with a known formulation, performing copolymerization reaction in a solvent, pouring the reaction solution to hexane for precipitation, washing the solid precipitate with hexane, isolation and drying. The base polymers were analyzed for composition by ¹H-NMR and ¹³C-NMR spectroscopy and for Mw and Mw/Mn by GPC versus polystyrene standards using THF solvent.

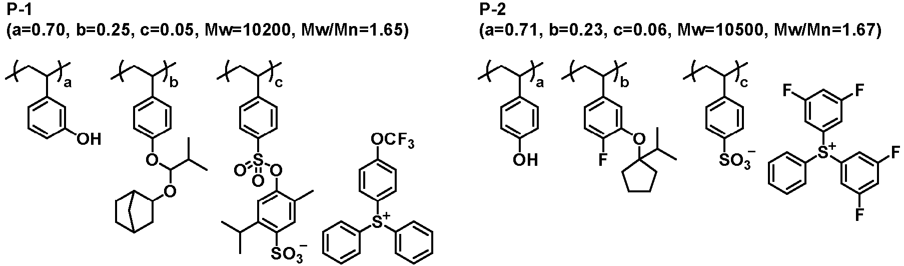

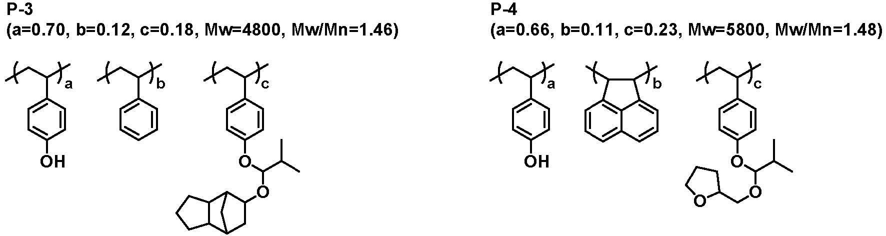

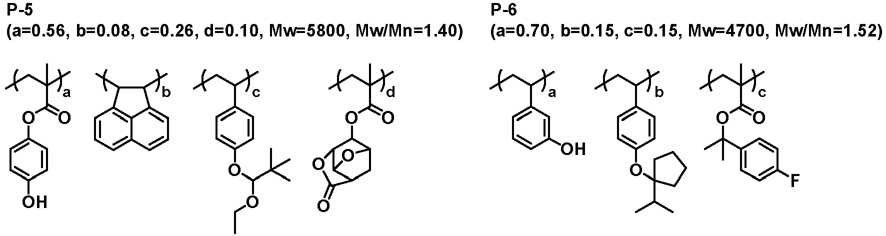

### [3] Preparation of chemically amplified positive resist compositions

### [Examples 2-1 to 2-50 and Comparative Examples 1-1 to 1-35]

A chemically amplified positive resist composition was prepared by dissolving selected components in an organic solvent in accordance with the formulation shown in Tables 1 to 3 and filtering the solution through a nylon filter with a pore size of 5 nm and a UPE filter with a pore size of 1 nm. The organic solvent was a mixture of 940 pbw of PGMEA, 1,870 pbw of EL, and 1,870 pbw of PGME.

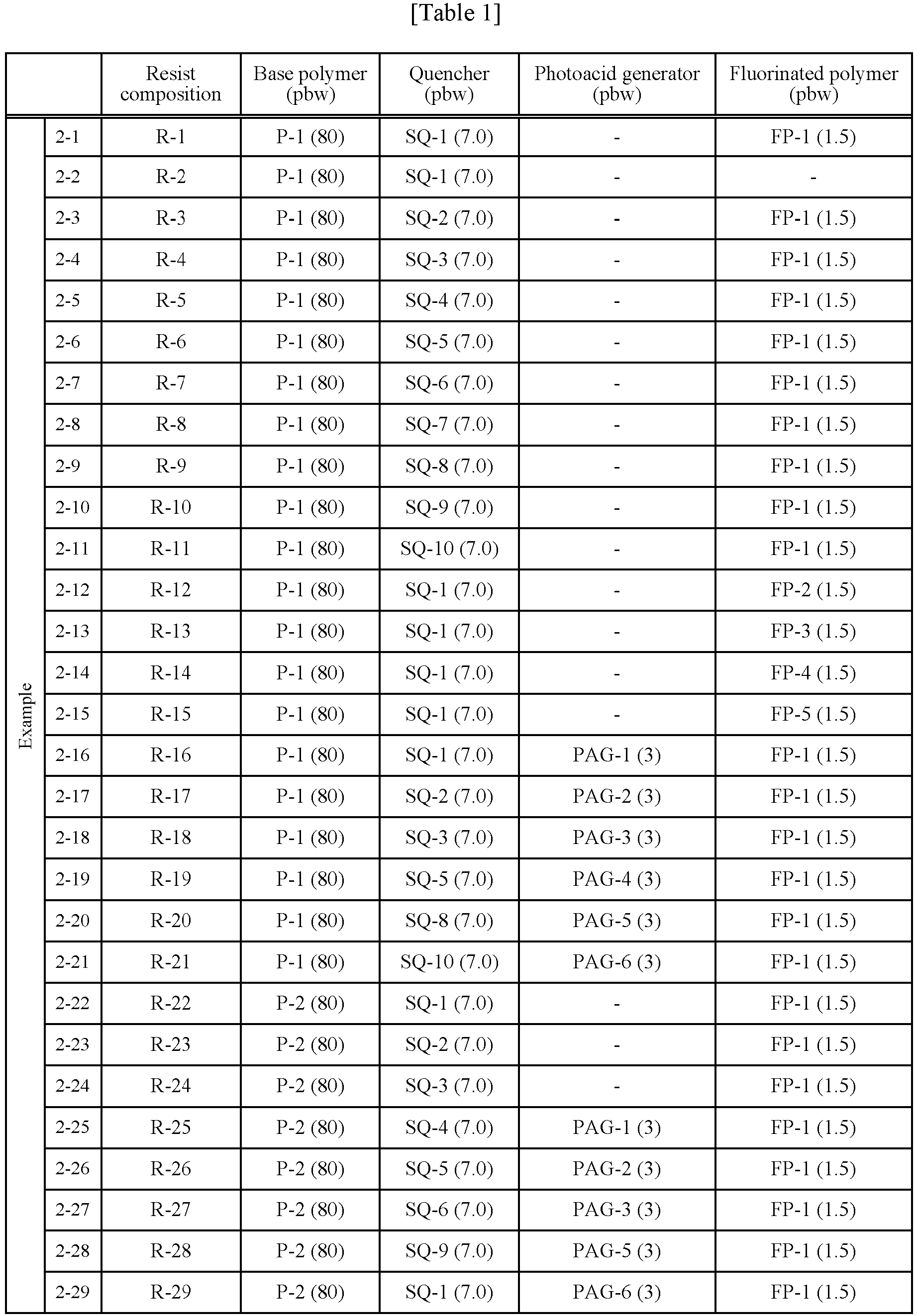

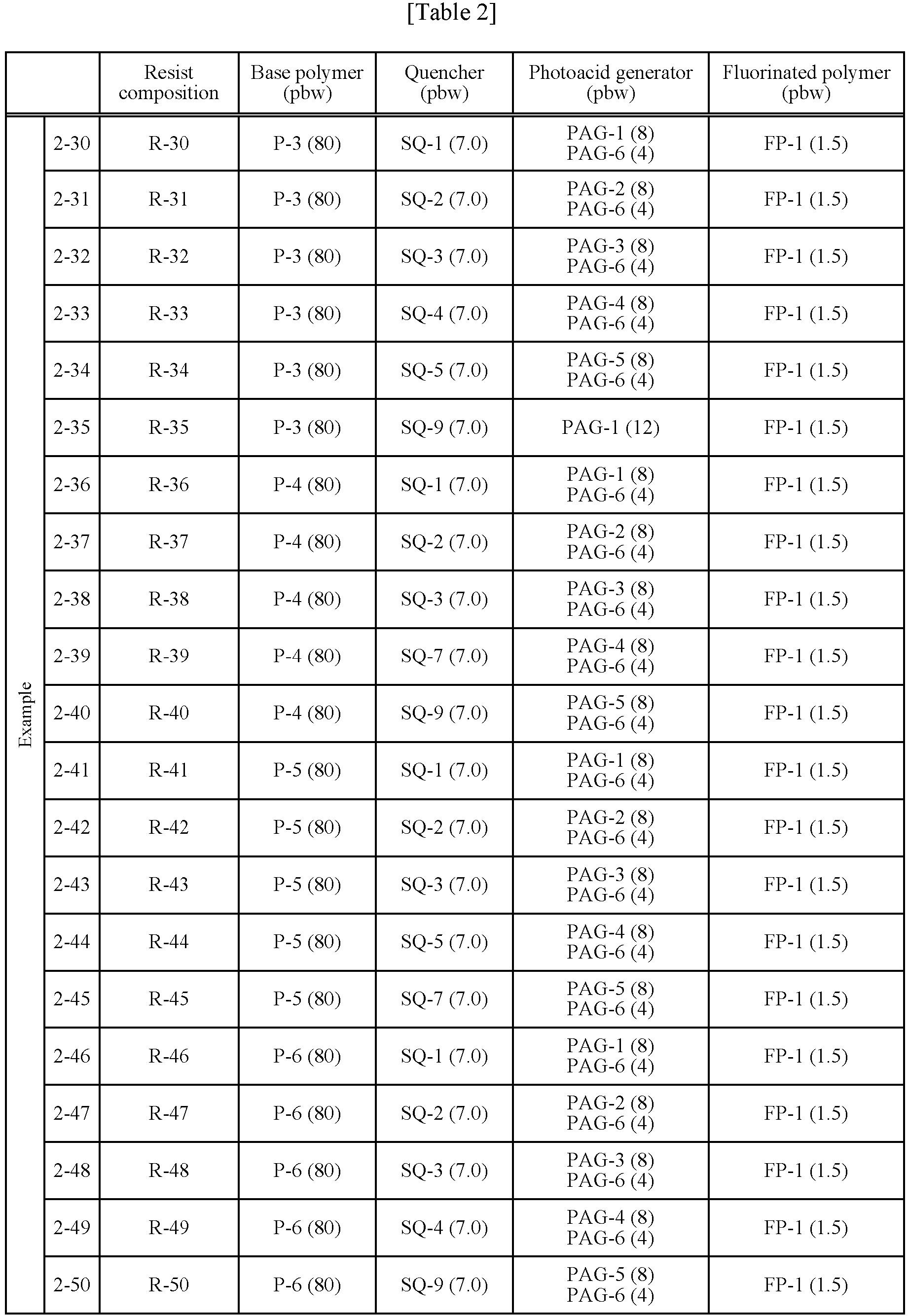

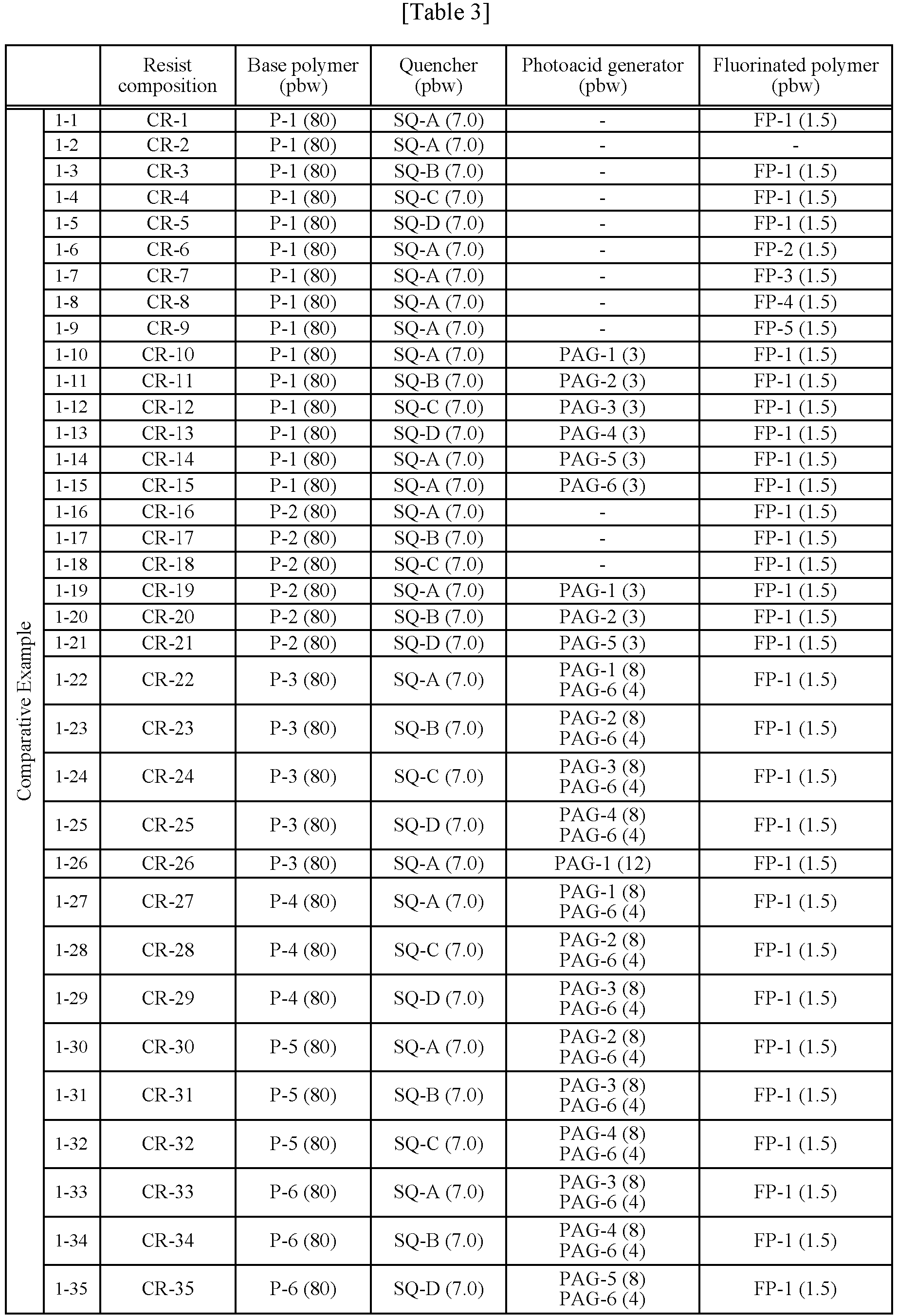

Photoacid generators PAG-1 to PAG-6, comparative quenchers SQ-A to SQ-D and fluorinated polymers FP-1 to FP-5 in Tables 1 to 3 are identified below.

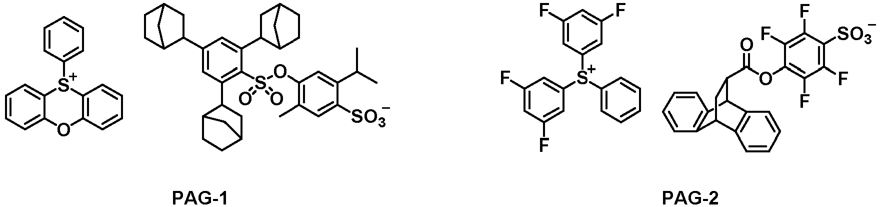

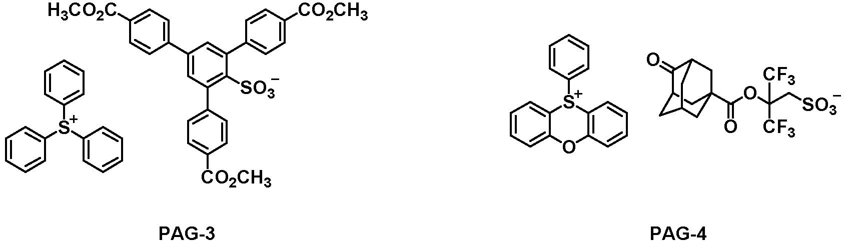

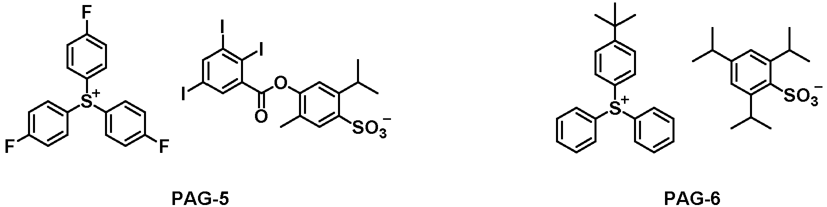

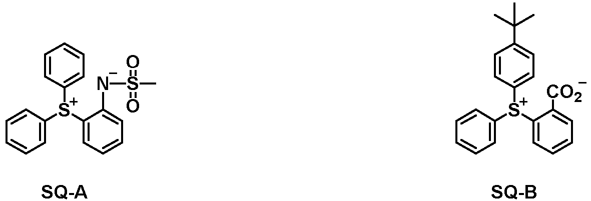

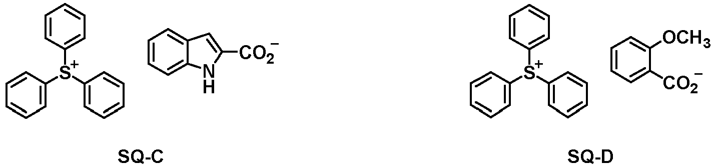

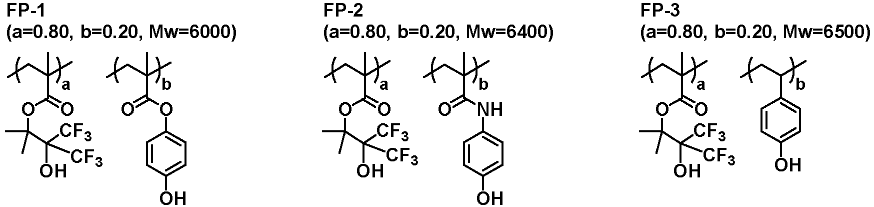

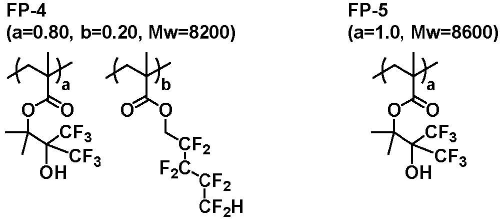

### [4] EB lithography test

### [Examples 3-1 to 3-50 and Comparative Examples 2-1 to 2-35]

A 152 mm-square photomask blank of reflection type for an EUV lithography mask, in which a chromium compound forms an outermost surface, was provided. Using ACT-M (Tokyo Electron Ltd.), each of the chemically amplified positive resist compositions (R-1 to R-50, CR-1 to CR-35) was spin coated onto the photomask blank, and prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding an outer rim portion extending 10 mm inward from the periphery, and an average film thickness and a film thickness range were computed therefrom.

Next, the resist film was exposed to EB using an EB writer system EBM-5000Plus (NuFlare Technology Inc., accelerating voltage 50 kV), then baked (PEB) at 110°C for 600 seconds, and developed in a 2.38 wt% TMAH aqueous solution, thereby yielding a positive pattern.

The resist pattern was evaluated as follows. The patterned mask blank was observed under a top-down scanning electron microscope (TD-SEM). The optimum dose (Eop) was defined as the exposure dose (µC/cm²) which provided a 1:1 resolution at the top and bottom of a 200-nm 1:1 line-and-space (LS) pattern. The resolution (or maximum resolution) was defined as the minimum size at the dose which provided a 1:1 resolution for a LS of 200 nm. The edge roughness (LER) of a 200-nm LS pattern was measured under SEM. The maximum resolution of isolated-space (IS) was defined as the minimum size at the dose which provided a 9:1 resolution at the top and bottom of 200 nm 9:1 line-ns-space (LS) pattern. The pattern was visually observed to judge whether or not the profile was rectangular. The results are shown in Tables 4 to 6.

**[Table 4]**

| | | Resist composition | Optimal exposure dose (µC/cm²) | L/S resolution (nm) | Maximum IS resolution (nm) | LER (nm) | Pattern profile |
|---|---|---|---|---|---|---|---|
| | 3-1 | R-1 | 205 | 28 | 18 | 2.6 | rectangular |
| | 3-2 | R-2 | 205 | 28 | 16 | 2.7 | rectangular |
| | 3-3 | R-3 | 200 | 28 | 18 | 2.8 | rectangular |
| | 3-4 | R-4 | 210 | 30 | 16 | 2.9 | rectangular |
| | 3-5 | R-5 | 200 | 28 | 18 | 2.7 | rectangular |
| | 3-6 | R-6 | 200 | 30 | 20 | 2.8 | rectangular |
| | 3-7 | R-7 | 205 | 32 | 16 | 2.9 | rectangular |
| | 3-8 | R-8 | 200 | 28 | 18 | 2.8 | rectangular |
| | 3-9 | R-9 | 205 | 28 | 16 | 2.9 | rectangular |
| | 3-10 | R-10 | 200 | 30 | 18 | 2.7 | rectangular |
| | 3-11 | R-11 | 210 | 30 | 18 | 3 | rectangular |
| | 3-12 | R-12 | 200 | 30 | 16 | 2.9 | rectangular |
| | 3-13 | R-13 | 205 | 32 | 18 | 2.7 | rectangular |
| | 3-14 | R-14 | 200 | 28 | 18 | 2.8 | rectangular |
| Example | 3-15 | R-15 | 205 | 28 | 18 | 2.9 | rectangular |
| | 3-16 | R-16 | 200 | 30 | 16 | 2.8 | rectangular |
| | 3-17 | R-17 | 200 | 30 | 16 | 3 | rectangular |
| | 3-18 | R-18 | 205 | 30 | 20 | 2.9 | rectangular |
| | 3-19 | R-19 | 200 | 28 | 18 | 2.8 | rectangular |
| | 3-20 | R-20 | 205 | 28 | 18 | 2.7 | rectangular |
| | 3-21 | R-21 | 200 | 28 | 16 | 2.9 | rectangular |
| | 3-22 | R-22 | 205 | 30 | 18 | 2.9 | rectangular |
| | 3-23 | R-23 | 205 | 30 | 18 | 2.7 | rectangular |
| | 3-24 | R-24 | 205 | 28 | 16 | 2.9 | rectangular |
| | 3-25 | R-25 | 210 | 28 | 18 | 2.8 | rectangular |
| | 3-26 | R-26 | 205 | 32 | 18 | 2.9 | rectangular |
| | 3-27 | R-27 | 205 | 30 | 16 | 2.7 | rectangular |
| | 3-28 | R-28 | 200 | 28 | 16 | 2.9 | rectangular |
| | 3-29 | R-29 | 205 | 28 | 20 | 2.9 | rectangular |

**[Table 5]**

| | | Resist composition | Optimal exposure dose (µC/cm²) | L/S resolution (nm) | Maximum IS resolution (nm) | LER (nm) | Pattern profile |
|---|---|---|---|---|---|---|---|
| | 3-30 | R-30 | 205 | 28 | 18 | 2.9 | rectangular |
| | 3-31 | R-31 | 200 | 30 | 18 | 2.9 | rectangular |
| | 3-32 | R-32 | 200 | 30 | 16 | 3 | rectangular |
| | 3-33 | R-33 | 205 | 38 | 18 | 2.7 | rectangular |
| | 3-34 | R-34 | 200 | 30 | 16 | 2.7 | rectangular |
| | 3-35 | R-35 | 210 | 30 | 20 | 2.8 | rectangular |
| | 3-36 | R-36 | 205 | 28 | 18 | 2.9 | rectangular |
| | 3-37 | R-37 | 200 | 28 | 20 | 2.9 | rectangular |
| | 3-38 | R-38 | 205 | 28 | 18 | 2.8 | rectangular |
| | 3-39 | R-39 | 205 | 28 | 20 | 3 | rectangular |
| Example | 3-40 | R-40 | 200 | 28 | 16 | 2.8 | rectangular |
| | 3-41 | R-41 | 205 | 30 | 16 | 2.9 | rectangular |
| | 3-42 | R-42 | 200 | 30 | 20 | 2.7 | rectangular |
| | 3-43 | R-43 | 205 | 28 | 16 | 2.6 | rectangular |
| | 3-44 | R-44 | 200 | 28 | 18 | 2.8 | rectangular |
| | 3-45 | R-45 | 210 | 28 | 16 | 2.7 | rectangular |
| | 3-46 | R-46 | 205 | 28 | 16 | 2.9 | rectangular |
| | 3-47 | R-47 | 200 | 30 | 18 | 2.7 | rectangular |
| | 3-48 | R-48 | 205 | 28 | 20 | 2.9 | rectangular |
| | 3-49 | R-49 | 210 | 32 | 16 | 2.7 | rectangular |
| | 3-50 | R-50 | 200 | 28 | 18 | 2.7 | rectangular |

**[Table 6]**

| | | Resist composition | Optimal exposure dose (µC/cm²) | L/S resolution (nm) | Maximum IS resolution (nm) | LER (nm) | Pattern profile |
|---|---|---|---|---|---|---|---|
| | 2-1 | CR-1 | 200 | 34 | 22 | 3.3 | rounded top |
| | 2-2 | CR-2 | 210 | 34 | 24 | 3.4 | footing |
| | 2-3 | CR-3 | 200 | 34 | 22 | 3.2 | rounded top |
| | 2-4 | CR-4 | 205 | 34 | 22 | 3.5 | footing |
| | 2-5 | CR-5 | 200 | 34 | 22 | 3.4 | rounded top |
| | 2-6 | CR-6 | 210 | 32 | 24 | 3.3 | rounded top |
| | 2-7 | CR-7 | 205 | 34 | 22 | 3.3 | rounded top |
| | 2-8 | CR-8 | 200 | 34 | 24 | 3.4 | rounded top |
| | 2-9 | CR-9 | 210 | 34 | 22 | 3.6 | rounded top |
| | 2-10 | CR-10 | 205 | 36 | 22 | 3.2 | footing |
| | 2-11 | CR-11 | 200 | 36 | 22 | 3.4 | rounded top |
| | 2-12 | CR-12 | 205 | 34 | 22 | 3.3 | footing |
| | 2-13 | CR-13 | 210 | 36 | 22 | 3.6 | rounded top |
| | 2-14 | CR-14 | 200 | 32 | 22 | 3.3 | rounded top |
| | 2-15 | CR-15 | 210 | 34 | 24 | 3.6 | rounded top |
| | 2-16 | CR-16 | 200 | 34 | 22 | 3.2 | footing |
| | 2-17 | CR-17 | 205 | 34 | 24 | 3.4 | rounded top |
| Comparative Example | 2-18 | CR-18 | 200 | 36 | 22 | 3.5 | rounded top |
| | 2-19 | CR-19 | 210 | 34 | 22 | 3.4 | footing |
| | 2-20 | CR-20 | 205 | 34 | 22 | 3.3 | rounded top |
| | 2-21 | CR-21 | 200 | 34 | 24 | 3.6 | footing |
| | 2-22 | CR-22 | 210 | 34 | 22 | 3.4 | rounded top |
| | 2-23 | CR-23 | 200 | 34 | 22 | 3.3 | footing |
| | 2-24 | CR-24 | 200 | 36 | 22 | 3.4 | rounded top |
| | 2-25 | CR-25 | 210 | 34 | 22 | 3.5 | rounded top |
| | 2-26 | CR-26 | 210 | 32 | 22 | 3.2 | rounded top |
| | 2-27 | CR-27 | 200 | 32 | 22 | 3.3 | footing |
| | 2-28 | CR-28 | 205 | 32 | 22 | 3.2 | rounded top |
| | 2-29 | CR-29 | 200 | 34 | 24 | 3.4 | rectangular |
| | 2-30 | CR-30 | 210 | 32 | 22 | 3.3 | rounded top |
| | 2-31 | CR-31 | 205 | 32 | 22 | 3.5 | footing |
| | 2-32 | CR-32 | 200 | 34 | 24 | 3.2 | rounded top |
| | 2-33 | CR-33 | 210 | 32 | 24 | 3.4 | rounded top |
| | 2-34 | CR-34 | 205 | 34 | 22 | 3.4 | rounded top |
| | 2-35 | CR-35 | 200 | 36 | 24 | 3.3 | footing |

The patterns from the inventive chemically amplified positive resist compositions (R-1 to R-50) all exhibited good resolution, good LER and a good rectangular profile. For the comparative resist compositions (CR-1 to CR-35), acid diffusion was not sufficiently optimized, and the patterns were poor in resolution, LER and rectangular profile.

The resist pattern forming process using the inventive chemically amplified positive resist composition is useful in the photolithography for the fabrication of semiconductor devices, especially the processing of photomask blanks of transmission and reflection types.

## Claims

1. A sulfonium salt having the formula (A): 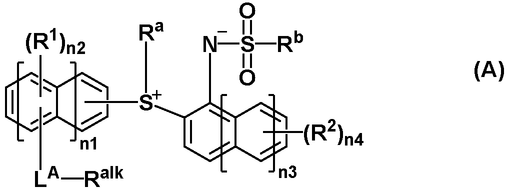 wherein n1 is 0 or 1, n2 is 0, 1, 2, 3, or 4, n3 is 0 or 1, n4 is 0, 1, 2, 3 or 4,
L^{A} is a single bond, ether bond, ester bond, sulfonic ester bond, carbonate bond, or carbamate bond,
R^{alk} is a C₆-C₁₈ alkyl group or a C₄-C₁₈ fluorinated alkyl group, wherein when R^{alk} is a C₆-C₁₈ alkyl group, the alkyl group has at least one straight chain structure having 6 or more carbon atoms, and when R^{alk} is a C₄-C₁₈ fluorinated alkyl group, the fluorinated alkyl group has at least two groups selected from -CF₂- and -CF₃, some constituent -CH₂-in the alkyl group and fluorinated alkyl group may be replaced by an ether bond or a carbonyl group, and the alkyl group and fluorinated alkyl group may contain a ring structure selected from a cyclopentane ring, a cyclohexane ring, an adamantane ring, a norbornene ring and a benzene ring at an end or in a carbon-carbon bond,
R^{a} is a C₁-C₃₀ hydrocarbyl group which may contain halogen atom or a heteroatom, two of R^{a} and two aromatic rings bonded to S⁺ may bond together to form a ring with a sulfur atom to which they are attached,
R^{b} is a C₁-C₁₈ hydrocarbyl group which may contain a heteroatom,
R¹ is halogen, nitro group, cyano group, hydroxy group, a C₁-C₂₀ hydrocarbyl group which may contain a heteroatom, C₁-C₂₀ hydrocarbyloxy group which may contain a heteroatom, or a C₁-C₂₀ hydrocarbylthio group which may contain a heteroatom, a plurality of R¹ may be identical or different and a plurality of R¹ may bond together to form a ring with the carbon atoms to which they are attached, when n2 is 2, 3 or 4,
R² is halogen, nitro group, cyano group, hydroxy group, a C₁-C₂₀ hydrocarbyl group which may contain a heteroatom, a C₁-C₂₀ hydrocarbyloxy group which may contain a heteroatom, or a C₁-C₂₀ hydrocarbylthio group which may contain a heteroatom, and a plurality of R² may be identical or different and a plurality of R² may bond together to form a ring with the carbon atoms to which they are attached, when n4 is 2, 3 or 4.

2. The sulfonium salt monomer of claim 1 which has the formula (A1): 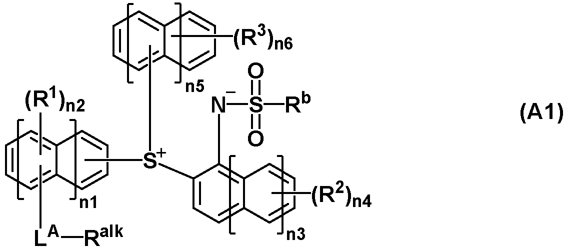 wherein n1 to n4, L^{A}, R^{alk}, R^{a}, R^{b}, R¹ and R² are as defined above,
n5 is 0 or 1, n6 is 0, 1, 2, 3 or 4,
R³ is halogen, nitro group, cyano group, hydroxy group, a C₁-C₂₀ hydrocarbyl group which may contain a heteroatom, a C₁-C₂₀ hydrocarbyloxy group which may contain a heteroatom, or a C₁-C₂₀ hydrocarbylthio group which may contain a heteroatom, and each R³ may be identical or different and a plurality of R³ may bond together to form a ring with the carbon atoms to which they are attached, when n6 is 2, 3 or 4.

3. The sulfonium salt of claim 2 which has the formula (A2): 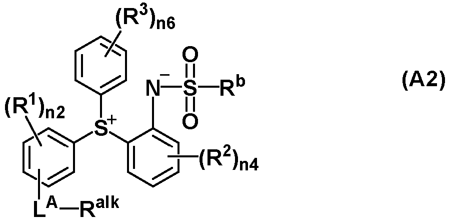 wherein n2, n4, n6, L^{A}, R^{alk}, R^{a}, R^{b}, R¹, R² and R³ are as defined above.

4. A quencher comprising the sulfonium salt of any one of claims 1 to 3.

5. A chemically amplified positive resist composition comprising the quencher of claim 4.

6. The chemically amplified positive resist composition of claim 5, further comprising a base polymer comprising a polymer adapted to increase its solubility in an alkaline developer by degrading under the action of an acid,
wherein the polymer comprises repeat units having the formula (B2-1) or (B2-2): 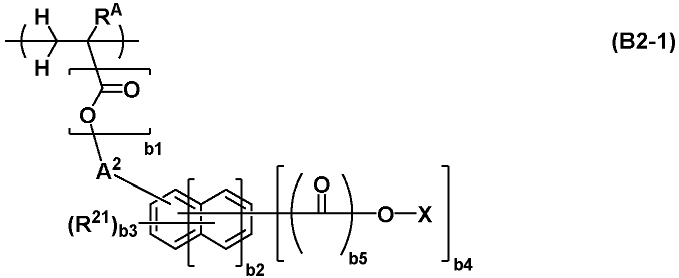
wherein R^{A} is hydrogen, fluorine, methyl group, or trifluoromethyl group,
b1 is 0 or 1, b2 is 0, 1 or 2, b3 is an integer which satisfies 0 ≤ b3 ≤ 5+2(b2)-b4, b4 is 1, 2 or 3, b5 is 0 or 1,
R²¹ is a halogen atom, C₁-C₆ saturated hydrocarbyl group which may be substituted with a halogen atom, C₁-C₆ saturated hydrocarbyloxy group which may be substituted with a halogen atom, or C₂-C₈ saturated hydrocarbylcarbonyloxy group which may be substituted with a halogen atom,
A² is a single bond or C₁-C₁₀ saturated hydrocarbylene group in which some constituent -CH₂- may be replaced by -O-, and
X is an acid labile group when b4 is 1, and X is hydrogen or an acid labile group, at least one being an acid labile group, when b4 is 2 or 3, 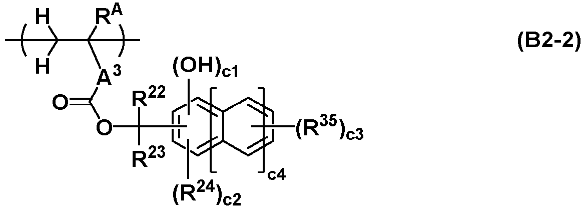
wherein c1 is 0, 1 or 2, c2 is 0, 1 or 2, c3 is 0, 1, 2, 3, 4 or 5, c4 is 0, 1 or 2,
R^{A} is hydrogen, fluorine, methyl group or trifluoromethyl group,
R²² and R²³ are each independently a C₁-C₁₀ hydrocarbyl group which may contain a heteroatom, and R²² and R²³ may bond together to form a ring with the carbon atom to which they are attached,
R²⁴ is each independently a fluorine atom, C₁-C₅ fluorinated alkyl group or C₁-C₅ fluorinated alkoxy group,
R²⁵ is each independently a C₁-C₁₀ hydrocarbyl group which may contain a heteroatom,
A³ is a single bond, phenylene group, naphthylene group or *-C(=O)-O-A³¹-, and A³¹ is a C₁-C₂₀ aliphatic hydrocarbylene group which may contain a hydroxy group, ether bond, ester bond or lactone ring, or a phenylene group or naphthylene group.

7. The chemically amplified positive resist composition of claim 6, wherein the polymer comprises repeat units having the formula (B1): 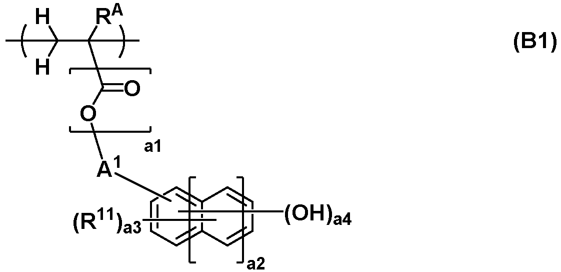 wherein a1 is 0 or 1, a2 is 0, 1 or 2, a3 is an integer which satisfies 0 ≤ a3 ≤ 5+2(a2)-a4, a4 is 1, 2 or 3,
R^{A} is hydrogen, fluorine, methyl group or trifluoromethyl group,
R¹¹ is a halogen atom, nitro group, carboxy group, a C₁-C₆ saturated hydrocarbyl group which may be substituted with a halogen atom, a C₁-C₆ saturated hydrocarbyloxy group which may be substituted with a halogen atom, or a C₂-C₈ saturated hydrocarbylcarbonyloxy group which may be substituted with a halogen atom, and
A¹ is a single bond or C₁-C₁₀ saturated hydrocarbylene group in which some constituent -CH₂- may be replaced by -O-.

8. The chemically amplified positive resist composition of claim 6 or 7, wherein the polymer further comprises repeat units of at least one type selected from repeat units having the formula (B3), repeat units having the formula (B4) and repeat units having the formula (B5): 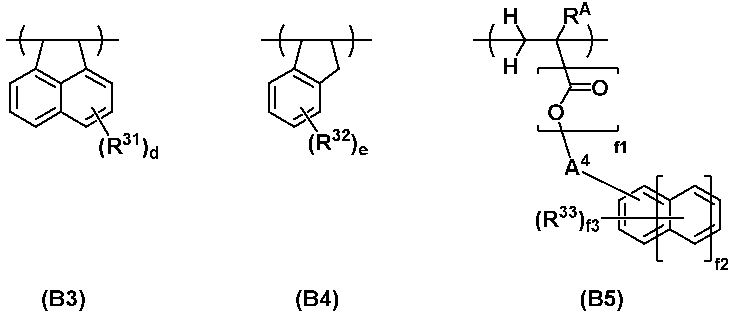 wherein d is 0, 1, 2, 3, 4, 5 or 6, e is 0, 1, 2, 3 or 4, f1 is 0 or 1, f2 is 0, 1 or 2, f3 is 0, 1, 2, 3, 4 or 5,
R^{A} is hydrogen, fluorine, methyl group or trifluoromethyl group,
R³¹ and R³² are each independently hydroxy group, halogen, a C₁-C₆ saturated hydrocarbyl group which may be substituted with a halogen atom, a C₁-C₆ saturated hydrocarbyloxy group which may be substituted with a halogen atom, or a C₂-C₈ saturated hydrocarbylcarbonyloxy group which may be substituted with a halogen atom,
R³³ is a C₁-C₂₀ saturated hydrocarbyl group, a C₁-C₂₀ saturated hydrocarbyloxy group, a C₂-C₂₀ saturated hydrocarbylcarbonyloxy group, a C₂-C₂₀ saturated hydrocarbyloxyhydrocarbyl group, a C₂-C₂₀ saturated hydrocarbylthiohydrocarbyl group, halogen atom, nitro group, or cyano group, and may be a hydroxy group when f2 is 1 or 2, and
A⁴ is a single bond or C₁-C₁₀ saturated hydrocarbylene group in which some constituent -CH₂- may be replaced by -O-.

9. The chemically amplified positive resist composition of any one of claims 6 to 8, wherein the polymer further comprises repeat units of at least one type selected from repeat units having the formula (B6), repeat units having the formula (B7), repeat units having the formula (B8), repeat units having the formula (B9), and repeat units having the formula (B10): 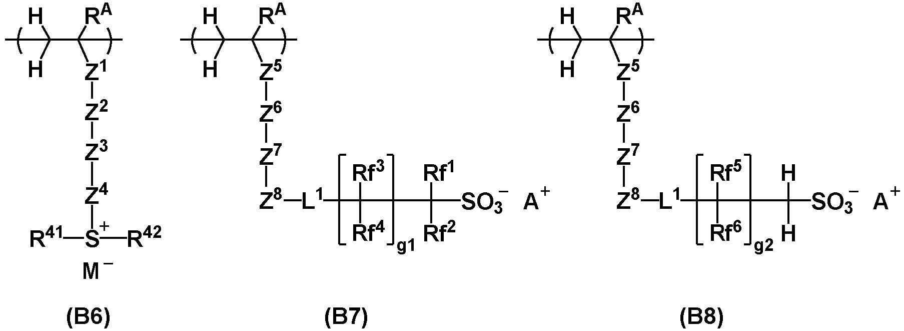 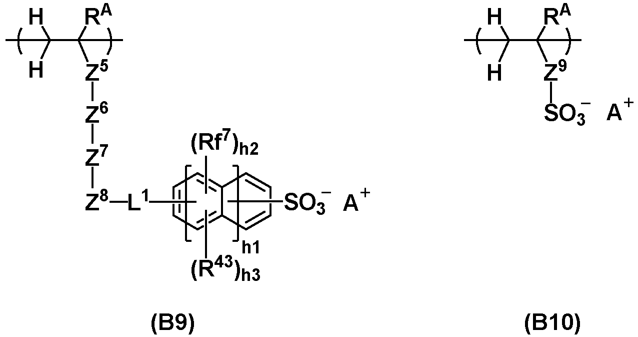 wherein g1 and g2 are each independently 0, 1, 2 or 3, h1 is 0 or 1, h2 is 0, 1, 2, 3 or 4, h3 is 0, 1, 2, 3 or 4, h2+h3 is from 0 to 4 when h1 is 0, and h2+h3 is from 0 to 6 when h1 is 1,
R^{A} is each independently hydrogen, fluorine, methyl group or trifluoromethyl group,
Z¹ is a single bond or optionally substituted phenylene group,
Z² is a single bond, *-C(=O)-O-Z²¹-, **-C(=O)-NH-Z²¹- or **-O-Z²¹-, Z²¹ is a C₁-C₆ aliphatic hydrocarbylene group, a phenylene group or a divalent group obtained by combining the foregoing, which may contain halogen, carbonyl group, ester bond, ether bond or hydroxy group,
Z³ is a single bond, ether bond, ester bond, amide bond, sulfonate ester bond, sulfonamide bond, carbonate bond or carbamate bond,
Z⁴ is a single bond, or a C₁-C₆ aliphatic hydrocarbylene group, phenylene group or a divalent group obtained by combining the foregoing, which may contain halogen, carbonyl group, ester bond, ether bond or hydroxy group,
Z⁵ is each independently a single bond, optionally substituted phenylene group, naphthylene group, or *-C(=O)-O-Z⁵¹-, Z⁵¹ is a C₁-C₁₀ aliphatic hydrocarbylene group which may contain halogen, hydroxy group, ether bond, ester bond or lactone ring, or phenylene group or naphthylene group,
Z⁶ is a single bond, ether bond, ester bond, amide bond, sulfonate ester bond, sulfonamide bond, carbonate bond or carbamate bond,
Z⁷ is each independently a single bond, ***-Z⁷¹-C(=O)-O-, ***-C(=O)-NH-Z⁷¹-, or ***-O-Z⁷¹-, Z⁷¹ is a C₁-C₂₀ hydrocarbylene group which may contain a heteroatom,
Z⁸ is each independently a single bond, ****-Z⁸¹-C(=O)-O-, ****-C(=O)-NH-Z⁸¹-, or ****-O-Z⁸¹-, Z⁸¹ is a C₁-C₂₀ hydrocarbylene group which may contain a heteroatom,
Z⁹ is a single bond, methylene group, ethylene group, phenylene group, fluorinated phenylene group, trifluoromethyl-substituted phenylene group, *-C(=O)-O-Z⁹¹-, *-C(=O)-N(H)-Z⁹¹-, or *-O-Z⁹¹-, Z⁹¹ is a C₁-C₆ aliphatic hydrocarbylene group, phenylene group, fluorinated phenylene group or trifluoromethyl substituted phenylene group, which may contain a carbonyl group, ester bond, ether bond or hydroxy group,
* designates a point of attachment to the carbon atom in the backbone, ** designates a point of attachment to Z¹, *** designates a point of attachment to Z⁶, **** designates a point of attachment to Z⁷,
L¹ is a single bond, ether bond, ester bond, carbonyl group, sulfonate ester bond, sulfonamide bond, carbonate bond or carbamate bond,
Rf¹ and Rf² are each independently fluorine, or a C₁-C₆ fluorinated saturated hydrocarbyl group,
Rf³ and Rf⁴ are each independently hydrogen, fluorine, or a C₁-C₆ fluorinated saturated hydrocarbyl group,
Rf⁵ and Rf⁶ are each independently hydrogen, fluorine, or a C₁-C₆ fluorinated saturated hydrocarbyl group, it is excluded that all Rf⁵ and Rf⁶ are hydrogen at the same time,
Rf⁷ is fluorine, a C₁-C₆ fluorinated alkyl group, C₁-C₆ fluorinated alkoxy group or C₁-C₆ fluorinated alkylthio group,
R⁴¹ and R⁴² are each independently a C₁-C₂₀ hydrocarbyl group which may contain a heteroatom, R⁴¹ and R⁴² may bond together to form a ring with sulfur to which they are attached,
R⁴³ is halogen exclusive of iodine, or a C₁-C₂₀ hydrocarbyl group which may contain a heteroatom, each R⁴³ may be identical or different and a plurality of R⁴³ may bond together to form a ring with the carbon atoms to which they are attached, when h3 is 2, 3 or 4,
M⁻ is a non-nucleophilic counter ion, and
A⁺ is an onium cation.

10. The chemically amplified positive resist composition of any one of claims 6 to 9, wherein repeat units having an aromatic ring structure account for 60 mol% or more of the overall repeat units of the polymer in the base polymer.

11. The chemically amplified positive resist composition of any one of claims 5 to 10, further comprising an organic solvent.

12. The chemically amplified positive resist composition of any one of claims 5 to 11, further comprising a photoacid generator.

13. The chemically amplified positive resist composition of any one of claims 5 to 12, further comprising a fluorinated polymer comprising repeat units of at least one type selected from repeat units having the formula (E1), repeat units having the formula (E2), repeat units having the formula (E3) and repeat units having the formula (E4) and optionally repeat units of at least one type selected from repeat units having the formula (E5) and repeat units having the formula (E6): 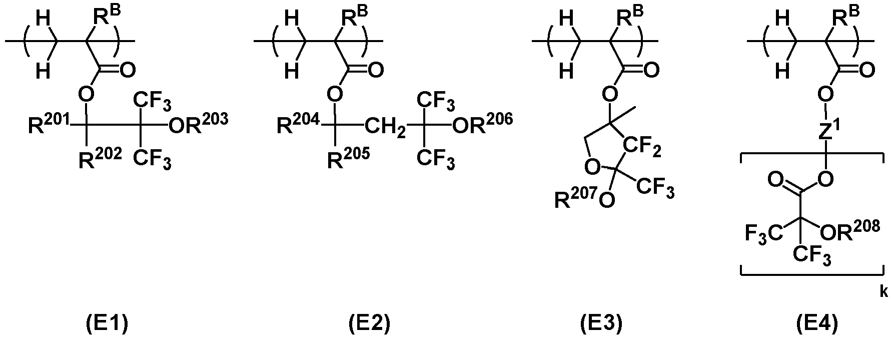 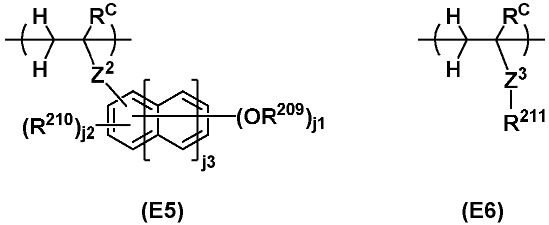 wherein j 1 is 1, 2 or 3, j2 is an integer meeting 0 ≤ j2 ≤ 5+2(j3)-j1, j3 is 0 or 1, k is 1, 2 or 3,
R^{B} is each independently hydrogen, fluorine, methyl group, or trifluoromethyl group,
R^{C} is each independently hydrogen or methyl group,
R²⁰¹, R²⁰², R²⁰⁴ and R²⁰⁵ are each independently hydrogen or a C₁-C₁₀ saturated hydrocarbyl group,
R²⁰³, R²⁰⁶, R²⁰⁷ and R²⁰⁸ are each independently hydrogen, a C₁-C₁₅ hydrocarbyl group, C₁-C₁₅ fluorinated hydrocarbyl group, or acid labile group, and when R²⁰³, R²⁰⁶, R²⁰⁷ and R²⁰⁸ each are a hydrocarbyl group or fluorinated hydrocarbyl group, an ether bond or carbonyl group may intervene in a carbon-carbon bond,
R²⁰⁹ is hydrogen or a C₁-C₅ straight or branched hydrocarbyl group in which a heteroatom-containing moiety may intervene in a carbon-carbon bond,
R²¹⁰ is a C₁-C₅ straight or branched hydrocarbyl group in which a heteroatom containing moiety may intervene in a carbon-carbon bond,
R²¹¹ is a C₁-C₂₀ saturated hydrocarbyl group in which at least one hydrogen is substituted by fluorine, and in which some constituent -CH₂- may be replaced by an ester bond or ether bond,
Z¹ is a C₁-C₂₀ (k+1)-valent hydrocarbon group or C₁-C₂₀ (k+1)-valent fluorinated hydrocarbon group,
Z² is a single bond, *-C(=O)-O- or *-C(=O)-NH-, * designates a point of attachment to the carbon atom in the backbone,
Z³ is a single bond, -O-, *-C(=O)-O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond or sulfonamide bond, and * designates a point of attachment to the carbon atom in the backbone.

14. A resist pattern forming process comprising the steps of:
applying the chemically amplified positive resist composition of any of claims 5 to 13 to form a resist film on a substrate,
exposing the resist film patternwise to high-energy radiation, and
developing the exposed resist film in an alkaline developer.

15. The resist pattern forming process of claim 14 wherein the high-energy radiation is EUV of wavelength 3 to 15 nm or EB.
